(19) **Europäisches Patentamt**
**European Patent Office**
**Office européen des brevets**

(11) **EP 3 489 688 A1**

(12) **EUROPEAN PATENT APPLICATION**

(43) Date of publication:
**29.05.2019 Bulletin 2019/22**

(51) Int Cl.:
***G01N 33/68*** (2006.01)

(21) Application number: **18189709.1**

(22) Date of filing: **23.06.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.06.2010 US 35796510 P**
**23.06.2010 US 35795610 P**
**14.07.2010 US 36430010 P**
**14.07.2010 US 36430410 P**

(62) Document number(s) of the earlier application(s) in accordance with Art. 76 EPC:
**15151607.7 / 2 899 545**
**11798515.0 / 2 585 826**

(71) Applicant: **Astute Medical, Inc.**
**San Diego, CA 92121 (US)**

(72) Inventors:
• **Anderberg, Joseph**
**Encinitas, CA 92024 (US)**

• **Gray, Jeff**
**Solana Beach, CA 92075 (US)**
• **McPherson, Paul**
**Encinitas, CA 92024 (US)**
• **Nakamura, Kevin**
**Cardiff by The Sea, CA 92007 (US)**
• **Kampf, James Patrick**
**San Diego, CA 92130 (US)**

(74) Representative: **Schiweck Weinzierl Koch**
**Patentanwälte Partnerschaft mbB**
**Ganghoferstraße 68 B**
**80339 München (DE)**

Remarks:
•Claims filed after the date of filing of the application/after the date of receipt of the divisional application (Rule 68(4) EPC).
•This application was filed on 20-08-2018 as a divisional application to the application mentioned under INID code 62.

(54) **METHODS AND COMPOSITIONS FOR DIAGNOSIS AND PROGNOSIS OF RENAL INJURY AND RENAL FAILURE**

(57)    The present invention relates to methods and compositions for monitoring, diagnosis, prognosis, and determination of treatment regimens in subjects suffering from or suspected of having a renal injury. In particular, the invention relates to using a one or more assays configured to detect a kidney injury marker selected from the group consisting of C-C Motif chemokine 24, Cancer antigen CA 15-3, C-C Motif chemokine 18, , Cathepsin D, C-X-C Motif chemokine 13, C-C motif chemokine 8, Interleukin-2 receptor alpha chain, Insulin-like growth factor-binding protein 3, Interleukin-11, Matrix Metalloproteinase-8, Transforming growth factor alpha, IgG1, and IgG2 as diagnostic and prognostic biomarkers in renal injuries.

EP 3 489 688 A1

**Description**

The present application claims priority to U.S. Provisional Patent Application No. 61/357,965 filed June 23, 2010; U.S. Provisional Patent Application No. 61/357,956 filed June 23, 2010; U.S. Provisional Patent Application No. 61/364,304 filed July 14, 2010; and U.S. Provisional Patent Application No. 61/364,300 filed July 14, 2010, each of which is hereby incorporated in its entirety including all tables, figures, and claims.

BACKGROUND OF THE INVENTION

The following discussion of the background of the invention is merely provided to aid the reader in understanding the invention and is not admitted to describe or constitute prior art to the present invention.
The kidney is responsible for water and solute excretion from the body. Its functions include maintenance of acid-base balance, regulation of electrolyte concentrations, control of blood volume, and regulation of blood pressure. As such, loss of kidney function through injury and/or disease results in substantial morbidity and mortality. A detailed discussion of renal injuries is provided in Harrison's Principles of Internal Medicine, 17th Ed., McGraw Hill, New York, pages 1741-1830, which are hereby incorporated by reference in their entirety. Renal disease and/or injury may be acute or chronic. Acute and chronic kidney disease are described as follows (from Current Medical Diagnosis & Treatment 2008, 47th Ed, McGraw Hill, New York, pages 785-815, which are hereby incorporated by reference in their entirety): "Acute renal failure is worsening of renal function over hours to days, resulting in the retention of nitrogenous wastes (such as urea nitrogen) and creatinine in the blood. Retention of these substances is called azotemia. Chronic renal failure (chronic kidney disease) results from an abnormal loss of renal function over months to years".
Acute renal failure (ARF, also known as acute kidney injury, or AKI) is an abrupt (typically detected within about 48 hours to 1 week) reduction in glomerular filtration. This loss of filtration capacity results in retention of nitrogenous (urea and creatinine) and non-nitrogenous waste products that are normally excreted by the kidney, a reduction in urine output, or both. It is reported that ARF complicates about 5% of hospital admissions, 4-15% of cardiopulmonary bypass surgeries, and up to 30% of intensive care admissions. ARF may be categorized as prerenal, intrinsic renal, or postrenal in causation. Intrinsic renal disease can be further divided into glomerular, tubular, interstitial, and vascular abnormalities. Major causes of ARF are described in the following table, which is adapted from the Merck Manual, 17th ed., Chapter 222, and which is hereby incorporated by reference in their entirety:

| Type | Risk Factors |
|---|---|
| **Prerenal** | |
| ECF volume depletion | Excessive diuresis, hemorrhage, GI losses, loss of intravascular fluid into the extravascular space (due to ascites, peritonitis, pancreatitis, or burns), loss of skin and mucus membranes, renal salt- and water-wasting states |
| Low cardiac output | Cardiomyopathy, MI, cardiac tamponade, pulmonary embolism, pulmonary hypertension, positive-pressure mechanical ventilation |
| Low systemic vascular resistance | Septic shock, liver failure, antihypertensive drugs |
| Increased renal vascular resistance | NSAIDs, cyclosporines, tacrolimus, hypercalcemia, anaphylaxis, anesthetics, renal artery obstruction, renal vein thrombosis, sepsis, hepatorenal syndrome |
| Decreased efferent arteriolar tone (leading to decreased GFR from reduced glomerular transcapillary pressure, especially in patients with bilateral renal artery stenosis) | ACE inhibitors or angiotensin II receptor blockers |
| **Intrinsic Renal** | |
| Acute tubular injury | Ischemia (prolonged or severe prerenal state): surgery, hemorrhage, arterial or venous obstruction; Toxins: NSAIDs, cyclosporines, tacrolimus, aminoglycosides, foscarnet, ethylene glycol, hemoglobin, myoglobin, ifosfamide, heavy metals, methotrexate, radiopaque contrast agents, streptozotocin |

(continued)

| Intrinsic Renal | |
|---|---|
| Acute glomerulonephritis | ANCA-associated: Crescentic glomerulonephritis, polyarteritis nodosa, Wegener's granulomatosis; Anti-GBM glomerulonephritis: Goodpasture's syndrome; Immune-complex: Lupus glomerulonephritis, postinfectious glomerulonephritis, cryoglobulinemic glomerulonephritis |
| Acute tubulo interstitial nephritis | Drug reaction (eg, β-lactams, NSAIDs, sulfonamides, ciprofloxacin, thiazide diuretics, furosemide, phenytoin, allopurinol, pyelonephritis, papillary necrosis |
| Acute vascular nephropathy | Vasculitis, malignant hypertension, thrombotic microangiopathies, scleroderma, atheroembolism |
| Infiltrative diseases | Lymphoma, sarcoidosis, leukemia |
| **Postrenal** | |
| **Type** | **Risk Factors** |
| Tubular precipitation | Uric acid (tumor lysis), sulfonamides, triamterene, acyclovir, indinavir, methotrexate, ethylene glycol ingestion, myeloma protein, myoglobin |
| Ureteral obstruction | Intrinsic: Calculi, clots, sloughed renal tissue, fungus ball, edema, malignancy, congenital defects; Extrinsic: Malignancy, retroperitoneal fibrosis, ureteral trauma during surgery or high impact injury |
| Bladder obstruction | Mechanical: Benign prostatic hyperplasia, prostate cancer, bladder cancer, urethral strictures, phimosis, paraphimosis, urethral valves, obstructed indwelling urinary catheter; Neurogenic: Anticholinergic drugs, upper or lower motor neuron lesion |

In the case of ischemic ARF, the course of the disease may be divided into four phases. During an initiation phase, which lasts hours to days, reduced perfusion of the kidney is evolving into injury. Glomerular ultrafiltration reduces, the flow of filtrate is reduced due to debris within the tubules, and back leakage of filtrate through injured epithelium occurs. Renal injury can be mediated during this phase by reperfusion of the kidney. Initiation is followed by an extension phase which is characterized by continued ischemic injury and inflammation and may involve endothelial damage and vascular congestion. During the maintenance phase, lasting from 1 to 2 weeks, renal cell injury occurs, and glomerular filtration and urine output reaches a minimum. A recovery phase can follow in which the renal epithelium is repaired and GFR gradually recovers. Despite this, the survival rate of subjects with ARF may be as low as about 60%.

Acute kidney injury caused by radiocontrast agents (also called contrast media) and other nephrotoxins such as cyclosporine, antibiotics including aminoglycosides and anticancer drugs such as cisplatin manifests over a period of days to about a week. Contrast induced nephropathy (CIN, which is AKI caused by radiocontrast agents) is thought to be caused by intrarenal vasoconstriction (leading to ischemic injury) and from the generation of reactive oxygen species that are directly toxic to renal tubular epithelial cells. CIN classically presents as an acute (onset within 24-48h) but reversible (peak 3-5 days, resolution within 1 week) rise in blood urea nitrogen and serum creatinine.

A commonly reported criteria for defining and detecting AKI is an abrupt (typically within about 2-7 days or within a period of hospitalization) elevation of serum creatinine. Although the use of serum creatinine elevation to define and detect AKI is well established, the magnitude of the serum creatinine elevation and the time over which it is measured to define AKI varies considerably among publications. Traditionally, relatively large increases in serum creatinine such as 100%, 200%, an increase of at least 100% to a value over 2 mg/dL and other definitions were used to define AKI. However, the recent trend has been towards using smaller serum creatinine rises to define AKI. The relationship between serum creatinine rise, AKI and the associated health risks are reviewed in Praught and Shlipak, Curr Opin Nephrol Hypertens 14:265-270, 2005 and Chertow et al, J Am Soc Nephrol 16: 3365-3370, 2005, which, with the references listed therein, are hereby incorporated by reference in their entirety. As described in these publications, acute worsening renal function (AKI) and increased risk of death and other detrimental outcomes are now known to be associated with very small increases in serum creatinine. These increases may be determined as a relative (percent) value or a nominal value. Relative increases in serum creatinine as small as 20% from the pre-injury value have been reported to indicate acutely worsening renal function (AKI) and increased health risk, but the more commonly reported value to define AKI and increased health risk is a relative increase of at least 25%. Nominal increases as small as 0.3 mg/dL, 0.2 mg/dL or even 0.1 mg/dL have been reported to indicate worsening renal function and increased risk of death. Various time periods for the serum creatinine to rise to these threshold values have been used to define AKI, for example, ranging from 2 days, 3 days, 7 days, or a variable period defined as the time the patient is in the hospital or intensive care unit. These studies indicate there is not a particular threshold serum creatinine rise (or time period for the rise) for worsening renal function or AKI, but rather a continuous increase in risk with increasing magnitude of serum creatinine rise.

One study (Lassnigg et all, J Am Soc Nephrol 15:1597-1605, 2004, hereby incorporated by reference in its entirety) investigated both increases and decreases in serum creatinine. Patients with a mild fall in serum creatinine of -0.1 to -0.3 mg/dL following heart surgery had the lowest mortality rate. Patients with a larger fall in serum creatinine (more than or equal to -0.4 mg/dL) or any increase in serum creatinine had a larger mortality rate. These findings caused the authors to conclude that even very subtle changes in renal function (as detected by small creatinine changes within 48 hours of surgery) seriously effect patient's outcomes. In an effort to reach consensus on a unified classification system for using serum creatinine to define AKI in clinical trials and in clinical practice, Bellomo et al., Crit Care. 8(4):R204-12, 2004, which is hereby incorporated by reference in its entirety, proposes the following classifications for stratifying AKI patients:

"Risk": serum creatinine increased 1.5 fold from baseline OR urine production of <0.5 ml/kg body weight/hr for 6 hours;

"Injury": serum creatinine increased 2.0 fold from baseline OR urine production <0.5 ml/kg/hr for 12 h;

"Failure": serum creatinine increased 3.0 fold from baseline OR creatinine >355 $\mu$mol/l (with a rise of >44) or urine output below 0.3 ml/kg/hr for 24 h or anuria for at least 12 hours;

And included two clinical outcomes:

"Loss": persistent need for renal replacement therapy for more than four weeks.

"ESRD": end stage renal disease the need for dialysis for more than 3 months.

These criteria are called the RIFLE criteria, which provide a useful clinical tool to classify renal status. As discussed in Kellum, Crit. Care Med. 36: S141-45, 2008 and Ricci et al., Kidney Int. 73, 538-546, 2008, each hereby incorporated by reference in its entirety, the RIFLE criteria provide a uniform definition of AKI which has been validated in numerous studies.

More recently, Mehta *et al., Crit. Care* 11:R31 (doi:10.1186.cc5713), 2007, hereby incorporated by reference in its entirety, proposes the following similar classifications for stratifying AKI patients, which have been modified from RIFLE:

"Stage I": increase in serum creatinine of more than or equal to 0.3 mg/dL ($\geq$ 26.4 $\mu$mol/L) or increase to more than or equal to 150% (1.5-fold) from baseline OR urine output less than 0.5 mL/kg per hour for more than 6 hours;

"Stage II": increase in serum creatinine to more than 200% (> 2-fold) from baseline OR urine output less than 0.5 mL/kg per hour for more than 12 hours;

"Stage III": increase in serum creatinine to more than 300% (> 3-fold) from baseline OR serum creatinine $\geq$ 354 $\mu$mol/L accompanied by an acute increase of at least 44 $\mu$mol/L OR urine output less than 0.3 mL/kg per hour for 24 hours or anuria for 12 hours.

The CIN Consensus Working Panel (McCollough et al, Rev Cardiovasc Med. 2006;7(4):177-197, hereby incorporated by reference in its entirety) uses a serum creatinine rise of 25% to define Contrast induced nephropathy (which is a type of AKI).Although various groups propose slightly different criteria for using serum creatinine to detect AKI, the consensus is that small changes in serum creatinine, such as 0.3 mg/dL or 25%, are sufficient to detect AKI (worsening renal function) and that the magnitude of the serum creatinine change is an indicator of the severity of the AKI and mortality risk.

Although serial measurement of serum creatinine over a period of days is an accepted method of detecting and diagnosing AKI and is considered one of the most important tools to evaluate AKI patients, serum creatinine is generally regarded to have several limitations in the diagnosis, assessment and monitoring of AKI patients. The time period for serum creatinine to rise to values (e.g., a 0.3 mg/dL or 25% rise) considered diagnostic for AKI can be 48 hours or longer depending on the definition used. Since cellular injury in AKI can occur over a period of hours, serum creatinine elevations detected at 48 hours or longer can be a late indicator of injury, and relying on serum creatinine can thus delay diagnosis of AKI. Furthermore, serum creatinine is not a good indicator of the exact kidney status and treatment needs during the most acute phases of AKI when kidney function is changing rapidly. Some patients with AKI will recover fully, some will need dialysis (either short term or long term) and some will have other detrimental outcomes including death, major adverse cardiac events and chronic kidney disease. Because serum creatinine is a marker of filtration rate, it does not differentiate between the causes of AKI (pre-renal, intrinsic renal, post-renal obstruction, atheroembolic, etc) or the category or location of injury in intrinsic renal disease (for example, tubular, glomerular or interstitial in origin). Urine output is similarly limited, Knowing these things can be of vital importance in managing and treating patients with AKI.

These limitations underscore the need for better methods to detect and assess AKI, particularly in the early and subclinical stages, but also in later stages when recovery and repair of the kidney can occur. Furthermore, there is a need to better identify patients who are at risk of having an AKI.

BRIEF SUMMARY OF THE INVENTION

It is an object of the invention to provide methods and compositions for evaluating renal function in a subject. As described herein, measurement of one or more biomarkers selected from the group consisting of Cancer antigen CA 15-3, C-C Motif chemokine 18, C-C Motif chemokine 24, Cathepsin D, C-X-C Motif chemokine 13, C-C motif chemokine 8, Interleukin-2 receptor alpha chain, Insulin-like growth factor-binding protein 3, Interleukin-11, Matrix Metalloproteinase-8, Transforming growth factor alpha, IgG1, and IgG2 (each referred to herein as a "kidney injury marker") can be used for diagnosis, prognosis, risk stratification, staging, monitoring, categorizing and determination of further diagnosis and treatment regimens in subjects suffering or at risk of suffering from an injury to renal function, reduced renal function, and/or acute renal failure (also called acute kidney injury).

The kidney injury markers of the present invention may be used, individually or in panels comprising a plurality of kidney injury markers, for risk stratification (that is, to identify subjects at risk for a future injury to renal function, for future progression to reduced renal function, for future progression to ARF, for future improvement in renal function, *etc.*); for diagnosis of existing disease (that is, to identify subjects who have suffered an injury to renal function, who have progressed to reduced renal function, who have progressed to ARF, *etc.*); for monitoring for deterioration or improvement of renal function; and for predicting a future medical outcome, such as improved or worsening renal function, a decreased or increased mortality risk, a decreased or increased risk that a subject will require renal replacement therapy (*i.e.,* hemodialysis, peritoneal dialysis, hemofiltration, and/or renal transplantation, a decreased or increased risk that a subject will recover from an injury to renal function, a decreased or increased risk that a subject will recover from ARF, a decreased or increased risk that a subject will progress to end stage renal disease, a decreased or increased risk that a subject will progress to chronic renal failure, a decreased or increased risk that a subject will suffer rejection of a transplanted kidney, *etc.*

In a first aspect, the present invention relates to methods for evaluating renal status in a subject. These methods comprise performing an assay method that is configured to detect one or more biomarkers selected from the group consisting of Cancer antigen CA 15-3, C-C Motif chemokine 18, C-C Motif chemokine 24, Cathepsin D, C-X-C Motif chemokine 13, C-C motif chemokine 8, Interleukin-2 receptor alpha chain, Insulin-like growth factor-binding protein 3, Interleukin-11, Matrix Metalloproteinase-8, Transforming growth factor alpha, IgG1, and IgG2 is/are then correlated to the renal status of the subject. This correlation to renal status may include correlating the assay result(s) to one or more of risk stratification, diagnosis, prognosis, staging, classifying and monitoring of the subject as described herein. Thus, the present invention utilizes one or more kidney injury markers of the present invention for the evaluation of renal injury.

In certain embodiments, the methods for evaluating renal status described herein are methods for risk stratification of the subject; that is, assigning a likelihood of one or more future changes in renal status to the subject. In these embodiments, the assay result(s) is/are correlated to one or more such future changes. The following are preferred risk stratification embodiments.

In preferred risk stratification embodiments, these methods comprise determining a subject's risk for a future injury to renal function, and the assay result(s) is/are correlated to a likelihood of such a future injury to renal function. For example, the measured concentration(s) may each be compared to a threshold value. For a "positive going" kidney injury marker, an increased likelihood of suffering a future injury to renal function is assigned to the subject when the measured concentration is above the threshold, relative to a likelihood assigned when the measured concentration is below the threshold. For a "negative going" kidney injury marker, an increased likelihood of suffering a future injury to renal function is assigned to the subject when the measured concentration is below the threshold, relative to a likelihood assigned when the measured concentration is above the threshold.

In other preferred risk stratification embodiments, these methods comprise determining a subject's risk for future reduced renal function, and the assay result(s) is/are correlated to a likelihood of such reduced renal function.

For example, the measured concentrations may each be compared to a threshold value. For a "positive going" kidney injury marker, an increased likelihood of suffering a future reduced renal function is assigned to the subject when the measured concentration is above the threshold, relative to a likelihood assigned when the measured concentration is below the threshold. For a "negative going" kidney injury marker, an increased likelihood of future reduced renal function is assigned to the subject when the measured concentration is below the threshold, relative to a likelihood assigned when the measured concentration is above the threshold.

In still other preferred risk stratification embodiments, these methods comprise determining a subject's likelihood for a future improvement in renal function, and the assay result(s) is/are correlated to a likelihood of such a future improvement in renal function. For example, the measured concentration(s) may each be compared to a threshold value. For a "positive going" kidney injury marker, an increased likelihood of a future improvement in renal function is assigned to the subject when the measured concentration is below the threshold, relative to a likelihood assigned when the measured concentration is above the threshold. For a "negative going" kidney injury marker, an increased likelihood of a future improvement in renal function is assigned to the subject when the measured concentration is above the threshold, relative to a likelihood assigned when the measured concentration is below the threshold.

In yet other preferred risk stratification embodiments, these methods comprise determining a subject's risk for progression to ARF, and the result(s) is/are correlated to a likelihood of such progression to ARF. For example, the measured concentration(s) may each be compared to a threshold value. For a "positive going" kidney injury marker, an increased likelihood of progression to ARF is assigned to the subject when the measured concentration is above the threshold, relative to a likelihood assigned when the measured concentration is below the threshold. For a "negative going" kidney injury marker, an increased likelihood of progression to ARF is assigned to the subject when the measured concentration is below the threshold, relative to a likelihood assigned when the measured concentration is above the threshold.

And in other preferred risk stratification embodiments, these methods comprise determining a subject's outcome risk, and the assay result(s) is/are correlated to a likelihood of the occurrence of a clinical outcome related to a renal injury suffered by the subject. For example, the measured concentration(s) may each be compared to a threshold value. For a "positive going" kidney injury marker, an increased likelihood of one or more of: acute kidney injury, progression to a worsening stage of AKI, mortality, a requirement for renal replacement therapy, a requirement for withdrawal of renal toxins, end stage renal disease, heart failure, stroke, myocardial infarction, progression to chronic kidney disease, etc., is assigned to the subject when the measured concentration is above the threshold, relative to a likelihood assigned when the measured concentration is below the threshold. For a "negative going" kidney injury marker, an increased likelihood of one or more of: acute kidney injury, progression to a worsening stage of AKI, mortality, a requirement for renal replacement therapy, a requirement for withdrawal of renal toxins, end stage renal disease, heart failure, stroke, myocardial infarction, progression to chronic kidney disease, etc., is assigned to the subject when the measured concentration is below the threshold, relative to a likelihood assigned when the measured concentration is above the threshold.

In such risk stratification embodiments, preferably the likelihood or risk assigned is that an event of interest is more or less likely to occur within 180 days of the time at which the body fluid sample is obtained from the subject. In particularly preferred embodiments, the likelihood or risk assigned relates to an event of interest occurring within a shorter time period such as 18 months, 120 days, 90 days, 60 days, 45 days, 30 days, 21 days, 14 days, 7 days, 5 days, 96 hours, 72 hours, 48 hours, 36 hours, 24 hours, 12 hours, or less. A risk at 0 hours of the time at which the body fluid sample is obtained from the subject is equivalent to diagnosis of a current condition.

In preferred risk stratification embodiments, the subject is selected for risk stratification based on the pre-existence in the subject of one or more known risk factors for prerenal, intrinsic renal, or postrenal ARF. For example, a subject undergoing or having undergone major vascular surgery, coronary artery bypass, or other cardiac surgery; a subject having pre-existing congestive heart failure, preeclampsia, eclampsia, diabetes mellitus, hypertension, coronary artery disease, proteinuria, renal insufficiency, glomerular filtration below the normal range, cirrhosis, serum creatinine above the normal range, or sepsis; or a subject exposed to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin are all preferred subjects for monitoring risks according to the methods described herein. This list is not meant to be limiting. By "pre-existence" in this context is meant that the risk factor exists at the time the body fluid sample is obtained from the subject. In particularly preferred embodiments, a subject is chosen for risk stratification based on an existing diagnosis of injury to renal function, reduced renal function, or ARF.

In other embodiments, the methods for evaluating renal status described herein are methods for diagnosing a renal injury in the subject; that is, assessing whether or not a subject has suffered from an injury to renal function, reduced renal function, or ARF. In these embodiments, the assay result(s), for example measured concentration(s) of one or more biomarkers selected from the group consisting of Cancer antigen CA 15-3, C-C Motif chemokine 18, C-C Motif chemokine 24, Cathepsin D, C-X-C Motif chemokine 13, C-C motif chemokine 8, Interleukin-2 receptor alpha chain, Insulin-like growth factor-binding protein 3, Interleukin-11, Matrix Metalloproteinase-8, Transforming growth factor alpha, IgG1, and IgG2 is/are correlated to the occurrence or nonoccurrence of a change in renal status. The following are preferred diagnostic embodiments.

In preferred diagnostic embodiments, these methods comprise diagnosing the occurrence or nonoccurrence of an injury to renal function, and the assay result(s) is/are correlated to the occurrence or nonoccurrence of such an injury. For example, each of the measured concentration(s) may be compared to a threshold value. For a positive going marker, an increased likelihood of the occurrence of an injury to renal function is assigned to the subject when the measured concentration is above the threshold (relative to the likelihood assigned when the measured concentration is below the threshold); alternatively, when the measured concentration is below the threshold, an increased likelihood of the nonoccurrence of an injury to renal function may be assigned to the subject (relative to the likelihood assigned when the measured concentration is above the threshold). For a negative going marker, an increased likelihood of the occurrence of an injury to renal function is assigned to the subject when the measured concentration is below the threshold (relative to the likelihood assigned when the measured concentration is above the threshold); alternatively, when the measured concentration is above the threshold, an increased likelihood of the nonoccurrence of an injury to renal function may be assigned to the subject (relative to the likelihood assigned when the measured concentration is below the threshold).

In other preferred diagnostic embodiments, these methods comprise diagnosing the occurrence or nonoccurrence of reduced renal function, and the assay result(s) is/are correlated to the occurrence or nonoccurrence of an injury causing reduced renal function. For example, each of the measured concentration(s) may be compared to a threshold value. For a positive going marker, an increased likelihood of the occurrence of an injury causing reduced renal function is assigned to the subject when the measured concentration is above the threshold (relative to the likelihood assigned when the measured concentration is below the threshold); alternatively, when the measured concentration is below the threshold, an increased likelihood of the nonoccurrence of an injury causing reduced renal function may be assigned to the subject (relative to the likelihood assigned when the measured concentration is above the threshold). For a negative going marker, an increased likelihood of the occurrence of an injury causing reduced renal function is assigned to the subject when the measured concentration is below the threshold (relative to the likelihood assigned when the measured concentration is above the threshold); alternatively, when the measured concentration is above the threshold, an increased likelihood of the nonoccurrence of an injury causing reduced renal function may be assigned to the subject (relative to the likelihood assigned when the measured concentration is below the threshold).

In yet other preferred diagnostic embodiments, these methods comprise diagnosing the occurrence or nonoccurrence of ARF, and the assay result(s) is/are correlated to the occurrence or nonoccurrence of an injury causing ARF. For example, each of the measured concentration(s) may be compared to a threshold value. For a positive going marker, an increased likelihood of the occurrence of ARF is assigned to the subject when the measured concentration is above the threshold (relative to the likelihood assigned when the measured concentration is below the threshold); alternatively, when the measured concentration is below the threshold, an increased likelihood of the nonoccurrence of ARF may be assigned to the subject (relative to the likelihood assigned when the measured concentration is above the threshold). For a negative going marker, an increased likelihood of the occurrence of ARF is assigned to the subject when the measured concentration is below the threshold (relative to the likelihood assigned when the measured concentration is above the threshold); alternatively, when the measured concentration is above the threshold, an increased likelihood of the nonoccurrence of ARF may be assigned to the subject (relative to the likelihood assigned when the measured concentration is below the threshold).

In still other preferred diagnostic embodiments, these methods comprise diagnosing a subject as being in need of renal replacement therapy, and the assay result(s) is/are correlated to a need for renal replacement therapy. For example, each of the measured concentration(s) may be compared to a threshold value. For a positive going marker, an increased likelihood of the occurrence of an injury creating a need for renal replacement therapy is assigned to the subject when the measured concentration is above the threshold (relative to the likelihood assigned when the measured concentration is below the threshold); alternatively, when the measured concentration is below the threshold, an increased likelihood of the nonoccurrence of an injury creating a need for renal replacement therapy may be assigned to the subject (relative to the likelihood assigned when the measured concentration is above the threshold). For a negative going marker, an increased likelihood of the occurrence of an injury creating a need for renal replacement therapy is assigned to the subject when the measured concentration is below the threshold (relative to the likelihood assigned when the measured concentration is above the threshold); alternatively, when the measured concentration is above the threshold, an increased likelihood of the nonoccurrence of an injury creating a need for renal replacement therapy may be assigned to the subject (relative to the likelihood assigned when the measured concentration is below the threshold).

In still other preferred diagnostic embodiments, these methods comprise diagnosing a subject as being in need of renal transplantation, and the assay result(s0 is/are correlated to a need for renal transplantation. For example, each of the measured concentration(s) may be compared to a threshold value. For a positive going marker, an increased likelihood of the occurrence of an injury creating a need for renal transplantation is assigned to the subject when the measured concentration is above the threshold (relative to the likelihood assigned when the measured concentration is below the threshold); alternatively, when the measured concentration is below the threshold, an increased likelihood of the nonoccurrence of an injury creating a need for renal transplantation may be assigned to the subject (relative to the likelihood assigned when the measured concentration is above the threshold). For a negative going marker, an increased likelihood of the occurrence of an injury creating a need for renal transplantation is assigned to the subject when the measured concentration is below the threshold (relative to the likelihood assigned when the measured concentration is above the threshold); alternatively, when the measured concentration is above the threshold, an increased likelihood of the nonoccurrence of an injury creating a need for renal transplantation may be assigned to the subject (relative to the likelihood assigned when the measured concentration is below the threshold).

In still other embodiments, the methods for evaluating renal status described herein are methods for monitoring a renal injury in the subject; that is, assessing whether or not renal function is improving or worsening in a subject who has suffered from an injury to renal function, reduced renal function, or ARF. In these embodiments, the assay result(s), for example measured concentration(s) of one or more biomarkers selected from the group consisting of Cancer antigen CA 15-3, C-C Motif chemokine 18, C-C Motif chemokine 24, Cathepsin D, C-X-C Motif chemokine 13, C-C motif chemokine 8, Interleukin-2 receptor alpha chain, Insulin-like growth factor-binding protein 3, Interleukin-11, Matrix Metalloproteinase-8, Transforming growth factor alpha, IgG1, and IgG2 is/are correlated to the occurrence or nonoccurrence of a change in renal status. The following are preferred monitoring embodiments.

In preferred monitoring embodiments, these methods comprise monitoring renal status in a subject suffering from an injury to renal function, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In other preferred monitoring embodiments, these methods comprise monitoring renal status in a subject suffering from reduced renal function, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In yet other preferred monitoring embodiments, these methods comprise monitoring renal status in a subject suffering from acute renal failure, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In other additional preferred monitoring embodiments, these methods comprise monitoring renal status in a subject at risk of an injury to renal function due to the pre-existence of one or more known risk factors for prerenal, intrinsic renal, or postrenal ARF, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In still other embodiments, the methods for evaluating renal status described herein are methods for classifying a renal injury in the subject; that is, determining whether a renal injury in a subject is prerenal, intrinsic renal, or postrenal; and/or further subdividing these classes into subclasses such as acute tubular injury, acute glomerulonephritis acute tubulointerstitial nephritis, acute vascular nephropathy, or infiltrative disease; and/or assigning a likelihood that a subject will progress to a particular RIFLE stage. In these embodiments, the assay result(s), for example measured concentration(s) of one or more biomarkers selected from the group consisting of Cancer antigen CA 15-3, C-C Motif chemokine 18, C-C Motif chemokine 24, Cathepsin D, C-X-C Motif chemokine 13, C-C motif chemokine 8, Interleukin-2 receptor alpha chain, Insulin-like growth factor-binding protein 3, Interleukin-11, Matrix Metalloproteinase-8, Transforming growth factor alpha, IgG1, and IgG2 is/are correlated to a particular class and/or subclass. The following are preferred classification embodiments.

In preferred classification embodiments, these methods comprise determining whether a renal injury in a subject is prerenal, intrinsic renal, or postrenal; and/or further subdividing these classes into subclasses such as acute tubular injury, acute glomerulonephritis acute tubulointerstitial nephritis, acute vascular nephropathy, or infiltrative disease; and/or assigning a likelihood that a subject will progress to a particular RIFLE stage, and the assay result(s) is/are correlated to the injury classification for the subject. For example, the measured concentration may be compared to a threshold value, and when the measured concentration is above the threshold, a particular classification is assigned; alternatively, when the measured concentration is below the threshold, a different classification may be assigned to the subject.

A variety of methods may be used by the skilled artisan to arrive at a desired threshold value for use in these methods. For example, the threshold value may be determined from a population of normal subjects by selecting a concentration representing the 75th, 85th, 90th, 95th, or 99th percentile of a kidney injury marker measured in such normal subjects. Alternatively, the threshold value may be determined from a "diseased" population of subjects, e.g., those suffering from an injury or having a predisposition for an injury (e.g., progression to ARF or some other clinical outcome such as death, dialysis, renal transplantation, etc.), by selecting a concentration representing the 75th, 85th, 90th, 95th, or 99th percentile of a kidney injury marker measured in such subjects. In another alternative, the threshold value may be determined from a prior measurement of a kidney injury marker in the same subject; that is, a temporal change in the level of a kidney injury marker in the subject may be used to assign risk to the subject.

The foregoing discussion is not meant to imply, however, that the kidney injury markers of the present invention must be compared to corresponding individual thresholds. Methods for combining assay results can comprise the use of multivariate logistical regression, loglinear modeling, neural network analysis, n-of-m analysis, decision tree analysis, calculating ratios of markers, etc. This list is not meant to be limiting. In these methods, a composite result which is determined by combining individual markers may be treated as if it is itself a marker; that is, a threshold may be determined for the composite result as described herein for individual markers, and the composite result for an individual patient compared to this threshold.

The ability of a particular test to distinguish two populations can be established using ROC analysis. For example, ROC curves established from a "first" subpopulation which is predisposed to one or more future changes in renal status, and a "second" subpopulation which is not so predisposed can be used to calculate a ROC curve, and the area under the curve provides a measure of the quality of the test. Preferably, the tests described herein provide a ROC curve area greater than 0.5, preferably at least 0.6, more preferably 0.7, still more preferably at least 0.8, even more preferably at least 0.9, and most preferably at least 0.95.

In certain aspects, the measured concentration of one or more kidney injury markers, or a composite of such markers, may be treated as continuous variables. For example, any particular concentration can be converted into a corresponding probability of a future reduction in renal function for the subject, the occurrence of an injury, a classification, etc. In yet another alternative, a threshold that can provide an acceptable level of specificity and sensitivity in separating a population of subjects into "bins" such as a "first" subpopulation (e.g., which is predisposed to one or more future changes in renal status, the occurrence of an injury, a classification, etc.) and a "second" subpopulation which is not so predisposed. A threshold value is selected to separate this first and second population by one or more of the following measures of test accuracy:

an odds ratio greater than 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less;

a specificity of greater than 0.5, preferably at least about 0.6, more preferably at least about 0.7, still more preferably at least about 0.8, even more preferably at least about 0.9 and most preferably at least about 0.95, with a corresponding sensitivity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;

a sensitivity of greater than 0.5, preferably at least about 0.6, more preferably at least about 0.7, still more preferably at least about 0.8, even more preferably at least about 0.9 and most preferably at least about 0.95, with a corresponding specificity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;

at least about 75% sensitivity, combined with at least about 75% specificity;

a positive likelihood ratio (calculated as sensitivity/(1-specificity)) of greater than 1, at least about 2, more preferably at least about 3, still more preferably at least about 5, and most preferably at least about 10; or

a negative likelihood ratio (calculated as (1-sensitivity)/specificity) of less than 1, less than or equal to about 0.5, more preferably less than or equal to about 0.3, and most preferably less than or equal to about 0.1.

The term "about" in the context of any of the above measurements refers to +/- 5% of a given measurement.

Multiple thresholds may also be used to assess renal status in a subject. For example, a "first" subpopulation which is predisposed to one or more future changes in renal status, the occurrence of an injury, a classification, etc., and a "second" subpopulation which is not so predisposed can be combined into a single group. This group is then subdivided into three or more equal parts (known as tertiles, quartiles, quintiles, etc., depending on the number of subdivisions). An odds ratio is assigned to subjects based on which subdivision they fall into. If one considers a tertile, the lowest or highest tertile can be used as a reference for comparison of the other subdivisions. This reference subdivision is assigned an odds ratio of 1. The second tertile is assigned an odds ratio that is relative to that first tertile. That is, someone in the second tertile might be 3 times more likely to suffer one or more future changes in renal status in comparison to someone in the first tertile. The third tertile is also assigned an odds ratio that is relative to that first tertile.

In certain embodiments, the assay method is an immunoassay. Antibodies for use in such assays will specifically bind a full length kidney injury marker of interest, and may also bind one or more polypeptides that are "related" thereto, as that term is defined hereinafter. Numerous immunoassay formats are known to those of skill in the art. Preferred body fluid samples are selected from the group consisting of urine, blood, serum, saliva, tears, and plasma. In the case of those kidney injury markers which are membrane proteins as described hereinafter, preferred assays detect soluble forms thereof.

The foregoing method steps should not be interpreted to mean that the kidney injury marker assay result(s) is/are used in isolation in the methods described herein. Rather, additional variables or other clinical indicia may be included in the methods described herein. For example, a risk stratification, diagnostic, classification, monitoring, etc. method may combine the assay result(s) with one or more variables measured for the subject selected from the group consisting of demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TIMI Risk Score for UA/NSTEMI, Framingham Risk Score, risk scores of Thakar et al. (J. Am. Soc. Nephrol. 16: 162-68, 2005), Mehran et al. (J. Am. Coll. Cardiol. 44: 1393-99, 2004), Wijeysundera et al. (JAMA 297: 1801-9, 2007), Goldstein and Chawla (Clin. J. Am. Soc. Nephrol. 5: 943-49, 2010), or Chawla et al. (Kidney Intl. 68: 2274-80, 2005)), a glomerular filtration rate, an estimated glomerular filtration rate, a urine production rate, a serum or plasma creatinine concentration, a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, a renal failure index calculated as urine sodium / (urine creatinine / plasma creatinine), a serum or plasma neutrophil gelatinase (NGAL) concentration, a urine NGAL concentration, a serum or plasma cystatin C concentration, a serum or plasma cardiac troponin concentration, a serum or plasma BNP concentration, a serum or plasma NTproBNP concentration, and a serum or plasma proBNP concentration. Other measures of renal function which may be combined with one or more kidney injury marker assay result(s) are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815, each of which are hereby incorporated by reference in their entirety.

When more than one marker is measured, the individual markers may be measured in samples obtained at the same time, or may be determined from samples obtained at different (e.g., an earlier or later) times. The individual markers may also be measured on the same or different body fluid samples. For example, one kidney injury marker may be measured in a serum or plasma sample and another kidney injury marker may be measured in a urine sample. In addition, assignment of a likelihood may combine an individual kidney injury marker assay result with temporal changes in one or more additional variables.

In various related aspects, the present invention also relates to devices and kits for performing the methods described herein. Suitable kits comprise reagents sufficient for performing an assay for at least one of the described kidney injury markers, together with instructions for performing the described threshold comparisons.

In certain embodiments, reagents for performing such assays are provided in an assay device, and such assay devices may be included in such a kit. Preferred reagents can comprise one or more solid phase antibodies, the solid phase antibody comprising antibody that detects the intended biomarker target(s) bound to a solid support. In the case of sandwich immunoassays, such reagents can also include one or more detectably labeled antibodies, the detectably labeled antibody comprising antibody that detects the intended biomarker target(s) bound to a detectable label. Additional optional elements that may be provided as part of an assay device are described hereinafter.

Detectable labels may include molecules that are themselves detectable (e.g., fluorescent moieties, electrochemical labels, ecl (electrochemical luminescence) labels, metal chelates, colloidal metal particles, etc.) as well as molecules that may be indirectly detected by production of a detectable reaction product (e.g., enzymes such as horseradish peroxidase, alkaline phosphatase, etc.) or through the use of a specific binding molecule which itself may be detectable (e.g., a labeled antibody that binds to the second antibody, biotin, digoxigenin, maltose, oligohistidine, 2,4-dintrobenzene, phenylarsenate, ssDNA, dsDNA, etc.).

Generation of a signal from the signal development element can be performed using various optical, acoustical, and electrochemical methods well known in the art. Examples of detection modes include fluorescence, radiochemical detection, reflectance, absorbance, amperometry, conductance, impedance, interferometry, ellipsometry, etc. In certain of these methods, the solid phase antibody is coupled to a transducer (e.g., a diffraction grating, electrochemical sensor, etc) for generation of a signal, while in others, a signal is generated by a transducer that is spatially separate from the solid phase antibody (e.g., a fluorometer that employs an excitation light source and an optical detector). This list is not meant to be limiting. Antibody-based biosensors may also be employed to determine the presence or amount of analytes that optionally eliminate the need for a labeled molecule.

## DETAILED DESCRIPTION OF THE INVENTION

The present invention relates to methods and compositions for diagnosis, differential diagnosis, risk stratification, monitoring, classifying and determination of treatment regimens in subjects suffering or at risk of suffering from injury to renal function, reduced renal function and/or acute renal failure through measurement of one or more kidney injury markers. In various embodiments, a measured concentration of one or more biomarkers selected from the group consisting of Cancer antigen CA 15-3, C-C Motif chemokine 18, C-C Motif chemokine 24, Cathepsin D, C-X-C Motif chemokine 13, C-C motif chemokine 8, Interleukin-2 receptor alpha chain, Insulin-like growth factor-binding protein 3, Interleukin-11, Matrix Metalloproteinase-8, Transforming growth factor alpha, IgG1, and IgG2 or one or more markers related thereto, are correlated to the renal status of the subject.

For purposes of this document, the following definitions apply:

As used herein, an "injury to renal function" is an abrupt (within 14 days, preferably within 7 days, more preferably within 72 hours, and still more preferably within 48 hours) measurable reduction in a measure of renal function. Such an injury may be identified, for example, by a decrease in glomerular filtration rate or estimated GFR, a reduction in urine output, an increase in serum creatinine, an increase in serum cystatin C, a requirement for renal replacement therapy, *etc.* "Improvement in Renal Function" is an abrupt (within 14 days, preferably within 7 days, more preferably within 72 hours, and still more preferably within 48 hours) measurable increase in a measure of renal function. Preferred methods for measuring and/or estimating GFR are described hereinafter.

As used herein, "reduced renal function" is an abrupt (within 14 days, preferably within 7 days, more preferably within 72 hours, and still more preferably within 48 hours) reduction in kidney function identified by an absolute increase in serum creatinine of greater than or equal to 0.1 mg/dL ($\geq$ 8.8 $\mu$mol/L), a percentage increase in serum creatinine of greater than or equal to 20% (1.2-fold from baseline), or a reduction in urine output (documented oliguria of less than 0.5 ml/kg per hour).

As used herein, "acute renal failure" or "ARF" is an abrupt (within 14 days, preferably within 7 days, more preferably within 72 hours, and still more preferably within 48 hours) reduction in kidney function identified by an absolute increase in serum creatinine of greater than or equal to 0.3 mg/dl ($\geq$ 26.4 $\mu$mol/l), a percentage increase in serum creatinine of greater than or equal to 50% (1. 5-fold from baseline), or a reduction in urine output (documented oliguria of less than 0.5 ml/kg per hour for at least 6 hours). This term is synonymous with "acute kidney injury" or "AKI."

As used herein, the term "C-C motif chemokine 8" refers to one or more polypeptides present in a biological sample that are derived from the C-C motif chemokine 8 precursor (Swiss-Prot P80075 (SEQ ID NO: 1)).

```
          10         20         30         40         50         60
     MKVSAALLCL LLMAATFSPQ GLAQPDSVSI PITCCFNVIN RKIPIQRLES YTRITNIQCP


          70         80         90
     KEAVIFKTKR GKEVCADPKE RWVRDSMKHL DQIFQNLKP
```

The following domains have been identified in C-C motif chemokine 8:

| Residues | Length | Domain ID |
|---|---|---|
| 1-23 | 23 | Signal peptide |
| 24-99 | 76 | C-C motif chemokine 8 |
| 29-99 | 76 | MCP-2(6-76) |

As used herein, the term "Interleukin-2 receptor subunit alpha" refers to one or more polypeptides present in a biological sample that are derived from the Interleukin-2 receptor subunit alpha precursor (Swiss-Prot P01589 (SEQ ID NO: 2)):

```
         10         20         30         40         50         60
MDSYLLMWGL LTFIMVPGCQ AELCDDDPPE IPHATFKAMA YKEGTMLNCE CKRGFRRIKS

         70         80         90        100        110        120
GSLYMLCTGN SSHSSWDNQC QCTSSATRNT TKQVTPQPEE QKERKTTEMQ SPMQPVDQAS

        130        140        150        160        170        180
LPGHCREPPP WENEATERIY HFVVGQMVYY QCVQGYRALH RGPAESVCKM THGKTRWTQP

        190        200        210        220        230        240
QLICTGEMET SQFPGEEKPQ ASPEGRPESE TSCLVTTTDF QIQTEMAATM ETSIFTTEYQ

        250        260        270
VAVAGCVFLL ISVLLLSGLT WQRRQRKSRR TI
```

Interleukin-2 receptor subunit alpha is a single-pass type I membrane protein having a large extracellular domain, some or all of which is present in soluble forms of Interleukin-2 receptor subunit alpha generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). The following domains have been identified in Interleukin-2 receptor subunit alpha:

| Residues | Length | Domain ID |
|---|---|---|
| 1-21 | 21 | Signal peptide |
| 22-272 | 251 | Interleukin-2 receptor subunit alpha |
| 22-240 | 219 | Extracellular domain |
| 241-259 | 19 | Transmembrane domain |
| 260-272 | 13 | Cytoplasmic domain |

As used herein, the term "Insulin-like growth factor-binding protein 3" refers to one or more polypeptides present in a biological sample that are derived from the Insulin-like growth factor-binding protein 3 precursor (Swiss-Prot P17936 (SEQ ID NO: 3)).

```
              10         20         30         40         50         60
        MQRARPTLWA AALTLLVLLR GPPVARAGAS SAGLGPVVRC EPCDARALAQ CAPPPAVCAE

              70         80         90        100        110        120
        LVREPGCGCC LTCALSEGQP CGIYTERCGS GLRCQPSPDE ARPLQALLDG RGLCVNASAV

             130        140        150        160        170        180
        SRLRAYLLPA PPAPGNASES EEDRSAGSVE SPSVSSTHRV SDPKFHPLHS KIIIIKKGHA

             190        200        210        220        230        240
        KDSQRYKVDY ESQSTDTQNF SSESKRETEY GPCRREMEDT LNHLKFLNVL SPRGVHIPNC

             250        260        270        280        290
        DKKGFYKKKQ CRPSKGRKRG FCWCVDKYGQ PLPGYTTKGK EDVHCYSMQS K
```

The following domains have been identified in Insulin-like growth factor-binding protein 3:

| Residues | Length | Domain ID |
|---|---|---|
| 1-27 | 27 | Signal peptide |
| 28-291 | 264 | Insulin-like growth factor-binding protein 3 |

As used herein, the term "Interleukin-11" refers to one or more polypeptides present in a biological sample that are derived from the Interleukin-11 precursor (Swiss-Prot P20809 (SEQ ID NO: 4)).

```
              10         20         30         40         50         60
        MNCVCRLVLV VLSLWPDTAV APGPPPGPPR VSPDPRAELD STVLLTRSLL ADTRQLAAQL

              70         80         90        100        110        120
        RDKFPADGDH NLDSLPTLAM SAGALGALQL PGVLTRLRAD LLSYLRHVQW LRRAGGSSLK

             130        140        150        160        170        180
        TLEPELGTLQ ARLDRLLRRL QLLMSRLALP QPPPDPPAPP LAPPSSAWGG IRAAHAILGG

             190
        LHLTLDWAVR GLLLLKTRL
```

The following domains have been identified in Interleukin-11:

| Residues | Length | Domain ID |
|---|---|---|
| 1-21 | 21 | Signal peptide |
| 22-199 | 178 | Interleukin-11 |

As used herein, the term "Neutrophil collagenase" (also known as MMP-8 and matrix metalloproteinase 8) refers to one or more polypeptides present in a biological sample that are derived from the Neutrophil collagenase precursor (Swiss-Prot P22894 (SEQ ID NO: 5)).

```
            10         20         30         40         50         60
     MFSLKTLPFL LLLHVQISKA FPVSSKEKNT KTVQDYLEKF YQLPSNQYQS TRKNGTNVIV

            70         80         90        100        110        120
     EKLKEMQRFF GLNVTGKPNE ETLDMMKKPR CGVPDSGGFM LTPGNPKWER TNLTYRIRNY

           130        140        150        160        170        180
     TPQLSEAEVE RAIKDAFELW SVASPLIFTR ISQGEADINI AFYQRDHGDN SPFDGPNGIL

           190        200        210        220        230        240
     AHAFQPGQGI GGDAHFDAEE TWTNTSANYN LFLVAAHEFG HSLGLAHSSD PGALMYPNYA

           250        260        270        280        290        300
     FRETSNYSLP QDDIDGIQAI YGLSSNPIQP TGPSTPKPCD PSLTFDAITT LRGEILFFKD

           310        320        330        340        350        360
     RYFWRRHPQL QRVEMNFISL FWPSLPTGIQ AAYEDFDRDL IFLFKGNQYW ALSGYDILQG

           370        380        390        400        410        420
     YPKDISNYGF PSSVQAIDAA VFYRSKTYFF VNDQFWRYDN QRQFMEPGYP KSISGAFPGI

           430        440        450        460
     ESKVDAVFQQ EHFFHVFSGP RYYAFDLIAQ RVTRVARGNK WLNCRYG
```

The following domains have been identified in Neutrophil collagenase:

| Residues | Length | Domain ID |
|---|---|---|
| 1-20 | 20 | Signal peptide |
| 21-100 | 80 | Activation peptide |
| 101-467 | 367 | Neutrophil collagenase |

As used herein, the term "Transforming growth factor alpha" refers to one or more polypeptides present in a biological sample that are derived from the Transforming growth factor alpha precursor (Swiss-Prot P01135 (SEQ ID NO: 6)).

```
            10         20         30         40         50         60
     MVPSAGQLAL FALGIVLAAC QALENSTSPL SADPPVAAAV VSHFNDCPDS HTQFCFHGTC

            70         80         90        100        110        120
     RFLVQEDKPA CVCHSGYVGA RCEHADLLAV VAASQKKQAI TALVVVSIVA LAVLIITCVL

           130        140        150        160
     IHCCQVRKHC EWCRALICRH EKPSALLKGR TACCHSETVV
```

The following domains have been identified in Transforming growth factor alpha:

| Residues | Length | Domain ID |
|---|---|---|
| 1-23 | 23 | Signal peptide |
| 24-160 | 131 | pro-Transforming growth factor alpha |
| 24-39 | 16 | propeptide |
| 40-89 | 50 | Transforming growth factor alpha |
| 90-160 | 71 | propeptide |
| 32 | | Missing in isoform 2 |

(continued)

| Residues | Length | Domain ID |
|---|---|---|
| 32 | | Missing in isoform 3 |
| 159-160 | | VV → ATLG in isoform 3 |
| 159-160 | | VV → GCRLY in isoform 4 |
| 32 | | Missing in isoform 5 |
| 159-160 | | VV → GCRLY in isoform 5 |

As used herein, the term "Cancer Antigen CA 15-3" refers to one or more polypeptides present in a biological sample that are derived from the Mucin-16 precursor (Swiss-Prot Q8WXI7 (SEQ ID NO: 7)):

```
            10         20         30         40         50         60

MLKPSGLPGS SSPTRSLMTG SRSTKATPEM DSGLTGATLS PKTSTGAIVV TEHTLPFTSP

            70         80         90        100        110        120

DKTLASPTSS VVGRTTQSLG VMSSALPEST SRGMTHSEQR TSPSLSPQVN GTPSRNYPAT

           130        140        150        160        170        180

SMVSGLSSPR TRTSSTEGNF TKEASTYTLT VETTSGPVTE KYTVPTETST TEGDSTETPW

           190        200        210        220        230        240

DTRYIPVKIT SPMKTFADST ASKENAPVSM TPAETTVTDS HTPGRTNPSF GTLYSSFLDL

           250        260        270        280        290        300

SPKGTPNSRG ETSLELILST TGYPFSSPEP GSAGHSRIST SAPLSSSASV LDNKISETSI

           310        320        330        340        350        360

FSGQSLTSPL SPGVPEARAS TMPNSAIPFS MTLSNAETSA ERVRSTISSL GTPSISTKQT

           370        380        390        400        410        420

AETILTFHAF AETMDIPSTH IAKTLASEWL GSPGTLGGTS TSALTTTSPS TTLVSEETNT

           430        440        450        460        470        480

HHSTSGKETE GTLNTSMTPL ETSAPGEESE MTATLVPTLG FTTLDSKIRS PSQVSSSHPT

           490        500        510        520        530        540

RELRTTGSTS GRQSSSTAAH GSSDILRATT SSTSKASSWT SESTAQQFSE PQHTQWVETS

           550        560        570        580        590        600
```

```
PSMKTERPPA  STSVAAPITT  SVPSVVSGFT  TLKTSSTKGI  WLEETSADTL  IGESTAGPTT
     610         620         630         640         650         660

HQFAVPTGIS  MTGGSSTRGS  QGTTHLLTRA  TASSETSADL  TLATNGVPVS  VSPAVSKTAA
     670         680         690         700         710         720

GSSPPGGTKP  SYTMVSSVIP  ETSSLQSSAF  REGTSLGLTP  LNTRHPFSSP  EPDSAGHTKI
     730         740         750         760         770         780

STSIPLLSSA  SVLEDKVSAT  STFSHHKATS  SITTGTPEIS  TKTKPSSAVL  SSMTLSNAAT
     790         800         810         820         830         840

SPERVRNATS  PLTHPSPSGE  ETAGSVLTLS  TSAETTDSPN  IHPTGTLTSE  SSESPSTLSL
     850         860         870         880         890         900

PSVSGVKTTF  SSSTPSTHLF  TSGEETEETS  NPSVSQPETS  VSRVRTTLAS  TSVPTPVFPT
     910         920         930         940         950         960

MDTWPTRSAQ  FSSSHLVSEL  RATSSTSVTN  STGSALPKIS  HLTGTATMSQ  TNRDTFNDSA
     970         980         990        1000        1010        1020

APQSTTWPET  SPRFKTGLPS  ATTTVSTSAT  SLSATVMVSK  FTSPATSSME  ATSIREPSTT
    1030        1040        1050        1060        1070        1080

ILTTETTNGP  GSMAVASTNI  PIGKGYITEG  RLDTSHLPIG  TTASSETSMD  FTMAKESVSM
    1090        1100        1110        1120        1130        1140

SVSPSQSMDA  AGSSTPGRTS  QFVDTFSDDV  YHLTSREITI  PRDGTSSALT  PQMTATHPPS
    1150        1160        1170        1180        1190        1200

PDPGSARSTW  LGILSSSPSS  PTPKVTMSST  FSTQRVTTSM  IMDTVETSRW  NMPNLPSTTS
    1210        1220        1230        1240        1250        1260

LTPSNIPTSG  AIGKSTLVPL  DTPSPATSLE  ASEGGLPTLS  TYPESTNTPS  IHLGAHASSE
    1270        1280        1290        1300        1310        1320

SPSTIKLTMA  SVVKPGSYTP  LTFPSIETHI  HVSTARMAYS  SGSSPEMTAP  GETNTGSTWD
    1330        1340        1350        1360        1370        1380

PTTYITTTDP  KDTSSAQVST  PHSVRTLRTT  ENHPKTESAT  PAAYSGSPKI  SSSPNLTSPA
    1390        1400        1410        1420        1430        1440
```

TKAWTITDTT EHSTQLHYTK LAEKSSGFET QSAPGPVSVV IPTSPTIGSS TLELTSDVPG

EPLVLAPSEQ TTITLPMATW LSTSLTEEMA STDLDISSPS SPMSTFAIFP PMSTPSHELS

KSEADTSAIR NTDSTTLDQH LGIRSLGRTG DLTTVPITPL TTTWTSVIEH STQAQDTLSA

TMSPTHVTQS LKDQTSIPAS ASPSHLTEVY PELGTQGRSS SEATTFWKPS TDTLSREIET

GPTNIQSTPP MDNTTTGSSS SGVTLGIAHL PIGTSSPAET STNMALERRS STATVSMAGT

MGLLVTSAPG RSISQSLGRV SSVLSESTTE GVTDSSKGSS PRLNTQGNTA LSSSLEPSYA

EGSQMSTSIP LTSSPTTPDV EFIGGSTFWT KEVTTVMTSD ISKSSARTES SSATLMSTAL

GSTENTGKEK LRTASMDLPS PTPSMEVTPW ISLTLSNAPN TTDSLDLSHG VHTSSAGTLA

TDRSLNTGVT RASRLENGSD TSSKSLSMGN STHTSMTDTE KSEVSSSIHP RPETSAPGAE

TTLTSTPGNR AISLTLPFSS IPVEEVISTG ITSGPDINSA PMTHSPITPP TIVWTSTGTI

EQSTQPLHAV SSEKVSVQTQ STPYVNSVAV SASPTHENSV SSGSSTSSPY SSASLESLDS

TISRRNAITS WLWDLTTSLP TTTWPSTSLS EALSSGHSGV SNPSSTTTEF PLFSAASTSA

AKQRNPETET HGPQNTAAST LNTDASSVTG LSETPVGASI SSEVPLPMAI TSRSDVSGLT

SESTANPSLG TASSAGTKLT RTISLPTSES LVSFRMNKDP WTVSIPLGSH PTTNTETSIP

```
VNSAGPPGLS  TVASDVIDTP  SDGAESIPTV  SFSPSPDTEV  TTISHFPEKT  THSFRTISSL
      2290        2300        2310        2320        2330        2340

THELTSRVTP  IPGDWMSSAM  STKPTGASPS  ITLGERRTIT  SAAPTTSPIV  LTASFTETST
      2350        2360        2370        2380        2390        2400

VSLDNETTVK  TSDILDARKT  NELPSDSSSS  SDLINTSIAS  STMDVTKTAS  ISPTSISGMT
      2410        2420        2430        2440        2450        2460

ASSSPSLFSS  DRPQVPTSTT  ETNTATSPSV  SSNTYSLDGG  SNVGGTPSTL  PPFTITHPVE
      2470        2480        2490        2500        2510        2520

TSSALLAWSR  PVRTFSTMVS  TDTASGENPT  SSNSVVTSVP  APGTWASVGS  TTDLPAMGFL
      2530        2540        2550        2560        2570        2580

KTSPAGEAHS  LLASTIEPAT  AFTPHLSAAV  VTGSSATSEA  SLLTTSESKA  IHSSPQTPTT
      2590        2600        2610        2620        2630        2640

PTSGANWETS  ATPESLLVVT  ETSDTTLTSK  ILVTDTILFS  TVSTPPSKFP  STGTLSGASF
      2650        2660        2670        2680        2690        2700

PTLLPDTPAI  PLTATEPTSS  LATSFDSTPL  VTIASDSLGT  VPETTLTMSE  TSNGDALVLK
      2710        2720        2730        2740        2750        2760

TVSNPDRSIP  GITIQGVTES  PLHPSSTSPS  KIVAPRNTTY  EGSITVALST  LPAGTTGSLV
      2770        2780        2790        2800        2810        2820

FSQSSENSET  TALVDSSAGL  ERASVMPLTT  GSQGMASSGG  IRSGSTHSTG  TKTFSSLPLT
      2830        2840        2850        2860        2870        2880

MNPGEVTAMS  EITTNRLTAT  QSTAPKGIPV  KPTSAESGLL  TPVSASSSPS  KAFASLTTAP
      2890        2900        2910        2920        2930        2940

PSTWGIPQST  LTFEFSEVPS  LDTKSASLPT  PGQSLNTIPD  SDASTASSSL  SKSPEKNPRA
      2950        2960        2970        2980        2990        3000

RMMTSTKAIS  ASSFQSTGFT  ETPEGSASPS  MAGHEPRVPT  SGTGDPRYAS  ESMSYPDPSK
      3010        3020        3030        3040        3050        3060

ASSAMTSTSL  ASKLTTLFST  GQAARSGSSS  SPISLSTEKE  TSFLSPTAST  SRKTSLFLGP
      3070        3080        3090        3100        3110        3120
```

```
SMARQPNILV HLQTSALTLS PTSTLNMSQE EPPELTSSQT IAEEEGTTAE TQTLTFTPSE
           3130       3140       3150       3160       3170       3180

TPTSLLPVSS PTEPTARRKS SPETWASSIS VPAKTSLVET TDGTLVTTIK MSSQAAQGNS
           3190       3200       3210       3220       3230       3240

TWPAPAEETG TSPAGTSPGS PEVSTTLKIM SSKEPSISPE IRSTVRNSPW KTPETTVPME
           3250       3260       3270       3280       3290       3300

TTVEPVTLQS TALGSGSTSI SHLPTGTTSP TKSPTENMLA TERVSLSPSP PEAWTNLYSG
           3310       3320       3330       3340       3350       3360

TPGGTRQSLA TMSSVSLESP TARSITGTGQ QSSPELVSKT TGMEFSMWHG STGGTTGDTH
           3370       3380       3390       3400       3410       3420

VSLSTSSNIL EDPVTSPNSV SSLTDKSKHK TETWVSTTAI PSTVLNNKIM AAEQQTSRSV
           3430       3440       3450       3460       3470       3480

DEAYSSTSSW SDQTSGSDIT LGASPDVTNT LYITSTAQTT SLVSLPSGDQ GITSLTNPSG
           3490       3500       3510       3520       3530       3540

GKTSSASSVT SPSIGLETLR ANVSAVKSDI APTAGHLSQT SSPAEVSILD VTTAPTPGIS
           3550       3560       3570       3580       3590       3600

TTITTMGTNS ISTTTPNPEV GMSTMDSTPA TERRTTSTEH PSTWSSTAAS DSWTVTDMTS
           3610       3620       3630       3640       3650       3660

NLKVARSPGT ISTMHTTSFL ASSTELDSMS TPHGRITVIG TSLVTPSSDA SAVKTETSTS
           3670       3680       3690       3700       3710       3720

ERTLSPSDTT ASTPISTFSR VQRMSISVPD ILSTSWTPSS TEAEDVPVSM VSTDHASTKT
           3730       3740       3750       3760       3770       3780

DPNTPLSTFL FDSLSTLDWD TGRSLSSATA TTSAPQGATT PQELTLETMI SPATSQLPFS
           3790       3800       3810       3820       3830       3840

IGHITSAVTP AAMARSSGVT FSRPDPTSKK AEQTSTQLPT TTSAHPGQVP RSAATTLDVI
           3850       3860       3870       3880       3890       3900

PHTAKTPDAT FQRQGQTALT TEARATSDSW NEKEKSTPSA PWITEMMNSV SEDTIKEVTS
           3910       3920       3930       3940       3950       3960
```

```
SSSVLKDPEY AGHKLGIWDD FIPKFGKAAH MRELPLLSPP QDKEAIHPST NTVETTGWVT

SSEHASHSTI PAHSASSKLT SPVVTTSTRE QAIVSMSTTT WPESTRARTE PNSFLTIELR

DVSPYMDTSS TTQTSIISSP GSTAITKGPR TEITSSKRIS SSFLAQSMRS SDSPSEAITR

LSNFPAMTES GGMILAMQTS PPGATSLSAP TLDTSATASW TGTPLATTQR FTYSEKTTLF

SKGPEDTSQP SPPSVEETSS SSSLVPIHAT TSPSNILLTS QGHSPSSTPP VTSVFLSETS

GLGKTTDMSR ISLEPGTSLP PNLSSTAGEA LSTYEASRDT KAIHHSADTA VTNMEATSSE

YSPIPGHTKP SKATSPLVTS HIMGDITSST SVFGSSETTE IETVSSVNQG LQERSTSQVA

SSATETSTVI THVSSGDATT HVTKTQATFS SGTSISSPHQ FITSTNTFTD VSTNPSTSLI

MTESSGVTIT TQTGPTGAAT QGPYLLDTST MPYLTETPLA VTPDFMQSEK TTLISKGPKD

VTWTSPPSVA ETSYPSSLTP FLVTTIPPAT STLQGQHTSS PVSATSVLTS GLVKTTDMLN

TSMEPVTNSP QNLNNPSNEI LATLAATTDI ETIHPSINKA VTNMGTASSA HVLHSTLPVS

SEPSTATSPM VPASSMGDAL ASISIPGSET TDIEGEPTSS LTAGRKENST LQEMNSTTES

NIILSNVSVG AITEATKMEV PSFDATFIPT PAQSTKFPDI FSVASSRLSN SPPMTISTHM

TTTQTGSSGA TSKIPLALDT STLETSAGTP SVVTEGFAHS KITTAMNNDV KDVSQTNPPF
```

QDEASSPSSQ APVLVTTLPS SVAFTPQWHS TSSPVSMSSV LTSSLVKTAG KVDTSLETVT

SSPQSMSNTL DDISVTSAAT TDIETTHPSI NTVVTNVGTT GSAFESHSTV SAYPEPSKVT

SPNVTTSTME DTTISRSIPK SSKTTRTETE TTSSLTPKLR ETSISQEITS STETSTVPYK

ELTGATTEVS RTDVTSSSST SFPGPDQSTV SLDISTETNT RLSTSPIMTE SAEITITTQT

GPHGATSQDT FTMDPSNTTP QAGIHSAMTH GFSQLDVTTL MSRIPQDVSW TSPPSVDKTS

SPSSFLSSPA MTTPSLISST LPEDKLSSPM TSLLTSGLVK ITDILRTRLE PVTSSLPNFS

STSDKILATS KDSKDTKEIF PSINTEETNV KANNSGHESH SPALADSETP KATTQMVITT

TVGDPAPSTS MPVHGSSETT NIKREPTYFL TPRLRETSTS QESSFPTDTS FLLSKVPTGT

ITEVSSTGVN SSSKISTPDH DKSTVPPDTF TGEIPRVFTS SIKTKSAEMT ITTQASPPES

ASHSTLPLDT STTLSQGGTH STVTQGFPYS EVTTLMGMGP GNVSWMTTPP VEETSSVSSL

MSSPAMTSPS PVSSTSPQSI PSSPLPVTAL PTSVLVTTTD VLGTTSPESV TSSPPNLSSI

THERPATYKD TAHTEAAMHH STNTAVTNVG TSGSGHKSQS SVLADSETSK ATPLMSTTST

LGDTSVSTST PNISQTNQIQ TEPTASLSPR LRESSTSEKT SSTTETNTAF SYVPTGAITQ

ASRTEISSSR TSISDLDRPT IAPDISTGMI TRLFTSPIMT KSAEMTVTTQ TTTPGATSQG

```
ILPWDTSTTL FQGGTHSTVS QGFPHSEITT LRSRTPGDVS WMTTPPVEET SSGFSLMSPS
       5650       5660       5670       5680       5690       5700

MTSPSPVSST SPESIPSSPL PVTALLTSVL VTTTNVLGTT SPETVTSSPP NLSSPTQERL
       5710       5720       5730       5740       5750       5760

TTYKDTAHTE AMHASMHTNT AVANVGTSIS GHESQSSVPA DSHTSKATSP MGITFAMGDT
       5770       5780       5790       5800       5810       5820

SVSTSTPAFF ETRIQTESTS SLIPGLRDTR TSEEINTVTE TSTVLSEVPT TTTTEVSRTE
       5830       5840       5850       5860       5870       5880

VITSSRTTIS GPDHSKMSPY ISTETITRLS TFPFVTGSTE MAITNQTGPI GTISQATLTL
       5890       5900       5910       5920       5930       5940

DTSSTASWEG THSPVTQRFP HSEETTTMSR STKGVSWQSP PSVEETSSPS SPVPLPAITS
       5950       5960       5970       5980       5990       6000

HSSLYSAVSG SSPTSALPVT SLLTSGRRKT IDMLDTHSEL VTSSLPSASS FSGEILTSEA
       6010       6020       6030       6040       6050       6060

STNTETIHFS ENTAETNMGT TNSMHKLHSS VSIHSQPSGH TPPKVTGSMM EDAIVSTSTP
       6070       6080       6090       6100       6110       6120

GSPETKNVDR DSTSPLTPEL KEDSTALVMN STTESNTVFS SVSLDAATEV SRAEVTYYDP
       6130       6140       6150       6160       6170       6180

TFMPASAQST KSPDISPEAS SSHSNSPPLT ISTHKTIATQ TGPSGVTSLG QLTLDTSTIA
       6190       6200       6210       6220       6230       6240

TSAGTPSART QDFVDSETTS VMNNDLNDVL KTSPFSAEEA NSLSSQAPLL VTTSPSPVTS
       6250       6260       6270       6280       6290       6300

TLQEHSTSSL VSVTSVPTPT LAKITDMDTN LEPVTRSPQN LRNTLATSEA TTDTHTMHPS
       6310       6320       6330       6340       6350       6360

INTAMANVGT TSSPNEFYFT VSPDSDPYKA TSAVVITSTS GDSIVSTSMP RSSAMKKIES
       6370       6380       6390       6400       6410       6420

ETTFSLIFRL RETSTSQKIG SSSDTSTVFD KAFTAATTEV SRTELTSSSR TSIQGTEKPT
       6430       6440       6450       6460       6470       6480
```

```
MSPDTSTRSV  TMLSTFAGLT  KSEERTIATQ  TGPHRATSQG  TLTWDTSITT  SQAGTHSAMT
      6490        6500        6510        6520        6530        6540

HGFSQLDLST  LTSRVPEYIS  GTSPPSVEKT  SSSSSLLSLP  AITSPSPVPT  TLPESRPSSP
      6550        6560        6570        6580        6590        6600

VHLTSLPTSG  LVKTTDMLAS  VASLPPNLGS  TSHKIPTTSE  DIKDTEKMYP  STNIAVTNVG
      6610        6620        6630        6640        6650        6660

TTTSEKESYS  SVPAYSEPPK  VTSPMVTSFN  IRDTIVSTSM  PGSSEITRIE  MESTFSVAHG
      6670        6680        6690        6700        6710        6720

LKGTSTSQDP  IVSTEKSAVL  HKLTTGATET  SRTEVASSRR  TSIPGPDHST  ESPDISTEVI
      6730        6740        6750        6760        6770        6780

PSLPISLGIT  ESSNMTIITR  TGPPLGSTSQ  GTFTLDTPTT  SSRAGTHSMA  TQEFPHSEMT
      6790        6800        6810        6820        6830        6840

TVMNKDPEIL  SWTIPPSIEK  TSFSSSLMPS  PAMTSPPVSS  TLPKTIHTTP  SPMTSLLTPS
      6850        6860        6870        6880        6890        6900

LVMTTDTLGT  SPEPTTSSPP  NLSSTSHVIL  TTDEDTTAIE  AMHPSTSTAA  TNVETTCSGH
      6910        6920        6930        6940        6950        6960

GSQSSVLTDS  EKTKATAPMD  TTSTMGHTTV  STSMSVSSET  TKIKRESTYS  LTPGLRETSI
      6970        6980        6990        7000        7010        7020

SQNASFSTDT  SIVLSEVPTG  TTAEVSRTEV  TSSGRTSIPG  PSQSTVLPEI  STRTMTRLFA
      7030        7040        7050        7060        7070        7080

SPTMTESAEM  TIPTQTGPSG  STSQDTLTLD  TSTTKSQAKT  HSTLTQRFPH  SEMTTLMSRG
      7090        7100        7110        7120        7130        7140

PGDMSWQSSP  SLENPSSLPS  LLSLPATTSP  PPISSTLPVT  ISSSPLPVTS  LLTSSPVTTT
      7150        7160        7170        7180        7190        7200

DMLHTSPELV  TSSPPKLSHT  SDERLTTGKD  TTNTEAVHPS  TNTAASNVEI  PSFGHESPSS
      7210        7220        7230        7240        7250        7260

ALADSETSKA  TSPMFITSTQ  EDTTVAISTP  HFLETSRIQK  ESISSLSPKL  RETGSSVETS
      7270        7280        7290        7300        7310        7320
```

```
SAIETSAVLS  EVSIGATTEI  SRTEVTSSSR  TSISGSAEST  MLPEISTTRK  IIKFPTSPIL
       7330        7340        7350        7360        7370        7380

AESSEMTIKT  QTSPPGSTSE  STFTLDTSTT  PSLVITHSTM  TQRLPHSEIT  TLVSRGAGDV
       7390        7400        7410        7420        7430        7440

PRPSSLPVEE  TSPPSSQLSL  SAMISPSPVS  STLPASSHSS  SASVTSPLTP  GQVKTTEVLD
       7450        7460        7470        7480        7490        7500

ASAEPETSSP  PSLSSTSVEI  LATSEVTTDT  EKIHPFPNTA  VTKVGTSSSG  HESPSSVLPD
       7510        7520        7530        7540        7550        7560

SETTKATSAM  GTISIMGDTS  VSTLTPALSN  TRKIQSEPAS  SLTTRLRETS  TSEETSLATE
       7570        7580        7590        7600        7610        7620

ANTVLSKVST  GATTEVSRTE  AISFSRTSMS  GPEQSTMSQD  ISIGTIPRIS  ASSVLTESAK
       7630        7640        7650        7660        7670        7680

MTITTQTGPS  ESTLESTLNL  NTATTPSWVE  THSIVIQGFP  HPEMTTSMGR  GPGGVSWPSP
       7690        7700        7710        7720        7730        7740

PFVKETSPPS  SPLSLPAVTS  PHPVSTTFLA  HIPPSPLPVT  SLLTSGPATT  TDILGTSTEP
       7750        7760        7770        7780        7790        7800

GTSSSSSLST  TSHERLTTYK  DTAHTEAVHP  STNTGGTNVA  TTSSGYKSQS  SVLADSSPMC
       7810        7820        7830        7840        7850        7860

TTSTMGDTSV  LTSTPAFLET  RRIQTELASS  LTPGLRESSG  SEGTSSGTKM  STVLSKVPTG
       7870        7880        7890        7900        7910        7920

ATTEISKEDV  TSIPGPAQST  ISPDISTRTV  SWFSTSPVMT  ESAEITMNTH  TSPLGATTQG
       7930        7940        7950        7960        7970        7980

TSTLATSSTT  SLTMTHSTIS  QGFSHSQMST  LMRRGPEDVS  WMSPPLLEKT  RPSFSLMSSP
       7990        8000        8010        8020        8030        8040

ATTSPSPVSS  TLPESISSSP  LPVTSLLTSG  LAKTTDMLHK  SSEPVTNSPA  NLSSTSVEIL
       8050        8060        8070        8080        8090        8100

ATSEVTTDTE  KTHPSSNRTV  TDVGTSSSGH  ESTSFVLADS  QTSKVTSPMV  ITSTMEDTSV
       8110        8120        8130        8140        8150        8160
```

STSTPGFFET SRIQTEPTSS LTLGLRKTSS SEGTSLATEM STVLSGVPTG ATAEVSRTEV

TSSSRTSISG FAQLTVSPET STETITRLPT SSIMTESAEM MIKTQTDPPG STPESTHTVD

ISTTPNWVET HSTVTQRFSH SEMTTLVSRS PGDMLWPSQS SVEETSSASS LLSLPATTSP

SPVSSTLVED FPSASLPVTS LLTPGLVITT DRMGISREPG TSSTSNLSST SHERLTTLED

TVDTEDMQPS THTAVTNVRT SISGHESQSS VLSDSETPKA TSPMGTTYTM GETSVSISTS

DFFETSRIQI EPTSSLTSGL RETSSSERIS SATEGSTVLS EVPSGATTEV SRTEVISSRG

TSMSGPDQFT ISPDISTEAI TRLSTSPIMT ESAESAITIE TGSPGATSEG TLTLDTSTTT

FWSGTHSTAS PGFSHSEMTT LMSRTPGDVP WPSLPSVEEA SSVSSSLSSP AMTSTSFFSA

LPESISSSPH PVTALLTLGP VKTTDMLRTS SEPETSSPPN LSSTSAEILA TSEVTKDREK

IHPSSNTPVV NVGTVIYKHL SPSSVLADLV TTKPTSPMAT TSTLGNTSVS TSTPAFPETM

MTQPTSSLTS GLREISTSQE TSSATERSAS LSGMPTGATT KVSRTEALSL GRTSTPGPAQ

STISPEISTE TITRISTPLT TTGSAEMTIT PKTGHSGASS QGTFTLDTSS RASWPGTHSA

ATHRSPHSGM TTPMSRGPED VSWPSRPSVE KTSPPSSLVS LSAVTSPSPL YSTPSESSHS

SPLRVTSLFT PVMMKTTDML DTSLEPVTTS PPSMNITSDE SLATSKATME TEAIQLSENT

```
AVTQMGTISA RQEFYSSYPG LPEPSKVTSP VVTSSTIKDI VSTTIPASSE ITRIEMESTS
        9010       9020       9030       9040       9050       9060

TLTPTPRETS TSQEIHSATK PSTVPYKALT SATIEDSMTQ VMSSSRGPSP DQSTMSQDIS
        9070       9080       9090       9100       9110       9120

SEVITRLSTS PIKAESTEMT ITTQTGSPGA TSRGTLTLDT STTFMSGTHS TASQGFSHSQ
        9130       9140       9150       9160       9170       9180

MTALMSRTPG DVPWLSHPSV EEASSASFSL SSPVMTSSSP VSSTLPDSIH SSSLPVTSLL
        9190       9200       9210       9220       9230       9240

TSGLVKTTEL LGTSSEPETS SPPNLSSTSA EILATTEVTT DTEKLEMTNV VTSGYTHESP
        9250       9260       9270       9280       9290       9300

SSVLADSVTT KATSSMGITY PTGDTNVLTS TPAFSDTSRI QTKSKLSLTP GLMETSISEE
        9310       9320       9330       9340       9350       9360

TSSATEKSTV LSSVPTGATT EVSRTEAISS SRTSIPGPAQ STMSSDTSME TITRISTPLT
        9370       9380       9390       9400       9410       9420

RKESTDMAIT PKTGPSGATS QGTFTLDSSS TASWPGTHSA TTQRFPQSVV TTPMSRGPED
        9430       9440       9450       9460       9470       9480

VSWPSPLSVE KNSPPSSLVS SSSVTSPSPL YSTPSGSSHS SPVPVTSLFT SIMMKATDML
        9490       9500       9510       9520       9530       9540

DASLEPETTS APNMNITSDE SLATSKATTE TEAIHVFENT AASHVETTSA TEELYSSSPG
        9550       9560       9570       9580       9590       9600

FSEPTKVISP VVTSSSIRDN MVSTTMPGSS GITRIEIESM SSLTPGLRET RTSQDITSST
        9610       9620       9630       9640       9650       9660

ETSTVLYKMS SGATPEVSRT EVMPSSRTSI PGPAQSTMSL DISDEVVTRL STSPIMTESA
        9670       9680       9690       9700       9710       9720

EITITTQTGY SLATSQVTLP LGTSMTFLSG THSTMSQGLS HSEMTNLMSR GPESLSWTSP
        9730       9740       9750       9760       9770       9780

RFVETTRSSS SLTSLPLTTS LSPVSSTLLD SSPSSPLPVT SLILPGLVKT TEVLDTSSEP
        9790       9800       9810       9820       9830       9840
```

```
KTSSSPNLSS TSVEIPATSE IMTDTEKIHP SSNTAVAKVR TSSSVHESHS SVLADSETTI
      9850       9860       9870       9880       9890       9900

TIPSMGITSA VDDTTVFTSN PAFSETRRIP TEPTFSLTPG FRETSTSEET TSITETSAVL
      9910       9920       9930       9940       9950       9960

YGVPTSATTE VSMTEIMSSN RTHIPDSDQS TMSPDIITEV ITRLSSSSMM SESTQMTITT
      9970       9980       9990      10000      10010      10020

QKSSPGATAQ STLTLATTTA PLARTHSTVP PRFLHSEMTT LMSRSPENPS WKSSPFVEKT
     10030      10040      10050      10060      10070      10080

SSSSSLLSLP VTTSPSVSST LPQSIPSSSF SVTSLLTPGM VKTTDTSTEP GTSLSPNLSG
     10090      10100      10110      10120      10130      10140

TSVEILAASE VTTDTEKIHP SSSMAVTNVG TTSSGHELYS SVSIHSEPSK ATYPVGTPSS
     10150      10160      10170      10180      10190      10200

MAETSISTSM PANFETTGFE AEPFSHLTSG FRKTNMSLDT SSVTPTNTPS SPGSTHLLQS
     10210      10220      10230      10240      10250      10260

SKTDFTSSAK TSSPDWPPAS QYTEIPVDII TPFNASPSIT ESTGITSFPE SRFTMSVTES
     10270      10280      10290      10300      10310      10320

THHLSTDLLP SAETISTGTV MPSLSEAMTS FATTGVPRAI SGSGSPFSRT ESGPGDATLS
     10330      10340      10350      10360      10370      10380

TIAESLPSST PVPFSSSTFT TTDSSTIPAL HEITSSSATP YRVDTSLGTE SSTTEGRLVM
     10390      10400      10410      10420      10430      10440

VSTLDTSSQP GRTSSTPILD TRMTESVELG TVTSAYQVPS LSTRLTRTDG IMEHITKIPN
     10450      10460      10470      10480      10490      10500

EAAHRGTIRP VKGPQTSTSP ASPKGLHTGG TKRMETTTTA LKTTTTALKT TSRATLTTSV
     10510      10520      10530      10540      10550      10560

YTPTLGTLTP LNASRQMAST ILTEMMITTP YVFPDVPETT SSLATSLGAE TSTALPRTTP
     10570      10580      10590      10600      10610      10620

SVLNRESETT ASLVSRSGAE RSPVIQTLDV SSSEPDTTAS WVIHPAETIP TVSKTTPNFF
     10630      10640      10650      10660      10670      10680
```

```
HSELDTVSST   ATSHGADVSS   AIPTNISPSE   LDALTPLVTI   SGTDTSTTFP   TLTKSPHETE
          10690        10700        10710        10720        10730        10740

TRTTWLTHPA   ETSSTIPRTI   PNFSHHESDA   TPSIATSPGA   ETSSAIPIMT   VSPGAEDLVT
          10750        10760        10770        10780        10790        10800

SQVTSSGTDR   NMTIPTLTLS   PGEPKTIASL   VTHPEAQTSS   AIPTSTISPA   VSRLVTSMVT
          10810        10820        10830        10840        10850        10860

SLAAKTSTTN   RALTNSPGEP   ATTVSLVTHP   AQTSPTVPWT   TSIFFHSKSD   TTPSMTTSHG
          10870        10880        10890        10900        10910        10920

AESSSAVPTP   TVSTEVPGVV   TPLVTSSRAV   ISTTIPILTL   SPGEPETTPS   MATSHGEEAS
          10930        10940        10950        10960        10970        10980

SAIPTPTVSP   GVPGVVTSLV   TSSRAVTSTT   IPILTFSLGE   PETTPSMATS   HGTEAGSAVP
          10990        11000        11010        11020        11030        11040

TVLPEVPGMV   TSLVASSRAV   TSTTLPTLTL   SPGEPETTPS   MATSHGAEAS   STVPTVSPEV
          11050        11060        11070        11080        11090        11100

PGVVTSLVTS   SSGVNSTSIP   TLILSPGELE   TTPSMATSHG   AEASSAVPTP   TVSPGVSGVV
          11110        11120        11130        11140        11150        11160

TPLVTSSRAV   TSTTIPILTL   SSSEPETTPS   MATSHGVEAS   SAVLTVSPEV   PGMVTSLVTS
          11170        11180        11190        11200        11210        11220

SRAVTSTTIP   TLTISSDEPE   TTTSLVTHSE   AKMISAIPTL   AVSPTVQGLV   TSLVTSSGSE
          11230        11240        11250        11260        11270        11280

TSAFSNLTVA   SSQPETIDSW   VAHPGTEASS   VVPTLTVSTG   EPFTNISLVT   HPAESSSTLP
          11290        11300        11310        11320        11330        11340

RTTSRFSHSE   LDTMPSTVTS   PEAESSSAIS   TTISPGIPGV   LTSLVTSSGR   DISATFPTVP
          11350        11360        11370        11380        11390        11400

ESPHESEATA   SWVTHPAVTS   TTVPRTTPNY   SHSEPDTTPS   IATSPGAEAT   SDFPTITVSP
          11410        11420        11430        11440        11450        11460

DVPDMVTSQV   TSSGTDTSIT   IPTLTLSSGE   PETTTSFITY   SETHTSSAIP   TLPVSPGASK
          11470        11480        11490        11500        11510        11520
```

MLTSLVISSG TDSTTTFPTL TETPYEPETT AIQLIHPAET NTMVPKTTPK FSHSKSDTTL

PVAITSPGPE ASSAVSTTTI SPDMSDLVTS LVPSSGTDTS TTFPTLSETP YEPETTVTWL

THPAETSTTV SGTIPNFSHR GSDTAPSMVT SPGVDTRSGV PTTTIPPSIP GVVTSQVTSS

ATDTSTAIPT LTPSPGEPET TASSATHPGT QTGFTVPIRT VPSSEPDTMA SWVTHPPQTS

TPVSRTTSSF SHSSPDATPV MATSPRTEAS SAVLTTISPG APEMVTSQIT SSGAATSTTV

PTLTHSPGMP ETTALLSTHP RTGTSKTFPA STVFPQVSET TASLTIRPGA ETSTALPTQT

TSSLFTLLVT GTSRVDLSPT ASPGVSAKTA PLSTHPGTET STMIPTSTLS LGLLETTGLL

ATSSSAETST STLTLTVSPA VSGLSSASIT TDKPQTVTSW NTETSPSVTS VGPPEFSRTV

TGTTMTLIPS EMPTPPKTSH GEGVSPTTIL RTTMVEATNL ATTGSSPTVA KTTTTFNTLA

GSLFTPLTTP GMSTLASESV TSRTSYNHRS WISTTSSYNR RYWTPATSTP VTSTFSPGIS

TSSIPSSTAA TVPFMVPFTL NFTITNLQYE EDMRHPGSRK FNATERELQG LLKPLFRNSS

LEYLYSGCRL ASLRPEKDSS AMAVDAICTH RPDPEDLGLD RERLYWELSN LTNGIQELGP

YTLDRNSLYV NGFTHRSSMP TTSTPGTSTV DVGTSGTPSS SPSPTAAGPL LMPFTLNFTI

TNLQYEEDMR RTGSRKFNTM ESVLQGLLKP LFKNTSVGPL YSGCRLTLLR PEKDGAATGV

DAICTHRLDP KSPGLNREQL YWELSKLTND IEELGPYTLD RNSLYVNGFT HQSSVSTTST
12370      12380      12390      12400      12410      12420

PGTSTVDLRT SGTPSSLSSP TIMAAGPLLV PFTLNFTITN LQYGEDMGHP GSRKFNTTER
12430      12440      12450      12460      12470      12480

VLQGLLGPIF KNTSVGPLYS GCRLTSLRSE KDGAATGVDA ICIHHLDPKS PGLNRERLYW
12490      12500      12510      12520      12530      12540

ELSQLTNGIK ELGPYTLDRN SLYVNGFTHR TSVPTTSTPG TSTVDLGTSG TPFSLPSPAT
12550      12560      12570      12580      12590      12600

AGPLLVLFTL NFTITNLKYE EDMHRPGSRK FNTTERVLQT LLGPMFKNTS VGLLYSGCRL
12610      12620      12630      12640      12650      12660

TLLRSEKDGA ATGVDAICTH RLDPKSPGLD REQLYWELSQ LTNGIKELGP YTLDRNSLYV
12670      12680      12690      12700      12710      12720

NGFTHWIPVP TSSTPGTSTV DLGSGTPSSL PSPTAAGPLL VPFTLNFTIT NLQYEEDMHH
12730      12740      12750      12760      12770      12780

PGSRKFNTTE RVLQGLLGPM FKNTSVGLLY SGCRLTLLRS EKDGAATGVD AICTHRLDPK
12790      12800      12810      12820      12830      12840

SPGVDREQLY WELSQLTNGI KELGPYTLDR NSLYVNGFTH QTSAPNTSTP GTSTVDLGTS
12850      12860      12870      12880      12890      12900

GTPSSLPSPT SAGPLLVPFT LNFTITNLQY EEDMRHPGSR KFNTTERVLQ GLLKPLFKST
12910      12920      12930      12940      12950      12960

SVGPLYSGCR LTLLRSEKDG AATGVDAICT HRLDPKSPGV DREQLYWELS QLTNGIKELG
12970      12980      12990      13000      13010      13020

PYTLDRNSLY VNGFTHQTSA PNTSTPGTST VDLGTSGTPS SLPSPTSAGP LLVPFTLNFT
13030      13040      13050      13060      13070      13080

ITNLQYEEDM HHPGSRKFNT TERVLQGLLG PMFKNTSVGL LYSGCRLTLL RPEKNGAATG
13090      13100      13110      13120      13130      13140

MDAICSHRLD PKSPGLNREQ LYWELSQLTH GIKELGPYTL DRNSLYVNGF THRSSVAPTS
13150      13160      13170      13180      13190      13200

```
TPGTSTVDLG  TSGTPSSLPS  PTTAVPLLVP  FTLNFTITNL  QYGEDMRHPG  SRKFNTTERV
        13210       13220       13230       13240       13250       13260
LQGLLGPLFK  NSSVGPLYSG  CRLISLRSEK  DGAATGVDAI  CTHHLNPQSP  GLDREQLYWQ
        13270       13280       13290       13300       13310       13320
LSQMTNGIKE  LGPYTLDRNS  LYVNGFTHRS  SGLTTSTPWT  STVDLGTSGT  PSPVPSPTTA
        13330       13340       13350       13360       13370       13380
GPLLVPFTLN  FTITNLQYEE  DMHRPGSRKF  NTTERVLQGL  LSPIFKNSSV  GPLYSGCRLT
        13390       13400       13410       13420       13430       13440
SLRPEKDGAA  TGMDAVCLYH  PNPKRPGLDR  EQLYWELSQL  THNITELGPY  SLDRDSLYVN
        13450       13460       13470       13480       13490       13500
GFTHQNSVPT  TSTPGTSTVY  WATTGTPSSF  PGHTEPGPLL  IPFTFNFTIT  NLHYEENMQH
        13510       13520       13530       13540       13550       13560
PGSRKFNTTE  RVLQGLLKPL  FKNTSVGPLY  SGCRLTSLRP  EKDGAATGMD  AVCLYHPNPK
        13570       13580       13590       13600       13610       13620
RPGLDREQLY  WELSQLTHNI  TELGPYSLDR  DSLYVNGFTH  QNSVPTTSTP  GTSTVYWATT
        13630       13640       13650       13660       13670       13680
GTPSSFPGHT  EPGPLLIPFT  FNFTITNLHY  EENMQHPGSR  KFNTTERVLQ  GLLKPLFKNT
        13690       13700       13710       13720       13730       13740
SVGPLYSGCR  LTLLRPEKHE  AATGVDTICT  HRVDPIGPGL  DRERLYWELS  QLTNSITELG
        13750       13760       13770       13780       13790       13800
PYTLDRDSLY  VNGFNPRSSV  PTTSTPGTST  VHLATSGTPS  SLPGHTAPVP  LLIPFTLNFT
        13810       13820       13830       13840       13850       13860
ITNLHYEENM  QHPGSRKFNT  TERVLQGLLK  PLFKNTSVGP  LYSGCRLTLL  RPEKHEAATG
        13870       13880       13890       13900       13910       13920
VDTICTHRVD  PIGPGLXXEX  LYWELSXLTX  XIXELGPYTL  DRXSLYVNGF  THXXSXPTTS
        13930       13940       13950       13960       13970       13980
TPGTSTVXXG  TSGTPSSXPX  XTSAGPLLVP  FTLNFTITNL  QYEEDMHHPG  SRKFNTTERV
        13990       14000       14010       14020       14030       14040
```

LQGLLGPMFK NTSVGLLYSG CRLTLLRPEK NGAATGMDAI CSHRLDPKSP GLDREQLYWE

LSQLTHGIKE LGPYTLDRNS LYVNGFTHRS SVAPTSTPGT STVDLGTSGT PSSLPSPTTA

VPLLVPFTLN FTITNLQYGE DMRHPGSRKF NTTERVLQGL LGPLFKNSSV GPLYSGCRLI

SLRSEKDGAA TGVDAICTHH LNPQSPGLDR EQLYWQLSQM TNGIKELGPY TLDRNSLYVN

GFTHRSSGLT TSTPWTSTVD LGTSGTPSPV PSPTTAGPLL VPFTLNFTIT NLQYEEDMHR

PGSRKFNATE RVLQGLLSPI FKNSSVGPLY SGCRLTSLRP EKDGAATGMD AVCLYHPNPK

RPGLDREQLY WELSQLTHNI TELGPYSLDR DSLYVNGFTH QSSMTTTRTP DTSTMHLATS

RTPASLSGPT TASPLLVLFT INCTITNLQY EEDMRRTGSR KFNTMESVLQ GLLKPLFKNT

SVGPLYSGCR LTLLRPKKDG AATGVDAICT HRLDPKSPGL NREQLYWELS KLTNDIEELG

PYTLDRNSLY VNGFTHQSSV STTSTPGTST VDLRTSGTPS SLSSPTIMXX XPLLXPFTXN

XTITNLXXXX XMXXPGSRKF NTTERVLQGL LRPLFKNTSV SSLYSGCRLT LLRPEKDGAA

TRVDAACTYR PDPKSPGLDR EQLYWELSQL THSITELGPY TLDRVSLYVN GFNPRSSVPT

TSTPGTSTVH LATSGTPSSL PGHTXXXPLL XPFTXNXTIT NLXXXXXMXX PGSRKFNTTE

RVLQGLLKPL FRNSSLEYLY SGCRLASLRP EKDSSAMAVD AICTHRPDPE DLGLDRERLY

```
WELSNLTNGI  QELGPYTLDR  NSLYVNGFTH  RSSGLTTSTP  WTSTVDLGTS  GTPSPVPSPT
            14890       14900       14910       14920       14930       14940

TAGPLLVPFT  LNFTITNLQY  EEDMHRPGSR  RFNTTERVLQ  GLLTPLFKNT  SVGPLYSGCR
            14950       14960       14970       14980       14990       15000

LTLLRPEKQE  AATGVDTICT  HRVDPIGPGL  DRERLYWELS  QLTNSITELG  PYTLDRDSLY
            15010       15020       15030       15040       15050       15060

VNGFNPWSSV  PTTSTPGTST  VHLATSGTPS  SLPGHTAPVP  LLIPFTLNFT  ITDLHYEENM
            15070       15080       15090       15100       15110       15120

QHPGSRKFNT  TERVLQGLLK  PLFKSTSVGP  LYSGCRLTLL  RPEKHGAATG  VDAICTLRLD
            15130       15140       15150       15160       15170       15180

PTGPGLDRER  LYWELSQLTN  SVTELGPYTL  DRDSLYVNGF  THRSSVPTTS  IPGTSAVHLE
            15190       15200       15210       15220       15230       15240

TSGTPASLPG  HTAPGPLLVP  FTLNFTITNL  QYEEDMRHPG  SRKFSTTERV  LQGLLKPLFK
            15250       15260       15270       15280       15290       15300

NTSVSSLYSG  CRLTLLRPEK  DGAATRVDAV  CTHRPDPKSP  GLDRERLYWK  LSQLTHGITE
            15310       15320       15330       15340       15350       15360

LGPYTLDRHS  LYVNGFTHQS  SMTTTRTPDT  STMHLATSRT  PASLSGPTTA  SPLLVLFTIN
            15370       15380       15390       15400       15410       15420

FTITNLRYEE  NMHHPGSRKF  NTTERVLQGL  LRPVFKNTSV  GPLYSGCRLT  TLRPKKDGAA
            15430       15440       15450       15460       15470       15480

TKVDAICTYR  PDPKSPGLDR  EQLYWELSQL  THSITELGPY  TQDRDSLYVN  GFTHRSSVPT
            15490       15500       15510       15520       15530       15540

TSIPGTSAVH  LETSGTPASL  PGHTAPGPLL  VPFTLNFTIT  NLQYEEDMRH  PGSRKFNTTE
            15550       15560       15570       15580       15590       15600

RVLQGLLKPL  FKSTSVGPLY  SGCRLTLLRP  EKRGAATGVD  TICTHRLDPL  NPGLDREQLY
            15610       15620       15630       15640       15650       15660

WELSKLTRGI  IELGPYLLDR  GSLYVNGFTH  RTSVPTTSTP  GTSTVDLGTS  GTPFSLPSPA
            15670       15680       15690       15700       15710       15720
```

```
XXXPLLXPFT  XNXTITNLXX  XXXMXXPGSR  KFNTTERVLQ  TLLGPMFKNT  SVGLLYSGCR

LTLLRSEKDG  AATGVDAICT  HRLDPKSPGV  DREQLYWELS  QLTNGIKELG  PYTLDRNSLY

VNGFTHWIPV  PTSSTPGTST  VDLGSGTPSS  LPSPTTAGPL  LVPFTLNFTI  TNLKYEEDMH

CPGSRKFNTT  ERVLQSLLGP  MFKNTSVGPL  YSGCRLTLLR  SEKDGAATGV  DAICTHRLDP

KSPGVDREQL  YWELSQLTNG  IKELGPYTLD  RNSLYVNGFT  HQTSAPNTST  PGTSTVDLGT

SGTPSSLPSP  TXXXPLLXPF  TXNXTITNLX  XXXXMXXPGS  RKFNTTEXVL  QGLLXPXFKN

XSVGXLYSGC  RLTXLRXEKX  GAATGXDAIC  XHXXXPKXPG  LXXEXLYWEL  SXLTXXIXEL

GPYTLDRXSL  YVNGFTHWIP  VPTSSTPGTS  TVDLGSGTPS  SLPSPTTAGP  LLVPFTLNFT

ITNLKYEEDM  HCPGSRKFNT  TERVLQSLLG  PMFKNTSVGP  LYSGCRLTSL  RSEKDGAATG

VDAICTHRVD  PKSPGVDREQ  LYWELSQLTN  GIKELGPYTL  DRNSLYVNGF  THQTSAPNTS

TPGTSTVXXG  TSGTPSSXPX  XTSAGPLLVP  FTLNFTITNL  QYEEDMHHPG  SRKFNTTERV

LQGLLGPMFK  NTSVGLLYSG  CRLTLLRPEK  NGATTGMDAI  CTHRLDPKSP  GLXXEXLYWE

LSXLTXXIXE  LGPYTLDRXS  LYVNGFTHXX  SXPTTSTPGT  STVXXGTSGT  PSSXPXXTXX

XPLLXPFTXN  XTITNLXXXX  XMXXPGSRKF  NTTERVLQGL  LKPLFRNSSL  EYLYSGCRLA
```

```
SLRPEKDSSA MAVDAICTHR PDPEDLGLDR ERLYWELSNL TNGIQELGPY TLDRNSLYVN

GFTHRSSMPT TSTPGTSTVD VGTSGTPSSS PSPTTAGPLL IPFTLNFTIT NLQYGEDMGH

PGSRKFNTTE RVLQGLLGPI FKNTSVGPLY SGCRLTSLRS EKDGAATGVD AICIHHLDPK

SPGLNRERLY WELSQLTNGI KELGPYTLDR NSLYVNGFTH RTSVPTTSTP GTSTVDLGTS

GTPFSLPSPA TAGPLLVLFT LNFTITNLKY EEDMHRPGSR KFNTTERVLQ TLLGPMFKNT

SVGLLYSGCR LTLLRSEKDG AATGVDAICT HRLDPKSPGL XXEXLYWELS XLTXXIXELG

PYTLDRXSLY VNGFTHXXSX PTTSTPGTST VXXGTSGTPS SXPXXTXXXP LLXPFTXNXT

ITNLXXXXXM XXPGSRKFNT TERVLQGLLR PVFKNTSVGP LYSGCRLTLL RPKKDGAATK

VDAICTYRPD PKSPGLDREQ LYWELSQLTH SITELGPYTQ DRDSLYVNGF THRSSVPTTS

IPGTSAVHLE TTGTPSSFPG HTEPGPLLIP FTFNFTITNL RYEENMQHPG SRKFNTTERV

LQGLLTPLFK NTSVGPLYSG CRLTLLRPEK QEAATGVDTI CTHRVDPIGP GLDRERLYWE

LSQLTNSITE LGPYTLDRDS LYVDGFNPWS SVPTTSTPGT STVHLATSGT PSPLPGHTAP

VPLLIPFTLN FTITDLHYEE NMQHPGSRKF NTTERVLQGL LKPLFKSTSV GPLYSGCRLT

LLRPEKHGAA TGVDAICTLR LDPTGPGLDR ERLYWELSQL TNSITELGPY TLDRDSLYVN
```

```
GFNPWSSVPT  TSTPGTSTVH  LATSGTPSSL  PGHTTAGPLL  VPFTLNFTIT  NLKYEEDMHC
        17410       17420       17430       17440       17450       17460

PGSRKFNTTE  RVLQSLHGPM  FKNTSVGPLY  SGCRLTLLRS  EKDGAATGVD  AICTHRLDPK
        17470       17480       17490       17500       17510       17520

SPGLXXEXLY  WELSXLTXXI  XELGPYTLDR  XSLYVNGFTH  XXSXPTTSTP  GTSTVXXGTS
        17530       17540       17550       17560       17570       17580

GTPSSXPXXT  XXXPLLXPFT  XNXTITNLXX  XXXMXXPGSR  KFNTTEXVLQ  GLLXPXFKNX
        17590       17600       17610       17620       17630       17640

SVGXLYSGCR  LTXLRXEKXG  AATGXDAICX  HXXXPKXPGL  XXEXLYWELS  XLTNSITELG
        17650       17660       17670       17680       17690       17700

PYTLDRDSLY  VNGFTHRSSM  PTTSIPGTSA  VHLETSGTPA  SLPGHTAPGP  LLVPFTLNFT
        17710       17720       17730       17740       17750       17760

ITNLQYEEDM  RHPGSRKFNT  TERVLQGLLK  PLFKSTSVGP  LYSGCRLTLL  RPEKRGAATG
        17770       17780       17790       17800       17810       17820

VDTICTHRLD  PLNPGLXXEX  LYWELSXLTX  XIXELGPYTL  DRXSLYVNGF  THXXSXPTTS
        17830       17840       17850       17860       17870       17880

TPGTSTVXXG  TSGTPSSXPX  XTXXXPLLXP  FTXNXTITNL  XXXXXMXXPG  SRKFNTTEXV
        17890       17900       17910       17920       17930       17940

LQGLLXPXFK  NXSVGXLYSG  CRLTXLRXEK  XGAATGXDAI  CXHXXXPKXP  GLXXEXLYWE
        17950       17960       17970       17980       17990       18000

LSXLTXXIXE  LGPYTLDRXS  LYVNGFHPRS  SVPTTSTPGT  STVHLATSGT  PSSLPGHTAP
        18010       18020       18030       18040       18050       18060

VPLLIPFTLN  FTITNLHYEE  NMQHPGSRKF  NTTERVLQGL  LGPMFKNTSV  GLLYSGCRLT
        18070       18080       18090       18100       18110       18120

LLRPEKNGAA  TGMDAICSHR  LDPKSPGLXX  EXLYWELSXL  TXXIXELGPY  TLDRXSLYVN
        18130       18140       18150       18160       18170       18180

GFTHXXSXPT  TSTPGTSTVX  XGTSGTPSSX  PXXTXXXPLL  XPFTXNXTIT  NLXXXXXMXX
        18190       18200       18210       18220       18230       18240
```

```
PGSRKFNTTE XVLQGLLXPX FKNXSVGXLY SGCRLTXLRX EKXGAATGXD AICXHXXXPK

XPGLXXEXLY WELSXLTXXI XELGPYTLDR XSLYVNGFTH QNSVPTTSTP GTSTVYWATT

GTPSSFPGHT EPGPLLIPFT FNFTITNLHY EENMQHPGSR KFNTTERVLQ GLLTPLFKNT

SVGPLYSGCR LTLLRPEKQE AATGVDTICT HRVDPIGPGL XXEXLYWELS XLTXXIXELG

PYTLDRXSLY VNGFTHXXSX PTTSTPGTST VXXGTSGTPS SXPXXTXXXP LLXPFTXNXT

ITNLXXXXXM XXPGSRKFNT TEXVLQGLLX PXFKNXSVGX LYSGCRLTXL RXEKXGAATG

XDAICXHXXX PKXPGLXXEX LYWELSXLTX XIXELGPYTL DRXSLYVNGF THRSSVPTTS

SPGTSTVHLA TSGTPSSLPG HTAPVPLLIP FTLNFTITNL HYEENMQHPG SRKFNTTERV

LQGLLKPLFK STSVGPLYSG CRLTLLRPEK HGAATGVDAI CTLRLDPTGP GLXXEXLYWE

LSXLTXXIXE LGPYTLDRXS LYVNGFTHXX SXPTTSTPGT STVXXGTSGT PSSXPXXTXX

XPLLXPFTXN XTITNLXXXX XMXXPGSRKF NTTEXVLQGL LXPXFKNXSV GXLYSGCRLT

XLRXEKXGAA TGXDAICXHX XXPKXPGLXX EXLYWELSXL TXXIXELGPY TLDRXSLYVN

GFTHRTSVPT TSTPGTSTVH LATSGTPSSL PGHTAPVPLL IPFTLNFTIT NLQYEEDMHR

PGSRKFNTTE RVLQGLLSPI FKNSSVGPLY SGCRLTSLRP EKDGAATGMD AVCLYHPNPK
```

```
RPGLDREQLY   CELSQLTHNI   TELGPYSLDR   DSLYVNGFTH   QNSVPTTSTP   GTSTVYWATT
       19090        19100        19110        19120        19130        19140

GTPSSFPGHT   XXXPLLXPFT   XNXTITNLXX   XXXMXXPGSR   KFNTTEXVLQ   GLLXPXFKNX
       19150        19160        19170        19180        19190        19200

SVGXLYSGCR   LTXLRXEKXG   AATGXDAICX   HXXXPKXPGL   XXEXLYWELS   XLTXXIXELG
       19210        19220        19230        19240        19250        19260

PYTLDRXSLY   VNGFTHWSSG   LTTSTPWTST   VDLGTSGTPS   PVPSPTTAGP   LLVPFTLNFT
       19270        19280        19290        19300        19310        19320

ITNLQYEEDM   HRPGSRKFNA   TERVLQGLLS   PIFKNTSVGP   LYSGCRLTLL   RPEKQEAATG
       19330        19340        19350        19360        19370        19380

VDTICTHRVD   PIGPGLXXEX   LYWELSXLTX   XIXELGPYTL   DRXSLYVNGF   THXXSXPTTS
       19390        19400        19410        19420        19430        19440

TPGTSTVXXG   TSGTPSSXPX   XTXXXPLLXP   FTXNXTITNL   XXXXXMXXPG   SRKFNTTEXV
       19450        19460        19470        19480        19490        19500

LQGLLXPXFK   NXSVGXLYSG   CRLTXLRXEK   XGAATGXDAI   CXHXXXPKXP   GLXXEXLYWE
       19510        19520        19530        19540        19550        19560

LSXLTXXIXE   LGPYTLDRXS   LYVNGFTHRS   FGLTTSTPWT   STVDLGTSGT   PSPVPSPTTA
       19570        19580        19590        19600        19610        19620

GPLLVPFTLN   FTITNLQYEE   DMHRPGSRKF   NTTERVLQGL   LTPLFRNTSV   SSLYSGCRLT
       19630        19640        19650        19660        19670        19680

LLRPEKDGAA   TRVDAVCTHR   PDPKSPGLXX   EXLYWELSXL   TXXIXELGPY   TLDRXSLYVN
       19690        19700        19710        19720        19730        19740

GFTHXXSXPT   TSTPGTSTVX   XGTSGTPSSX   PXXTXXXPLL   XPFTXNXTIT   NLXXXXXMXX
       19750        19760        19770        19780        19790        19800

PGSRKFNTTE   XVLQGLLXPX   FKNXSVGXLY   SGCRLTXLRX   EKXGAATGXD   AICXHXXXPK
       19810        19820        19830        19840        19850        19860

XPGLXXEXLY   WELSXLTXXI   XELGPYTLDR   XSLYVNGFTH   WIPVPTSSTP   GTSTVDLGSG
       19870        19880        19890        19900        19910        19920
```

```
TPSSLPSPTT AGPLLVPFTL NFTITNLQYG EDMGHPGSRK FNTTERVLQG LLGPIFKNTS
          19930      19940      19950      19960      19970      19980

VGPLYSGCRL TSLRSEKDGA ATGVDAICIH HLDPKSPGLX XEXLYWELSX LTXXIXELGP
          19990      20000      20010      20020      20030      20040

YTLDRXSLYV NGFTHXXSXP TTSTPGTSTV XXGTSGTPSS XPXXTXXXPL LXPFTXNXTI
          20050      20060      20070      20080      20090      20100

TNLXXXXXMX XPGSRKFNTT EXVLQGLLXP XFKNXSVGXL YSGCRLTXLR XEKXGAATGX
          20110      20120      20130      20140      20150      20160

DAICXHXXXP KXPGLXXEXL YWELSXLTXX IXELGPYTLD RXSLYVNGFT HQTFAPNTST
          20170      20180      20190      20200      20210      20220

PGTSTVDLGT SGTPSSLPSP TSAGPLLVPF TLNFTITNLQ YEEDMHHPGS RKFNTTERVL
          20230      20240      20250      20260      20270      20280

QGLLGPMFKN TSVGLLYSGC RLTLLRPEKN GAATRVDAVC THRPDPKSPG LXXEXLYWEL
          20290      20300      20310      20320      20330      20340

SXLTXXIXEL GPYTLDRXSL YVNGFTHXXS XPTTSTPGTS TVXXGTSGTP SSXPXXTAPV
          20350      20360      20370      20380      20390      20400

PLLIPFTLNF TITNLHYEEN MQHPGSRKFN TTERVLQGLL KPLFKSTSVG PLYSGCRLTL
          20410      20420      20430      20440      20450      20460

LRPEKHGAAT GVDAICTLRL DPTGPGLDRE RLYWELSQLT NSVTELGPYT LDRDSLYVNG
          20470      20480      20490      20500      20510      20520

FTQRSSVPTT SIPGTSAVHL ETSGTPASLP GHTAPGPLLV PFTLNFTITN LQYEVDMRHP
          20530      20540      20550      20560      20570      20580

GSRKFNTTER VLQGLLKPLF KSTSVGPLYS GCRLTLLRPE KRGAATGVDT ICTHRLDPLN
          20590      20600      20610      20620      20630      20640

PGLDREQLYW ELSKLTRGII ELGPYLLDRG SLYVNGFTHR NFVPITSTPG TSTVHLGTSE
          20650      20660      20670      20680      20690      20700

TPSSLPRPIV PGPLLVPFTL NFTITNLQYE EAMRHPGSRK FNTTERVLQG LLRPLFKNTS
          20710      20720      20730      20740      20750      20760
```

```
IGPLYSSCRL  TLLRPEKDKA  ATRVDAICTH  HPDPQSPGLN  REQLYWELSQ  LTHGITELGP
    20770       20780       20790       20800       20810       20820

YTLDRDSLYV  DGFTHWSPIP  TTSTPGTSIV  NLGTSGIPPS  LPETTXXXPL  LXPFTXNXTI
    20830       20840       20850       20860       20870       20880

TNLXXXXXMX  XPGSRKFNTT  ERVLQGLLKP  LFKSTSVGPL  YSGCRLTLLR  PEKDGVATRV
    20890       20900       20910       20920       20930       20940

DAICTHRPDP  KIPGLDRQQL  YWELSQLTHS  ITELGPYTLD  RDSLYVNGFT  QRSSVPTTST
    20950       20960       20970       20980       20990       21000

PGTFTVQPET  SETPSSLPGP  TATGPVLLPF  TLNFTITNLQ  YEEDMHRPGS  RKFNTTERVL
    21010       21020       21030       21040       21050       21060

QGLLMPLFKN  TSVSSLYSGC  RLTLLRPEKD  GAATRVDAVC  THRPDPKSPG  LDRERLYWKL
    21070       21080       21090       21100       21110       21120

SQLTHGITEL  GPYTLDRHSL  YVNGFTHQSS  MTTTRTPDTS  TMHLATSRTP  ASLSGPTTAS
    21130       21140       21150       21160       21170       21180

PLLVLFTINF  TITNLRYEEN  MHHPGSRKFN  TTERVLQGLL  RPVFKNTSVG  PLYSGCRLTL
    21190       21200       21210       21220       21230       21240

LRPKKDGAAT  KVDAICTYRP  DPKSPGLDRE  QLYWELSQLT  HSITELGPYT  LDRDSLYVNG
    21250       21260       21270       21280       21290       21300

FTQRSSVPTT  SIPGTPTVDL  GTSGTPVSKP  GPSAASPLLV  LFTLNFTITN  LRYEENMQHP
    21310       21320       21330       21340       21350       21360

GSRKFNTTER  VLQGLLRSLF  KSTSVGPLYS  GCRLTLLRPE  KDGTATGVDA  ICTHHPDPKS
    21370       21380       21390       21400       21410       21420

PRLDREQLYW  ELSQLTHNIT  ELGHYALDND  SLFVNGFTHR  SSVSTTSTPG  TPTVYLGASK
    21430       21440       21450       21460       21470       21480

TPASIFGPSA  ASHLLILFTL  NFTITNLRYE  ENMWPGSRKF  NTTERVLQGL  LRPLFKNTSV
    21490       21500       21510       21520       21530       21540

GPLYSGSRLT  LLRPEKDGEA  TGVDAICTHR  PDPTGPGLDR  EQLYLELSQL  THSITELGPY
    21550       21560       21570       21580       21590       21600
```

```
TLDRDSLYVN GFTHRSSVPT TSTGVVSEEP FTLNFTINNL RYMADMGQPG SLKFNITDNV
       21610      21620      21630      21640      21650      21660
MKHLLSPLFQ RSSLGARYTG CRVIALRSVK NGAETRVDLL CTYLQPLSGP GLPIKQVFHE
       21670      21680      21690      21700      21710      21720
LSQQTHGITR LGPYSLDKDS LYLNGYNEPG LDEPPTTPKP ATTFLPPLSE ATTAMGYHLK
       21730      21740      21750      21760      21770      21780
TLTLNFTISN LQYSPDMGKG SATFNSTEGV LQHLLRPLFQ KSSMGPFYLG CQLISLRPEK
       21790      21800      21810      21820      21830      21840
DGAATGVDTT CTYHPDPVGP GLDIQQLYWE LSQLTHGVTQ LGFYVLDRDS LFINGYAPQN
       21850      21860      21870      21880      21890      21900
LSIRGEYQIN FHIVNWNLSN PDPTSSEYIT LLRDIQDKVT TLYKGSQLHD TFRFCLVTNL
       21910      21920      21930      21940      21950      21960
TMDSVLVTVK ALFSSNLDPS LVEQVFLDKT LNASFHWLGS TYQLVDIHVT EMESSVYQPT
       21970      21980      21990      22000      22010      22020
SSSSTQHFYL NFTITNLPYS QDKAQPGTTN YQRNKRNIED ALNQLFRNSS IKSYFSDCQV
       22030      22040      22050      22060      22070      22080
STFRSVPNRH HTGVDSLCNF SPLARRVDRV AIYEEFLRMT RNGTQLQNFT LDRSSVLVDG
       22090      22100      22110      22120      22130      22140
YSPNRNEPLT GNSDLPFWAV ILIGLAGLLG LITCLICGVL VTTRRRKKEG EYNVQQQCPG
       22150
YYQSHLDLED LQ
```

Cancer antigen CA 15-3 is a single-pass type I membrane protein having a large extracellular domain, some or all of which is present in soluble forms Cancer antigen CA 15-3 generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). The following domains have been identified in Cancer antigen CA 15-3:

| Residues | Length | Domain ID |
| --- | --- | --- |
| 1-22152 | 22152 | Cancer antigen CA 15-3 |
| 1-22096 | 22096 | Extracellular domain |
| 22097-22117 | 21 | Transmembrane domain |
| 22118-22152 | 35 | Cytoplasmic domain |

As used herein, the term "C-C motif chemokine 18" refers to one or more polypeptides present in a biological sample that are derived from the C-C motif chemokine 18 precursor (Swiss-Prot P55774 (SEQ ID NO: 8)).

```
            10         20         30         40         50         60
   MKGLAAALLV LVCTMALCSC AQVGTNKELC CLVYTSWQIP QKFIVDYSET SPQCPKPGVI

            70         80
   LLTKRGRQIC ADPNKKWVQK YISDLKLNA
```

The following domains have been identified in C-C motif chemokine 18:

| Residues | Length | Domain ID |
|----------|--------|-----------|
| 1-20 | 20 | Signal peptide |
| 21-89 | 69 | C-C motif chemokine 18 |
| 21-88 | 68 | CCL 18 (1-68) |
| 23-89 | 67 | CCL 18 (3-69) |
| 24-89 | 66 | CCL 18 (4-69) |

As used herein, the term "C-C motif chemokine 24" refers to one or more polypeptides present in a biological sample that are derived from the C-C motif chemokine 24 precursor (Swiss-Prot 000175 (SEQ ID NO: 9)).

```
            10         20         30         40         50         60
   MAGLMTIVTS LLFLGVCAHH IIPTGSVVIP SPCCMFFVSK RIPENRVVSY QLSSRSTCLK

            70         80         90        100        110
   AGVIFTTKKG QQFCGDPKQE WVQRYMKNLD AKQKKASPRA RAVAVKGPVQ RYPGNQTTC
```

The following domains have been identified in C-C motif chemokine 24:

| Residues | Length | Domain ID |
|----------|--------|-----------|
| 1-26 | 26 | Signal peptide |
| 27-119 | 93 | C-C motif chemokine 24 |

As used herein, the term "Cathepsin D" refers to one or more polypeptides present in a biological sample that are derived from the Cathepsin D precursor (Swiss-Prot P07339 (SEQ ID NO: 10)).

```
            10         20         30         40         50         60
   MAGLMTIVTS LLFLGVCAHH IIPTGSVVIP SPCCMFFVSK RIPENRVVSY QLSSRSTCLK

            70         80         90        100        110
   AGVIFTTKKG QQFCGDPKQE WVQRYMKNLD AKQKKASPRA RAVAVKGPVQ RYPGNQTTC
```

The following domains have been identified in Capthesin D:

| Residues | Length | Domain ID |
|----------|--------|-----------|
| 1-18 | 18 | Signal peptide |
| 19-64 | 46 | Activation peptide |
| 65-412 | 348 | Cathepsin D |
| 65-161 | 348 | Cathepsin D light chain |
| 169-412 | 348 | Cathepsin D heavy chain |

As used herein, the term "C-X-C Motif chemokine 13" refers to one or more polypeptides present in a biological sample that are derived from the C-X-C Motif chemokine 13 precursor (Swiss-Prot 043927 (SEQ ID NO: 11)).

```
            10         20         30         40         50         60
      MKFISTSLLL MLLVSSLSPV QGVLEVYYTS LRCRCVQESS VFIPRRFIDR IQILPRGNGC

            70         80         90        100
      PRKEIIVWKK NKSIVCVDPQ AEWIQRMMEV LRKRSSSTLP VPVFKRKIP
```

The following domains have been identified in C-X-C Motif chemokine 13:

| Residues | Length | Domain ID |
|----------|--------|-----------|
| 1-22 | 22 | Signal peptide |
| 23-109 | 87 | C-X-C Motif chemokine 13 |

As used herein, the term "IgG1" refers to subclass 1 of the glycoprotein immunoglobulin G (IgG), a major effector molecule of the humoral immune response in man. Antibodies of the IgG class express their predominant activity during a secondary antibody response. The basic immunoglobulin G molecule has a four-chain structure, comprising two identical heavy (H) chains and two identical light (L) chains, linked together by inter-chain disulfide bonds. Each heavy chain is encoded by 4 distinct types of gene segments, designated $V_H$ (variable), D (diversity), $J_H$ (joining) and $C_H$ (constant). The variable region of the heavy chain is encoded by the $V_H$, D and $J_H$ segments. The light chains are encoded by the 3 gene segments, $V_L$, $J_L$ and $C_L$. The variable region of the light chains is encoded by the $V_L$ and $J_L$ segments.

As used herein, the term "IgG2" refers to subclass 2 of the glycoprotein immunoglobulin G (IgG), a major effector molecule of the humoral immune response in man. Antibodies of the IgG class express their predominant activity during a secondary antibody response. The basic immunoglobulin G molecule has a four-chain structure, comprising two identical heavy (H) chains and two identical light (L) chains, linked together by inter-chain disulfide bonds. Each heavy chain is encoded by 4 distinct types of gene segments, designated $V_H$ (variable), D (diversity), $J_H$ (joining) and $C_H$ (constant). The variable region of the heavy chain is encoded by the $V_H$, D and $J_H$ segments. The light chains are encoded by the 3 gene segments, $V_L$, $J_L$ and $C_L$. The variable region of the light chains is encoded by the $V_L$ and $J_L$ segments.

The length and flexibility of the hinge region varies among the IgG subclasses. The hinge region of IgG1 encompasses amino acids 216-231 and since it is freely flexible, the Fab fragments can rotate about their axes of symmetry and move within a sphere centered at the first of two inter-heavy chain disulfide bridges (23). IgG2 has a shorter hinge than IgG1, with 12 amino acid residues and four disulfide bridges. The hinge region of IgG2 lacks a glycine residue, it is relatively short and contains a rigid poly-proline double helix, stabilised by extra inter-heavy chain disulfide bridges. These properties restrict the flexibility of the IgG2 molecule (24). IgG3 differs from the other subclasses by its unique extended hinge region (about four times as long as the IgG1 hinge), containing 62 amino acids (including 21 prolines and 11 cysteines), forming an inflexible poly-proline double helix (25,26). In IgG3 the Fab fragments are relatively far away from the Fc fragment, giving the molecule a greater flexibility. The elongated hinge in IgG3 is also responsible for its higher molecular weight compared to the other subclasses. The hinge region of IgG4 is shorter than that of IgG1 and its flexibility is intermediate between that of IgG1 and IgG2.

The four IgG subclasses also differ with respect to the number of inter-heavy chain disulfide bonds in the hinge region (26). The structural differences between the IgG subclasses are also reflected in their susceptibility to proteolytic enzymes. IgG3 is very susceptible to cleavage by these enzymes, whereas IgG2 is relatively resistant. IgG1 and IgG4 exhibit an intermediary sensitivity, depending upon the enzyme used. Since these proteolytic enzymes all cleave IgG molecules near or within the hinge region, it is likely that the high sensitivity of IgG3 to enzyme digestion is related to its accessible hinge. Another structural difference between the human IgG subclasses is the linkage of the heavy and light chain by a disulfide bond. This bond links the carboxy-terminal of the light chain with the cysteine residue at position 220 (in IgG) or at position 131 (in IgG2, IgG3 and IgG4) of the CH1 sequence of the heavy chain.

As a consequence of the structural differences, the four IgG subclasses may be distinguished from one another, for example using antibodies that are specific for differences between the iso forms. In the present application, a level of IgG1 is determined using an assay which distinguishes this subclass, relative to the other subclasses.

As used herein, the term "relating a signal to the presence or amount" of an analyte reflects the following understanding. Assay signals are typically related to the presence or amount of an analyte through the use of a standard curve calculated using known concentrations of the analyte of interest. As the term is used herein, an assay is "configured to detect" an analyte if an assay can generate a detectable signal indicative of the presence or amount of a physiologically relevant concentration of the analyte. Because an antibody epitope is on the order of 8 amino acids, an immunoassay configured to detect a marker of interest will also detect polypeptides related to the marker sequence, so long as those polypeptides contain the epitope(s) necessary to bind to the antibody or antibodies used in the assay. The term "related marker" as used herein with regard to a biomarker such as one of the kidney injury markers described herein refers to one or more fragments, variants, etc., of a particular marker or its biosynthetic parent that may be detected as a surrogate for the marker itself or as independent biomarkers. The term also refers to one or more polypeptides present in a biological sample that are derived from the biomarker precursor complexed to additional species, such as binding proteins, receptors, heparin, lipids, sugars, etc.

In this regard, the skilled artisan will understand that the signals obtained from an immunoassay are a direct result of complexes formed between one or more antibodies and the target biomolecule (*i.e.,* the analyte) and polypeptides containing the necessary epitope(s) to which the antibodies bind. While such assays may detect the full length biomarker and the assay result be expressed as a concentration of a biomarker of interest, the signal from the assay is actually a result of all such "immunoreactive" polypeptides present in the sample. Expression of biomarkers may also be determined by means other than immunoassays, including protein measurements (such as dot blots, western blots, chromatographic methods, mass spectrometry, *etc*.) and nucleic acid measurements (mRNA quatitation). This list is not meant to be limiting.

The term "positive going" marker as that term is used herein refer to a marker that is determined to be elevated in subjects suffering from a disease or condition, relative to subjects not suffering from that disease or condition. The term "negative going" marker as that term is used herein refer to a marker that is determined to be reduced in subjects suffering from a disease or condition, relative to subjects not suffering from that disease or condition.

The term "subject" as used herein refers to a human or non-human organism. Thus, the methods and compositions described herein are applicable to both human and veterinary disease. Further, while a subject is preferably a living organism, the invention described herein may be used in post-mortem analysis as well. Preferred subjects are humans, and most preferably "patients," which as used herein refers to living humans that are receiving medical care for a disease or condition. This includes persons with no defined illness who are being investigated for signs of pathology.

Preferably, an analyte is measured in a sample. Such a sample may be obtained from a subject, or may be obtained from biological materials intended to be provided to the subject. For example, a sample may be obtained from a kidney being evaluated for possible transplantation into a subject, and an analyte measurement used to evaluate the kidney for preexisting damage. Preferred samples are body fluid samples.

The term "body fluid sample" as used herein refers to a sample of bodily fluid obtained for the purpose of diagnosis, prognosis, classification or evaluation of a subject of interest, such as a patient or transplant donor. In certain embodiments, such a sample may be obtained for the purpose of determining the outcome of an ongoing condition or the effect of a treatment regimen on a condition. Preferred body fluid samples include blood, serum, plasma, cerebrospinal fluid, urine, saliva, sputum, and pleural effusions. In addition, one of skill in the art would realize that certain body fluid samples would be more readily analyzed following a fractionation or purification procedure, for example, separation of whole blood into serum or plasma components.

The term "diagnosis" as used herein refers to methods by which the skilled artisan can estimate and/or determine the probability ("a likelihood") of whether or not a patient is suffering from a given disease or condition. In the case of the present invention, "diagnosis" includes using the results of an assay, most preferably an immunoassay, for a kidney injury marker of the present invention, optionally together with other clinical characteristics, to arrive at a diagnosis (that is, the occurrence or nonoccurrence) of an acute renal injury or ARF for the subject from which a sample was obtained and assayed. That such a diagnosis is "determined" is not meant to imply that the diagnosis is 100% accurate. Many biomarkers are indicative of multiple conditions. The skilled clinician does not use biomarker results in an informational vacuum, but rather test results are used together with other clinical indicia to arrive at a diagnosis. Thus, a measured biomarker level on one side of a predetermined diagnostic threshold indicates a greater likelihood of the occurrence of disease in the subject relative to a measured level on the other side of the predetermined diagnostic threshold.

Similarly, a prognostic risk signals a probability ("a likelihood") that a given course or outcome will occur. A level or a change in level of a prognostic indicator, which in turn is associated with an increased probability of morbidity (e.g., worsening renal function, future ARF, or death) is referred to as being "indicative of an increased likelihood" of an adverse outcome in a patient.

Marker Assays

In general, immunoassays involve contacting a sample containing or suspected of containing a biomarker of interest with at least one antibody that specifically binds to the biomarker. A signal is then generated indicative of the presence or amount of complexes formed by the binding of polypeptides in the sample to the antibody. The signal is then related to the presence or amount of the biomarker in the sample. Numerous methods and devices are well known to the skilled artisan for the detection and analysis of biomarkers. *See, e.g.,* U.S. Patents 6,143,576; 6,113,855; 6,019,944; 5,985,579; 5,947,124; 5,939,272; 5,922,615; 5,885,527; 5,851,776; 5,824,799; 5,679,526; 5,525,524; and 5,480,792, and The Immunoassay Handbook, David Wild, ed. Stockton Press, New York, 1994, each of which is hereby incorporated by reference in its entirety, including all tables, figures and claims.

The assay devices and methods known in the art can utilize labeled molecules in various sandwich, competitive, or non-competitive assay formats, to generate a signal that is related to the presence or amount of the biomarker of interest. Suitable assay formats also include chromatographic, mass spectrographic, and protein "blotting" methods. Additionally, certain methods and devices, such as biosensors and optical immunoassays, may be employed to determine the presence or amount of analytes without the need for a labeled molecule. *See, e.g.,* U.S. Patents 5,631,171; and 5,955,377, each of which is hereby incorporated by reference in its entirety, including all tables, figures and claims. One skilled in the art also recognizes that robotic instrumentation including but not limited to Beckman ACCESS®, Abbott AXSYM®, Roche ELECSYS®, Dade Behring STRATUS® systems are among the immunoassay analyzers that are capable of performing immunoassays. But any suitable immunoassay may be utilized, for example, enzyme-linked immunoassays (ELISA), radioimmunoassays (RIAs), competitive binding assays, and the like.

Antibodies or other polypeptides may be immobilized onto a variety of solid supports for use in assays. Solid phases that may be used to immobilize specific binding members include include those developed and/or used as solid phases in solid phase binding assays. Examples of suitable solid phases include membrane filters, cellulose-based papers, beads (including polymeric, latex and paramagnetic particles), glass, silicon wafers, microparticles, nanoparticles, TentaGels, AgroGels, PEGA gels, SPOCC gels, and multiple-well plates. An assay strip could be prepared by coating the antibody or a plurality of antibodies in an array on solid support. This strip could then be dipped into the test sample and then processed quickly through washes and detection steps to generate a measurable signal, such as a colored spot. Antibodies or other polypeptides may be bound to specific zones of assay devices either by conjugating directly to an assay device surface, or by indirect binding. In an example of the later case, antibodies or other polypeptides may be immobilized on particles or other solid supports, and that solid support immobilized to the device surface.

Biological assays require methods for detection, and one of the most common methods for quantitation of results is to conjugate a detectable label to a protein or nucleic acid that has affinity for one of the components in the biological system being studied. Detectable labels may include molecules that are themselves detectable (*e.g.,* fluorescent moieties, electrochemical labels, metal chelates, *etc.*) as well as molecules that may be indirectly detected by production of a detectable reaction product (*e.g.,* enzymes such as horseradish peroxidase, alkaline phosphatase, *etc.*) or by a specific binding molecule which itself may be detectable (e.g., biotin, digoxigenin, maltose, oligohistidine, 2,4-dintrobenzene, phenylarsenate, ssDNA, dsDNA, etc.).

Preparation of solid phases and detectable label conjugates often comprise the use of chemical cross-linkers. Cross-linking reagents contain at least two reactive groups, and are divided generally into homo functional cross-linkers (containing identical reactive groups) and heterofunctional cross-linkers (containing non-identical reactive groups). Homobifunctional cross-linkers that couple through amines, sulfhydryls or react non-specifically are available from many commercial sources. Maleimides, alkyl and aryl halides, alpha-haloacyls and pyridyl disulfides are thiol reactive groups. Maleimides, alkyl and aryl halides, and alpha-haloacyls react with sulfhydryls to form thiol ether bonds, while pyridyl disulfides react with sulfhydryls to produce mixed disulfides. The pyridyl disulfide product is cleavable. Imidoesters are also very useful for protein-protein cross-links. A variety of heterobifunctional cross-linkers, each combining different attributes for successful conjugation, are commercially available.

In certain aspects, the present invention provides kits for the analysis of the described kidney injury markers. The kit comprises reagents for the analysis of at least one test sample which comprise at least one antibody that a kidney injury marker. The kit can also include devices and instructions for performing one or more of the diagnostic and/or prognostic correlations described herein. Preferred kits will comprise an antibody pair for performing a sandwich assay, or a labeled species for performing a competitive assay, for the analyte. Preferably, an antibody pair comprises a first antibody conjugated to a solid phase and a second antibody conjugated to a detectable label, wherein each of the first and second antibodies that bind a kidney injury marker. Most preferably each of the antibodies are monoclonal antibodies. The instructions for use of the kit and performing the correlations can be in the form of labeling, which refers to any written or recorded material that is attached to, or otherwise accompanies a kit at any time during its manufacture, transport, sale or use. For example, the term labeling encompasses advertising leaflets and brochures, packaging materials, instructions, audio or video cassettes, computer discs, as well as writing imprinted directly on kits.

Antibodies

The term "antibody" as used herein refers to a peptide or polypeptide derived from, modeled after or substantially encoded by an immunoglobulin gene or immunoglobulin genes, or fragments thereof, capable of specifically binding an antigen or epitope. *See, e.g.* Fundamental Immunology, 3rd Edition, W.E. Paul, ed., Raven Press, N.Y. (1993); Wilson (1994; J. Immunol. Methods 175:267-273; Yarmush (1992) J. Biochem. Biophys. Methods 25:85-97. The term antibody includes antigen-binding portions, i.e., "antigen binding sites," (e.g., fragments, subsequences, complementarity determining regions (CDRs)) that retain capacity to bind antigen, including (i) a Fab fragment, a monovalent fragment consisting of the VL, VH, CL and CHI domains; (ii) a F(ab')2 fragment, a bivalent fragment comprising two Fab fragments linked by a disulfide bridge at the hinge region; (iii) a Fd fragment consisting of the VH and CHI domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Single chain antibodies are also included by reference in the term "antibody."

Antibodies used in the immunoassays described herein preferably specifically bind to a kidney injury marker of the present invention. The term "specifically binds" is not intended to indicate that an antibody binds exclusively to its intended target since, as noted above, an antibody binds to any polypeptide displaying the epitope(s) to which the antibody binds. Rather, an antibody "specifically binds" if its affinity for its intended target is about 5-fold greater when compared to its affinity for a non-target molecule which does not display the appropriate epitope(s). Preferably the affinity of the antibody will be at least about 5 fold, preferably 10 fold, more preferably 25-fold, even more preferably 50-fold, and most preferably 100-fold or more, greater for a target molecule than its affinity for a non-target molecule. In preferred embodiments, Preferred antibodies bind with affinities of at least about $10^7$ $M^{-1}$, and preferably between about $10^8$ $M^{-1}$ to about $10^9$ $M^{-1}$, about $10^9$ $M^{-1}$ to about $10^{10}$ $M^{-1}$, or about $10^{10}$ $M^{-1}$ to about $10^{12}$ $M^{-1}$.

Affinity is calculated as $K_d = k_{off}/k_{on}$ ($k_{off}$ is the dissociation rate constant, $K_{on}$ is the association rate constant and $K_d$ is the equilibrium constant). Affinity can be determined at equilibrium by measuring the fraction bound (r) of labeled ligand at various concentrations (c). The data are graphed using the Scatchard equation: r/c = K(n-r): where r = moles of bound ligand/mole of receptor at equilibrium; c = free ligand concentration at equilibrium; K = equilibrium association constant; and n = number of ligand binding sites per receptor molecule. By graphical analysis, r/c is plotted on the Y-axis versus r on the X-axis, thus producing a Scatchard plot. Antibody affinity measurement by Scatchard analysis is well known in the art. *See, e.g.,* van Erp et al., J. Immunoassay 12: 425-43, 1991; Nelson and Griswold, Comput. Methods Programs Biomed. 27: 65-8, 1988.

The term "epitope" refers to an antigenic determinant capable of specific binding to an antibody. Epitopes usually consist of chemically active surface groupings of molecules such as amino acids or sugar side chains and usually have specific three dimensional structural characteristics, as well as specific charge characteristics. Conformational and nonconformational epitopes are distinguished in that the binding to the former but not the latter is lost in the presence of denaturing solvents.

Numerous publications discuss the use of phage display technology to produce and screen libraries of polypeptides for binding to a selected analyte. *See, e.g,* Cwirla et al., Proc. Natl. Acad. Sci. USA 87, 6378-82, 1990; Devlin et al., Science 249, 404-6, 1990, Scott and Smith, Science 249, 386-88, 1990; and Ladner et al., U.S. Pat. No. 5,571,698. A basic concept of phage display methods is the establishment of a physical association between DNA encoding a polypeptide to be screened and the polypeptide. This physical association is provided by the phage particle, which displays a polypeptide as part of a capsid enclosing the phage genome which encodes the polypeptide. The establishment of a physical association between polypeptides and their genetic material allows simultaneous mass screening of very large numbers of phage bearing different polypeptides. Phage displaying a polypeptide with affinity to a target bind to the target and these phage are enriched by affinity screening to the target. The identity of polypeptides displayed from these phage can be determined from their respective genomes. Using these methods a polypeptide identified as having a binding affinity for a desired target can then be synthesized in bulk by conventional means. *See, e.g.,* U.S. Patent No. 6,057,098, which is hereby incorporated in its entirety, including all tables, figures, and claims.

The antibodies that are generated by these methods may then be selected by first screening for affinity and specificity with the purified polypeptide of interest and, if required, comparing the results to the affinity and specificity of the antibodies with polypeptides that are desired to be excluded from binding. The screening procedure can involve immobilization of the purified polypeptides in separate wells of microtiter plates. The solution containing a potential antibody or groups of antibodies is then placed into the respective microtiter wells and incubated for about 30 min to 2 h. The microtiter wells are then washed and a labeled secondary antibody (for example, an anti-mouse antibody conjugated to alkaline phosphatase if the raised antibodies are mouse antibodies) is added to the wells and incubated for about 30 min and then washed. Substrate is added to the wells and a color reaction will appear where antibody to the immobilized polypeptide(s) are present.

The antibodies so identified may then be further analyzed for affinity and specificity in the assay design selected. In the development of immunoassays for a target protein, the purified target protein acts as a standard with which to judge the sensitivity and specificity of the immunoassay using the antibodies that have been selected. Because the binding affinity of various antibodies may differ; certain antibody pairs (e.g., in sandwich assays) may interfere with one another sterically, etc., assay performance of an antibody may be a more important measure than absolute affinity and specificity of an antibody.

While the present application describes antibody-based binding assays in detail, alternatives to antibodies as binding species in assays are well known in the art. These include receptors for a particular target, aptamers, etc. Aptamers are oligonucleic acid or peptide molecules that bind to a specific target molecule. Aptamers are usually created by selecting them from a large random sequence pool, but natural aptamers also exist. High-affinity aptamers containing modified nucleotides conferring improved characteristics on the ligand, such as improved in vivo stability or improved delivery characteristics. Examples of such modifications include chemical substitutions at the ribose and/or phosphate and/or base positions, and may include amino acid side chain functionalities.

Assay Correlations

The term "correlating" as used herein in reference to the use of biomarkers refers to comparing the presence or amount of the biomarker(s) in a patient to its presence or amount in persons known to suffer from, or known to be at risk of, a given condition; or in persons known to be free of a given condition. Often, this takes the form of comparing an assay result in the form of a biomarker concentration to a predetermined threshold selected to be indicative of the occurrence or nonoccurrence of a disease or the likelihood of some future outcome.

Selecting a diagnostic threshold involves, among other things, consideration of the probability of disease, distribution of true and false diagnoses at different test thresholds, and estimates of the consequences of treatment (or a failure to treat) based on the diagnosis. For example, when considering administering a specific therapy which is highly efficacious and has a low level of risk, few tests are needed because clinicians can accept substantial diagnostic uncertainty. On the other hand, in situations where treatment options are less effective and more risky, clinicians often need a higher degree of diagnostic certainty. Thus, cost/benefit analysis is involved in selecting a diagnostic threshold.

Suitable thresholds may be determined in a variety of ways. For example, one recommended diagnostic threshold for the diagnosis of acute myocardial infarction using cardiac troponin is the 97.5th percentile of the concentration seen in a normal population. Another method may be to look at serial samples from the same patient, where a prior "baseline" result is used to monitor for temporal changes in a biomarker level.

Population studies may also be used to select a decision threshold. Reciever Operating Characteristic ("ROC") arose from the field of signal dectection therory developed during World War II for the analysis of radar images, and ROC analysis is often used to select a threshold able to best distinguish a "diseased" subpopulation from a "nondiseased" subpopulation. A false positive in this case occurs when the person tests positive, but actually does not have the disease. A false negative, on the other hand, occurs when the person tests negative, suggesting they are healthy, when they actually do have the disease. To draw a ROC curve, the true positive rate (TPR) and false positive rate (FPR) are determined as the decision threshold is varied continuously. Since TPR is equivalent with sensitivity and FPR is equal to 1 - specificity, the ROC graph is sometimes called the sensitivity vs (1 - specificity) plot. A perfect test will have an area under the ROC curve of 1.0; a random test will have an area of 0.5. A threshold is selected to provide an acceptable level of specificity and sensitivity.

In this context, "diseased" is meant to refer to a population having one characteristic (the presence of a disease or condition or the occurrence of some outcome) and "nondiseased" is meant to refer to a population lacking the characteristic. While a single decision threshold is the simplest application of such a method, multiple decision thresholds may be used. For example, below a first threshold, the absence of disease may be assigned with relatively high confidence, and above a second threshold the presence of disease may also be assigned with relatively high confidence. Between the two thresholds may be considered indeterminate. This is meant to be exemplary in nature only.

In addition to threshold comparisons, other methods for correlating assay results to a patient classification (occurrence or nonoccurrence of disease, likelihood of an outcome, etc.) include decision trees, rule sets, Bayesian methods, and neural network methods. These methods can produce probability values representing the degree to which a subject belongs to one classification out of a plurality of classifications.

Measures of test accuracy may be obtained as described in Fischer et al., Intensive Care Med. 29: 1043-51, 2003, and used to determine the effectiveness of a given biomarker. These measures include sensitivity and specificity, predictive values, likelihood ratios, diagnostic odds ratios, and ROC curve areas. The area under the curve ("AUC") of a ROC plot is equal to the probability that a classifier will rank a randomly chosen positive instance higher than a randomly chosen negative one. The area under the ROC curve may be thought of as equivalent to the Mann-Whitney U test, which tests for the median difference between scores obtained in the two groups considered if the groups are of continuous data, or to the Wilcoxon test of ranks.

As discussed above, suitable tests may exhibit one or more of the following results on these various measures: a specificity of greater than 0.5, preferably at least 0.6, more preferably at least 0.7, still more preferably at least 0.8, even more preferably at least 0.9 and most preferably at least 0.95, with a corresponding sensitivity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; a sensitivity of greater than 0.5, preferably at least 0.6, more preferably at least 0.7, still more preferably at least 0.8, even more preferably at least 0.9 and most preferably at least 0.95, with a corresponding specificity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; at least 75% sensitivity, combined with at least 75% specificity; a ROC curve area of greater than 0.5, preferably at least 0.6, more preferably 0.7, still more preferably at least 0.8, even more preferably at least 0.9, and most preferably at least 0.95; an odds ratio different from 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less; a positive likelihood ratio (calculated as sensitivity/(1-specificity)) of greater than 1, at least 2, more preferably at least 3, still more preferably at least 5, and most preferably at least 10; and or a negative likelihood ratio (calculated as (1-sensitivity)/specificity) of less than 1, less than or equal to 0.5, more preferably less than or equal to 0.3, and most preferably less than or equal to 0.1

Additional clinical indicia may be combined with the kidney injury marker assay result(s) of the present invention. These include other biomarkers related to renal status. Examples include the following, which recite the common biomarker name, followed by the Swiss-Prot entry number for that biomarker or its parent: Actin (P68133); Adenosine deaminase binding protein (DPP4, P27487); Alpha-1-acid glycoprotein 1 (P02763); Alpha-1-microglobulin (P02760); Albumin (P02768); Angiotensinogenase (Renin, P00797); Annexin A2 (P07355); Beta-glucuronidase (P08236); B-2-microglobulin (P61679); Beta-galactosidase (P16278); BMP-7 (P18075); Brain natriuretic peptide (proBNP, BNP-32, NTproBNP; P16860); Calcium-binding protein Beta (S100-beta, P04271); Carbonic anhydrase (Q16790); Casein Kinase 2 (P68400); Ceruloplasmin (P00450); Clusterin (PI0909); Complement C3 (P01024); Cysteine-rich protein (CYR61, 000622); Cytochrome C (P99999); Epidermal growth factor (EGF, P01133); Endothelin-1 (P05305); Exosomal Fetuin-A (P02765); Fatty acid-binding protein, heart (FABP3, P05413); Fatty acid-binding protein, liver (P07148); Ferritin (light chain, P02793; heavy chain P02794); Fructose-1,6-biphosphatase (P09467); GRO-alpha (CXCL1, (P09341); Growth Hormone (P01241); Hepatocyte growth factor (P14210); Insulin-like growth factor I (P01343); Immunoglobulin G; Immunoglobulin Light Chains (Kappa and Lambda); Interferon gamma (P01308); Lysozyme (P61626); Interleukin-1alpha (P01583); Interleukin-2 (P60568); Interleukin-4 (P60568); Interleukin-9 (P15248); Interleukin-12p40 (P29460); Interleukin-13 (P35225); Interleukin-16 (Q14005); L1 cell adhesion molecule (P32004); Lactate dehydrogenase (P00338); Leucine Aminopeptidase (P28838); Meprin A-alpha subunit (Q16819); Meprin A-beta subunit (Q16820); Midkine (P21741); MIP2-alpha (CXCL2, P19875); MMP-2 (P08253); MMP-9 (P14780); Netrin-1 (095631); Neutral endopeptidase (P08473); Osteopontin (P10451); Renal papillary antigen 1 (RPA1); Renal papillary antigen 2 (RPA2); Retinol binding protein (P09455); Ribonuclease; S100 calcium-binding protein A6 (P06703); Serum Amyloid P Component (P02743); Sodium/Hydrogen exchanger isoform (NHE3, P48764); Spermidine/spermine N1-acetyltransferase (P21673); TGF-Beta1 (P01137); Transferrin (P02787); Trefoil factor 3 (TFF3, Q07654); Toll-Like protein 4 (000206); Total protein; Tubulointerstitial nephritis antigen (Q9UJW2); Uromodulin (Tamm-Horsfall protein, P07911).

For purposes of risk stratification, Adiponectin (Q15848); Alkaline phosphatase (P05186); Aminopeptidase N (P15144); CalbindinD28k (P05937); Cystatin C (P01034); 8 subunit of FIFO ATPase (P03928); Gamma-glutamyltransferase (P19440); GSTa (alpha-glutathione-S-transferase, P08263); GSTpi (Glutathione-S-transferase P; GST class-pi; P09211); IGFBP-1 (P08833); IGFBP-2 (P18065); IGFBP-6 (P24592); Integral membrane protein 1 (Itm1, P46977); Interleukin-6 (P05231); Interleukin-8 (P10145); Interleukin-18 (Q14116); IP-10 (10 kDa interferon-gamma-induced protein, P02778); IRPR (IFRD1, 000458); Isovaleryl-CoA dehydrogenase (IVD, P26440); I-TAC/CXCL11 (014625); Keratin 19 (P08727); Kim-1 (Hepatitis A virus cellular receptor 1, O43656); L-arginine:glycine amidinotransferase (P50440); Leptin (P41159); Lipocalin2 (NGAL, P80188); MCP-1 (P13500); MIG (Gamma-interferon-induced monokine Q07325); MIP-1a (P10147); MIP-3a (P78556); MIP-1beta (P13236); MIP-1d (Q16663); NAG (N-acetyl-beta-D-glucosaminidase, P54802); Organic ion transporter (OCT2, 015244); Osteoprotegerin (014788); P8 protein (060356); Plasminogen activator inhibitor 1 (PAI-1, P05121); ProANP(1-98) (P01160); Protein phosphatase 1-beta (PPI-beta, P62140); Rab GDI-beta (P50395); Renal kallikrein (Q86U61); RT1.B-1 (alpha) chain of the integral membrane protein (Q5Y7A8); Soluble tumor necrosis factor receptor superfamily member 1A (sTNFR-I, P19438); Soluble tumor necrosis factor receptor superfamily member 1B (sTNFR-II, P20333); Tissue inhibitor of metalloproteinases 3 (TIMP-3, P35625); uPAR (Q03405) may be combined with the kidney injury marker assay result(s) of the present invention.

Other clinical indicia which may be combined with the kidney injury marker assay result(s) of the present invention includes demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (*e.g.,* blood pressure, temperature, respiration rate), risk scores (APACHE score, PREDICT score, TIMI Risk Score for UA/NSTEMI, Framingham Risk Score), a urine total protein measurement, a glomerular filtration rate, an estimated glomerular filtration rate, a urine production rate, a serum or plasma creatinine concentration, a renal papillary antigen 1 (RPA1) measurement; a renal papillary antigen 2 (RPA2) measurement; a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, and/or a renal failure index calculated as urine sodium / (urine creatinine / plasma creatinine). Other measures of renal function which may be combined with the kidney injury marker assay result(s) are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815, each of which are hereby incorporated by reference in their entirety.

Combining assay results/clinical indicia in this manner can comprise the use of multivariate logistical regression, loglinear modeling, neural network analysis, n-of-m analysis, decision tree analysis, etc. This list is not meant to be limiting.

Diagnosis of Acute Renal Failure

As noted above, the terms "acute renal (or kidney) injury" and "acute renal (or kidney) failure" as used herein are defined in part in terms of changes in serum creatinine from a baseline value. Most definitions of ARF have common elements, including the use of serum creatinine and, often, urine output. Patients may present with renal dysfunction without an available baseline measure of renal function for use in this comparison. In such an event, one may estimate a baseline serum creatinine value by assuming the patient initially had a normal GFR. Glomerular filtration rate (GFR) is the volume of fluid filtered from the renal (kidney) glomerular capillaries into the Bowman's capsule per unit time. Glomerular filtration rate (GFR) can be calculated by measuring any chemical that has a steady level in the blood, and is freely filtered but neither reabsorbed nor secreted by the kidneys. GFR is typically expressed in units of ml/min:

$$GFR = \frac{\text{Urine Concentration} \times \text{Urine Flow}}{\text{Plasma Concentration}}$$

By normalizing the GFR to the body surface area, a GFR of approximately 75-100 ml/min per 1.73 m$^2$ can be assumed. The rate therefore measured is the quantity of the substance in the urine that originated from a calculable volume of blood.

There are several different techniques used to calculate or estimate the glomerular filtration rate (GFR or eGFR). In clinical practice, however, creatinine clearance is used to measure GFR. Creatinine is produced naturally by the body (creatinine is a metabolite of creatine, which is found in muscle). It is freely filtered by the glomerulus, but also actively secreted by the renal tubules in very small amounts such that creatinine clearance overestimates actual GFR by 10-20%. This margin of error is acceptable considering the ease with which creatinine clearance is measured.

Creatinine clearance (CCr) can be calculated if values for creatinine's urine concentration ($U_{Cr}$), urine flow rate (V), and creatinine's plasma concentration ($P_{Cr}$) are known. Since the product of urine concentration and urine flow rate yields creatinine's excretion rate, creatinine clearance is also said to be its excretion rate ($U_{Cr} \times V$) divided by its plasma concentration. This is commonly represented mathematically as:

$$C_{Cr} = \frac{U_{Cr} \times V}{P_{Cr}}$$

Commonly a 24 hour urine collection is undertaken, from empty-bladder one morning to the contents of the bladder the following morning, with a comparative blood test then taken:

$$C_{Cr} = \frac{U_{Cr} \times 24\text{-hour volume}}{P_{Cr} \times 24 \times 60 mins}$$

To allow comparison of results between people of different sizes, the CCr is often corrected for the body surface area (BSA) and expressed compared to the average sized man as ml/min/1.73 m2. While most adults have a BSA that approaches 1.7 (1.6-1.9), extremely obese or slim patients should have their CCr corrected for their actual BSA:

$$C_{Cr-corrected} = \frac{C_{Cr} \times 1.73}{BSA}$$

The accuracy of a creatinine clearance measurement (even when collection is complete) is limited because as glomerular filtration rate (GFR) falls creatinine secretion is increased, and thus the rise in serum creatinine is less. Thus, creatinine excretion is much greater than the filtered load, resulting in a potentially large overestimation of the GFR (as much as a twofold difference). However, for clinical purposes it is important to determine whether renal function is stable or getting worse or better. This is often determined by monitoring serum creatinine alone. Like creatinine clearance, the serum creatinine will not be an accurate reflection of GFR in the non-steady-state condition of ARF. Nonetheless, the degree to which serum creatinine changes from baseline will reflect the change in GFR. Serum creatinine is readily and easily measured and it is specific for renal function.

For purposes of determining urine output on a Urine output on a mL/kg/hr basis, hourly urine collection and measurement is adequate. In the case where, for example, only a cumulative 24-h output was available and no patient weights are provided, minor modifications of the RIFLE urine output criteria have been described. For example, Bagshaw et al., Nephrol. Dial. Transplant. 23: 1203-1210, 2008, assumes an average patient weight of 70 kg, and patients are assigned a RIFLE classification based on the following: <35 mL/h (Risk), <21 mL/h (Injury) or <4 mL/h (Failure).

Selecting a Treatment Regimen

Once a diagnosis is obtained, the clinician can readily select a treatment regimen that is compatible with the diagnosis, such as initiating renal replacement therapy, withdrawing delivery of compounds that are known to be damaging to the kidney, kidney transplantation, delaying or avoiding procedures that are known to be damaging to the kidney, modifying diuretic administration, initiating goal directed therapy, etc. The skilled artisan is aware of appropriate treatments for numerous diseases discussed in relation to the methods of diagnosis described herein. See, e.g., Merck Manual of Diagnosis and Therapy, 17th Ed. Merck Research Laboratories, Whitehouse Station, NJ, 1999. In addition, since the methods and compositions described herein provide prognostic information, the markers of the present invention may be used to monitor a course of treatment. For example, improved or worsened prognostic state may indicate that a particular treatment is or is not efficacious.

One skilled in the art readily appreciates that the present invention is well adapted to carry out the objects and obtain the ends and advantages mentioned, as well as those inherent therein. The examples provided herein are representative of preferred embodiments, are exemplary, and are not intended as limitations on the scope of the invention.

Example 1: Contrast-induced nephropathy sample collection

The objective of this sample collection study is to collect samples of plasma and urine and clinical data from patients before and after receiving intravascular contrast media. Approximately 250 adults undergoing radiographic/angiographic procedures involving intravascular administration of iodinated contrast media are enrolled. To be enrolled in the study, each patient must meet all of the following inclusion criteria and none of the following exclusion criteria:

Inclusion Criteria
males and females 18 years of age or older;
undergoing a radiographic / angiographic procedure (such as a CT scan or coronary intervention) involving the intravascular administration of contrast media;
expected to be hospitalized for at least 48 hours after contrast administration.
able and willing to provide written informed consent for study participation and to comply with all study procedures.

Exclusion Criteria
renal transplant recipients;
acutely worsening renal function prior to the contrast procedure;
already receiving dialysis (either acute or chronic) or in imminent need of dialysis at enrollment;
expected to undergo a major surgical procedure (such as involving cardiopulmonary bypass) or an additional imaging procedure with contrast media with significant risk for further renal insult within the 48 hrs following contrast administration;
participation in an interventional clinical study with an experimental therapy within the previous 30 days;
known infection with human immunodeficiency virus (HIV) or a hepatitis virus.

Immediately prior to the first contrast administration (and after any pre-procedure hydration), an EDTA anti-coagulated blood sample (10 mL) and a urine sample (10 mL) are collected from each patient. Blood and urine samples are then collected at 4 ($\pm$0.5), 8 ($\pm$1), 24 ($\pm$2) 48 ($\pm$2), and 72 ($\pm$2) hrs following the last administration of contrast media during the index contrast procedure. Blood is collected via direct venipuncture or via other available venous access, such as an existing femoral sheath, central venous line, peripheral intravenous line or hep-lock. These study blood samples are processed to plasma at the clinical site, frozen and shipped to Astute Medical, Inc., San Diego, CA. The study urine samples are frozen and shipped to Astute Medical, Inc.

Serum creatinine is assessed at the site immediately prior to the first contrast administration (after any pre-procedure hydration) and at 4 ($\pm$0.5), 8 ($\pm$1), 24 ($\pm$2) and 48 ($\pm$2)), and 72 ($\pm$2) hours following the last administration of contrast (ideally at the same time as the study samples are obtained). In addition, each patient's status is evaluated through day 30 with regard to additional serum and urine creatinine measurements, a need for dialysis, hospitalization status, and adverse clinical outcomes (including mortality).

Prior to contrast administration, each patient is assigned a risk based on the following assessment: systolic blood pressure <80 mm Hg = 5 points; intra-arterial balloon pump = 5 points; congestive heart failure (Class III-IV or history of pulmonary edema) = 5 points; age >75 yrs = 4 points; hematocrit level <39% for men, <35% for women = 3 points; diabetes = 3 points; contrast media volume = 1 point for each 100 mL; serum creatinine level >1.5 g/dL = 4 points OR estimated GFR 40-60 mL/min/1.73 m$^2$ = 2 points, 20-40 mL/min/1.73 m$^2$ = 4 points, < 20 mL/min/1.73 m$^2$ = 6 points. The risks assigned are as follows: risk for CIN and dialysis: 5 or less total points = risk of CIN - 7.5%, risk of dialysis - 0.04%; 6-10 total points = risk of CIN - 14%, risk of dialysis - 0.12%; 11-16 total points = risk of CIN - 26.1%, risk of dialysis - 1.09%; >16 total points = risk of CIN - 57.3%, risk of dialysis - 12.8%.

Example 2: Cardiac surgery sample collection

The objective of this sample collection study is to collect samples of plasma and urine and clinical data from patients before and after undergoing cardiovascular surgery, a procedure known to be potentially damaging to kidney function. Approximately 900 adults undergoing such surgery are enrolled. To be enrolled in the study, each patient must meet all of the following inclusion criteria and none of the following exclusion criteria:

Inclusion Criteria
males and females 18 years of age or older;
undergoing cardiovascular surgery;
Toronto/Ottawa Predictive Risk Index for Renal Replacement risk score of at least 2 (Wijeysundera et al., JAMA 297: 1801-9, 2007); and
able and willing to provide written informed consent for study participation and to comply with all study procedures.
Exclusion Criteria
known pregnancy;
previous renal transplantation;
acutely worsening renal function prior to enrollment (e.g., any category of RIFLE criteria);
already receiving dialysis (either acute or chronic) or in imminent need of dialysis at enrollment;
currently enrolled in another clinical study or expected to be enrolled in another clinical study within 7 days of cardiac surgery that involves drug infusion or a therapeutic intervention for AKI;
known infection with human immunodeficiency virus (HIV) or a hepatitis virus.

Within 3 hours prior to the first incision (and after any pre-procedure hydration), an EDTA anti-coagulated blood sample (10 mL), whole blood (3 mL), and a urine sample (35 mL) are collected from each patient. Blood and urine samples are then collected at 3 ($\pm$0.5), 6 ($\pm$0.5), 12 ($\pm$1), 24 ($\pm$2) and 48 ($\pm$2) hrs following the procedure and then daily on days 3 through 7 if the subject remains in the hospital. Blood is collected via direct venipuncture or via other available venous access, such as an existing femoral sheath, central venous line, peripheral intravenous line or hep-lock. These study blood samples are frozen and shipped to Astute Medical, Inc., San Diego, CA. The study urine samples are frozen and shipped to Astute Medical, Inc.

Example 3: Acutely ill subject sample collection

The objective of this study is to collect samples from acutely ill patients. Approximately 1900 adults expected to be in the ICU for at least 48 hours will be enrolled. To be enrolled in the study, each patient must meet all of the following inclusion criteria and none of the following exclusion criteria:
Inclusion Criteria
males and females 18 years of age or older;

Study population 1: approximately 300 patients that have at least one of:

shock (SBP < 90 mmHg and/or need for vasopressor support to maintain MAP > 60 mmHg and/or documented drop in SBP of at least 40 mmHg); and

sepsis;

Study population 2: approximately 300 patients that have at least one of:

IV antibiotics ordered in computerized physician order entry (CPOE) within 24 hours of enrollment;

contrast media exposure within 24 hours of enrollment;

increased Intra-Abdominal Pressure with acute decompensated heart failure; and severe trauma as the primary reason for ICU admission and likely to be hospitalized in the ICU for 48 hours after enrollment;

Study population 3: approximately 300 patients expected to be hospitalized through acute care setting (ICU or ED) with a known risk factor for acute renal injury (*e.g.* sepsis, hypotension/shock (Shock = systolic BP < 90 mmHg and/or the need for vasopressor support to maintain a MAP > 60 mmHg and/or a documented drop in SBP > 40 mmHg), major trauma, hemorrhage, or major surgery); and/or expected to be hospitalized to the ICU for at least 24 hours after enrollment;

Study population 4: approximately 1000 patients that are 21 years of age or older, within 24 hours of being admitted into the ICU, expected to have an indwelling urinary catheter for at least 48 hours after enrollment, and have at least one of the following acute conditions within 24 hours prior to enrollment:

(i) respiratory SOFA score of $\geq$ 2 (PaO2/FiO2 <300), (ii) cardiovascular SOFA score of $\geq$ 1 (MAP < 70 mm Hg and/or any vasopressor required).

Exclusion Criteria
known pregnancy;
institutionalized individuals;
previous renal transplantation;
known acutely worsening renal function prior to enrollment (e.g., any category of RIFLE criteria);
received dialysis (either acute or chronic) within 5 days prior to enrollment or in imminent need of dialysis at the time of enrollment;
known infection with human immunodeficiency virus (HIV) or a hepatitis virus;
meets any of the following:

(i) active bleeding with an anticipated need for > 4 units PRBC in a day;

(ii) hemoglobin < 7 g/dL;

(iii) any other condition that in the physician's opinion would contraindicate drawing serial blood samples for clinical study purposes;

meets only the SBP < 90 mmHg inclusion criterion set forth above, and does not have shock in the attending physician's or principal investigator's opinion;

After obtaining informed consent, an EDTA anti-coagulated blood sample (10 mL) and a urine sample (25-50 mL) are collected from each patient. Blood and urine samples are then collected at 4 ($\pm$ 0.5) and 8 ($\pm$ 1) hours after contrast administration (if applicable); at 12 ($\pm$ 1), 24 ($\pm$ 2), 36 ($\pm$ 2), 48 ($\pm$ 2), 60 ($\pm$ 2), 72 ($\pm$ 2), and 84 ($\pm$ 2) hours after enrollment, and thereafter daily up to day 7 to day 14 while the subject is hospitalized. Blood is collected via direct venipuncture or via other available venous access, such as an existing femoral sheath, central venous line, peripheral intravenous line or hep-lock. These study blood samples are processed to plasma at the clinical site, frozen and shipped to Astute Medical, Inc., San Diego, CA. The study urine samples are frozen and shipped to Astute Medical, Inc.

Example 4. Immunoassay format

Analytes are measured using standard sandwich enzyme immunoassay techniques. A first antibody which binds the analyte is immobilized in wells of a 96 well polystyrene microplate. Analyte standards and test samples are pipetted into the appropriate wells and any analyte present is bound by the immobilized antibody. After washing away any unbound substances, a horseradish peroxidase-conjugated second antibody which binds the analyte is added to the wells, thereby forming sandwich complexes with the analyte (if present) and the first antibody. Following a wash to remove any unbound antibody-enzyme reagent, a substrate solution comprising tetramethylbenzidine and hydrogen peroxide is added to the wells. Color develops in proportion to the amount of analyte present in the sample. The color development is stopped and the intensity of the color is measured at 540 nm or 570 nm. An analyte concentration is assigned to the test sample by comparison to a standard curve determined from the analyte standards.

Units for the concentrations reported in the following data tables are as follows: Cancer antigen CA 15-3 - U/mL, C-C Motif chemokine 18 - ng/mL, C-C Motif chemokine 24 - pg/mL, Cathepsin D - pg/mL, C-X-C Motif chemokine 13 - pg/mL, C-C motif chemokine 8 - pg/mL, Interleukin-2 receptor alpha chain - pg/mL, Insulin-like growth factor-binding protein 3 - ng/mL, Interleukin-11 - pg/mL, Matrix Metalloproteinase-8 - pg/mL, Transforming growth factor alpha - pg/mL, IgG1 - ng/mL, and IgG2 - ng/mL. In the case of those kidney injury markers which are membrane proteins as described herein, the assays used in these examples detect soluble forms thereof.

Commercially-available reagents were sourced from the following vendors:

| Analyte | Assay Source | Catalog number |
|---|---|---|
| CA 15-3 | EMD Chemicals | Cat. # BPHCP001-6 |
| Cathepsin D | Millipore | Cat. #HNDG3-36K |
| C-C motif chemokine 18 | Millipore | Cat. #HNDG2-36K |
| C-C motif chemokine 24 | Millipore | Cat. # MPXHCYP2-62K |
| C-C motif chemokine 8 | Millipore | Cat. # MPXHCYP2-62K |
| C-X-C motif chemokine 13 | Millipore | Cat. # MPXHCYP2-62K |
| Immunoglobulin G, subclass 1 | Millipore | Cat. # HGAM-301 |
| Immunoglobulin G, subclass 2 | Millipore | Cat. # HGAM-301 |
| Insulin-like growth factor-binding protein 3 | Millipore | Cat. # HIGFBP-53K |
| Interleukin-11 | Millipore | Cat. # MPXHCYP3-63K |
| Interleukin-2 receptor alpha chain | Millipore | Cat. # MPXHCYTO-60K |
| Matrix Metalloproteinase-8 | R&D Systems | Cat. #LMP000, Cat. #LMP908 |
| Transforming growth factor alpha | Millipore | Cat. # MPXHCYTO-60K |

Example 5. Apparently Healthy Donor and Chronic Disease Patient Samples

Human urine samples from donors with no known chronic or acute disease ("Apparently Healthy Donors") were purchased from two vendors (Golden West Biologicals, Inc., 27625 Commerce Center Dr., Temecula, CA 92590 and Virginia Medical Research, Inc., 915 First Colonial Rd., Virginia Beach, VA 23454). The urine samples were shipped and stored frozen at less than -20° C. The vendors supplied demographic information for the individual donors including gender, race (Black /White), smoking status and age.

Human urine samples from donors with various chronic diseases ("Chronic Disease Patients") including congestive heart failure, coronary artery disease, chronic kidney disease, chronic obstructive pulmonary disease, diabetes mellitus and hypertension were purchased from Virginia Medical Research, Inc., 915 First Colonial Rd., Virginia Beach, VA 23454. The urine samples were shipped and stored frozen at less than -20 degrees centigrade. The vendor provided a case report form for each individual donor with age, gender, race (Black/White), smoking status and alcohol use, height, weight, chronic disease(s) diagnosis, current medications and previous surgeries.

Example 6. Use of Kidney Injury Markers for evaluating renal status in patients

Patients from the intensive care unit (ICU) were enrolled in the following study. Each patient was classified by kidney status as non-injury (0), risk of injury (R), injury (I), and failure (F) according to the maximum stage reached within 7 days of enrollment as determined by the RIFLE criteria. EDTA anti-coagulated blood samples (10 mL) and a urine samples (25-30 mL) were collected from each patient at enrollment, 4 ($\pm$ 0.5) and 8 ($\pm$ 1) hours after contrast administration (if applicable); at 12 ($\pm$ 1), 24 ($\pm$ 2), and 48 ($\pm$ 2) hours after enrollment, and thereafter daily up to day 7 to day 14 while the subject is hospitalized. Markers were each measured by standard immunoassay methods using commercially available assay reagents in the urine samples and the plasma component of the blood samples collected.

Two cohorts were defined to represent a "diseased" and a "normal" population. While these terms are used for convenience, "diseased" and "normal" simply represent two cohorts for comparison (say RIFLE 0 vs RIFLE R, I and F; RIFLE 0 vs RIFLE R; RIFLE 0 and R vs RIFLE I and F; etc.). The time "prior max stage" represents the time at which a sample is collected, relative to the time a particular patient reaches the lowest disease stage as defined for that cohort, binned into three groups which are +/- 12 hours. For example, "24 hr prior" which uses 0 vs R, I, F as the two cohorts would mean 24 hr (+/- 12 hours) prior to reaching stage R (or I if no sample at R, or F if no sample at R or I).

A receiver operating characteristic (ROC) curve was generated for each biomarker measured and the area under each ROC curve (AUC) is determined. Patients in Cohort 2 were also separated according to the reason for adjudication to cohort 2 as being based on serum creatinine measurements (sCr), being based on urine output (UO), or being based on either serum creatinine measurements or urine output. Using the same example discussed above (0 vs R, I, F), for those patients adjudicated to stage R, I, or F on the basis of serum creatinine measurements alone, the stage 0 cohort may include patients adjudicated to stage R, I, or F on the basis of urine output; for those patients adjudicated to stage R, I, or F on the basis of urine output alone, the stage 0 cohort may include patients adjudicated to stage R, I, or F on the basis of serum creatinine measurements; and for those patients adjudicated to stage R, I, or F on the basis of serum creatinine measurements or urine output, the stage 0 cohort contains only patients in stage 0 for both serum creatinine measurements and urine output. Also, in the data for patients adjudicated on the basis of serum creatinine measurements or urine output, the adjudication method which yielded the most severe RIFLE stage is used.

The ability to distinguish cohort 1 from Cohort 2 was determined using ROC analysis. SE is the standard error of the AUC, n is the number of sample or individual patients ("pts," as indicated). Standard errors are calculated as described in Hanley, J. A., and McNeil, B.J., The meaning and use of the area under a receiver operating characteristic (ROC) curve. Radiology (1982) 143: 29-36; p values are calculated with a two-tailed Z-test. An AUC < 0.5 is indicative of a negative going marker for the comparison, and an AUC > 0.5 is indicative of a positive going marker for the comparison.

Various threshold (or "cutoff') concentrations were selected, and the associated sensitivity and specificity for distinguishing cohort 1 from cohort 2 are determined. OR is the odds ratio calculated for the particular cutoff concentration, and 95% CI is the confidence interval for the odds ratio.

Table 1: Comparison of marker levels in urine samples collected from Cohort 1 (patients that did not progress beyond RIFLE stage 0) and in urine samples collected from subjects at 0, 24 hours, and 48 hours prior to reaching stage R, I or F in Cohort 2.

**c-C motif chemokine 18**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.336 | 0.757 | 0.336 | 0.545 | 0.336 | 0.551 |
| Average | 2.29 | 3.44 | 2.29 | 2.98 | 2.29 | 2.60 |
| Stdev | 7.31 | 7.76 | 7.31 | 7.93 | 7.31 | 8.13 |
| p(t-test) | | 0.13 | | 0.35 | | 0.78 |
| Min | 3.13E-5 | 0.000952 | 3.13E-5 | 0.00194 | 3.13E-5 | 0.0160 |
| Max | 40.0 | 40.0 | 40.0 | 40.0 | 40.0 | 40.0 |
| n (Samp) | 463 | 119 | 463 | 128 | 463 | 47 |
| n (Patient) | 223 | 119 | 223 | 128 | 223 | 47 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 0.399 | 1.05 | 0.399 | 0.966 | 0.399 | 0.486 |
| Average | 2.26 | 5.09 | 2.26 | 2.77 | 2.26 | 3.91 |
| Stdev | 6.86 | 9.91 | 6.86 | 5.93 | 6.86 | 10.7 |
| p(t-test) | | 0.012 | | 0.62 | | 0.24 |
| Min | 3.13E-5 | 0.000952 | 3.13E-5 | 0.00497 | 3.13E-5 | 0.0172 |
| Max | 40.0 | 40.0 | 40.0 | 37.8 | 40.0 | 40.0 |
| n (Samp) | 1015 | 40 | 1015 | 46 | 1015 | 26 |
| n (Patient) | 374 | 40 | 374 | 46 | 374 | 26 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 0.377 | 0.996 | 0.377 | 0.708 | 0.377 | 0.701 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 2.54 | 4.28 | 2.54 | 4.16 | 2.54 | 3.83 |
| Stdev | 7.63 | 9.11 | 7.63 | 9.73 | 7.63 | 9.66 |
| p(t-test) | | 0.042 | | 0.054 | | 0.30 |
| Min | 3.13E-5 | 0.0153 | 3.13E-5 | 0.00194 | 3.13E-5 | 0.0160 |
| Max | 40.0 | 40.0 | 40.0 | 40.0 | 40.0 | 40.0 |
| n (Samp) | 436 | 107 | 436 | 117 | 436 | 44 |
| n (Patient) | 173 | 107 | 173 | 117 | 173 | 44 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.64 | 0.66 | 0.65 | 0.59 | 0.60 | 0.60 | 0.55 | 0.53 | 0.57 |
| SE | 0.030 | 0.048 | 0.031 | 0.029 | 0.045 | 0.030 | 0.045 | 0.058 | 0.047 |
| p | 1.1E-6 | 6.4E-4 | 1.9E-6 | 0.0024 | 0.024 | 0.0012 | 0.28 | 0.66 | 0.12 |
| nCohort 1 | 463 | 1015 | 436 | 463 | 1015 | 436 | 463 | 1015 | 436 |
| nCohort 2 | 119 | 40 | 107 | 128 | 46 | 117 | 47 | 26 | 44 |
| Cutoff 1 | 0.369 | 0.674 | 0.403 | 0.228 | 0.249 | 0.369 | 0.244 | 0.225 | 0.315 |
| Sens 1 | 71% | 70% | 70% | 70% | 72% | 70% | 70% | 73% | 70% |
| Spec 1 | 52% | 63% | 52% | 36% | 35% | 49% | 38% | 32% | 45% |
| Cutoff 2 | 0.223 | 0.315 | 0.258 | 0.142 | 0.150 | 0.177 | 0.134 | 0.129 | 0.162 |
| Sens 2 | 81% | 80% | 80% | 80% | 80% | 80% | 81% | 81% | 82% |
| Spec 2 | 35% | 44% | 36% | 26% | 24% | 27% | 25% | 20% | 25% |
| Cutoff 3 | 0.111 | 0.138 | 0.138 | 0.0629 | 0.0568 | 0.0751 | 0.0403 | 0.0403 | 0.124 |
| Sens 3 | 91% | 90% | 91% | 91% | 91% | 91% | 91% | 92% | 91% |
| Spec 3 | 21% | 21% | 21% | 12% | 8% | 13% | 9% | 7% | 20% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 4 | 0.751 | 0.893 | 0.906 | 0.751 | 0.893 | 0.906 | 0.751 | 0.893 | 0.906 |
| Sens 4 | 50% | 62% | 50% | 45% | 52% | 46% | 40% | 35% | 36% |
| Spec 4 | 70% | 70% | 70% | 70% | 70% | 70% | 70% | 70% | 70% |
| Cutoff 5 | 1.12 | 1.51 | 1.63 | 1.12 | 1.51 | 1.63 | 1.12 | 1.51 | 1.63 |
| Sens 5 | 40% | 45% | 37% | 37% | 39% | 32% | 21% | 27% | 20% |
| Spec 5 | 80% | 80% | 80% | 80% | 80% | 80% | 80% | 80% | 80% |
| Cutoff 6 | 3.17 | 3.53 | 3.59 | 3.17 | 3.53 | 3.59 | 3.17 | 3.53 | 3.59 |
| Sens 6 | 24% | 22% | 26% | 16% | 20% | 16% | 9% | 12% | 11% |
| Spec 6 | 90% | 90% | 90% | 90% | 90% | 90% | 90% | 90% | 90% |
| OR Quart 2 | 1.3 | 1.2 | 1.6 | 0.73 | 0.39 | 0.75 | 0.78 | 0.83 | 0.65 |
| p Value | 0.40 | 0.77 | 0.22 | 0.32 | 0.12 | 0.40 | 0.61 | 0.76 | 0.43 |
| 95% CI of | 0.68 | 0.36 | 0.77 | 0.39 | 0.12 | 0.38 | 0.30 | 0.25 | 0.22 |
| OR Quart2 | 2.6 | 4.0 | 3.3 | 1.4 | 1.3 | 1.5 | 2.0 | 2.8 | 1.9 |
| OR Quart 3 | 2.0 | 2.0 | 2.2 | 1.3 | 1.2 | 1.6 | 1.7 | 1.3 | 2.0 |
| p Value | 0.029 | 0.20 | 0.022 | 0.32 | 0.66 | 0.14 | 0.22 | 0.59 | 0.10 |
| 95% CI of | 1.1 | 0.68 | 1.1 | 0.75 | 0.51 | 0.87 | 0.73 | 0.46 | 0.87 |
| OR Quart3 | 3.9 | 6.0 | 4.5 | 2.4 | 2.8 | 2.8 | 3.9 | 3.9 | 4.8 |
| OR Quart 4 | 3.8 | 4.0 | 4.1 | 2.3 | 2.1 | 2.2 | 1.3 | 1.2 | 1.4 |
| p Value | 1.9E-5 | 0.0066 | 2.5E-5 | 0.0027 | 0.067 | 0.0059 | 0.53 | 0.78 | 0.49 |
| 95% CI of | 2.1 | 1.5 | 2.1 | 1.3 | 0.95 | 1.3 | 0.56 | 0.39 | 0.55 |
| OR Quart4 | 7.0 | 11 | 8.0 | 4.0 | 4.5 | 4.0 | 3.1 | 3.5 | 3.4 |

EP 3 489 688 A1

(continued)

| C-C motif chemokine 24 | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 8.31 | 20.6 | 8.31 | 15.9 | 8.31 | 9.14 |
| Average | 19.2 | 46.8 | 19.2 | 57.8 | 19.2 | 18.4 |
| Stdev | 49.6 | 108 | 49.6 | 237 | 49.6 | 34.6 |
| p(t-test) | | 4.9E-5 | | 0.0012 | | 0.91 |
| Min | 0.0120 | 0.0160 | 0.0120 | 0.0120 | 0.0120 | 0.0193 |
| Max | 730 | 1010 | 730 | 2380 | 730 | 216 |
| n (Samp) | 463 | 120 | 463 | 130 | 463 | 47 |
| n (Patient) | 223 | 120 | 223 | 130 | 223 | 47 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 11.9 | 28.7 | 11.9 | 20.3 | 11.9 | 13.7 |
| Average | 31.8 | 46.7 | 31.8 | 39.1 | 31.8 | 31.2 |
| Stdev | 113 | 62.4 | 113 | 63.5 | 113 | 54.6 |
| p(t-test) | | 0.41 | | 0.66 | | 0.98 |
| Min | 0.0120 | 0.0160 | 0.0120 | 0.0196 | 0.0120 | 0.0215 |
| Max | 2380 | 290 | 2380 | 347 | 2380 | 216 |
| n (Samp) | 1019 | 40 | 1019 | 46 | 1019 | 26 |
| n (Patient) | 375 | 40 | 375 | 46 | 375 | 26 |
| UO only | 0hr prior to AKI stage | | 24hr prior to AKI stage | | 24hr prior to AKI stage | |
| | Cohort 1 | Cohort 2 | Cohort 1 | Cohort 2 | Cohort 1 | Cohort 2 |
| Median | 8.53 | 25.8 | 8.53 | 18.2 | 8.53 | 13.1 |
| Average | 21.8 | 85.0 | 21.8 | 86.7 | 21.8 | 49.4 |

| UO only | 0hr prior to AKI stage | | 24hr prior to AKI stage | | 24hr prior to AKI stage | |
|---|---|---|---|---|---|---|
| | Cohort 1 | Cohort 2 | Cohort 1 | Cohort 2 | Cohort 1 | Cohort 2 |
| Stdev | 53.7 | 298 | 53.7 | 309 | 53.7 | 182 |
| p(t-test) | | 3.6E-5 | | 3.6E-5 | | 0.020 |
| Min | 0.0120 | 0.0160 | 0.0120 | 0.0120 | 0.0120 | 0.0160 |
| Max | 730 | 2790 | 730 | 2380 | 730 | 1170 |
| n (Samp) | 435 | 108 | 435 | 119 | 435 | 44 |
| n (Patient) | 173 | 108 | 173 | 119 | 173 | 44 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.66 | 0.62 | 0.69 | 0.63 | 0.59 | 0.65 | 0.52 | 0.50 | 0.56 |
| SE | 0.029 | 0.048 | 0.030 | 0.029 | 0.045 | 0.030 | 0.045 | 0.058 | 0.047 |
| p | 2.7E-8 | 0.0095 | 1.5E-10 | 7.5E-6 | 0.035 | 1.1E-6 | 0.66 | 0.94 | 0.22 |
| nCohort 1 | 463 | 1019 | 435 | 463 | 1019 | 435 | 463 | 1019 | 435 |
| nCohort 2 | 120 | 40 | 108 | 130 | 46 | 119 | 47 | 26 | 44 |
| Cutoff 1 | 8.53 | 10.4 | 11.5 | 7.45 | 9.88 | 8.20 | 3.27 | 1.48 | 5.35 |
| Sens 1 | 70% | 70% | 70% | 70% | 72% | 71% | 70% | 73% | 70% |
| Spec 1 | 51% | 47% | 59% | 48% | 46% | 49% | 35% | 22% | 42% |
| Cutoff 2 | 4.36 | 0.405 | 6.04 | 3.71 | 3.71 | 4.18 | 0.405 | 0.0635 | 1.03 |
| Sens 2 | 80% | 80% | 81% | 80% | 80% | 81% | 81% | 81% | 82% |
| Spec 2 | 38% | 20% | 43% | 36% | 28% | 38% | 28% | 18% | 28% |
| Cutoff 3 | 0.0469 | 0.0344 | 0.0562 | 0.0552 | 0.0469 | 0.0562 | 0.0235 | 0.0277 | 0.0263 |
| Sens 3 | 91% | 90% | 91% | 90% | 91% | 91% | 94% | 92% | 91% |
| Spec 3 | 19% | 11% | 23% | 22% | 13% | 23% | 8% | 7% | 7% |
| Cutoff 4 | 16.0 | 23.8 | 16.7 | 16.0 | 23.8 | 16.7 | 16.0 | 23.8 | 16.7 |

EP 3 489 688 A1

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 4 | 57% | 57% | 61% | 50% | 46% | 50% | 36% | 38% | 41% |
| Spec 4 | 70% | 70% | 70% | 70% | 70% | 70% | 70% | 70% | 70% |
| Cutoff 5 | 25.6 | 36.4 | 25.7 | 25.6 | 36.4 | 25.7 | 25.6 | 36.4 | 25.7 |
| Sens 5 | 46% | 40% | 50% | 35% | 28% | 39% | 15% | 23% | 20% |
| Spec 5 | 80% | 80% | 80% | 80% | 80% | 80% | 80% | 80% | 80% |
| Cutoff 6 | 40.7 | 62.3 | 48.2 | 40.7 | 62.3 | 48.2 | 40.7 | 62.3 | 48.2 |
| Sens 6 | 30% | 20% | 28% | 22% | 15% | 22% | 9% | 12% | 9% |
| Spec 6 | 90% | 90% | 90% | 90% | 90% | 90% | 90% | 90% | 90% |
| OR Quart 2 | 1.2 | 0.54 | 1.8 | 2.0 | 0.87 | 2.2 | 2.0 | 0.55 | 1.6 |
| p Value | 0.62 | 0.28 | 0.14 | 0.041 | 0.79 | 0.023 | 0.14 | 0.29 | 0.34 |
| 95% CI of | 0.60 | 0.18 | 0.83 | 1.0 | 0.31 | 1.1 | 0.81 | 0.18 | 0.60 |
| OR Quart2 | 2.3 | 1.6 | 3.8 | 3.7 | 2.4 | 4.5 | 4.8 | 1.7 | 4.3 |
| OR Quart 3 | 1.5 | 0.77 | 2.2 | 2.2 | 1.8 | 2.6 | 1.4 | 0.33 | 2.1 |
| p Value | 0.20 | 0.61 | 0.037 | 0.015 | 0.20 | 0.0068 | 0.48 | 0.096 | 0.12 |
| 95% CI of | 0.80 | 0.28 | 1.0 | 1.2 | 0.74 | 1.3 | 0.55 | 0.087 | 0.82 |
| OR Quart3 | 2.9 | 2.1 | 4.6 | 4.2 | 4.3 | 5.1 | 3.6 | 1.2 | 5.4 |
| OR Quart 4 | 4.3 | 2.2 | 6.3 | 3.9 | 2.2 | 4.4 | 1.7 | 1.0 | 1.8 |
| p Value | 1.9E-6 | 0.059 | 1.3E-7 | 1.2E-5 | 0.073 | 1.0E-5 | 0.27 | 0.99 | 0.24 |
| 95% CI of | 2.3 | 0.97 | 3.2 | 2.1 | 0.93 | 2.3 | 0.67 | 0.39 | 0.67 |
| OR Quart4 | 7.7 | 4.9 | 13 | 7.2 | 5.2 | 8.4 | 4.2 | 2.6 | 4.7 |
| **C-C motif chemokine 8** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 1.21 | | 1.72 | 1.21 | | 2.97 | 1.21 | | 2.77 |

| C-C motif chemokine 8 | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Average | 10.1 | 9.75 | 10.1 | 13.6 | 10.1 | 10.8 |
| Stdev | 24.9 | 21.6 | 24.9 | 33.2 | 24.9 | 34.3 |
| p(t-test) | | 0.88 | | 0.19 | | 0.86 |
| Min | 0.0250 | 0.0635 | 0.0250 | 0.0250 | 0.0250 | 0.0250 |
| Max | 250 | 158 | 250 | 294 | 250 | 213 |
| n (Samp) | 463 | 120 | 463 | 130 | 463 | 47 |
| n (Patient) | 223 | 120 | 223 | 130 | 223 | 47 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 1.36 | 2.23 | 1.36 | 6.03 | 1.36 | 2.14 |
| Average | 10.6 | 16.4 | 10.6 | 20.7 | 10.6 | 18.6 |
| Stdev | 32.3 | 36.8 | 32.3 | 46.5 | 32.3 | 45.7 |
| p(t-test) | | 0.27 | | 0.042 | | 0.22 |
| Min | 0.0250 | 0.0635 | 0.0250 | 0.0250 | 0.0250 | 0.0250 |
| Max | 492 | 185 | 492 | 279 | 492 | 213 |
| n (Samp) | 1019 | 40 | 1019 | 46 | 1019 | 26 |
| n (Patient) | 375 | 40 | 375 | 46 | 375 | 26 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 1.21 | 2.59 | 1.21 | 2.77 | 1.21 | 2.16 |
| Average | 10.6 | 12.3 | 10.6 | 16.1 | 10.6 | 12.5 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | 26.0 | 27.1 | 26.0 | 36.2 | 26.0 | 37.4 |
| p(t-test) | | 0.54 | | 0.066 | | 0.67 |
| Min | 0.0250 | 0.0701 | 0.0250 | 0.0250 | 0.0250 | 0.0472 |
| Max | 250 | 176 | 250 | 294 | 250 | 214 |
| n (Samp) | 435 | 108 | 435 | 119 | 435 | 44 |
| n (Patient) | 173 | 108 | 173 | 119 | 173 | 44 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.57 | 0.58 | 0.59 | 0.60 | 0.62 | 0.60 | 0.56 | 0.55 | 0.53 |
| SE | 0.030 | 0.048 | 0.032 | 0.029 | 0.045 | 0.030 | 0.045 | 0.059 | 0.046 |
| p | 0.017 | 0.092 | 0.0041 | 9.0E-4 | 0.0090 | 8.1E-4 | 0.21 | 0.42 | 0.47 |
| nCohort 1 | 463 | 1019 | 435 | 463 | 1019 | 435 | 463 | 1019 | 435 |
| nCohort 2 | 120 | 40 | 108 | 130 | 46 | 119 | 47 | 26 | 44 |
| Cutoff 1 | 0.453 | 0.802 | 0.802 | 0.491 | 0.802 | 0.567 | 0.802 | 0.802 | 0.491 |
| Sens 1 | 72% | 72% | 73% | 73% | 74% | 71% | 74% | 73% | 70% |
| Spec 1 | 43% | 44% | 46% | 43% | 44% | 45% | 47% | 44% | 42% |
| Cutoff 2 | 0.154 | 0.240 | 0.239 | 0.239 | 0.239 | 0.240 | 0.239 | 0.136 | 0.154 |
| Sens 2 | 82% | 85% | 81% | 80% | 80% | 81% | 81% | 81% | 82% |
| Spec 2 | 33% | 35% | 33% | 36% | 32% | 36% | 36% | 25% | 29% |
| Cutoff 3 | 0.0945 | 0.127 | 0.127 | 0.0775 | 0.0775 | 0.127 | 0.0569 | 0.0705 | 0.0701 |
| Sens 3 | 90% | 90% | 91% | 92% | 93% | 91% | 94% | 92% | 91% |
| Spec 3 | 22% | 22% | 23% | 18% | 14% | 23% | 10% | 12% | 12% |
| Cutoff 4 | 5.70 | 6.40 | 5.77 | 5.70 | 6.40 | 5.77 | 5.70 | 6.40 | 5.77 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 4 | 33% | 38% | 39% | 41% | 50% | 41% | 23% | 35% | 20% |
| Spec 4 | 70% | 70% | 70% | 70% | 70% | 70% | 70% | 70% | 70% |
| Cutoff 5 | 11.1 | 11.3 | 11.7 | 11.1 | 11.3 | 11.7 | 11.1 | 11.3 | 11.7 |
| Sens 5 | 19% | 22% | 24% | 25% | 35% | 24% | 13% | 19% | 14% |
| Spec 5 | 80% | 80% | 80% | 80% | 80% | 80% | 80% | 80% | 80% |
| Cutoff 6 | 28.3 | 24.5 | 25.3 | 28.3 | 24.5 | 25.3 | 28.3 | 24.5 | 25.3 |
| Sens 6 | 9% | 12% | 15% | 12% | 20% | 16% | 4% | 12% | 7% |
| Spec 6 | 90% | 90% | 90% | 90% | 90% | 90% | 90% | 90% | 90% |
| OR Quart 2 | 2.4 | 2.5 | 1.8 | 1.8 | 0.87 | 1.8 | 1.1 | 1.0 | 1.3 |
| p Value | 0.0066 | 0.096 | 0.074 | 0.061 | 0.79 | 0.075 | 0.80 | 1.0 | 0.62 |
| 95% CI of | 1.3 | 0.85 | 0.94 | 0.97 | 0.31 | 0.94 | 0.40 | 0.32 | 0.47 |
| OR Quart2 | 4.7 | 7.1 | 3.6 | 3.5 | 2.4 | 3.6 | 3.3 | 3.1 | 3.6 |
| OR Quart 3 | 3.4 | 2.5 | 2.7 | 3.0 | 1.5 | 3.1 | 4.0 | 1.3 | 3.2 |
| p Value | 1.6E-4 | 0.096 | 0.0028 | 4.7E-4 | 0.37 | 4.9E-4 | 0.0021 | 0.59 | 0.011 |
| 95% CI of | 1.8 | 0.85 | 1.4 | 1.6 | 0.61 | 1.6 | 1.7 | 0.46 | 1.3 |
| OR Quart3 | 6.3 | 7.1 | 5.1 | 5.4 | 3.8 | 5.9 | 9.7 | 3.9 | 7.9 |
| OR Quart 4 | 1.8 | 2.2 | 2.0 | 2.6 | 2.5 | 2.7 | 1.1 | 1.00 | 1.1 |
| p Value | 0.076 | 0.14 | 0.038 | 0.0027 | 0.036 | 0.0029 | 0.80 | 0.99 | 0.80 |
| 95% CI of | 0.94 | 0.77 | 1.0 | 1.4 | 1.1 | 1.4 | 0.40 | 0.32 | 0.40 |
| OR Quart4 | 3.6 | 6.5 | 3.9 | 4.7 | 5.7 | 5.1 | 3.3 | 3.1 | 3.3 |
| **Cathepsin D** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 65300 | | 94300 | 65300 | | 87400 | 65300 | | 69000 |

EP 3 489 688 A1

| Cathepsin D | | | | | | |
|---|---|---|---|---|---|---|
| sCr or UO | 0hr prior to AKI stage | | 24hr prior to AKI stage | | 48hr prior to AKI stage | |
| Average | 69400 | 98700 | 69400 | 88000 | 69400 | 77300 |
| Stdev | 35600 | 44500 | 35600 | 43900 | 35600 | 42200 |
| p(t-test) | | 1.3E-13 | | 9.3E-7 | | 0.16 |
| Min | 656 | 2920 | 656 | 3320 | 656 | 4810 |
| Max | 200000 | 200000 | 200000 | 200000 | 200000 | 200000 |
| n (Samp) | 463 | 119 | 463 | 128 | 463 | 47 |
| n (Patient) | 223 | 119 | 223 | 128 | 223 | 47 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 74500 | 106000 | 74500 | 85500 | 74500 | 75700 |
| Average | 79700 | 104000 | 79700 | 94500 | 79700 | 79600 |
| Stdev | 40600 | 50300 | 40600 | 50700 | 40600 | 47400 |
| p(t-test) | | 2.7E-4 | | 0.017 | | 0.99 |
| Min | 656 | 2920 | 656 | 4880 | 656 | 5800 |
| Max | 200000 | 200000 | 200000 | 200000 | 200000 | 200000 |
| n (Samp) | 1015 | 40 | 1015 | 46 | 1015 | 26 |
| n (Patient) | 374 | 40 | 374 | 46 | 374 | 26 |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 70200 | 99000 | 70200 | 88700 | 70200 | 80600 |
| Average | 75500 | 103000 | 75500 | 93900 | 75500 | 83900 |
| Stdev | 38400 | 43300 | 38400 | 42600 | 38400 | 41000 |
| p(t-test) | | 1.6E-10 | | 8.5E-6 | | 0.17 |

EP 3 489 688 A1

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Min | 2520 | 12600 | 2520 | 3320 | 2520 | 4810 |
| Max | 200000 | 200000 | 200000 | 200000 | 200000 | 200000 |
| n (Samp) | 436 | 107 | 436 | 117 | 436 | 44 |
| n (Patient) | 173 | 107 | 173 | 117 | 173 | 44 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.70 | 0.65 | 0.69 | 0.63 | 0.59 | 0.63 | 0.55 | 0.48 | 0.57 |
| SE | 0.029 | 0.048 | 0.031 | 0.029 | 0.045 | 0.030 | 0.045 | 0.058 | 0.047 |
| p | 1.1E-11 | 0.0015 | 6.3E-10 | 6.6E-6 | 0.037 | 8.8E-6 | 0.23 | 0.78 | 0.14 |
| nCohort 1 | 463 | 1015 | 436 | 463 | 1015 | 436 | 463 | 1015 | 436 |
| nCohort 2 | 119 | 40 | 107 | 128 | 46 | 117 | 47 | 26 | 44 |
| Cutoff 1 | 72900 | 81200 | 74300 | 65000 | 70400 | 70900 | 56000 | 46800 | 61900 |
| Sens 1 | 71% | 70% | 70% | 70% | 72% | 70% | 70% | 73% | 70% |
| Spec 1 | 58% | 57% | 55% | 50% | 46% | 52% | 38% | 21% | 41% |
| Cutoff 2 | 62500 | 63200 | 69000 | 52000 | 58400 | 58800 | 45500 | 42000 | 54100 |
| Sens 2 | 81% | 80% | 80% | 80% | 80% | 80% | 81% | 81% | 82% |
| Spec 2 | 46% | 37% | 48% | 32% | 32% | 38% | 25% | 18% | 31% |
| Cutoff 3 | 43600 | 43100 | 54100 | 26100 | 19700 | 41500 | 22000 | 22000 | 39600 |
| Sens 3 | 91% | 90% | 91% | 91% | 91% | 91% | 91% | 92% | 91% |
| Spec 3 | 24% | 18% | 31% | 11% | 5% | 18% | 8% | 6% | 16% |
| Cutoff 4 | 82600 | 98000 | 90300 | 82600 | 98000 | 90300 | 82600 | 98000 | 90300 |
| Sens 4 | 60% | 52% | 55% | 55% | 41% | 48% | 40% | 31% | 41% |
| Spec 4 | 70% | 70% | 70% | 70% | 70% | 70% | 70% | 70% | 70% |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Cutoff 5 | 99400 | 112000 | 108000 | 99400 | 112000 | 108000 | 99400 | 112000 | 108000 |
| Sens 5 | 48% | 40% | 43% | 36% | 37% | 37% | 23% | 15% | 20% |
| Spec 5 | 80% | 80% | 80% | 80% | 80% | 80% | 80% | 80% | 80% |
| Cutoff 6 | 121000 | 135000 | 126000 | 121000 | 135000 | 126000 | 121000 | 135000 | 126000 |
| Sens 6 | 29% | 25% | 27% | 21% | 20% | 22% | 13% | 8% | 18% |
| Spec 6 | 90% | 90% | 90% | 90% | 90% | 90% | 90% | 90% | 90% |
| OR Quart 2 | 1.1 | 0.56 | 2.3 | 1.0 | 1.0 | 1.2 | 0.99 | 0.62 | 2.0 |
| p Value | 0.72 | 0.36 | 0.042 | 0.89 | 1.0 | 0.60 | 0.99 | 0.41 | 0.17 |
| 95% CI of | 0.55 | 0.16 | 1.0 | 0.55 | 0.37 | 0.61 | 0.40 | 0.20 | 0.75 |
| OR Quart2 | 2.4 | 1.9 | 5.1 | 2.0 | 2.7 | 2.4 | 2.5 | 1.9 | 5.1 |
| OR Quart 3 | 2.4 | 1.3 | 3.4 | 1.9 | 1.7 | 2.0 | 1.2 | 0.62 | 1.6 |
| p Value | 0.012 | 0.62 | 0.0017 | 0.042 | 0.27 | 0.031 | 0.66 | 0.41 | 0.33 |
| 95% CI of | 1.2 | 0.47 | 1.6 | 1.0 | 0.68 | 1.1 | 0.51 | 0.20 | 0.61 |
| OR Quart3 | 4.6 | 3.5 | 7.3 | 3.4 | 4.1 | 3.8 | 2.9 | 1.9 | 4.4 |
| OR Quart 4 | 5.4 | 3.0 | 6.6 | 3.1 | 2.2 | 3.3 | 1.6 | 1.0 | 2.0 |
| p Value | 1.6E-7 | 0.014 | 4.8E-7 | 1.3E-4 | 0.073 | 1.2E-4 | 0.30 | 0.99 | 0.17 |
| 95% CI of | 2.9 | 1.2 | 3.2 | 1.7 | 0.93 | 1.8 | 0.67 | 0.37 | 0.75 |
| OR Quart4 | 10 | 7.2 | 14 | 5.4 | 5.2 | 6.1 | 3.6 | 2.7 | 5.1 |

**C-X-C motif chemokine 13**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.0163 | 1.10 | 0.0163 | 0.590 | 0.0163 | 0.448 |
| Average | 8.44 | 7.15 | 8.44 | 7.71 | 8.44 | 6.34 |
| Stdev | 53.2 | 23.6 | 53.2 | 21.5 | 53.2 | 16.8 |

EP 3 489 688 A1

**C-X-C motif chemokine 13**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.80 | | 0.88 | | 0.79 |
| Min | 0.00269 | 0.00269 | 0.00269 | 0.00269 | 0.00269 | 0.00269 |
| Max | 832 | 210 | 832 | 131 | 832 | 75.7 |
| n (Samp) | 463 | 120 | 463 | 130 | 463 | 47 |
| n (Patient) | 223 | 120 | 223 | 130 | 223 | 47 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 0.0222 | 1.41 | 0.0222 | 0.880 | 0.0222 | 0.520 |
| Average | 7.21 | 16.4 | 7.21 | 8.42 | 7.21 | 6.73 |
| Stdev | 40.5 | 51.0 | 40.5 | 20.0 | 40.5 | 17.0 |
| p(t-test) | | 0.16 | | 0.84 | | 0.95 |
| Min | 0.00269 | 0.00471 | 0.00269 | 0.00269 | 0.00269 | 0.00269 |
| Max | 832 | 252 | 832 | 79.1 | 832 | 77.1 |
| n (Samp) | 1019 | 40 | 1019 | 46 | 1019 | 26 |
| n (Patient) | 375 | 40 | 375 | 46 | 375 | 26 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 0.0163 | 1.68 | 0.0163 | 0.772 | 0.0163 | 0.644 |
| Average | 8.43 | 27.2 | 8.43 | 26.4 | 8.43 | 16.1 |
| Stdev | 54.5 | 187 | 54.5 | 171 | 54.5 | 67.5 |
| p(t-test) | | 0.072 | | 0.062 | | 0.39 |

EP 3 489 688 A1

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Min | 0.00269 | 0.00269 | 0.00269 | 0.00269 | 0.00269 | 0.00474 |
| Max | 832 | 1930 | 832 | 1850 | 832 | 440 |
| n (Samp) | 435 | 108 | 435 | 119 | 435 | 44 |
| n (Patient) | 173 | 108 | 173 | 119 | 173 | 44 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.64 | 0.63 | 0.68 | 0.58 | 0.58 | 0.61 | 0.57 | 0.54 | 0.61 |
| SE | 0.030 | 0.048 | 0.031 | 0.029 | 0.045 | 0.030 | 0.045 | 0.059 | 0.047 |
| p | 2.9E-6 | 0.0070 | 6.2E-9 | 0.0096 | 0.075 | 2.8E-4 | 0.14 | 0.50 | 0.020 |
| nCohort 1 | 463 | 1019 | 435 | 463 | 1019 | 435 | 463 | 1019 | 435 |
| nCohort 2 | 120 | 40 | 108 | 130 | 46 | 119 | 47 | 26 | 44 |
| Cutoff 1 | 0.0222 | 0.0193 | 0.392 | 0.0126 | 0.0126 | 0.0155 | 0.0151 | 0.0130 | 0.0174 |
| Sens 1 | 70% | 72% | 70% | 72% | 72% | 72% | 70% | 73% | 70% |
| Spec 1 | 55% | 47% | 62% | 37% | 32% | 48% | 44% | 34% | 50% |
| Cutoff 2 | 0.0126 | 0.0126 | 0.0193 | 0.00821 | 0.00821 | 0.00930 | 0.00821 | 0.0104 | 0.00930 |
| Sens 2 | 82% | 80% | 81% | 83% | 87% | 83% | 81% | 81% | 82% |
| Spec 2 | 37% | 32% | 51% | 26% | 21% | 27% | 26% | 27% | 27% |
| Cutoff 3 | 0.00821 | 0.0104 | 0.00821 | 0.00564 | 0.00789 | 0.00564 | 0.00471 | 0.00471 | 0.00789 |
| Sens 3 | 90% | 90% | 92% | 91% | 91% | 92% | 96% | 92% | 91% |
| Spec 3 | 26% | 27% | 22% | 17% | 18% | 14% | 10% | 8% | 20% |
| Cutoff 4 | 1.14 | 1.32 | 1.05 | 1.14 | 1.32 | 1.05 | 1.14 | 1.32 | 1.05 |
| Sens 4 | 49% | 50% | 58% | 36% | 43% | 44% | 34% | 38% | 41% |
| Spec 4 | 70% | 70% | 70% | 70% | 70% | 70% | 70% | 70% | 70% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 5 | 3.19 | 3.33 | 2.67 | 3.19 | 3.33 | 2.67 | 3.19 | 3.33 | 2.67 |
| Sens 5 | 28% | 35% | 38% | 22% | 28% | 29% | 19% | 19% | 27% |
| Spec 5 | 80% | 80% | 80% | 80% | 80% | 80% | 80% | 80% | 80% |
| Cutoff 6 | 10.2 | 10.2 | 8.59 | 10.2 | 10.2 | 8.59 | 10.2 | 10.2 | 8.59 |
| Sens 6 | 13% | 20% | 18% | 14% | 15% | 22% | 13% | 15% | 16% |
| Spec 6 | 90% | 90% | 90% | 90% | 90% | 90% | 90% | 90% | 90% |
| OR Quart 2 | 1.7 | 2.3 | 1.1 | 1.3 | 0.63 | 1.0 | 1.3 | 1.2 | 0.99 |
| p Value | 0.16 | 0.17 | 0.86 | 0.44 | 0.34 | 0.89 | 0.64 | 0.76 | 0.99 |
| 95% CI of | 0.81 | 0.69 | 0.49 | 0.69 | 0.24 | 0.54 | 0.48 | 0.36 | 0.34 |
| OR Quart2 | 3.4 | 7.5 | 2.4 | 2.3 | 1.6 | 2.0 | 3.3 | 4.0 | 2.9 |
| OR Quart 3 | 3.8 | 2.5 | 3.5 | 2.2 | 1.2 | 2.3 | 2.1 | 1.8 | 2.1 |
| p Value | 7.4E-5 | 0.12 | 3.3E-4 | 0.0084 | 0.68 | 0.0060 | 0.092 | 0.29 | 0.12 |
| 95% CI of | 2.0 | 0.79 | 1.8 | 1.2 | 0.52 | 1.3 | 0.88 | 0.60 | 0.82 |
| OR Quart3 | 7.3 | 8.2 | 7.0 | 3.8 | 2.7 | 4.2 | 5.2 | 5.5 | 5.4 |
| OR Quart 4 | 3.8 | 4.5 | 4.5 | 1.8 | 1.4 | 2.3 | 1.7 | 1.2 | 2.5 |
| p Value | 7.4E-5 | 0.0079 | 1.3E-5 | 0.048 | 0.43 | 0.0066 | 0.27 | 0.77 | 0.057 |
| 95% CI of | 2.0 | 1.5 | 2.3 | 1.0 | 0.62 | 1.3 | 0.67 | 0.36 | 0.97 |
| OR Quart4 | 7.3 | 13 | 8.8 | 3.2 | 3.1 | 4.2 | 4.2 | 4.0 | 6.2 |

**Insulin-like growth factor-binding protein 3**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 298 | 508 | 298 | 491 | 298 | 620 |
| Average | 559 | 748 | 559 | 1030 | 559 | 733 |
| Stdev | 935 | 758 | 935 | 1970 | 935 | 684 |

EP 3 489 688 A1

(continued)

| Insulin-like growth factor-binding protein 3 | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| p(t-test) | | 0.027 | | 6.9E-5 | | 0.21 |
| Min | 3.84 | 5.67 | 3.84 | 8.21 | 3.84 | 3.83 |
| Max | 12500 | 4890 | 12500 | 12500 | 12500 | 3220 |
| n (Samp) | 499 | 142 | 499 | 141 | 499 | 48 |
| n (Patient) | 235 | 142 | 235 | 141 | 235 | 48 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 388 | 432 | 388 | 376 | 388 | 423 |
| Average | 665 | 827 | 665 | 917 | 665 | 635 |
| Stdev | 1060 | 1290 | 1060 | 1400 | 1060 | 583 |
| p(t-test) | | 0.32 | | 0.10 | | 0.88 |
| Min | 0.0478 | 5.67 | 0.0478 | 14.5 | 0.0478 | 3.83 |
| Max | 12500 | 7720 | 12500 | 8160 | 12500 | 1890 |
| n (Samp) | 1097 | 46 | 1097 | 51 | 1097 | 28 |
| n (Patient) | 397 | 46 | 397 | 51 | 397 | 28 |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 311 | 599 | 311 | 590 | 311 | 699 |
| Average | 617 | 1010 | 617 | 1130 | 617 | 771 |
| Stdev | 1150 | 1400 | 1150 | 2020 | 1150 | 659 |
| p(t-test) | | 0.0012 | | 1.9E-4 | | 0.37 |
| Min | 3.84 | 21.2 | 3.84 | 8.21 | 3.84 | 10.2 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 12500 | 12500 | 12500 | 12500 | 12500 | 3220 |
| n (Samp) | 487 | 129 | 487 | 131 | 487 | 47 |
| n (Patient) | 190 | 129 | 190 | 131 | 190 | 47 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.63 | 0.52 | 0.67 | 0.60 | 0.53 | 0.63 | 0.62 | 0.52 | 0.64 |
| SE | 0.028 | 0.044 | 0.028 | 0.028 | 0.042 | 0.029 | 0.045 | 0.056 | 0.045 |
| p | 5.3E-6 | 0.58 | 1.7E-9 | 3.0E-4 | 0.43 | 3.4E-6 | 0.0095 | 0.68 | 0.0020 |
| nCohort 1 | 499 | 1097 | 487 | 499 | 1097 | 487 | 499 | 1097 | 487 |
| nCohort 2 | 142 | 46 | 129 | 141 | 51 | 131 | 48 | 28 | 47 |
| Cutoff 1 | 275 | 205 | 349 | 225 | 202 | 314 | 219 | 174 | 369 |
| Sens 1 | 70% | 72% | 71% | 70% | 71% | 70% | 71% | 71% | 70% |
| Spec 1 | 48% | 32% | 54% | 42% | 32% | 51% | 41% | 28% | 56% |
| Cutoff 2 | 191 | 136 | 257 | 116 | 163 | 176 | 145 | 110 | 191 |
| Sens 2 | 80% | 80% | 81% | 80% | 80% | 80% | 81% | 82% | 81% |
| Spec 2 | 39% | 23% | 45% | 28% | 26% | 34% | 32% | 20% | 36% |
| Cutoff 3 | 105 | 27.5 | 141 | 46.2 | 43.4 | 80.9 | 51.9 | 43.4 | 77.9 |
| Sens 3 | 90% | 91% | 91% | 90% | 90% | 90% | 92% | 93% | 91% |
| Spec 3 | 27% | 5% | 30% | 11% | 7% | 19% | 14% | 7% | 19% |
| Cutoff 4 | 585 | 710 | 587 | 585 | 710 | 587 | 585 | 710 | 587 |
| Sens 4 | 43% | 35% | 51% | 44% | 41% | 50% | 52% | 36% | 55% |
| Spec 4 | 70% | 70% | 70% | 70% | 70% | 70% | 70% | 70% | 70% |
| Cutoff 5 | 829 | 997 | 859 | 829 | 997 | 859 | 829 | 997 | 859 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 5 | 34% | 26% | 40% | 36% | 27% | 40% | 35% | 29% | 32% |
| Spec 5 | 80% | 80% | 80% | 80% | 80% | 80% | 80% | 80% | 80% |
| Cutoff 6 | 1260 | 1420 | 1320 | 1260 | 1420 | 1320 | 1260 | 1420 | 1320 |
| Sens 6 | 20% | 17% | 22% | 21% | 18% | 21% | 19% | 14% | 15% |
| Spec 6 | 90% | 90% | 90% | 90% | 90% | 90% | 90% | 90% | 90% |
| OR Quart 2 | 1.8 | 1.00 | 2.6 | 1.1 | 1.6 | 0.89 | 1.5 | 1.0 | 1.6 |
| p Value | 0.067 | 0.99 | 0.0083 | 0.77 | 0.23 | 0.73 | 0.46 | 1.0 | 0.41 |
| 95% CI of | 0.96 | 0.42 | 1.3 | 0.61 | 0.73 | 0.47 | 0.54 | 0.35 | 0.52 |
| OR Quart2 | 3.3 | 2.3 | 5.4 | 2.0 | 3.7 | 1.7 | 3.9 | 2.9 | 5.1 |
| OR Quart 3 | 2.5 | 0.90 | 3.6 | 1.4 | 0.69 | 1.6 | 1.5 | 0.85 | 2.5 |
| p Value | 0.0030 | 0.82 | 3.1E-4 | 0.20 | 0.46 | 0.14 | 0.46 | 0.78 | 0.089 |
| 95% CI of | 1.4 | 0.38 | 1.8 | 0.82 | 0.26 | 0.86 | 0.54 | 0.28 | 0.87 |
| OR Quart3 | 4.5 | 2.2 | 7.3 | 2.5 | 1.8 | 2.8 | 3.9 | 2.6 | 7.4 |
| OR Quart 4 | 3.5 | 1.3 | 6.2 | 2.5 | 1.9 | 3.0 | 3.3 | 1.1 | 5.0 |
| p Value | 2.1E-5 | 0.55 | 1.2E-7 | 8.6E-4 | 0.13 | 7.9E-5 | 0.0081 | 0.80 | 0.0016 |
| 95% CI of | 2.0 | 0.57 | 3.2 | 1.5 | 0.84 | 1.8 | 1.4 | 0.41 | 1.8 |
| OR Quart4 | 6.3 | 2.9 | 12 | 4.2 | 4.1 | 5.3 | 8.1 | 3.2 | 14 |

**Immunoglogulin G1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2600 | 4230 | 2600 | 4600 | 2600 | 3720 |
| Average | 5310 | 6310 | 5310 | 7320 | 5310 | 5200 |
| Stdev | 7650 | 5510 | 7650 | 9110 | 7650 | 4150 |
| p(t-test) | | 0.18 | | 0.013 | | 0.92 |

**Immunoglogulin G1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Min | 59.7 | 342 | 59.7 | 138 | 59.7 | 177 |
| Max | 80000 | 28400 | 80000 | 80000 | 80000 | 16300 |
| n (Samp) | 461 | 119 | 461 | 126 | 461 | 47 |
| n (Patient) | 222 | 119 | 222 | 126 | 222 | 47 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 3180 | 5010 | 3180 | 6800 | 3180 | 4830 |
| Average | 5750 | 7340 | 5750 | 8240 | 5750 | 6160 |
| Stdev | 7310 | 6940 | 7310 | 7030 | 7310 | 4600 |
| p(t-test) | | 0.18 | | 0.024 | | 0.78 |
| Min | 3.36 | 342 | 3.36 | 388 | 3.36 | 177 |
| Max | 80000 | 28400 | 80000 | 25600 | 80000 | 14900 |
| n (Samp) | 1011 | 40 | 1011 | 46 | 1011 | 26 |
| n (Patient) | 373 | 40 | 373 | 46 | 373 | 26 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 2690 | 5000 | 2690 | 5650 | 2690 | 3900 |
| Average | 5520 | 7480 | 5520 | 8340 | 5520 | 6110 |
| Stdev | 7740 | 6370 | 7740 | 9920 | 7740 | 5690 |
| p(t-test) | | 0.016 | | 0.0012 | | 0.62 |
| Min | 59.7 | 663 | 59.7 | 138 | 59.7 | 550 |

EP 3 489 688 A1

(continued)

UO only

| | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 80000 | 35700 | 80000 | 80000 | 80000 | 25300 |
| n (Samp) | 433 | 107 | 433 | 115 | 433 | 44 |
| n (Patient) | 171 | 107 | 171 | 115 | 171 | 44 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.63 | 0.60 | 0.66 | 0.62 | 0.61 | 0.64 | 0.59 | 0.57 | 0.62 |
| SE | 0.030 | 0.048 | 0.031 | 0.029 | 0.045 | 0.030 | 0.045 | 0.059 | 0.047 |
| p | $1.2E-5$ | 0.033 | $2.2E-7$ | $7.0E-5$ | 0.017 | $2.8E-6$ | 0.053 | 0.23 | 0.014 |
| nCohort 1 | 461 | 1011 | 433 | 461 | 1011 | 433 | 461 | 1011 | 433 |
| nCohort 2 | 119 | 40 | 107 | 126 | 46 | 115 | 47 | 26 | 44 |
| Cutoff 1 | 2770 | 3070 | 3090 | 2420 | 2620 | 3070 | 2500 | 2440 | 2880 |
| Sens 1 | 71% | 70% | 70% | 71% | 72% | 70% | 70% | 73% | 70% |
| Spec 1 | 52% | 49% | 55% | 46% | 42% | 55% | 48% | 39% | 52% |
| Cutoff 2 | 2010 | 2840 | 2510 | 1690 | 1550 | 1990 | 1690 | 1690 | 2370 |
| Sens 2 | 81% | 80% | 80% | 80% | 80% | 80% | 81% | 81% | 82% |
| Spec 2 | 39% | 45% | 46% | 31% | 23% | 38% | 31% | 26% | 44% |
| Cutoff 3 | 1390 | 1090 | 1600 | 924 | 911 | 1280 | 1080 | 831 | 1670 |
| Sens 3 | 91% | 90% | 91% | 90% | 91% | 90% | 91% | 92% | 91% |
| Spec 3 | 25% | 14% | 29% | 16% | 12% | 21% | 18% | 10% | 30% |
| Cutoff 4 | 4740 | 5870 | 4990 | 4740 | 5870 | 4990 | 4740 | 5870 | 4990 |
| Sens 4 | 45% | 32% | 50% | 49% | 52% | 53% | 36% | 46% | 36% |
| Spec 4 | 70% | 70% | 70% | 70% | 70% | 70% | 70% | 70% | 70% |
| Cutoff 5 | 7460 | 8580 | 7650 | 7460 | 8580 | 7650 | 7460 | 8580 | 7650 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Sens 5 | 29% | 30% | 38% | 33% | 41% | 38% | 28% | 35% | 27% |
| Spec 5 | 80% | 80% | 80% | 80% | 80% | 80% | 80% | 80% | 80% |
| Cutoff 6 | 13400 | 13900 | 14200 | 13400 | 13900 | 14200 | 13400 | 13900 | 14200 |
| Sens 6 | 12% | 18% | 12% | 16% | 22% | 17% | 6% | 4% | 11% |
| Spec 6 | 90% | 90% | 90% | 90% | 90% | 90% | 90% | 90% | 90% |
| OR Quart 2 | 1.9 | 1.3 | 2.0 | 0.94 | 0.69 | 1.4 | 2.5 | 0.80 | 6.0 |
| p Value | 0.085 | 0.59 | 0.090 | 0.85 | 0.46 | 0.37 | 0.088 | 0.74 | 0.022 |
| 95% CI of | 0.92 | 0.46 | 0.90 | 0.48 | 0.26 | 0.68 | 0.87 | 0.21 | 1.3 |
| OR Quart2 | 3.8 | 3.9 | 4.3 | 1.8 | 1.8 | 2.8 | 7.5 | 3.0 | 27 |
| OR Quart 3 | 3.6 | 2.4 | 3.8 | 2.2 | 0.69 | 2.9 | 3.0 | 1.0 | 10 |
| p Value | 1.6E-4 | 0.078 | 3.4E-4 | 0.0077 | 0.46 | 0.0015 | 0.039 | 1.0 | 0.0020 |
| 95% CI of | 1.8 | 0.91 | 1.8 | 1.2 | 0.26 | 1.5 | 1.1 | 0.29 | 2.4 |
| OR Quart3 | 6.9 | 6.3 | 7.9 | 4.0 | 1.8 | 5.6 | 8.7 | 3.5 | 46 |
| OR Quart 4 | 3.7 | 2.0 | 5.1 | 2.6 | 2.3 | 3.8 | 3.5 | 2.5 | 7.1 |
| p Value | 1.0E-4 | 0.16 | 8.4E-6 | 0.0011 | 0.034 | 4.2E-5 | 0.017 | 0.096 | 0.011 |
| 95% CI of | 1.9 | 0.75 | 2.5 | 1.5 | 1.1 | 2.0 | 1.2 | 0.85 | 1.6 |
| OR Quart4 | 7.1 | 5.5 | 10 | 4.7 | 5.0 | 7.3 | 9.9 | 7.1 | 32 |

**Immunoglobulin G2**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 7990 | 11000 | 7990 | 12100 | 7990 | 9560 |
| Average | 16000 | 20900 | 16000 | 26500 | 16000 | 16600 |
| Stdev | 26200 | 31200 | 26200 | 45400 | 26200 | 26800 |
| p(t-test) |  | 0.077 |  | 8.8E-4 |  | 0.87 |

| Immunoglogulin G2 | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Min | 119 | 819 | 119 | 640 | 119 | 1050 |
| Max | 240000 | 240000 | 240000 | 240000 | 240000 | 172000 |
| n (Samp) | 461 | 119 | 461 | 126 | 461 | 47 |
| n (Patient) | 222 | 119 | 222 | 126 | 222 | 47 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 9050 | 11500 | 9050 | 13400 | 9050 | 9970 |
| Average | 17700 | 24000 | 17700 | 35800 | 17700 | 22000 |
| Stdev | 29600 | 43100 | 29600 | 55000 | 29600 | 34000 |
| p(t-test) | | 0.19 | | 1.1E-4 | | 0.46 |
| Min | 119 | 819 | 119 | 1070 | 119 | 1310 |
| Max | 240000 | 240000 | 240000 | 240000 | 240000 | 172000 |
| n (Samp) | 1011 | 40 | 1011 | 46 | 1011 | 26 |
| n (Patient) | 373 | 40 | 373 | 46 | 373 | 26 |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 8540 | 13200 | 8540 | 13500 | 8540 | 10800 |
| Average | 17400 | 26300 | 17400 | 29800 | 17400 | 21300 |
| Stdev | 29900 | 38600 | 29900 | 50500 | 29900 | 38200 |
| p(t-test) | | 0.010 | | 8.8E-4 | | 0.43 |
| Min | 334 | 2930 | 334 | 640 | 334 | 1050 |
| Max | 240000 | 240000 | 240000 | 240000 | 240000 | 240000 |

EP 3 489 688 A1

(continued)

| UO only | 0hr prior to AKI stage | | 24hr prior to AKI stage | | | 48hr prior to AKI stage | |
|---|---|---|---|---|---|---|---|
| | Cohort 1 | Cohort 2 | Cohort 1 | Cohort 2 | | Cohort 1 | Cohort 2 |
| n (Samp) | 433 | 107 | 433 | 115 | | 433 | 44 |
| n (Patient) | 171 | 107 | 171 | 115 | | 171 | 44 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.60 | 0.55 | 0.63 | 0.59 | 0.58 | 0.61 | 0.54 | 0.56 | 0.56 |
| SE | 0.030 | 0.048 | 0.031 | 0.029 | 0.045 | 0.031 | 0.045 | 0.059 | 0.047 |
| p | 0.0016 | 0.26 | 2.4E-5 | 0.0022 | 0.064 | 4.8E-4 | 0.40 | 0.30 | 0.21 |
| nCohort 1 | 461 | 1011 | 433 | 461 | 1011 | 433 | 461 | 1011 | 433 |
| nCohort 2 | 119 | 40 | 107 | 126 | 46 | 115 | 47 | 26 | 44 |
| Cutoff 1 | 7640 | 7760 | 8630 | 6690 | 5500 | 7670 | 6460 | 6690 | 7030 |
| Sens 1 | 71% | 70% | 70% | 71% | 72% | 70% | 70% | 73% | 70% |
| Spec 1 | 48% | 43% | 51% | 42% | 29% | 45% | 41% | 37% | 42% |
| Cutoff 2 | 5300 | 6310 | 6460 | 4860 | 4630 | 5930 | 4240 | 6520 | 4680 |
| Sens 2 | 81% | 80% | 80% | 80% | 80% | 80% | 81% | 81% | 82% |
| Spec 2 | 33% | 34% | 38% | 29% | 23% | 33% | 25% | 36% | 24% |
| Cutoff 3 | 3890 | 2220 | 4750 | 2560 | 3170 | 3280 | 2800 | 3420 | 3060 |
| Sens 3 | 91% | 90% | 91% | 90% | 91% | 91% | 91% | 92% | 91% |
| Spec 3 | 21% | 9% | 24% | 12% | 13% | 14% | 14% | 15% | 13% |
| Cutoff 4 | 14400 | 15000 | 14500 | 14400 | 15000 | 14500 | 14400 | 15000 | 14500 |
| Sens 4 | 36% | 30% | 44% | 43% | 43% | 45% | 34% | 42% | 36% |
| Spec 4 | 70% | 70% | 70% | 70% | 70% | 70% | 70% | 70% | 70% |
| Cutoff 5 | 19800 | 21300 | 19800 | 19800 | 21300 | 19800 | 19800 | 21300 | 19800 |
| Sens 5 | 25% | 25% | 33% | 31% | 37% | 33% | 21% | 27% | 23% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 5 | 80% | 80% | 80% | 80% | 80% | 80% | 80% | 80% | 80% |
| Cutoff 6 | 32800 | 36600 | 35300 | 32800 | 36600 | 35300 | 32800 | 36600 | 35300 |
| Sens 6 | 16% | 15% | 20% | 18% | 26% | 17% | 9% | 12% | 11% |
| Spec 6 | 90% | 90% | 90% | 90% | 90% | 90% | 90% | 90% | 90% |
| OR Quart 2 | 1.4 | 0.87 | 1.7 | 1.1 | 0.63 | 1.3 | 0.89 | 1.4 | 0.88 |
| p Value | 0.32 | 0.79 | 0.16 | 0.77 | 0.34 | 0.41 | 0.81 | 0.56 | 0.80 |
| 95% CI of | 0.72 | 0.31 | 0.82 | 0.59 | 0.24 | 0.69 | 0.35 | 0.44 | 0.33 |
| OR Quart2 | 2.7 | 2.4 | 3.4 | 2.0 | 1.6 | 2.5 | 2.3 | 4.5 | 2.4 |
| OR Quart 3 | 3.0 | 1.8 | 2.9 | 1.7 | 1.0 | 1.9 | 1.7 | 1.0 | 1.6 |
| p Value | 4.5E-4 | 0.20 | 0.0020 | 0.064 | 1.0 | 0.046 | 0.22 | 1.0 | 0.28 |
| 95% CI of | 1.6 | 0.74 | 1.5 | 0.97 | 0.43 | 1.0 | 0.73 | 0.29 | 0.68 |
| OR Quart3 | 5.5 | 4.3 | 5.7 | 3.1 | 2.3 | 3.5 | 3.9 | 3.5 | 3.9 |
| OR Quart 4 | 2.2 | 1.4 | 3.3 | 2.1 | 1.6 | 2.6 | 1.2 | 1.8 | 1.5 |
| p Value | 0.016 | 0.49 | 5.4E-4 | 0.0091 | 0.25 | 0.0025 | 0.66 | 0.29 | 0.38 |
| 95% CI of | 1.2 | 0.55 | 1.7 | 1.2 | 0.72 | 1.4 | 0.51 | 0.60 | 0.61 |
| OR Quart4 | 4.0 | 3.5 | 6.4 | 3.8 | 3.4 | 4.7 | 2.9 | 5.5 | 3.6 |
| **Interleukin-11** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 109 | | 223 | 109 | | 165 | 109 | | 120 |
| Average | 186 | | 304 | 186 | | 233 | 186 | | 193 |
| Stdev | 225 | | 268 | 225 | | 205 | 225 | | 328 |
| p(t-test) | | | 1.2E-6 | | | 0.033 | | | 0.83 |
| Min | 0.154 | | 13.2 | 0.154 | | 7.35 | 0.154 | | 0.0822 |

EP 3 489 688 A1

(continued)

| Interleukin-11 | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Max | 1980 | 1780 | 1980 | 1050 | 1980 | 2260 |
| n (Samp) | 461 | 119 | 461 | 129 | 461 | 47 |
| n (Patient) | 223 | 119 | 223 | 129 | 223 | 47 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 131 | 226 | 131 | 202 | 131 | 124 |
| Average | 216 | 253 | 216 | 240 | 216 | 167 |
| Stdev | 249 | 173 | 249 | 191 | 249 | 139 |
| p(t-test) | | 0.35 | | 0.52 | | 0.32 |
| Min | 0.0822 | 13.2 | 0.0822 | 7.35 | 0.0822 | 0.0822 |
| Max | 2260 | 659 | 2260 | 996 | 2260 | 558 |
| n (Samp) | 1017 | 40 | 1017 | 46 | 1017 | 26 |
| n (Patient) | 375 | 40 | 375 | 46 | 375 | 26 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 131 | 232 | 131 | 169 | 131 | 138 |
| Average | 206 | 349 | 206 | 264 | 206 | 244 |
| Stdev | 234 | 381 | 234 | 315 | 234 | 448 |
| p(t-test) | | 1.1E-6 | | 0.026 | | 0.35 |
| Min | 2.83 | 41.8 | 2.83 | 8.36 | 2.83 | 14.5 |
| Max | 1980 | 2900 | 1980 | 2590 | 1980 | 2260 |

EP 3 489 688 A1

79

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Samp) | 434 | 107 | 434 | 118 | 434 | 44 |
| n (Patient) | 173 | 107 | 173 | 118 | 173 | 44 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.70 | 0.61 | 0.69 | 0.62 | 0.58 | 0.59 | 0.52 | 0.47 | 0.48 |
| SE | 0.029 | 0.048 | 0.031 | 0.029 | 0.045 | 0.030 | 0.045 | 0.058 | 0.046 |
| p | 6.9E-12 | 0.029 | 1.2E-9 | 7.4E-5 | 0.072 | 0.0033 | 0.69 | 0.62 | 0.68 |
| nCohort 1 | 461 | 1017 | 434 | 461 | 1017 | 434 | 461 | 1017 | 434 |
| nCohort 2 | 119 | 40 | 107 | 129 | 46 | 118 | 47 | 26 | 44 |
| Cutoff 1 | 144 | 111 | 152 | 106 | 105 | 111 | 79.8 | 66.2 | 79.8 |
| Sens 1 | 71% | 70% | 70% | 71% | 72% | 70% | 70% | 73% | 70% |
| Spec 1 | 61% | 43% | 56% | 48% | 40% | 44% | 36% | 23% | 31% |
| Cutoff 2 | 107 | 78.7 | 121 | 76.0 | 76.5 | 85.7 | 66.1 | 57.1 | 66.1 |
| Sens 2 | 81% | 80% | 80% | 81% | 80% | 81% | 81% | 81% | 82% |
| Spec 2 | 49% | 29% | 47% | 34% | 28% | 32% | 30% | 19% | 24% |
| Cutoff 3 | 73.6 | 41.6 | 87.1 | 55.8 | 40.7 | 66.2 | 53.9 | 41.4 | 31.8 |
| Sens 3 | 91% | 90% | 91% | 91% | 91% | 91% | 91% | 92% | 91% |
| Spec 3 | 33% | 10% | 33% | 25% | 10% | 24% | 23% | 10% | 6% |
| Cutoff 4 | 189 | 218 | 217 | 189 | 218 | 217 | 189 | 218 | 217 |
| Sens 4 | 58% | 55% | 52% | 43% | 46% | 37% | 21% | 27% | 23% |
| Spec 4 | 70% | 70% | 70% | 70% | 70% | 70% | 70% | 70% | 70% |
| Cutoff 5 | 254 | 308 | 289 | 254 | 308 | 289 | 254 | 308 | 289 |
| Sens 5 | 46% | 35% | 42% | 31% | 30% | 28% | 17% | 15% | 18% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 5 | 80% | 80% | 80% | 80% | 80% | 80% | 80% | 80% | 80% |
| Cutoff 6 | 399 | 503 | 414 | 399 | 503 | 414 | 399 | 503 | 414 |
| Sens 6 | 27% | 8% | 28% | 15% | 7% | 15% | 6% | 4% | 7% |
| Spec 6 | 90% | 90% | 90% | 90% | 90% | 90% | 90% | 90% | 90% |
| OR Quart 2 | 2.7 | 1.6 | 2.6 | 2.3 | 1.6 | 1.8 | 2.5 | 1.6 | 1.6 |
| p Value | 0.017 | 0.40 | 0.024 | 0.013 | 0.35 | 0.081 | 0.055 | 0.40 | 0.27 |
| 95% CI of | 1.2 | 0.52 | 1.1 | 1.2 | 0.61 | 0.93 | 0.98 | 0.52 | 0.68 |
| OR Quart2 | 6.1 | 5.0 | 5.9 | 4.5 | 4.2 | 3.4 | 6.2 | 5.0 | 4.0 |
| OR Quart 3 | 4.6 | 1.8 | 4.2 | 3.3 | 1.3 | 2.3 | 2.1 | 1.0 | 1.4 |
| p Value | 1.1E-4 | 0.29 | 3.7E-4 | 3.0E-4 | 0.62 | 0.011 | 0.12 | 1.0 | 0.49 |
| 95% CI of | 2.1 | 0.60 | 1.9 | 1.7 | 0.47 | 1.2 | 0.83 | 0.29 | 0.55 |
| OR Quart3 | 10 | 5.5 | 9.2 | 6.3 | 3.5 | 4.2 | 5.5 | 3.5 | 3.4 |
| OR Quart 4 | 9.0 | 3.8 | 7.2 | 3.6 | 2.8 | 2.4 | 1.5 | 1.6 | 1.0 |
| p Value | 1.2E-8 | 0.0097 | 4.4E-7 | 8.9E-5 | 0.021 | 0.0072 | 0.45 | 0.40 | 0.99 |
| 95% CI of | 4.2 | 1.4 | 3.3 | 1.9 | 1.2 | 1.3 | 0.54 | 0.52 | 0.39 |
| OR Quart4 | 19 | 10 | 15 | 6.8 | 6.9 | 4.4 | 4.0 | 5.0 | 2.6 |

**Interleukin-2 receptor alpha chain**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 434 | 788 | 434 | 572 | 434 | 526 |
| Average | 833 | 1150 | 833 | 1190 | 833 | 986 |
| Stdev | 1060 | 1220 | 1060 | 1640 | 1060 | 1440 |
| p(t-test) | | 0.0055 | | 0.0028 | | 0.37 |
| Min | 0.0317 | 0.0339 | 0.0317 | 0.0339 | 0.0317 | 0.120 |

| Interleukin-2 receptor alpha chain | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Max | 10400 | 6080 | 10400 | 9090 | 10400 | 7740 |
| n (Samp) | 462 | 120 | 462 | 130 | 462 | 47 |
| n (Patient) | 223 | 120 | 223 | 130 | 223 | 47 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 622 | 431 | 622 | 570 | 622 | 515 |
| Average | 1060 | 1110 | 1060 | 1190 | 1060 | 1020 |
| Stdev | 1380 | 1720 | 1380 | 1550 | 1380 | 1450 |
| p(t-test) | | 0.82 | | 0.56 | | 0.87 |
| Min | 0.0317 | 0.0339 | 0.0317 | 0.0365 | 0.0317 | 0.141 |
| Max | 10400 | 7330 | 10400 | 7190 | 10400 | 5640 |
| n (Samp) | 1019 | 40 | 1019 | 46 | 1019 | 26 |
| n (Patient) | 375 | 40 | 375 | 46 | 375 | 26 |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 454 | 942 | 454 | 734 | 454 | 533 |
| Average | 840 | 1310 | 840 | 1330 | 840 | 981 |
| Stdev | 1070 | 1260 | 1070 | 1690 | 1070 | 1430 |
| p(t-test) | | 8.8E-5 | | 1.3E-4 | | 0.42 |
| Min | 0.0317 | 0.0339 | 0.0317 | 0.0339 | 0.0317 | 0.120 |
| Max | 10400 | 6080 | 10400 | 9090 | 10400 | 7740 |
| n (Samp) | 436 | 108 | 436 | 119 | 436 | 44 |

EP 3 489 688 A1

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | Cohort 2 | | Cohort 1 | Cohort 2 | |
| n (Patient) | 173 | | 108 | 173 | 119 | | 173 | 44 | |
| | | | | | | | | | |
| | 0hr prior to AKI stage | | | 24hr prior to AKI stage | | | 48hr prior to AKI stage | | |
| | sCr or UO | sCr only | UO only | sCr or UO | sCr only | UO only | sCr or UO | sCr only | UO only |
| AUC | 0.60 | 0.44 | 0.65 | 0.54 | 0.50 | 0.58 | 0.52 | 0.47 | 0.52 |
| SE | 0.030 | 0.048 | 0.031 | 0.029 | 0.044 | 0.030 | 0.045 | 0.058 | 0.046 |
| p | 9.8E-4 | 0.19 | 1.9E-6 | 0.14 | 0.93 | 0.0089 | 0.64 | 0.58 | 0.66 |
| nCohort 1 | 462 | 1019 | 436 | 462 | 1019 | 436 | 462 | 1019 | 436 |
| nCohort 2 | 120 | 40 | 108 | 130 | 46 | 119 | 47 | 26 | 44 |
| Cutoff 1 | 394 | 73.3 | 548 | 216 | 226 | 280 | 213 | 195 | 205 |
| Sens 1 | 70% | 70% | 70% | 70% | 72% | 71% | 70% | 73% | 70% |
| Spec 1 | 48% | 13% | 55% | 34% | 27% | 37% | 34% | 24% | 31% |
| Cutoff 2 | 228 | 38.4 | 342 | 139 | 148 | 152 | 139 | 158 | 135 |
| Sens 2 | 80% | 80% | 81% | 80% | 80% | 81% | 81% | 81% | 82% |
| Spec 2 | 35% | 9% | 42% | 25% | 20% | 26% | 25% | 21% | 23% |
| Cutoff 3 | 55.0 | 23.9 | 128 | 33.7 | 35.6 | 49.3 | 45.1 | 126 | 40.9 |
| Sens 3 | 90% | 90% | 91% | 90% | 91% | 91% | 91% | 92% | 91% |
| Spec 3 | 16% | 8% | 23% | 13% | 9% | 14% | 14% | 17% | 12% |
| Cutoff 4 | 932 | 1150 | 928 | 932 | 1150 | 928 | 932 | 1150 | 928 |
| Sens 4 | 46% | 30% | 51% | 37% | 35% | 42% | 34% | 19% | 34% |
| Spec 4 | 70% | 70% | 70% | 70% | 70% | 70% | 70% | 70% | 70% |
| Cutoff 5 | 1410 | 1690 | 1330 | 1410 | 1690 | 1330 | 1410 | 1690 | 1330 |
| Sens 5 | 24% | 18% | 31% | 25% | 24% | 31% | 19% | 15% | 23% |
| Spec 5 | 80% | 80% | 80% | 80% | 80% | 80% | 80% | 80% | 80% |

EP 3 489 688 A1

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Cutoff 6 | 2100 | 2510 | 2210 | 2100 | 2510 | 2210 | 2100 | 2510 | 2210 |
| Sens 6 | 15% | 15% | 18% | 16% | 15% | 18% | 11% | 12% | 11% |
| Spec 6 | 90% | 90% | 90% | 90% | 90% | 90% | 90% | 90% | 90% |
| OR Quart 2 | 0.89 | 0.49 | 1.7 | 0.88 | 0.74 | 0.94 | 1.1 | 1.2 | 1.1 |
| p Value | 0.72 | 0.20 | 0.19 | 0.66 | 0.51 | 0.85 | 0.82 | 0.76 | 0.81 |
| 95% CI of | 0.46 | 0.17 | 0.78 | 0.50 | 0.31 | 0.51 | 0.45 | 0.36 | 0.44 |
| OR Quart2 | 1.7 | 1.5 | 3.6 | 1.6 | 1.8 | 1.7 | 2.7 | 4.0 | 2.9 |
| OR Quart 3 | 2.1 | 0.90 | 4.0 | 1.1 | 1.0 | 1.2 | 1.3 | 1.4 | 1.5 |
| p Value | 0.011 | 0.82 | 1.0E-4 | 0.78 | 0.99 | 0.47 | 0.51 | 0.56 | 0.37 |
| 95% CI of | 1.2 | 0.36 | 2.0 | 0.62 | 0.44 | 0.69 | 0.56 | 0.44 | 0.62 |
| OR Quart3 | 3.8 | 2.2 | 8.1 | 1.9 | 2.3 | 2.2 | 3.2 | 4.5 | 3.7 |
| OR Quart 4 | t2.0 | 1.6 | 4.2 | 1.3 | 1.1 | 1.8 | 1.3 | 1.6 | 1.4 |
| p Value | 0.023 | 0.23 | 6.6E-5 | 0.34 | 0.83 | 0.038 | 0.53 | 0.40 | 0.49 |
| 95% CI of | 1.1 | 0.73 | 2.1 | 0.76 | 0.49 | 1.0 | 0.56 | 0.52 | 0.55 |
| OR Quart4 | 3.5 | 3.7 | 8.4 | 2.2 | 2.4 | 3.2 | 3.1 | 5.0 | 3.4 |

**Neutrophil collagenase**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 2540 | 4730 | 2540 | 5520 | 2540 | 3780 |
| Average | 11100 | 18100 | 11100 | 19900 | 11100 | 16200 |
| Stdev | 40300 | 56700 | 40300 | 45400 | 40300 | 36300 |
| p(t-test) |  | 0.099 |  | 0.028 |  | 0.41 |
| Min | 0.114 | 150 | 0.114 | 22.5 | 0.114 | 50.0 |
| Max | 670000 | 625000 | 670000 | 312000 | 670000 | 236000 |

| Neutrophil collagenase | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| n (Samp) | 498 | 142 | 498 | 140 | 498 | 48 |
| n (Patient) | 235 | 142 | 235 | 140 | 235 | 48 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 3460 | 3530 | 3460 | 7850 | 3460 | 4980 |
| Average | 15100 | 14000 | 15100 | 36300 | 15100 | 16200 |
| Stdev | 44100 | 25900 | 44100 | 106000 | 44100 | 44400 |
| p(t-test) | | 0.86 | | 0.0024 | | 0.90 |
| Min | 0.114 | 150 | 0.114 | 40.5 | 0.114 | 111 |
| Max | 670000 | 128000 | 670000 | 625000 | 670000 | 236000 |
| n (Samp) | 1095 | 46 | 1095 | 51 | 1095 | 28 |
| n (Patient) | 398 | 46 | 398 | 51 | 398 | 28 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 2550 | 5870 | 2550 | 6090 | 2550 | 4960 |
| Average | 8970 | 26600 | 8970 | 24800 | 8970 | 11700 |
| Stdev | 22700 | 78300 | 22700 | 53900 | 22700 | 16600 |
| p(t-test) | | 1.7E-5 | | 6.8E-7 | | 0.42 |
| Min | 0.114 | 0.260 | 0.114 | 22.5 | 0.114 | 50.0 |
| Max | 300000 | 625000 | 300000 | 327000 | 300000 | 66900 |
| n (Samp) | 485 | 129 | 485 | 130 | 485 | 47 |

EP 3 489 688 A1

(continued)

UO only

| | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Patient) | 190 | 129 | 190 | 130 | 190 | 47 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.62 | 0.54 | 0.65 | 0.61 | 0.57 | 0.64 | 0.58 | 0.54 | 0.59 |
| SE | 0.028 | 0.044 | 0.029 | 0.028 | 0.043 | 0.029 | 0.045 | 0.056 | 0.045 |
| p | 2.5E-5 | 0.33 | 1.5E-7 | 6.4E-5 | 0.091 | 9.6E-7 | 0.075 | 0.51 | 0.055 |
| nCohort 1 | 498 | 1095 | 485 | 498 | 1095 | 485 | 498 | 1095 | 485 |
| nCohort 2 | 142 | 46 | 129 | 140 | 51 | 130 | 48 | 28 | 47 |
| Cutoff 1 | 2030 | 1700 | 2620 | 2250 | 1970 | 2450 | 1430 | 1820 | 1550 |
| Sens 1 | 70% | 72% | 71% | 70% | 71% | 70% | 71% | 71% | 70% |
| Spec 1 | 44% | 34% | 51% | 47% | 37% | 49% | 37% | 36% | 38% |
| Cutoff 2 | 1110 | 977 | 1710 | 1060 | 935 | 1460 | 870 | 1410 | 898 |
| Sens 2 | 80% | 80% | 81% | 80% | 80% | 80% | 81% | 82% | 81% |
| Spec 2 | 31% | 22% | 40% | 30% | 21% | 36% | 27% | 29% | 26% |
| Cutoff 3 | 765 | 418 | 844 | 536 | 546 | 804 | 191 | 365 | 387 |
| Sens 3 | 90% | 91% | 91% | 90% | 90% | 90% | 92% | 93% | 91% |
| Spec 3 | 25% | 11% | 25% | 19% | 14% | 25% | 7% | 10% | 13% |
| Cutoff 4 | 6260 | 8700 | 6150 | 6260 | 8700 | 6150 | 6260 | 8700 | 6150 |
| Sens 4 | 45% | 41% | 49% | 46% | 49% | 50% | 44% | 32% | 47% |
| Spec 4 | 70% | 70% | 70% | 70% | 70% | 70% | 70% | 70% | 70% |
| Cutoff 5 | 10000 | 14900 | 9570 | 10000 | 14900 | 9570 | 10000 | 14900 | 9570 |
| Sens 5 | 35% | 28% | 39% | 35% | 27% | 38% | 35% | 18% | 34% |
| Spec 5 | 80% | 80% | 80% | 80% | 80% | 80% | 80% | 80% | 80% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 6 | 20800 | 32400 | 21900 | 20800 | 32400 | 21900 | 20800 | 32400 | 21900 |
| Sens 6 | 16% | 7% | 20% | 19% | 14% | 20% | 21% | 7% | 15% |
| Spec 6 | 90% | 90% | 90% | 90% | 90% | 90% | 90% | 90% | 90% |
| OR Quart 2 | 1.4 | 0.74 | 2.1 | 1.3 | 0.52 | 1.8 | 1.1 | 2.0 | 1.1 |
| p Value | 0.29 | 0.51 | 0.026 | 0.46 | 0.18 | 0.083 | 0.82 | 0.25 | 0.81 |
| 95% CI of | 0.76 | 0.31 | 1.1 | 0.69 | 0.21 | 0.93 | 0.44 | 0.60 | 0.44 |
| OR Quart2 | 2.5 | 1.8 | 4.2 | 2.3 | 1.3 | 3.4 | 2.8 | 6.8 | 2.9 |
| OR Quart 3 | 2.1 | 0.66 | 2.6 | 2.0 | 1.0 | 2.4 | 1.2 | 2.5 | 1.2 |
| p Value | 0.012 | 0.37 | 0.0038 | 0.018 | 1.0 | 0.0053 | 0.64 | 0.12 | 0.64 |
| 95% CI of | 1.2 | 0.26 | 1.4 | 1.1 | 0.46 | 1.3 | 0.50 | 0.79 | 0.50 |
| OR Quart3 | 3.7 | 1.6 | 5.1 | 3.5 | 2.2 | 4.6 | 3.1 | 8.2 | 3.1 |
| OR Quart 4 | 2.9 | 1.4 | 4.6 | 2.6 | 1.4 | 3.7 | 2.1 | 1.5 | 2.0 |
| p Value | 1.6E-4 | 0.35 | 2.4E-6 | 7.1E-4 | 0.36 | 2.1E-5 | 0.076 | 0.53 | 0.10 |
| 95% CI of | 1.7 | 0.67 | 2.4 | 1.5 | 0.68 | 2.0 | 0.92 | 0.42 | 0.87 |
| OR Quart4 | 5.1 | 3.1 | 8.6 | 4.5 | 2.9 | 6.9 | 4.9 | 5.4 | 4.7 |

**Protransforming growth factor alpha**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5.17 | 7.34 | 5.17 | 8.58 | 5.17 | 5.71 |
| Average | 10.2 | 12.2 | 10.2 | 11.9 | 10.2 | 7.65 |
| Stdev | 23.4 | 16.8 | 23.4 | 11.0 | 23.4 | 7.10 |
| p(t-test) | | 0.38 | | 0.43 | | 0.46 |
| Min | 0.00184 | 0.00574 | 0.00184 | 0.00603 | 0.00184 | 0.00440 |
| Max | 361 | 135 | 361 | 54.1 | 361 | 32.4 |

EP 3 489 688 A1

| Protransforming growth factor alpha | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| n (Samp) | 462 | 116 | 462 | 130 | 462 | 47 |
| n (Patient) | 223 | 116 | 223 | 130 | 223 | 47 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 6.18 | 7.25 | 6.18 | 7.37 | 6.18 | 4.95 |
| Average | 10.8 | 9.21 | 10.8 | 10.0 | 10.8 | 6.94 |
| Stdev | 19.7 | 7.72 | 19.7 | 10.1 | 19.7 | 6.70 |
| p(t-test) | | 0.61 | | 0.79 | | 0.32 |
| Min | 0.00184 | 0.00574 | 0.00184 | 0.00574 | 0.00184 | 0.00574 |
| Max | 361 | 30.2 | 361 | 50.4 | 361 | 27.7 |
| n (Samp) | 1014 | 39 | 1014 | 46 | 1014 | 26 |
| n (Patient) | 375 | 39 | 375 | 46 | 375 | 26 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 5.14 | 8.56 | 5.14 | 10.2 | 5.14 | 6.78 |
| Average | 10.3 | 14.4 | 10.3 | 13.6 | 10.3 | 8.83 |
| Stdev | 24.0 | 18.7 | 24.0 | 12.8 | 24.0 | 7.87 |
| p(t-test) | | 0.10 | | 0.15 | | 0.68 |
| Min | 0.00184 | 0.00603 | 0.00184 | 0.00603 | 0.00184 | 0.00440 |
| Max | 361 | 135 | 361 | 77.6 | 361 | 36.0 |
| n (Samp) | 436 | 104 | 436 | 119 | 436 | 44 |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | Cohort 2 | | Cohort 1 | Cohort 2 | |
| n (Patient) | 173 | | 104 | 173 | 119 | | 173 | 44 | |
| | | | | | | | | | |
| | 0hr prior to AKI stage | | | 24hr prior to AKI stage | | | 48hr prior to AKI stage | | |
| | sCr or UO | sCr only | UO only | sCr or UO | sCr only | UO only | sCr or UO | sCr only | UO only |
| AUC | 0.59 | 0.52 | 0.64 | 0.63 | 0.52 | 0.67 | 0.52 | 0.43 | 0.55 |
| SE | 0.030 | 0.048 | 0.032 | 0.029 | 0.044 | 0.030 | 0.045 | 0.059 | 0.047 |
| p | 0.0022 | 0.67 | 1.7E-5 | 5.1E-6 | 0.61 | 2.5E-8 | 0.73 | 0.25 | 0.24 |
| nCohort 1 | 462 | 1014 | 436 | 462 | 1014 | 436 | 462 | 1014 | 436 |
| nCohort 2 | 116 | 39 | 104 | 130 | 46 | 119 | 47 | 26 | 44 |
| Cutoff 1 | 4.08 | 3.95 | 5.24 | 5.35 | 3.53 | 6.12 | 2.73 | 2.73 | 3.27 |
| Sens 1 | 71% | 72% | 70% | 70% | 72% | 71% | 70% | 73% | 70% |
| Spec 1 | 41% | 32% | 51% | 52% | 29% | 56% | 29% | 22% | 32% |
| Cutoff 2 | 3.05 | 2.30 | 3.71 | 3.25 | 3.07 | 3.61 | 2.55 | 2.17 | 2.52 |
| Sens 2 | 80% | 82% | 81% | 80% | 80% | 81% | 81% | 81% | 82% |
| Spec 2 | 32% | 18% | 36% | 34% | 25% | 35% | 27% | 17% | 23% |
| Cutoff 3 | 2.14 | 0.804 | 2.37 | 2.15 | 0.00713 | 2.17 | 1.65 | 1.60 | 1.79 |
| Sens 3 | 91% | 92% | 90% | 90% | 91% | 92% | 91% | 92% | 91% |
| Spec 3 | 23% | 6% | 22% | 23% | 2% | 21% | 18% | 12% | 18% |
| Cutoff 4 | 8.54 | 9.97 | 8.15 | 8.54 | 9.97 | 8.15 | 8.54 | 9.97 | 8.15 |
| Sens 4 | 43% | 41% | 51% | 51% | 33% | 61% | 32% | 27% | 41% |
| Spec 4 | 70% | 70% | 70% | 70% | 70% | 70% | 70% | 70% | 70% |
| Cutoff 5 | 11.8 | 13.4 | 11.2 | 11.8 | 13.4 | 11.2 | 11.8 | 13.4 | 11.2 |
| Sens 5 | 30% | 23% | 38% | 36% | 28% | 45% | 21% | 12% | 30% |
| Spec 5 | 80% | 80% | 80% | 80% | 80% | 80% | 80% | 80% | 80% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 6 | 19.3 | 22.6 | 19.8 | 19.3 | 22.6 | 19.8 | 19.3 | 22.6 | 19.8 |
| Sens 6 | 17% | 8% | 20% | 16% | 9% | 20% | 6% | 4% | 7% |
| Spec 6 | 90% | 90% | 90% | 90% | 90% | 90% | 90% | 90% | 90% |
| OR Quart 2 | 1.8 | 0.69 | 1.9 | 1.2 | 0.81 | 1.1 | 1.6 | 1.0 | 0.56 |
| p Value | 0.064 | 0.46 | 0.077 | 0.51 | 0.65 | 0.87 | 0.28 | 1.0 | 0.24 |
| 95% CI of | 0.97 | 0.26 | 0.93 | 0.65 | 0.33 | 0.52 | 0.68 | 0.29 | 0.21 |
| OR Quart2 | 3.5 | 1.8 | 4.0 | 2.4 | 2.0 | 2.2 | 3.9 | 3.5 | 1.5 |
| OR Quart 3 | 1.7 | 1.0 | 2.5 | 2.4 | 1.0 | 2.1 | 1.2 | 1.6 | 0.73 |
| p Value | 0.11 | 1.0 | 0.013 | 0.0040 | 1.0 | 0.021 | 0.64 | 0.40 | 0.49 |
| 95% CI of | 0.89 | 0.41 | 1.2 | 1.3 | 0.43 | 1.1 | 0.50 | 0.52 | 0.30 |
| OR Quart3 | 3.2 | 2.4 | 5.0 | 4.4 | 2.3 | 4.0 | 3.1 | 5.0 | 1.8 |
| OR Quart 4 | 2.7 | 1.2 | 4.0 | 3.4 | 1.4 | 4.3 | 1.5 | 1.6 | 1.4 |
| p Value | 0.0017 | 0.67 | 7.6E-5 | 5.8E-5 | 0.42 | 3.7E-6 | 0.39 | 0.40 | 0.42 |
| 95% CI of | 1.4 | 0.51 | 2.0 | 1.9 | 0.62 | 2.3 | 0.61 | 0.52 | 0.62 |
| OR Quart4 | 4.9 | 2.8 | 7.8 | 6.1 | 3.1 | 7.9 | 3.6 | 5.0 | 3.1 |

**CA 15-3**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 423 | 434 | 423 | 434 | 423 | 434 |
| Average | 280 | 376 | 280 | 378 | 280 | 361 |
| Stdev | 244 | 215 | 244 | 212 | 244 | 141 |
| p(t-test) | | 0.0018 | | 0.0022 | | 0.098 |
| Min | 3.03 | 11.6 | 3.03 | 6.39 | 3.03 | 21.6 |
| Max | 784 | 784 | 784 | 784 | 784 | 434 |

(continued)

| CA 15-3 | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| n (Samp) | 194 | 86 | 194 | 76 | 194 | 26 |
| n (Patient) | 123 | 86 | 123 | 76 | 123 | 26 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 434 | 434 | 434 | 434 | 434 | 434 |
| Average | 331 | 306 | 331 | 360 | 331 | 296 |
| Stdev | 227 | 228 | 227 | 202 | 227 | 186 |
| p(t-test) | | 0.59 | | 0.50 | | 0.55 |
| Min | 1.17 | 9.20 | 1.17 | 20.6 | 1.17 | 11.7 |
| Max | 784 | 784 | 784 | 784 | 784 | 434 |
| n (Samp) | 428 | 26 | 428 | 28 | 428 | 16 |
| n (Patient) | 210 | 26 | 210 | 28 | 210 | 16 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 434 | 434 | 434 | 434 | 434 | 434 |
| Average | 286 | 395 | 286 | 377 | 286 | 352 |
| Stdev | 233 | 193 | 233 | 216 | 233 | 147 |
| p(t-test) | | 2.9E-4 | | 0.0041 | | 0.16 |
| Min | 3.03 | 12.2 | 3.03 | 6.39 | 3.03 | 28.4 |
| Max | 784 | 784 | 784 | 784 | 784 | 434 |
| n (Samp) | 210 | 78 | 210 | 72 | 210 | 27 |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| n (Patient) | 119 | | 78 | 119 | | 72 | 119 | | 27 |
| | | | | | | | | | |
| | 0hr prior to AKI stage | | | 24hr prior to AKI stage | | | 48hr prior to AKI stage | | |
| | sCr or UO | sCr only | UO only | sCr or UO | sCr only | UO only | sCr or UO | sCr only | UO only |
| AUC | 0.62 | 0.47 | 0.63 | 0.63 | 0.55 | 0.62 | 0.62 | 0.48 | 0.60 |
| SE | 0.037 | 0.059 | 0.038 | 0.039 | 0.058 | 0.040 | 0.062 | 0.074 | 0.061 |
| p | 0.0010 | 0.64 | 5.3E-4 | 0.0013 | 0.42 | 0.0027 | 0.056 | 0.81 | 0.099 |
| nCohort 1 | 194 | 428 | 210 | 194 | 428 | 210 | 194 | 428 | 210 |
| nCohort 2 | 86 | 26 | 78 | 76 | 28 | 72 | 26 | 16 | 27 |
| Cutoff 1 | 320 | 66.6 | 427 | 413 | 413 | 427 | 413 | 113 | 427 |
| Sens 1 | 71% | 73% | 74% | 72% | 71% | 71% | 73% | 75% | 70% |
| Spec 1 | 47% | 25% | 47% | 50% | 39% | 47% | 50% | 30% | 47% |
| Cutoff 2 | 123 | 52.6 | 253 | 129 | 88.9 | 64.1 | 248 | 62.4 | 239 |
| Sens 2 | 80% | 81% | 81% | 80% | 82% | 81% | 81% | 81% | 81% |
| Spec 2 | 44% | 23% | 45% | 45% | 28% | 34% | 47% | 24% | 44% |
| Cutoff 3 | 48.4 | 25.1 | 52.2 | 33.2 | 24.5 | 42.9 | 68.6 | 21.3 | 68.6 |
| Sens 3 | 91% | 92% | 91% | 91% | 93% | 90% | 92% | 94% | 93% |
| Spec 3 | 33% | 13% | 32% | 25% | 12% | 28% | 39% | 11% | 35% |
| Cutoff 4 | 434 | 434 | 434 | 434 | 434 | 434 | 434 | 434 | 434 |
| Sens 4 | 12% | 8% | 10% | 12% | 7% | 14% | 0% | 0% | 0% |
| Spec 4 | 91% | 90% | 92% | 91% | 90% | 92% | 91% | 90% | 92% |
| Cutoff 5 | 434 | 434 | 434 | 434 | 434 | 434 | 434 | 434 | 434 |
| Sens 5 | 12% | 8% | 10% | 12% | 7% | 14% | 0% | 0% | 0% |
| Spec 5 | 91% | 90% | 92% | 91% | 90% | 92% | 91% | 90% | 92% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 6 | 434 | 434 | 434 | 434 | 434 | 434 | 434 | 434 | 434 |
| Sens 6 | 12% | 8% | 10% | 12% | 7% | 14% | 0% | 0% | 0% |
| Spec 6 | 91% | 90% | 92% | 91% | 90% | 92% | 91% | 90% | 92% |
| OR Quart 2 | 5.8 | 0.50 | 5.9 | 3.3 | 4.5 | 4.2 | 6.6 | >6.3 | 10 |
| p Value | 8.1E-5 | 0.57 | 1.4E-4 | 0.0094 | 0.0091 | 0.0014 | 0.085 | <0.090 | 0.029 |
| 95% CI of | 2.4 | 0.045 | 2.4 | 1.3 | 1.5 | 1.7 | 0.77 | >0.75 | 1.3 |
| OR Quart2 | 14 | 5.6 | 15 | 8.1 | 14 | 10 | 57 | na | 85 |
| OR Quart 3 | 9.2 | 9.1 | 8.8 | 9.7 | 1.5 | 5.5 | 28 | >6.3 | 23 |
| p Value | 6.4E-7 | 0.0037 | 2.6E-6 | 4.7E-7 | 0.52 | 1.4E-4 | 0.0014 | <0.090 | 0.0026 |
| 95% CI of | 3.8 | 2.1 | 3.5 | 4.0 | 0.42 | 2.3 | 3.7 | >0.75 | 3.0 |
| OR Quart3 | 22 | 41 | 22 | 23 | 5.6 | 13 | 220 | na | 180 |
| OR Quart 4 | 1.3 | 3.7 | 1.2 | 1.1 | 0.49 | 1.3 | 0 | >4.1 | 0 |
| p Value | 0.61 | 0.11 | 0.79 | 0.82 | 0.42 | 0.64 | na | <0.21 | na |
| 95% CI of | 0.48 | 0.75 | 0.40 | 0.41 | 0.088 | 0.47 | na | >0.46 | na |
| OR Quart4 | 3.5 | 18 | 3.4 | 3.1 | 2.7 | 3.4 | na | na | na |

Table 2: Comparison of marker levels in urine samples collected from Cohort 1 (patients that did not progress beyond RIFLE stage 0 or R) and in urine samples collected from subjects at 0, 24 hours, and 48 hours prior to reaching stage I or F in Cohort 2.

**C-C motif chemokine 18**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.418 | 0.977 | 0.418 | 1.37 | 0.418 | 0.524 |
| Average | 2.19 | 3.40 | 2.19 | 5.34 | 2.19 | 6.72 |
| Stdev | 6.72 | 6.53 | 6.72 | 10.3 | 6.72 | 13.8 |
| p(t-test) | | 0.17 | | 3.2E-4 | | 1.1E-4 |
| Min | 3.13E-5 | 0.00308 | 3.13E-5 | 0.0139 | 3.13E-5 | 0.00215 |
| Max | 40.0 | 40.0 | 40.0 | 40.0 | 40.0 | 40.0 |
| n (Samp) | 927 | 62 | 927 | 70 | 927 | 39 |
| n (Patient) | 360 | 62 | 360 | 70 | 360 | 39 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.455 | 1.90 | 0.455 | 1.34 | 0.455 | 1.26 |
| Average | 2.54 | 8.19 | 2.54 | 6.52 | 2.54 | 5.50 |
| Stdev | 7.37 | 12.4 | 7.37 | 10.7 | 7.37 | 11.1 |
| p(t-test) | | 0.0035 | | 0.024 | | 0.10 |
| Min | 3.13E-5 | 0.192 | 3.13E-5 | 0.150 | 3.13E-5 | 0.0404 |
| Max | 40.0 | 40.0 | 40.0 | 40.0 | 40.0 | 37.8 |
| n (Samp) | 1230 | 15 | 1230 | 18 | 1230 | 17 |
| n (Patient) | 440 | 15 | 440 | 18 | 440 | 17 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.456 | 0.951 | 0.456 | 1.71 | 0.456 | 0.692 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 2.32 | 4.12 | 2.32 | 6.37 | 2.32 | 8.23 |
| Stdev | 6.87 | 8.22 | 6.87 | 11.6 | 6.87 | 14.6 |
| p(t-test) | | 0.060 | | 2.5E-5 | | 4.6E-6 |
| Min | 3.13E-5 | 0.00308 | 3.13E-5 | 0.0139 | 3.13E-5 | 0.00215 |
| Max | 40.0 | 40.0 | 40.0 | 40.0 | 40.0 | 40.0 |
| n (Samp) | 815 | 57 | 815 | 63 | 815 | 34 |
| n (Patient) | 282 | 57 | 282 | 63 | 282 | 34 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.65 | 0.79 | 0.63 | 0.67 | 0.70 | 0.68 | 0.58 | 0.66 | 0.60 |
| SE | 0.039 | 0.070 | 0.041 | 0.036 | 0.070 | 0.038 | 0.049 | 0.073 | 0.052 |
| p | 1.9E-4 | 2.6E-5 | 0.0015 | 2.2E-6 | 0.0046 | 4.5E-6 | 0.094 | 0.025 | 0.047 |
| nCohort 1 | 927 | 1230 | 815 | 927 | 1230 | 815 | 927 | 1230 | 815 |
| nCohort 2 | 62 | 15 | 57 | 70 | 18 | 63 | 39 | 17 | 34 |
| Cutoff 1 | 0.351 | 1.29 | 0.351 | 0.408 | 0.533 | 0.408 | 0.372 | 0.415 | 0.373 |
| Sens 1 | 71% | 73% | 70% | 70% | 72% | 71% | 72% | 71% | 71% |
| Spec 1 | 46% | 75% | 44% | 50% | 54% | 48% | 47% | 48% | 45% |
| Cutoff 2 | 0.231 | 0.985 | 0.231 | 0.282 | 0.340 | 0.282 | 0.131 | 0.388 | 0.131 |
| Sens 2 | 81% | 80% | 81% | 80% | 83% | 81% | 82% | 82% | 82% |
| Spec 2 | 33% | 69% | 31% | 41% | 43% | 38% | 21% | 47% | 19% |
| Cutoff 3 | 0.156 | 0.767 | 0.147 | 0.150 | 0.181 | 0.142 | 0.0423 | 0.225 | 0.0950 |
| Sens 3 | 90% | 93% | 91% | 90% | 94% | 90% | 92% | 94% | 91% |
| Spec 3 | 25% | 63% | 22% | 25% | 26% | 21% | 7% | 30% | 13% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 4 | 0.900 | 1.02 | 0.982 | 0.900 | 1.02 | 0.982 | 0.900 | 1.02 | 0.982 |
| Sens 4 | 53% | 73% | 49% | 57% | 61% | 59% | 38% | 59% | 44% |
| Spec 4 | 70% | 70% | 70% | 70% | 70% | 70% | 70% | 70% | 70% |
| Cutoff 5 | 1.48 | 1.77 | 1.61 | 1.48 | 1.77 | 1.61 | 1.48 | 1.77 | 1.61 |
| Sens 5 | 42% | 53% | 42% | 49% | 44% | 51% | 33% | 35% | 38% |
| Spec 5 | 80% | 80% | 80% | 80% | 80% | 80% | 80% | 80% | 80% |
| Cutoff 6 | 3.30 | 3.86 | 3.53 | 3.30 | 3.86 | 3.53 | 3.30 | 3.86 | 3.53 |
| Sens 6 | 26% | 33% | 28% | 29% | 28% | 33% | 23% | 24% | 24% |
| Spec 6 | 90% | 90% | 90% | 90% | 90% | 90% | 90% | 90% | 90% |
| OR Quart 2 | 2.2 | >1.0 | 1.8 | 1.9 | 3.0 | 1.5 | 1.00 | 5.0 | 0.71 |
| p Value | 0.088 | <1.00 | 0.19 | 0.14 | 0.34 | 0.37 | 0.99 | 0.14 | 0.56 |
| 95% CI of | 0.89 | >0.062 | 0.74 | 0.80 | 0.31 | 0.61 | 0.37 | 0.59 | 0.22 |
| OR Quart2 | 5.5 | na | 4.4 | 4.6 | 29 | 3.8 | 2.7 | 43 | 2.3 |
| OR Quart 3 | 1.6 | >5.1 | 1.0 | 1.4 | 5.1 | 1.0 | 1.3 | 3.0 | 1.3 |
| p Value | 0.34 | <0.14 | 1.0 | 0.48 | 0.14 | 1.0 | 0.63 | 0.34 | 0.61 |
| 95% CI of | 0.61 | >0.59 | 0.37 | 0.55 | 0.59 | 0.37 | 0.49 | 0.31 | 0.47 |
| OR Quart3 | 4.2 | na | 2.7 | 3.5 | 44 | 2.7 | 3.3 | 29 | 3.6 |
| OR Quart 4 | 4.5 | >9.2 | 3.7 | 5.1 | 9.2 | 5.0 | 1.7 | 8.2 | 1.9 |
| p Value | 4.5E-4 | <0.035 | 0.0016 | 5.4E-5 | 0.035 | 7.1E-5 | 0.27 | 0.048 | 0.18 |
| 95% CI of | 1.9 | >1.2 | 1.6 | 2.3 | 1.2 | 2.3 | 0.67 | 1.0 | 0.74 |
| OR Quart4 | 11 | na | 8.4 | 11 | 73 | 11 | 4.1 | 66 | 4.9 |

EP 3 489 688 A1

| C-C motif chemokine 24 | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 11.1 | 26.1 | 11.1 | 27.2 | 11.1 | 13.3 |
| Average | 25.6 | 78.6 | 25.6 | 100 | 25.6 | 65.6 |
| Stdev | 60.0 | 180 | 60.0 | 331 | 60.0 | 197 |
| p(t-test) | | 4.5E-8 | | 1.1E-8 | | 5.4E-4 |
| Min | 0.0120 | 0.0123 | 0.0120 | 0.0347 | 0.0120 | 0.0123 |
| Max | 1090 | 1010 | 1090 | 2380 | 1090 | 1170 |
| n (Samp) | 928 | 62 | 928 | 70 | 928 | 39 |
| n (Patient) | 361 | 62 | 361 | 70 | 361 | 39 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 13.4 | 60.3 | 13.4 | 28.9 | 13.4 | 25.4 |
| Average | 37.8 | 104 | 37.8 | 60.4 | 37.8 | 46.1 |
| Stdev | 125 | 123 | 125 | 68.1 | 125 | 67.5 |
| p(t-test) | | 0.041 | | 0.44 | | 0.78 |
| Min | 0.0120 | 0.444 | 0.0120 | 2.39 | 0.0120 | 0.0196 |
| Max | 2380 | 373 | 2380 | 214 | 2380 | 249 |
| n (Samp) | 1232 | 15 | 1232 | 18 | 1232 | 17 |
| n (Patient) | 441 | 15 | 441 | 18 | 441 | 17 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 11.3 | 26.8 | 11.3 | 27.4 | 11.3 | 15.7 |

EP 3 489 688 A1

(continued)

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Average | 26.4 | | 125 | 26.4 | | 139 | 26.4 | | 79.3 |
| Stdev | 63.0 | | 403 | 63.0 | | 416 | 63.0 | | 210 |
| p(t-test) | | | 1.9E-9 | | | 1.9E-11 | | | 5.4E-5 |
| Min | 0.0120 | | 0.0123 | 0.0120 | | 0.0347 | 0.0120 | | 0.0123 |
| Max | 1090 | | 2790 | 1090 | | 2380 | 1090 | | 1170 |
| n (Samp) | 817 | | 57 | 817 | | 63 | 817 | | 34 |
| n (Patient) | 283 | | 57 | 283 | | 63 | 283 | | 34 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.68 | 0.75 | 0.68 | 0.68 | 0.69 | 0.69 | 0.52 | 0.59 | 0.56 |
| SE | 0.039 | 0.073 | 0.040 | 0.036 | 0.070 | 0.038 | 0.048 | 0.073 | 0.052 |
| p | 5.1E-6 | 5.2E-4 | 5.7E-6 | 5.9E-7 | 0.0067 | 3.0E-7 | 0.61 | 0.20 | 0.22 |
| nCohort 1 | 928 | 1232 | 817 | 928 | 1232 | 817 | 928 | 1232 | 817 |
| nCohort 2 | 62 | 15 | 57 | 70 | 18 | 63 | 39 | 17 | 34 |
| Cutoff 1 | 10.4 | 25.9 | 11.3 | 14.5 | 15.6 | 14.5 | 4.24 | 14.9 | 4.59 |
| Sens 1 | 71% | 73% | 70% | 70% | 72% | 71% | 72% | 71% | 71% |
| Spec 1 | 48% | 69% | 50% | 57% | 55% | 58% | 32% | 54% | 33% |
| Cutoff 2 | 6.47 | 21.9 | 6.55 | 9.55 | 8.15 | 9.78 | 0.303 | 2.37 | 2.59 |
| Sens 2 | 81% | 80% | 81% | 80% | 83% | 81% | 82% | 82% | 82% |
| Spec 2 | 37% | 65% | 38% | 46% | 39% | 46% | 22% | 24% | 27% |
| Cutoff 3 | 2.85 | 2.85 | 1.36 | 4.66 | 3.22 | 5.39 | 0.0277 | 0.0277 | 0.0562 |
| Sens 3 | 90% | 93% | 91% | 90% | 94% | 90% | 92% | 94% | 91% |
| Spec 3 | 28% | 25% | 24% | 33% | 26% | 35% | 8% | 7% | 19% |

EP 3 489 688 A1

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 4 | 22.3 | 26.6 | 21.8 | 22.3 | 26.6 | 21.8 | 22.3 | 26.6 | 21.8 |
| Sens 4 | 58% | 67% | 60% | 57% | 61% | 59% | 36% | 41% | 44% |
| Spec 4 | 70% | 70% | 70% | 70% | 70% | 70% | 70% | 70% | 70% |
| Cutoff 5 | 34.1 | 40.7 | 33.4 | 34.1 | 40.7 | 33.4 | 34.1 | 40.7 | 33.4 |
| Sens 5 | 42% | 67% | 44% | 39% | 39% | 41% | 21% | 24% | 26% |
| Spec 5 | 80% | 80% | 80% | 80% | 80% | 80% | 80% | 80% | 80% |
| Cutoff 6 | 57.3 | 69.9 | 62.1 | 57.3 | 69.9 | 62.1 | 57.3 | 69.9 | 62.1 |
| Sens 6 | 27% | 33% | 28% | 20% | 28% | 21% | 15% | 18% | 21% |
| Spec 6 | 90% | 90% | 90% | 90% | 90% | 90% | 90% | 90% | 90% |
| OR Quart 2 | 2.4 | 0.50 | 2.0 | 5.2 | 3.0 | 4.2 | 1.3 | 0.25 | 1.5 |
| p Value | 0.077 | 0.57 | 0.16 | 0.0096 | 0.34 | 0.029 | 0.64 | 0.21 | 0.44 |
| 95% CI of | 0.91 | 0.045 | 0.75 | 1.5 | 0.31 | 1.2 | 0.49 | 0.028 | 0.53 |
| OR Quart2 | 6.4 | 5.5 | 5.6 | 18 | 29 | 15 | 3.2 | 2.2 | 4.3 |
| OR Quart 3 | 2.4 | 1.00 | 2.1 | 6.8 | 6.1 | 6.1 | 1.5 | 2.0 | 1.5 |
| p Value | 0.076 | 1.00 | 0.16 | 0.0023 | 0.095 | 0.0045 | 0.37 | 0.25 | 0.44 |
| 95% CI of | 0.91 | 0.14 | 0.76 | 2.0 | 0.73 | 1.7 | 0.61 | 0.60 | 0.53 |
| OR Quart3 | 6.4 | 7.1 | 5.6 | 23 | 51 | 21 | 3.8 | 6.8 | 4.3 |
| OR Quart 4 | 5.1 | 5.1 | 5.0 | 12 | 8.2 | 12 | 1.1 | 1.00 | 1.7 |
| p Value | 3.8E-4 | 0.036 | 5.2E-4 | 3.5E-5 | 0.048 | 5.4E-5 | 0.81 | 1.00 | 0.32 |
| 95% CI of | 2.1 | 1.1 | 2.0 | 3.8 | 1.0 | 3.6 | 0.43 | 0.25 | 0.60 |
| OR Quart4 | 13 | 24 | 12 | 41 | 66 | 39 | 3.0 | 4.0 | 4.7 |

| C-C motif chemokine 8 | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 1.21 | 5.60 | 1.21 | 6.10 | 1.21 | 1.51 |
| Average | 9.02 | 18.6 | 9.02 | 19.2 | 9.02 | 18.6 |
| Stdev | 25.8 | 33.5 | 25.8 | 42.6 | 25.8 | 41.8 |
| p(t-test) | | 0.0056 | | 0.0027 | | 0.028 |
| Min | 0.0250 | 0.0775 | 0.0250 | 0.0250 | 0.0250 | 0.0250 |
| Max | 473 | 162 | 473 | 294 | 473 | 214 |
| n (Samp) | 928 | 62 | 928 | 70 | 928 | 39 |
| n (Patient) | 361 | 62 | 361 | 70 | 361 | 39 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 1.36 | 7.59 | 1.36 | 8.78 | 1.36 | 2.77 |
| Average | 11.2 | 23.8 | 11.2 | 40.3 | 11.2 | 11.6 |
| Stdev | 32.4 | 36.8 | 32.4 | 75.9 | 32.4 | 16.3 |
| p(t-test) | | 0.14 | | 2.5E-4 | | 0.96 |
| Min | 0.0250 | 0.127 | 0.0250 | 0.0569 | 0.0250 | 0.0250 |
| Max | 492 | 116 | 492 | 279 | 492 | 46.5 |
| n (Samp) | 1232 | 15 | 1232 | 18 | 1232 | 17 |
| n (Patient) | 441 | 15 | 441 | 18 | 441 | 17 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 1.21 | 5.77 | 1.21 | 6.84 | 1.21 | 2.14 |

(continued)

| UO only | 0hr prior to AKI stage | | | 24hr prior to AKI stage | | | 48hr prior to AKI stage | |
|---|---|---|---|---|---|---|---|---|
| | Cohort 1 | | Cohort 2 | Cohort 1 | | Cohort 2 | Cohort 1 | Cohort 2 |
| Average | 9.52 | | 22.6 | 9.52 | | 22.9 | 9.52 | 20.3 |
| Stdev | 27.3 | | 39.9 | 27.3 | | 45.9 | 27.3 | 44.5 |
| p(t-test) | | | 7.8E-4 | | | 4.5E-4 | | 0.029 |
| Min | 0.0250 | | 0.0775 | 0.0250 | | 0.0250 | 0.0250 | 0.0250 |
| Max | 473 | | 176 | 473 | | 294 | 473 | 214 |
| n (Samp) | 817 | | 57 | 817 | | 63 | 817 | 34 |
| n (Patient) | 283 | | 57 | 283 | | 63 | 283 | 34 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.67 | 0.63 | 0.69 | 0.65 | 0.67 | 0.66 | 0.56 | 0.55 | 0.57 |
| SE | 0.039 | 0.078 | 0.040 | 0.037 | 0.071 | 0.039 | 0.049 | 0.072 | 0.052 |
| p | 8.0E-6 | 0.093 | 2.4E-6 | 4.7E-5 | 0.016 | 2.2E-5 | 0.18 | 0.49 | 0.16 |
| nCohort 1 | 928 | 1232 | 817 | 928 | 1232 | 817 | 928 | 1232 | 817 |
| nCohort 2 | 62 | 15 | 57 | 70 | 18 | 63 | 39 | 17 | 34 |
| Cutoff 1 | 1.55 | 0.240 | 2.74 | 1.21 | 3.16 | 1.55 | 0.438 | 0.802 | 0.453 |
| Sens 1 | 71% | 73% | 70% | 71% | 72% | 71% | 72% | 71% | 71% |
| Spec 1 | 54% | 33% | 59% | 51% | 59% | 54% | 41% | 43% | 41% |
| Cutoff 2 | 0.802 | 0.239 | 0.802 | 0.240 | 0.239 | 0.453 | 0.154 | 0.127 | 0.240 |
| Sens 2 | 82% | 80% | 86% | 81% | 89% | 81% | 85% | 82% | 85% |
| Spec 2 | 45% | 31% | 46% | 36% | 31% | 41% | 29% | 21% | 35% |
| Cutoff 3 | 0.154 | 0.127 | 0.240 | 0.154 | 0.154 | 0.127 | 0.0775 | 0.0705 | 0.127 |
| Sens 3 | 92% | 93% | 91% | 90% | 94% | 90% | 92% | 94% | 91% |
| Spec 3 | 29% | 21% | 35% | 29% | 27% | 21% | 16% | 11% | 21% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 4 | 5.57 | 6.87 | 5.57 | 5.57 | 6.87 | 5.57 | 5.57 | 6.87 | 5.57 |
| Sens 4 | 50% | 53% | 51% | 53% | 61% | 56% | 33% | 35% | 35% |
| Spec 4 | 70% | 70% | 70% | 70% | 70% | 70% | 70% | 70% | 70% |
| Cutoff 5 | 10.6 | 12.1 | 11.1 | 10.6 | 12.1 | 11.1 | 10.6 | 12.1 | 11.1 |
| Sens 5 | 39% | 33% | 40% | 30% | 44% | 33% | 31% | 35% | 24% |
| Spec 5 | 80% | 80% | 80% | 80% | 80% | 80% | 80% | 80% | 80% |
| Cutoff 6 | 21.9 | 26.4 | 22.0 | 21.9 | 26.4 | 22.0 | 21.9 | 26.4 | 22.0 |
| Sens 6 | 24% | 27% | 28% | 20% | 22% | 25% | 18% | 18% | 18% |
| Spec 6 | 90% | 90% | 90% | 90% | 90% | 90% | 90% | 90% | 90% |
| OR Quart 2 | 5.2 | 1.5 | 2.8 | 2.4 | 4.0 | 1.4 | 2.7 | 1.0 | 3.1 |
| p Value | 0.0096 | 0.66 | 0.079 | 0.077 | 0.21 | 0.46 | 0.065 | 1.0 | 0.053 |
| 95% CI of | 1.5 | 0.25 | 0.89 | 0.91 | 0.45 | 0.54 | 0.94 | 0.25 | 0.98 |
| OR Quart2 | 18 | 9.0 | 9.0 | 6.4 | 36 | 3.9 | 7.6 | 4.0 | 9.8 |
| OR Quart 3 | 6.4 | 1.5 | 4.5 | 4.1 | 4.0 | 3.0 | 1.8 | 0.75 | 2.0 |
| p Value | 0.0032 | 0.66 | 0.0075 | 0.0025 | 0.21 | 0.013 | 0.29 | 0.70 | 0.25 |
| 95% CI of | 1.9 | 0.25 | 1.5 | 1.6 | 0.45 | 1.3 | 0.60 | 0.17 | 0.60 |
| OR Quart3 | 22 | 9.0 | 14 | 10 | 36 | 7.4 | 5.5 | 3.4 | 6.8 |
| OR Quart 4 | 9.5 | 3.5 | 6.9 | 4.9 | 9.2 | 4.1 | 2.5 | 1.5 | 2.6 |
| p Value | 2.6E-4 | 0.12 | 4.2E-4 | 5.6E-4 | 0.036 | 0.0013 | 0.095 | 0.53 | 0.12 |
| 95% CI of | 2.8 | 0.73 | 2.4 | 2.0 | 1.2 | 1.7 | 0.85 | 0.42 | 0.79 |
| OR Quart4 | 32 | 17 | 20 | 12 | 73 | 9.6 | 7.1 | 5.4 | 8.3 |

| Cathepsin D | | | | | | |
|---|---|---|---|---|---|---|
| sCr or UO | 0hr prior to AKI stage | | 24hr prior to AKI stage | | 48hr prior to AKI stage | |
|  | Cohort 1 | Cohort 2 | Cohort 1 | Cohort 2 | Cohort 1 | Cohort 2 |
| Median | 72900 | 109000 | 72900 | 93800 | 72900 | 87500 |
| Average | 77100 | 113000 | 77100 | 103000 | 77100 | 90300 |
| Stdev | 39300 | 46500 | 39300 | 43200 | 39300 | 45100 |
| p(t-test) |  | 8.8E-12 |  | 1.9E-7 |  | 0.042 |
| Min | 656 | 3710 | 656 | 22600 | 656 | 4320 |
| Max | 200000 | 200000 | 200000 | 200000 | 200000 | 200000 |
| n (Samp) | 927 | 62 | 927 | 70 | 927 | 39 |
| n (Patient) | 360 | 62 | 360 | 70 | 360 | 39 |
| | | | | | | |
| sCr only | 0hr prior to AKI stage | | 24hr prior to AKI stage | | 48hr prior to AKI stage | |
|  | Cohort 1 | Cohort 2 | Cohort 1 | Cohort 2 | Cohort 1 | Cohort 2 |
| Median | 76100 | 141000 | 76100 | 98000 | 76100 | 111000 |
| Average | 82300 | 128000 | 82300 | 112000 | 82300 | 97600 |
| Stdev | 42300 | 39000 | 42300 | 43000 | 42300 | 43200 |
| p(t-test) |  | 3.2E-5 |  | 0.0034 |  | 0.14 |
| Min | 656 | 70300 | 656 | 52100 | 656 | 22100 |
| Max | 200000 | 193000 | 200000 | 200000 | 200000 | 200000 |
| n (Samp) | 1230 | 15 | 1230 | 18 | 1230 | 17 |
| n (Patient) | 440 | 15 | 440 | 18 | 440 | 17 |
| | | | | | | |
| UO only | 0hr prior to AKI stage | | 24hr prior to AKI stage | | 48hr prior to AKI stage | |
|  | Cohort 1 | Cohort 2 | Cohort 1 | Cohort 2 | Cohort 1 | Cohort 2 |
| Median | 74300 | 111000 | 74300 | 97500 | 74300 | 94700 |

EP 3 489 688 A1

| UO only | 0hr prior to AKI stage | | | 24hr prior to AKI stage | | | 48hr prior to AKI stage | |
|---|---|---|---|---|---|---|---|---|
| | Cohort 1 | | Cohort 2 | Cohort 1 | | Cohort 2 | Cohort 1 | Cohort 2 |
| Average | 79900 | | 114000 | 79900 | | 105000 | 79900 | 97200 |
| Stdev | 38900 | | 47800 | 38900 | | 44600 | 38900 | 44600 |
| p(t-test) | | | 4.5E-10 | | | 9.0E-7 | | 0.012 |
| Min | 2520 | | 3710 | 2520 | | 22600 | 2520 | 4320 |
| Max | 200000 | | 200000 | 200000 | | 200000 | 200000 | 200000 |
| n (Samp) | 815 | | 57 | 815 | | 63 | 815 | 34 |
| n (Patient) | 282 | | 57 | 282 | | 63 | 282 | 34 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.73 | 0.79 | 0.72 | 0.67 | 0.69 | 0.67 | 0.59 | 0.61 | 0.63 |
| SE | 0.037 | 0.070 | 0.039 | 0.036 | 0.070 | 0.038 | 0.049 | 0.073 | 0.052 |
| p | 1.1E-9 | 3.2E-5 | 4.4E-8 | 2.2E-6 | 0.0060 | 1.4E-5 | 0.067 | 0.13 | 0.015 |
| nCohort 1 | 927 | 1230 | 815 | 927 | 1230 | 815 | 927 | 1230 | 815 |
| nCohort 2 | 62 | 15 | 57 | 70 | 18 | 63 | 39 | 17 | 34 |
| Cutoff 1 | 83000 | 100000 | 84700 | 78100 | 84700 | 77500 | 65500 | 70400 | 73300 |
| Sens 1 | 71% | 73% | 70% | 70% | 72% | 71% | 72% | 71% | 71% |
| Spec 1 | 62% | 70% | 60% | 57% | 58% | 53% | 43% | 44% | 49% |
| Cutoff 2 | 73800 | 94300 | 76300 | 68200 | 79000 | 68200 | 55600 | 55600 | 65500 |
| Sens 2 | 81% | 80% | 81% | 80% | 83% | 81% | 82% | 82% | 82% |
| Spec 2 | 52% | 66% | 53% | 45% | 53% | 43% | 31% | 27% | 40% |
| Cutoff 3 | 61400 | 73800 | 59600 | 54900 | 53700 | 55200 | 38800 | 40900 | 54900 |
| Sens 3 | 90% | 93% | 91% | 90% | 94% | 90% | 92% | 94% | 91% |
| Spec 3 | 37% | 47% | 33% | 30% | 25% | 28% | 15% | 16% | 27% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 4 | 92900 | 101000 | 96900 | 92900 | 101000 | 96900 | 92900 | 101000 | 96900 |
| Sens 4 | 61% | 67% | 61% | 51% | 50% | 51% | 46% | 59% | 47% |
| Spec 4 | 70% | 70% | 70% | 70% | 70% | 70% | 70% | 70% | 70% |
| Cutoff 5 | 111000 | 117000 | 112000 | 111000 | 117000 | 112000 | 111000 | 117000 | 112000 |
| Sens 5 | 48% | 53% | 49% | 39% | 44% | 40% | 26% | 35% | 26% |
| Spec 5 | 80% | 80% | 80% | 80% | 80% | 80% | 80% | 80% | 80% |
| Cutoff 6 | 130000 | 140000 | 133000 | 130000 | 140000 | 133000 | 130000 | 140000 | 133000 |
| Sens 6 | 37% | 53% | 32% | 23% | 28% | 24% | 18% | 6% | 21% |
| Spec 6 | 90% | 90% | 90% | 90% | 90% | 90% | 90% | 90% | 90% |
| OR Quart 2 | 2.3 | >2.0 | 1.2 | 2.9 | 2.0 | 2.7 | 2.0 | 2.0 | 2.7 |
| p Value | 0.17 | <0.57 | 0.76 | 0.044 | 0.57 | 0.065 | 0.20 | 0.42 | 0.14 |
| 95% CI of | 0.70 | >0.18 | 0.36 | 1.0 | 0.18 | 0.94 | 0.68 | 0.36 | 0.71 |
| OR Quart2 | 7.6 | na | 4.0 | 8.2 | 22 | 7.7 | 6.0 | 11 | 10 |
| OR Quart 3 | 4.2 | >4.1 | 3.1 | 4.7 | 7.1 | 3.8 | 2.0 | 1.00 | 3.1 |
| p Value | 0.011 | <0.21 | 0.029 | 0.0020 | 0.067 | 0.0091 | 0.20 | 1.00 | 0.094 |
| 95% CI of | 1.4 | >0.45 | 1.1 | 1.8 | 0.87 | 1.4 | 0.69 | 0.14 | 0.82 |
| OR Quart3 | 13 | na | 8.8 | 13 | 58 | 11 | 6.1 | 7.1 | 12 |
| OR Quart 4 | 9.3 | >9.2 | 7.1 | 6.4 | 8.2 | 6.0 | 2.9 | 4.6 | 4.9 |
| p Value | 3.3E-5 | <0.035 | 7.2E-5 | 1.7E-4 | 0.048 | 3.2E-4 | 0.044 | 0.053 | 0.014 |
| 95% CI of | 3.3 | >1.2 | 2.7 | 2.4 | 1.0 | 2.3 | 1.0 | 0.98 | 1.4 |
| OR Quart4 | 27 | na | 19 | 17 | 66 | 16 | 8.2 | 21 | 17 |

| C-X-C motif chemokine 13 | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 0.0222 | 1.42 | 0.0222 | 1.46 | 0.0222 | 0.232 |
| Average | 5.79 | 13.4 | 5.79 | 16.4 | 5.79 | 17.2 |
| Stdev | 38.4 | 34.8 | 38.4 | 33.5 | 38.4 | 71.5 |
| p(t-test) | | 0.13 | | 0.025 | | 0.083 |
| Min | 0.00269 | 0.00471 | 0.00269 | 0.00269 | 0.00269 | 0.00269 |
| Max | 832 | 210 | 832 | 131 | 832 | 440 |
| n (Samp) | 928 | 62 | 928 | 70 | 928 | 39 |
| n (Patient) | 361 | 62 | 361 | 70 | 361 | 39 |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 0.115 | 3.89 | 0.115 | 3.31 | 0.115 | 1.39 |
| Average | 8.27 | 22.4 | 8.27 | 20.2 | 8.27 | 9.64 |
| Stdev | 41.2 | 45.9 | 41.2 | 50.6 | 41.2 | 20.1 |
| p(t-test) | | 0.19 | | 0.22 | | 0.89 |
| Min | 0.00269 | 0.00627 | 0.00269 | 0.00822 | 0.00269 | 0.00269 |
| Max | 832 | 171 | 832 | 210 | 832 | 79.0 |
| n (Samp) | 1232 | 15 | 1232 | 18 | 1232 | 17 |
| n (Patient) | 441 | 15 | 441 | 18 | 441 | 17 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 0.0222 | 1.79 | 0.0222 | 1.86 | 0.0222 | 1.09 |
| Average | 6.06 | 48.6 | 6.06 | 48.7 | 6.06 | 20.3 |

EP 3 489 688 A1

(continued)

| UO only | 0hr prior to AKI stage | | | 24hr prior to AKI stage | | | 48hr prior to AKI stage | |
|---|---|---|---|---|---|---|---|---|
| | Cohort 1 | | Cohort 2 | Cohort 1 | Cohort 2 | | Cohort 1 | Cohort 2 |
| Stdev | 40.6 | | 257 | 40.6 | 234 | | 40.6 | 76.3 |
| p(t-test) | | | 4.8E-5 | | 1.0E-5 | | | 0.057 |
| Min | 0.00269 | | 0.00471 | 0.00269 | 0.00269 | | 0.00269 | 0.00269 |
| Max | 832 | | 1930 | 832 | 1850 | | 832 | 440 |
| n (Samp) | 817 | | 57 | 817 | 63 | | 817 | 34 |
| n (Patient) | 283 | | 57 | 283 | 63 | | 283 | 34 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.67 | 0.68 | 0.69 | 0.67 | 0.68 | 0.69 | 0.56 | 0.56 | 0.62 |
| SE | 0.039 | 0.077 | 0.040 | 0.036 | 0.070 | 0.038 | 0.048 | 0.073 | 0.052 |
| p | 8.2E-6 | 0.020 | 1.1E-6 | 2.1E-6 | 0.012 | 3.2E-7 | 0.23 | 0.43 | 0.020 |
| nCohort 1 | 928 | 1232 | 817 | 928 | 1232 | 817 | 928 | 1232 | 817 |
| nCohort 2 | 62 | 15 | 57 | 70 | 18 | 63 | 39 | 17 | 34 |
| Cutoff 1 | 0.352 | 1.02 | 0.362 | 0.335 | 0.605 | 0.392 | 0.0109 | 0.0130 | 0.0174 |
| Sens 1 | 71% | 73% | 72% | 70% | 72% | 71% | 74% | 71% | 71% |
| Spec 1 | 58% | 64% | 58% | 58% | 58% | 59% | 29% | 33% | 47% |
| Cutoff 2 | 0.0126 | 0.0109 | 0.0193 | 0.0130 | 0.0109 | 0.0140 | 0.00930 | 0.00564 | 0.0109 |
| Sens 2 | 81% | 80% | 81% | 80% | 83% | 81% | 82% | 82% | 82% |
| Spec 2 | 34% | 25% | 48% | 36% | 25% | 37% | 28% | 13% | 27% |
| Cutoff 3 | 0.00930 | 0.00756 | 0.00930 | 0.00834 | 0.00821 | 0.0109 | 0.00564 | 0.00269 | 0.00930 |
| Sens 3 | 90% | 93% | 95% | 90% | 100% | 90% | 92% | 94% | 91% |
| Spec 3 | 28% | 15% | 26% | 25% | 19% | 27% | 15% | 2% | 26% |
| Cutoff 4 | 1.13 | 1.62 | 1.13 | 1.13 | 1.62 | 1.13 | 1.13 | 1.62 | 1.13 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 4 | 56% | 67% | 60% | 56% | 67% | 59% | 44% | 47% | 50% |
| Spec 4 | 70% | 70% | 70% | 70% | 70% | 70% | 70% | 70% | 70% |
| Cutoff 5 | 2.67 | 3.52 | 2.66 | 2.67 | 3.52 | 2.66 | 2.67 | 3.52 | 2.66 |
| Sens 5 | 39% | 53% | 44% | 40% | 50% | 43% | 28% | 35% | 32% |
| Spec 5 | 80% | 80% | 80% | 80% | 80% | 80% | 80% | 80% | 80% |
| Cutoff 6 | 8.75 | 11.3 | 8.56 | 8.75 | 11.3 | 8.56 | 8.75 | 11.3 | 8.56 |
| Sens 6 | 26% | 33% | 28% | 26% | 22% | 30% | 15% | 18% | 21% |
| Spec 6 | 90% | 90% | 90% | 90% | 90% | 90% | 90% | 90% | 90% |
| OR Quart 2 | 2.7 | 0.33 | 2.0 | 1.9 | 0.66 | 1.5 | 1.4 | 0.20 | 3.1 |
| p Value | 0.065 | 0.34 | 0.20 | 0.18 | 0.65 | 0.43 | 0.47 | 0.14 | 0.096 |
| 95% CI of | 0.94 | 0.034 | 0.69 | 0.74 | 0.11 | 0.53 | 0.54 | 0.023 | 0.82 |
| OR Quart2 | 7.6 | 3.2 | 6.1 | 4.8 | 4.0 | 4.3 | 3.9 | 1.7 | 12 |
| OR Quart 3 | 3.4 | 0.66 | 3.1 | 2.9 | 0.66 | 3.6 | 1.1 | 0.60 | 2.7 |
| p Value | 0.020 | 0.65 | 0.029 | 0.020 | 0.66 | 0.0075 | 0.80 | 0.48 | 0.14 |
| 95% CI of | 1.2 | 0.11 | 1.1 | 1.2 | 0.11 | 1.4 | 0.41 | 0.14 | 0.71 |
| OR Quart3 | 9.3 | 4.0 | 8.8 | 6.9 | 4.0 | 9.1 | 3.2 | 2.5 | 10 |
| OR Quart 4 | 6.2 | 3.0 | 6.0 | 4.9 | 3.8 | 5.2 | 2.1 | 1.6 | 4.9 |
| p Value | 2.4E-4 | 0.097 | 3.2E-4 | 2.1E-4 | 0.044 | 3.4E-4 | 0.13 | 0.41 | 0.014 |
| 95% CI of | 2.3 | 0.82 | 2.3 | 2.1 | 1.0 | 2.1 | 0.81 | 0.52 | 1.4 |
| OR Quart4 | 16 | 11 | 16 | 11 | 14 | 13 | 5.2 | 5.0 | 17 |

**Insulin-like growth factor-binding protein 3**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 355 | 654 | 355 | 566 | 355 | 559 |

| Insulin-like growth factor-binding protein 3 | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Average | 611 | 1050 | 611 | 1210 | 611 | 688 |
| Stdev | 946 | 1310 | 946 | 2170 | 946 | 574 |
| p(t-test) | | 3.3E-4 | | 2.2E-6 | | 0.59 |
| Min | 0.0478 | 17.3 | 0.0478 | 32.5 | 0.0478 | 0.311 |
| Max | 12500 | 7720 | 12500 | 12500 | 12500 | 2100 |
| n (Samp) | 1005 | 70 | 1005 | 80 | 1005 | 46 |
| n (Patient) | 386 | 70 | 386 | 80 | 386 | 46 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 408 | 542 | 408 | 618 | 408 | 540 |
| Average | 715 | 1100 | 715 | 1280 | 715 | 797 |
| Stdev | 1130 | 1190 | 1130 | 1880 | 1130 | 745 |
| p(t-test) | | 0.17 | | 0.024 | | 0.74 |
| Min | 0.0478 | 38.8 | 0.0478 | 44.3 | 0.0478 | 44.1 |
| Max | 12500 | 4040 | 12500 | 8160 | 12500 | 2990 |
| n (Samp) | 1341 | 16 | 1341 | 21 | 1341 | 20 |
| n (Patient) | 472 | 16 | 472 | 21 | 472 | 20 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 367 | 713 | 367 | 646 | 367 | 701 |
| Average | 630 | 1320 | 630 | 1340 | 630 | 812 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | 1030 | 1980 | 1030 | 2260 | 1030 | 675 |
| p(t-test) | | 1.7E-6 | | 6.5E-7 | | 0.27 |
| Min | 0.0478 | 17.3 | 0.0478 | 32.5 | 0.0478 | 0.311 |
| Max | 12500 | 12500 | 12500 | 12500 | 12500 | 2870 |
| n (Samp) | 897 | 65 | 897 | 73 | 897 | 40 |
| n (Patient) | 310 | 65 | 310 | 73 | 310 | 40 |

| | 0hr prior to AKI stage | | | 124hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.65 | 0.61 | 0.67 | 0.62 | 0.62 | 0.64 | 0.57 | 0.58 | 0.61 |
| SE | 0.037 | 0.075 | 0.038 | 0.035 | 0.066 | 0.036 | 0.045 | 0.067 | 0.048 |
| p | 3.7E-5 | 0.13 | 4.9E-6 | 3.5E-4 | 0.080 | 6.4E-5 | 0.097 | 0.22 | 0.023 |
| nCohort 1 | 1005 | 1341 | 897 | 1005 | 1341 | 897 | 1005 | 1341 | 897 |
| nCohort 2 | 70 | 16 | 65 | 80 | 21 | 73 | 46 | 20 | 40 |
| Cutoff 1 | 408 | 212 | 411 | 313 | 287 | 319 | 201 | 294 | 271 |
| Sens 1 | 70% | 75% | 71% | 70% | 71% | 71% | 72% | 70% | 70% |
| Spec 1 | 54% | 33% | 53% | 46% | 40% | 45% | 35% | 41% | 40% |
| Cutoff 2 | 259 | 210 | 262 | 205 | 259 | 245 | 138 | 249 | 191 |
| Sens 2 | 80% | 81% | 80% | 80% | 81% | 81% | 80% | 80% | 80% |
| Spec 2 | 42% | 33% | 40% | 35% | 38% | 38% | 27% | 37% | 31% |
| Cutoff 3 | 118 | 163 | 118 | 114 | 163 | 118 | 43.4 | 139 | 52.6 |
| Sens 3 | 90% | 94% | 91% | 90% | 90% | 90% | 91% | 90% | 90% |
| Spec 3 | 24% | 26% | 21% | 23% | 26% | 21% | 8% | 24% | 9% |
| Cutoff 4 | 651 | 746 | 645 | 651 | 746 | 645 | 651 | 746 | 645 |

110

EP 3 489 688 A1

(continued)

|  | 0hr prior to AKI stage | | | 124hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Sens 4 | 51% | 44% | 57% | 45% | 43% | 51% | 46% | 45% | 50% |
| Spec 4 | 70% | 70% | 70% | 70% | 70% | 70% | 70% | 70% | 70% |
| Cutoff 5 | 922 | 1040 | 926 | 922 | 1040 | 926 | 922 | 1040 | 926 |
| Sens 5 | 33% | 38% | 37% | 35% | 43% | 38% | 33% | 30% | 40% |
| Spec 5 | 80% | 80% | 80% | 80% | 80% | 80% | 80% | 80% | 80% |
| Cutoff 6 | 1350 | 1540 | 1330 | 1350 | 1540 | 1330 | 1350 | 1540 | 1330 |
| Sens 6 | 23% | 19% | 26% | 22% | 19% | 27% | 15% | 10% | 22% |
| Spec 6 | 90% | 90% | 90% | 90% | 90% | 90% | 90% | 90% | 90% |
| OR Quart 2 | 1.4 | 4.0 | 1.00 | 1.8 | 2.5 | 1.6 | 1.00 | 2.0 | 1.1 |
| p Value | 0.49 | 0.21 | 0.99 | 0.14 | 0.27 | 0.23 | 0.99 | 0.32 | 0.79 |
| 95% CI of | 0.55 | 0.45 | 0.37 | 0.83 | 0.48 | 0.73 | 0.39 | 0.50 | 0.41 |
| OR Quart2 | 3.5 | 36 | 2.7 | 3.8 | 13 | 3.7 | 2.6 | 8.1 | 3.2 |
| OR Quart 3 | 2.9 | 4.0 | 2.5 | 1.8 | 2.5 | 1.8 | 1.1 | 1.3 | 0.85 |
| p Value | 0.012 | 0.21 | 0.034 | 0.14 | 0.27 | 0.17 | 0.82 | 0.70 | 0.78 |
| 95% CI of | 1.3 | 0.45 | 1.1 | 0.83 | 0.49 | 0.79 | 0.44 | 0.30 | 0.28 |
| OR Quart3 | 6.6 | 36 | 5.8 | 3.8 | 13 | 3.9 | 2.8 | 6.0 | 2.6 |
| OR Quart 4 | 3.9 | 7.1 | 4.1 | 3.0 | 4.6 | 3.3 | 2.1 | 2.4 | 2.9 |
| p Value | 8.3E-4 | 0.067 | 5.3E-4 | 0.0021 | 0.053 | 0.0017 | 0.083 | 0.22 | 0.020 |
| 95% CI of | 1.8 | 0.87 | 1.8 | 1.5 | 0.98 | 1.6 | 0.91 | 0.60 | 1.2 |
| OR Quart4 | 8.8 | 58 | 9.2 | 6.2 | 21 | 6.8 | 4.7 | 9.2 | 6.9 |

| Immunoglogulin G1 | | | | | | | | | |
|---|---|---|---|---|---|---|---|---|---|
| sCr or UO | 0hr prior to AKI stage | | | 24hr prior to AKI stage | | | 48hr prior to AKI stage | | |
|  | Cohort 1 | | Cohort 2 | Cohort 1 | | Cohort 2 | | Cohort 1 | Cohort 2 |
| Median | 3060 | | 5910 | 3060 | | 7970 | | 3060 | 4550 |

**Immunoglogulin G1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 5430 | 8350 | 5430 | 10300 | 5430 | 6440 |
| Stdev | 6740 | 7390 | 6740 | 11400 | 6740 | 6380 |
| p(t-test) | | 0.0011 | | 6.3E-8 | | 0.36 |
| Min | 59.7 | 447 | 59.7 | 634 | 59.7 | 55.9 |
| Max | 80000 | 35700 | 80000 | 80000 | 80000 | 25300 |
| n (Samp) | 922 | 62 | 922 | 70 | 922 | 39 |
| n (Patient) | 358 | 62 | 358 | 70 | 358 | 39 |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 3410 | 5220 | 3410 | 10900 | 3410 | 9840 |
| Average | 6040 | 9080 | 6040 | 10000 | 6040 | 9430 |
| Stdev | 7380 | 7850 | 7380 | 8360 | 7380 | 7740 |
| p(t-test) | | 0.11 | | 0.023 | | 0.060 |
| Min | 3.36 | 504 | 3.36 | 658 | 3.36 | 177 |
| Max | 80000 | 24700 | 80000 | 26100 | 80000 | 23800 |
| n (Samp) | 1225 | 15 | 1225 | 18 | 1225 | 17 |
| n (Patient) | 438 | 15 | 438 | 18 | 438 | 17 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 3060 | 6850 | 3060 | 8300 | 3060 | 5080 |
| Average | 5550 | 9370 | 5550 | 11400 | 5550 | 7220 |
| Stdev | 6860 | 7890 | 6860 | 12000 | 6860 | 6330 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 6.2E-5 | | 2.1E-9 | | 0.16 |
| Min | 33.0 | 447 | 33.0 | 634 | 33.0 | 55.9 |
| Max | 80000 | 35700 | 80000 | 80000 | 80000 | 25300 |
| n (Samp) | 810 | 57 | 810 | 63 | 810 | 34 |
| n (Patient) | 280 | 57 | 280 | 63 | 280 | 34 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.66 | 0.64 | 0.69 | 0.67 | 0.61 | 0.71 | 0.55 | 0.60 | 0.61 |
| SE | 0.039 | 0.078 | 0.040 | 0.036 | 0.071 | 0.038 | 0.048 | 0.073 | 0.052 |
| p | 3.4E-5 | 0.068 | 3.2E-6 | 2.5E-6 | 0.11 | 2.0E-8 | 0.35 | 0.17 | 0.031 |
| nCohort 1 | 922 | 1225 | 810 | 922 | 1225 | 810 | 922 | 1225 | 810 |
| nCohort 2 | 62 | 15 | 57 | 70 | 18 | 63 | 39 | 17 | 34 |
| Cutoff 1 | 3550 | 3550 | 4050 | 4350 | 2320 | 4540 | 1740 | 1740 | 2930 |
| Sens 1 | 71% | 73% | 70% | 70% | 72% | 71% | 72% | 71% | 71% |
| Spec 1 | 57% | 52% | 61% | 64% | 34% | 65% | 27% | 25% | 48% |
| Cutoff 2 | 2720 | 2920 | 2720 | 2010 | 1090 | 2630 | 1390 | 831 | 1740 |
| Sens 2 | 81% | 80% | 81% | 80% | 83% | 81% | 82% | 82% | 82% |
| Spec 2 | 45% | 43% | 46% | 33% | 13% | 44% | 20% | 9% | 28% |
| Cutoff 3 | 1130 | 933 | 1880 | 1190 | 673 | 1600 | 541 | 541 | 1390 |
| Sens 3 | 90% | 93% | 91% | 90% | 94% | 90% | 92% | 94% | 91% |
| Spec 3 | 15% | 11% | 30% | 16% | 6% | 24% | 5% | 5% | 21% |
| Cutoff 4 | 5430 | 6270 | 5590 | 5430 | 6270 | 5590 | 5430 | 6270 | 5590 |
| Sens 4 | 52% | 47% | 58% | 60% | 56% | 62% | 44% | 59% | 47% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 4 | 70% | 70% | 70% | 70% | 70% | 70% | 70% | 70% | 70% |
| Cutoff 5 | 8100 | 8950 | 8470 | 8100 | 8950 | 8470 | 8100 | 8950 | 8470 |
| Sens 5 | 42% | 33% | 44% | 49% | 56% | 49% | 31% | 53% | 32% |
| Spec 5 | 80% | 80% | 80% | 80% | 80% | 80% | 80% | 80% | 80% |
| Cutoff 6 | 13100 | 14200 | 13400 | 13100 | 14200 | 13400 | 13100 | 14200 | 13400 |
| Sens 6 | 18% | 27% | 23% | 31% | 44% | 32% | 18% | 29% | 21% |
| Spec 6 | 90% | 90% | 90% | 90% | 90% | 90% | 90% | 90% | 90% |
| OR Quart 2 | 1.4 | 1.0 | 1.8 | 0.57 | 1.00 | 0.62 | 0.69 | 0.20 | 0.83 |
| p Value | 0.46 | 1.0 | 0.29 | 0.25 | 1.00 | 0.40 | 0.46 | 0.14 | 0.76 |
| 95% CI of | 0.54 | 0.14 | 0.60 | 0.22 | 0.25 | 0.20 | 0.26 | 0.023 | 0.25 |
| OR Quart2 | 3.9 | 7.1 | 5.5 | 1.5 | 4.0 | 1.9 | 1.8 | 1.7 | 2.8 |
| OR Quart 3 | 2.7 | 2.5 | 3.1 | 1.2 | 0 | 1.9 | 0.90 | 0.20 | 1.9 |
| p Value | 0.029 | 0.27 | 0.030 | 0.69 | na | 0.14 | 0.81 | 0.14 | 0.22 |
| 95% CI of | 1.1 | 0.49 | 1.1 | 0.53 | na | 0.80 | 0.36 | 0.023 | 0.68 |
| OR Quart3 | 6.6 | 13 | 8.8 | 2.6 | na | 4.7 | 2.2 | 1.7 | 5.2 |
| OR Quart 4 | 4.2 | 3.0 | 6.3 | 3.4 | 2.5 | 5.0 | 1.3 | 2.0 | 2.1 |
| p Value | 9.4E-4 | 0.18 | 2.2E-4 | 3.4E-4 | 0.12 | 7.1E-5 | 0.53 | 0.20 | 0.16 |
| 95% CI of | 1.8 | 0.61 | 2.4 | 1.8 | 0.79 | 2.3 | 0.56 | 0.68 | 0.76 |
| OR Quart4 | 9.9 | 15 | 17 | 6.8 | 8.2 | 11 | 3.1 | 6.0 | 5.6 |

**Immunoglogulin G2**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 8730 | 13800 | 8730 | 16800 | 8730 | 8880 |
| Average | 16700 | 27200 | 16700 | 38600 | 16700 | 23800 |

**Immunoglogulin G2**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | 28100 | 43200 | 28100 | 60500 | 28100 | 43900 |
| p(t-test) | | 0.0063 | | 2.5E-8 | | 0.13 |
| Min | 119 | 810 | 119 | 1870 | 119 | 25.4 |
| Max | 240000 | 240000 | 240000 | 240000 | 240000 | 240000 |
| n (Samp) | 922 | 62 | 922 | 70 | 922 | 39 |
| n (Patient) | 358 | 62 | 358 | 70 | 358 | 39 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 9310 | 11800 | 9310 | 16700 | 9310 | 40000 |
| Average | 18900 | 26000 | 18900 | 39600 | 18900 | 50100 |
| Stdev | 33500 | 44100 | 33500 | 59100 | 33500 | 64700 |
| p(t-test) | | 0.41 | | 0.010 | | 1.8E-4 |
| Min | 119 | 3150 | 119 | 3190 | 119 | 1310 |
| Max | 240000 | 182000 | 240000 | 240000 | 240000 | 240000 |
| n (Samp) | 1225 | 15 | 1225 | 18 | 1225 | 17 |
| n (Patient) | 438 | 15 | 438 | 18 | 438 | 17 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 9320 | 14200 | 9320 | 18200 | 9320 | 9690 |
| Average | 17600 | 34200 | 17600 | 42800 | 17600 | 23600 |
| Stdev | 28600 | 53700 | 28600 | 63300 | 28600 | 43000 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 9.3E-5 | | 3.9E-9 | | 0.24 |
| Min | 334 | 810 | 334 | 1870 | 334 | 25.4 |
| Max | 240000 | 240000 | 240000 | 240000 | 240000 | 240000 |
| n (Samp) | 810 | 57 | 810 | 63 | 810 | 34 |
| n (Patient) | 280 | 57 | 280 | 63 | 280 | 34 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.63 | 0.61 | 0.63 | 0.65 | 0.62 | 0.67 | 0.51 | 0.63 | 0.54 |
| SE | 0.039 | 0.078 | 0.041 | 0.037 | 0.071 | 0.038 | 0.047 | 0.073 | 0.052 |
| p | 8.5E-4 | 0.15 | 9.9E-4 | 3.0E-5 | 0.090 | 1.2E-5 | 0.90 | 0.075 | 0.44 |
| nCohort 1 | 922 | 1225 | 810 | 922 | 1225 | 810 | 922 | 1225 | 810 |
| nCohort 2 | 62 | 15 | 57 | 70 | 18 | 63 | 39 | 17 | 34 |
| Cutoff 1 | 8220 | 8900 | 8240 | 8630 | 6340 | 9450 | 5240 | 6690 | 8010 |
| Sens 1 | 71% | 73% | 70% | 70% | 72% | 71% | 72% | 71% | 71% |
| Spec 1 | 47% | 48% | 45% | 50% | 33% | 51% | 28% | 36% | 44% |
| Cutoff 2 | 6900 | 8400 | 6900 | 5620 | 4630 | 6460 | 4240 | 4240 | 4620 |
| Sens 2 | 81% | 80% | 81% | 80% | 83% | 81% | 82% | 82% | 82% |
| Spec 2 | 39% | 45% | 38% | 31% | 22% | 35% | 21% | 20% | 21% |
| Cutoff 3 | 4910 | 5870 | 4400 | 3990 | 3980 | 4130 | 1800 | 1800 | 2270 |
| Sens 3 | 90% | 93% | 91% | 90% | 94% | 90% | 92% | 94% | 91% |
| Spec 3 | 25% | 30% | 20% | 20% | 18% | 19% | 6% | 5% | 8% |
| Cutoff 4 | 14400 | 15700 | 14900 | 14400 | 15700 | 14900 | 14400 | 15700 | 14900 |
| Sens 4 | 45% | 40% | 47% | 53% | 50% | 54% | 28% | 59% | 32% |

EP 3 489 688 A1

116

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 4 | 70% | 70% | 70% | 70% | 70% | 70% | 70% | 70% | 70% |
| Cutoff 5 | 19700 | 21200 | 20100 | 19700 | 21200 | 20100 | 19700 | 21200 | 20100 |
| Sens 5 | 44% | 33% | 40% | 43% | 44% | 43% | 23% | 59% | 26% |
| Spec 5 | 80% | 80% | 80% | 80% | 80% | 80% | 80% | 80% | 80% |
| Cutoff 6 | 32100 | 37400 | 35300 | 32100 | 37400 | 35300 | 32100 | 37400 | 35300 |
| Sens 6 | 19% | 7% | 25% | 31% | 28% | 30% | 18% | 53% | 15% |
| Spec 6 | 90% | 90% | 90% | 90% | 90% | 90% | 90% | 90% | 90% |
| OR Quart 2 | 2.5 | 5.1 | 1.3 | 0.91 | 0.75 | 1.1 | 0.63 | 0.75 | 1.0 |
| p Value | 0.043 | 0.14 | 0.51 | 0.83 | 0.70 | 0.81 | 0.34 | 0.70 | 1.0 |
| 95% CI of | 1.0 | 0.59 | 0.56 | 0.39 | 0.17 | 0.44 | 0.24 | 0.17 | 0.37 |
| OR Quart2 | 6.2 | 44 | 3.3 | 2.1 | 3.4 | 2.8 | 1.6 | 3.4 | 2.7 |
| OR Quart 3 | 1.6 | 3.0 | 1.1 | 1.1 | 0.50 | 1.5 | 1.1 | 0 | 1.1 |
| p Value | 0.34 | 0.34 | 0.82 | 0.84 | 0.42 | 0.38 | 0.83 | na | 0.80 |
| 95% CI of | 0.61 | 0.31 | 0.44 | 0.49 | 0.090 | 0.62 | 0.47 | na | 0.43 |
| OR Quart3 | 4.2 | 29 | 2.8 | 2.4 | 2.7 | 3.5 | 2.5 | na | 3.0 |
| OR Quart 4 | 4.2 | 6.1 | 3.1 | 3.1 | 2.3 | 3.8 | 0.81 | 2.5 | 1.1 |
| p Value | 9.4E-4 | 0.095 | 0.0043 | 0.0011 | 0.17 | 6.1E-4 | 0.64 | 0.12 | 0.80 |
| 95% CI of | 1.8 | 0.73 | 1.4 | 1.6 | 0.69 | 1.8 | 0.33 | 0.79 | 0.43 |
| OR Quart4 | 9.9 | 51 | 6.9 | 6.2 | 7.5 | 8.3 | 2.0 | 8.2 | 3.0 |

**Interleukin-11**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 129 | 241 | 129 | 212 | 129 | 120 |
| Average | 209 | 341 | 209 | 288 | 209 | 207 |

| Interleukin-11 | | | | | | | |
|---|---|---|---|---|---|---|---|
| sCr or UO | 0hr prior to AKI stage | | 24hr prior to AKI stage | | 48hr prior to AKI stage | | |
| | Cohort 1 | Cohort 2 | Cohort 1 | Cohort 2 | Cohort 1 | Cohort 2 | |
| Stdev | 243 | 387 | 243 | 334 | 243 | 338 | |
| p(t-test) | | 8.2E-5 | | 0.012 | | 0.97 | |
| Min | 0.0822 | 21.2 | 0.0822 | 28.3 | 0.0822 | 14.5 | |
| Max | 2260 | 2900 | 2260 | 2590 | 2260 | 2140 | |
| n (Samp) | 929 | 62 | 929 | 69 | 929 | 39 | |
| n (Patient) | 361 | 62 | 361 | 69 | 361 | 39 | |
| sCr only | 0hr prior to AKI stage | | 24hr prior to AKI stage | | 48hr prior to AKI stage | | |
| | Cohort 1 | Cohort 2 | Cohort 1 | | Cohort 2 | Cohort 1 | Cohort 2 |
| Median | 135 | 158 | 135 | | 224 | 135 | 174 |
| Average | 231 | 256 | 231 | | 258 | 231 | 192 |
| Stdev | 303 | 226 | 303 | | 144 | 303 | 127 |
| p(t-test) | | 0.75 | | | 0.71 | | 0.59 |
| Min | 0.0822 | 21.2 | 0.0822 | | 32.3 | 0.0822 | 35.3 |
| Max | 3780 | 830 | 3780 | | 547 | 3780 | 549 |
| n (Samp) | 1232 | 15 | 1232 | | 18 | 1232 | 17 |
| n (Patient) | 441 | 15 | 441 | | 18 | 441 | 17 |
| | | | | | | | |
| UO only | 0hr prior to AKI stage | | 24hr prior to AKI stage | | 48hr prior to AKI stage | | |
| | Cohort 1 | Cohort 2 | Cohort 1 | | Cohort 2 | Cohort 1 | Cohort 2 |
| Median | 139 | 285 | 139 | | 210 | 139 | 121 |
| Average | 217 | 374 | 217 | | 307 | 217 | 221 |
| Stdev | 246 | 416 | 246 | | 374 | 246 | 360 |
| p(t-test) | | 1.2E-5 | | | 0.0083 | | 0.94 |

EP 3 489 688 A1

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Min | 0.0822 | 27.2 | 0.0822 | 28.3 | 0.0822 | 14.5 |
| Max | 2260 | 2900 | 2260 | 2590 | 2260 | 2140 |
| n (Samp) | 817 | 57 | 817 | 62 | 817 | 34 |
| n (Patient) | 283 | 57 | 283 | 62 | 283 | 34 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.68 | 0.58 | 0.69 | 0.64 | 0.65 | 0.62 | 0.50 | 0.54 | 0.49 |
| SE | 0.038 | 0.078 | 0.040 | 0.037 | 0.071 | 0.039 | 0.047 | 0.072 | 0.051 |
| p | 2.4E-6 | 0.33 | 2.1E-6 | 1.6E-4 | 0.033 | 0.0015 | 0.95 | 0.55 | 0.79 |
| nCohort 1 | 929 | 1232 | 817 | 929 | 1232 | 817 | 929 | 1232 | 817 |
| nCohort 2 | 62 | 15 | 57 | 69 | 18 | 62 | 39 | 17 | 34 |
| Cutoff 1 | 149 | 136 | 158 | 123 | 182 | 123 | 88.1 | 135 | 88.1 |
| Sens 1 | 71% | 73% | 70% | 71% | 72% | 71% | 72% | 71% | 71% |
| Spec 1 | 57% | 50% | 56% | 49% | 62% | 45% | 35% | 50% | 31% |
| Cutoff 2 | 119 | 87.1 | 128 | 101 | 105 | 101 | 71.2 | 93.8 | 74.7 |
| Sens 2 | 81% | 80% | 81% | 81% | 83% | 81% | 82% | 82% | 82% |
| Spec 2 | 47% | 32% | 46% | 40% | 39% | 37% | 26% | 35% | 24% |
| Cutoff 3 | 79.8 | 63.1 | 85.3 | 76.3 | 89.4 | 79.6 | 54.9 | 41.4 | 62.9 |
| Sens 3 | 90% | 93% | 91% | 91% | 94% | 90% | 92% | 94% | 91% |
| Spec 3 | 31% | 20% | 29% | 28% | 33% | 28% | 18% | 10% | 19% |
| Cutoff 4 | 212 | 230 | 230 | 212 | 230 | 230 | 212 | 230 | 230 |
| Sens 4 | 55% | 33% | 54% | 51% | 44% | 45% | 26% | 29% | 26% |
| Spec 4 | 70% | 70% | 70% | 70% | 70% | 70% | 70% | 70% | 70% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 5 | 301 | 328 | 307 | 301 | 328 | 307 | 301 | 328 | 307 |
| Sens 5 | 42% | 33% | 44% | 33% | 33% | 34% | 13% | 12% | 15% |
| Spec 5 | 80% | 80% | 80% | 80% | 80% | 80% | 80% | 80% | 80% |
| Cutoff 6 | 461 | 509 | 460 | 461 | 509 | 460 | 461 | 509 | 460 |
| Sens 6 | 26% | 13% | 28% | 13% | 6% | 15% | 5% | 6% | 6% |
| Spec 6 | 90% | 90% | 90% | 90% | 90% | 90% | 90% | 90% | 90% |
| OR Quart 2 | 2.8 | 1.00 | 4.2 | 2.6 | 3.0 | 2.8 | 1.6 | 1.0 | 1.3 |
| p Value | 0.079 | 1.00 | 0.029 | 0.053 | 0.34 | 0.036 | 0.34 | 1.0 | 0.59 |
| 95% CI of | 0.89 | 0.14 | 1.2 | 0.99 | 0.31 | 1.1 | 0.61 | 0.20 | 0.46 |
| OR Quart2 | 9.0 | 7.1 | 15 | 6.8 | 29 | 7.3 | 4.2 | 5.0 | 3.9 |
| OR Quart 3 | 4.8 | 3.0 | 4.5 | 3.7 | 8.2 | 3.0 | 2.1 | 2.4 | 2.2 |
| p Value | 0.0054 | 0.18 | 0.019 | 0.0053 | 0.048 | 0.025 | 0.13 | 0.22 | 0.11 |
| 95% CI of | 1.6 | 0.61 | 1.3 | 1.5 | 1.0 | 1.1 | 0.82 | 0.61 | 0.84 |
| OR Quart3 | 14 | 15 | 16 | 9.4 | 66 | 7.7 | 5.2 | 9.2 | 6.0 |
| OR Quart 4 | 8.0 | 2.5 | 11 | 4.9 | 6.1 | 4.1 | 1.0 | 1.3 | 1.2 |
| p Value | 1.2E-4 | 0.27 | 9.9E-5 | 5.6E-4 | 0.096 | 0.0024 | 1.0 | 0.71 | 0.77 |
| 95% CI of | 2.8 | 0.48 | 3.3 | 2.0 | 0.73 | 1.7 | 0.35 | 0.30 | 0.39 |
| OR Quart4 | 23 | 13 | 36 | 12 | 51 | 10 | 2.9 | 6.0 | 3.6 |

**Interleukin-2 receptor alpha chain**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 554 | 923 | 554 | 868 | 554 | 524 |
| Average | 909 | 1440 | 909 | 1430 | 909 | 1190 |
| Stdev | 1090 | 1520 | 1090 | 1780 | 1090 | 1700 |

| Interleukin-2 receptor alpha chain | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| p(t-test) | | 3.1E-4 | | 3.0E-4 | | 0.12 |
| Min | 0.0317 | 0.0482 | 0.0317 | 0.0482 | 0.0317 | 0.0383 |
| Max | 10400 | 6080 | 10400 | 9090 | 10400 | 7740 |
| n (Samp) | 930 | 62 | 930 | 70 | 930 | 39 |
| n (Patient) | 361 | 62 | 361 | 70 | 361 | 39 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 621 | 499 | 621 | 957 | 621 | 421 |
| Average | 1100 | 1150 | 1100 | 1680 | 1100 | 881 |
| Stdev | 1420 | 1520 | 1420 | 1930 | 1420 | 923 |
| p(t-test) | | 0.89 | | 0.086 | | 0.54 |
| Min | 0.0317 | 0.0482 | 0.0317 | 0.0482 | 0.0317 | 51.4 |
| Max | 10400 | 6080 | 10400 | 7190 | 10400 | 2590 |
| n (Samp) | 1234 | 15 | 1234 | 18 | 1234 | 17 |
| n (Patient) | 441 | 15 | 441 | 18 | 441 | 17 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 563 | 1080 | 563 | 911 | 563 | 728 |
| Average | 927 | 1640 | 927 | 1570 | 927 | 1380 |
| Stdev | 1110 | 1540 | 1110 | 1840 | 1110 | 1780 |
| p(t-test) | | 5.2E-6 | | 2.7E-5 | | 0.022 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Min | 0.0317 | 0.249 | 0.0317 | 0.0632 | 0.0317 | 0.0383 |
| Max | 10400 | 6080 | 10400 | 9090 | 10400 | 7740 |
| n (Samp) | 819 | 57 | 819 | 63 | 819 | 34 |
| n (Patient) | 283 | 57 | 283 | 63 | 283 | 34 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.61 | 0.51 | 0.66 | 0.60 | 0.58 | 0.62 | 0.51 | 0.48 | 0.55 |
| SE | 0.039 | 0.076 | 0.040 | 0.037 | 0.071 | 0.039 | 0.048 | 0.071 | 0.052 |
| p | 0.0042 | 0.85 | 1.0E-4 | 0.0093 | 0.24 | 0.0014 | 0.78 | 0.75 | 0.31 |
| nCohort 1 | 930 | 1234 | 819 | 930 | 1234 | 819 | 930 | 1234 | 819 |
| nCohort 2 | 62 | 15 | 57 | 70 | 18 | 63 | 39 | 17 | 34 |
| Cutoff 1 | 446 | 411 | 528 | 417 | 373 | 466 | 226 | 226 | 333 |
| Sens 1 | 71% | 73% | 70% | 70% | 72% | 71% | 72% | 71% | 71% |
| Spec 1 | 45% | 40% | 48% | 44% | 37% | 45% | 31% | 27% | 36% |
| Cutoff 2 | 278 | 277 | 368 | 272 | 235 | 318 | 135 | 135 | 189 |
| Sens 2 | 81% | 80% | 81% | 80% | 83% | 81% | 82% | 82% | 82% |
| Spec 2 | 35% | 31% | 39% | 34% | 28% | 35% | 21% | 18% | 26% |
| Cutoff 3 | 112 | 21.0 | 167 | 109 | 55.0 | 152 | 35.7 | 66.8 | 35.7 |
| Sens 3 | 90% | 93% | 91% | 90% | 94% | 90% | 92% | 94% | 91% |
| Spec 3 | 18% | 8% | 24% | 17% | 11% | 23% | 11% | 12% | 10% |
| Cutoff 4 | 1050 | 1150 | 1060 | 1050 | 1150 | 1060 | 1050 | 1150 | 1060 |
| Sens 4 | 44% | 40% | 51% | 39% | 50% | 41% | 28% | 24% | 35% |
| Spec 4 | 70% | 70% | 70% | 70% | 70% | 70% | 70% | 70% | 70% |

EP 3 489 688 A1

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 5 | 1490 | 1740 | 1500 | 1490 | 1740 | 1500 | 1490 | 1740 | 1500 |
| Sens 5 | 34% | 13% | 40% | 31% | 33% | 35% | 21% | 24% | 29% |
| Spec 5 | 80% | 80% | 80% | 80% | 80% | 80% | 80% | 80% | 80% |
| Cutoff 6 | 2250 | 2620 | 2290 | 2250 | 2620 | 2290 | 2250 | 2620 | 2290 |
| Sens 6 | 23% | 7% | 28% | 17% | 17% | 21% | 18% | 0% | 21% |
| Spec 6 | 90% | 90% | 90% | 90% | 90% | 90% | 90% | 90% | 90% |
| OR Quart 2 | 2.3 | 1.7 | 2.2 | 1.9 | 1.3 | 2.4 | 1.2 | 0.75 | 1.7 |
| p Value | 0.050 | 0.48 | 0.11 | 0.12 | 0.70 | 0.062 | 0.65 | 0.70 | 0.31 |
| 95% CI of | 1.0 | 0.40 | 0.84 | 0.84 | 0.30 | 0.96 | 0.50 | 0.17 | 0.61 |
| OR Quart2 | 5.5 | 7.1 | 6.0 | 4.1 | 6.0 | 5.9 | 3.0 | 3.4 | 4.8 |
| OR Quart 3 | 1.7 | 0.66 | 2.2 | 2.0 | 1.0 | 2.7 | 1.0 | 1.5 | 1.0 |
| p Value | 0.27 | 0.66 | 0.11 | 0.090 | 1.0 | 0.029 | 1.0 | 0.53 | 1.0 |
| 95% CI of | 0.68 | 0.11 | 0.84 | 0.90 | 0.20 | 1.1 | 0.39 | 0.42 | 0.32 |
| OR Quart3 | 4.1 | 4.0 | 6.0 | 4.3 | 5.0 | 6.6 | 2.6 | 5.4 | 3.2 |
| OR Quart 4 | 3.1 | 1.7 | 4.6 | 2.4 | 2.7 | 3.4 | 1.1 | 1.0 | 2.0 |
| p Value | 0.0077 | 0.48 | 0.0011 | 0.023 | 0.14 | 0.0064 | 0.82 | 1.00 | 0.16 |
| 95% CI of | 1.3 | 0.40 | 1.8 | 1.1 | 0.71 | 1.4 | 0.44 | 0.25 | 0.75 |
| OR Quart4 | 7.0 | 7.1 | 11 | 5.2 | 10 | 8.0 | 2.8 | 4.0 | 5.6 |

**Neutrophil collagenase**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3070 | 6050 | 3070 | 8280 | 3070 | 3260 |
| Average | 13400 | 18500 | 13400 | 34000 | 13400 | 9350 |
| Stdev | 38800 | 30300 | 38800 | 83200 | 38800 | 15200 |

EP 3 489 688 A1

(continued)

| Neutrophil collagenase | | | | | | | |
|---|---|---|---|---|---|---|---|
| sCr or UO | 0hr prior to AKI stage | | 24hr prior to AKI stage | | | 48hr prior to AKI stage | |
| | Cohort 1 | Cohort 2 | Cohort 1 | Cohort 2 | | Cohort 1 | Cohort 2 |
| p(t-test) | | 0.29 | | 5.1E-5 | | | 0.48 |
| Min | 0.114 | 0.159 | 0.114 | 25.8 | | 0.114 | 111 |
| Max | 670000 | 136000 | 670000 | 670000 | | 670000 | 66900 |
| n (Samp) | 1005 | 69 | 1005 | 80 | | 1005 | 45 |
| n (Patient) | 387 | 69 | 387 | 80 | | 387 | 45 |
| sCr only | 0hr prior to AKI stage | | 24hr prior to AKI stage | | | 48hr prior to AKI stage | |
| | Cohort 1 | Cohort 2 | Cohort 1 | | Cohort 2 | Cohort 1 | Cohort 2 |
| Median | 3570 | 4670 | 3570 | | 6260 | 3570 | 5710 |
| Average | 14700 | 20300 | 14700 | | 69300 | 14700 | 17200 |
| Stdev | 41000 | 40500 | 41000 | | 157000 | 41000 | 27500 |
| p(t-test) | | 0.59 | | | 4.2E-8 | | 0.79 |
| Min | 0.114 | 0.260 | 0.114 | | 25.8 | 0.114 | 111 |
| Max | 670000 | 128000 | 670000 | | 625000 | 670000 | 115000 |
| n (Samp) | 1339 | 16 | 1339 | | 21 | 1339 | 19 |
| n (Patient) | 473 | 16 | 473 | | 21 | 473 | 19 |
| | | | | | | | |
| UO only | 0hr prior to AKI stage | | 24hr prior to AKI stage | | | 48hr prior to AKI stage | |
| | Cohort 1 | Cohort 2 | Cohort 1 | | Cohort 2 | Cohort 1 | Cohort 2 |
| Median | 3120 | 7870 | 3120 | | 10400 | 3120 | 3470 |
| Average | 13700 | 31600 | 13700 | | 42800 | 13700 | 10000 |
| Stdev | 35000 | 78300 | 35000 | | 94000 | 35000 | 15600 |
| p(t-test) | | 4.5E-4 | | | 2.0E-8 | | 0.51 |
| Min | 0.114 | 0.159 | 0.114 | | 0.260 | 0.114 | 25.8 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 365000 | 561000 | 365000 | 670000 | 365000 | 66900 |
| n (Samp) | 896 | 64 | 896 | 73 | 896 | 40 |
| n (Patient) | 311 | 64 | 311 | 73 | 311 | 40 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.64 | 0.52 | 0.67 | 0.66 | 0.57 | 0.70 | 0.50 | 0.57 | 0.52 |
| SE | 0.037 | 0.074 | 0.038 | 0.034 | 0.065 | 0.035 | 0.044 | 0.069 | 0.047 |
| p | 1.9E-4 | 0.75 | 1.2E-5 | 2.1E-6 | 0.32 | 2.4E-8 | 0.92 | 0.34 | 0.70 |
| nCohort 1 | 1005 | 1339 | 896 | 1005 | 1339 | 896 | 1005 | 1339 | 896 |
| nCohort 2 | 69 | 16 | 64 | 80 | 21 | 73 | 45 | 19 | 40 |
| Cutoff 1 | 3000 | 1060 | 3260 | 3320 | 1020 | 4640 | 1270 | 2660 | 1680 |
| Sens 1 | 71% | 75% | 70% | 70% | 71% | 71% | 71% | 74% | 70% |
| Spec 1 | 50% | 23% | 51% | 52% | 22% | 59% | 30% | 43% | 36% |
| Cutoff 2 | 2400 | 1050 | 2830 | 2190 | 978 | 3080 | 742 | 751 | 813 |
| Sens 2 | 81% | 81% | 81% | 80% | 81% | 81% | 80% | 84% | 80% |
| Spec 2 | 45% | 23% | 48% | 43% | 22% | 50% | 20% | 18% | 21% |
| Cutoff 3 | 1050 | 358 | 1720 | 962 | 570 | 1550 | 387 | 191 | 571 |
| Sens 3 | 91% | 94% | 91% | 90% | 90% | 90% | 91% | 95% | 90% |
| Spec 3 | 25% | 9% | 36% | 24% | 14% | 34% | 12% | 4% | 16% |
| Cutoff 4 | 7950 | 9120 | 7860 | 7950 | 9120 | 7860 | 7950 | 9120 | 7860 |
| Sens 4 | 45% | 38% | 50% | 52% | 43% | 58% | 27% | 47% | 30% |
| Spec 4 | 70% | 70% | 70% | 70% | 70% | 70% | 70% | 70% | 70% |
| Cutoff 5 | 13800 | 15700 | 13900 | 13800 | 15700 | 13900 | 13800 | 15700 | 13900 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 5 | 33% | 19% | 36% | 39% | 33% | 41% | 20% | 32% | 22% |
| Spec 5 | 80% | 80% | 80% | 80% | 80% | 80% | 80% | 80% | 80% |
| Cutoff 6 | 29000 | 32400 | 30900 | 29000 | 32400 | 30900 | 29000 | 32400 | 30900 |
| Sens 6 | 17% | 12% | 19% | 26% | 24% | 29% | 9% | 16% | 10% |
| Spec 6 | 90% | 90% | 90% | 90% | 90% | 90% | 90% | 90% | 90% |
| OR Quart 2 | 2.4 | 0.40 | 4.2 | 1.2 | 0.28 | 2.2 | 0.92 | 0.59 | 1.0 |
| p Value | 0.063 | 0.27 | 0.011 | 0.68 | 0.12 | 0.11 | 0.84 | 0.48 | 1.0 |
| 95% CI of | 0.95 | 0.076 | 1.4 | 0.52 | 0.058 | 0.83 | 0.40 | 0.14 | 0.39 |
| OR Quart2 | 5.8 | 2.1 | 13 | 2.7 | 1.4 | 6.0 | 2.1 | 2.5 | 2.6 |
| OR Quart 3 | 2.7 | 0.80 | 4.2 | 1.9 | 0.42 | 3.4 | 0.83 | 0.80 | 1.1 |
| p Value | 0.030 | 0.73 | 0.011 | 0.10 | 0.22 | 0.011 | 0.66 | 0.74 | 0.81 |
| 95% CI of | 1.1 | 0.21 | 1.4 | 0.88 | 0.11 | 1.3 | 0.35 | 0.21 | 0.45 |
| OR Quart3 | 6.5 | 3.0 | 13 | 4.0 | 1.7 | 8.5 | 1.9 | 3.0 | 2.8 |
| OR Quart 4 | 4.3 | 1.00 | 7.8 | 3.6 | 1.3 | 6.6 | 1.0 | 1.4 | 1.4 |
| p Value | 6.9E-4 | 1.00 | 1.6E-4 | 3.2E-4 | 0.61 | 2.9E-5 | 0.99 | 0.57 | 0.50 |
| 95% CI of | 1.9 | 0.29 | 2.7 | 1.8 | 0.48 | 2.7 | 0.44 | 0.44 | 0.56 |
| OR Quart4 | 10 | 3.5 | 23 | 7.2 | 3.5 | 16 | 2.3 | 4.5 | 3.3 |

**Protransforming growth factor alpha**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5.81 | 8.51 | 5.81 | 10.3 | 5.81 | 6.15 |
| Average | 10.7 | 12.5 | 10.7 | 16.0 | 10.7 | 9.77 |
| Stdev | 22.4 | 12.1 | 22.4 | 19.8 | 22.4 | 10.6 |
| p(t-test) | | 0.54 | | 0.057 | | 0.80 |

126

EP 3 489 688 A1

**Protransforming growth factor alpha**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Min | 0.00184 | 0.00603 | 0.00184 | 0.00603 | 0.00184 | 0.00454 |
| Max | 361 | 58.8 | 361 | 135 | 361 | 52.3 |
| n (Samp) | 925 | 62 | 925 | 69 | 925 | 39 |
| n (Patient) | 361 | 62 | 361 | 69 | 361 | 39 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 6.55 | 12.3 | 6.55 | 13.2 | 6.55 | 6.54 |
| Average | 11.4 | 11.8 | 11.4 | 16.9 | 11.4 | 10.6 |
| Stdev | 21.7 | 6.57 | 21.7 | 16.6 | 21.7 | 11.4 |
| p(t-test) | | 0.93 | | 0.28 | | 0.89 |
| Min | 0.00184 | 0.00603 | 0.00184 | 0.00603 | 0.00184 | 0.00574 |
| Max | 361 | 27.7 | 361 | 59.6 | 361 | 45.2 |
| n (Samp) | 1228 | 15 | 1228 | 18 | 1228 | 17 |
| n (Patient) | 441 | 15 | 441 | 18 | 441 | 17 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 5.87 | 8.48 | 5.87 | 10.9 | 5.87 | 6.31 |
| Average | 11.1 | 13.7 | 11.1 | 16.6 | 11.1 | 10.8 |
| Stdev | 23.7 | 14.0 | 23.7 | 19.7 | 23.7 | 11.4 |
| p(t-test) | | 0.42 | | 0.075 | | 0.94 |
| Min | 0.00184 | 0.0114 | 0.00184 | 0.781 | 0.00184 | 0.00454 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 361 | 63.1 | 361 | 135 | 361 | 52.3 |
| n (Samp) | 814 | 57 | 814 | 62 | 814 | 34 |
| n (Patient) | 283 | 57 | 283 | 62 | 283 | 34 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.62 | 0.66 | 0.63 | 0.65 | 0.63 | 0.67 | 0.53 | 0.53 | 0.54 |
| SE | 0.039 | 0.077 | 0.041 | 0.037 | 0.071 | 0.039 | 0.048 | 0.072 | 0.052 |
| p | 0.0018 | 0.035 | 0.0012 | 8.3E-5 | 0.071 | 6.1E-6 | 0.57 | 0.71 | 0.40 |
| nCohort 1 | 925 | 1228 | 814 | 925 | 1228 | 814 | 925 | 1228 | 814 |
| nCohort 2 | 62 | 15 | 57 | 69 | 18 | 62 | 39 | 17 | 34 |
| Cutoff 1 | 6.53 | 9.29 | 6.56 | 6.12 | 4.91 | 7.51 | 3.64 | 5.14 | 3.71 |
| Sens 1 | 71% | 73% | 70% | 71% | 72% | 71% | 72% | 71% | 71% |
| Spec 1 | 54% | 66% | 54% | 51% | 39% | 61% | 31% | 41% | 31% |
| Cutoff 2 | 5.18 | 7.12 | 5.42 | 4.16 | 3.53 | 5.57 | 2.57 | 2.47 | 3.00 |
| Sens 2 | 81% | 80% | 81% | 81% | 83% | 81% | 82% | 82% | 82% |
| Spec 2 | 45% | 54% | 47% | 36% | 28% | 48% | 21% | 18% | 25% |
| Cutoff 3 | 2.06 | 3.69 | 2.04 | 2.10 | 2.84 | 2.37 | 1.71 | 1.85 | 0.530 |
| Sens 3 | 90% | 93% | 91% | 91% | 94% | 90% | 92% | 94% | 91% |
| Spec 3 | 17% | 29% | 16% | 17% | 22% | 19% | 14% | 14% | 5% |
| Cutoff 4 | 9.53 | 10.3 | 9.60 | 9.53 | 10.3 | 9.60 | 9.53 | 10.3 | 9.60 |
| Sens 4 | 42% | 60% | 44% | 55% | 56% | 58% | 41% | 29% | 44% |
| Spec 4 | 70% | 70% | 70% | 70% | 70% | 70% | 70% | 70% | 70% |
| Cutoff 5 | 12.4 | 14.0 | 12.4 | 12.4 | 14.0 | 12.4 | 12.4 | 14.0 | 12.4 |

EP 3 489 688 A1

128

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 5 | 34% | 40% | 37% | 39% | 44% | 42% | 28% | 24% | 32% |
| Spec 5 | 80% | 80% | 80% | 80% | 80% | 80% | 80% | 80% | 80% |
| Cutoff 6 | 20.3 | 23.5 | 21.6 | 20.3 | 23.5 | 21.6 | 20.3 | 23.5 | 21.6 |
| Sens 6 | 16% | 7% | 16% | 20% | 28% | 21% | 10% | 12% | 12% |
| Spec 6 | 90% | 90% | 90% | 90% | 90% | 90% | 90% | 90% | 90% |
| OR Quart 2 | 1.00 | 2.0 | 1.00 | 1.3 | 1.7 | 1.4 | 1.3 | 1.3 | 1.3 |
| p Value | 0.99 | 0.57 | 0.99 | 0.51 | 0.48 | 0.46 | 0.63 | 0.74 | 0.61 |
| 95% CI of | 0.37 | 0.18 | 0.34 | 0.56 | 0.40 | 0.54 | 0.49 | 0.33 | 0.47 |
| OR Quart2 | 2.7 | 22 | 2.9 | 3.3 | 7.1 | 3.9 | 3.3 | 4.7 | 3.6 |
| OR Quart 3 | 3.1 | 4.0 | 3.2 | 1.8 | 0 | 2.1 | 1.1 | 0.75 | 0.71 |
| p Value | 0.0080 | 0.21 | 0.0094 | 0.16 | na | 0.12 | 0.81 | 0.70 | 0.56 |
| 95% CI of | 1.3 | 0.45 | 1.3 | 0.79 | na | 0.82 | 0.43 | 0.17 | 0.22 |
| OR Quart3 | 7.0 | 36 | 7.7 | 4.2 | na | 5.2 | 3.0 | 3.4 | 2.3 |
| OR Quart 4 | 3.1 | 8.2 | 3.4 | 3.9 | 3.4 | 5.0 | 1.5 | 1.2 | 1.9 |
| p Value | 0.0080 | 0.048 | 0.0064 | 4.5E-4 | 0.065 | 1.9E-4 | 0.36 | 0.74 | 0.18 |
| 95% CI of | 1.3 | 1.0 | 1.4 | 1.8 | 0.93 | 2.1 | 0.61 | 0.33 | 0.75 |
| OR Quart4 | 7.0 | 66 | 8.1 | 8.4 | 12 | 12 | 3.8 | 4.7 | 4.9 |

**CA 15-3**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 434 | 434 | 434 | 434 | 434 | 434 |
| Average | 314 | 393 | 314 | 352 | 314 | 329 |
| Stdev | 236 | 116 | 236 | 212 | 236 | 171 |
| p(t-test) | | 0.041 | | 0.28 | | 0.75 |

| CA 15-3 | | | | | | | |
|---|---|---|---|---|---|---|---|
| sCr or UO | 0hr prior to AKI stage | | 24hr prior to AKI stage | | 48hr prior to AKI stage | | |
| | Cohort 1 | Cohort 2 | Cohort 1 | Cohort 2 | Cohort 1 | Cohort 2 | |
| Min | 2.21 | 1.17 | 2.21 | 21.3 | 2.21 | 4.72 | |
| Max | 784 | 434 | 784 | 784 | 784 | 434 | |
| n (Samp) | 407 | 39 | 407 | 50 | 407 | 27 | |
| n (Patient) | 214 | 39 | 214 | 50 | 214 | 27 | |
| sCr only | 0hr prior to AKI stage | | 24hr prior to AKI stage | | 48hr prior to AKI stage | | |
| | Cohort 1 | Cohort 2 | Cohort 1 | Cohort 2 | Cohort 1 | Cohort 2 | |
| Median | nd | nd | 434 | 434 | 434 | 434 | |
| Average | nd | nd | 329 | 350 | 329 | 351 | |
| Stdev | nd | nd | 223 | 223 | 223 | 152 | |
| p(t-test) | nd | nd | | 0.78 | | 0.75 | |
| Min | nd | nd | 1.17 | 39.8 | 1.17 | 43.5 | |
| Max | nd | nd | 784 | 784 | 784 | 434 | |
| n (Samp) | nd | nd | 535 | 10 | 535 | 11 | |
| n (Patient) | nd | nd | 256 | 10 | 256 | 11 | |
| | | | | | | | |
| UO only | 0hr prior to AKI stage | | 24hr prior to AKI stage | | 48hr prior to AKI stage | | |
| | Cohort 1 | Cohort 2 | Cohort 1 | Cohort 2 | Cohort 1 | Cohort 2 | |
| Median | 434 | 434 | 434 | 434 | 434 | 434 | |
| Average | 316 | 394 | 316 | 375 | 316 | 327 | |
| Stdev | 232 | 116 | 232 | 215 | 232 | 177 | |
| p(t-test) | | 0.039 | | 0.095 | | 0.81 | |
| Min | 2.21 | 1.17 | 2.21 | 21.3 | 2.21 | 4.72 | |
| Max | 784 | 434 | 784 | 784 | 784 | 434 | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| n (Samp) | 380 | | 39 | 380 | | 47 | 380 | | 25 |
| n (Patient) | 189 | | 39 | 189 | | 47 | 189 | | 25 |
| | | | | | | | | | |
| | 0hr prior to AKI stage | | | 24hr prior to AKI stage | | | 48hr prior to AKI stage | | |
| | sCr or UO | sCr only | UO only | sCr or UO | sCr only | UO only | sCr or UO | sCr only | UO only |
| AUC | 0.60 | nd | 0.60 | 0.57 | 0.52 | 0.59 | 0.54 | 0.54 | 0.53 |
| SE | 0.050 | nd | 0.050 | 0.044 | 0.094 | 0.046 | 0.058 | 0.090 | 0.061 |
| p | 0.055 | nd | 0.048 | 0.11 | 0.80 | 0.056 | 0.54 | 0.62 | 0.60 |
| nCohort 1 | 407 | nd | 380 | 407 | 535 | 380 | 407 | 535 | 380 |
| nCohort 2 | 39 | nd | 39 | 50 | 10 | 47 | 27 | 11 | 25 |
| Cutoff 1 | 427 | nd | 427 | 239 | 265 | 427 | 427 | 427 | 427 |
| Sens 1 | 82% | nd | 85% | 70% | 70% | 70% | 70% | 73% | 72% |
| Spec 1 | 43% | nd | 42% | 39% | 35% | 42% | 43% | 39% | 42% |
| Cutoff 2 | 427 | nd | 427 | 80.0 | 167 | 87.5 | 75.8 | 265 | 75.8 |
| Sens 2 | 82% | nd | 85% | 80% | 80% | 81% | 81% | 82% | 80% |
| Spec 2 | 43% | nd | 42% | 31% | 32% | 30% | 30% | 35% | 29% |
| Cutoff 3 | 265 | nd | 265 | 43.7 | 45.0 | 42.6 | 18.9 | 75.8 | 18.8 |
| Sens 3 | 92% | nd | 92% | 90% | 90% | 91% | 93% | 91% | 92% |
| Spec 3 | 40% | nd | 39% | 22% | 20% | 22% | 12% | 26% | 11% |
| Cutoff 4 | 434 | nd | 434 | 434 | 434 | 434 | 434 | 434 | 434 |
| Sens 4 | 0% | nd | 0% | 12% | 10% | 15% | 0% | 0% | 0% |
| Spec 4 | 90% | nd | 91% | 90% | 91% | 91% | 90% | 91% | 91% |
| Cutoff 5 | 434 | nd | 434 | 434 | 434 | 434 | 434 | 434 | 434 |
| Sens 5 | 0% | nd | 0% | 12% | 10% | 15% | 0% | 0% | 0% |

EP 3 489 688 A1

131

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 5 | 90% | nd | 91% | 90% | 91% | 91% | 90% | 91% | 91% |
| Cutoff 6 | 434 | nd | 434 | 434 | 434 | 434 | 434 | 434 | 434 |
| Sens 6 | 0% | nd | 0% | 12% | 10% | 15% | 0% | 0% | 0% |
| Spec 6 | 90% | nd | 91% | 90% | 91% | 91% | 90% | 91% | 91% |
| OR Quart 2 | 8.3 | nd | 11 | 1.0 | 3.6 | 1.3 | 0.99 | 11 | 1.0 |
| p Value | 8.3E-4 | nd | 1.3E-4 | 1.0 | 0.11 | 0.59 | 0.99 | 0.025 | 1.0 |
| 95% CI of | 2.4 | nd | 3.2 | 0.36 | 0.74 | 0.45 | 0.24 | 1.3 | 0.24 |
| OR Quart2 | 29 | nd | 38 | 2.8 | 18 | 4.0 | 4.1 | 84 | 4.1 |
| OR Quart 3 | 5.6 | nd | 3.5 | 4.3 | 0 | 5.3 | 5.6 | 0 | 4.9 |
| p Value | 0.0077 | nd | 0.060 | 6.1E-4 | na | 4.4E-4 | 0.0026 | na | 0.0057 |
| 95% CI of | 1.6 | nd | 0.95 | 1.9 | na | 2.1 | 1.8 | na | 1.6 |
| OR Quart3 | 20 | nd | 13 | 9.9 | na | 14 | 17 | na | 15 |
| OR Quart 4 | 0 | nd | 0 | 0.73 | 0.49 | 1.2 | 0 | 0 | 0 |
| p Value | na | nd | na | 0.57 | 0.57 | 0.79 | na | na | na |
| 95% CI of | na | nd | na | 0.24 | 0.044 | 0.38 | na | na | na |
| OR Quart4 | na | nd | na | 2.2 | 5.5 | 3.6 | na | na | na |

Table 3: Comparison of marker levels in urine samples collected within 12 hours of reaching stage R from Cohort 1 (patients that reached, but did not progress beyond, RIFLE stage R) and from Cohort 2 (patients that reached RIFLE stage I or F).

**C-C motif chemokine 8**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 1.72 | 4.28 | 1.72 | 3.74 | 1.72 | 4.29 |
| Average | 11.4 | 14.2 | 20.6 | 8.92 | 10.2 | 10.3 |
| Stdev | 46.1 | 35.5 | 47.4 | 11.7 | 48.3 | 20.5 |
| p(t-test) | | 0.71 | | 0.37 | | 1.00 |
| Min | 0.0635 | 0.0250 | 0.0635 | 0.206 | 0.0701 | 0.0250 |
| Max | 473 | 214 | 214 | 40.5 | 473 | 108 |
| n (Samp) | 124 | 45 | 49 | 14 | 97 | 31 |
| n (Patient) | 124 | 45 | 49 | 14 | 97 | 31 |
| | | | | | | |
| | At Enrollment | | | | | |
| | sCr or UO | | sCr only | | UO only | |
| AUC | 0.58 | | 0.56 | | 0.57 | |
| SE | 0.051 | | 0.089 | | 0.060 | |
| p | 0.097 | | 0.48 | | 0.22 | |
| nCohort 1 | 124 | | 49 | | 97 | |
| nCohort 2 | 45 | | 14 | | 31 | |
| Cutoff 1 | 0.829 | | 1.21 | | 0.746 | |
| Sens 1 | 71% | | 79% | | 74% | |
| Spec 1 | 38% | | 45% | | 34% | |
| Cutoff 2 | 0.627 | | 0.756 | | 0.555 | |
| Sens 2 | 80% | | 86% | | 81% | |
| Spec 2 | 35% | | 33% | | 34% | |
| Cutoff 3 | 0.136 | | 0.306 | | 0.0775 | |
| Sens 3 | 91% | | 93% | | 90% | |
| Spec 3 | 19% | | 22% | | 6% | |
| Cutoff 4 | 4.86 | | 9.22 | | 4.72 | |
| Sens 4 | 44% | | 29% | | 48% | |
| Spec 4 | 70% | | 71% | | 70% | |
| Cutoff 5 | 8.21 | | 19.7 | | 7.85 | |
| Sens 5 | 31% | | 21% | | 35% | |
| Spec 5 | 81% | | 82% | | 80% | |
| Cutoff 6 | 21.9 | | 57.1 | | 19.7 | |
| Sens 6 | 11% | | 0% | | 13% | |
| Spec 6 | 90% | | 92% | | 91% | |

(continued)

| | At Enrollment | | |
|---|---|---|---|
| | sCr or UO | sCr only | UO only |
| OR Quart 2 | 2.7 | 2.2 | 1.0 |
| p Value | 0.074 | 0.42 | 1.0 |
| 95% CI of | 0.91 | 0.33 | 0.28 |
| OR Quart2 | 8.0 | 14 | 3.5 |
| OR Quart 3 | 1.9 | 3.0 | 1.2 |
| p Value | 0.27 | 0.24 | 0.76 |
| 95% CI of | 0.61 | 0.48 | 0.36 |
| OR Quart3 | 5.7 | 18 | 4.1 |
| OR Quart 4 | 3.6 | 1.5 | 2.6 |
| p Value | 0.019 | 0.68 | 0.10 |
| 95% CI of | 1.2 | 0.21 | 0.83 |
| OR Quart4 | 10 | 11 | 8.1 |

**Immunoglogulin G1**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 4050 | 6810 | 4770 | 9980 | 3950 | 7590 |
| Average | 6150 | 8820 | 8120 | 10500 | 6060 | 7790 |
| Stdev | 6120 | 7880 | 8760 | 8330 | 5490 | 5330 |
| p(t-test) | | 0.023 | | 0.37 | | 0.13 |
| Min | 342 | 572 | 342 | 699 | 424 | 634 |
| Max | 35500 | 30900 | 35500 | 28400 | 27500 | 22200 |
| n (Samp) | 122 | 44 | 48 | 14 | 96 | 30 |
| n (Patient) | 122 | 44 | 48 | 14 | 96 | 30 |

| | At Enrollment | | |
|---|---|---|---|
| | sCr or UO | sCr only | UO only |
| AUC | 0.60 | 0.59 | 0.61 |
| SE | 0.051 | 0.089 | 0.061 |
| p | 0.054 | 0.32 | 0.060 |
| nCohort 1 | 122 | 48 | 96 |
| nCohort 2 | 44 | 14 | 30 |
| Cutoff 1 | 3600 | 3630 | 4450 |
| Sens 1 | 70% | 71% | 70% |
| Spec 1 | 45% | 42% | 57% |
| Cutoff 2 | 1790 | 1480 | 2710 |
| Sens 2 | 82% | 86% | 80% |
| Spec 2 | 20% | 17% | 33% |

(continued)

| | | At Enrollment | | |
|---|---|---|---|---|
| | | sCr or UO | sCr only | UO only |
| Cutoff 3 | | 1470 | 1420 | 1600 |
| Sens 3 | | 91% | 93% | 90% |
| Spec 3 | | 13% | 17% | 14% |
| Cutoff 4 | 6730 | | 6730 | 7270 |
| Sens 4 | 50% | | 64% | 53% |
| Spec 4 | 70% | | 71% | 71% |
| Cutoff 5 | 9090 | | 16800 | 10500 |
| Sens 5 | 36% | | 21% | 27% |
| Spec 5 | 80% | | 81% | 80% |
| Cutoff 6 | 13500 | | 22800 | 13100 |
| Sens 6 | 23% | | 7% | 17% |
| Spec 6 | 90% | | 92% | 91% |
| OR Quart 2 | 0.62 | | 0.18 | 0.43 |
| p Value | 0.39 | | 0.15 | 0.27 |
| 95% CI of | 0.21 | | 0.018 | 0.098 |
| OR Quart2 | 1.8 | | 1.9 | 1.9 |
| OR Quart 3 | 1.1 | | 1.0 | 2.3 |
| p Value | 0.80 | | 1.0 | 0.16 |
| 95% CI of | 0.42 | | 0.20 | 0.72 |
| OR Quart3 | 3.1 | | 5.0 | 7.3 |
| OR Quart 4 | 1.9 | | 1.2 | 1.9 |
| p Value | 0.18 | | 0.78 | 0.28 |
| 95% CI of | 0.74 | | 0.26 | 0.59 |
| OR Quart4 | 4.9 | | 5.9 | 6.1 |

| **Interleukin-11** | | | | | | |
|---|---|---|---|---|---|---|
| | sCr or UO | | sCr only | | UO only | |
| | Cohort 1 | Cohort 2 | Cohort 1 | Cohort 2 | Cohort 1 | Cohort 2 |
| Median | 215 | 179 | 223 | 200 | 217 | 157 |
| Average | 309 | 246 | 315 | 224 | 310 | 194 |
| Stdev | 297 | 329 | 363 | 163 | 298 | 153 |
| p(t-test) | | 0.24 | | 0.37 | | 0.044 |
| Min | 13.2 | 14.5 | 13.2 | 31.9 | 41.4 | 14.5 |

(continued)

| Interleukin-11 | | | | | | |
|---|---|---|---|---|---|---|
| | sCr or UO | | sCr only | | UO only | |
| | Cohort 1 | Cohort 2 | Cohort 1 | Cohort 2 | Cohort 1 | Cohort 2 |
| Max | 1780 | 2140 | 2140 | 547 | 1780 | 753 |
| n (Samp) | 124 | 44 | 49 | 14 | 97 | 30 |
| n (Patient) | 124 | 44 | 49 | 14 | 97 | 30 |
| | | | | | | |
| | At Enrollment | | | | | |
| | sCr or UO | | sCr only | | UO only | |
| AUC | 0.41 | | 0.45 | | 0.37 | |
| SE | 0.051 | | 0.089 | | 0.061 | |
| p | 0.087 | | 0.61 | | 0.037 | |
| nCohort 1 | 124 | | 49 | | 97 | |
| nCohort 2 | 44 | | 14 | | 30 | |
| Cutoff 1 | 113 | | 113 | | 101 | |
| Sens 1 | 70% | | 71% | | 70% | |
| Spec 1 | 23% | | 33% | | 16% | |
| Cutoff 2 | 85.8 | | 50.4 | | 87.1 | |
| Sens 2 | 82% | | 86% | | 80% | |
| Spec 2 | 15% | | 12% | | 13% | |
| Cutoff 3 | 42.5 | | 41.4 | | 42.5 | |
| Sens 3 | 91% | | 93% | | 90% | |
| Spec 3 | 5% | | 10% | | 3% | |
| Cutoff 4 | 347 | | 390 | | 341 | |
| Sens 4 | 16% | | 21% | | 10% | |
| Spec 4 | 70% | | 71% | | 70% | |
| Cutoff 5 | 458 | | 426 | | 469 | |
| Sens 5 | 9% | | 14% | | 3% | |
| Spec 5 | 81% | | 82% | | 80% | |
| Cutoff 6 | 613 | | 659 | | 601 | |
| Sens 6 | 5% | | 0% | | 3% | |
| Spec 6 | 90% | | 92% | | 91% | |
| OR Quart 2 | 1.6 | | 3.2 | | 3.2 | |
| p Value | 0.42 | | 0.21 | | 0.11 | |
| 95% CI of | 0.53 | | 0.52 | | 0.77 | |
| OR Quart2 | 4.6 | | 20 | | 14 | |
| OR Quart 3 | 2.5 | | 1.6 | | 3.2 | |

(continued)

|  | At Enrollment | | |
|---|---|---|---|
|  | sCr or UO | sCr only | UO only |
| p Value | 0.083 | 0.63 | 0.11 |
| 95% CI of | 0.89 | 0.23 | 0.77 |
| OR Quart3 | 7.0 | 11 | 14 |
| OR Quart 4 | 2.2 | 2.5 | 5.3 |
| p Value | 0.13 | 0.33 | 0.019 |
| 95% CI of | 0.79 | 0.39 | 1.3 |
| OR Quart4 | 6.4 | 17 | 22 |

**Neutrophil collagenase**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 4380 | 7320 | 3490 | 12000 | 5330 | 5620 |
| Average | 13500 | 36800 | 8510 | 15400 | 17400 | 43100 |
| Stdev | 23700 | 118000 | 13200 | 19200 | 27900 | 138000 |
| p(t-test) |  | 0.027 |  | 0.10 |  | 0.066 |
| Min | 150 | 23.8 | 23.8 | 25.8 | 124 | 0.260 |
| Max | 128000 | 670000 | 63000 | 62500 | 128000 | 670000 |
| n (Samp) | 138 | 58 | 56 | 16 | 108 | 42 |
| n (Patient) | 138 | 58 | 56 | 16 | 108 | 42 |

|  | At Enrollment | | |
|---|---|---|---|
|  | sCr or UO | sCr only | UO only |
| AUC | 0.58 | 0.61 | 0.54 |
| SE | 0.046 | 0.083 | 0.053 |
| p | 0.096 | 0.19 | 0.47 |
| nCohort 1 | 138 | 56 | 108 |
| nCohort 2 | 58 | 16 | 42 |
| Cutoff 1 | 2620 | 1580 | 2940 |
| Sens 1 | 71% | 75% | 71% |
| Spec 1 | 41% | 36% | 39% |
| Cutoff 2 | 1580 | 987 | 1630 |
| Sens 2 | 81% | 81% | 81% |
| Spec 2 | 32% | 25% | 27% |
| Cutoff 3 | 566 | 365 | 1110 |
| Sens 3 | 91% | 94% | 90% |
| Spec 3 | 10% | 11% | 21% |

(continued)

| | At Enrollment | | |
|---|---|---|---|
| | sCr or UO | sCr only | UO only |
| Cutoff 4 | 9550 | 7860 | 12900 |
| Sens 4 | 41% | 56% | 38% |
| Spec 4 | 70% | 71% | 70% |
| Cutoff 5 | 15700 | 10700 | 22200 |
| Sens 5 | 33% | 56% | 24% |
| Spec 5 | 80% | 80% | 81% |
| Cutoff 6 | 48400 | 28300 | 60700 |
| Sens 6 | 12% | 12% | 5% |
| Spec 6 | 91% | 91% | 91% |
| OR Quart 2 | 1.9 | 0.70 | 1.9 |
| p Value | 0.17 | 0.67 | 0.23 |
| 95% CI of OR Quart2 | 0.76 | 0.13 | 0.67 |
| | 4.7 | 3.7 | 5.3 |
| OR Quart 3 | 1.4 | 0.44 | 0.85 |
| p Value | 0.48 | 0.38 | 0.77 |
| 95% CI of OR Quart3 | 0.55 | 0.069 | 0.27 |
| | 3.6 | 2.8 | 2.6 |
| OR Quart 4 | 2.5 | 2.2 | 2.1 |
| p Value | 0.049 | 0.28 | 0.15 |
| 95% CI of OR Quart4 | 1.0 | 0.52 | 0.76 |
| | 6.1 | 9.6 | 5.9 |

Table 4: Comparison of the maximum marker levels in urine samples collected from Cohort 1 (patients that did not progress beyond RIFLE stage 0) and the maximum values in urine samples collected from subjects between enrollment and 0, 24 hours, and 48 hours prior to reaching stage F in Cohort 2.

| C-C motif chemokine 18 | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 0.531 | 2.90 | 0.531 | 2.72 | 0.531 | 2.38 |
| Average | 2.66 | 12.9 | 2.66 | 10.2 | 2.66 | 9.02 |
| Stdev | 7.47 | 16.2 | 7.47 | 14.4 | 7.47 | 15.4 |

(continued)

| C-C motif chemokine 18 | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| p(t-test) | | 1.0E-8 | | 8.6E-6 | | 0.0030 |
| Min | 3.13E-5 | 0.226 | 3.13E-5 | 0.204 | 3.13E-5 | 0.126 |
| Max | 40.0 | 40.0 | 40.0 | 40.0 | 40.0 | 40.0 |
| n (Samp) | 223 | 30 | 223 | 30 | 223 | 16 |
| n (Patient) | 223 | 30 | 223 | 30 | 223 | 16 |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 0.649 | 2.95 | 0.649 | 2.72 | 0.649 | 2.95 |
| Average | 3.73 | 15.1 | 3.73 | 8.44 | 3.73 | 8.52 |
| Stdev | 9.09 | 18.0 | 9.09 | 11.7 | 9.09 | 13.3 |
| p(t-test) | | 3.0E-5 | | 0.070 | | 0.17 |
| Min | 3.13E-5 | 0.460 | 3.13E-5 | 0.204 | 3.13E-5 | 0.460 |
| Max | 40.0 | 40.0 | 40.0 | 40.0 | 40.0 | 37.8 |
| n (Samp) | 374 | 13 | 374 | 13 | 374 | 7 |
| n (Patient) | 374 | 13 | 374 | 13 | 374 | 7 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 0.620 | 2.95 | 0.620 | 2.85 | 0.620 | 2.53 |
| Average | 3.05 | 13.9 | 3.05 | 13.2 | 3.05 | 9.95 |
| Stdev | 7.82 | 16.8 | 7.82 | 16.7 | 7.82 | 16.3 |
| p(t-test) | | 3.6E-7 | | 1.8E-6 | | 0.0048 |
| Min | 3.13E-5 | 0.226 | 3.13E-5 | 0.226 | 3.13E-5 | 0.126 |
| Max | 40.0 | 40.0 | 40.0 | 40.0 | 40.0 | 40.0 |
| n (Samp) | 173 | 23 | 173 | 23 | 173 | 14 |
| n (Patient) | 173 | 23 | 173 | 23 | 173 | 14 |
| | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.82 | 0.79 | 0.80 | 0.80 | 0.75 | 0.79 | 0.72 | 0.78 | 0.68 |
| SE | 0.048 | 0.076 | 0.057 | 0.050 | 0.079 | 0.058 | 0.074 | 0.10 | 0.081 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| p | 2.2E-11 | 1.2E-4 | 1.0E-7 | 2.6E-9 | 0.0014 | 6.2E-7 | 0.0028 | 0.0079 | 0.027 |
| nCohort 1 | 223 | 374 | 173 | 223 | 374 | 173 | 223 | 374 | 173 |
| nCohort 2 | 30 | 13 | 23 | 30 | 13 | 23 | 16 | 7 | 14 |
| Cutoff 1 | 1.98 | 1.97 | 1.98 | 1.98 | 1.97 | 1.98 | 0.714 | 1.99 | 1.34 |
| Sens 1 | 70% | 77% | 74% | 70% | 77% | 74% | 75% | 71% | 71% |
| Spec 1 | 83% | 76% | 76% | 83% | 76% | 76% | 61% | 76% | 71% |
| Cutoff 2 | 1.48 | 1.04 | 1.51 | 1.04 | 1.04 | 1.34 | 0.451 | 1.97 | 0.228 |
| Sens 2 | 80% | 85% | 83% | 80% | 85% | 83% | 81% | 86% | 86% |
| Spec 2 | 78% | 63% | 71% | 72% | 63% | 71% | 45% | 76% | 18% |
| Cutoff 3 | 0.531 | 0.531 | 0.862 | 0.451 | 0.451 | 0.767 | 0.225 | 0.451 | 0.205 |
| Sens 3 | 90% | 92% | 91% | 90% | 92% | 91% | 94% | 100% | 93% |
| Spec 3 | 50% | 43% | 60% | 45% | 39% | 57% | 28% | 39% | 18% |
| Cutoff 4 | 1.03 | 1.41 | 1.34 | 1.03 | 1.41 | 1.34 | 1.03 | 1.41 | 1.34 |
| Sens 4 | 83% | 77% | 87% | 80% | 77% | 83% | 69% | 86% | 71% |
| Spec 4 | 70% | 70% | 71% | 70% | 70% | 71% | 70% | 70% | 71% |
| Cutoff 5 | 1.71 | 2.82 | 2.68 | 1.71 | 2.82 | 2.68 | 1.71 | 2.82 | 2.68 |
| Sens 5 | 73% | 54% | 61% | 73% | 46% | 57% | 62% | 57% | 50% |
| Spec 5 | 80% | 80% | 80% | 80% | 80% | 80% | 80% | 80% | 80% |
| Cutoff 6 | 3.97 | 8.25 | 4.19 | 3.97 | 8.25 | 4.19 | 3.97 | 8.25 | 4.19 |
| Sens 6 | 43% | 38% | 43% | 37% | 31% | 39% | 25% | 29% | 21% |
| Spec 6 | 90% | 90% | 90% | 90% | 90% | 90% | 90% | 90% | 90% |
| OR Quart 2 | 3.1 | >2.0 | 0 | 3.1 | 0.99 | 0 | 3.1 | >1.0 | 0 |
| p Value | 0.33 | <0.57 | na | 0.33 | 0.99 | na | 0.34 | <0.99 | na |
| 95% CI of | 0.31 | >0.18 | na | 0.31 | 0.061 | na | 0.31 | >0.062 | na |
| OR Quart2 | 31 | na | na | 31 | 16 | na | 30 | na | na |
| OR Quart 3 | 4.2 | >3.1 | 3.9 | 4.2 | 3.0 | 3.9 | 0.98 | >0 | 0.98 |
| p Value | 0.20 | <0.34 | 0.100 | 0.20 | 0.34 | 0.100 | 0.99 | <na | 0.98 |
| 95% CI of | 0.46 | >0.31 | 0.77 | 0.46 | 0.31 | 0.77 | 0.060 | >na | 0.19 |
| OR Quart3 | 39 | na | 20 | 39 | 30 | 20 | 16 | na | 5.1 |
| OR Quart 4 | 32 | >8.6 | 9.4 | 32 | 8.5 | 9.4 | 13 | >6.3 | 2.9 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value | 8.4E-4 | <0.044 | 0.0045 | 8.4E-4 | 0.045 | 0.0045 | 0.016 | <0.090 | 0.13 |
| 95% CI of | 4.2 | >1.1 | 2.0 | 4.2 | 1.0 | 2.0 | 1.6 | >0.75 | 0.73 |
| OR Quart4 | 250 | na | 44 | 250 | 70 | 44 | 100 | na | 12 |

**C-C motif chemokine 24**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 13.0 | 51.0 | 13.0 | 30.9 | 13.0 | 26.4 |
| Average | 26.0 | 308 | 26.0 | 269 | 26.0 | 95.6 |
| Stdev | 58.4 | 690 | 58.4 | 598 | 58.4 | 288 |
| p(t-test) | | 5.8E-9 | | 1.0E-8 | | 0.0037 |
| Min | 0.0120 | 2.55 | 0.0120 | 2.55 | 0.0120 | 2.55 |
| Max | 730 | 2790 | 730 | 2380 | 730 | 1170 |
| n (Samp) | 223 | 30 | 223 | 30 | 223 | 16 |
| n (Patient) | 223 | 30 | 223 | 30 | 223 | 16 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 17.8 | 30.9 | 17.8 | 28.7 | 17.8 | 28.0 |
| Average | 48.4 | 93.4 | 48.4 | 63.7 | 48.4 | 53.5 |
| Stdev | 160 | 110 | 160 | 74.6 | 160 | 86.7 |
| p(t-test) | | 0.32 | | 0.73 | | 0.93 |
| Min | 0.0120 | 2.55 | 0.0120 | 2.55 | 0.0120 | 2.55 |
| Max | 2380 | 373 | 2380 | 249 | 2380 | 249 |
| n (Samp) | 375 | 13 | 375 | 13 | 375 | 7 |
| n (Patient) | 375 | 13 | 375 | 13 | 375 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 13.9 | 61.9 | 13.9 | 41.6 | 13.9 | 28.3 |
| Average | 30.6 | 390 | 30.6 | 342 | 30.6 | 112 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | 66.3 | 773 | 66.3 | 669 | 66.3 | 306 |
| p(t-test) | | 7.2E-9 | | 9.0E-9 | | 0.0051 |
| Min | 0.0120 | 3.62 | 0.0120 | 3.62 | 0.0120 | 3.62 |
| Max | 730 | 2790 | 730 | 2380 | 730 | 1170 |
| n (Samp) | 173 | 23 | 173 | 23 | 173 | 14 |
| n (Patient) | 173 | 23 | 173 | 23 | 173 | 14 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.81 | 0.72 | 0.83 | 0.78 | 0.66 | 0.81 | 0.66 | 0.58 | 0.70 |
| SE | 0.049 | 0.082 | 0.054 | 0.051 | 0.084 | 0.056 | 0.076 | 0.11 | 0.081 |
| p | 2.2E-10 | 0.0078 | 6.4E-10 | 4.2E-8 | 0.051 | 2.2E-8 | 0.034 | 0.49 | 0.015 |
| nCohort 1 | 223 | 375 | 173 | 223 | 375 | 173 | 223 | 375 | 173 |
| nCohort 2 | 30 | 13 | 23 | 30 | 13 | 23 | 16 | 7 | 14 |
| Cutoff 1 | 28.4 | 27.3 | 30.4 | 27.2 | 25.9 | 27.8 | 16.6 | 26.9 | 20.4 |
| Sens 1 | 70% | 77% | 74% | 70% | 77% | 74% | 75% | 71% | 71% |
| Spec 1 | 78% | 64% | 77% | 76% | 62% | 75% | 61% | 63% | 66% |
| Cutoff 2 | 25.4 | 25.4 | 27.8 | 20.4 | 10.9 | 26.6 | 8.65 | 8.65 | 16.6 |
| Sens 2 | 80% | 85% | 83% | 80% | 85% | 83% | 81% | 86% | 86% |
| Spec 2 | 73% | 61% | 75% | 68% | 35% | 73% | 40% | 31% | 58% |
| Cutoff 3 | 16.6 | 8.65 | 19.3 | 10.9 | 8.65 | 19.3 | 3.21 | 2.51 | 3.71 |
| Sens 3 | 90% | 92% | 91% | 90% | 92% | 91% | 94% | 100% | 93% |
| Spec 3 | 61% | 31% | 63% | 48% | 31% | 63% | 28% | 18% | 25% |
| Cutoff 4 | 23.3 | 36.9 | 24.6 | 23.3 | 36.9 | 24.6 | 23.3 | 36.9 | 24.6 |
| Sens 4 | 80% | 46% | 83% | 77% | 38% | 83% | 56% | 14% | 64% |
| Spec 4 | 71% | 70% | 71% | 71% | 70% | 71% | 71% | 70% | 71% |
| Cutoff 5 | 35.1 | 51.0 | 40.3 | 35.1 | 51.0 | 40.3 | 35.1 | 51.0 | 40.3 |
| Sens 5 | 53% | 46% | 61% | 43% | 38% | 52% | 19% | 14% | 29% |
| Spec 5 | 80% | 80% | 80% | 80% | 80% | 80% | 80% | 80% | 80% |
| Cutoff 6 | 62.8 | 87.2 | 74.4 | 62.8 | 87.2 | 74.4 | 62.8 | 87.2 | 74.4 |
| Sens 6 | 43% | 31% | 48% | 37% | 15% | 39% | 6% | 14% | 14% |
| Spec 6 | 90% | 90% | 90% | 90% | 90% | 90% | 90% | 90% | 90% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart 2 | 2.0 | 1.0 | 0 | 3.1 | 2.0 | 0 | 3.1 | 0.99 | 0 |
| p Value | 0.57 | 1.0 | na | 0.33 | 0.57 | na | 0.34 | 0.99 | na |
| 95% CI of | 0.18 | 0.062 | na | 0.31 | 0.18 | na | 0.31 | 0.061 | na |
| OR Quart2 | 23 | 16 | na | 31 | 23 | na | 30 | 16 | na |
| OR Quart 3 | 12 | 5.2 | 9.4 | 12 | 5.2 | 12 | 4.1 | 4.1 | 3.2 |
| p Value | 0.021 | 0.13 | 0.039 | 0.021 | 0.13 | 0.019 | 0.21 | 0.21 | 0.17 |
| 95% CI of | 1.4 | 0.60 | 1.1 | 1.4 | 0.60 | 1.5 | 0.45 | 0.45 | 0.61 |
| OR Quart3 | 94 | 46 | 78 | 94 | 46 | 100 | 38 | 38 | 17 |
| OR Quart 4 | 22 | 6.3 | 19 | 21 | 5.2 | 16 | 8.9 | 0.99 | 3.2 |
| p Value | 0.0030 | 0.090 | 0.0053 | 0.0039 | 0.13 | 0.0098 | 0.042 | 0.99 | 0.17 |
| 95% CI of | 2.9 | 0.75 | 2.4 | 2.6 | 0.60 | 1.9 | 1.1 | 0.061 | 0.61 |
| OR Quart4 | 170 | 54 | 150 | 160 | 46 | 130 | 74 | 16 | 17 |

**C-C motif chemokine 8**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.74 | 24.0 | 2.74 | 22.9 | 2.74 | 13.6 |
| Average | 11.9 | 175 | 11.9 | 169 | 11.9 | 250 |
| Stdev | 27.9 | 664 | 27.9 | 665 | 27.9 | 912 |
| p(t-test) | | 2.8E-4 | | 4.6E-4 | | 9.2E-5 |
| Min | 0.0250 | 0.206 | 0.0250 | 0.136 | 0.0250 | 0.136 |
| Max | 250 | 3670 | 250 | 3670 | 250 | 3670 |
| n (Samp) | 223 | 30 | 223 | 30 | 223 | 16 |
| n (Patient) | 223 | 30 | 223 | 30 | 223 | 16 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 4.72 | 22.7 | 4.72 | 22.7 | 4.72 | 22.7 |
| Average | 17.7 | 49.4 | 17.7 | 45.4 | 17.7 | 36.6 |
| Stdev | 47.6 | 82.2 | 47.6 | 79.2 | 47.6 | 47.0 |

(continued)

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.022 | | 0.045 | | 0.30 |
| Min | 0.0250 | 0.206 | 0.0250 | 0.206 | 0.0250 | 4.53 |
| Max | 492 | 279 | 492 | 279 | 492 | 140 |
| n (Samp) | 375 | 13 | 375 | 13 | 375 | 7 |
| n (Patient) | 375 | 13 | 375 | 13 | 375 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 4.72 | 30.8 | 4.72 | 30.8 | 4.72 | 13.6 |
| Average | 14.8 | 220 | 14.8 | 209 | 14.8 | 284 |
| Stdev | 32.0 | 755 | 32.0 | 757 | 32.0 | 975 |
| p(t-test) | | 3.9E-4 | | 7.8E-4 | | 2.7E-4 |
| Min | 0.0250 | 0.829 | 0.0250 | 0.136 | 0.0250 | 0.136 |
| Max | 250 | 3670 | 250 | 3670 | 250 | 3670 |
| n(Samp) | 173 | 23 | 173 | 23 | 173 | 14 |
| n (Patient) | 173 | 23 | 173 | 23 | 173 | 14 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.79 | 0.70 | 0.82 | 0.77 | 0.69 | 0.78 | 0.70 | 0.77 | 0.65 |
| SE | 0.051 | 0.083 | 0.056 | 0.052 | 0.083 | 0.059 | 0.075 | 0.11 | 0.082 |
| p | 6.8E-9 | 0.017 | 1.3E-8 | 3.2E-7 | 0.022 | 2.1E-6 | 0.0080 | 0.010 | 0.078 |
| nCohort 1 | 223 | 375 | 173 | 223 | 375 | 173 | 223 | 375 | 173 |
| nCohort 2 | 30 | 13 | 23 | 30 | 13 | 23 | 16 | 7 | 14 |
| Cutoff 1 | 10.4 | 4.68 | 12.5 | 9.39 | 4.35 | 10.4 | 4.35 | 21.7 | 4.35 |
| Sens 1 | 70% | 77% | 74% | 70% | 77% | 74% | 75% | 71% | 71% |
| Spec 1 | 74% | 48% | 73% | 71% | 48% | 69% | 56% | 81% | 47% |
| Cutoff 2 | 7.37 | 4.35 | 9.05 | 4.35 | 3.16 | 8.05 | 1.21 | 7.37 | 0.756 |
| Sens 2 | 80% | 85% | 83% | 80% | 85% | 83% | 81% | 86% | 86% |
| Spec 2 | 67% | 48% | 66% | 56% | 46% | 65% | 39% | 59% | 26% |
| Cutoff 3 | 1.21 | 0.438 | 4.35 | 0.650 | 0.438 | 1.21 | 0.438 | 4.35 | 0.438 |
| Sens 3 | 90% | 92% | 91% | 90% | 92% | 91% | 94% | 100% | 93% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 3 | 39% | 24% | 47% | 32% | 24% | 31% | 30% | 48% | 21% |
| Cutoff 4 | 9.38 | 12.0 | 11.1 | 9.38 | 12.0 | 11.1 | 9.38 | 12.0 | 11.1 |
| Sens 4 | 73% | 62% | 74% | 70% | 62% | 70% | 56% | 71% | 57% |
| Spec 4 | 70% | 70% | 71% | 70% | 70% | 71% | 70% | 70% | 71% |
| Cutoff 5 | 12.8 | 20.3 | 18.0 | 12.8 | 20.3 | 18.0 | 12.8 | 20.3 | 18.0 |
| Sens 5 | 63% | 62% | 70% | 63% | 62% | 65% | 50% | 71% | 43% |
| Spec 5 | 80% | 80% | 80% | 80% | 80% | 80% | 80% | 80% | 80% |
| Cutoff 6 | 31.5 | 36.6 | 34.7 | 31.5 | 36.6 | 34.7 | 31.5 | 36.6 | 34.7 |
| Sens 6 | 37% | 23% | 48% | 37% | 15% | 48% | 19% | 14% | 21% |
| Spec 6 | 90% | 90% | 90% | 90% | 90% | 90% | 90% | 90% | 90% |
| OR Quart 2 | 3.1 | 0.49 | 2.0 | 2.1 | 1.0 | 1.0 | 3.1 | >1.0 | 1.5 |
| p Value | 0.33 | 0.57 | 0.57 | 0.41 | 1.0 | 1.0 | 0.34 | <1.0 | 0.67 |
| 95% CI of | 0.31 | 0.044 | 0.18 | 0.36 | 0.14 | 0.14 | 0.31 | >0.062 | 0.24 |
| OR Quart2 | 31 | 5.5 | 23 | 12 | 7.2 | 7.4 | 30 | na | 9.4 |
| OR Quart 3 | 7.8 | 1.0 | 4.3 | 2.6 | 0.49 | 2.1 | 4.1 | >1.0 | 1.5 |
| p Value | 0.059 | 1.0 | 0.20 | 0.26 | 0.57 | 0.41 | 0.21 | <0.99 | 0.67 |
| 95% CI of | 0.92 | 0.14 | 0.46 | 0.49 | 0.044 | 0.36 | 0.45 | >0.062 | 0.24 |
| OR Quart3 | 65 | 7.2 | 40 | 14 | 5.5 | 12 | 38 | na | 9.4 |
| OR Quart 4 | 26 | 4.3 | 23 | 13 | 4.3 | 10 | 8.9 | >5.2 | 3.2 |
| p Value | 0.0018 | 0.071 | 0.0029 | 8.9E-4 | 0.071 | 0.0029 | 0.042 | <0.13 | 0.17 |
| 95% CI of | 3.4 | 0.88 | 2.9 | 2.9 | 0.88 | 2.2 | 1.1 | >0.60 | 0.61 |
| OR Quart4 | 200 | 21 | 180 | 58 | 21 | 48 | 74 | na | 17 |

**Cathepsin D**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 71300 | 128000 | 71300 | 123000 | 71300 | 121000 |
| Average | 74100 | 136000 | 74100 | 131000 | 74100 | 130000 |
| Stdev | 38900 | 41600 | 38900 | 39100 | 38900 | 42500 |
| p(t-test) | | 1.8E-14 | | 7.3E-13 | | 7.9E-8 |

(continued)

| Cathepsin D | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Min | 656 | 65600 | 656 | 65600 | 656 | 65600 |
| Max | 200000 | 200000 | 200000 | 200000 | 200000 | 200000 |
| n (Samp) | 223 | 30 | 223 | 30 | 223 | 16 |
| n (Patient) | 223 | 30 | 223 | 30 | 223 | 16 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 84600 | 130000 | 84600 | 120000 | 84600 | 117000 |
| Average | 90900 | 127000 | 90900 | 121000 | 90900 | 127000 |
| Stdev | 44700 | 34200 | 44700 | 29100 | 44700 | 24800 |
| p(t-test) | | 0.0040 | | 0.018 | | 0.035 |
| Min | 656 | 71400 | 656 | 71400 | 656 | 110000 |
| Max | 200000 | 188000 | 200000 | 181000 | 200000 | 181000 |
| n (Samp) | 374 | 13 | 374 | 13 | 374 | 7 |
| n (Patient) | 374 | 13 | 374 | 13 | 374 | 7 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 78900 | 138000 | 78900 | 130000 | 78900 | 125000 |
| Average | 84300 | 143000 | 84300 | 138000 | 84300 | 129000 |
| Stdev | 40600 | 41200 | 40600 | 40000 | 40600 | 43300 |
| p(t-test) | | 7.6E-10 | | 9.9E-9 | | 1.0E-4 |
| Min | 2520 | 65600 | 2520 | 65600 | 2520 | 65600 |
| Max | 200000 | 200000 | 200000 | 200000 | 200000 | 200000 |
| n (Samp) | 173 | 23 | 173 | 23 | 173 | 14 |
| n (Patient) | 173 | 23 | 173 | 23 | 173 | 14 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.86 | 0.74 | 0.84 | 0.85 | 0.72 | 0.83 | 0.84 | 0.77 | 0.78 |
| SE | 0.044 | 0.080 | 0.053 | 0.045 | 0.081 | 0.054 | 0.063 | 0.11 | 0.075 |
| p | 4.4E-16 | 0.0021 | 9.5E-11 | 1.2E-14 | 0.0069 | 1.7E-9 | 9.2E-8 | 0.011 | 1.9E-4 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| nCohort 1 | 223 | 374 | 173 | 223 | 374 | 173 | 223 | 374 | 173 |
| nCohort 2 | 30 | 13 | 23 | 30 | 13 | 23 | 16 | 7 | 14 |
| Cutoff 1 | 117000 | 109000 | 119000 | 112000 | 109000 | 112000 | 109000 | 115000 | 109000 |
| Sens 1 | 70% | 77% | 74% | 70% | 77% | 74% | 75% | 71% | 71% |
| Spec 1 | 86% | 70% | 81% | 84% | 70% | 77% | 83% | 72% | 74% |
| Cutoff 2 | 109000 | 92900 | 111000 | 109000 | 89500 | 110000 | 99400 | 112000 | 96100 |
| Sens 2 | 80% | 85% | 83% | 80% | 85% | 83% | 81% | 86% | 86% |
| Spec 2 | 83% | 56% | 75% | 83% | 53% | 75% | 76% | 72% | 65% |
| Cutoff 3 | 84400 | 84400 | 96100 | 84400 | 84400 | 96100 | 66900 | 109000 | 66900 |
| Sens 3 | 90% | 92% | 91% | 90% | 92% | 91% | 94% | 100% | 93% |
| Spec 3 | 67% | 50% | 65% | 67% | 50% | 65% | 48% | 70% | 39% |
| Cutoff 4 | 92600 | 110000 | 103000 | 92600 | 110000 | 103000 | 92600 | 110000 | 103000 |
| Sens 4 | 87% | 69% | 87% | 83% | 69% | 87% | 88% | 86% | 71% |
| Spec 4 | 70% | 70% | 71% | 70% | 70% | 71% | 70% | 70% | 71% |
| Cutoff 5 | 104000 | 133000 | 117000 | 104000 | 133000 | 117000 | 104000 | 133000 | 117000 |
| Sens 5 | 80% | 38% | 78% | 80% | 23% | 70% | 75% | 14% | 57% |
| Spec 5 | 80% | 80% | 80% | 80% | 80% | 80% | 80% | 80% | 80% |
| Cutoff 6 | 133000 | 151000 | 143000 | 133000 | 151000 | 143000 | 133000 | 151000 | 143000 |
| Sens 6 | 43% | 15% | 43% | 40% | 8% | 39% | 38% | 14% | 36% |
| Spec 6 | 90% | 90% | 90% | 90% | 90% | 90% | 90% | 90% | 90% |
| OR Quart 2 | >3.1 | >2.0 | >2.1 | >3.1 | >2.0 | >2.1 | >2.0 | >0 | >2.0 |
| p Value | <0.33 | <0.57 | <0.55 | <0.33 | <0.57 | <0.55 | <0.57 | <na | <0.56 |
| 95% CI of | >0.32 | >0.18 | >0.18 | >0.32 | >0.18 | >0.18 | >0.18 | >na | >0.18 |
| OR Quart2 | na | na | na | na | na | na | na | na | na |
| OR Quart 3 | >3.1 | >4.1 | >5.6 | >3.1 | >5.2 | >6.8 | >2.0 | >5.3 | >4.3 |
| p Value | <0.33 | <0.21 | <0.12 | <0.33 | <0.13 | <0.081 | <0.57 | <0.13 | <0.20 |
| 95% CI of | >0.32 | >0.45 | >0.63 | >0.32 | >0.60 | >0.79 | >0.18 | >0.60 | >0.46 |
| OR Quart3 | na | na | na | na | na | na | na | na | na |
| OR Quart 4 | >38 | >7.5 | >24 | >38 | >6.3 | >22 | >15 | >2.0 | >9.4 |
| p Value | <4.8E-4 | <0.062 | <0.0027 | <4.8E-4 | <0.090 | <0.0036 | <0.011 | <0.57 | <0.038 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| 95% CI of | >4.9 | >0.90 | >3.0 | >4.9 | >0.75 | >2.7 | >1.9 | >0.18 | >1.1 |
| OR Quart4 | na | na | na | na | na | na | na | na | na |

**C-X-C motif chemokine 13**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.224 | 9.29 | 0.224 | 8.44 | 0.224 | 3.11 |
| Average | 10.4 | 107 | 10.4 | 104 | 10.4 | 39.9 |
| Stdev | 61.2 | 355 | 61.2 | 341 | 61.2 | 109 |
| p(t-test) |  | 2.4E-4 |  | 2.5E-4 |  | 0.082 |
| Min | 0.00269 | 0.0163 | 0.00269 | 0.0163 | 0.00269 | 0.0163 |
| Max | 832 | 1930 | 832 | 1850 | 832 | 440 |
| n (Samp) | 223 | 30 | 223 | 30 | 223 | 16 |
| n (Patient) | 223 | 30 | 223 | 30 | 223 | 16 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.772 | 8.40 | 0.772 | 5.14 | 0.772 | 5.14 |
| Average | 11.8 | 18.6 | 11.8 | 16.7 | 11.8 | 15.2 |
| Stdev | 53.0 | 27.8 | 53.0 | 27.8 | 53.0 | 28.4 |
| p(t-test) |  | 0.65 |  | 0.74 |  | 0.87 |
| Min | 0.00269 | 0.0174 | 0.00269 | 0.0174 | 0.00269 | 0.0222 |
| Max | 832 | 80.5 | 832 | 79.0 | 832 | 79.0 |
| n (Samp) | 375 | 13 | 375 | 13 | 375 | 7 |
| n (Patient) | 375 | 13 | 375 | 13 | 375 | 7 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.392 | 23.5 | 0.392 | 20.4 | 0.392 | 2.87 |
| Average | 12.0 | 139 | 12.0 | 135 | 12.0 | 45.2 |
| Stdev | 68.6 | 402 | 68.6 | 387 | 68.6 | 117 |
| p(t-test) |  | 1.9E-4 |  | 1.9E-4 |  | 0.10 |
| Min | 0.00269 | 0.0163 | 0.00269 | 0.0163 | 0.00269 | 0.0163 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 832 | 1930 | 832 | 1850 | 832 | 440 |
| n (Samp) | 173 | 23 | 173 | 23 | 173 | 14 |
| n (Patient) | 173 | 23 | 173 | 23 | 173 | 14 |
| | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.82 | 0.73 | 0.83 | 0.81 | 0.71 | 0.82 | 0.73 | 0.70 | 0.72 |
| SE | 0.048 | 0.081 | 0.054 | 0.049 | 0.082 | 0.055 | 0.073 | 0.11 | 0.079 |
| p | 5.8E-11 | 0.0040 | 1.5E-9 | 4.8E-10 | 0.011 | 3.4E-9 | 0.0014 | 0.078 | 0.0048 |
| nCohort 1 | 223 | 375 | 173 | 223 | 375 | 173 | 223 | 375 | 173 |
| nCohort 2 | 30 | 13 | 23 | 30 | 13 | 23 | 16 | 7 | 14 |
| Cutoff 1 | 4.27 | 2.65 | 2.84 | 2.84 | 2.57 | 2.84 | 1.18 | 3.20 | 2.32 |
| Sens 1 | 70% | 77% | 74% | 70% | 77% | 74% | 75% | 71% | 71% |
| Spec 1 | 80% | 67% | 71% | 74% | 66% | 71% | 65% | 70% | 70% |
| Cutoff 2 | 2.65 | 0.449 | 2.65 | 2.49 | 0.449 | 2.65 | 0.448 | 0.449 | 0.448 |
| Sens 2 | 80% | 85% | 83% | 80% | 85% | 83% | 81% | 86% | 86% |
| Spec 2 | 74% | 45% | 71% | 73% | 45% | 71% | 56% | 45% | 51% |
| Cutoff 3 | 0.0193 | 0.0193 | 0.448 | 0.0193 | 0.0193 | 0.448 | 0.0155 | 0.0193 | 0.0155 |
| Sens 3 | 90% | 92% | 91% | 90% | 92% | 91% | 100% | 100% | 100% |
| Spec 3 | 42% | 32% | 51% | 42% | 32% | 51% | 39% | 32% | 31% |
| Cutoff 4 | 1.82 | 3.20 | 2.49 | 1.82 | 3.20 | 2.49 | 1.82 | 3.20 | 2.49 |
| Sens 4 | 80% | 69% | 83% | 80% | 62% | 83% | 69% | 71% | 64% |
| Spec 4 | 70% | 70% | 71% | 70% | 70% | 71% | 70% | 70% | 71% |
| Cutoff 5 | 4.64 | 7.41 | 5.36 | 4.64 | 7.41 | 5.36 | 4.64 | 7.41 | 5.36 |
| Sens 5 | 67% | 54% | 65% | 60% | 46% | 65% | 38% | 43% | 36% |
| Spec 5 | 80% | 80% | 80% | 80% | 80% | 80% | 80% | 80% | 80% |
| Cutoff 6 | 12.3 | 18.7 | 14.4 | 12.3 | 18.7 | 14.4 | 12.3 | 18.7 | 14.4 |
| Sens 6 | 47% | 31% | 57% | 43% | 23% | 52% | 25% | 14% | 29% |
| Spec 6 | 90% | 90% | 90% | 90% | 90% | 90% | 90% | 90% | 90% |
| OR Quart 2 | >4.3 | >3.1 | >3.2 | >4.3 | >3.1 | >3.2 | >3.1 | >2.0 | >3.1 |
| p Value | <0.20 | <0.33 | <0.32 | <0.20 | <0.33 | <0.32 | <0.33 | <0.57 | <0.33 |
| 95% CI of | >0.46 | >0.32 | >0.32 | >0.46 | >0.32 | >0.32 | >0.31 | >0.18 | >0.31 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart2 | na | na | na | na | na | na | na | na | na |
| OR Quart 3 | >6.6 | >1.0 | >5.6 | >7.9 | >3.1 | >5.6 | >6.6 | >1.0 | >5.5 |
| p Value | <0.084 | <0.99 | <0.12 | <0.057 | <0.33 | <0.12 | <0.086 | <0.99 | <0.13 |
| 95% CI of | >0.77 | >0.062 | >0.63 | >0.94 | >0.32 | >0.63 | >0.76 | >0.062 | >0.61 |
| OR Quart3 | na | na | na | na | na | na | na | na | na |
| OR Quart 4 | >29 | >9.9 | >22 | >27 | >7.5 | >22 | >7.8 | >4.1 | >6.7 |
| p Value | <0.0013 | <0.031 | <0.0036 | <0.0017 | <0.061 | <0.0036 | <0.059 | <0.21 | <0.083 |
| 95% CI of | >3.7 | >1.2 | >2.7 | >3.4 | >0.91 | >2.7 | >0.93 | >0.45 | >0.78 |
| OR Quart4 | na | na | na | na | na | na | na | na | na |

**Insulin-like growth factor-binding protein 3**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 375 | 686 | 375 | 686 | 375 | 609 |
| Average | 692 | 2080 | 692 | 1580 | 692 | 840 |
| Stdev | 1130 | 3120 | 1130 | 2400 | 1130 | 578 |
| p(t-test) | | 1.1E-6 | | 3.9E-4 | | 0.55 |
| Min | 3.84 | 108 | 3.84 | 88.0 | 3.84 | 186 |
| Max | 12500 | 12500 | 12500 | 12500 | 12500 | 2060 |
| n (Samp) | 235 | 35 | 235 | 35 | 235 | 21 |
| n (Patient) | 235 | 35 | 235 | 35 | 235 | 21 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 574 | 862 | 574 | 862 | 574 | 1000 |
| Average | 949 | 1440 | 949 | 1370 | 949 | 1640 |
| Stdev | 1480 | 1900 | 1480 | 1860 | 1480 | 2210 |
| p(t-test) | | 0.19 | | 0.26 | | 0.13 |
| Min | 3.84 | 108 | 3.84 | 88.0 | 3.84 | 411 |

(continued)

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 12500 | 8160 | 12500 | 8160 | 12500 | 8160 |
| n (Samp) | 397 | 17 | 397 | 17 | 397 | 11 |
| n (Patient) | 397 | 17 | 397 | 17 | 397 | 11 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 434 | 993 | 434 | 993 | 434 | 533 |
| Average | 846 | 2640 | 846 | 1930 | 846 | 792 |
| Stdev | 1490 | 3590 | 1490 | 2790 | 1490 | 631 |
| p(t-test) | | 9.6E-6 | | 0.0028 | | 0.89 |
| Min | 3.84 | 186 | 3.84 | 186 | 3.84 | 186 |
| Max | 12500 | 12500 | 12500 | 12500 | 12500 | 2060 |
| n (Samp) | 190 | 25 | 190 | 25 | 190 | 16 |
| n (Patient) | 190 | 25 | 190 | 25 | 190 | 16 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.70 | 0.62 | 0.70 | 0.69 | 0.61 | 0.69 | 0.66 | 0.68 | 0.58 |
| SE | 0.052 | 0.074 | 0.061 | 0.052 | 0.074 | 0.062 | 0.067 | 0.090 | 0.077 |
| p | 7.3E-5 | 0.11 | 0.0011 | 2.0E-4 | 0.14 | 0.0025 | 0.019 | 0.049 | 0.33 |
| nCohort 1 | 235 | 397 | 190 | 235 | 397 | 190 | 235 | 397 | 190 |
| nCohort 2 | 35 | 17 | 25 | 35 | 17 | 25 | 21 | 11 | 16 |
| Cutoff 1 | 478 | 543 | 478 | 452 | 543 | 452 | 452 | 662 | 293 |
| Sens 1 | 71% | 71% | 72% | 71% | 71% | 72% | 71% | 73% | 75% |
| Spec 1 | 58% | 49% | 54% | 57% | 49% | 53% | 57% | 55% | 36% |
| Cutoff 2 | 388 | 411 | 388 | 323 | 411 | 323 | 323 | 543 | 267 |
| Sens 2 | 80% | 82% | 80% | 80% | 82% | 80% | 81% | 82% | 81% |
| Spec 2 | 51% | 40% | 46% | 44% | 40% | 38% | 44% | 49% | 33% |
| Cutoff 3 | 258 | 108 | 267 | 238 | 108 | 257 | 258 | 456 | 193 |
| Sens 3 | 91% | 94% | 92% | 91% | 94% | 92% | 90% | 91% | 94% |
| Spec 3 | 37% | 14% | 33% | 34% | 14% | 31% | 37% | 44% | 24% |
| Cutoff 4 | 736 | 1060 | 836 | 736 | 1060 | 836 | 736 | 1060 | 836 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 4 | 49% | 41% | 52% | 49% | 41% | 52% | 43% | 45% | 38% |
| Spec 4 | 70% | 70% | 70% | 70% | 70% | 70% | 70% | 70% | 70% |
| Cutoff 5 | 1100 | 1380 | 1130 | 1100 | 1380 | 1130 | 1100 | 1380 | 1130 |
| Sens 5 | 40% | 35% | 44% | 40% | 29% | 44% | 29% | 27% | 25% |
| Spec 5 | 80% | 80% | 80% | 80% | 80% | 80% | 80% | 80% | 80% |
| Cutoff 6 | 1500 | 1870 | 1560 | 1500 | 1870 | 1560 | 1500 | 1870 | 1560 |
| Sens 6 | 34% | 24% | 40% | 34% | 18% | 40% | 24% | 18% | 12% |
| Spec 6 | 90% | 90% | 90% | 90% | 90% | 90% | 90% | 90% | 90% |
| OR Quart 2 | 3.7 | 2.0 | 5.3 | 3.7 | 2.0 | 2.6 | >5.4 | >3.1 | 2.0 |
| p Value | 0.11 | 0.42 | 0.13 | 0.11 | 0.42 | 0.27 | <0.13 | <0.33 | 0.42 |
| 95% CI of | 0.75 | 0.36 | 0.60 | 0.75 | 0.36 | 0.48 | >0.62 | >0.32 | 0.36 |
| OR Quart2 | 19 | 11 | 47 | 19 | 11 | 14 | na | na | 12 |
| OR Quart 3 | 7.1 | 2.0 | 9.0 | 7.1 | 2.0 | 3.8 | >9.1 | >4.2 | 3.3 |
| p Value | 0.013 | 0.42 | 0.041 | 0.013 | 0.42 | 0.11 | <0.040 | <0.21 | 0.16 |
| 95% CI of | 1.5 | 0.37 | 1.1 | 1.5 | 0.37 | 0.75 | >1.1 | >0.46 | 0.63 |
| OR Quart3 | 33 | 11 | 75 | 33 | 11 | 19 | na | na | 17 |
| OR Quart 4 | 8.4 | 3.6 | 13 | 8.4 | 3.6 | 6.5 | >9.1 | >4.2 | 2.0 |
| p Value | 0.0062 | 0.11 | 0.015 | 0.0062 | 0.11 | 0.018 | <0.040 | <0.21 | 0.42 |
| 95% CI of | 1.8 | 0.74 | 1.7 | 1.8 | 0.74 | 1.4 | >1.1 | >0.46 | 0.36 |
| OR Quart4 | 39 | 18 | 110 | 39 | 18 | 31 | na | na | 12 |

**Immunoglogulin G1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3510 | 14800 | 3510 | 14600 | 3510 | 9360 |
| Average | 6360 | 16700 | 6360 | 15900 | 6360 | 11200 |
| Stdev | 8760 | 14300 | 8760 | 14100 | 8760 | 6710 |
| p(t-test) | | 6.8E-8 | | 5.0E-7 | | 0.032 |
| Min | 59.7 | 980 | 59.7 | 980 | 59.7 | 2380 |
| Max | 80000 | 80000 | 80000 | 80000 | 80000 | 25300 |

(continued)

| Immunoglogulin G1 | | | | | | | |
|---|---|---|---|---|---|---|---|
| sCr or UO | 0hr prior to AKI stage | | 24hr prior to AKI stage | | | 48hr prior to AKI stage | |
| | Cohort 1 | Cohort 2 | Cohort 1 | Cohort 2 | | Cohort 1 | Cohort 2 |
| n (Samp) | 222 | 30 | 222 | 30 | | 222 | 16 |
| n (Patient) | 222 | 30 | 222 | 30 | | 222 | 16 |
| sCr only | 0hr prior to AKI stage | | 24hr prior to AKI stage | | | 48hr prior to AKI stage | |
| | Cohort 1 | Cohort 2 | Cohort 1 | | Cohort 2 | Cohort 1 | Cohort 2 |
| Median | 4660 | 11200 | 4660 | | 11200 | 4660 | 9840 |
| Average | 7920 | 13300 | 7920 | | 12300 | 7920 | 10200 |
| Stdev | 9560 | 8270 | 9560 | | 7830 | 9560 | 4610 |
| p(t-test) | | 0.046 | | | 0.099 | | 0.53 |
| Min | 59.7 | 980 | 59.7 | | 980 | 59.7 | 2880 |
| Max | 80000 | 28300 | 80000 | | 28300 | 80000 | 15300 |
| n (Samp) | 373 | 13 | 373 | | 13 | 373 | 7 |
| n (Patient) | 373 | 13 | 373 | | 13 | 373 | 7 |
| | | | | | | | |
| UO only | 0hr prior to AKI stage | | 24hr prior to AKI stage | | | 48hr prior to AKI stage | |
| | Cohort 1 | Cohort 2 | Cohort 1 | | Cohort 2 | Cohort 1 | Cohort 2 |
| Median | 3840 | 14900 | 3840 | | 14800 | 3840 | 10900 |
| Average | 6750 | 18700 | 6750 | | 18000 | 6750 | 11800 |
| Stdev | 9200 | 15600 | 9200 | | 15300 | 9200 | 7010 |
| p(t-test) | | 2.8E-7 | | | 1.2E-6 | | 0.046 |
| Min | 59.7 | 2880 | 59.7 | | 2880 | 59.7 | 2380 |
| Max | 80000 | 80000 | 80000 | | 80000 | 80000 | 25300 |
| n(Samp) | 171 | 23 | 171 | | 23 | 171 | 14 |
| n (Patient) | 171 | 23 | 171 | | 23 | 171 | 14 |
| | | | | | | | |
| | 0hr prior to AKI stage | | | 24hr prior to AKI stage | | | 48hr prior to AKI stage | | |
| | sCr or UO | sCr only | UO only | sCr or UO | sCr only | UO only | sCr or UO | sCr only | UO only |
| AUC | 0.84 | 0.73 | 0.86 | 0.83 | 0.71 | 0.86 | 0.77 | 0.70 | 0.76 |
| SE | 0.047 | 0.081 | 0.050 | 0.048 | 0.082 | 0.051 | 0.070 | 0.11 | 0.076 |
| p | 5.5E-13 | 0.0051 | 4.7E-13 | 7.0E-12 | 0.012 | 1.8E-12 | 1.1E-4 | 0.077 | 5.1E-4 |
| nCohort 1 | 222 | 373 | 171 | 222 | 373 | 171 | 222 | 373 | 171 |
| nCohort 2 | 30 | 13 | 23 | 30 | 13 | 23 | 16 | 7 | 14 |

(continued)

| | | 0hr prior to AKI stage | | | 24hr prior to AKI stage | | | 48hr prior to AKI stage | | |
|---|---|---|---|---|---|---|---|---|---|---|
| | | sCr or UO | sCr only | UO only | sCr or UO | sCr only | UO only | sCr or UO | sCr only | UO only |
| Cutoff 1 | | 9450 | 7480 | 11000 | 9450 | 6410 | 11000 | 6410 | 8560 | 8690 |
| Sens 1 | | 70% | 77% | 74% | 70% | 77% | 74% | 75% | 71% | 71% |
| Spec 1 | | 80% | 66% | 85% | 80% | 61% | 85% | 72% | 70% | 80% |
| Cutoff 2 | | 8150 | 6410 | 8710 | 8150 | 5190 | 8710 | 6150 | 6410 | 3840 |
| Sens 2 | | 80% | 85% | 83% | 80% | 85% | 83% | 81% | 86% | 86% |
| Spec 2 | | 77% | 61% | 80% | 77% | 53% | 80% | 72% | 61% | 50% |
| Cutoff 3 | | 6150 | 2840 | 6150 | 5190 | 2840 | 6150 | 2840 | 2840 | 2840 |
| Sens 3 | | 90% | 92% | 91% | 90% | 92% | 91% | 94% | 100% | 93% |
| Spec 3 | | 72% | 32% | 71% | 64% | 32% | 71% | 43% | 32% | 38% |
| Cutoff 4 | | 5990 | 8650 | 6090 | 5990 | 8650 | 6090 | 5990 | 8650 | 6090 |
| Sens 4 | | 90% | 62% | 91% | 87% | 62% | 91% | 81% | 57% | 79% |
| Spec 4 | | 70% | 70% | 70% | 70% | 70% | 70% | 70% | 70% | 70% |
| Cutoff 5 | | 9450 | 12900 | 8710 | 9450 | 12900 | 8710 | 9450 | 12900 | 8710 |
| Sens 5 | | 70% | 46% | 83% | 70% | 46% | 83% | 50% | 43% | 64% |
| Spec 5 | | 80% | 80% | 80% | 80% | 80% | 80% | 80% | 80% | 80% |
| Cutoff 6 | | 15800 | 17100 | 15800 | 15800 | 17100 | 15800 | 15800 | 17100 | 15800 |
| Sens 6 | | 40% | 38% | 43% | 30% | 23% | 35% | 19% | 0% | 21% |
| Spec 6 | | 90% | 90% | 90% | 90% | 90% | 90% | 90% | 90% | 90% |
| OR Quart 2 | | 1.0 | 0.99 | >1.0 | 1.0 | 0.99 | >1.0 | >2.0 | >1.0 | >3.2 |
| p Value | | 1.0 | 0.99 | <1.0 | 1.0 | 0.99 | <1.0 | <0.57 | <0.99 | <0.32 |
| 95% CI of | | 0.061 | 0.061 | >0.061 | 0.061 | 0.061 | >0.061 | >0.18 | >0.062 | >0.32 |
| OR Quart2 | | 16 | 16 | na | 16 | 16 | na | na | na | na |
| OR Quart 3 | | 9.0 | 4.1 | >5.6 | 9.0 | 4.1 | >5.6 | >3.2 | >3.1 | >1.0 |
| p Value | | 0.041 | 0.21 | <0.12 | 0.041 | 0.21 | <0.12 | <0.33 | <0.33 | <0.99 |
| 95% CI of | | 1.1 | 0.45 | >0.63 | 1.1 | 0.45 | >0.63 | >0.32 | >0.32 | >0.062 |
| OR Quart3 | | 74 | 38 | na | 74 | 38 | na | na | na | na |
| OR Quart 4 | | 29 | 7.4 | >26 | 29 | 7.4 | >26 | >13 | >3.1 | >12 |
| p Value | | 0.0013 | 0.064 | <0.0021 | 0.0013 | 0.064 | <0.0021 | <0.015 | <0.33 | <0.019 |
| 95% CI of | | 3.7 | 0.89 | >3.2 | 3.7 | 0.89 | >3.2 | >1.7 | >0.32 | >1.5 |

| OR Quart4 | 220 | 61 | na | 220 | 61 | na | na | na | na |
|---|---|---|---|---|---|---|---|---|---|

(continued)

| Immunoglogulin G2 | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 9710 | 38600 | 9710 | 36400 | 9710 | 27800 |
| Average | 20300 | 71300 | 20300 | 65400 | 20300 | 46400 |
| Stdev | 34400 | 73800 | 34400 | 72200 | 34400 | 58500 |
| p(t-test) | | 7.4E-10 | | 3.2E-8 | | 0.0059 |
| Min | 119 | 2380 | 119 | 2380 | 119 | 4940 |
| Max | 240000 | 240000 | 240000 | 240000 | 240000 | 240000 |
| n (Samp) | 222 | 30 | 222 | 30 | 222 | 16 |
| n (Patient) | 222 | 30 | 222 | 30 | 222 | 16 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 13000 | 52100 | 13000 | 36800 | 13000 | 20100 |
| Average | 26100 | 73800 | 26100 | 62100 | 26100 | 62000 |
| Stdev | 40800 | 71300 | 40800 | 67800 | 40800 | 84800 |
| p(t-test) | | 7.0E-5 | | 0.0025 | | 0.025 |
| Min | 119 | 2380 | 119 | 2380 | 119 | 4940 |
| Max | 240000 | 240000 | 240000 | 240000 | 240000 | 240000 |
| n (Samp) | 373 | 13 | 373 | 13 | 373 | 7 |
| n (Patient) | 373 | 13 | 373 | 13 | 373 | 7 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 10700 | 40500 | 10700 | 40500 | 10700 | 35700 |
| Average | 22500 | 81500 | 22500 | 77900 | 22500 | 54600 |
| Stdev | 39100 | 84000 | 39100 | 81100 | 39100 | 62500 |
| p(t-test) | | 4.1E-8 | | 1.7E-7 | | 0.0056 |
| Min | 334 | 8300 | 334 | 8300 | 334 | 8300 |
| Max | 240000 | 240000 | 240000 | 240000 | 240000 | 240000 |
| n (Samp) | 171 | 23 | 171 | 23 | 171 | 14 |
| n (Patient) | 171 | 23 | 171 | 23 | 171 | 14 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.80 | 0.73 | 0.81 | 0.79 | 0.70 | 0.81 | 0.74 | 0.65 | 0.76 |
| SE | 0.050 | 0.081 | 0.056 | 0.051 | 0.083 | 0.056 | 0.073 | 0.11 | 0.077 |
| p | 3.6E-9 | 0.0041 | 1.7E-8 | 1.2E-8 | 0.018 | 2.2E-8 | 9.4E-4 | 0.18 | 8.5E-4 |
| nCohort 1 | 222 | 373 | 171 | 222 | 373 | 171 | 222 | 373 | 171 |
| nCohort 2 | 30 | 13 | 23 | 30 | 13 | 23 | 16 | 7 | 14 |
| Cutoff 1 | 19300 | 20100 | 14800 | 19300 | 13300 | 14800 | 13700 | 13300 | 14800 |
| Sens 1 | 70% | 77% | 74% | 70% | 77% | 74% | 75% | 71% | 71% |
| Spec 1 | 73% | 67% | 65% | 73% | 52% | 65% | 64% | 52% | 65% |
| Cutoff 2 | 14500 | 8240 | 14500 | 14500 | 8240 | 14500 | 9820 | 8240 | 9820 |
| Sens 2 | 80% | 85% | 83% | 80% | 85% | 83% | 81% | 86% | 86% |
| Spec 2 | 66% | 33% | 64% | 66% | 33% | 64% | 50% | 33% | 46% |
| Cutoff 3 | 8830 | 4910 | 12300 | 8830 | 4910 | 12300 | 8240 | 4910 | 8540 |
| Sens 3 | 90% | 92% | 91% | 90% | 92% | 91% | 94% | 100% | 93% |
| Spec 3 | 48% | 19% | 54% | 48% | 19% | 54% | 45% | 19% | 42% |
| Cutoff 4 | 17500 | 21800 | 18100 | 17500 | 21800 | 18100 | 17500 | 21800 | 18100 |
| Sens 4 | 70% | 69% | 70% | 70% | 62% | 70% | 56% | 43% | 57% |
| Spec 4 | 70% | 70% | 70% | 70% | 70% | 70% | 70% | 70% | 70% |
| Cutoff 5 | 23700 | 31900 | 22900 | 23700 | 31900 | 22900 | 23700 | 31900 | 22900 |
| Sens 5 | 63% | 62% | 65% | 60% | 54% | 65% | 50% | 43% | 57% |
| Spec 5 | 80% | 80% | 80% | 80% | 80% | 80% | 80% | 80% | 80% |
| Cutoff 6 | 40900 | 52000 | 44400 | 40900 | 52000 | 44400 | 40900 | 52000 | 44400 |
| Sens 6 | 47% | 54% | 48% | 43% | 46% | 48% | 31% | 43% | 36% |
| Spec 6 | 90% | 90% | 90% | 90% | 90% | 90% | 90% | 90% | 90% |
| OR Quart 2 | 3.1 | 0.49 | >3.1 | 3.1 | 0.49 | >3.1 | >4.2 | 1.0 | >3.2 |
| p Value | 0.33 | 0.56 | <0.33 | 0.33 | 0.56 | <0.33 | <0.20 | 1.0 | <0.32 |
| 95% CI of | 0.31 | 0.044 | >0.31 | 0.31 | 0.044 | >0.31 | >0.46 | 0.062 | >0.32 |
| OR Quart2 | 31 | 5.5 | na | 31 | 5.5 | na | na | 16 | na |
| OR Quart 3 | 7.8 | 1.0 | >5.6 | 9.0 | 1.5 | >5.6 | >4.3 | 2.0 | >3.2 |
| p Value | 0.059 | 1.0 | <0.12 | 0.041 | 0.65 | <0.12 | <0.20 | 0.57 | <0.32 |
| 95% CI of | 0.92 | 0.14 | >0.63 | 1.1 | 0.25 | >0.63 | >0.47 | 0.18 | >0.32 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart3 | 65 | 7.2 | na | 74 | 9.3 | na | na | 23 | na |
| OR Quart 4 | 27 | 4.2 | >21 | 25 | 3.7 | >21 | >9.1 | 3.1 | >9.4 |
| p Value | 0.0017 | 0.073 | <0.0039 | 0.0021 | 0.11 | <0.0039 | <0.041 | 0.34 | <0.038 |
| 95% CI of | 3.5 | 0.87 | >2.7 | 3.2 | 0.74 | >2.7 | >1.1 | 0.31 | >1.1 |
| OR Quart4 | 210 | 20 | na | 190 | 18 | na | na | 30 | na |
| **Interleukin-11** | | | | | | | | | |
| sCr or UO | 0hr prior to AKI stage | | | 24hr prior to AKI stage | | | 48hr prior to AKI stage | | |
|  | Cohort 1 | | Cohort 2 | Cohort 1 | | Cohort 2 | Cohort 1 | | Cohort 2 |
| Median | 129 | | 344 | 129 | | 270 | 129 | | 266 |
| Average | 208 | | 537 | 208 | | 472 | 208 | | 492 |
| Stdev | 256 | | 608 | 256 | | 560 | 256 | | 637 |
| p(t-test) | | | 2.2E-7 | | | 1.5E-5 | | | 2.6E-4 |
| Min | 0.154 | | 48.0 | 0.154 | | 48.0 | 0.154 | | 85.8 |
| Max | 1980 | | 2900 | 1980 | | 2590 | 1980 | | 2140 |
| n (Samp) | 223 | | 30 | 223 | | 30 | 223 | | 16 |
| n (Patient) | 223 | | 30 | 223 | | 30 | 223 | | 16 |
| sCr only | 0hr prior to AKI stage | | | 24hr prior to AKI stage | | | 48hr prior to AKI stage | | |
|  | Cohort 1 | | Cohort 2 | Cohort 1 | | Cohort 2 | Cohort 1 | | Cohort 2 |
| Median | 174 | | 295 | 174 | | 219 | 174 | | 212 |
| Average | 285 | | 322 | 285 | | 313 | 285 | | 237 |
| Stdev | 315 | | 259 | 315 | | 262 | 315 | | 112 |
| p(t-test) | | | 0.67 | | | 0.75 | | | 0.69 |
| Min | 0.154 | | 48.0 | 0.154 | | 48.0 | 0.154 | | 115 |
| Max | 2260 | | 1040 | 2260 | | 1040 | 2260 | | 436 |
| n (Samp) | 375 | | 13 | 375 | | 13 | 375 | | 7 |
| n (Patient) | 375 | | 13 | 375 | | 13 | 375 | | 7 |
|  | | | | | | | | | |
| UO only | 0hr prior to AKI stage | | | 24hr prior to AKI stage | | | 48hr prior to AKI stage | | |
|  | Cohort 1 | | Cohort 2 | Cohort 1 | | Cohort 2 | Cohort 1 | | Cohort 2 |
| Median | 179 | | 396 | 179 | | 295 | 179 | | 321 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 263 | 664 | 263 | 585 | 263 | 582 |
| Stdev | 283 | 683 | 283 | 640 | 283 | 682 |
| p(t-test) | | 7.1E-7 | | 3.7E-5 | | 5.7E-4 |
| Min | 2.83 | 90.9 | 2.83 | 90.9 | 2.83 | 85.8 |
| Max | 1980 | 2900 | 1980 | 2590 | 1980 | 2140 |
| n(Samp) | 173 | 23 | 173 | 23 | 173 | 14 |
| n (Patient) | 173 | 23 | 173 | 23 | 173 | 14 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.77 | 0.60 | 0.76 | 0.74 | 0.59 | 0.71 | 0.73 | 0.57 | 0.68 |
| SE | 0.052 | 0.084 | 0.061 | 0.054 | 0.084 | 0.063 | 0.074 | 0.11 | 0.081 |
| P | 1.7E-7 | 0.23 | 2.4E-5 | 1.0E-5 | 0.29 | 0.0010 | 0.0019 | 0.54 | 0.026 |
| nCohort 1 | 223 | 375 | 173 | 223 | 375 | 173 | 223 | 375 | 173 |
| nCohort 2 | 30 | 13 | 23 | 30 | 13 | 23 | 16 | 7 | 14 |
| Cutoff 1 | 230 | 135 | 250 | 180 | 135 | 212 | 143 | 179 | 235 |
| Sens 1 | 70% | 77% | 74% | 70% | 77% | 74% | 75% | 71% | 71% |
| Spec 1 | 73% | 40% | 64% | 64% | 40% | 58% | 57% | 52% | 62% |
| Cutoff 2 | 143 | 114 | 212 | 135 | 114 | 143 | 135 | 135 | 114 |
| Sens 2 | 80% | 85% | 83% | 80% | 85% | 83% | 81% | 86% | 86% |
| Spec 2 | 57% | 33% | 58% | 55% | 33% | 44% | 55% | 40% | 34% |
| Cutoff 3 | 114 | 110 | 128 | 110 | 110 | 114 | 85.8 | 114 | 89.1 |
| Sens 3 | 90% | 92% | 91% | 90% | 92% | 91% | 94% | 100% | 93% |
| Spec 3 | 47% | 32% | 36% | 46% | 32% | 34% | 37% | 33% | 24% |
| Cutoff 4 | 212 | 301 | 299 | 212 | 301 | 299 | 212 | 301 | 299 |
| Sens 4 | 77% | 38% | 61% | 67% | 38% | 48% | 62% | 14% | 50% |
| Spec 4 | 70% | 70% | 71% | 70% | 70% | 71% | 70% | 70% | 71% |
| Cutoff 5 | 301 | 461 | 383 | 301 | 461 | 383 | 301 | 461 | 383 |
| Sens 5 | 53% | 15% | 52% | 43% | 15% | 43% | 44% | 0% | 43% |
| Spec 5 | 80% | 80% | 80% | 80% | 80% | 80% | 80% | 80% | 80% |
| Cutoff 6 | 487 | 694 | 569 | 487 | 694 | 569 | 487 | 694 | 569 |
| Sens 6 | 33% | 8% | 39% | 30% | 8% | 35% | 19% | 0% | 29% |
| Spec 6 | 90% | 90% | 90% | 90% | 90% | 90% | 90% | 90% | 90% |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart 2 | 4.2 | 3.1 | 3.1 | 4.2 | 3.1 | 2.1 | >3.1 | >2.0 | 0.98 |
| p Value | 0.20 | 0.34 | 0.33 | 0.20 | 0.34 | 0.41 | <0.33 | <0.57 | 0.98 |
| 95% CI of | 0.46 | 0.31 | 0.31 | 0.46 | 0.31 | 0.36 | >0.31 | >0.18 | 0.13 |
| OR Quart2 | 39 | 30 | 31 | 39 | 30 | 12 | na | na | 7.3 |
| OR Quart 3 | 7.8 | 5.2 | 8.0 | 12 | 5.2 | 3.3 | >5.4 | >4.2 | 1.5 |
| p Value | 0.059 | 0.13 | 0.056 | 0.021 | 0.13 | 0.16 | <0.13 | <0.20 | 0.67 |
| 95% CI of | 0.92 | 0.60 | 0.95 | 1.4 | 0.60 | 0.63 | >0.61 | >0.46 | 0.24 |
| OR Quart3 | 65 | 46 | 68 | 94 | 46 | 17 | na | na | 9.4 |
| OR Quart 4 | 24 | 4.1 | 16 | 19 | 4.1 | 6.8 | >9.1 | >1.0 | 3.8 |
| p Value | 0.0023 | 0.21 | 0.0098 | 0.0051 | 0.21 | 0.016 | <0.041 | <1.0 | 0.10 |
| 95% CI of | 3.1 | 0.45 | 1.9 | 2.4 | 0.45 | 1.4 | >1.1 | >0.062 | 0.76 |
| OR Quart4 | 190 | 38 | 130 | 150 | 38 | 33 | na | na | 20 |

**Interleukin-2 receptor alpha chain**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 634 | 1730 | 634 | 1720 | 634 | 980 |
| Average | 1020 | 2350 | 1020 | 2180 | 1020 | 2070 |
| Stdev | 1210 | 2340 | 1210 | 2280 | 1210 | 2470 |
| p(t-test) |  | 1.6E-6 |  | 2.4E-5 |  | 0.0026 |
| Min | 0.0708 | 0.118 | 0.0708 | 0.118 | 0.0708 | 437 |
| Max | 10400 | 10400 | 10400 | 10400 | 10400 | 10400 |
| n (Samp) | 223 | 30 | 223 | 30 | 223 | 16 |
| n (Patient) | 223 | 30 | 223 | 30 | 223 | 16 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 880 | 1300 | 880 | 1300 | 880 | 2450 |

(continued)

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 1390 | 2050 | 1390 | 2040 | 1390 | 2570 |
| Stdev | 1620 | 2100 | 1620 | 2100 | 1620 | 2370 |
| p(t-test) | | 0.16 | | 0.16 | | 0.059 |
| Min | 0.0708 | 0.118 | 0.0708 | 0.118 | 0.0708 | 437 |
| Max | 10400 | 7190 | 10400 | 7190 | 10400 | 7190 |
| n (Samp) | 375 | 13 | 375 | 13 | 375 | 7 |
| n (Patient) | 375 | 13 | 375 | 13 | 375 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 767 | 2490 | 767 | 2200 | 767 | 1140 |
| Average | 1130 | 2710 | 1130 | 2480 | 1130 | 2170 |
| Stdev | 1300 | 2450 | 1300 | 2410 | 1300 | 2610 |
| p(t-test) | | 3.0E-6 | | 5.1E-5 | | 0.0095 |
| Min | 0.116 | 50.9 | 0.116 | 50.9 | 0.116 | 437 |
| Max | 10400 | 10400 | 10400 | 10400 | 10400 | 10400 |
| n (Samp) | 173 | 23 | 173 | 23 | 173 | 14 |
| n (Patient) | 173 | 23 | 173 | 23 | 173 | 14 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.70 | 0.59 | 0.74 | 0.69 | 0.59 | 0.73 | 0.70 | 0.69 | 0.68 |
| SE | 0.056 | 0.084 | 0.061 | 0.056 | 0.084 | 0.063 | 0.075 | 0.11 | 0.082 |
| p | 2.8E-4 | 0.31 | 7.2E-5 | 6.9E-4 | 0.31 | 3.1E-4 | 0.0094 | 0.098 | 0.030 |
| nCohort 1 | 223 | 375 | 173 | 223 | 375 | 173 | 223 | 375 | 173 |
| nCohort 2 | 30 | 13 | 23 | 30 | 13 | 23 | 16 | 7 | 14 |
| Cutoff 1 | 750 | 493 | 1070 | 750 | 493 | 954 | 712 | 960 | 750 |
| Sens 1 | 70% | 77% | 74% | 70% | 77% | 74% | 75% | 71% | 71% |
| Spec 1 | 53% | 32% | 63% | 53% | 32% | 59% | 52% | 53% | 49% |
| Cutoff 2 | 491 | 420 | 491 | 491 | 420 | 491 | 594 | 594 | 491 |
| Sens 2 | 80% | 85% | 83% | 80% | 85% | 83% | 81% | 86% | 86% |
| Spec 2 | 44% | 29% | 41% | 44% | 29% | 41% | 48% | 38% | 41% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 3 | 384 | 55.0 | 411 | 384 | 55.0 | 411 | 484 | 420 | 484 |
| Sens 3 | 90% | 92% | 91% | 90% | 92% | 91% | 94% | 100% | 93% |
| Spec 3 | 40% | 9% | 38% | 40% | 9% | 38% | 44% | 29% | 41% |
| Cutoff 4 | 1250 | 1600 | 1270 | 1250 | 1600 | 1270 | 1250 | 1600 | 1270 |
| Sens 4 | 60% | 46% | 65% | 57% | 46% | 61% | 44% | 57% | 50% |
| Spec 4 | 70% | 70% | 71% | 70% | 70% | 71% | 70% | 70% | 71% |
| Cutoff 5 | 1690 | 2070 | 1890 | 1690 | 2070 | 1890 | 1690 | 2070 | 1890 |
| Sens 5 | 50% | 46% | 57% | 50% | 46% | 57% | 44% | 57% | 43% |
| Spec 5 | 80% | 80% | 80% | 80% | 80% | 80% | 80% | 80% | 80% |
| Cutoff 6 | 2530 | 3270 | 2620 | 2530 | 3270 | 2620 | 2530 | 3270 | 2620 |
| Sens 6 | 37% | 23% | 43% | 27% | 23% | 30% | 19% | 29% | 21% |
| Spec 6 | 90% | 90% | 90% | 90% | 90% | 90% | 90% | 90% | 90% |
| OR Quart 2 | 2.1 | 1.5 | 5.5 | 2.1 | 1.5 | 5.5 | >4.2 | >2.0 | >5.5 |
| p Value | 0.31 | 0.65 | 0.13 | 0.31 | 0.65 | 0.13 | <0.20 | <0.57 | <0.13 |
| 95% CI of | 0.50 | 0.25 | 0.61 | 0.50 | 0.25 | 0.61 | >0.46 | >0.18 | >0.61 |
| OR Quart2 | 8.8 | 9.3 | 49 | 8.8 | 9.3 | 49 | na | na | na |
| OR Quart 3 | 2.1 | 1.0 | 4.3 | 2.1 | 1.0 | 4.3 | >5.4 | >1.0 | >3.1 |
| p Value | 0.31 | 1.0 | 0.20 | 0.31 | 1.0 | 0.20 | <0.13 | <0.99 | <0.33 |
| 95% CI of | 0.50 | 0.14 | 0.46 | 0.50 | 0.14 | 0.46 | >0.61 | >0.062 | >0.31 |
| OR Quart3 | 8.8 | 7.2 | 40 | 8.8 | 7.2 | 40 | na | na | na |
| OR Quart 4 | 6.1 | 3.1 | 17 | 6.1 | 3.1 | 17 | >7.8 | >4.1 | >6.7 |
| p Value | 0.0061 | 0.17 | 0.0072 | 0.0061 | 0.17 | 0.0072 | <0.059 | <0.21 | <0.083 |
| 95% CI of | 1.7 | 0.62 | 2.2 | 1.7 | 0.62 | 2.2 | >0.93 | >0.45 | >0.78 |
| OR Quart4 | 22 | 16 | 140 | 22 | 16 | 140 | na | na | na |

**Neutrophil collagenase**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3750 | 21100 | 3750 | 20800 | 3750 | 20800 |
| Average | 17900 | 92500 | 17900 | 89700 | 17900 | 60700 |

(continued)

| Neutrophil collagenase | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Stdev | 56800 | 164000 | 56800 | 165000 | 56800 | 137000 |
| p(t-test) | | 4.4E-7 | | 1.1E-6 | | 0.0059 |
| Min | 0.114 | 25.8 | 0.114 | 25.8 | 0.114 | 580 |
| Max | 670000 | 649000 | 670000 | 649000 | 670000 | 625000 |
| n (Samp) | 235 | 34 | 235 | 34 | 235 | 20 |
| n (Patient) | 235 | 34 | 235 | 34 | 235 | 20 |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 6390 | 10800 | 6390 | 10800 | 6390 | 21100 |
| Average | 25600 | 113000 | 25600 | 86800 | 25600 | 37900 |
| Stdev | 65500 | 215000 | 65500 | 175000 | 65500 | 47300 |
| p(t-test) | | 5.6E-6 | | 7.7E-4 | | 0.54 |
| Min | 0.114 | 25.8 | 0.114 | 25.8 | 0.114 | 2660 |
| Max | 670000 | 649000 | 670000 | 649000 | 670000 | 151000 |
| n (Samp) | 398 | 17 | 398 | 17 | 398 | 11 |
| n (Patient) | 398 | 17 | 398 | 17 | 398 | 11 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 4250 | 48900 | 4250 | 41500 | 4250 | 21000 |
| Average | 14300 | 131000 | 14300 | 110000 | 14300 | 114000 |
| Stdev | 32500 | 200000 | 32500 | 178000 | 32500 | 215000 |
| p(t-test) | | 2.8E-12 | | 1.9E-10 | | 3.3E-8 |
| Min | 0.114 | 580 | 0.114 | 580 | 0.114 | 580 |
| Max | 300000 | 649000 | 300000 | 649000 | 300000 | 649000 |
| n (Samp) | 190 | 24 | 190 | 24 | 190 | 15 |
| n (Patient) | 190 | 24 | 190 | 24 | 190 | 15 |
| | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.75 | 0.61 | 0.82 | 0.74 | 0.60 | 0.81 | 0.75 | 0.67 | 0.76 |
| SE | 0.051 | 0.074 | 0.054 | 0.051 | 0.074 | 0.055 | 0.065 | 0.091 | 0.074 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p | 1.2E-6 | 0.13 | 1.8E-9 | 3.1E-6 | 0.18 | 1.4E-8 | 1.0E-4 | 0.057 | 3.2E-4 |
| nCohort 1 | 235 | 398 | 190 | 235 | 398 | 190 | 235 | 398 | 190 |
| nCohort 2 | 34 | 17 | 24 | 34 | 17 | 24 | 20 | 11 | 15 |
| Cutoff 1 | 10800 | 5530 | 20000 | 7010 | 5530 | 14600 | 7010 | 6050 | 7160 |
| Sens 1 | 71% | 71% | 71% | 71% | 71% | 71% | 70% | 73% | 73% |
| Spec 1 | 72% | 46% | 82% | 64% | 46% | 76% | 64% | 48% | 63% |
| Cutoff 2 | 3260 | 3170 | 11900 | 3260 | 3000 | 7160 | 5930 | 5930 | 5930 |
| Sens 2 | 82% | 82% | 83% | 82% | 82% | 83% | 80% | 82% | 80% |
| Spec 2 | 46% | 33% | 74% | 46% | 32% | 63% | 60% | 48% | 58% |
| Cutoff 3 | 2710 | 112 | 3260 | 2630 | 112 | 3260 | 3260 | 3170 | 3260 |
| Sens 3 | 91% | 94% | 92% | 91% | 94% | 92% | 90% | 91% | 93% |
| Spec 3 | 42% | 1% | 42% | 41% | 1% | 42% | 46% | 33% | 42% |
| Cutoff 4 | 9820 | 16300 | 9300 | 9820 | 16300 | 9300 | 9820 | 16300 | 9300 |
| Sens 4 | 71% | 47% | 83% | 68% | 47% | 79% | 65% | 55% | 67% |
| Spec 4 | 70% | 70% | 70% | 70% | 70% | 70% | 70% | 70% | 70% |
| Cutoff 5 | 18200 | 27300 | 19400 | 18200 | 27300 | 19400 | 18200 | 27300 | 19400 |
| Sens 5 | 59% | 35% | 71% | 56% | 35% | 67% | 55% | 36% | 60% |
| Spec 5 | 80% | 80% | 80% | 80% | 80% | 80% | 80% | 80% | 80% |
| Cutoff 6 | 33500 | 55700 | 32200 | 33500 | 55700 | 32200 | 33500 | 55700 | 32200 |
| Sens 6 | 41% | 29% | 54% | 41% | 29% | 54% | 35% | 27% | 47% |
| Spec 6 | 90% | 90% | 90% | 90% | 90% | 90% | 90% | 90% | 90% |
| OR Quart 2 | 1.4 | 3.1 | 2.0 | 1.4 | 3.1 | 2.0 | 2.0 | >4.2 | 1.0 |
| p Value | 0.70 | 0.17 | 0.58 | 0.70 | 0.17 | 0.58 | 0.58 | <0.21 | 1.0 |
| 95% CI of | 0.29 | 0.61 | 0.18 | 0.29 | 0.61 | 0.18 | 0.18 | >0.46 | 0.061 |
| OR Quart2 | 6.3 | 16 | 23 | 6.3 | 16 | 23 | 23 | na | 16 |
| OR Quart 3 | 2.5 | 0.99 | 4.2 | 2.9 | 0.99 | 5.4 | 6.4 | >2.0 | 4.3 |
| p Value | 0.20 | 0.99 | 0.20 | 0.13 | 0.99 | 0.13 | 0.090 | <0.56 | 0.20 |
| 95% CI of | 0.62 | 0.14 | 0.46 | 0.73 | 0.14 | 0.61 | 0.75 | >0.18 | 0.46 |
| OR Quart3 | 10 | 7.2 | 39 | 11 | 7.2 | 48 | 55 | na | 39 |
| OR Quart 4 | 8.9 | 3.6 | 24 | 8.3 | 3.6 | 22 | 13 | >5.2 | 10 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| p Value | 7.5E-4 | 0.11 | 0.0025 | 0.0011 | 0.11 | 0.0034 | 0.016 | <0.14 | 0.029 |
| 95% CI of | 2.5 | 0.74 | 3.0 | 2.3 | 0.74 | 2.8 | 1.6 | >0.60 | 1.3 |
| OR Quart4 | 32 | 18 | 190 | 30 | 18 | 170 | 100 | na | 86 |

**Protransforming growth factor alpha**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 6.37 | 16.8 | 6.37 | 15.7 | 6.37 | 14.5 |
| Average | 13.8 | 25.7 | 13.8 | 24.3 | 13.8 | 25.1 |
| Stdev | 31.8 | 23.8 | 31.8 | 23.3 | 31.8 | 28.7 |
| p(t-test) |  | 0.049 |  | 0.081 |  | 0.17 |
| Min | 0.00574 | 2.98 | 0.00574 | 1.41 | 0.00574 | 5.53 |
| Max | 361 | 122 | 361 | 122 | 361 | 122 |
| n (Samp) | 223 | 30 | 223 | 30 | 223 | 16 |
| n (Patient) | 223 | 30 | 223 | 30 | 223 | 16 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 9.29 | 15.5 | 9.29 | 15.5 | 9.29 | 15.9 |
| Average | 16.5 | 19.3 | 16.5 | 18.4 | 16.5 | 22.4 |
| Stdev | 29.1 | 13.5 | 29.1 | 13.8 | 29.1 | 17.1 |
| p(t-test) |  | 0.73 |  | 0.81 |  | 0.59 |
| Min | 0.00574 | 2.98 | 0.00574 | 1.41 | 0.00574 | 5.53 |
| Max | 361 | 50.4 | 361 | 50.4 | 361 | 50.4 |
| n (Samp) | 375 | 13 | 375 | 13 | 375 | 7 |
| n (Patient) | 375 | 13 | 375 | 13 | 375 | 7 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 7.74 | 18.0 | 7.74 | 17.8 | 7.74 | 14.5 |
| Average | 16.2 | 30.0 | 16.2 | 26.9 | 16.2 | 25.0 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | 35.5 | 26.9 | 35.5 | 25.4 | 35.5 | 30.2 |
| p(t-test) | | 0.073 | | 0.16 | | 0.37 |
| Min | 0.00662 | 5.53 | 0.00662 | 5.53 | 0.00662 | 5.53 |
| Max | 361 | 122 | 361 | 122 | 361 | 122 |
| n (Samp) | 173 | 23 | 173 | 23 | 173 | 14 |
| n (Patient) | 173 | 23 | 173 | 23 | 173 | 14 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.80 | 0.67 | 0.79 | 0.78 | 0.65 | 0.78 | 0.77 | 0.69 | 0.73 |
| SE | 0.050 | 0.083 | 0.058 | 0.051 | 0.084 | 0.059 | 0.070 | 0.11 | 0.079 |
| p | 2.3E-9 | 0.038 | 6.7E-7 | 3.5E-8 | 0.079 | 3.0E-6 | 8.9E-5 | 0.086 | 0.0037 |
| nCohort 1 | 223 | 375 | 173 | 223 | 375 | 173 | 223 | 375 | 173 |
| nCohort 2 | 30 | 13 | 23 | 30 | 13 | 23 | 16 | 7 | 14 |
| Cutoff 1 | 13.1 | 12.2 | 13.6 | 12.7 | 11.3 | 12.7 | 11.4 | 12.6 | 12.4 |
| Sens 1 | 70% | 77% | 74% | 70% | 77% | 74% | 75% | 71% | 71% |
| Spec 1 | 78% | 64% | 73% | 77% | 59% | 72% | 72% | 65% | 72% |
| Cutoff 2 | 12.1 | 11.3 | 12.1 | 11.1 | 9.43 | 11.4 | 9.43 | 9.43 | 8.10 |
| Sens 2 | 80% | 85% | 83% | 80% | 85% | 83% | 81% | 86% | 86% |
| Spec 2 | 76% | 59% | 71% | 72% | 51% | 69% | 65% | 51% | 52% |
| Cutoff 3 | 8.13 | 5.52 | 8.10 | 8.13 | 5.52 | 8.10 | 6.18 | 5.52 | 6.18 |
| Sens 3 | 90% | 92% | 91% | 90% | 92% | 91% | 94% | 100% | 93% |
| Spec 3 | 59% | 31% | 52% | 59% | 31% | 52% | 48% | 31% | 40% |
| Cutoff 4 | 10.8 | 15.5 | 11.8 | 10.8 | 15.5 | 11.8 | 10.8 | 15.5 | 11.8 |
| Sens 4 | 83% | 54% | 83% | 80% | 54% | 78% | 75% | 57% | 71% |
| Spec 4 | 70% | 70% | 71% | 70% | 70% | 71% | 70% | 70% | 71% |
| Cutoff 5 | 14.8 | 20.2 | 17.9 | 14.8 | 20.2 | 17.9 | 14.8 | 20.2 | 17.9 |
| Sens 5 | 57% | 31% | 52% | 53% | 31% | 48% | 44% | 43% | 36% |
| Spec 5 | 80% | 80% | 80% | 80% | 80% | 80% | 80% | 80% | 80% |
| Cutoff 6 | 25.0 | 33.1 | 28.7 | 25.0 | 33.1 | 28.7 | 25.0 | 33.1 | 28.7 |
| Sens 6 | 30% | 15% | 35% | 27% | 15% | 30% | 25% | 29% | 21% |
| Spec 6 | 90% | 90% | 90% | 90% | 90% | 90% | 90% | 90% | 90% |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart 2 | 2.0 | 1.0 | >3.2 | 2.0 | 1.0 | >3.2 | >2.0 | >1.0 | >3.1 |
| p Value | 0.57 | 1.0 | <0.32 | 0.57 | 1.0 | <0.32 | <0.57 | <1.0 | <0.33 |
| 95% CI of | 0.18 | 0.062 | >0.32 | 0.18 | 0.062 | >0.32 | >0.18 | >0.062 | >0.31 |
| OR Quart2 | 23 | 16 | na | 23 | 16 | na | na | na | na |
| OR Quart 3 | 10 | 6.3 | >8.2 | 10 | 7.5 | >9.6 | >4.2 | >3.1 | >6.7 |
| p Value | 0.029 | 0.090 | <0.054 | 0.029 | 0.062 | <0.037 | <0.20 | <0.33 | <0.083 |
| 95% CI of | 1.3 | 0.75 | >0.97 | 1.3 | 0.90 | >1.1 | >0.46 | >0.32 | >0.78 |
| OR Quart3 | 84 | 54 | na | 84 | 62 | na | na | na | na |
| OR Quart 4 | 24 | 5.2 | >18 | 24 | 4.1 | >16 | >12 | >3.1 | >5.5 |
| p Value | 0.0023 | 0.13 | <0.0068 | 0.0023 | 0.21 | <0.0093 | <0.021 | <0.34 | <0.13 |
| 95% CI of | 3.1 | 0.60 | >2.2 | 3.1 | 0.45 | >2.0 | >1.5 | >0.31 | >0.61 |
| OR Quart4 | 190 | 46 | na | 190 | 38 | na | na | na | na |

| CA 15-3 | | | | | | | | | |
|---|---|---|---|---|---|---|---|---|---|
| sCr or UO | 0hr prior to AKI stage | | | 24hr prior to AKI stage | | | 48hr prior to AKI stage | | |
|  | Cohort 1 | | Cohort 2 | Cohort 1 | Cohort 2 | | Cohort 1 | | Cohort 2 |
| Median | 434 | | 434 | 434 | 434 | | 434 | | 434 |
| Average | 327 | | 448 | 327 | 426 | | 327 | | 393 |
| Stdev | 253 | | 156 | 253 | 184 | | 253 | | 109 |
| p(t-test) | | | 0.050 | | 0.11 | | | | 0.36 |
| Min | 3.03 | | 63.1 | 3.03 | 39.8 | | 3.03 | | 63.1 |
| Max | 784 | | 784 | 784 | 784 | | 784 | | 434 |
| n (Samp) | 123 | | 18 | 123 | 18 | | 123 | | 13 |
| n (Patient) | 123 | | 18 | 123 | 18 | | 123 | | 13 |
| sCr only | 0hr prior to AKI stage | | | 24hr prior to AKI stage | | | 48hr prior to AKI stage | | |
|  | Cohort 1 | | Cohort 2 | Cohort 1 | | Cohort 2 | Cohort 1 | | Cohort 2 |
| Median | 434 | | 434 | 434 | | 434 | 434 | | 434 |
| Average | 379 | | 451 | 379 | | 415 | 379 | | 413 |
| Stdev | 241 | | 121 | 241 | | 174 | 241 | | 59.2 |

(continued)

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.33 | | 0.63 | | 0.69 |
| Min | 3.03 | 266 | 3.03 | 39.8 | 3.03 | 266 |
| Max | 784 | 784 | 784 | 784 | 784 | 434 |
| n (Samp) | 210 | 11 | 210 | 11 | 210 | 8 |
| n (Patient) | 210 | 11 | 210 | 11 | 210 | 8 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 434 | 434 | 434 | 434 | 434 | 434 |
| Average | 338 | 441 | 338 | 441 | 338 | 388 |
| Stdev | 243 | 172 | 243 | 172 | 243 | 131 |
| p(t-test) | | 0.19 | | 0.19 | | 0.57 |
| Min | 3.03 | 63.1 | 3.03 | 63.1 | 3.03 | 63.1 |
| Max | 784 | 784 | 784 | 784 | 784 | 434 |
| n (Samp) | 119 | 10 | 119 | 10 | 119 | 8 |
| n (Patient) | 119 | 10 | 119 | 10 | 119 | 8 |
| | | | | | | |
| | 0hr prior to AKI stage | | | 24hr prior to AKI stage | | | 48hr prior to AKI stage | | |
| | sCr or UO | sCr only | UO only | sCr or UO | sCr only | UO only | sCr or UO | sCr only | UO only |
| AUC | 0.65 | 0.58 | 0.64 | 0.63 | 0.54 | 0.64 | 0.60 | 0.54 | 0.58 |
| SE | 0.074 | 0.092 | 0.098 | 0.075 | 0.091 | 0.098 | 0.087 | 0.11 | 0.11 |
| p | 0.037 | 0.38 | 0.14 | 0.083 | 0.65 | 0.14 | 0.27 | 0.70 | 0.49 |
| nCohort 1 | 123 | 210 | 119 | 123 | 210 | 119 | 123 | 210 | 119 |
| nCohort 2 | 18 | 11 | 10 | 18 | 11 | 10 | 13 | 8 | 8 |
| Cutoff 1 | 413 | 413 | 363 | 413 | 413 | 363 | 413 | 413 | 363 |
| Sens 1 | 89% | 91% | 90% | 83% | 82% | 90% | 85% | 88% | 88% |
| Spec 1 | 42% | 32% | 37% | 42% | 32% | 37% | 42% | 32% | 37% |
| Cutoff 2 | 413 | 413 | 363 | 413 | 413 | 363 | 413 | 413 | 363 |
| Sens 2 | 89% | 91% | 90% | 83% | 82% | 90% | 85% | 88% | 88% |
| Spec 2 | 42% | 32% | 37% | 42% | 32% | 37% | 42% | 32% | 37% |
| Cutoff 3 | 248 | 413 | 363 | 61.3 | 253 | 363 | 248 | 253 | 61.3 |

| Sens 3 | 94% | 91% | 90% | 94% | 91% | 90% | 92% | 100% | 100% |
|---|---|---|---|---|---|---|---|---|---|

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 3 | 40% | 32% | 37% | 28% | 29% | 37% | 40% | 29% | 25% |
| Cutoff 4 | 434 | 434 | 434 | 434 | 434 | 434 | 434 | 434 | 434 |
| Sens 4 | 17% | 9% | 20% | 17% | 9% | 20% | 0% | 0% | 0% |
| Spec 4 | 86% | 83% | 87% | 86% | 83% | 87% | 86% | 83% | 87% |
| Cutoff 5 | 434 | 434 | 434 | 434 | 434 | 434 | 434 | 434 | 434 |
| Sens 5 | 17% | 9% | 20% | 17% | 9% | 20% | 0% | 0% | 0% |
| Spec 5 | 86% | 83% | 87% | 86% | 83% | 87% | 86% | 83% | 87% |
| Cutoff 6 | 784 | 784 | 784 | 784 | 784 | 784 | 784 | 784 | 784 |
| Sens 6 | 0% | 0% | 0% | 0% | 0% | 0% | 0% | 0% | 0% |
| Spec 6 | 100% | 100% | 100% | 100% | 100% | 100% | 100% | 100% | 100% |
| OR Quart 2 | >12 | >8.0 | 7.2 | 12 | 6.6 | 7.2 | >10 | >5.4 | 0 |
| p Value | <0.022 | <0.055 | 0.077 | 0.023 | 0.085 | 0.077 | <0.032 | <0.13 | na |
| 95% CI of | >1.4 | >0.95 | 0.81 | 1.4 | 0.77 | 0.81 | >1.2 | >0.61 | na |
| OR Quart2 | na | na | 63 | 99 | 57 | 63 | na | na | na |
| OR Quart 3 | >7.2 | >3.2 | 1.0 | 5.7 | 3.1 | 1.0 | >4.5 | >3.2 | 8.4 |
| p Value | <0.074 | <0.32 | 1.0 | 0.12 | 0.33 | 1.0 | <0.19 | <0.32 | 0.054 |
| 95% CI of | >0.82 | >0.32 | 0.060 | 0.63 | 0.31 | 0.060 | >0.48 | >0.32 | 0.97 |
| OR Quart3 | na | na | 17 | 51 | 31 | 17 | na | na | 73 |
| OR Quart 4 | >3.2 | >1.0 | 2.0 | 3.1 | 0.98 | 2.0 | >1.0 | >0 | 0 |
| p Value | <0.33 | <1.0 | 0.58 | 0.34 | 0.99 | 0.58 | <0.98 | <na | na |
| 95% CI of | >0.31 | >0.061 | 0.17 | 0.31 | 0.060 | 0.17 | >0.062 | >na | na |
| OR Quart4 | na | na | 23 | 31 | 16 | 23 | na | na | na |

Table 5: Comparison of marker levels in EDTA samples collected from Cohort 1 (patients that did not progress beyond RIFLE stage 0) and in EDTA samples collected from subjects at 0, 24 hours, and 48 hours prior to reaching stage R, I or F in Cohort 2.

| C-C motif chemokine 18 | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 161 | 203 | 161 | 207 | 161 | 190 |
| Average | 217 | 302 | 217 | 307 | 217 | 310 |
| Stdev | 171 | 316 | 171 | 299 | 171 | 318 |
| p(t-test) | | 0.024 | | 0.013 | | 0.038 |
| Min | 37.8 | 40.9 | 37.8 | 41.6 | 37.8 | 82.0 |
| Max | 1020 | 2110 | 1020 | 1650 | 1020 | 1550 |
| n (Samp) | 121 | 53 | 121 | 53 | 121 | 26 |
| n (Patient) | 87 | 53 | 87 | 53 | 87 | 26 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 180 | 221 | 180 | 279 | 180 | 220 |
| Average | 257 | 264 | 257 | 296 | 257 | 262 |
| Stdev | 251 | 185 | 251 | 166 | 251 | 160 |
| p(t-test) | | 0.92 | | 0.56 | | 0.96 |
| Min | 34.0 | 40.9 | 34.0 | 41.6 | 34.0 | 66.4 |
| Max | 2110 | 848 | 2110 | 679 | 2110 | 548 |
| n (Samp) | 288 | 16 | 288 | 15 | 288 | 10 |
| n (Patient) | 161 | 16 | 161 | 15 | 161 | 10 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 167 | 192 | 167 | 201 | 167 | 182 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 229 | 303 | 229 | 298 | 229 | 314 |
| Stdev | 174 | 336 | 174 | 297 | 174 | 336 |
| p(t-test) | | 0.063 | | 0.053 | | 0.070 |
| | Cohort 1 | Cohort 2 | Cohort 1 Cohort 2 | | Cohort 1 | Cohort 2 |
| Min | 37.8 | 46.6 | 37.8 59.1 | | 37.8 | 66.1 |
| Max | 848 | 2110 | 848 1650 | | 848 | 1550 |
| n (Samp) | 125 | 43 | 125 54 | | 125 | 23 |
| n (Patient) | 80 | 43 | 80 54 | | 80 | 23 |
| | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.61 | 0.57 | 0.58 | 0.60 | 0.63 | 0.57 | 0.60 | 0.57 | 0.56 |
| SE | 0.048 | 0.076 | 0.052 | 0.048 | 0.079 | 0.047 | 0.064 | 0.096 | 0.067 |
| p | 0.024 | 0.33 | 0.11 | 0.039 | 0.10 | 0.14 | 0.10 | 0.48 | 0.33 |
| nCohort 1 | 121 | 288 | 125 | 121 | 288 | 125 | 121 | 288 | 125 |
| nCohort 2 | 53 | 16 | 43 | 53 | 15 | 54 | 26 | 10 | 23 |
| Cutoff 1 | 162 | 167 | 161 | 147 | 169 | 154 | 138 | 186 | 118 |
| Sens 1 | 72% | 75% | 72% | 72% | 73% | 70% | 73% | 70% | 74% |
| Spec 1 | 50% | 45% | 48% | 45% | 46% | 45% | 44% | 53% | 33% |
| Cutoff 2 | 132 | 162 | 132 | 107 | 162 | 107 | 118 | 152 | 105 |
| Sens 2 | 81% | 81% | 81% | 81% | 80% | 81% | 81% | 80% | 83% |
| Spec 2 | 40% | 43% | 38% | 28% | 43% | 25% | 36% | 40% | 24% |
| Cutoff 3 | 99.3 | 114 | 99.3 | 73.3 | 126 | 73.3 | 102 | 102 | 82.0 |
| Sens 3 | 91% | 94% | 91% | 91% | 93% | 91% | 92% | 90% | 91% |

EP 3 489 688 A1

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 3 | 24% | 26% | 20% | 12% | 32% | 10% | 26% | 20% | 14% |
| Cutoff 4 | 228 | 283 | 274 | 228 | 283 | 274 | 228 | 283 | 274 |
| Sens 4 | 47% | 31% | 35% | 43% | 47% | 33% | 46% | 30% | 30% |
| Spec 4 | 70% | 70% | 70% | 70% | 70% | 70% | 70% | 70% | 70% |
| Cutoff 5 | 302 | 361 | 345 | 302 | 361 | 345 | 302 | 361 | 345 |
| Sens 5 | 32% | 19% | 30% | 32% | 33% | 26% | 23% | 20% | 26% |
| Spec 5 | 80% | 80% | 80% | 80% | 80% | 80% | 80% | 80% | 80% |
| Cutoff 6 | 464 | 516 | 484 | 464 | 516 | 484 | 464 | 516 | 484 |
| Sens 6 | 15% | 6% | 14% | 19% | 7% | 17% | 23% | 10% | 22% |
| Spec 6 | 90% | 90% | 90% | 90% | 90% | 90% | 90% | 90% | 90% |
| OR Quart 2 | 1.3 | 1.5 | 1.0 | 1.1 | 4.1 | 0.58 | 2.6 | 0.49 | 0.78 |
| p Value | 0.65 | 0.65 | 1.0 | 0.85 | 0.21 | 0.29 | 0.20 | 0.56 | 0.72 |
| 95% CI of OR Quart2 | 0.46 – 3.4 | 0.25 – 9.4 | 0.34 – 3.0 | 0.41 – 2.9 | 0.45 – 38 | 0.21 – 1.6 | 0.61 – 11 | 0.043 – 5.5 | 0.19 – 3.2 |
| OR Quart 3 | 2.0 | 3.8 | 2.1 | 1.6 | 4.1 | 1.9 | 3.5 | 2.1 | 1.8 |
| p Value | 0.15 | 0.11 | 0.14 | 0.34 | 0.21 | 0.14 | 0.077 | 0.41 | 0.36 |
| 95% CI of OR Quart3 | 0.77 – 5.3 | 0.75 – 19 | 0.78 – 5.8 | 0.61 – 4.1 | 0.45 – 38 | 0.80 – 4.7 | 0.87 – 14 | 0.37 – 12 | 0.52 – 6.0 |
| OR Quart 4 | 2.6 | 2.1 | 1.9 | 2.3 | 6.3 | 1.3 | 2.6 | 1.5 | 1.2 |
| p Value | 0.047 | 0.41 | 0.21 | 0.081 | 0.091 | 0.53 | 0.20 | 0.66 | 0.74 |
| 95% CI of OR Quart4 | 1.0 – 6.8 | 0.37 – 12 | 0.69 – 5.2 | 0.90 – 5.8 | 0.74 – 54 | 0.54 – 3.3 | 0.61 – 11 | 0.24 – 9.2 | 0.34 – 4.5 |

| C-C motif chemokine 24 | | | | | | | |
|---|---|---|---|---|---|---|---|
| sCr or UO | 0hr prior to AKI stage | | 24hr prior to AKI stage | | 48hr prior to AKI stage | | |
|  | Cohort 1 | Cohort 2 | Cohort 1 | Cohort 2 | Cohort 1 | Cohort 2 | |
| Median | 225 | 409 | 225 | 258 | 225 | 177 | |
| sCr or UO | 0hr prior to AKI stage | | 24hr prior to AKI stage | | 48hr prior to AKI stage | | |
|  | Cohort 1 | Cohort 2 | Cohort 1 | Cohort 2 | Cohort 1 | Cohort 2 | |
| Average | 418 | 443 | 418 | 391 | 418 | 265 | |
| Stdev | 512 | 312 | 512 | 316 | 512 | 209 | |
| p(t-test) |  | 0.77 |  | 0.75 |  | 0.22 | |
| Min | 0.0260 | 47.3 | 0.0260 | 54.7 | 0.0260 | 2.15 | |
| Max | 2920 | 1470 | 2920 | 1380 | 2920 | 579 | |
| n (Samp) | 93 | 45 | 93 | 42 | 93 | 18 | |
| n (Patient) | 64 | 45 | 64 | 42 | 64 | 18 | |
|  | | | | | | | |
| sCr only | 0hr prior to AKI stage | | 24hr prior to AKI stage | | 48hr prior to AKI stage | | |
|  | Cohort 1 | Cohort 2 | Cohort 1 | Cohort 2 | Cohort 1 | Cohort 2 | |
| Median | 225 | 533 | 225 | 459 | 225 | 554 | |
| Average | 357 | 535 | 357 | 456 | 357 | 615 | |
| Stdev | 403 | 315 | 403 | 282 | 403 | 353 | |
| p(t-test) |  | 0.10 |  | 0.31 |  | 0.060 | |
| Min | 0.0260 | 105 | 0.0260 | 94.6 | 0.0260 | 141 | |
| Max | 2920 | 1260 | 2920 | 931 | 2920 | 1100 | |
| n (Samp) | 224 | 14 | 224 | 18 | 224 | 9 | |
| n (Patient) | 131 | 14 | 131 | 18 | 131 | 9 | |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 270 | 409 | 270 | 259 | 270 | 126 |
| Average | 453 | 490 | 453 | 392 | 453 | 232 |
| Stdev | 493 | 461 | 493 | 344 | 493 | 212 |
| p(t-test) | | 0.69 | | 0.47 | | 0.091 |
| Min | 24.7 | 47.3 | 24.7 | 38.1 | 24.7 | 2.15 |
| Max | 2920 | 2430 | 2920 | 1380 | 2920 | 637 |
| n (Samp) | 102 | 37 | 102 | 41 | 102 | 15 |
| n (Patient) | 63 | 37 | 63 | 41 | 63 | 15 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.61 | 0.72 | 0.55 | 0.55 | 0.65 | 0.49 | 0.43 | 0.75 | 0.34 |
| SE | 0.052 | 0.079 | 0.056 | 0.054 | 0.073 | 0.054 | 0.076 | 0.096 | 0.081 |
| p | 0.042 | 0.0051 | 0.39 | 0.36 | 0.041 | 0.83 | 0.39 | 0.0091 | 0.042 |
| nCohort 1 | 93 | 224 | 102 | 93 | 224 | 102 | 93 | 224 | 102 |
| nCohort 2 | 45 | 14 | 37 | 42 | 18 | 41 | 18 | 9 | 15 |
| Cutoff 1 | 221 | 350 | 188 | 171 | 207 | 155 | 96.1 | 400 | 85.2 |
| Sens 1 | 71% | 71% | 70% | 71% | 72% | 71% | 72% | 78% | 73% |
| Spec 1 | 49% | 69% | 30% | 37% | 47% | 25% | 24% | 73% | 13% |
| Cutoff 2 | 171 | 221 | 154 | 108 | 196 | 92.4 | 85.2 | 212 | 70.8 |
| Sens 2 | 80% | 86% | 81% | 81% | 83% | 83% | 83% | 89% | 80% |
| Spec 2 | 37% | 50% | 25% | 25% | 46% | 15% | 18% | 47% | 11% |
| Cutoff 3 | 122 | 207 | 92.4 | 90.5 | 108 | 80.9 | 2.15 | 140 | 2.15 |
| Sens 3 | 91% | 93% | 92% | 90% | 94% | 90% | 94% | 100% | 93% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 3 | 26% | 47% | 15% | 19% | 27% | 12% | 1% | 31% | 0% |
| Cutoff 4 | 393 | 365 | 541 | 393 | 365 | 541 | 393 | 365 | 541 |
| Sens 4 | 51% | 64% | 38% | 36% | 56% | 24% | 33% | 78% | 13% |
| Spec 4 | 71% | 70% | 71% | 71% | 70% | 71% | 71% | 70% | 71% |
| Cutoff 5 | 664 | 548 | 705 | 664 | 548 | 705 | 664 | 548 | 705 |
| Sens 5 | 18% | 50% | 22% | 21% | 39% | 17% | 0% | 56% | 0% |
| Spec 5 | 81% | 80% | 80% | 81% | 80% | 80% | 81% | 80% | 80% |
| Cutoff 6 | 940 | 855 | 993 | 940 | 855 | 993 | 940 | 855 | 993 |
| Sens 6 | 7% | 14% | 8% | 7% | 6% | 10% | 0% | 33% | 0% |
| Spec 6 | 90% | 90% | 90% | 90% | 90% | 90% | 90% | 90% | 90% |
| OR Quart 2 | 2.1 | >3.1 | 0.96 | 0.96 | 5.3 | 1.3 | 1.8 | >2.1 | 6.0 |
| p Value | 0.19 | <0.33 | 0.95 | 0.94 | 0.13 | 0.60 | 0.45 | <0.56 | 0.11 |
| 95% CI of | 0.69 | >0.31 | 0.31 | 0.33 | 0.60 | 0.47 | 0.39 | >0.18 | 0.66 |
| OR Quart2 | 6.7 | na | 2.9 | 2.8 | 47 | 3.7 | 8.4 | na | 55 |
| OR Quart 3 | 3.7 | >3.2 | 1.5 | 1.7 | 3.1 | 1.3 | 1.8 | >1.0 | 0 |
| p Value | 0.021 | <0.33 | 0.46 | 0.34 | 0.33 | 0.60 | 0.45 | <0.99 | na |
| 95% CI of | 1.2 | >0.32 | 0.51 | 0.59 | 0.31 | 0.47 | 0.39 | >0.062 | na |
| OR Quart3 | 11 | na | 4.3 | 4.6 | 31 | 3.7 | 8.4 | na | na |
| OR Quart 4 | 2.8 | >9.1 | 1.3 | 1.3 | 10 | 1.2 | 1.9 | >6.6 | 13 |
| p Value | 0.075 | <0.041 | 0.63 | 0.65 | 0.030 | 0.73 | 0.42 | <0.086 | 0.019 |
| 95% CI of | 0.90 | >1.1 | 0.44 | 0.45 | 1.3 | 0.42 | 0.41 | >0.77 | 1.5 |
| OR Quart4 | 8.4 | na | 3.8 | 3.6 | 83 | 3.4 | 8.9 | na | 110 |

| C-C motif chemokine 8 | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 12.8 | 12.4 | 12.8 | 13.6 | 12.8 | 16.9 |
| Average | 17.4 | 13.3 | 17.4 | 18.7 | 17.4 | 20.9 |
| Stdev | 23.1 | 9.56 | 23.1 | 26.9 | 23.1 | 17.7 |
| p(t-test) | | 0.24 | | 0.78 | | 0.55 |
| Min | 0.162 | 0.162 | 0.162 | 0.170 | 0.162 | 1.32 |
| Max | 148 | 50.3 | 148 | 180 | 148 | 72.1 |
| n (Samp) | 93 | 45 | 93 | 42 | 93 | 18 |
| n (Patient) | 64 | 45 | 64 | 42 | 64 | 18 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 13.2 | 11.3 | 13.2 | 12.7 | 13.2 | 18.1 |
| Average | 16.7 | 12.7 | 16.7 | 22.4 | 16.7 | 23.8 |
| Stdev | 20.5 | 10.7 | 20.5 | 37.7 | 20.5 | 15.3 |
| p(t-test) | | 0.47 | | 0.30 | | 0.30 |
| Min | 0.162 | 0.162 | 0.162 | 0.170 | 0.162 | 11.5 |
| Max | 180 | 38.1 | 180 | 169 | 180 | 59.4 |
| n (Samp) | 224 | 14 | 224 | 18 | 224 | 9 |
| n (Patient) | 131 | 14 | 131 | 18 | 131 | 9 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 12.3 | 12.4 | 12.3 | 13.8 | 12.3 | 16.3 |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Average | 17.6 | | 12.7 | 17.6 | 24.9 | | 17.6 | 16.9 | |
| Stdev | 22.8 | | 9.01 | 22.8 | 40.3 | | 22.8 | 16.3 | |
| p(t-test) | | | 0.20 | | 0.17 | | | 0.90 | |
| Min | 0.162 | | 0.170 | 0.162 | 0.162 | | 0.162 | 0.162 | |
| Max | 148 | | 50.3 | 148 | 180 | | 148 | 72.1 | |
| n (Samp) | 102 | | 37 | 102 | 41 | | 102 | 15 | |
| n (Patient) | 63 | | 37 | 63 | 41 | | 63 | 15 | |
| | | | | | | | | | |
| | 0hr prior to AKI stage | | | 24hr prior to AKI stage | | | 48hr prior to AKI stage | | |
| | sCr or UO | sCr only | UO only | sCr or UO | sCr only | UO only | sCr or UO | sCr only | UO only |
| AUC | 0.46 | 0.43 | 0.47 | 0.54 | 0.50 | 0.55 | 0.61 | 0.71 | 0.55 |
| SE | 0.053 | 0.082 | 0.056 | 0.054 | 0.071 | 0.054 | 0.076 | 0.098 | 0.082 |
| p | 0.51 | 0.41 | 0.57 | 0.52 | 0.98 | 0.37 | 0.14 | 0.029 | 0.55 |
| nCohort 1 | 93 | 224 | 102 | 93 | 224 | 102 | 93 | 224 | 102 |
| nCohort 2 | 45 | 14 | 37 | 42 | 18 | 41 | 18 | 9 | 15 |
| Cutoff 1 | 7.62 | 6.24 | 8.03 | 10.1 | 10.6 | 9.32 | 12.9 | 14.4 | 11.3 |
| Sens 1 | 71% | 71% | 70% | 71% | 72% | 71% | 72% | 78% | 73% |
| Spec 1 | 25% | 19% | 28% | 37% | 36% | 30% | 53% | 57% | 44% |
| Cutoff 2 | 6.24 | 1.20 | 6.24 | 7.60 | 6.24 | 6.24 | 11.3 | 11.5 | 9.54 |
| Sens 2 | 82% | 86% | 81% | 81% | 83% | 83% | 83% | 89% | 80% |
| Spec 2 | 19% | 7% | 20% | 25% | 19% | 20% | 39% | 38% | 33% |
| Cutoff 3 | 1.16 | 1.16 | 1.16 | 4.81 | 1.67 | 4.81 | 9.47 | 11.3 | 1.20 |
| Sens 3 | 93% | 93% | 95% | 90% | 94% | 90% | 94% | 100% | 93% |
| Spec 3 | 6% | 5% | 8% | 18% | 9% | 19% | 29% | 38% | 10% |

EP 3 489 688 A1

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Cutoff 4 | 17.2 | 16.9 | 17.2 | 17.2 | 16.9 | 17.2 | 17.2 | 16.9 | 17.2 |
| Sens 4 | 18% | 29% | 19% | 36% | 33% | 34% | 39% | 67% | 33% |
| Spec 4 | 71% | 70% | 71% | 71% | 70% | 71% | 71% | 70% | 71% |
| Cutoff 5 | 20.1 | 19.7 | 22.1 | 20.1 | 19.7 | 22.1 | 20.1 | 19.7 | 22.1 |
| Sens 5 | 11% | 14% | 8% | 24% | 28% | 22% | 17% | 44% | 7% |
| Spec 5 | 81% | 80% | 80% | 81% | 80% | 80% | 81% | 80% | 80% |
| Cutoff 6 | 28.4 | 26.8 | 31.0 | 28.4 | 26.8 | 31.0 | 28.4 | 26.8 | 31.0 |
| Sens 6 | 4% | 14% | 3% | 10% | 17% | 12% | 17% | 22% | 7% |
| Spec 6 | 90% | 90% | 90% | 90% | 90% | 90% | 90% | 90% | 90% |
| OR Quart 2 | 2.4 | 1.0 | 2.9 | 0.96 | 0.98 | 0.60 | 4.3 | >2.1 | 1.6 |
| p Value | 0.11 | 0.98 | 0.065 | 0.94 | 0.98 | 0.37 | 0.20 | <0.56 | 0.64 |
| 95% CI of | 0.83 | 0.20 | 0.94 | 0.34 | 0.27 | 0.20 | 0.45 | >0.18 | 0.24 |
| OR Quart2 | 6.7 | 5.3 | 8.7 | 2.7 | 3.6 | 1.8 | 42 | na | 10 |
| OR Quart 3 | 1.5 | 1.0 | 1.7 | 0.96 | 0.58 | 1.4 | 14 | >3.2 | 6.1 |
| p Value | 0.42 | 1.0 | 0.38 | 0.94 | 0.47 | 0.50 | 0.015 | <0.32 | 0.031 |
| 95% CI of | 0.53 | 0.19 | 0.52 | 0.34 | 0.13 | 0.52 | 1.7 | >0.32 | 1.2 |
| OR Quart3 | 4.5 | 5.2 | 5.3 | 2.7 | 2.5 | 3.8 | 120 | na | 31 |
| OR Quart 4 | 1.8 | 1.8 | 1.7 | 1.3 | 0.98 | 1.1 | 3.1 | >4.2 | 0.47 |
| p Value | 0.26 | 0.45 | 0.35 | 0.66 | 0.98 | 0.85 | 0.34 | <0.20 | 0.54 |
| 95% CI of | 0.64 | 0.40 | 0.54 | 0.45 | 0.27 | 0.40 | 0.30 | >0.46 | 0.040 |
| OR Quart4 | 5.3 | 7.7 | 5.6 | 3.5 | 3.6 | 3.1 | 32 | na | 5.4 |

| Cathepsin D | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 234000 | 264000 | 234000 | 249000 | 234000 | 239000 |
| Average | 255000 | 257000 | 255000 | 278000 | 255000 | 272000 |
| Stdev | 136000 | 138000 | 136000 | 149000 | 136000 | 179000 |
| p(t-test) | | 0.93 | | 0.32 | | 0.59 |
| Min | 34100 | 55100 | 34100 | 55100 | 34100 | 54000 |
| Max | 1020000 | 653000 | 1020000 | 648000 | 1020000 | 853000 |
| n (Samp) | 120 | 53 | 120 | 53 | 120 | 26 |
| n (Patient) | 86 | 53 | 86 | 53 | 86 | 26 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 231000 | 210000 | 231000 | 325000 | 231000 | 175000 |
| Average | 266000 | 222000 | 266000 | 302000 | 266000 | 221000 |
| Stdev | 175000 | 110000 | 175000 | 145000 | 175000 | 99200 |
| p(t-test) | | 0.32 | | 0.44 | | 0.41 |
| Min | 34100 | 55100 | 34100 | 78800 | 34100 | 129000 |
| Max | 1870000 | 413000 | 1870000 | 507000 | 1870000 | 437000 |
| n (Samp) | 287 | 16 | 287 | 15 | 287 | 10 |
| n (Patient) | 160 | 16 | 160 | 15 | 160 | 10 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 239000 | 280000 | 239000 | 252000 | 239000 | 248000 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 265000 | 296000 | 265000 | 295000 | 265000 | 291000 |
| Stdev | 136000 | 216000 | 136000 | 164000 | 136000 | 186000 |
| p(t-test) | | 0.27 | | 0.21 | | 0.42 |
| Min | 69400 | 62900 | 69400 | 55100 | 69400 | 54000 |
| Max | 1020000 | 1350000 | 1020000 | 655000 | 1020000 | 853000 |
| n (Samp) | 124 | 43 | 124 | 54 | 124 | 23 |
| n (Patient) | 79 | 43 | 79 | 54 | 79 | 23 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.50 | 0.44 | 0.52 | 0.53 | 0.60 | 0.54 | 0.50 | 0.44 | 0.52 |
| SE | 0.048 | 0.076 | 0.052 | 0.048 | 0.079 | 0.047 | 0.063 | 0.096 | 0.066 |
| p | 0.94 | 0.41 | 0.64 | 0.47 | 0.23 | 0.42 | 0.98 | 0.53 | 0.77 |
| nCohort 1 | 120 | 287 | 124 | 120 | 287 | 124 | 120 | 287 | 124 |
| nCohort 2 | 53 | 16 | 43 | 53 | 15 | 54 | 26 | 10 | 23 |
| Cutoff 1 | 143000 | 143000 | 167000 | 167000 | 185000 | 169000 | 161000 | 157000 | 169000 |
| Sens 1 | 72% | 75% | 72% | 72% | 73% | 70% | 73% | 70% | 74% |
| Spec 1 | 18% | 23% | 28% | 29% | 38% | 28% | 25% | 28% | 29% |
| Cutoff 2 | 125000 | 126000 | 126000 | 136000 | 167000 | 136000 | 154000 | 154000 | 161000 |
| Sens 2 | 81% | 81% | 81% | 81% | 80% | 81% | 81% | 80% | 83% |
| Spec 2 | 11% | 15% | 7% | 14% | 33% | 10% | 21% | 27% | 23% |
| Cutoff 3 | 93800 | 87200 | 105000 | 116000 | 130000 | 114000 | 107000 | 130000 | 107000 |
| Sens 3 | 91% | 94% | 91% | 91% | 93% | 91% | 92% | 90% | 91% |
| Spec 3 | 6% | 4% | 6% | 8% | 17% | 6% | 7% | 17% | 6% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 4 | 300000 | 307000 | 307000 | 300000 | 307000 | 307000 | 300000 | 307000 | 307000 |
| Sens 4 | 36% | 25% | 40% | 42% | 53% | 41% | 27% | 20% | 30% |
| Spec 4 | 70% | 70% | 70% | 70% | 70% | 70% | 70% | 70% | 70% |
| Cutoff 5 | 330000 | 362000 | 342000 | 330000 | 362000 | 342000 | 330000 | 362000 | 342000 |
| Sens 5 | 32% | 12% | 26% | 36% | 33% | 39% | 19% | 10% | 26% |
| Spec 5 | 80% | 80% | 81% | 80% | 80% | 81% | 80% | 80% | 81% |
| Cutoff 6 | 407000 | 462000 | 426000 | 407000 | 462000 | 426000 | 407000 | 462000 | 426000 |
| Sens 6 | 15% | 0% | 14% | 25% | 20% | 22% | 15% | 0% | 17% |
| Spec 6 | 90% | 90% | 90% | 90% | 90% | 90% | 90% | 90% | 90% |
| OR Quart 2 | 0.35 | 0.74 | 0.40 | 0.57 | 0.99 | 0.42 | 1.5 | 3.1 | 0.97 |
| p Value | 0.036 | 0.70 | 0.10 | 0.24 | 0.99 | 0.083 | 0.52 | 0.33 | 0.96 |
| 95% CI of | 0.13 | 0.16 | 0.13 | 0.22 | 0.19 | 0.16 | 0.46 | 0.32 | 0.26 |
| OR Quart2 | 0.93 | 3.4 | 1.2 | 1.5 | 5.0 | 1.1 | 4.8 | 31 | 3.7 |
| OR Quart 3 | 0.46 | 1.0 | 0.86 | 0.49 | 1.0 | 0.57 | 0.81 | 4.2 | 1.4 |
| p Value | 0.11 | 1.0 | 0.75 | 0.15 | 1.0 | 0.24 | 0.74 | 0.20 | 0.56 |
| 95% CI of | 0.18 | 0.24 | 0.33 | 0.19 | 0.20 | 0.22 | 0.22 | 0.46 | 0.41 |
| OR Quart3 | 1.2 | 4.2 | 2.2 | 1.3 | 5.1 | 1.5 | 2.9 | 39 | 5.1 |
| OR Quart 4 | 1.1 | 1.3 | 1.2 | 1.4 | 2.1 | 1.7 | 1.2 | 2.1 | 1.2 |
| p Value | 0.90 | 0.72 | 0.69 | 0.43 | 0.32 | 0.23 | 0.72 | 0.56 | 0.78 |
| 95% CI of | 0.45 | 0.33 | 0.48 | 0.60 | 0.50 | 0.72 | 0.37 | 0.18 | 0.33 |
| OR Quart4 | 2.5 | 5.0 | 3.1 | 3.4 | 8.5 | 4.0 | 4.2 | 23 | 4.3 |

EP 3 489 688 A1

**C-X-C motif chemokine 13**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 45.8 | 52.8 | 45.8 | 69.6 | 45.8 | 80.6 |
| Average | 138 | 116 | 138 | 118 | 138 | 224 |
| Stdev | 297 | 292 | 297 | 216 | 297 | 275 |
| p(t-test) | | 0.68 | | 0.69 | | 0.26 |
| Min | 9.90 | 17.0 | 9.90 | 11.3 | 9.90 | 15.9 |
| Max | 1790 | 2000 | 1790 | 1340 | 1790 | 918 |
| n (Samp) | 93 | 45 | 93 | 42 | 93 | 18 |
| n (Patient) | 64 | 45 | 64 | 42 | 64 | 18 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 48.7 | 62.3 | 48.7 | 69.5 | 48.7 | 43.9 |
| Average | 125 | 207 | 125 | 187 | 125 | 128 |
| Stdev | 242 | 517 | 242 | 455 | 242 | 238 |
| p(t-test) | | 0.26 | | 0.33 | | 0.96 |
| Min | 9.90 | 25.4 | 9.90 | 18.6 | 9.90 | 17.3 |
| Max | 1790 | 2000 | 1790 | 2000 | 1790 | 758 |
| n (Samp) | 224 | 14 | 224 | 18 | 224 | 9 |
| n (Patient) | 131 | 14 | 131 | 18 | 131 | 9 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 47.3 | 56.6 | 47.3 | 69.3 | 47.3 | 81.6 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 123 | 76.0 | 123 | 170 | 123 | 229 |
| Stdev | 242 | 57.1 | 242 | 285 | 242 | 257 |
| p(t-test) | | 0.25 | | 0.32 | | 0.12 |
| Min | 9.90 | 17.0 | 9.90 | 11.3 | 9.90 | 15.9 |
| Max | 1790 | 287 | 1790 | 1340 | 1790 | 918 |
| n (Samp) | 102 | 37 | 102 | 41 | 102 | 15 |
| n (Patient) | 63 | 37 | 63 | 41 | 63 | 15 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.54 | 0.57 | 0.52 | 0.55 | 0.58 | 0.58 | 0.63 | 0.50 | 0.64 |
| SE | 0.053 | 0.082 | 0.056 | 0.054 | 0.073 | 0.054 | 0.076 | 0.099 | 0.081 |
| p | 0.47 | 0.42 | 0.66 | 0.38 | 0.25 | 0.15 | 0.096 | 0.97 | 0.082 |
| nCohort 1 | 93 | 224 | 102 | 93 | 224 | 102 | 93 | 224 | 102 |
| nCohort 2 | 45 | 14 | 37 | 42 | 18 | 41 | 18 | 9 | 15 |
| Cutoff 1 | 37.0 | 46.6 | 37.3 | 33.7 | 52.7 | 39.4 | 41.8 | 34.7 | 47.3 |
| Sens 1 | 71% | 71% | 70% | 71% | 72% | 71% | 72% | 78% | 73% |
| Spec 1 | 44% | 48% | 41% | 42% | 53% | 41% | 46% | 37% | 52% |
| Cutoff 2 | 34.0 | 28.0 | 34.5 | 28.3 | 32.8 | 33.7 | 29.5 | 34.0 | 29.5 |
| Sens 2 | 80% | 86% | 81% | 81% | 83% | 80% | 83% | 89% | 80% |
| Spec 2 | 42% | 27% | 35% | 35% | 35% | 34% | 41% | 36% | 33% |
| Cutoff 3 | 23.0 | 25.8 | 18.7 | 20.5 | 24.4 | 22.7 | 16.8 | 17.2 | 16.8 |
| Sens 3 | 91% | 93% | 92% | 90% | 94% | 90% | 94% | 100% | 93% |
| Spec 3 | 26% | 24% | 12% | 17% | 21% | 20% | 12% | 12% | 11% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 4 | 106 | 98.9 | 102 | 106 | 98.9 | 102 | 106 | 98.9 | 102 |
| Sens 4 | 24% | 29% | 24% | 33% | 33% | 39% | 39% | 22% | 47% |
| Spec 4 | 71% | 70% | 71% | 71% | 70% | 71% | 71% | 70% | 71% |
| Cutoff 5 | 170 | 136 | 140 | 170 | 136 | 140 | 170 | 136 | 140 |
| Sens 5 | 9% | 14% | 11% | 7% | 22% | 22% | 39% | 11% | 47% |
| Spec 5 | 81% | 80% | 80% | 81% | 80% | 80% | 81% | 80% | 80% |
| Cutoff 6 | 251 | 254 | 232 | 251 | 254 | 232 | 251 | 254 | 232 |
| Sens 6 | 4% | 7% | 3% | 7% | 6% | 15% | 39% | 11% | 47% |
| Spec 6 | 90% | 90% | 90% | 90% | 90% | 90% | 90% | 90% | 90% |
| OR Quart 2 | 2.3 | 1.5 | 2.7 | 1.5 | 1.5 | 1.3 | 0.96 | 2.1 | 0.64 |
| p Value | 0.13 | 0.66 | 0.11 | 0.44 | 0.66 | 0.61 | 0.96 | 0.56 | 0.64 |
| 95% CI of | 0.78 | 0.24 | 0.81 | 0.51 | 0.24 | 0.43 | 0.18 | 0.18 | 0.099 |
| OR Quart2 | 6.7 | 9.3 | 8.7 | 4.7 | 9.3 | 4.1 | 5.2 | 23 | 4.2 |
| OR Quart 3 | 3.4 | 3.2 | 3.9 | 2.9 | 4.5 | 2.0 | 1.7 | 5.5 | 1.0 |
| p Value | 0.024 | 0.16 | 0.023 | 0.050 | 0.066 | 0.21 | 0.48 | 0.13 | 1.0 |
| 95% CI of | 1.2 | 0.62 | 1.2 | 1.0 | 0.91 | 0.68 | 0.37 | 0.62 | 0.18 |
| OR Quart3 | 10.0 | 17 | 12 | 8.6 | 22 | 5.9 | 8.1 | 48 | 5.4 |
| OR Quart 4 | 1.3 | 1.5 | 1.4 | 1.5 | 2.6 | 2.3 | 2.7 | 1.0 | 2.6 |
| p Value | 0.61 | 0.66 | 0.56 | 0.44 | 0.27 | 0.14 | 0.19 | 0.99 | 0.19 |
| 95% CI of | 0.43 | 0.24 | 0.41 | 0.51 | 0.48 | 0.77 | 0.61 | 0.062 | 0.61 |
| OR Quart4 | 4.1 | 9.3 | 5.1 | 4.7 | 14 | 6.6 | 12 | 17 | 11 |

(continued)

| Insulin-like growth factor-binding protein 3 | | | | | |
|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 3010 | 3320 | 3010 | 3210 | 3010 | 3990 |
| Average | 3360 | 3500 | 3360 | 3620 | 3360 | 4050 |
| Stdev | 1520 | 1470 | 1520 | 1710 | 1520 | 1940 |
| p(t-test) | | 0.58 | | 0.33 | | 0.050 |
| Min | 823 | 651 | 823 | 730 | 823 | 804 |
| Max | 8260 | 7620 | 8260 | 8600 | 8260 | 7140 |
| n (Samp) | 121 | 53 | 121 | 53 | 121 | 26 |
| n (Patient) | 87 | 53 | 87 | 53 | 87 | 26 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 3050 | 3580 | 3050 | 4790 | 3050 | 5000 |
| Average | 3390 | 4110 | 3390 | 4440 | 3390 | 4510 |
| Stdev | 1500 | 1990 | 1500 | 1990 | 1500 | 1960 |
| p(t-test) | | 0.065 | | 0.0096 | | 0.022 |
| Min | 651 | 1820 | 651 | 1210 | 651 | 1660 |
| Max | 8260 | 7620 | 8260 | 8600 | 8260 | 7140 |
| n (Samp) | 288 | 16 | 288 | 15 | 288 | 10 |
| n (Patient) | 161 | 16 | 161 | 15 | 161 | 10 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 2960 | 3320 | 2960 | 3220 | 2960 | 3300 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 3290 | 3420 | 3290 | 3520 | 3290 | 3630 |
| Stdev | 1510 | 1370 | 1510 | 1580 | 1510 | 1780 |
| p(t-test) | | 0.61 | | 0.36 | | 0.34 |
| Min | 823 | 651 | 823 | 730 | 823 | 804 |
| Max | 8260 | 7300 | 8260 | 7520 | 8260 | 7100 |
| n (Samp) | 125 | 43 | 125 | 54 | 125 | 23 |
| n (Patient) | 80 | 43 | 80 | 54 | 80 | 23 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.54 | 0.59 | 0.55 | 0.54 | 0.66 | 0.55 | 0.61 | 0.67 | 0.56 |
| SE | 0.048 | 0.077 | 0.052 | 0.048 | 0.078 | 0.047 | 0.064 | 0.095 | 0.067 |
| p | 0.42 | 0.23 | 0.32 | 0.41 | 0.039 | 0.33 | 0.090 | 0.068 | 0.41 |
| nCohort 1 | 121 | 288 | 125 | 121 | 288 | 125 | 121 | 288 | 125 |
| nCohort 2 | 53 | 16 | 43 | 53 | 15 | 54 | 26 | 10 | 23 |
| Cutoff 1 | 2620 | 2560 | 2670 | 2370 | 3020 | 2430 | 2700 | 3050 | 2430 |
| Sens 1 | 72% | 75% | 72% | 72% | 73% | 70% | 73% | 70% | 74% |
| Spec 1 | 40% | 36% | 44% | 30% | 50% | 34% | 44% | 50% | 34% |
| Cutoff 2 | 2160 | 2070 | 2160 | 2290 | 2800 | 2290 | 2040 | 2430 | 2000 |
| Sens 2 | 81% | 81% | 81% | 81% | 80% | 81% | 81% | 80% | 83% |
| Spec 2 | 26% | 19% | 28% | 27% | 43% | 30% | 23% | 31% | 22% |
| Cutoff 3 | 1960 | 1940 | 1740 | 1780 | 2130 | 1740 | 1490 | 2430 | 1490 |
| Sens 3 | 91% | 94% | 91% | 91% | 93% | 91% | 92% | 90% | 91% |
| Spec 3 | 20% | 15% | 11% | 12% | 20% | 11% | 7% | 31% | 7% |

EP 3 489 688 A1

187

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 4 | 4130 | 4130 | 4040 | 4130 | 4130 | 4040 | 4130 | 4130 | 4040 |
| Sens 4 | 28% | 44% | 33% | 34% | 53% | 37% | 50% | 60% | 39% |
| Spec 4 | 70% | 70% | 70% | 70% | 70% | 70% | 70% | 70% | 70% |
| Cutoff 5 | 4680 | 4650 | 4560 | 4680 | 4650 | 4560 | 4680 | 4650 | 4560 |
| Sens 5 | 17% | 38% | 16% | 26% | 53% | 26% | 38% | 60% | 30% |
| Spec 5 | 80% | 80% | 80% | 80% | 80% | 80% | 80% | 80% | 80% |
| Cutoff 6 | 5410 | 5490 | 5410 | 5410 | 5490 | 5410 | 5410 | 5490 | 5410 |
| Sens 6 | 8% | 31% | 5% | 19% | 40% | 15% | 31% | 40% | 22% |
| Spec 6 | 90% | 90% | 90% | 90% | 90% | 90% | 90% | 90% | 90% |
| OR Quart 2 | 0.86 | 0.74 | 1.6 | 1.5 | 0.65 | 2.1 | 0.44 | 3.0 | 0.63 |
| p Value | 0.76 | 0.70 | 0.42 | 0.37 | 0.64 | 0.12 | 0.28 | 0.34 | 0.50 |
| 95% CI of | 0.33 | 0.16 | 0.53 | 0.60 | 0.11 | 0.83 | 0.10 | 0.31 | 0.16 |
| OR Quart2 | 2.2 | 3.4 | 4.6 | 4.0 | 4.0 | 5.6 | 1.9 | 30 | 2.4 |
| OR Quart 3 | 1.9 | 0.74 | 2.8 | 1.4 | 0.65 | 1.6 | 0.97 | 0 | 0.81 |
| p Value | 0.18 | 0.70 | 0.051 | 0.47 | 0.64 | 0.36 | 0.96 | na | 0.74 |
| 95% CI of | 0.76 | 0.16 | 0.99 | 0.55 | 0.11 | 0.60 | 0.28 | na | 0.22 |
| OR Quart3 | 4.6 | 3.4 | 7.8 | 3.7 | 4.0 | 4.2 | 3.3 | na | 2.9 |
| OR Quart 4 | 0.97 | 1.5 | 1.8 | 1.9 | 2.8 | 2.1 | 2.1 | 6.3 | 1.4 |
| p Value | 0.95 | 0.52 | 0.29 | 0.18 | 0.14 | 0.12 | 0.19 | 0.091 | 0.55 |
| 95% CI of | 0.38 | 0.42 | 0.61 | 0.74 | 0.72 | 0.83 | 0.69 | 0.75 | 0.44 |
| OR Quart4 | 2.5 | 5.7 | 5.1 | 4.8 | 11 | 5.6 | 6.5 | 54 | 4.6 |

(continued)

| Immunoglogulin G1 | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 8750000 | 8400000 | 8750000 | 9530000 | 8750000 | 7580000 |
| Average | 1.06E7 | 9010000 | 1.06E7 | 1.24E7 | 1.06E7 | 1.30E7 |
| Stdev | 6040000 | 4090000 | 6040000 | 8340000 | 6040000 | 9920000 |
| p(t-test) | | 0.14 | | 0.19 | | 0.28 |
| Min | 3800000 | 2060000 | 3800000 | 3530000 | 3800000 | 4390000 |
| Max | 3.48E7 | 2.33E7 | 3.48E7 | 4.18E7 | 3.48E7 | 3.57E7 |
| n (Samp) | 94 | 35 | 94 | 37 | 94 | 10 |
| n (Patient) | 67 | 35 | 67 | 37 | 67 | 10 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 8820000 | 7580000 | 8820000 | 1.02E7 | nd | nd |
| Average | 1.02E7 | 9650000 | 1.02E7 | 1.51E7 | nd | nd |
| Stdev | 5310000 | 6060000 | 5310000 | 1.34E7 | nd | nd |
| p(t-test) | | 0.73 | | 0.021 | nd | nd |
| Min | 2060000 | 3270000 | 2060000 | 4390000 | nd | nd |
| Max | 3.48E7 | 2.33E7 | 3.48E7 | 4.18E7 | nd | nd |
| n (Samp) | 205 | 10 | 205 | 8 | nd | nd |
| n (Patient) | 126 | 10 | 126 | 8 | nd | nd |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 7990000 | 8420000 | 7990000 | 9200000 | 7990000 | 7690000 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 9560000 | 8580000 | 9560000 | 1.21E7 | 9560000 | 1.34E7 |
| Stdev | 5620000 | 3160000 | 5620000 | 7850000 | 5620000 | 9630000 |
| p(t-test) | | 0.38 | | 0.039 | | 0.043 |
| Min | 3270000 | 2060000 | 3270000 | 3530000 | 3270000 | 4390000 |
| Max | 3.48E7 | 1.63E7 | 3.48E7 | 4.18E7 | 3.48E7 | 3.57E7 |
| n (Samp) | 101 | 28 | 101 | 36 | 101 | 12 |
| n (Patient) | 65 | 28 | 65 | 36 | 65 | 12 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.45 | 0.42 | 0.51 | 0.55 | 0.56 | 0.61 | 0.54 | nd | 0.62 |
| SE | 0.058 | 0.097 | 0.062 | 0.057 | 0.11 | 0.056 | 0.098 | nd | 0.091 |
| p | 0.38 | 0.42 | 0.82 | 0.41 | 0.55 | 0.041 | 0.69 | nd | 0.18 |
| nCohort 1 | 94 | 205 | 101 | 94 | 205 | 101 | 94 | nd | 101 |
| nCohort 2 | 35 | 10 | 28 | 37 | 8 | 36 | 10 | nd | 12 |
| Cutoff 1 | 6710000 | 6260000 | 7160000 | 7600000 | 7160000 | 7600000 | 7140000 | nd | 7140000 |
| Sens 1 | 71% | 70% | 71% | 70% | 75% | 72% | 90% | nd | 92% |
| Spec 1 | 27% | 17% | 44% | 40% | 33% | 50% | 31% | nd | 41% |
| Cutoff 2 | 6260000 | 5810000 | 6300000 | 6740000 | 5340000 | 6940000 | 7140000 | nd | 7140000 |
| Sens 2 | 80% | 80% | 82% | 81% | 88% | 81% | 90% | nd | 92% |
| Spec 2 | 20% | 15% | 34% | 29% | 12% | 38% | 31% | nd | 41% |
| Cutoff 3 | 5320000 | 5320000 | 5320000 | 5340000 | 4360000 | 5370000 | 7140000 | nd | 7140000 |
| Sens 3 | 94% | 90% | 93% | 92% | 100% | 92% | 90% | nd | 92% |
| Spec 3 | 10% | 8% | 13% | 14% | 4% | 21% | 31% | nd | 41% |

EP 3 489 688 A1

190

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 4 | 1.13E7 | 1.11E 7 | 1.05E7 | 1.13E7 | 1.11E7 | 1.05E7 | 1.13E7 | nd | 1.05E7 |
| Sens 4 | 23% | 30% | 25% | 32% | 38% | 36% | 40% | nd | 42% |
| Spec 4 | 70% | 70% | 70% | 70% | 70% | 70% | 70% | nd | 70% |
| Cutoff 5 | 1.43E7 | 1.34E7 | 1.17E7 | 1.43E7 | 1.34E7 | 1.17E7 | 1.43E7 | nd | 1.17E7 |
| Sens 5 | 9% | 20% | 14% | 30% | 25% | 36% | 30% | nd | 42% |
| Spec 5 | 81% | 80% | 80% | 81% | 80% | 80% | 81% | nd | 80% |
| Cutoff 6 | 1.98E7 | 1.71E7 | 1.59E7 | 1.98E7 | 1.71E7 | 1.59E7 | 1.98E7 | nd | 1.59E7 |
| Sens 6 | 3% | 10% | 4% | 16% | 25% | 19% | 20% | nd | 25% |
| Spec 6 | 90% | 90% | 90% | 90% | 90% | 90% | 90% | nd | 90% |
| OR Quart 2 | 1.2 | 0 | 1.5 | 2.2 | 0.49 | 2.7 | 6.0 | nd | 5.9 |
| p Value | 0.72 | na | 0.52 | 0.19 | 0.57 | 0.13 | 0.12 | nd | 0.12 |
| 95% CI of | 0.39 | na | 0.42 | 0.69 | 0.043 | 0.74 | 0.64 | nd | 0.64 |
| OR Quart2 | 3.9 | na | 5.4 | 6.8 | 5.6 | 9.8 | 55 | nd | 54 |
| OR Quart 3 | 1.9 | 1.0 | 2.5 | 1.6 | 1.5 | 3.1 | 0 | nd | 1.0 |
| p Value | 0.24 | 1.0 | 0.15 | 0.42 | 0.65 | 0.080 | na | nd | 1.0 |
| 95% CI of | 0.64 | 0.19 | 0.73 | 0.50 | 0.25 | 0.87 | na | nd | 0.059 |
| OR Quart3 | 5.9 | 5.2 | 8.2 | 5.2 | 9.5 | 11 | na | nd | 17 |
| OR Quart 4 | 1.5 | 1.4 | 1.2 | 2.2 | 0.98 | 4.4 | 4.5 | nd | 5.6 |
| p Value | 0.52 | 0.68 | 0.78 | 0.19 | 0.98 | 0.019 | 0.19 | nd | 0.13 |
| 95% CI of | 0.47 | 0.30 | 0.33 | 0.69 | 0.13 | 1.3 | 0.47 | nd | 0.61 |
| OR Quart4 | 4.5 | 6.5 | 4.4 | 6.8 | 7.2 | 15 | 44 | nd | 52 |

EP 3 489 688 A1

(continued)

**Immunoglogulin G2**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.41E7 | 1.03E7 | 1.41E7 | 1.08E7 | 1.41E7 | 1.08E7 |
| Average | 1.56E7 | 1.09E7 | 1.56E7 | 1.31E7 | 1.56E7 | 1.03E7 |
| Stdev | 1.10E7 | 8880000 | 1.10E7 | 6520000 | 1.10E7 | 6120000 |
| p(t-test) | | 0.027 | | 0.19 | | 0.14 |
| Min | 219000 | 190000 | 219000 | 1750000 | 219000 | 1750000 |
| Max | 5.80E7 | 4.70E7 | 5.80E7 | 3.11E7 | 5.80E7 | 1.77E7 |
| n (Samp) | 94 | 35 | 94 | 37 | 94 | 10 |
| n (Patient) | 67 | 35 | 67 | 37 | 67 | 10 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.03E7 | 1.21E7 | 1.03E7 | 1.34E7 | nd | nd |
| Average | 1.30E7 | 1.47E7 | 1.30E7 | 1.41E7 | nd | nd |
| Stdev | 9120000 | 1.27E7 | 9120000 | 9110000 | nd | nd |
| p(t-test) | | 0.57 | | 0.74 | nd | nd |
| Min | 190000 | 1750000 | 190000 | 1750000 | nd | nd |
| Max | 5.80E7 | 4.70E7 | 5.80E7 | 2.70E7 | nd | nd |
| n (Samp) | 205 | 10 | 205 | 8 | nd | nd |
| n (Patient) | 126 | 10 | 126 | 8 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.03E7 | 1.03E7 | 1.03E7 | 1.08E7 | 1.03E7 | 1.08E7 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 1.41E7 | 1.02E7 | 1.41E7 | 1.38E7 | 1.41E7 | 1.34E7 |
| Stdev | 1.02E7 | 7510000 | 1.02E7 | 7620000 | 1.02E7 | 1.20E7 |
| p(t-test) | | 0.060 | | 0.87 | | 0.82 |
| Min | 219000 | 190000 | 219000 | 1750000 | 219000 | 1750000 |
| Max | 5.80E7 | 2.91E7 | 5.80E7 | 3.69E7 | 5.80E7 | 4.70E7 |
| n (Samp) | 101 | 28 | 101 | 36 | 101 | 12 |
| n (Patient) | 65 | 28 | 65 | 36 | 65 | 12 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.38 | 0.52 | 0.41 | 0.47 | 0.56 | 0.53 | 0.39 | nd | 0.48 |
| SE | 0.057 | 0.095 | 0.063 | 0.057 | 0.11 | 0.057 | 0.099 | nd | 0.089 |
| p | 0.036 | 0.79 | 0.14 | 0.59 | 0.58 | 0.58 | 0.27 | nd | 0.79 |
| nCohort 1 | 94 | 205 | 101 | 94 | 205 | 101 | 94 | nd | 101 |
| nCohort 2 | 35 | 10 | 28 | 37 | 8 | 36 | 10 | nd | 12 |
| Cutoff 1 | 6810000 | 9110000 | 2450000 | 1.02E7 | 1.02E7 | 9110000 | 6700000 | nd | 6700000 |
| Sens 1 | 71% | 80% | 71% | 70% | 75% | 75% | 70% | nd | 75% |
| Spec 1 | 19% | 31% | 15% | 38% | 41% | 31% | 16% | nd | 20% |
| Cutoff 2 | 1750000 | 9110000 | 219000 | 7340000 | 4550000 | 7850000 | 4550000 | nd | 4550000 |
| Sens 2 | 80% | 80% | 96% | 84% | 88% | 83% | 80% | nd | 83% |
| Spec 2 | 9% | 31% | 1% | 21% | 18% | 26% | 14% | nd | 17% |
| Cutoff 3 | 563000 | 1930000 | 219000 | 4550000 | 563000 | 5450000 | 1750000 | nd | 1750000 |
| Sens 3 | 97% | 90% | 96% | 92% | 100% | 92% | 90% | nd | 92% |
| Spec 3 | 2% | 11% | 1% | 14% | 1% | 18% | 9% | nd | 8% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 4 | 1.77E7 | 1.61E7 | 1.64E7 | 1.77E7 | 1.61E7 | 1.64E7 | 1.77E7 | nd | 1.64E7 |
| Sens 4 | 14% | 30% | 18% | 16% | 50% | 31% | 0% | nd | 33% |
| Spec 4 | 71% | 70% | 72% | 71% | 70% | 72% | 71% | nd | 72% |
| Cutoff 5 | 2.34E7 | 1.77E7 | 2.09E7 | 2.34E7 | 1.77E7 | 2.09E7 | 2.34E7 | nd | 2.09E7 |
| Sens 5 | 6% | 20% | 7% | 8% | 25% | 11% | 0% | nd | 8% |
| Spec 5 | 81% | 81% | 80% | 81% | 81% | 80% | 81% | nd | 80% |
| Cutoff 6 | 3.13E7 | 2.52E7 | 2.66E7 | 3.13E7 | 2.52E7 | 2.66E7 | 3.13E7 | nd | 2.66E7 |
| Sens 6 | 3% | 10% | 4% | 0% | 25% | 8% | 0% | nd | 8% |
| Spec 6 | 91% | 91% | 90% | 91% | 91% | 90% | 91% | nd | 90% |
| OR Quart 2 | 1.5 | 1.5 | 1.6 | 2.2 | 1.0 | 1.2 | >4.7 | nd | 2.9 |
| p Value | 0.51 | 0.66 | 0.49 | 0.16 | 1.0 | 0.77 | <0.18 | nd | 0.22 |
| 95% CI of | 0.46 | 0.24 | 0.44 | 0.72 | 0.14 | 0.38 | >0.49 | nd | 0.52 |
| OR Quart2 | 4.9 | 9.4 | 5.6 | 7.1 | 7.4 | 3.7 | na | nd | 17 |
| OR Quart 3 | 2.0 | 0.98 | 1.6 | 2.6 | 0.49 | 2.1 | >2.2 | nd | 0.50 |
| p Value | 0.23 | 0.98 | 0.49 | 0.10 | 0.57 | 0.18 | <0.54 | nd | 0.58 |
| 95% CI of | 0.64 | 0.13 | 0.44 | 0.83 | 0.043 | 0.71 | >0.18 | nd | 0.043 |
| OR Quart3 | 6.5 | 7.2 | 5.6 | 8.0 | 5.6 | 6.3 | na | nd | 5.8 |
| OR Quart 4 | 2.4 | 1.5 | 2.2 | 1.5 | 1.5 | 1.3 | >4.7 | nd | 2.2 |
| p Value | 0.14 | 0.66 | 0.21 | 0.51 | 0.66 | 0.61 | <0.18 | nd | 0.37 |
| 95% CI of | 0.75 | 0.24 | 0.64 | 0.46 | 0.24 | 0.43 | >0.49 | nd | 0.38 |
| OR Quart4 | 7.4 | 9.4 | 7.5 | 4.9 | 9.4 | 4.1 | na | nd | 13 |

| Interleukin-11 | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 60.0 | 90.7 | 60.0 | 74.1 | 60.0 | 44.3 |
| Average | 245 | 134 | 245 | 115 | 245 | 90.2 |
| Stdev | 951 | 286 | 951 | 141 | 951 | 135 |
| p(t-test) | | 0.45 | | 0.38 | | 0.49 |
| Min | 0.368 | 0.359 | 0.368 | 0.359 | 0.368 | 0.359 |
| Max | 6920 | 1940 | 6920 | 802 | 6920 | 569 |
| n (Samp) | 94 | 45 | 94 | 42 | 94 | 18 |
| n (Patient) | 65 | 45 | 65 | 42 | 65 | 18 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 59.4 | 91.0 | 59.4 | 89.3 | 59.4 | 67.2 |
| Average | 148 | 236 | 148 | 465 | 148 | 118 |
| Stdev | 621 | 502 | 621 | 1250 | 621 | 171 |
| p(t-test) | | 0.60 | | 0.060 | | 0.89 |
| Min | 0.359 | 0.480 | 0.359 | 0.442 | 0.359 | 20.4 |
| Max | 6920 | 1940 | 6920 | 5420 | 6920 | 569 |
| n (Samp) | 225 | 14 | 225 | 18 | 225 | 9 |
| n (Patient) | 132 | 14 | 132 | 18 | 132 | 9 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 47.4 | 71.6 | 47.4 | 83.4 | 47.4 | 38.4 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 218 | 83.1 | 218 | 107 | 218 | 60.7 |
| Stdev | 909 | 62.4 | 909 | 125 | 909 | 69.0 |
| p(t-test) | | 0.37 | | 0.44 | | 0.51 |
| Min | 0.368 | 0.359 | 0.368 | 0.359 | 0.368 | 0.359 |
| Max | 6920 | 234 | 6920 | 741 | 6920 | 233 |
| n (Samp) | 103 | 37 | 103 | 41 | 103 | 15 |
| n (Patient) | 64 | 37 | 64 | 41 | 64 | 15 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.53 | 0.57 | 0.53 | 0.53 | 0.64 | 0.57 | 0.47 | 0.56 | 0.44 |
| SE | 0.053 | 0.082 | 0.056 | 0.054 | 0.073 | 0.054 | 0.075 | 0.10 | 0.082 |
| p | 0.56 | 0.39 | 0.57 | 0.53 | 0.048 | 0.22 | 0.73 | 0.56 | 0.50 |
| nCohort 1 | 94 | 225 | 103 | 94 | 225 | 103 | 94 | 225 | 103 |
| nCohort 2 | 45 | 14 | 37 | 42 | 18 | 41 | 18 | 9 | 15 |
| Cutoff 1 | 46.9 | 38.4 | 42.3 | 41.7 | 55.3 | 47.4 | 33.9 | 43.7 | 20.2 |
| Sens 1 | 71% | 71% | 70% | 71% | 72% | 71% | 72% | 78% | 73% |
| Spec 1 | 46% | 39% | 47% | 41% | 49% | 50% | 40% | 44% | 33% |
| Cutoff 2 | 29.7 | 6.00 | 15.1 | 15.1 | 29.7 | 20.2 | 20.2 | 38.4 | 7.90 |
| Sens 2 | 82% | 86% | 81% | 81% | 83% | 83% | 83% | 89% | 87% |
| Spec 2 | 34% | 16% | 26% | 23% | 31% | 33% | 31% | 39% | 24% |
| Cutoff 3 | 0.608 | 0.442 | 0.442 | 6.00 | 10.7 | 6.00 | 7.90 | 20.2 | 0.442 |
| Sens 3 | 91% | 100% | 92% | 90% | 94% | 90% | 94% | 100% | 93% |
| Spec 3 | 9% | 8% | 6% | 15% | 23% | 16% | 22% | 28% | 6% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 4 | 139 | 113 | 118 | 139 | 113 | 118 | 139 | 113 | 118 |
| Sens 4 | 24% | 29% | 24% | 29% | 44% | 29% | 17% | 11% | 13% |
| Spec 4 | 70% | 71% | 71% | 70% | 71% | 71% | 70% | 71% | 71% |
| Cutoff 5 | 190 | 155 | 159 | 190 | 155 | 159 | 190 | 155 | 159 |
| Sens 5 | 9% | 29% | 14% | 19% | 44% | 17% | 17% | 11% | 13% |
| Spec 5 | 81% | 80% | 81% | 81% | 80% | 81% | 81% | 80% | 81% |
| Cutoff 6 | 270 | 209 | 221 | 270 | 209 | 221 | 270 | 209 | 221 |
| Sens 6 | 4% | 21% | 3% | 7% | 33% | 7% | 6% | 11% | 7% |
| Spec 6 | 90% | 91% | 90% | 90% | 91% | 90% | 90% | 91% | 90% |
| OR Quart 2 | 1.5 | 0.64 | 1.2 | 1.4 | 0.98 | 2.2 | 1.4 | >3.1 | 1.6 |
| p Value | 0.46 | 0.64 | 0.77 | 0.58 | 0.98 | 0.17 | 0.69 | <0.33 | 0.61 |
| 95% CI of | 0.51 | 0.10 | 0.38 | 0.46 | 0.19 | 0.71 | 0.28 | >0.31 | 0.25 |
| OR Quart2 | 4.3 | 4.0 | 3.7 | 4.0 | 5.1 | 6.8 | 6.9 | na | 10 |
| OR Quart 3 | 3.1 | 1.7 | 2.7 | 2.3 | 1.3 | 3.6 | 3.3 | >5.5 | 3.5 |
| p Value | 0.033 | 0.48 | 0.072 | 0.12 | 0.71 | 0.023 | 0.10 | <0.13 | 0.15 |
| 95% CI of | 1.1 | 0.39 | 0.92 | 0.80 | 0.29 | 1.2 | 0.78 | >0.62 | 0.65 |
| OR Quart3 | 8.6 | 7.4 | 7.8 | 6.5 | 6.2 | 11 | 14 | na | 19 |
| OR Quart 4 | 1.1 | 1.3 | 1.2 | 1.4 | 2.9 | 1.7 | 1.0 | >1.0 | 2.2 |
| p Value | 0.83 | 0.71 | 0.77 | 0.58 | 0.13 | 0.39 | 1.0 | <1.0 | 0.37 |
| 95% CI of | 0.38 | 0.29 | 0.38 | 0.46 | 0.72 | 0.52 | 0.18 | >0.061 | 0.38 |
| OR Quart4 | 3.4 | 6.2 | 3.7 | 4.0 | 11 | 5.3 | 5.4 | na | 13 |

(continued)

| Interleukin-2 receptor alpha chain | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 18.1 | 22.6 | 18.1 | 18.3 | 18.1 | 20.6 |
| Average | 38.7 | 84.1 | 38.7 | 115 | 38.7 | 90.3 |
| Stdev | 51.1 | 146 | 51.1 | 221 | 51.1 | 180 |
| p(t-test) | | 0.0079 | | 0.0018 | | 0.020 |
| Min | 0.0290 | 0.0290 | 0.0290 | 0.0290 | 0.0290 | 0.0515 |
| Max | 232 | 715 | 232 | 1040 | 232 | 714 |
| n (Samp) | 94 | 45 | 94 | 42 | 94 | 18 |
| n (Patient) | 65 | 45 | 65 | 42 | 65 | 18 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 18.3 | 47.9 | 18.3 | 42.6 | 18.3 | 22.6 |
| Average | 61.1 | 86.0 | 61.1 | 95.8 | 61.1 | 148 |
| Stdev | 121 | 135 | 121 | 174 | 121 | 282 |
| p(t-test) | | 0.46 | | 0.26 | | 0.049 |
| Min | 0.0290 | 0.0461 | 0.0290 | 0.0290 | 0.0290 | 0.0290 |
| Max | 1040 | 518 | 1040 | 715 | 1040 | 862 |
| n (Samp) | 225 | 14 | 225 | 18 | 225 | 9 |
| n (Patient) | 132 | 14 | 132 | 18 | 132 | 9 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 29.5 | 13.6 | 29.5 | 39.6 | 29.5 | 24.1 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 46.4 | 89.1 | 46.4 | 152 | 46.4 | 98.0 |
| Stdev | 53.6 | 159 | 53.6 | 261 | 53.6 | 192 |
| p(t-test) | | 0.018 | | 1.3E-4 | | 0.027 |
| Min | 0.0290 | 0.0290 | 0.0290 | 0.0290 | 0.0290 | 0.0515 |
| Max | 232 | 715 | 232 | 1040 | 232 | 714 |
| n (Samp) | 103 | 37 | 103 | 41 | 103 | 15 |
| n (Patient) | 64 | 37 | 64 | 41 | 64 | 15 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.56 | 0.60 | 0.51 | 0.56 | 0.58 | 0.57 | 0.56 | 0.53 | 0.54 |
| SE | 0.053 | 0.082 | 0.056 | 0.054 | 0.073 | 0.054 | 0.076 | 0.100 | 0.081 |
| p | 0.25 | 0.22 | 0.80 | 0.31 | 0.29 | 0.21 | 0.46 | 0.79 | 0.60 |
| nCohort 1 | 94 | 225 | 103 | 94 | 225 | 103 | 94 | 225 | 103 |
| nCohort 2 | 45 | 14 | 37 | 42 | 18 | 41 | 18 | 9 | 15 |
| Cutoff 1 | 2.97 | 8.15 | 2.33 | 0.0569 | 11.0 | 4.31 | 5.94 | 0.0569 | 14.2 |
| Sens 1 | 71% | 71% | 70% | 71% | 72% | 71% | 72% | 78% | 73% |
| Spec 1 | 32% | 40% | 27% | 24% | 43% | 30% | 37% | 21% | 40% |
| Cutoff 2 | 0.0515 | 2.19 | 0.0515 | 0.0515 | 0.0515 | 0.0515 | 0.0612 | 0.0515 | 5.94 |
| Sens 2 | 84% | 86% | 84% | 81% | 89% | 83% | 83% | 89% | 80% |
| Spec 2 | 17% | 30% | 17% | 17% | 16% | 17% | 29% | 16% | 31% |
| Cutoff 3 | 0.0359 | 0.0359 | 0.0290 | 0.0359 | 0.0290 | 0.0359 | 0.0515 | 0 | 0.0612 |
| Sens 3 | 91% | 100% | 95% | 90% | 94% | 95% | 94% | 100% | 93% |
| Spec 3 | 11% | 9% | 5% | 11% | 4% | 10% | 17% | 0% | 23% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 4 | 41.0 | 45.8 | 56.7 | 41.0 | 45.8 | 56.7 | 41.0 | 45.8 | 56.7 |
| Sens 4 | 44% | 50% | 35% | 45% | 50% | 41% | 28% | 33% | 33% |
| Spec 4 | 70% | 70% | 71% | 70% | 70% | 71% | 70% | 70% | 71% |
| Cutoff 5 | 68.3 | 84.9 | 89.4 | 68.3 | 84.9 | 89.4 | 68.3 | 84.9 | 89.4 |
| Sens 5 | 36% | 36% | 27% | 31% | 22% | 34% | 28% | 33% | 27% |
| Spec 5 | 81% | 80% | 81% | 81% | 80% | 81% | 81% | 80% | 81% |
| Cutoff 6 | 117 | 153 | 132 | 117 | 153 | 132 | 117 | 153 | 132 |
| Sens 6 | 22% | 14% | 19% | 24% | 11% | 29% | 17% | 33% | 13% |
| Spec 6 | 90% | 90% | 90% | 90% | 90% | 90% | 90% | 90% | 90% |
| OR Quart 2 | 1.3 | 1.5 | 0.87 | 0.66 | 0.72 | 0.74 | 1.8 | 0.32 | 3.4 |
| p Value | 0.66 | 0.66 | 0.79 | 0.43 | 0.68 | 0.59 | 0.45 | 0.32 | 0.16 |
| 95% CI of | 0.45 | 0.24 | 0.30 | 0.23 | 0.16 | 0.25 | 0.39 | 0.032 | 0.62 |
| OR Quart2 | 3.5 | 9.3 | 2.5 | 1.9 | 3.4 | 2.2 | 8.4 | 3.1 | 18 |
| OR Quart 3 | 0.60 | 1.5 | 0.62 | 0.39 | 0.98 | 0.87 | 1.8 | 0.65 | 1.6 |
| p Value | 0.37 | 0.66 | 0.41 | 0.10 | 0.98 | 0.79 | 0.45 | 0.65 | 0.64 |
| 95% CI of | 0.20 | 0.24 | 0.21 | 0.13 | 0.23 | 0.30 | 0.39 | 0.11 | 0.24 |
| OR Quart3 | 1.8 | 9.3 | 1.9 | 1.2 | 4.1 | 2.5 | 8.4 | 4.1 | 10 |
| OR Quart 4 | 2.0 | 3.2 | 1.1 | 1.4 | 1.8 | 1.7 | 1.8 | 0.98 | 2.1 |
| p Value | 0.16 | 0.17 | 0.79 | 0.46 | 0.36 | 0.32 | 0.45 | 0.98 | 0.42 |
| 95% CI of | 0.75 | 0.61 | 0.41 | 0.55 | 0.50 | 0.61 | 0.39 | 0.19 | 0.35 |
| OR Quart4 | 5.5 | 16 | 3.2 | 3.8 | 6.6 | 4.5 | 8.4 | 5.1 | 12 |

**Protransforming growth factor alpha**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.51 | 1.45 | 1.51 | 2.44 | 1.51 | 4.16 |
| Average | 6.28 | 6.62 | 6.28 | 13.7 | 6.28 | 7.24 |
| Stdev | 30.0 | 24.2 | 30.0 | 43.6 | 30.0 | 9.10 |
| p(t-test) | | 0.95 | | 0.25 | | 0.89 |
| Min | 0.00228 | 0.00228 | 0.00228 | 0.00305 | 0.00228 | 0.00305 |
| Max | 287 | 162 | 287 | 245 | 287 | 30.3 |
| n (Samp) | 94 | 45 | 94 | 42 | 94 | 18 |
| n (Patient) | 65 | 45 | 65 | 42 | 65 | 18 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.47 | 2.13 | 1.47 | 3.26 | 1.47 | 0.885 |
| Average | 7.40 | 5.15 | 7.40 | 31.0 | 7.40 | 4.52 |
| Stdev | 27.1 | 8.71 | 27.1 | 67.7 | 27.1 | 9.72 |
| p(t-test) | | 0.76 | | 0.0027 | | 0.75 |
| Min | 0.00228 | 0.00228 | 0.00228 | 0.00305 | 0.00228 | 0.0105 |
| Max | 287 | 30.8 | 287 | 245 | 287 | 30.3 |
| n (Samp) | 225 | 14 | 225 | 18 | 225 | 9 |
| n (Patient) | 132 | 14 | 132 | 18 | 132 | 9 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.22 | 1.26 | 1.22 | 2.41 | 1.22 | 4.80 |

(continued)

| | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 5.94 | 6.29 | 5.94 | 9.09 | 5.94 | 6.62 |
| Stdev | 28.7 | 26.3 | 28.7 | 25.6 | 28.7 | 7.52 |
| p(t-test) | | 0.95 | | 0.54 | | 0.93 |
| Min | 0.00228 | 0.00305 | 0.00228 | 0.00228 | 0.00228 | 0.00305 |
| Max | 287 | 162 | 287 | 153 | 287 | 28.3 |
| n (Samp) | 103 | 37 | 103 | 41 | 103 | 15 |
| n (Patient) | 64 | 37 | 64 | 41 | 64 | 15 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.51 | 0.50 | 0.51 | 0.58 | 0.63 | 0.59 | 0.71 | 0.49 | 0.73 |
| SE | 0.053 | 0.080 | 0.056 | 0.054 | 0.073 | 0.054 | 0.073 | 0.099 | 0.078 |
| p | 0.86 | 0.99 | 0.81 | 0.12 | 0.067 | 0.084 | 0.0047 | 0.90 | 0.0034 |
| nCohort 1 | 94 | 225 | 103 | 94 | 225 | 103 | 94 | 225 | 103 |
| nCohort 2 | 45 | 14 | 37 | 42 | 18 | 41 | 18 | 9 | 15 |
| Cutoff 1 | 0.597 | 0.490 | 0.597 | 0.666 | 0.875 | 0.875 | 1.35 | 0.674 | 3.84 |
| Sens 1 | 71% | 71% | 70% | 71% | 72% | 71% | 72% | 78% | 73% |
| Spec 1 | 37% | 29% | 41% | 38% | 38% | 46% | 48% | 34% | 81% |
| Cutoff 2 | 0.0907 | 0.00228 | 0.0907 | 0.490 | 0.637 | 0.141 | 0.875 | 0.666 | 1.35 |
| Sens 2 | 80% | 86% | 81% | 81% | 83% | 80% | 83% | 89% | 80% |
| Spec 2 | 21% | 4% | 22% | 32% | 34% | 27% | 41% | 34% | 52% |
| Cutoff 3 | 0.00305 | 0 | 0.00305 | 0.00305 | 0.0223 | 0.00305 | 0.340 | 0.00584 | 0.0907 |
| Sens 3 | 91% | 100% | 95% | 93% | 94% | 93% | 94% | 100% | 93% |
| Spec 3 | 15% | 0% | 17% | 15% | 20% | 17% | 29% | 17% | 22% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 4 | 3.05 | 3.27 | 2.83 | 3.05 | 3.27 | 2.83 | 3.05 | 3.27 | 2.83 |
| Sens 4 | 24% | 29% | 19% | 38% | 50% | 41% | 67% | 22% | 73% |
| Spec 4 | 70% | 70% | 71% | 70% | 70% | 71% | 70% | 70% | 71% |
| Cutoff 5 | 3.96 | 4.80 | 3.84 | 3.96 | 4.80 | 3.84 | 3.96 | 4.80 | 3.84 |
| Sens 5 | 24% | 29% | 19% | 33% | 39% | 32% | 56% | 11% | 73% |
| Spec 5 | 81% | 80% | 81% | 81% | 80% | 81% | 81% | 80% | 81% |
| Cutoff 6 | 6.50 | 9.07 | 7.79 | 6.50 | 9.07 | 7.79 | 6.50 | 9.07 | 7.79 |
| Sens 6 | 13% | 14% | 8% | 24% | 33% | 22% | 28% | 11% | 20% |
| Spec 6 | 90% | 90% | 90% | 90% | 90% | 90% | 90% | 90% | 90% |
| OR Quart 2 | 1.4 | 0.47 | 1.4 | 1.6 | 2.6 | 1.3 | 5.9 | 2.1 | 0.96 |
| p Value | 0.50 | 0.40 | 0.59 | 0.42 | 0.27 | 0.59 | 0.12 | 0.56 | 0.97 |
| 95% CI of | 0.52 | 0.083 | 0.46 | 0.53 | 0.48 | 0.46 | 0.64 | 0.18 | 0.13 |
| OR Quart2 | 3.9 | 2.7 | 4.0 | 4.5 | 14 | 3.9 | 54 | 23 | 7.3 |
| OR Quart 3 | 1.1 | 0.98 | 1.8 | 1.2 | 1.5 | 1.3 | 2.1 | 5.4 | 0.48 |
| p Value | 0.86 | 0.98 | 0.29 | 0.78 | 0.66 | 0.59 | 0.56 | 0.13 | 0.56 |
| 95% CI of | 0.39 | 0.23 | 0.61 | 0.39 | 0.24 | 0.46 | 0.18 | 0.61 | 0.041 |
| OR Quart3 | 3.1 | 4.1 | 5.0 | 3.5 | 9.3 | 3.9 | 24 | 47 | 5.6 |
| OR Quart 4 | 1.1 | 0.98 | 0.84 | 2.3 | 4.4 | 2.0 | 15 | 1.0 | 6.8 |
| p Value | 0.86 | 0.98 | 0.77 | 0.12 | 0.069 | 0.20 | 0.013 | 0.99 | 0.021 |
| 95% CI of | 0.39 | 0.23 | 0.27 | 0.80 | 0.89 | 0.70 | 1.8 | 0.062 | 1.3 |
| OR Quart4 | 3.1 | 4.1 | 2.6 | 6.5 | 22 | 5.6 | 130 | 17 | 34 |

(continued)

| CA 15-3 | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 0.248 | 0.246 | 0.248 | 0.380 | 0.248 | 0.210 |
| Average | 0.476 | 0.678 | 0.476 | 1.88 | 0.476 | 3.33 |
| Stdev | 0.801 | 1.63 | 0.801 | 6.00 | 0.801 | 12.1 |
| p(t-test) | | 0.28 | | 0.012 | | 0.010 |
| Min | 0.00165 | 0.00165 | 0.00165 | 0.0177 | 0.00165 | 0.0355 |
| Max | 6.54 | 11.0 | 6.54 | 36.7 | 6.54 | 60.0 |
| n (Samp) | 121 | 53 | 121 | 53 | 121 | 26 |
| n (Patient) | 87 | 53 | 87 | 53 | 87 | 26 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 0.276 | 0.254 | 0.276 | 0.528 | 0.276 | 0.360 |
| Average | 1.11 | 0.413 | 1.11 | 1.07 | 1.11 | 0.876 |
| Stdev | 4.63 | 0.570 | 4.63 | 2.34 | 4.63 | 1.70 |
| p(t-test) | | 0.55 | | 0.98 | | 0.87 |
| Min | 0.00165 | 0.0294 | 0.00165 | 0.0463 | 0.00165 | 0.0355 |
| Max | 60.0 | 2.27 | 60.0 | 9.48 | 60.0 | 5.66 |
| n (Samp) | 288 | 16 | 288 | 15 | 288 | 10 |
| n (Patient) | 161 | 16 | 161 | 15 | 161 | 10 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 0.292 | 0.246 | 0.292 | 0.395 | 0.292 | 0.399 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 0.541 | 0.734 | 0.541 | 1.88 | 0.541 | 3.76 |
| Stdev | 0.887 | 1.78 | 0.887 | 5.95 | 0.887 | 12.8 |
| p(t-test) | | 0.36 | | 0.015 | | 0.0055 |
| Min | 0.00165 | 0.00165 | 0.00165 | 0.0177 | 0.00165 | 0.0642 |
| Max | 6.54 | 11.0 | 6.54 | 36.7 | 6.54 | 60.0 |
| n (Samp) | 125 | 43 | 125 | 54 | 125 | 23 |
| n (Patient) | 80 | 43 | 80 | 54 | 80 | 23 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.51 | 0.43 | 0.49 | 0.62 | 0.62 | 0.60 | 0.52 | 0.49 | 0.54 |
| SE | 0.048 | 0.076 | 0.051 | 0.047 | 0.079 | 0.047 | 0.063 | 0.093 | 0.067 |
| p | 0.78 | 0.35 | 0.85 | 0.012 | 0.14 | 0.036 | 0.76 | 0.95 | 0.51 |
| nCohort 1 | 121 | 288 | 125 | 121 | 288 | 125 | 121 | 288 | 125 |
| nCohort 2 | 53 | 16 | 43 | 53 | 15 | 54 | 26 | 10 | 23 |
| Cutoff 1 | 0.171 | 0.0734 | 0.172 | 0.203 | 0.345 | 0.235 | 0.126 | 0.147 | 0.148 |
| Sens 1 | 72% | 75% | 72% | 72% | 73% | 70% | 73% | 70% | 74% |
| Spec 1 | 32% | 6% | 28% | 40% | 60% | 41% | 25% | 24% | 26% |
| Cutoff 2 | 0.126 | 0.0598 | 0.136 | 0.143 | 0.187 | 0.186 | 0.107 | 0.100 | 0.126 |
| Sens 2 | 81% | 81% | 81% | 83% | 80% | 81% | 81% | 80% | 83% |
| Spec 2 | 25% | 5% | 22% | 26% | 32% | 30% | 22% | 15% | 22% |
| Cutoff 3 | 0.0598 | 0.0294 | 0.0734 | 0.117 | 0.117 | 0.127 | 0.0762 | 0.0762 | 0.107 |
| Sens 3 | 91% | 94% | 91% | 91% | 93% | 91% | 92% | 90% | 91% |
| Spec 3 | 7% | 2% | 8% | 23% | 18% | 22% | 8% | 7% | 20% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 4 | 0.373 | 0.447 | 0.411 | 0.373 | 0.447 | 0.411 | 0.373 | 0.447 | 0.411 |
| Sens 4 | 34% | 19% | 33% | 51% | 60% | 48% | 46% | 50% | 48% |
| Spec 4 | 70% | 70% | 70% | 70% | 70% | 70% | 70% | 70% | 70% |
| Cutoff 5 | 0.536 | 0.685 | 0.720 | 0.536 | 0.685 | 0.720 | 0.536 | 0.685 | 0.720 |
| Sens 5 | 25% | 12% | 16% | 34% | 27% | 26% | 27% | 30% | 22% |
| Spec 5 | 80% | 80% | 80% | 80% | 80% | 80% | 80% | 80% | 80% |
| Cutoff 6 | 1.08 | 1.41 | 1.14 | 1.08 | 1.41 | 1.14 | 1.08 | 1.41 | 1.14 |
| Sens 6 | 9% | 6% | 9% | 17% | 7% | 19% | 15% | 10% | 17% |
| Spec 6 | 90% | 90% | 90% | 90% | 90% | 90% | 90% | 90% | 90% |
| OR Quart 2 | 1.3 | 1.4 | 0.77 | 0.75 | 0.32 | 1.2 | 0.97 | 0.24 | 0.81 |
| p Value | 0.53 | 0.70 | 0.61 | 0.57 | 0.33 | 0.67 | 0.95 | 0.21 | 0.74 |
| 95% CI of | 0.54 | 0.29 | 0.28 | 0.27 | 0.033 | 0.47 | 0.30 | 0.027 | 0.22 |
| OR Quart2 | 3.3 | 6.3 | 2.1 | 2.0 | 3.1 | 3.2 | 3.1 | 2.2 | 2.9 |
| OR Quart 3 | 0.89 | 1.4 | 1.3 | 1.4 | 1.3 | 1.7 | 0.24 | 0.24 | 0.63 |
| p Value | 0.81 | 0.70 | 0.63 | 0.48 | 0.71 | 0.27 | 0.086 | 0.21 | 0.50 |
| 95% CI of | 0.34 | 0.29 | 0.49 | 0.55 | 0.29 | 0.66 | 0.046 | 0.026 | 0.16 |
| OR Quart3 | 2.3 | 6.3 | 3.3 | 3.6 | 6.2 | 4.3 | 1.2 | 2.2 | 2.4 |
| OR Quart 4 | 1.3 | 1.7 | 0.88 | 2.2 | 2.4 | 2.1 | 1.5 | 1.0 | 1.4 |
| p Value | 0.53 | 0.47 | 0.80 | 0.087 | 0.21 | 0.13 | 0.45 | 0.98 | 0.55 |
| 95% CI of | 0.54 | 0.39 | 0.33 | 0.89 | 0.61 | 0.82 | 0.51 | 0.24 | 0.44 |
| OR Quart4 | 3.3 | 7.4 | 2.4 | 5.5 | 9.8 | 5.2 | 4.6 | 4.2 | 4.6 |

EP 3 489 688 A1

Table 6: Comparison of marker levels in EDTA samples collected from Cohort 1 (patients that did not progress beyond RIFLE stage 0 or R) and in EDTA samples collected from subjects at 0, 24 hours, and 48 hours prior to reaching stage I or F in Cohort 2.

| C-C motif chemokine 18 | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 172 | 215 | 172 | 248 | 172 | 220 |
| Average | 240 | 229 | 240 | 325 | 240 | 302 |
| Stdev | 220 | 100 | 220 | 300 | 220 | 325 |
| p(t-test) | | 0.85 | | 0.062 | | 0.24 |
| Min | 31.7 | 51.5 | 31.7 | 92.4 | 31.7 | 103 |
| Max | 2110 | 378 | 2110 | 1650 | 2110 | 1550 |
| n (Samp) | 282 | 16 | 282 | 28 | 282 | 20 |
| n (Patient) | 160 | 16 | 160 | 28 | 160 | 20 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | nd | nd | 180 | 266 |
| Average | nd | nd | nd | nd | 252 | 280 |
| Stdev | nd | nd | nd | nd | 237 | 101 |
| p(t-test) | nd | nd | nd | nd | | 0.77 |
| Min | nd | nd | nd | nd | 31.7 | 154 |
| Max | nd | nd | nd | nd | 2110 | 422 |
| n (Samp) | nd | nd | nd | nd | 353 | 6 |
| n (Patient) | nd | nd | nd | nd | 193 | 6 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 176 | 238 | 176 | 254 | 176 | 198 |
| Average | 251 | 231 | 251 | 328 | 251 | 310 |
| Stdev | 226 | 108 | 226 | 307 | 226 | 354 |
| p(t-test) | | 0.75 | | 0.11 | | 0.32 |
| Min | 37.8 | 51.5 | 37.8 | 92.4 | 37.8 | 103 |
| Max | 2110 | 378 | 2110 | 1650 | 2110 | 1550 |
| n (Samp) | 258 | 13 | 258 | 26 | 258 | 17 |
| n (Patient) | 140 | 13 | 140 | 26 | 140 | 17 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.57 | nd | 0.55 | 0.64 | nd | 0.63 | 0.59 | 0.67 | 0.55 |
| SE | 0.076 | nd | 0.084 | 0.059 | nd | 0.061 | 0.069 | 0.12 | 0.074 |
| p | 0.33 | nd | 0.56 | 0.020 | nd | 0.040 | 0.18 | 0.17 | 0.46 |
| nCohort 1 | 282 | nd | 258 | 282 | nd | 258 | 282 | 353 | 258 |
| nCohort 2 | 16 | nd | 13 | 28 | nd | 26 | 20 | 6 | 17 |
| Cutoff 1 | 161 | nd | 159 | 169 | nd | 189 | 152 | 207 | 120 |
| Sens 1 | 75% | nd | 77% | 71% | nd | 73% | 70% | 83% | 71% |
| Spec 1 | 45% | nd | 42% | 48% | nd | 55% | 41% | 59% | 30% |
| Cutoff 2 | 159 | nd | 144 | 132 | nd | 150 | 118 | 207 | 117 |
| Sens 2 | 81% | nd | 85% | 82% | nd | 81% | 80% | 83% | 82% |
| Spec 2 | 44% | nd | 38% | 36% | nd | 40% | 31% | 59% | 29% |
| Cutoff 3 | 87.9 | nd | 87.9 | 107 | nd | 107 | 105 | 152 | 103 |
| Sens 3 | 94% | nd | 92% | 93% | nd | 92% | 90% | 100% | 94% |
| Spec 3 | 19% | nd | 18% | 27% | nd | 26% | 26% | 39% | 24% |
| Cutoff 4 | 268 | nd | 278 | 268 | nd | 278 | 268 | 276 | 278 |
| Sens 4 | 38% | nd | 38% | 46% | nd | 46% | 40% | 50% | 35% |
| Spec 4 | 70% | nd | 70% | 70% | nd | 70% | 70% | 70% | 70% |
| Cutoff 5 | 345 | nd | 384 | 345 | nd | 384 | 345 | 347 | 384 |
| Sens 5 | 19% | nd | 0% | 32% | nd | 19% | 15% | 33% | 12% |
| Spec 5 | 80% | nd | 80% | 80% | nd | 80% | 80% | 80% | 80% |
| Cutoff 6 | 497 | nd | 537 | 497 | nd | 537 | 497 | 497 | 537 |
| Sens 6 | 0% | nd | 0% | 11% | nd | 12% | 10% | 0% | 12% |
| Spec 6 | 90% | nd | 90% | 90% | nd | 90% | 90% | 90% | 90% |
| OR Quart 2 | 2.0 | nd | 1.5 | 3.7 | nd | 1.4 | 1.3 | >1.0 | 1.3 |
| p Value | 0.42 | nd | 0.66 | 0.11 | nd | 0.70 | 0.71 | <1.0 | 0.71 |
| 95% CI of OR Quart2 | 0.36 | nd | 0.24 | 0.74 | nd | 0.29 | 0.29 | >0.062 | 0.29 |
| | 11 | nd | 9.3 | 18 | nd | 6.3 | 6.2 | na | 6.2 |
| OR Quart 3 | 2.6 | nd | 2.0 | 4.3 | nd | 3.3 | 2.1 | >2.0 | 1.7 |
| p Value | 0.26 | nd | 0.42 | 0.069 | nd | 0.084 | 0.31 | <0.57 | 0.48 |
| 95% CI of | 0.49 | nd | 0.36 | 0.89 | nd | 0.85 | 0.50 | >0.18 | 0.39 |
| OR Quart3 | 14 | nd | 11 | 21 | nd | 13 | 8.7 | na | 7.4 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart 4 | 2.6 | nd | 2.0 | 6.2 | nd | 3.7 | 2.4 | >3.1 | 1.7 |
| p Value | 0.27 | nd | 0.42 | 0.021 | nd | 0.054 | 0.21 | <0.34 | 0.48 |
| 95% CI of | 0.48 | nd | 0.36 | 1.3 | nd | 0.98 | 0.61 | >0.31 | 0.39 |
| OR Quart4 | 14 | nd | 11 | 29 | nd | 14 | 9.8 | na | 7.4 |

**C-C motif chemokine 24**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 259 | 392 | 259 | 198 | 259 | 323 |
| Average | 397 | 544 | 397 | 325 | 397 | 429 |
| Stdev | 402 | 578 | 402 | 311 | 402 | 404 |
| p(t-test) | | 0.17 | | 0.36 | | 0.75 |
| Min | 0.0260 | 40.3 | 0.0260 | 13.1 | 0.0260 | 12.4 |
| Max | 2920 | 2430 | 2920 | 1100 | 2920 | 1380 |
| n (Samp) | 216 | 16 | 216 | 28 | 216 | 17 |
| n (Patient) | 132 | 16 | 132 | 28 | 132 | 17 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 270 | 238 | 270 | 173 | 270 | 323 |
| Average | 409 | 394 | 409 | 345 | 409 | 471 |
| Stdev | 412 | 311 | 412 | 478 | 412 | 461 |
| p(t-test) | | 0.90 | | 0.45 | | 0.58 |
| Min | 2.15 | 40.3 | 2.15 | 13.1 | 2.15 | 12.4 |
| Max | 2920 | 980 | 2920 | 2430 | 2920 | 1380 |
| n (Samp) | 198 | 13 | 198 | 29 | 198 | 15 |
| n (Patient) | 117 | 13 | 117 | 29 | 117 | 15 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.58 | nd | 0.51 | 0.43 | nd | 0.39 | 0.51 | nd | 0.50 |
| SE | 0.077 | nd | 0.083 | 0.059 | nd | 0.059 | 0.073 | nd | 0.078 |
| p | 0.29 | nd | 0.93 | 0.23 | nd | 0.064 | 0.89 | nd | 0.99 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| nCohort 1 | 216 | nd | 198 | 216 | nd | 198 | 216 | nd | 198 |
| nCohort 2 | 16 | nd | 13 | 28 | nd | 29 | 17 | nd | 15 |
| Cutoff 1 | 201 | nd | 173 | 85.2 | nd | 80.9 | 181 | nd | 96.1 |
| Sens 1 | 75% | nd | 77% | 71% | nd | 72% | 71% | nd | 73% |
| Spec 1 | 38% | nd | 29% | 12% | nd | 11% | 33% | nd | 15% |
| Cutoff 2 | 171 | nd | 154 | 69.9 | nd | 67.2 | 85.2 | nd | 85.2 |
| Sens 2 | 81% | nd | 85% | 82% | nd | 83% | 82% | nd | 80% |
| Spec 2 | 31% | nd | 26% | 10% | nd | 9% | 12% | nd | 12% |
| Cutoff 3 | 67.2 | nd | 67.2 | 33.2 | nd | 25.7 | 25.7 | nd | 25.7 |
| Sens 3 | 94% | nd | 92% | 93% | nd | 93% | 94% | nd | 93% |
| Spec 3 | 10% | nd | 9% | 2% | nd | 2% | 2% | nd | 2% |
| Cutoff 4 | 438 | nd | 440 | 438 | nd | 440 | 438 | nd | 440 |
| Sens 4 | 50% | nd | 38% | 32% | nd | 28% | 41% | nd | 40% |
| Spec 4 | 70% | nd | 70% | 70% | nd | 70% | 70% | nd | 70% |
| Cutoff 5 | 580 | nd | 625 | 580 | nd | 625 | 580 | nd | 625 |
| Sens 5 | 44% | nd | 31% | 21% | nd | 14% | 24% | nd | 33% |
| Spec 5 | 80% | nd | 80% | 80% | nd | 80% | 80% | nd | 80% |
| Cutoff 6 | 881 | nd | 940 | 881 | nd | 940 | 881 | nd | 940 |
| Sens 6 | 12% | nd | 8% | 11% | nd | 3% | 18% | nd | 27% |
| Spec 6 | 90% | nd | 90% | 90% | nd | 90% | 90% | nd | 90% |
| OR Quart 2 | 2.6 | nd | 2.6 | 1.0 | nd | 1.2 | 0.38 | nd | 0.31 |
| p Value | 0.26 | nd | 0.27 | 1.0 | nd | 0.75 | 0.26 | nd | 0.17 |
| 95% CI of OR Quart2 | 0.49 | nd | 0.48 | 0.30 | nd | 0.35 | 0.070 | nd | 0.060 |
| | 14 | nd | 14 | 3.3 | nd | 4.3 | 2.0 | nd | 1.6 |
| OR Quart 3 | 1.0 | nd | 0.48 | 0.82 | nd | 1.2 | 0.79 | nd | 0.31 |
| p Value | 1.0 | nd | 0.55 | 0.75 | nd | 0.75 | 0.73 | nd | 0.17 |
| 95% CI of OR Quart3 | 0.14 | nd | 0.042 | 0.24 | nd | 0.35 | 0.20 | nd | 0.060 |
| | 7.3 | nd | 5.5 | 2.8 | nd | 4.3 | 3.1 | nd | 1.6 |
| OR Quart 4 | 3.8 | nd | 2.6 | 2.0 | nd | 2.8 | 1.2 | nd | 0.83 |
| p Value | 0.10 | nd | 0.27 | 0.20 | nd | 0.068 | 0.77 | nd | 0.78 |
| 95% CI of | 0.76 | nd | 0.48 | 0.69 | nd | 0.93 | 0.35 | nd | 0.24 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart4 | 19 | nd | 14 | 5.9 | nd | 8.7 | 4.2 | nd | 2.9 |

**C-C motif chemokine 8**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 12.7 | 14.8 | 12.7 | 13.7 | 12.7 | 17.5 |
| Average | 18.3 | 18.6 | 18.3 | 17.2 | 18.3 | 24.4 |
| Stdev | 26.5 | 10.7 | 26.5 | 18.2 | 26.5 | 17.8 |
| p(t-test) |  | 0.97 |  | 0.83 |  | 0.36 |
| Min | 0.162 | 6.68 | 0.162 | 1.20 | 0.162 | 9.54 |
| Max | 180 | 41.0 | 180 | 101 | 180 | 72.1 |
| n (Samp) | 216 | 16 | 216 | 28 | 216 | 17 |
| n (Patient) | 132 | 16 | 132 | 28 | 132 | 17 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 12.3 | 14.5 | 12.3 | 13.8 | 12.3 | 16.3 |
| Average | 19.1 | 16.6 | 19.1 | 16.6 | 19.1 | 23.8 |
| Stdev | 28.1 | 9.85 | 28.1 | 17.6 | 28.1 | 18.9 |
| p(t-test) |  | 0.74 |  | 0.63 |  | 0.53 |
| Min | 0.162 | 6.68 | 0.162 | 1.20 | 0.162 | 9.54 |
| Max | 180 | 41.0 | 180 | 101 | 180 | 72.1 |
| n (Samp) | 198 | 13 | 198 | 29 | 198 | 15 |
| n (Patient) | 117 | 13 | 117 | 29 | 117 | 15 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.61 | nd | 0.58 | 0.53 | nd | 0.53 | 0.70 | nd | 0.68 |
| SE | 0.077 | nd | 0.085 | 0.059 | nd | 0.058 | 0.073 | nd | 0.078 |
| p | 0.14 | nd | 0.37 | 0.57 | nd | 0.56 | 0.0050 | nd | 0.021 |
| nCohort 1 | 216 | nd | 198 | 216 | nd | 198 | 216 | nd | 198 |
| nCohort 2 | 16 | nd | 13 | 28 | nd | 29 | 17 | nd | 15 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 1 | 12.8 | nd | 9.69 | 10.7 | nd | 10.7 | 15.4 | nd | 13.7 |
| Sens 1 | 75% | nd | 85% | 71% | nd | 72% | 71% | nd | 73% |
| Spec 1 | 50% | nd | 35% | 39% | nd | 42% | 62% | nd | 56% |
| Cutoff 2 | 9.69 | nd | 9.69 | 7.48 | nd | 7.48 | 13.0 | nd | 13.0 |
| Sens 2 | 88% | nd | 85% | 82% | nd | 83% | 82% | nd | 80% |
| Spec 2 | 33% | nd | 35% | 24% | nd | 25% | 52% | nd | 54% |
| Cutoff 3 | 8.22 | nd | 8.22 | 4.81 | nd | 4.81 | 11.3 | nd | 11.3 |
| Sens 3 | 94% | nd | 92% | 93% | nd | 93% | 94% | nd | 93% |
| Spec 3 | 28% | nd | 29% | 18% | nd | 19% | 41% | nd | 44% |
| Cutoff 4 | 17.1 | nd | 17.0 | 17.1 | nd | 17.0 | 17.1 | nd | 17.0 |
| Sens 4 | 31% | nd | 23% | 25% | nd | 21% | 53% | nd | 40% |
| Spec 4 | 70% | nd | 70% | 70% | nd | 70% | 70% | nd | 70% |
| Cutoff 5 | 20.1 | nd | 20.3 | 20.1 | nd | 20.3 | 20.1 | nd | 20.3 |
| Sens 5 | 25% | nd | 15% | 21% | nd | 17% | 29% | nd | 27% |
| Spec 5 | 80% | nd | 80% | 80% | nd | 80% | 80% | nd | 80% |
| Cutoff 6 | 29.8 | nd | 33.4 | 29.8 | nd | 33.4 | 29.8 | nd | 33.4 |
| Sens 6 | 19% | nd | 8% | 11% | nd | 3% | 24% | nd | 20% |
| Spec 6 | 90% | nd | 91% | 90% | nd | 91% | 90% | nd | 91% |
| OR Quart 2 | 4.2 | nd | 3.1 | 0.82 | nd | 0.80 | >3.2 | nd | >2.1 |
| p Value | 0.20 | nd | 0.34 | 0.75 | nd | 0.73 | <0.32 | nd | <0.56 |
| 95% CI of OR Quart2 | 0.46 | nd | 0.31 | 0.24 | nd | 0.23 | >0.32 | nd | >0.18 |
| | 39 | nd | 30 | 2.8 | nd | 2.8 | na | nd | na |
| OR Quart 3 | 7.8 | nd | 7.8 | 1.8 | nd | 2.2 | >9.3 | nd | >9.4 |
| p Value | 0.058 | nd | 0.060 | 0.29 | nd | 0.14 | <0.039 | nd | <0.038 |
| 95% CI of OR Quart3 | 0.93 | nd | 0.92 | 0.61 | nd | 0.77 | >1.1 | nd | >1.1 |
| | 66 | nd | 65 | 5.3 | nd | 6.4 | na | nd | na |
| OR Quart 4 | 4.2 | nd | 2.0 | 1.2 | nd | 0.98 | >6.6 | nd | >5.4 |
| p Value | 0.20 | nd | 0.58 | 0.77 | nd | 0.97 | <0.086 | nd | <0.13 |
| 95% CI of OR Quart4 | 0.46 | nd | 0.18 | 0.38 | nd | 0.30 | >0.77 | nd | >0.61 |
| | 39 | nd | 23 | 3.8 | nd | 3.2 | na | nd | na |

(continued)

**Cathepsin D**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 229000 | 205000 | 229000 | 234000 | 229000 | 249000 |
| Average | 257000 | 294000 | 257000 | 292000 | 257000 | 281000 |
| Stdev | 147000 | 295000 | 147000 | 170000 | 147000 | 135000 |
| p(t-test) | | 0.36 | | 0.24 | | 0.47 |
| Min | 34100 | 127000 | 34100 | 90500 | 34100 | 83000 |
| Max | 1020000 | 1350000 | 1020000 | 655000 | 1020000 | 561000 |
| n (Samp) | 281 | 16 | 281 | 28 | 281 | 20 |
| n (Patient) | 159 | 16 | 159 | 28 | 159 | 20 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | nd | nd | 227000 | 309000 |
| Average | nd | nd | nd | nd | 260000 | 329000 |
| Stdev | nd | nd | nd | nd | 166000 | 197000 |
| p(t-test) | nd | nd | nd | nd | | 0.32 |
| Min | nd | nd | nd | nd | 34100 | 131000 |
| Max | nd | nd | nd | nd | 1870000 | 561000 |
| n (Samp) | nd | nd | nd | nd | 352 | 6 |
| n (Patient) | nd | nd | nd | nd | 192 | 6 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 236000 | 218000 | 236000 | 267000 | 236000 | 262000 |
| Average | 266000 | 237000 | 266000 | 332000 | 266000 | 310000 |
| Stdev | 148000 | 92200 | 148000 | 261000 | 148000 | 146000 |
| p(t-test) | | 0.49 | | 0.046 | | 0.24 |
| Min | 54000 | 130000 | 54000 | 90500 | 54000 | 83000 |
| Max | 1020000 | 411000 | 1020000 | 1350000 | 1020000 | 655000 |
| n (Samp) | 257 | 13 | 257 | 26 | 257 | 17 |
| n (Patient) | 139 | 13 | 139 | 26 | 139 | 17 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.49 | nd | 0.48 | 0.55 | nd | 0.56 | 0.57 | 0.60 | 0.61 |
| SE | 0.075 | nd | 0.083 | 0.058 | nd | 0.061 | 0.069 | 0.12 | 0.075 |
| p | 0.92 | nd | 0.76 | 0.42 | nd | 0.36 | 0.29 | 0.42 | 0.15 |
| nCohort 1 | 281 | nd | 257 | 281 | nd | 257 | 281 | 352 | 257 |
| nCohort 2 | 16 | nd | 13 | 28 | nd | 26 | 20 | 6 | 17 |
| Cutoff 1 | 159000 | nd | 167000 | 148000 | nd | 154000 | 212000 | 157000 | 244000 |
| Sens 1 | 75% | nd | 77% | 71% | nd | 73% | 70% | 83% | 71% |
| Spec 1 | 30% | nd | 32% | 26% | nd | 25% | 44% | 29% | 54% |
| Cutoff 2 | 143000 | nd | 159000 | 141000 | nd | 142000 | 169000 | 157000 | 212000 |
| Sens 2 | 81% | nd | 85% | 82% | nd | 81% | 80% | 83% | 82% |
| Spec 2 | 24% | nd | 28% | 22% | nd | 20% | 34% | 29% | 42% |
| Cutoff 3 | 129000 | nd | 137000 | 126000 | nd | 126000 | 157000 | 130000 | 166000 |
| Sens 3 | 94% | nd | 92% | 93% | nd | 92% | 90% | 100% | 94% |
| Spec 3 | 18% | nd | 19% | 16% | nd | 13% | 30% | 17% | 31% |
| Cutoff 4 | 305000 | nd | 310000 | 305000 | nd | 310000 | 305000 | 306000 | 310000 |
| Sens 4 | 31% | nd | 23% | 43% | nd | 38% | 35% | 50% | 35% |
| Spec 4 | 70% | nd | 70% | 70% | nd | 70% | 70% | 70% | 70% |
| Cutoff 5 | 357000 | nd | 374000 | 357000 | nd | 374000 | 357000 | 352000 | 374000 |
| Sens 5 | 19% | nd | 8% | 32% | nd | 35% | 25% | 50% | 29% |
| Spec 5 | 80% | nd | 80% | 80% | nd | 80% | 80% | 80% | 80% |
| Cutoff 6 | 450000 | nd | 469000 | 450000 | nd | 469000 | 450000 | 450000 | 469000 |
| Sens 6 | 6% | nd | 0% | 21% | nd | 19% | 15% | 33% | 18% |
| Spec 6 | 90% | nd | 90% | 90% | nd | 90% | 90% | 90% | 90% |
| OR Quart 2 | 0.49 | nd | 0.67 | 0.60 | nd | 0.68 | 3.2 | 2.0 | 4.1 |
| p Value | 0.42 | nd | 0.66 | 0.39 | nd | 0.53 | 0.17 | 0.57 | 0.21 |
| 95% CI of | 0.088 | nd | 0.11 | 0.19 | nd | 0.21 | 0.62 | 0.18 | 0.45 |
| OR Quart2 | 2.8 | nd | 4.1 | 1.9 | nd | 2.3 | 16 | 22 | 38 |
| OR Quart 3 | 1.6 | nd | 2.1 | 0.73 | nd | 0.68 | 3.2 | 0 | 7.7 |
| p Value | 0.50 | nd | 0.31 | 0.58 | nd | 0.53 | 0.17 | na | 0.060 |
| 95% CI of | 0.42 | nd | 0.50 | 0.24 | nd | 0.21 | 0.62 | na | 0.92 |
| OR Quart3 | 5.8 | nd | 8.8 | 2.2 | nd | 2.3 | 16 | na | 64 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart 4 | 1.0 | nd | 0.67 | 1.1 | nd | 1.3 | 3.1 | 3.0 | 5.2 |
| p Value | 0.98 | nd | 0.66 | 0.82 | nd | 0.62 | 0.17 | 0.34 | 0.14 |
| 95% CI of OR Quart4 | 0.24 | nd | 0.11 | 0.41 | nd | 0.46 | 0.61 | 0.31 | 0.60 |
| | 4.2 | nd | 4.1 | 3.1 | nd | 3.7 | 16 | 30 | 46 |

**C-X-C motif chemokine 13**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 47.3 | 51.3 | 47.3 | 50.8 | 47.3 | 47.6 |
| Average | 124 | 177 | 124 | 105 | 124 | 180 |
| Stdev | 237 | 487 | 237 | 245 | 237 | 278 |
| p(t-test) | | 0.44 | | 0.69 | | 0.36 |
| Min | 9.90 | 15.3 | 9.90 | 12.9 | 9.90 | 12.5 |
| Max | 1790 | 2000 | 1790 | 1340 | 1790 | 918 |
| n (Samp) | 216 | 16 | 216 | 28 | 216 | 17 |
| n (Patient) | 132 | 16 | 132 | 28 | 132 | 17 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 52.2 | 49.8 | 52.2 | 53.6 | 52.2 | 50.0 |
| Average | 123 | 54.6 | 123 | 103 | 123 | 159 |
| Stdev | 218 | 26.9 | 218 | 241 | 218 | 247 |
| p(t-test) | | 0.26 | | 0.64 | | 0.55 |
| Min | 9.90 | 15.3 | 9.90 | 12.9 | 9.90 | 12.5 |
| Max | 1790 | 109 | 1790 | 1340 | 1790 | 918 |
| n (Samp) | 198 | 13 | 198 | 29 | 198 | 15 |
| n (Patient) | 117 | 13 | 117 | 29 | 117 | 15 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.49 | nd | 0.44 | 0.50 | nd | 0.48 | 0.51 | nd | 0.51 |
| SE | 0.075 | nd | 0.085 | 0.058 | nd | 0.058 | 0.073 | nd | 0.078 |
| p | 0.94 | nd | 0.51 | 0.94 | nd | 0.69 | 0.89 | nd | 0.85 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| nCohort 1 | 216 | nd | 198 | 216 | nd | 198 | 216 | nd | 198 |
| nCohort 2 | 16 | nd | 13 | 28 | nd | 29 | 17 | nd | 15 |
| Cutoff 1 | 35.2 | nd | 35.2 | 39.1 | nd | 39.1 | 34.2 | nd | 34.2 |
| Sens 1 | 75% | nd | 77% | 71% | nd | 72% | 71% | nd | 73% |
| Spec 1 | 37% | nd | 34% | 42% | nd | 40% | 36% | nd | 33% |
| Cutoff 2 | 34.2 | nd | 34.2 | 29.3 | nd | 29.3 | 27.8 | nd | 27.8 |
| Sens 2 | 81% | nd | 85% | 82% | nd | 83% | 82% | nd | 80% |
| Spec 2 | 36% | nd | 33% | 30% | nd | 27% | 25% | nd | 23% |
| Cutoff 3 | 25.2 | nd | 24.9 | 23.5 | nd | 23.5 | 15.4 | nd | 15.4 |
| Sens 3 | 94% | nd | 92% | 93% | nd | 93% | 94% | nd | 93% |
| Spec 3 | 22% | nd | 19% | 20% | nd | 18% | 6% | nd | 6% |
| Cutoff 4 | 102 | nd | 106 | 102 | nd | 106 | 102 | nd | 106 |
| Sens 4 | 12% | nd | 8% | 14% | nd | 10% | 29% | nd | 33% |
| Spec 4 | 70% | nd | 70% | 70% | nd | 70% | 70% | nd | 70% |
| Cutoff 5 | 140 | nd | 141 | 140 | nd | 141 | 140 | nd | 141 |
| Sens 5 | 6% | nd | 0% | 7% | nd | 3% | 29% | nd | 33% |
| Spec 5 | 80% | nd | 80% | 80% | nd | 80% | 80% | nd | 80% |
| Cutoff 6 | 251 | nd | 254 | 251 | nd | 254 | 251 | nd | 254 |
| Sens 6 | 6% | nd | 0% | 4% | nd | 3% | 24% | nd | 20% |
| Spec 6 | 90% | nd | 90% | 90% | nd | 90% | 90% | nd | 90% |
| OR Quart 2 | 9.1 | nd | >6.8 | 3.1 | nd | 4.8 | 1.7 | nd | 1.0 |
| p Value | 0.040 | nd | <0.082 | 0.063 | nd | 0.020 | 0.47 | nd | 1.0 |
| 95% CI of OR Quart2 | 1.1 | nd | >0.79 | 0.94 | nd | 1.3 | 0.39 | nd | 0.24 |
| | 75 | nd | na | 10 | nd | 18 | 7.6 | nd | 4.2 |
| OR Quart 3 | 5.4 | nd | >5.5 | 2.5 | nd | 3.4 | 1.4 | nd | 0.48 |
| p Value | 0.13 | nd | <0.12 | 0.15 | nd | 0.080 | 0.70 | nd | 0.41 |
| 95% CI of OR Quart3 | 0.61 | nd | >0.62 | 0.72 | nd | 0.86 | 0.29 | nd | 0.084 |
| | 48 | nd | na | 8.5 | nd | 13 | 6.4 | nd | 2.7 |
| OR Quart 4 | 2.0 | nd | >2.1 | 1.0 | nd | 1.8 | 1.7 | nd | 1.2 |
| p Value | 0.57 | nd | <0.54 | 1.0 | nd | 0.45 | 0.48 | nd | 0.75 |
| 95% CI of | 0.18 | nd | >0.19 | 0.24 | nd | 0.40 | 0.39 | nd | 0.32 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart4 | 23 | nd | na | 4.2 | nd | 7.8 | 7.5 | nd | 4.9 |

**Insulin-like growth factor-binding protein 3**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3220 | 2570 | 3220 | 3020 | 3220 | 2890 |
| Average | 3530 | 3330 | 3530 | 3480 | 3530 | 3450 |
| Stdev | 1570 | 1940 | 1570 | 1530 | 1570 | 2040 |
| p(t-test) | | 0.63 | | 0.87 | | 0.83 |
| Min | 823 | 698 | 823 | 651 | 823 | 730 |
| Max | 8600 | 6560 | 8600 | 7360 | 8600 | 7520 |
| n (Samp) | 282 | 16 | 282 | 28 | 282 | 20 |
| n (Patient) | 160 | 16 | 160 | 28 | 160 | 20 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | nd | nd | nd | nd | 3090 | 3030 |
| Average | nd | nd | nd | nd | 3480 | 3870 |
| Stdev | nd | nd | nd | nd | 1600 | 1920 |
| p(t-test) | nd | nd | nd | nd | | 0.55 |
| Min | nd | nd | nd | nd | 651 | 2160 |
| Max | nd | nd | nd | nd | 8600 | 6560 |
| n (Samp) | nd | nd | nd | nd | 353 | 6 |
| n (Patient) | nd | nd | nd | nd | 193 | 6 |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 3060 | 2280 | 3060 | 3020 | 3060 | 3020 |
| Average | 3410 | 2650 | 3410 | 3540 | 3410 | 3240 |
| Stdev | 1460 | 1430 | 1460 | 1610 | 1460 | 1950 |
| p(t-test) | | 0.071 | | 0.66 | | 0.66 |
| Min | 823 | 698 | 823 | 651 | 823 | 730 |
| Max | 8260 | 6250 | 8260 | 7360 | 8260 | 7520 |
| n (Samp) | 258 | 13 | 258 | 26 | 258 | 17 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Patient) | 140 | 13 | 140 | 26 | 140 | 17 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.44 | nd | 0.34 | 0.50 | nd | 0.53 | 0.46 | 0.56 | 0.44 |
| SE | 0.076 | nd | 0.084 | 0.057 | nd | 0.060 | 0.068 | 0.12 | 0.074 |
| p | 0.43 | nd | 0.052 | 0.99 | nd | 0.67 | 0.57 | 0.62 | 0.45 |
| nCohort 1 | 282 | nd | 258 | 282 | nd | 258 | 282 | 353 | 258 |
| nCohort 2 | 16 | nd | 13 | 28 | nd | 26 | 20 | 6 | 17 |
| Cutoff 1 | 2130 | nd | 1950 | 2410 | nd | 2410 | 2370 | 2430 | 2040 |
| Sens 1 | 75% | nd | 77% | 71% | nd | 73% | 70% | 83% | 71% |
| Spec 1 | 21% | nd | 15% | 28% | nd | 29% | 27% | 31% | 19% |
| Cutoff 2 | 1950 | nd | 1610 | 2220 | nd | 2260 | 1880 | 2430 | 1590 |
| Sens 2 | 81% | nd | 85% | 82% | nd | 81% | 80% | 83% | 82% |
| Spec 2 | 15% | nd | 7% | 23% | nd | 24% | 12% | 31% | 7% |
| Cutoff 3 | 1210 | nd | 1210 | 2130 | nd | 2130 | 1490 | 2150 | 730 |
| Sens 3 | 94% | nd | 92% | 93% | nd | 92% | 90% | 100% | 94% |
| Spec 3 | 2% | nd | 3% | 21% | nd | 22% | 6% | 22% | 0% |
| Cutoff 4 | 4250 | nd | 4130 | 4250 | nd | 4130 | 4250 | 4230 | 4130 |
| Sens 4 | 25% | nd | 8% | 36% | nd | 38% | 30% | 33% | 29% |
| Spec 4 | 70% | nd | 70% | 70% | nd | 70% | 70% | 70% | 70% |
| Cutoff 5 | 4870 | nd | 4680 | 4870 | nd | 4680 | 4870 | 4800 | 4680 |
| Sens 5 | 25% | nd | 8% | 14% | nd | 27% | 20% | 33% | 18% |
| Spec 5 | 80% | nd | 80% | 80% | nd | 80% | 80% | 80% | 80% |
| Cutoff 6 | 5740 | nd | 5510 | 5740 | nd | 5510 | 5740 | 5740 | 5510 |
| Sens 6 | 25% | nd | 8% | 7% | nd | 8% | 20% | 33% | 12% |
| Spec 6 | 90% | nd | 90% | 90% | nd | 90% | 90% | 90% | 90% |
| OR Quart 2 | 0.75 | nd | 3.1 | 0.86 | nd | 1.2 | 0.59 | 2.0 | 1.7 |
| p Value | 0.71 | nd | 0.33 | 0.79 | nd | 0.77 | 0.48 | 0.57 | 0.47 |
| 95% CI of | 0.16 | nd | 0.31 | 0.27 | nd | 0.38 | 0.14 | 0.18 | 0.39 |
| OR Quart2 | 3.5 | nd | 30 | 2.7 | nd | 3.7 | 2.6 | 22 | 7.5 |
| OR Quart 3 | 0.49 | nd | 3.1 | 1.2 | nd | 0.65 | 1.2 | 0.99 | 1.0 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| p Value | 0.41 | nd | 0.33 | 0.79 | nd | 0.51 | 0.75 | 0.99 | 1.0 |
| 95% CI of OR Quart3 | 0.086 | nd | 0.31 | 0.40 | nd | 0.17 | 0.36 | 0.061 | 0.19 |
|  | 2.7 | nd | 30 | 3.4 | nd | 2.4 | 4.2 | 16 | 5.1 |
| OR Quart 4 | 1.9 | nd | 6.6 | 1.0 | nd | 1.6 | 1.2 | 2.0 | 2.1 |
| p Value | 0.34 | nd | 0.085 | 0.98 | nd | 0.42 | 0.74 | 0.57 | 0.30 |
| 95% CI of OR Quart4 | 0.52 | nd | 0.77 | 0.34 | nd | 0.53 | 0.36 | 0.18 | 0.51 |
|  | 6.6 | nd | 56 | 3.0 | nd | 4.7 | 4.2 | 22 | 8.9 |

**Immunoglogulin G1**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 8800000 | 7170000 | 8800000 | 1.04E7 | 8800000 | 9000000 |
| Average | 1.04E7 | 7280000 | 1.04E7 | 1.29E7 | 1.04E7 | 9890000 |
| Stdev | 5920000 | 1700000 | 5920000 | 7430000 | 5920000 | 4770000 |
| p(t-test) |  | 0.12 |  | 0.076 |  | 0.82 |
| Min | 2060000 | 4390000 | 2060000 | 4620000 | 2060000 | 4390000 |
| Max | 4.18E7 | 9590000 | 4.18E7 | 2.98E7 | 4.18E7 | 2.05E7 |
| n (Samp) | 205 | 9 | 205 | 20 | 205 | 8 |
| n (Patient) | 127 | 9 | 127 | 20 | 127 | 8 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 8690000 | 7170000 | 8690000 | 1.01E7 | 8690000 | 8470000 |
| Average | 1.01E7 | 7280000 | 1.01E7 | 1.21E7 | 1.01E7 | 9950000 |
| Stdev | 5930000 | 1700000 | 5930000 | 6630000 | 5930000 | 5150000 |
| p(t-test) |  | 0.15 |  | 0.19 |  | 0.94 |
| Min | 2060000 | 4390000 | 2060000 | 4620000 | 2060000 | 4390000 |
| Max | 4.18E7 | 9590000 | 4.18E7 | 2.63E7 | 4.18E7 | 2.05E7 |
| n (Samp) | 191 | 9 | 191 | 18 | 191 | 7 |
| n (Patient) | 113 | 9 | 113 | 18 | 113 | 7 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.34 | nd | 0.36 | 0.61 | nd | 0.60 | 0.51 | nd | 0.52 |
| SE | 0.10 | nd | 0.10 | 0.070 | nd | 0.073 | 0.10 | nd | 0.11 |
| p | 0.11 | nd | 0.17 | 0.11 | nd | 0.15 | 0.94 | nd | 0.89 |
| nCohort 1 | 205 | nd | 191 | 205 | nd | 191 | 205 | nd | 191 |
| nCohort 2 | 9 | nd | 9 | 20 | nd | 18 | 8 | nd | 7 |
| Cutoff 1 | 6940000 | nd | 6940000 | 7810000 | nd | 7810000 | 8010000 | nd | 8010000 |
| Sens 1 | 78% | nd | 78% | 75% | nd | 72% | 75% | nd | 71% |
| Spec 1 | 28% | nd | 31% | 40% | nd | 43% | 44% | nd | 47% |
| Cutoff 2 | 5340000 | nd | 5340000 | 7160000 | nd | 7140000 | 6940000 | nd | 6940000 |
| Sens 2 | 89% | nd | 89% | 80% | nd | 83% | 88% | nd | 86% |
| Spec 2 | 13% | nd | 14% | 34% | nd | 34% | 28% | nd | 31% |
| Cutoff 3 | 4360000 | nd | 4360000 | 6940000 | nd | 6940000 | 4360000 | nd | 4360000 |
| Sens 3 | 100% | nd | 100% | 95% | nd | 94% | 100% | nd | 100% |
| Spec 3 | 5% | nd | 6% | 28% | nd | 31% | 5% | nd | 6% |
| Cutoff 4 | 1.13E7 | nd | 1.11E7 | 1.13E7 | nd | 1.11E7 | 1.13E7 | nd | 1.11E7 |
| Sens 4 | 0% | nd | 0% | 40% | nd | 39% | 25% | nd | 29% |
| Spec 4 | 71% | nd | 70% | 71% | nd | 70% | 71% | nd | 70% |
| Cutoff 5 | 1.38E7 | nd | 1.33E7 | 1.38E7 | nd | 1.33E7 | 1.38E7 | nd | 1.33E7 |
| Sens 5 | 0% | nd | 0% | 25% | nd | 22% | 12% | nd | 14% |
| Spec 5 | 80% | nd | 80% | 80% | nd | 80% | 80% | nd | 80% |
| Cutoff 6 | 1.71E7 | nd | 1.63E7 | 1.71E7 | nd | 1.63E7 | 1.71E7 | nd | 1.63E7 |
| Sens 6 | 0% | nd | 0% | 20% | nd | 22% | 12% | nd | 14% |
| Spec 6 | 90% | nd | 91% | 90% | nd | 91% | 90% | nd | 91% |
| OR Quart 2 | >2.1 | nd | >2.1 | 6.6 | nd | 6.7 | 3.1 | nd | 3.1 |
| p Value | <0.55 | nd | <0.55 | 0.086 | nd | 0.085 | 0.33 | nd | 0.34 |
| 95% CI of | >0.19 | nd | >0.18 | 0.77 | nd | 0.77 | 0.31 | nd | 0.31 |
| OR Quart2 | na | nd | na | 57 | nd | 57 | 31 | nd | 31 |
| OR Quart 3 | >5.5 | nd | >5.6 | 9.2 | nd | 6.7 | 3.1 | nd | 2.0 |
| p Value | <0.13 | nd | <0.12 | 0.040 | nd | 0.085 | 0.33 | nd | 0.57 |
| 95% CI of | >0.62 | nd | >0.63 | 1.1 | nd | 0.77 | 0.31 | nd | 0.18 |
| OR Quart3 | na | nd | na | 76 | nd | 57 | 31 | nd | 23 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart 4 | >2.1 | nd | >2.1 | 5.3 | nd | 5.3 | 0.98 | nd | 0.98 |
| p Value | <0.55 | nd | <0.55 | 0.13 | nd | 0.13 | 0.99 | nd | 0.99 |
| 95% CI of | >0.19 | nd | >0.18 | 0.60 | nd | 0.60 | 0.060 | nd | 0.060 |
| OR Quart4 | na | nd | na | 47 | nd | 47 | 16 | nd | 16 |

**Immunoglogulin G2**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.08E7 | 1.02E7 | 1.08E7 | 1.02E7 | 1.08E7 | 1.05E7 |
| Average | 1.37E7 | 8150000 | 1.37E7 | 1.05E7 | 1.37E7 | 1.01E7 |
| Stdev | 9640000 | 6300000 | 9640000 | 5640000 | 9640000 | 5710000 |
| p(t-test) |  | 0.090 |  | 0.15 |  | 0.30 |
| Min | 190000 | 1750000 | 190000 | 1750000 | 190000 | 1750000 |
| Max | 5.80E7 | 1.70E7 | 5.80E7 | 2.70E7 | 5.80E7 | 1.70E7 |
| n (Samp) | 205 | 9 | 205 | 20 | 205 | 8 |
| n (Patient) | 127 | 9 | 127 | 20 | 127 | 8 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.03E7 | 1.02E7 | 1.03E7 | 1.02E7 | 1.03E7 | 1.02E7 |
| Average | 1.33E7 | 8150000 | 1.33E7 | 1.05E7 | 1.33E7 | 1.00E7 |
| Stdev | 9390000 | 6300000 | 9390000 | 5580000 | 9390000 | 6160000 |
| p(t-test) |  | 0.11 |  | 0.22 |  | 0.37 |
| Min | 190000 | 1750000 | 190000 | 1750000 | 190000 | 1750000 |
| Max | 5.80E7 | 1.70E7 | 5.80E7 | 2.70E7 | 5.80E7 | 1.70E7 |
| n (Samp) | 191 | 9 | 191 | 18 | 191 | 7 |
| n (Patient) | 113 | 9 | 113 | 18 | 113 | 7 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.34 | nd | 0.35 | 0.39 | nd | 0.40 | 0.41 | nd | 0.41 |
| SE | 0.10 | nd | 0.10 | 0.070 | nd | 0.073 | 0.11 | nd | 0.12 |
| p | 0.10 | nd | 0.13 | 0.11 | nd | 0.16 | 0.38 | nd | 0.42 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| nCohort 1 | 205 | nd | 191 | 205 | nd | 191 | 205 | nd | 191 |
| nCohort 2 | 9 | nd | 9 | 20 | nd | 18 | 8 | nd | 7 |
| Cutoff 1 | 1930000 | nd | 1930000 | 8850000 | nd | 8850000 | 7850000 | nd | 7850000 |
| Sens 1 | 78% | nd | 78% | 70% | nd | 72% | 75% | nd | 71% |
| Spec 1 | 9% | nd | 9% | 27% | nd | 28% | 25% | nd | 27% |
| Cutoff 2 | 563000 | nd | 219000 | 6700000 | nd | 6700000 | 1930000 | nd | 1930000 |
| Sens 2 | 100% | nd | 100% | 80% | nd | 83% | 88% | nd | 86% |
| Spec 2 | 1% | nd | 1% | 20% | nd | 21% | 9% | nd | 9% |
| Cutoff 3 | 563000 | nd | 219000 | 4550000 | nd | 219000 | 563000 | nd | 219000 |
| Sens 3 | 100% | nd | 100% | 90% | nd | 100% | 100% | nd | 100% |
| Spec 3 | 1% | nd | 1% | 18% | nd | 1% | 1% | nd | 1% |
| Cutoff 4 | 1.64E7 | nd | 1.64E7 | 1.64E7 | nd | 1.64E7 | 1.64E7 | nd | 1.64E7 |
| Sens 4 | 22% | nd | 22% | 10% | nd | 6% | 12% | nd | 14% |
| Spec 4 | 71% | nd | 72% | 71% | nd | 72% | 71% | nd | 72% |
| Cutoff 5 | 1.85E7 | nd | 1.84E7 | 1.85E7 | nd | 1.84E7 | 1.85E7 | nd | 1.84E7 |
| Sens 5 | 0% | nd | 0% | 5% | nd | 6% | 0% | nd | 0% |
| Spec 5 | 80% | nd | 80% | 80% | nd | 80% | 80% | nd | 80% |
| Cutoff 6 | 2.55E7 | nd | 2.52E7 | 2.55E7 | nd | 2.52E7 | 2.55E7 | nd | 2.52E7 |
| Sens 6 | 0% | nd | 0% | 5% | nd | 6% | 0% | nd | 0% |
| Spec 6 | 90% | nd | 90% | 90% | nd | 90% | 90% | nd | 90% |
| OR Quart 2 | 1.0 | nd | 0 | 6.7 | nd | 6.8 | 3.2 | nd | 2.1 |
| p Value | 0.99 | nd | na | 0.083 | nd | 0.082 | 0.32 | nd | 0.55 |
| 95% CI of OR Quart2 | 0.062 | nd | na | 0.78 | nd | 0.79 | 0.32 | nd | 0.18 |
| | 17 | nd | na | 58 | nd | 58 | 32 | nd | 24 |
| OR Quart 3 | 3.1 | nd | 1.5 | 9.3 | nd | 8.1 | 2.1 | nd | 2.0 |
| p Value | 0.33 | nd | 0.65 | 0.038 | nd | 0.055 | 0.56 | nd | 0.57 |
| 95% CI of OR Quart3 | 0.31 | nd | 0.24 | 1.1 | nd | 0.96 | 0.18 | nd | 0.18 |
| | 31 | nd | 9.6 | 77 | nd | 68 | 24 | nd | 23 |
| OR Quart 4 | 4.3 | nd | 2.1 | 5.5 | nd | 4.3 | 2.1 | nd | 2.1 |
| p Value | 0.20 | nd | 0.41 | 0.13 | nd | 0.20 | 0.56 | nd | 0.55 |
| 95% CI of | 0.47 | nd | 0.36 | 0.62 | nd | 0.47 | 0.18 | nd | 0.18 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart4 | 40 | nd | 12 | 49 | nd | 40 | 24 | nd | 24 |

**Interleukin-11**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 67.2 | 37.8 | 67.2 | 89.1 | 67.2 | 51.1 |
| Average | 162 | 185 | 162 | 98.0 | 162 | 109 |
| Stdev | 635 | 477 | 635 | 75.8 | 635 | 142 |
| p(t-test) | | 0.89 | | 0.60 | | 0.73 |
| Min | 0.359 | 0.442 | 0.359 | 0.480 | 0.359 | 0.480 |
| Max | 6920 | 1940 | 6920 | 392 | 6920 | 569 |
| n (Samp) | 217 | 16 | 217 | 28 | 217 | 17 |
| n (Patient) | 133 | 16 | 133 | 28 | 133 | 17 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 62.2 | 33.9 | 62.2 | 90.7 | 62.2 | 55.3 |
| Average | 156 | 47.3 | 156 | 86.9 | 156 | 88.3 |
| Stdev | 660 | 52.1 | 660 | 50.4 | 660 | 80.0 |
| p(t-test) | | 0.55 | | 0.57 | | 0.69 |
| Min | 0.359 | 0.442 | 0.359 | 0.480 | 0.359 | 0.480 |
| Max | 6920 | 176 | 6920 | 198 | 6920 | 233 |
| n (Samp) | 199 | 13 | 199 | 29 | 199 | 15 |
| n (Patient) | 118 | 13 | 118 | 29 | 118 | 15 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.43 | nd | 0.37 | 0.57 | nd | 0.57 | 0.51 | nd | 0.53 |
| SE | 0.077 | nd | 0.085 | 0.059 | nd | 0.059 | 0.073 | nd | 0.079 |
| p | 0.36 | nd | 0.14 | 0.24 | nd | 0.21 | 0.91 | nd | 0.70 |
| nCohort 1 | 217 | nd | 199 | 217 | nd | 199 | 217 | nd | 199 |
| nCohort 2 | 16 | nd | 13 | 28 | nd | 29 | 17 | nd | 15 |
| Cutoff 1 | 7.90 | nd | 6.00 | 55.3 | nd | 55.3 | 33.9 | nd | 33.9 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 1 | 75% | nd | 77% | 71% | nd | 72% | 71% | nd | 80% |
| Spec 1 | 21% | nd | 18% | 46% | nd | 47% | 35% | nd | 36% |
| Cutoff 2 | 6.00 | nd | 0.608 | 47.0 | nd | 31.8 | 20.2 | nd | 33.9 |
| Sens 2 | 81% | nd | 85% | 82% | nd | 83% | 82% | nd | 80% |
| Spec 2 | 17% | nd | 12% | 44% | nd | 32% | 27% | nd | 36% |
| Cutoff 3 | 0.442 | nd | 0.442 | 15.1 | nd | 7.90 | 0.608 | nd | 6.07 |
| Sens 3 | 94% | nd | 92% | 93% | nd | 93% | 94% | nd | 93% |
| Spec 3 | 8% | nd | 8% | 23% | nd | 22% | 12% | nd | 21% |
| Cutoff 4 | 118 | nd | 113 | 118 | nd | 113 | 118 | nd | 113 |
| Sens 4 | 19% | nd | 15% | 25% | nd | 21% | 29% | nd | 27% |
| Spec 4 | 70% | nd | 70% | 70% | nd | 70% | 70% | nd | 70% |
| Cutoff 5 | 163 | nd | 158 | 163 | nd | 158 | 163 | nd | 158 |
| Sens 5 | 19% | nd | 8% | 14% | nd | 10% | 29% | nd | 27% |
| Spec 5 | 80% | nd | 82% | 80% | nd | 82% | 80% | nd | 82% |
| Cutoff 6 | 246 | nd | 209 | 246 | nd | 209 | 246 | nd | 209 |
| Sens 6 | 12% | nd | 0% | 4% | nd | 0% | 6% | nd | 13% |
| Spec 6 | 90% | nd | 90% | 90% | nd | 90% | 90% | nd | 90% |
| OR Quart 2 | 0.67 | nd | 2.0 | 2.1 | nd | 1.3 | 2.5 | nd | 3.2 |
| p Value | 0.66 | nd | 0.57 | 0.31 | nd | 0.73 | 0.21 | nd | 0.17 |
| 95% CI of OR Quart2 | 0.11 | nd | 0.18 | 0.50 | nd | 0.32 | 0.61 | nd | 0.61 |
| | 4.1 | nd | 23 | 8.9 | nd | 5.0 | 10 | nd | 17 |
| OR Quart 3 | 1.8 | nd | 6.6 | 5.2 | nd | 4.7 | 0.65 | nd | 1.5 |
| p Value | 0.45 | nd | 0.085 | 0.013 | nd | 0.0095 | 0.65 | nd | 0.65 |
| 95% CI of OR Quart3 | 0.40 | nd | 0.77 | 1.4 | nd | 1.5 | 0.11 | nd | 0.25 |
| | 7.7 | nd | 57 | 19 | nd | 15 | 4.1 | nd | 9.5 |
| OR Quart 4 | 2.2 | nd | 4.2 | 2.1 | nd | 1.3 | 1.7 | nd | 2.0 |
| p Value | 0.30 | nd | 0.20 | 0.32 | nd | 0.73 | 0.48 | nd | 0.42 |
| 95% CI of OR Quart4 | 0.51 | nd | 0.46 | 0.49 | nd | 0.32 | 0.39 | nd | 0.36 |
| | 9.1 | nd | 39 | 8.7 | nd | 5.0 | 7.5 | nd | 12 |

(continued)

| Interleukin-2 receptor alpha chain | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 18.3 | 18.7 | 18.3 | 25.1 | 18.3 | 22.6 |
| Average | 59.5 | 84.0 | 59.5 | 65.2 | 59.5 | 121 |
| Stdev | 126 | 139 | 126 | 140 | 126 | 224 |
| p(t-test) | | 0.46 | | 0.82 | | 0.072 |
| Min | 0.0290 | 0.0290 | 0.0290 | 0.0290 | 0.0290 | 0.0461 |
| Max | 1040 | 518 | 1040 | 715 | 1040 | 862 |
| n (Samp) | 217 | 16 | 217 | 28 | 217 | 17 |
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| n (Patient) | 133 | 16 | 133 | 28 | 133 | 17 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 24.1 | 23.9 | 24.1 | 28.8 | 24.1 | 30.0 |
| Average | 67.8 | 95.2 | 67.8 | 84.5 | 67.8 | 135 |
| Stdev | 136 | 152 | 136 | 162 | 136 | 236 |
| p(t-test) | | 0.49 | | 0.55 | | 0.084 |
| Min | 0.0290 | 0.0290 | 0.0290 | 0.0290 | 0.0290 | 0.0461 |
| Max | 1040 | 518 | 1040 | 715 | 1040 | 862 |
| n (Samp) | 199 | 13 | 199 | 29 | 199 | 15 |
| n (Patient) | 118 | 13 | 118 | 29 | 118 | 15 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.57 | nd | 0.55 | 0.50 | nd | 0.52 | 0.56 | nd | 0.56 |
| SE | 0.077 | nd | 0.085 | 0.058 | nd | 0.058 | 0.075 | nd | 0.079 |
| p | 0.38 | nd | 0.52 | 0.99 | nd | 0.78 | 0.43 | nd | 0.47 |
| nCohort 1 | 217 | nd | 199 | 217 | nd | 199 | 217 | nd | 199 |
| nCohort 2 | 16 | nd | 13 | 28 | nd | 29 | 17 | nd | 15 |
| Cutoff 1 | 10.6 | nd | 11.0 | 0.0515 | nd | 6.51 | 3.62 | nd | 3.62 |
| Sens 1 | 75% | nd | 77% | 75% | nd | 72% | 71% | nd | 73% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 1 | 42% | nd | 38% | 17% | nd | 34% | 32% | nd | 29% |
| Cutoff 2 | 6.86 | nd | 10.6 | 0.0429 | nd | 0.0359 | 0.0569 | nd | 0.0612 |
| Sens 2 | 81% | nd | 85% | 86% | nd | 86% | 88% | nd | 80% |
| Spec 2 | 39% | nd | 38% | 9% | nd | 9% | 23% | nd | 24% |
| Cutoff 3 | 1.56 | nd | 1.56 | 0.0290 | nd | 0.0290 | 0.0515 | nd | 0.0569 |
| Sens 3 | 94% | nd | 92% | 96% | nd | 97% | 94% | nd | 93% |
| Spec 3 | 30% | nd | 26% | 5% | nd | 6% | 17% | nd | 20% |
| Cutoff 4 | 48.3 | nd | 54.6 | 48.3 | nd | 54.6 | 48.3 | nd | 54.6 |
| Sens 4 | 31% | nd | 31% | 32% | nd | 38% | 35% | nd | 40% |
| Spec 4 | 70% | nd | 70% | 70% | nd | 70% | 70% | nd | 70% |
| Cutoff 5 | 88.9 | nd | 102 | 88.9 | nd | 102 | 88.9 | nd | 102 |
| Sens 5 | 31% | nd | 31% | 11% | nd | 14% | 29% | nd | 27% |
| Spec 5 | 80% | nd | 80% | 80% | nd | 80% | 80% | nd | 80% |
| Cutoff 6 | 148 | nd | 153 | 148 | nd | 153 | 148 | nd | 153 |
| Sens 6 | 19% | nd | 23% | 11% | nd | 14% | 24% | nd | 27% |
| Spec 6 | 90% | nd | 90% | 90% | nd | 90% | 90% | nd | 90% |
| OR Quart 2 | 7.8 | nd | 6.6 | 0.20 | nd | 0.59 | 0.98 | nd | 0.72 |
| p Value | 0.058 | nd | 0.085 | 0.043 | nd | 0.38 | 0.98 | nd | 0.68 |
| 95% CI of OR Quart2 | 0.93 | nd | 0.77 | 0.040 | nd | 0.18 | 0.23 | nd | 0.15 |
| | 66 | nd | 57 | 0.95 | nd | 1.9 | 4.1 | nd | 3.4 |
| OR Quart 3 | 3.1 | nd | 2.0 | 0.87 | nd | 1.1 | 1.0 | nd | 0.74 |
| p Value | 0.33 | nd | 0.57 | 0.79 | nd | 0.79 | 1.0 | nd | 0.70 |
| 95% CI of OR Quart3 | 0.31 | nd | 0.18 | 0.31 | nd | 0.41 | 0.24 | nd | 0.16 |
| | 31 | nd | 23 | 2.4 | nd | 3.2 | 4.2 | nd | 3.5 |
| OR Quart 4 | 5.3 | nd | 4.2 | 0.98 | nd | 0.86 | 1.2 | nd | 1.2 |
| p Value | 0.13 | nd | 0.20 | 0.97 | nd | 0.78 | 0.75 | nd | 0.75 |
| 95% CI of OR Quart4 | 0.60 | nd | 0.46 | 0.36 | nd | 0.29 | 0.32 | nd | 0.32 |
| | 47 | nd | 39 | 2.7 | nd | 2.5 | 4.9 | nd | 4.9 |

(continued)

| Neutrophil collagenase | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 1090 | 1320 | 1090 | 1800 | 1090 | 1160 |
| Average | 2990 | 1400 | 2990 | 5630 | 2990 | 4630 |
| Stdev | 6890 | 1170 | 6890 | 9430 | 6890 | 7310 |
| p(t-test) | | 0.36 | | 0.064 | | 0.31 |
| Min | 57.2 | 21.8 | 57.2 | 1.14 | 57.2 | 131 |
| Max | 55000 | 3920 | 55000 | 38700 | 55000 | 28000 |
| n (Samp) | 281 | 16 | 281 | 28 | 281 | 20 |
| n (Patient) | 160 | 16 | 160 | 28 | 160 | 20 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | nd | nd | nd | nd | 1130 | 885 |
| Average | nd | nd | nd | nd | 3250 | 9310 |
| Stdev | nd | nd | nd | nd | 6960 | 15400 |
| p(t-test) | nd | nd | nd | nd | | 0.040 |
| Min | nd | nd | nd | nd | 57.2 | 264 |
| Max | nd | nd | nd | nd | 55000 | 38700 |
| n (Samp) | nd | nd | nd | nd | 352 | 6 |
| n (Patient) | nd | nd | nd | nd | 193 | 6 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 1100 | 1420 | 1100 | 2360 | 1100 | 1160 |
| Average | 3390 | 1590 | 3390 | 5940 | 3390 | 4490 |
| Stdev | 7790 | 1190 | 7790 | 9700 | 7790 | 7460 |
| p(t-test) | | 0.41 | | 0.12 | | 0.57 |
| Min | 40.0 | 196 | 40.0 | 21.8 | 40.0 | 1.14 |
| Max | 55000 | 3920 | 55000 | 38700 | 55000 | 28000 |
| n (Samp) | 257 | 13 | 257 | 26 | 257 | 17 |
| n (Patient) | 140 | 13 | 140 | 26 | 140 | 17 |
| | | | | | | |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.47 | nd | 0.52 | 0.61 | nd | 0.62 | 0.55 | 0.51 | 0.54 |
| SE | 0.075 | nd | 0.083 | 0.059 | nd | 0.061 | 0.068 | 0.12 | 0.074 |
| p | 0.72 | nd | 0.83 | 0.065 | nd | 0.045 | 0.44 | 0.96 | 0.57 |
| nCohort 1 | 281 | nd | 257 | 281 | nd | 257 | 281 | 352 | 257 |
| nCohort 2 | 16 | nd | 13 | 28 | nd | 26 | 20 | 6 | 17 |
| Cutoff 1 | 520 | nd | 669 | 799 | nd | 906 | 792 | 618 | 792 |
| Sens 1 | 75% | nd | 77% | 71% | nd | 73% | 70% | 83% | 71% |
| Spec 1 | 21% | nd | 30% | 38% | nd | 42% | 37% | 26% | 37% |
| Cutoff 2 | 318 | nd | 520 | 554 | nd | 743 | 724 | 618 | 724 |
| Sens 2 | 81% | nd | 85% | 82% | nd | 81% | 80% | 83% | 82% |
| Spec 2 | 8% | nd | 21% | 23% | nd | 35% | 35% | 26% | 34% |
| Cutoff 3 | 193 | nd | 200 | 414 | nd | 414 | 451 | 261 | 131 |
| Sens 3 | 94% | nd | 92% | 93% | nd | 92% | 90% | 100% | 94% |
| Spec 3 | 2% | nd | 3% | 15% | nd | 14% | 17% | 5% | 2% |
| Cutoff 4 | 1700 | nd | 1830 | 1700 | nd | 1830 | 1700 | 1880 | 1830 |
| Sens 4 | 31% | nd | 31% | 50% | nd | 54% | 35% | 33% | 35% |
| Spec 4 | 70% | nd | 70% | 70% | nd | 70% | 70% | 70% | 70% |
| Cutoff 5 | 2830 | nd | 3110 | 2830 | nd | 3110 | 2830 | 3180 | 3110 |
| Sens 5 | 12% | nd | 15% | 39% | nd | 38% | 25% | 33% | 24% |
| Spec 5 | 80% | nd | 80% | 80% | nd | 80% | 80% | 80% | 80% |
| Cutoff 6 | 4950 | nd | 5930 | 4950 | nd | 5930 | 4950 | 7160 | 5930 |
| Sens 6 | 0% | nd | 0% | 29% | nd | 31% | 25% | 33% | 24% |
| Spec 6 | 90% | nd | 90% | 90% | nd | 90% | 90% | 90% | 90% |
| OR Quart 2 | 1.3 | nd | 0.65 | 0.82 | nd | 0.78 | 2.1 | 3.0 | 1.3 |
| p Value | 0.72 | nd | 0.64 | 0.75 | nd | 0.71 | 0.31 | 0.34 | 0.71 |
| 95% CI of OR Quart2 | 0.33 | nd | 0.10 | 0.24 | nd | 0.20 | 0.50 | 0.31 | 0.29 |
| | 5.0 | nd | 4.0 | 2.8 | nd | 3.0 | 8.7 | 30 | 6.2 |
| OR Quart 3 | 0.49 | nd | 1.7 | 0.65 | nd | 0.78 | 1.4 | 0 | 1.4 |
| p Value | 0.42 | nd | 0.47 | 0.52 | nd | 0.71 | 0.70 | na | 0.70 |
| 95% CI of OR Quart3 | 0.088 | nd | 0.39 | 0.18 | nd | 0.20 | 0.29 | na | 0.29 |
| | 2.8 | nd | 7.5 | 2.4 | nd | 3.0 | 6.3 | na | 6.3 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart 4 | 1.3 | nd | 0.98 | 2.4 | nd | 2.9 | 2.4 | 2.0 | 2.1 |
| p Value | 0.72 | nd | 0.99 | 0.099 | nd | 0.055 | 0.21 | 0.57 | 0.32 |
| 95% CI of | 0.33 | nd | 0.19 | 0.85 | nd | 0.98 | 0.61 | 0.18 | 0.49 |
| OR Quart4 | 5.0 | nd | 5.1 | 6.6 | nd | 8.7 | 9.8 | 22 | 8.6 |

**Protransforming growth factor alpha**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.45 | 1.67 | 1.45 | 2.22 | 1.45 | 2.57 |
| Average | 5.56 | 16.8 | 5.56 | 13.3 | 5.56 | 21.2 |
| Stdev | 26.1 | 35.1 | 26.1 | 32.4 | 26.1 | 41.5 |
| p(t-test) |  | 0.11 |  | 0.15 |  | 0.025 |
| Min | 0.00228 | 0.00228 | 0.00228 | 0.00228 | 0.00228 | 0.00228 |
| Max | 287 | 137 | 287 | 162 | 287 | 153 |
| n (Samp) | 217 | 16 | 217 | 28 | 217 | 17 |
| n (Patient) | 133 | 16 | 133 | 28 | 133 | 17 |

| UO only | 0hr prior to AKI stage | | | 24hr prior to AKI stage | | | 48hr prior to AKI stage | | |
|---|---|---|---|---|---|---|---|---|---|
|  | Cohort 1 | | Cohort 2 | Cohort 1 | | Cohort 2 | Cohort 1 | | Cohort 2 |
| Median | 1.26 | | 1.22 | 1.26 | | 1.87 | 1.26 | | 2.57 |
| Average | 5.63 | | 17.9 | 5.63 | | 12.3 | 5.63 | | 21.8 |
| Stdev | 27.2 | | 38.5 | 27.2 | | 31.7 | 27.2 | | 44.0 |
|  | Cohort | 1 | Cohort 2 | Cohort 1 | | Cohort 2 | Cohort 1 | | Cohort 2 |
| p(t-test) |  | | 0.13 |  | | 0.23 |  | | 0.036 |
| Min | 0.00228 | | 0.00228 | 0.00228 | | 0.00228 | 0.00228 | | 0.00228 |
| Max | 287 | | 137 | 287 | | 162 | 287 | | 153 |
| n (Samp) | 199 | | 13 | 199 | | 29 | 199 | | 15 |
| n (Patient) | 118 | | 13 | 118 | | 29 | 118 | | 15 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.60 | nd | 0.58 | 0.61 | nd | 0.62 | 0.66 | nd | 0.64 |
| SE | 0.077 | nd | 0.085 | 0.060 | nd | 0.059 | 0.074 | nd | 0.080 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p | 0.21 | nd | 0.32 | 0.075 | nd | 0.033 | 0.036 | nd | 0.084 |
| nCohort 1 | 217 | nd | 199 | 217 | nd | 199 | 217 | nd | 199 |
| nCohort 2 | 16 | nd | 13 | 28 | nd | 29 | 17 | nd | 15 |
| Cutoff 1 | 0.597 | nd | 0.597 | 0.885 | nd | 0.885 | 1.70 | nd | 0.666 |
| Sens 1 | 75% | nd | 77% | 71% | nd | 72% | 71% | nd | 73% |
| Spec 1 | 35% | nd | 38% | 41% | nd | 44% | 54% | nd | 39% |
| Cutoff 2 | 0.540 | nd | 0.537 | 0.704 | nd | 0.704 | 0.674 | nd | 0.190 |
| Sens 2 | 81% | nd | 85% | 82% | nd | 83% | 82% | nd | 80% |
| Spec 2 | 35% | nd | 36% | 37% | nd | 40% | 37% | nd | 28% |
| Cutoff 3 | 0.0223 | nd | 0.0223 | 0.190 | nd | 0.309 | 0.00228 | nd | 0.00228 |
| Sens 3 | 94% | nd | 92% | 93% | nd | 93% | 94% | nd | 93% |
| Spec 3 | 19% | nd | 21% | 25% | nd | 31% | 5% | nd | 5% |
| Cutoff 4 | 3.05 | nd | 2.70 | 3.05 | nd | 2.70 | 3.05 | nd | 2.70 |
| Sens 4 | 44% | nd | 38% | 39% | nd | 38% | 47% | nd | 47% |
| Spec 4 | 71% | nd | 70% | 71% | nd | 70% | 71% | nd | 70% |
| Cutoff 5 | 4.18 | nd | 4.00 | 4.18 | nd | 4.00 | 4.18 | nd | 4.00 |
| Sens 5 | 38% | nd | 31% | 32% | nd | 31% | 47% | nd | 47% |
| Spec 5 | 80% | nd | 80% | 80% | nd | 80% | 80% | nd | 80% |
| Cutoff 6 | 6.50 | nd | 6.43 | 6.50 | nd | 6.43 | 6.50 | nd | 6.43 |
| Sens 6 | 31% | nd | 31% | 25% | nd | 24% | 41% | nd | 40% |
| Spec 6 | 90% | nd | 90% | 90% | nd | 90% | 90% | nd | 90% |
| OR Quart 2 | 3.2 | nd | 2.7 | 3.8 | nd | 5.9 | 0.64 | nd | 1.5 |
| p Value | 0.16 | nd | 0.26 | 0.052 | nd | 0.027 | 0.64 | nd | 0.66 |
| 95% CI of | 0.62 | nd | 0.49 | 0.99 | nd | 1.2 | 0.10 | nd | 0.24 |
| OR Quart2 | 17 | nd | 14 | 15 | nd | 28 | 4.0 | nd | 9.4 |
| OR Quart 3 | 1.0 | nd | 1.0 | 1.7 | nd | 3.8 | 1.4 | nd | 1.5 |
| p Value | 1.0 | nd | 1.0 | 0.47 | nd | 0.10 | 0.70 | nd | 0.65 |
| 95% CI of | 0.14 | nd | 0.14 | 0.39 | nd | 0.76 | 0.29 | nd | 0.25 |
| OR Quart3 | 7.3 | nd | 7.4 | 7.6 | nd | 19 | 6.4 | nd | 9.5 |
| OR Quart 4 | 3.2 | nd | 2.1 | 3.7 | nd | 5.9 | 2.9 | nd | 3.8 |
| p Value | 0.17 | nd | 0.41 | 0.055 | nd | 0.027 | 0.13 | nd | 0.11 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of | 0.61 | nd | 0.36 | 0.97 | nd | 1.2 | 0.72 | nd | 0.75 |
| OR Quart4 | 16 | nd | 12 | 14 | nd | 28 | 11 | nd | 19 |

**CA 15-3**

| sCr or UO | 0hr prior to AKI stage | | | 24hr prior to AKI stage | | | 48hr prior to AKI stage | | |
|---|---|---|---|---|---|---|---|---|---|
| | Cohort | 1 | Cohort 2 | Cohort 1 | | Cohort 2 | Cohort 1 | | Cohort 2 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.253 | 0.375 | 0.253 | 0.552 | 0.253 | 0.502 |
| Average | 0.895 | 1.25 | 0.895 | 1.93 | 0.895 | 1.79 |
| Stdev | 4.27 | 3.04 | 4.27 | 4.80 | 4.27 | 4.30 |
| p(t-test) | | 0.75 | | 0.23 | | 0.37 |
| Min | 0.00165 | 0.00154 | 0.00165 | 0.0463 | 0.00165 | 0.0355 |
| Max | 60.0 | 12.5 | 60.0 | 24.0 | 60.0 | 17.8 |
| n (Samp) | 282 | 16 | 282 | 28 | 282 | 20 |
| n (Patient) | 160 | 16 | 160 | 28 | 160 | 20 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | nd | nd | 0.292 | 0.334 |
| Average | nd | nd | nd | nd | 1.05 | 0.350 |
| Stdev | nd | nd | nd | nd | 4.23 | 0.248 |
| p(t-test) | nd | nd | nd | nd | | 0.69 |
| Min | nd | nd | nd | nd | 0.00154 | 0.0355 |
| Max | nd | nd | nd | nd | 60.0 | 0.682 |
| n (Samp) | nd | nd | nd | nd | 353 | 6 |
| n (Patient) | nd | nd | nd | nd | 193 | 6 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.284 | 0.366 | 0.284 | 0.588 | 0.284 | 0.530 |
| Average | 0.965 | 1.40 | 0.965 | 2.08 | 0.965 | 2.07 |
| Stdev | 4.46 | 3.37 | 4.46 | 4.96 | 4.46 | 4.63 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.73 | | 0.23 | | 0.32 |
| Min | 0.00165 | 0.00154 | 0.00165 | 0.109 | 0.00165 | 0.141 |
| Max | 60.0 | 12.5 | 60.0 | 24.0 | 60.0 | 17.8 |
| n (Samp) | 258 | 13 | 258 | 26 | 258 | 17 |
| n (Patient) | 140 | 13 | 140 | 26 | 140 | 17 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.59 | nd | 0.58 | 0.66 | nd | 0.66 | 0.64 | 0.48 | 0.67 |
| SE | 0.077 | nd | 0.085 | 0.058 | nd | 0.061 | 0.069 | 0.12 | 0.074 |
| p | 0.25 | nd | 0.35 | 0.0064 | nd | 0.0078 | 0.035 | 0.87 | 0.019 |
| nCohort 1 | 282 | nd | 258 | 282 | nd | 258 | 282 | 353 | 258 |
| nCohort 2 | 16 | nd | 13 | 28 | nd | 26 | 20 | 6 | 17 |
| Cutoff 1 | 0.266 | nd | 0.261 | 0.249 | nd | 0.249 | 0.392 | 0.187 | 0.409 |
| Sens 1 | 75% | nd | 77% | 71% | nd | 73% | 70% | 83% | 71% |
| Spec 1 | 51% | nd | 47% | 50% | nd | 46% | 69% | 31% | 67% |
| Cutoff 2 | 0.191 | nd | 0.191 | 0.187 | nd | 0.191 | 0.176 | 0.187 | 0.176 |
| Sens 2 | 81% | nd | 85% | 82% | nd | 81% | 80% | 83% | 82% |
| Spec 2 | 34% | nd | 30% | 33% | nd | 30% | 32% | 31% | 28% |
| Cutoff 3 | 0.0177 | nd | 0.0177 | 0.133 | nd | 0.136 | 0.147 | 0.0338 | 0.147 |
| Sens 3 | 94% | nd | 92% | 93% | nd | 92% | 90% | 100% | 94% |
| Spec 3 | 2% | nd | 2% | 23% | nd | 21% | 25% | 3% | 22% |
| Cutoff 4 | 0.409 | nd | 0.456 | 0.409 | nd | 0.456 | 0.409 | 0.476 | 0.456 |
| Sens 4 | 38% | nd | 38% | 57% | nd | 58% | 65% | 50% | 59% |
| Spec 4 | 70% | nd | 70% | 70% | nd | 70% | 70% | 70% | 70% |
| Cutoff 5 | 0.614 | nd | 0.708 | 0.614 | nd | 0.708 | 0.614 | 0.701 | 0.708 |
| Sens 5 | 31% | nd | 23% | 46% | nd | 38% | 30% | 0% | 29% |
| Spec 5 | 80% | nd | 80% | 80% | nd | 80% | 80% | 80% | 80% |
| Cutoff 6 | 1.14 | nd | 1.20 | 1.14 | nd | 1.20 | 1.14 | 1.40 | 1.20 |
| Sens 6 | 19% | nd | 15% | 25% | nd | 31% | 15% | 0% | 18% |
| Spec 6 | 90% | nd | 90% | 90% | nd | 90% | 90% | 90% | 90% |
| OR Quart 2 | 0.32 | nd | 1.5 | 1.2 | nd | 1.7 | 0.99 | 2.0 | 0.99 |
| p Value | 0.33 | nd | 0.66 | 0.75 | nd | 0.47 | 0.99 | 0.57 | 0.99 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of | 0.033 | nd | 0.24 | 0.32 | nd | 0.39 | 0.19 | 0.18 | 0.13 |
| OR Quart2 | 3.1 | nd | 9.3 | 4.8 | nd | 7.5 | 5.0 | 23 | 7.2 |
| OR Quart3 | 2.1 | nd | 1.5 | 1.3 | nd | 1.7 | 1.4 | 2.0 | 2.6 |
| p Value | 0.31 | nd | 0.66 | 0.73 | nd | 0.47 | 0.70 | 0.57 | 0.27 |
| 95% CI of | 0.50 | nd | 0.24 | 0.33 | nd | 0.39 | 0.29 | 0.18 | 0.48 |
| OR Quart3 | 8.7 | nd | 9.3 | 4.9 | nd | 7.5 | 6.3 | 23 | 14 |
| OR Quart4 | 2.1 | nd | 2.6 | 4.0 | nd | 5.1 | 3.6 | 1.0 | 4.3 |
| p Value | 0.32 | nd | 0.27 | 0.019 | nd | 0.015 | 0.058 | 0.99 | 0.071 |
| 95% CI of | 0.49 | nd | 0.48 | 1.3 | nd | 1.4 | 0.96 | 0.062 | 0.88 |
| OR Quart4 | 8.6 | nd | 14 | 13 | nd | 19 | 14 | 16 | 21 |

Table 7: Comparison of marker levels in EDTA samples collected within 12 hours of reaching stage R from Cohort 1 (patients that reached, but did not progress beyond, RIFLE stage R) and from Cohort 2 (patients that reached RIFLE stage I or F).

| **C-C motif chemokine 8** | | | | | | |
|---|---|---|---|---|---|---|
| | sCr or UO | | sCr only | | UO only | |
| | Cohort 1 | Cohort 2 | Cohort 1 | Cohort 2 | Cohort 1 | Cohort 2 |
| Median | 13.7 | 13.8 | nd | nd | 13.8 | 13.8 |
| Average | 19.7 | 14.2 | nd | nd | 13.8 | 13.4 |
| Stdev | 31.0 | 7.93 | nd | nd | 10.1 | 8.09 |
| p(t-test) | | 0.45 | nd | nd | | 0.88 |
| Min | 0.162 | 1.20 | nd | nd | 0.170 | 1.20 |
| Max | 166 | 34.9 | nd | nd | 50.3 | 34.9 |
| n (Samp) | 41 | 19 | nd | nd | 31 | 15 |
| n (Patient) | 41 | 19 | nd | nd | 31 | 15 |
| | | | | | | |
| | At Enrollment | | | | | |
| | sCr or UO | | sCr only | | UO only | |
| AUC | 0.52 | | nd | | 0.50 | |
| SE | 0.081 | | nd | | 0.092 | |
| p | 0.77 | | nd | | 0.97 | |
| nCohort 1 | 41 | | nd | | 31 | |
| nCohort 2 | 19 | | nd | | 15 | |

(continued)

| | At Enrollment | | |
|---|---|---|---|
| | sCr or UO | sCr only | UO only |
| Cutoff 1 | 10.7 | nd | 10.6 |
| Sens 1 | 74% | nd | 73% |
| Spec 1 | 41% | nd | 42% |
| Cutoff 2 | 7.48 | nd | 8.22 |
| Sens 2 | 84% | nd | 80% |
| Spec 2 | 22% | nd | 32% |
| Cutoff 3 | 1.16 | nd | 1.16 |
| Sens 3 | 100% | nd | 100% |
| Spec 3 | 7% | nd | 6% |
| Cutoff 4 | 16.9 | nd | 16.9 |
| Sens 4 | 26% | nd | 13% |
| Spec 4 | 71% | nd | 71% |
| Cutoff 5 | 18.9 | nd | 18.9 |
| Sens 5 | 21% | nd | 13% |
| Spec 5 | 80% | nd | 81% |
| Cutoff 6 | 26.8 | nd | 25.2 |
| Sens 6 | 5% | nd | 7% |
| Spec 6 | 90% | nd | 90% |
| OR Quart 2 | 1.4 | nd | 6.0 |
| p Value | 0.69 | nd | 0.068 |
| 95% CI of | 0.29 | nd | 0.87 |
| OR Quart2 | 6.6 | nd | 41 |
| OR Quart 3 | 1.4 | nd | 2.5 |
| p Value | 0.69 | nd | 0.35 |
| 95% CI of | 0.29 | nd | 0.36 |
| OR Quart3 | 6.6 | nd | 17 |
| OR Quart 4 | 1.4 | nd | 1.9 |
| p Value | 0.69 | nd | 0.54 |
| 95% CI of | 0.29 | nd | 0.25 |
| OR Quart4 | 6.6 | nd | 14 |

| **Immunoglogulin G1** | | | | | | |
|---|---|---|---|---|---|---|
| | sCr or UO | | sCr only | | UO only | |
| | Cohort 1 | Cohort 2 | Cohort 1 | Cohort 2 | Cohort 1 | Cohort 2 |
| Median | 8820000 | 8470000 | nd | nd | 8840000 | 8470000 |
| Average | 9870000 | 1.04E7 | nd | nd | 9700000 | 8840000 |
| Stdev | 4230000 | 6820000 | nd | nd | 3800000 | 2660000 |
| p(t-test) | | 0.74 | nd | nd | | 0.58 |

(continued)

| Immunoglogulin G1 | | | | | | |
|---|---|---|---|---|---|---|
| | sCr or UO | | sCr only | | UO only | |
| | Cohort 1 | Cohort 2 | Cohort 1 | Cohort 2 | Cohort 1 | Cohort 2 |
| Min | 2060000 | 4620000 | nd | nd | 2060000 | 4620000 |
| Max | 2.33E7 | 2.72E7 | nd | nd | 1.75E7 | 1.20E7 |
| n (Samp) | 41 | 9 | nd | nd | 30 | 7 |
| n (Patient) | 41 | 9 | nd | nd | 30 | 7 |
| | | | | | | |
| | | At Enrollment | | | | |
| | | sCr or UO | sCr only | | UO only | |
| AUC | | 0.46 | nd | | 0.45 | |
| SE | | 0.11 | nd | | 0.12 | |
| p | | 0.73 | nd | | 0.66 | |
| nCohort 1 | 41 | | nd | | 30 | |
| nCohort 2 | 9 | | nd | | 7 | |
| Cutoff 1 | 7140000 | | nd | | 7600000 | |
| Sens 1 | 78% | | nd | | 71% | |
| Spec 1 | 29% | | nd | | 33% | |
| Cutoff 2 | 4620000 | | nd | | 6710000 | |
| Sens 2 | 89% | | nd | | 86% | |
| Spec 2 | 2% | | nd | | 27% | |
| Cutoff 3 | 2060000 | | nd | | 2060000 | |
| Sens 3 | 100% | | nd | | 100% | |
| Spec 3 | 2% | | nd | | 3% | |
| Cutoff 4 | 1.17E7 | | nd | | 1.15E7 | |
| Sens 4 | 33% | | nd | | 29% | |
| Spec 4 | 71% | | nd | | 70% | |
| Cutoff 5 | 1.34E7 | | nd | | 1.34E7 | |
| Sens 5 | 11% | | nd | | 0% | |
| Spec 5 | 80% | | nd | | 80% | |
| Cutoff 6 | 1.59E7 | | nd | | 1.39E7 | |
| Sens 6 | 11% | | nd | | 0% | |
| Spec 6 | 90% | | nd | | 90% | |
| OR Quart 2 | 4.0 | | nd | | 0.50 | |
| p Value | 0.26 | | nd | | 0.60 | |
| 95% CI of | 0.35 | | nd | | 0.037 | |
| OR Quart2 | 45 | | nd | | 6.7 | |
| OR Quart 3 | 3.6 | | nd | | 2.0 | |
| p Value | 0.30 | | nd | | 0.51 | |

(continued)

| | At Enrollment | | | |
|---|---|---|---|---|
| | sCr or UO | | sCr only | UO only |
| 95% CI of | 0.32 | | nd | 0.25 |
| OR Quart3 | 40 | | nd | 16 |
| OR Quart 4 | 2.4 | | nd | 0.50 |
| p Value | 0.50 | | nd | 0.60 |
| 95% CI of | 0.19 | | nd | 0.037 |
| OR Quart4 | 31 | | nd | 6.7 |

**Interleukin-11**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 91.3 | 87.4 | nd | nd | 91.9 | 76.6 |
| Average | 112 | 86.0 | nd | nd | 98.1 | 77.8 |
| Stdev | 130 | 49.3 | nd | nd | 86.1 | 52.9 |
| p(t-test) | | 0.40 | nd | nd | | 0.41 |
| Min | 0.359 | 0.480 | nd | nd | 0.359 | 0.480 |
| Max | 741 | 191 | nd | nd | 371 | 191 |
| n (Samp) | 41 | 19 | nd | nd | 31 | 15 |
| n (Patient) | 41 | 19 | nd | nd | 31 | 15 |

| | At Enrollment | | |
|---|---|---|---|
| | sCr or UO | sCr only | UO only |
| AUC | 0.47 | nd | 0.45 |
| SE | 0.081 | nd | 0.092 |
| p | 0.76 | nd | 0.61 |
| nCohort 1 | 41 | nd | 31 |
| nCohort 2 | 19 | nd | 15 |
| Cutoff 1 | 55.3 | nd | 47.0 |
| Sens 1 | 79% | nd | 73% |
| Spec 1 | 34% | nd | 35% |
| Cutoff 2 | 47.3 | nd | 10.7 |
| Sens 2 | 84% | nd | 80% |
| Spec 2 | 34% | nd | 26% |
| Cutoff 3 | 0.736 | nd | 0.736 |
| Sens 3 | 95% | nd | 93% |
| Spec 3 | 20% | nd | 26% |
| Cutoff 4 | 139 | nd | 146 |
| Sens 4 | 11% | nd | 7% |
| Spec 4 | 71% | nd | 71% |

(continued)

|  | At Enrollment | | |
|---|---|---|---|
|  | sCr or UO | sCr only | UO only |
| Cutoff 5 | 163 | nd | 163 |
| Sens 5 | 11% | nd | 7% |
| Spec 5 | 80% | nd | 81% |
| Cutoff 6 | 190 | nd | 184 |
| Sens 6 | 5% | nd | 7% |
| Spec 6 | 90% | nd | 90% |
| OR Quart 2 | 4.3 | nd | 13 |
| p Value | 0.11 | nd | 0.033 |
| 95% CI of | 0.71 | nd | 1.2 |
| OR Quart2 | 27 | nd | 140 |
| OR Quart 3 | 7.4 | nd | 7.9 |
| p Value | 0.029 | nd | 0.085 |
| 95% CI of | 1.2 | nd | 0.75 |
| OR Quart3 | 45 | nd | 82 |
| OR Quart 4 | 1.6 | nd | 4.1 |
| p Value | 0.63 | nd | 0.25 |
| 95% CI of | 0.23 | nd | 0.36 |
| OR Quart4 | 11 | nd | 47 |

Table 8: Comparison of the maximum marker levels in EDTA samples collected from Cohort 1 (patients that did not progress beyond RIFLE stage 0) and the maximum values in EDTA samples collected from subjects between enrollment and 0, 24 hours, and 48 hours prior to reaching stage F in Cohort 2.

| C-C motif chemokine 18 | | | | | | |
|---|---|---|---|---|---|---|
| sCr or UO | 0hr prior to AKI stage | | 24hr prior to AKI stage | | 48hr prior to AKI stage | |
|  | Cohort 1 | Cohort 2 | Cohort 1 | Cohort 2 | Cohort 1 | Cohort 2 |
| Median | 170 | 303 | 170 | 303 | nd | nd |
| Average | 230 | 472 | 230 | 463 | nd | nd |
| sCr or UO | 0hr prior to AKI stage | | 24hr prior to AKI stage | | 48hr prior to AKI stage | |
|  | Cohort 1 | Cohort 2 | Cohort 1 | Cohort 2 | Cohort 1 | Cohort 2 |
| Stdev | 178 | 512 | 178 | 518 | nd | nd |
| p(t-test) |  | 0.0041 |  | 0.0058 | nd | nd |
| Min | 38.6 | 103 | 38.6 | 103 | nd | nd |
| Max | 1020 | 1650 | 1020 | 1650 | nd | nd |
| n (Samp) | 87 | 8 | 87 | 8 | nd | nd |
| n (Patient) | 87 | 8 | 87 | 8 | nd | nd |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 173 | 380 | 173 | 380 | nd | nd |
| Average | 247 | 580 | 247 | 580 | nd | nd |
| Stdev | 181 | 557 | 181 | 557 | nd | nd |
| p(t-test) | | 6.5E-4 | | 6.5E-4 | nd | nd |
| Min | 38.6 | 118 | 38.6 | 118 | nd | nd |
| Max | 848 | 1650 | 848 | 1650 | nd | nd |
| n (Samp) | 80 | 6 | 80 | 6 | nd | nd |
| n (Patient) | 80 | 6 | 80 | 6 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.68 | nd | 0.75 | 0.65 | nd | 0.75 | nd | nd | nd |
| SE | 0.11 | nd | 0.12 | 0.11 | nd | 0.12 | nd | nd | nd |
| p | 0.10 | nd | 0.037 | 0.18 | nd | 0.037 | nd | nd | nd |
| nCohort 1 | 87 | nd | 80 | 87 | nd | 80 | nd | nd | nd |
| nCohort 2 | 8 | nd | 6 | 8 | nd | 6 | nd | nd | nd |
| Cutoff 1 | 186 | nd | 264 | 120 | nd | 264 | nd | nd | nd |
| Sens 1 | 75% | nd | 83% | 75% | nd | 83% | nd | nd | nd |
| Spec 1 | 56% | nd | 65% | 31% | nd | 65% | nd | nd | nd |
| Cutoff 2 | 118 | nd | 264 | 118 | nd | 264 | nd | nd | nd |
| Sens 2 | 88% | nd | 83% | 88% | nd | 83% | nd | nd | nd |
| Spec 2 | 30% | nd | 65% | 30% | nd | 65% | nd | nd | nd |
| Cutoff 3 | 103 | nd | 117 | 103 | nd | 117 | nd | nd | nd |
| Sens 3 | 100% | nd | 100% | 100% | nd | 100% | nd | nd | nd |
| Spec 3 | 21% | nd | 25% | 21% | nd | 25% | nd | nd | nd |
| Cutoff 4 | 246 | nd | 283 | 246 | nd | 283 | nd | nd | nd |
| Sens 4 | 62% | nd | 67% | 62% | nd | 67% | nd | nd | nd |
| Spec 4 | 70% | nd | 70% | 70% | nd | 70% | nd | nd | nd |
| Cutoff 5 | 309 | nd | 379 | 309 | nd | 379 | nd | nd | nd |
| Sens 5 | 50% | nd | 50% | 50% | nd | 50% | nd | nd | nd |
| Spec 5 | 80% | nd | 80% | 80% | nd | 80% | nd | nd | nd |
| Cutoff 6 | 484 | nd | 507 | 484 | nd | 507 | nd | nd | nd |
| Sens 6 | 25% | nd | 33% | 25% | nd | 33% | nd | nd | nd |
| Spec 6 | 91% | nd | 90% | 91% | nd | 90% | nd | nd | nd |
| OR Quart 2 | 0.96 | nd | 0 | 2.0 | nd | 0 | nd | nd | nd |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| p Value | 0.98 | nd | na | 0.58 | nd | na | nd | nd | nd |
| 95% CI of | 0.056 | nd | na | 0.17 | nd | na | nd | nd | nd |
| OR Quart2 | 16 | nd | na | 24 | nd | na | nd | nd | nd |
| OR Quart 3 | 2.0 | nd | 1.0 | 0.96 | nd | 1.0 | nd | nd | nd |
| p Value | 0.58 | nd | 1.0 | 0.98 | nd | 1.0 | nd | nd | nd |
| 95% CI of | 0.17 | nd | 0.058 | 0.056 | nd | 0.058 | nd | nd | nd |
| OR Quart3 | 24 | nd | 17 | 16 | nd | 17 | nd | nd | nd |
| OR Quart 4 | 4.4 | nd | 4.4 | 4.4 | nd | 4.4 | nd | nd | nd |
| p Value | 0.20 | nd | 0.20 | 0.20 | nd | 0.20 | nd | nd | nd |
| 95% CI of | 0.45 | nd | 0.45 | 0.45 | nd | 0.45 | nd | nd | nd |
| OR Quart4 | 43 | nd | 44 | 43 | nd | 44 | nd | nd | nd |

**C-C motif chemokine 24**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 222 | 259 | 222 | 259 | 222 | 155 |
| Average | 400 | 476 | 400 | 366 | 400 | 299 |
| Stdev | 493 | 643 | 493 | 299 | 493 | 235 |
| p(t-test) |  | 0.64 |  | 0.82 |  | 0.60 |
| Min | 0.0260 | 86.4 | 0.0260 | 86.4 | 0.0260 | 86.4 |
| Max | 2920 | 2430 | 2920 | 1100 | 2920 | 614 |
| n (Samp) | 64 | 12 | 64 | 12 | 64 | 7 |
| n (Patient) | 64 | 12 | 64 | 12 | 64 | 7 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 245 | 554 | 245 | 554 | nd | nd |
| Average | 386 | 755 | 386 | 534 | nd | nd |
| Stdev | 423 | 843 | 423 | 341 | nd | nd |
| p(t-test) |  | 0.049 |  | 0.40 | nd | nd |
| Min | 0.0260 | 95.2 | 0.0260 | 95.2 | nd | nd |
| Max | 2920 | 2430 | 2920 | 1100 | nd | nd |
| n (Samp) | 131 | 6 | 131 | 6 | nd | nd |

(continued)

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Patient) | 131 | 6 | 131 | 6 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 282 | 163 | 282 | 163 | 282 | 148 |
| Average | 467 | 237 | 467 | 237 | 467 | 249 |
| Stdev | 499 | 182 | 499 | 182 | 499 | 213 |
| p(t-test) | | 0.20 | | 0.20 | | 0.30 |
| Min | 24.7 | 86.4 | 24.7 | 86.4 | 24.7 | 86.4 |
| Max | 2920 | 614 | 2920 | 614 | 2920 | 614 |
| n (Samp) | 63 | 8 | 63 | 8 | 63 | 6 |
| n (Patient) | 63 | 8 | 63 | 8 | 63 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.54 | 0.68 | 0.35 | 0.54 | 0.67 | 0.35 | 0.48 | nd | 0.35 |
| SE | 0.093 | 0.12 | 0.11 | 0.093 | 0.12 | 0.11 | 0.12 | nd | 0.13 |
| p | 0.63 | 0.14 | 0.17 | 0.66 | 0.16 | 0.17 | 0.85 | nd | 0.23 |
| nCohort 1 | 64 | 131 | 63 | 64 | 131 | 63 | 64 | nd | 63 |
| nCohort 2 | 12 | 6 | 8 | 12 | 6 | 8 | 7 | nd | 6 |
| Cutoff 1 | 154 | 282 | 138 | 154 | 282 | 138 | 134 | nd | 89.8 |
| Sens 1 | 75% | 83% | 75% | 75% | 83% | 75% | 71% | nd | 83% |
| Spec 1 | 36% | 56% | 22% | 36% | 56% | 22% | 30% | nd | 13% |
| Cutoff 2 | 134 | 282 | 89.8 | 134 | 282 | 89.8 | 94.1 | nd | 89.8 |
| Sens 2 | 83% | 83% | 88% | 83% | 83% | 88% | 86% | nd | 83% |
| Spec 2 | 30% | 56% | 13% | 30% | 56% | 13% | 22% | nd | 13% |
| Cutoff 3 | 94.1 | 95.2 | 85.2 | 94.1 | 95.2 | 85.2 | 85.2 | nd | 85.2 |
| Sens 3 | 92% | 100% | 100% | 92% | 100% | 100% | 100% | nd | 100% |
| Spec 3 | 22% | 22% | 11% | 22% | 22% | 11% | 17% | nd | 11% |
| Cutoff 4 | 400 | 463 | 567 | 400 | 463 | 567 | 400 | nd | 567 |
| Sens 4 | 42% | 67% | 12% | 42% | 67% | 12% | 43% | nd | 17% |
| Spec 4 | 70% | 70% | 71% | 70% | 70% | 71% | 70% | nd | 71% |
| Cutoff 5 | 657 | 625 | 715 | 657 | 625 | 715 | 657 | nd | 715 |
| Sens 5 | 8% | 17% | 0% | 8% | 17% | 0% | 0% | nd | 0% |
| Spec 5 | 81% | 80% | 81% | 81% | 80% | 81% | 81% | nd | 81% |
| Cutoff 6 | 855 | 859 | 991 | 855 | 859 | 991 | 855 | nd | 991 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Sens 6 | 8% | 17% | 0% | 8% | 17% | 0% | 0% | nd | 0% |
| Spec 6 | 91% | 90% | 90% | 91% | 90% | 90% | 91% | nd | 90% |
| OR Quart 2 | 1.6 | 0 | 1.0 | 1.6 | 0 | 1.0 | 0.47 | nd | 1.1 |
| p Value | 0.63 | na | 1.0 | 0.63 | na | 1.0 | 0.55 | nd | 0.97 |
| 95% CI of | 0.23 | na | 0.058 | 0.23 | na | 0.058 | 0.039 | nd | 0.061 |
| OR Quart2 | 11 | na | 17 | 11 | na | 17 | 5.7 | nd | 18 |
| OR Quart 3 | 2.3 | 2.1 | 3.4 | 2.3 | 2.1 | 3.4 | 1.0 | nd | 1.1 |
| p Value | 0.38 | 0.56 | 0.31 | 0.38 | 0.56 | 0.31 | 1.0 | nd | 0.97 |
| 95% CI of | 0.36 | 0.18 | 0.32 | 0.36 | 0.18 | 0.32 | 0.13 | nd | 0.061 |
| OR Quart3 | 14 | 24 | 36 | 14 | 24 | 36 | 8.0 | nd | 18 |
| OR Quart 4 | 1.6 | 3.1 | 3.6 | 1.6 | 3.1 | 3.6 | 1.1 | nd | 3.6 |
| p Value | 0.63 | 0.34 | 0.29 | 0.63 | 0.34 | 0.29 | 0.95 | nd | 0.29 |
| 95% CI of | 0.23 | 0.31 | 0.34 | 0.23 | 0.31 | 0.34 | 0.13 | nd | 0.34 |
| OR Quart4 | 11 | 31 | 39 | 11 | 31 | 39 | 8.6 | nd | 39 |

**C-C motif chemokine 8**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 13.9 | 21.7 | 13.9 | 21.7 | 13.9 | 23.8 |
| Average | 20.1 | 42.4 | 20.1 | 42.3 | 20.1 | 37.2 |
| Stdev | 26.8 | 48.6 | 26.8 | 48.7 | 26.8 | 35.5 |
| p(t-test) |  | 0.025 |  | 0.026 |  | 0.13 |
| Min | 1.20 | 6.24 | 1.20 | 6.24 | 1.20 | 6.24 |
| Max | 148 | 169 | 148 | 169 | 148 | 101 |
| n (Samp) | 64 | 12 | 64 | 12 | 64 | 7 |
| n (Patient) | 64 | 12 | 64 | 12 | 64 | 7 |
| sCr only | 0hr prior to AKI stage | | 24hr prior to AKI stage | | 48hr prior to AKI stage | |
|  | Cohort 1 | Cohort 2 | Cohort 1 | Cohort 2 | Cohort 1 | Cohort 2 |
| Median | 15.1 | 21.8 | 15.1 | 21.1 | nd | nd |
| Average | 20.2 | 55.5 | 20.2 | 55.3 | nd | nd |
| Stdev | 25.1 | 65.4 | 25.1 | 65.6 | nd | nd |
| p(t-test) |  | 0.0027 |  | 0.0028 | nd | nd |
| Min | 1.20 | 6.24 | 1.20 | 6.24 | nd | nd |

(continued)

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 180 | 169 | 180 | 169 | nd | nd |
| n (Samp) | 131 | 6 | 131 | 6 | nd | nd |
| n (Patient) | 131 | 6 | 131 | 6 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 13.7 | 21.7 | 13.7 | 21.7 | 13.7 | 20.4 |
| Average | 21.2 | 26.3 | 21.2 | 26.3 | 21.2 | 26.5 |
| Stdev | 27.6 | 20.6 | 27.6 | 20.6 | 27.6 | 23.7 |
| p(t-test) | | 0.61 | | 0.61 | | 0.65 |
| Min | 1.20 | 6.24 | 1.20 | 6.24 | 1.20 | 6.24 |
| Max | 148 | 72.1 | 148 | 72.1 | 148 | 72.1 |
| n (Samp) | 63 | 8 | 63 | 8 | 63 | 6 |
| n (Patient) | 63 | 8 | 63 | 8 | 63 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.70 | 0.66 | 0.66 | 0.70 | 0.65 | 0.66 | 0.69 | nd | 0.63 |
| SE | 0.090 | 0.12 | 0.11 | 0.090 | 0.12 | 0.11 | 0.12 | nd | 0.13 |
| p | 0.025 | 0.21 | 0.15 | 0.030 | 0.24 | 0.15 | 0.097 | nd | 0.29 |
| nCohort 1 | 64 | 131 | 63 | 64 | 131 | 63 | 64 | nd | 63 |
| nCohort 2 | 12 | 6 | 8 | 12 | 6 | 8 | 7 | nd | 6 |
| Cutoff 1 | 14.2 | 13.4 | 14.2 | 13.7 | 13.4 | 14.2 | 16.9 | nd | 11.3 |
| Sens 1 | 75% | 83% | 75% | 75% | 83% | 75% | 71% | nd | 83% |
| Spec 1 | 52% | 44% | 54% | 50% | 44% | 54% | 64% | nd | 41% |
| Cutoff 2 | 13.0 | 13.4 | 11.3 | 13.0 | 13.4 | 11.3 | 11.3 | nd | 11.3 |
| Sens 2 | 83% | 83% | 88% | 83% | 83% | 88% | 86% | nd | 83% |
| Spec 2 | 48% | 44% | 41% | 48% | 44% | 41% | 36% | nd | 41% |
| Cutoff 3 | 11.3 | 5.84 | 4.81 | 11.3 | 5.84 | 4.81 | 4.81 | nd | 4.81 |
| Sens 3 | 92% | 100% | 100% | 92% | 100% | 100% | 100% | nd | 100% |
| Spec 3 | 36% | 13% | 14% | 36% | 13% | 14% | 16% | nd | 14% |
| Cutoff 4 | 18.9 | 18.9 | 20.1 | 18.9 | 18.9 | 20.1 | 18.9 | nd | 20.1 |
| Sens 4 | 58% | 50% | 50% | 58% | 50% | 50% | 57% | nd | 50% |
| Spec 4 | 70% | 70% | 73% | 70% | 70% | 73% | 70% | nd | 73% |
| Cutoff 5 | 22.4 | 22.1 | 24.3 | 22.4 | 22.1 | 24.3 | 22.4 | nd | 24.3 |
| Sens 5 | 50% | 50% | 38% | 50% | 50% | 38% | 57% | nd | 33% |
| Spec 5 | 81% | 80% | 81% | 81% | 80% | 81% | 81% | nd | 81% |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Cutoff 6 | 30.2 | 30.5 | 39.5 | 30.2 | 30.5 | 39.5 | 30.2 | nd | 39.5 |
| Sens 6 | 33% | 33% | 12% | 33% | 33% | 12% | 29% | nd | 17% |
| Spec 6 | 91% | 90% | 92% | 91% | 90% | 92% | 91% | nd | 92% |
| OR Quart 2 | 3.4 | 2.1 | 0.94 | 3.4 | 2.1 | 0.94 | 0.94 | nd | 1.0 |
| p Value | 0.31 | 0.56 | 0.97 | 0.31 | 0.56 | 0.97 | 0.97 | nd | 1.0 |
| 95% CI of OR Quart2 | 0.32 36 | 0.18 24 | 0.054 16 | 0.32 36 | 0.18 24 | 0.054 16 | 0.054 16 | nd nd | 0.057 17 |
| OR Quart 3 | 2.1 | 0 | 2.0 | 2.1 | 0 | 2.0 | 0.94 | nd | 1.0 |
| p Value | 0.55 | na | 0.59 | 0.55 | na | 0.59 | 0.97 | nd | 1.0 |
| 95% CI of OR Quart3 | 0.18 26 | na na | 0.16 24 | 0.18 26 | na na | 0.16 24 | 0.054 16 | nd nd | 0.057 17 |
| OR Quart 4 | 8.3 | 3.1 | 4.6 | 8.3 | 3.1 | 4.6 | 4.6 | nd | 3.2 |
| p Value | 0.063 | 0.34 | 0.20 | 0.063 | 0.34 | 0.20 | 0.20 | nd | 0.34 |
| 95% CI of OR Quart4 | 0.89 78 | 0.31 31 | 0.46 46 | 0.89 78 | 0.31 31 | 0.46 46 | 0.46 46 | nd nd | 0.30 34 |

**Cathepsin D**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 233000 | 479000 | 233000 | 479000 | nd | nd |
| Average | 256000 | 538000 | 256000 | 452000 | nd | nd |
| Stdev | 149000 | 348000 | 149000 | 144000 | nd | nd |
| p(t-test) |  | 2.6E-5 |  | 5.9E-4 | nd | nd |
| Min | 34100 | 266000 | 34100 | 266000 | nd | nd |
| Max | 1020000 | 1350000 | 1020000 | 655000 | nd | nd |
| n (Samp) | 86 | 8 | 86 | 8 | nd | nd |
| n (Patient) | 86 | 8 | 86 | 8 | nd | nd |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 247000 | 479000 | 247000 | 479000 | nd | nd |
| Average | 278000 | 442000 | 278000 | 442000 | nd | nd |
| Stdev | 153000 | 128000 | 153000 | 128000 | nd | nd |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.012 | | 0.012 | nd | nd |
| Min | 69400 | 266000 | 69400 | 266000 | nd | nd |
| Max | 1020000 | 561000 | 1020000 | 561000 | nd | nd |
| n (Samp) | 79 | 6 | 79 | 6 | nd | nd |
| n (Patient) | 79 | 6 | 79 | 6 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.86 | nd | 0.83 | 0.86 | nd | 0.83 | nd | nd | nd |
| SE | 0.084 | nd | 0.10 | 0.084 | nd | 0.10 | nd | nd | nd |
| p | 1.3E-5 | nd | 0.0016 | 1.8E-5 | nd | 0.0016 | nd | nd | nd |
| nCohort 1 | 86 | nd | 79 | 86 | nd | 79 | nd | nd | nd |
| nCohort 2 | 8 | nd | 6 | 8 | nd | 6 | nd | nd | nd |
| Cutoff 1 | 307000 | nd | 307000 | 307000 | nd | 307000 | nd | nd | nd |
| Sens 1 | 75% | nd | 83% | 75% | nd | 83% | nd | nd | nd |
| Spec 1 | 74% | nd | 67% | 74% | nd | 67% | nd | nd | nd |
| Cutoff 2 | 307000 | nd | 307000 | 307000 | nd | 307000 | nd | nd | nd |
| Sens 2 | 88% | nd | 83% | 88% | nd | 83% | nd | nd | nd |
| Spec 2 | 73% | nd | 67% | 73% | nd | 67% | nd | nd | nd |
| Cutoff 3 | 263000 | nd | 263000 | 263000 | nd | 263000 | nd | nd | nd |
| Sens 3 | 100% | nd | 100% | 100% | nd | 100% | nd | nd | nd |
| Spec 3 | 66% | nd | 58% | 66% | nd | 58% | nd | nd | nd |
| Cutoff 4 | 302000 | nd | 310000 | 302000 | nd | 310000 | nd | nd | nd |
| Sens 4 | 88% | nd | 67% | 88% | nd | 67% | nd | nd | nd |
| Spec 4 | 71% | nd | 71% | 71% | nd | 71% | nd | nd | nd |
| Cutoff 5 | 324000 | nd | 374000 | 324000 | nd | 374000 | nd | nd | nd |
| Sens 5 | 62% | nd | 67% | 62% | nd | 67% | nd | nd | nd |
| Spec 5 | 80% | nd | 81% | 80% | nd | 81% | nd | nd | nd |
| Cutoff 6 | 442000 | nd | 471000 | 442000 | nd | 471000 | nd | nd | nd |
| Sens 6 | 62% | nd | 50% | 62% | nd | 50% | nd | nd | nd |
| Spec 6 | 91% | nd | 91% | 91% | nd | 91% | nd | nd | nd |
| OR Quart 2 | >0 | nd | >0 | >0 | nd | >0 | nd | nd | nd |
| p Value | <na | nd | <na | <na | nd | <na | nd | nd | nd |
| 95% CI of | >na | nd | >na | >na | nd | >na | nd | nd | nd |
| OR Quart2 | na | nd | na | na | nd | na | nd | nd | nd |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart 3 | >3.4 | nd | >2.2 | >3.4 | nd | >2.2 | nd | nd | nd |
| p Value | <0.30 | nd | <0.53 | <0.30 | nd | <0.53 | nd | nd | nd |
| 95% CI of | >0.33 | nd | >0.19 | >0.33 | nd | >0.19 | nd | nd | nd |
| OR Quart3 | na | nd | na | na | nd | na | nd | nd | nd |
| OR Quart 4 | >6.1 | nd | >4.7 | >6.1 | nd | >4.7 | nd | nd | nd |
| p Value | <0.11 | nd | <0.19 | <0.11 | nd | <0.19 | nd | nd | nd |
| 95% CI of | >0.65 | nd | >0.48 | >0.65 | nd | >0.48 | nd | nd | nd |
| OR Quart4 | na | nd | na | na | nd | na | nd | nd | nd |

**C-X-C motif chemokine 13**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 47.3 | 83.0 | 47.3 | 83.0 | 47.3 | 82.8 |
| Average | 162 | 306 | 162 | 306 | 162 | 190 |
| Stdev | 351 | 564 | 351 | 564 | 351 | 234 |
| p(t-test) |  | 0.24 |  | 0.24 |  | 0.84 |
| Min | 9.90 | 15.1 | 9.90 | 15.1 | 9.90 | 45.0 |
| Max | 1790 | 2000 | 1790 | 2000 | 1790 | 685 |
| n (Samp) | 64 | 12 | 64 | 12 | 64 | 7 |
| n (Patient) | 64 | 12 | 64 | 12 | 64 | 7 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 53.6 | 83.0 | 53.6 | 83.0 | nd | nd |
| Average | 150 | 399 | 150 | 399 | nd | nd |
| Stdev | 294 | 785 | 294 | 785 | nd | nd |
| p(t-test) |  | 0.070 |  | 0.070 | nd | nd |
| Min | 9.90 | 15.1 | 9.90 | 15.1 | nd | nd |
| Max | 1790 | 2000 | 1790 | 2000 | nd | nd |
| n (Samp) | 131 | 6 | 131 | 6 | nd | nd |
| n (Patient) | 131 | 6 | 131 | 6 | nd | nd |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 51.9 | 77.2 | 51.9 | 77.2 | 51.9 | 77.2 |

(continued)

| UO only | 0hr prior to AKI stage | | 24hr prior to AKI stage | | 48hr prior to AKI stage | |
| --- | --- | --- | --- | --- | --- | --- |
| | Cohort 1 | Cohort 2 | Cohort 1 | Cohort 2 | Cohort 1 | Cohort 2 |
| Average | 149 | 180 | 149 | 180 | 149 | 208 |
| Stdev | 298 | 221 | 298 | 221 | 298 | 251 |
| p(t-test) | | 0.78 | | 0.78 | | 0.64 |
| Min | 9.90 | 35.6 | 9.90 | 35.6 | 9.90 | 45.0 |
| Max | 1790 | 685 | 1790 | 685 | 1790 | 685 |
| n (Samp) | 63 | 8 | 63 | 8 | 63 | 6 |
| n (Patient) | 63 | 8 | 63 | 8 | 63 | 6 |

| | 0hr prior to AKI stage | | | 24hr prior to AKI stage | | | 48hr prior to AKI stage | | |
| --- | --- | --- | --- | --- | --- | --- | --- | --- | --- |
| | sCr or UO | sCr only | UO only | sCr or UO | sCr only | UO only | sCr or UO | sCr only | UO only |
| AUC | 0.65 | 0.60 | 0.64 | 0.65 | 0.60 | 0.64 | 0.67 | nd | 0.66 |
| SE | 0.092 | 0.12 | 0.11 | 0.092 | 0.12 | 0.11 | 0.12 | nd | 0.13 |
| p | 0.11 | 0.45 | 0.22 | 0.11 | 0.45 | 0.22 | 0.15 | nd | 0.20 |
| nCohort 1 | 64 | 131 | 63 | 64 | 131 | 63 | 64 | nd | 63 |
| nCohort 2 | 12 | 6 | 8 | 12 | 6 | 8 | 7 | nd | 6 |
| Cutoff 1 | 66.4 | 70.0 | 66.4 | 66.4 | 70.0 | 66.4 | 68.9 | nd | 66.4 |
| Sens 1 | 75% | 83% | 75% | 75% | 83% | 75% | 71% | nd | 83% |
| Spec 1 | 58% | 56% | 54% | 58% | 56% | 54% | 58% | nd | 54% |
| Cutoff 2 | 44.3 | 70.0 | 44.3 | 44.3 | 70.0 | 44.3 | 66.4 | nd | 66.4 |
| Sens 2 | 83% | 83% | 88% | 83% | 83% | 88% | 86% | nd | 83% |
| Spec 2 | 45% | 56% | 41% | 45% | 56% | 41% | 58% | nd | 54% |
| Cutoff 3 | 35.5 | 15.0 | 35.5 | 35.5 | 15.0 | 35.5 | 44.3 | nd | 44.3 |
| Sens 3 | 92% | 100% | 100% | 92% | 100% | 100% | 100% | nd | 100% |
| Spec 3 | 39% | 5% | 32% | 39% | 5% | 32% | 45% | nd | 41% |
| Cutoff 4 | 106 | 114 | 111 | 106 | 114 | 111 | 106 | nd | 111 |
| Sens 4 | 42% | 33% | 38% | 42% | 33% | 38% | 29% | nd | 33% |
| Spec 4 | 70% | 70% | 71% | 70% | 70% | 71% | 70% | nd | 71% |
| Cutoff 5 | 170 | 155 | 140 | 170 | 155 | 140 | 170 | nd | 140 |
| Sens 5 | 25% | 17% | 38% | 25% | 17% | 38% | 29% | nd | 33% |
| Spec 5 | 81% | 80% | 81% | 81% | 80% | 81% | 81% | nd | 81% |
| Cutoff 6 | 254 | 260 | 254 | 254 | 260 | 254 | 254 | nd | 254 |
| Sens 6 | 25% | 17% | 25% | 25% | 17% | 25% | 29% | nd | 33% |
| Spec 6 | 91% | 90% | 90% | 91% | 90% | 90% | 91% | nd | 90% |
| OR Quart 2 | 2.1 | 0 | >2.1 | 2.1 | 0 | >2.1 | >1.0 | nd | >1.1 |
| p Value | 0.55 | na | <0.55 | 0.55 | na | <0.55 | <1.0 | nd | <0.97 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of | 0.18 | na | >0.18 | 0.18 | na | >0.18 | >0.058 | nd | >0.061 |
| OR Quart2 | 26 | na | na | 26 | na | na | na | nd | na |
| OR Quart 3 | 4.8 | 3.2 | >3.4 | 4.8 | 3.2 | >3.4 | >4.9 | nd | >3.6 |
| p Value | 0.18 | 0.33 | <0.31 | 0.18 | 0.33 | <0.31 | <0.18 | nd | <0.29 |
| 95% CI of | 0.48 | 0.32 | >0.32 | 0.48 | 0.32 | >0.32 | >0.49 | nd | >0.34 |
| OR Quart3 | 48 | 32 | na | 48 | 32 | na | na | nd | na |
| OR Quart 4 | 6.4 | 2.0 | >3.4 | 6.4 | 2.0 | >3.4 | >2.1 | nd | >2.1 |
| p Value | 0.11 | 0.58 | <0.31 | 0.11 | 0.58 | <0.31 | <0.55 | nd | <0.55 |
| 95% CI of | 0.67 | 0.17 | >0.32 | 0.67 | 0.17 | >0.32 | >0.18 | nd | >0.18 |
| OR Quart4 | 61 | 23 | na | 61 | 23 | na | na | nd | na |

**Insulin-like growth factor-binding protein 3**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3530 | 4230 | 3530 | 3620 | nd | nd |
| Average | 3640 | 4460 | 3640 | 4160 | nd | nd |
| Stdev | 1520 | 1990 | 1520 | 1840 | nd | nd |
| p(t-test) | | 0.16 | | 0.36 | nd | nd |
| Min | 823 | 2070 | 823 | 2070 | nd | nd |
| Max | 8260 | 7360 | 8260 | 7360 | nd | nd |
| n (Samp) | 87 | 8 | 87 | 8 | nd | nd |
| n (Patient) | 87 | 8 | 87 | 8 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3370 | 4230 | 3370 | 4230 | nd | nd |
| Average | 3670 | 4490 | 3670 | 4490 | nd | nd |
| Stdev | 1550 | 2000 | 1550 | 2000 | nd | nd |
| p(t-test) | | 0.22 | | 0.22 | nd | nd |
| Min | 823 | 2070 | 823 | 2070 | nd | nd |
| Max | 8260 | 7360 | 8260 | 7360 | nd | nd |
| n (Samp) | 80 | 6 | 80 | 6 | nd | nd |
| n (Patient) | 80 | 6 | 80 | 6 | nd | nd |

(continued)

| UO only | 0hr prior to AKI stage | | 24hr prior to AKI stage | | | 48hr prior to AKI stage | | |
|---|---|---|---|---|---|---|---|---|
| | Cohort 1 | Cohort 2 | Cohort 1 | | Cohort 2 | Cohort 1 | | Cohort 2 |
| | | | | | | | | |
| | 0hr prior to AKI stage | | 24hr prior to AKI stage | | | 48hr prior to AKI stage | | |
| | sCr or UO | sCr only | UO only | sCr or UO | sCr only | UO only | sCr or UO | sCr only | UO only |
| AUC | 0.63 | nd | 0.64 | 0.59 | nd | 0.64 | nd | nd | nd |
| SE | 0.11 | nd | 0.13 | 0.11 | nd | 0.13 | nd | nd | nd |
| p | 0.23 | nd | 0.25 | 0.43 | nd | 0.25 | nd | nd | nd |
| nCohort 1 | 87 | nd | 80 | 87 | nd | 80 | nd | nd | nd |
| nCohort 2 | 8 | nd | 6 | 8 | nd | 6 | nd | nd | nd |
| Cutoff 1 | 3050 | nd | 3090 | 3050 | nd | 3090 | nd | nd | nd |
| Sens 1 | 75% | nd | 83% | 75% | nd | 83% | nd | nd | nd |
| Spec 1 | 46% | nd | 48% | 46% | nd | 48% | nd | nd | nd |
| Cutoff 2 | 2400 | nd | 3090 | 2400 | nd | 3090 | nd | nd | nd |
| Sens 2 | 88% | nd | 83% | 88% | nd | 83% | nd | nd | nd |
| Spec 2 | 22% | nd | 48% | 22% | nd | 48% | nd | nd | nd |
| Cutoff 3 | 2040 | nd | 2040 | 2040 | nd | 2040 | nd | nd | nd |
| Sens 3 | 100% | nd | 100% | 100% | nd | 100% | nd | nd | nd |
| Spec 3 | 16% | nd | 15% | 16% | nd | 15% | nd | nd | nd |
| Cutoff 4 | 4330 | nd | 4220 | 4330 | nd | 4220 | nd | nd | nd |
| Sens 4 | 50% | nd | 50% | 38% | nd | 50% | nd | nd | nd |
| Spec 4 | 70% | nd | 70% | 70% | nd | 70% | nd | nd | nd |
| Cutoff 5 | 4990 | nd | 4990 | 4990 | nd | 4990 | nd | nd | nd |
| Sens 5 | 50% | nd | 50% | 38% | nd | 50% | nd | nd | nd |
| Spec 5 | 80% | nd | 80% | 80% | nd | 80% | nd | nd | nd |
| Cutoff 6 | 5650 | nd | 5590 | 5650 | nd | 5590 | nd | nd | nd |
| Sens 6 | 38% | nd | 33% | 25% | nd | 33% | nd | nd | nd |
| Spec 6 | 91% | nd | 90% | 91% | nd | 90% | nd | nd | nd |
| OR Quart 2 | 0.95 | nd | 2.0 | 0.95 | nd | 2.0 | nd | nd | nd |
| p Value | 0.96 | nd | 0.58 | 0.96 | nd | 0.58 | nd | nd | nd |
| 95% CI of | 0.12 | nd | 0.17 | 0.12 | nd | 0.17 | nd | nd | nd |
| OR Quart2 | 7.4 | nd | 24 | 7.4 | nd | 24 | nd | nd | nd |
| OR Quart 3 | 0 | nd | 0 | 0.46 | nd | 0 | nd | nd | nd |
| p Value | na | nd | na | 0.53 | nd | na | nd | nd | nd |
| 95% CI of | na | nd | na | 0.039 | nd | na | nd | nd | nd |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart3 | na | nd | na | 5.4 | nd | na | nd | nd | nd |
| OR Quart 4 | 2.1 | nd | 3.2 | 1.5 | nd | 3.2 | nd | nd | nd |
| p Value | 0.42 | nd | 0.34 | 0.67 | nd | 0.34 | nd | nd | nd |
| 95% CI of OR Quart4 | 0.35 | nd | 0.30 | 0.23 | nd | 0.30 | nd | nd | nd |
|  | 13 | nd | 33 | 9.9 | nd | 33 | nd | nd | nd |

**Interleukin-11**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 98.9 | 101 | 98.9 | 101 | 98.9 | 90.7 |
| Average | 333 | 576 | 333 | 576 | 333 | 129 |
| Stdev | 1130 | 1530 | 1130 | 1530 | 1130 | 76.7 |
| p(t-test) |  | 0.52 |  | 0.52 |  | 0.64 |
| Min | 0.368 | 31.8 | 0.368 | 31.8 | 0.368 | 38.4 |
| Max | 6920 | 5420 | 6920 | 5420 | 6920 | 233 |
| n (Samp) | 65 | 12 | 65 | 12 | 65 | 7 |
| n (Patient) | 65 | 12 | 65 | 12 | 65 | 7 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 91.9 | 83.6 | 91.9 | 83.6 | nd | nd |
| Average | 218 | 143 | 218 | 143 | nd | nd |
| Stdev | 804 | 136 | 804 | 136 | nd | nd |
| p(t-test) |  | 0.82 |  | 0.82 | nd | nd |
| Min | 0.359 | 31.8 | 0.359 | 31.8 | nd | nd |
| Max | 6920 | 389 | 6920 | 389 | nd | nd |
| n (Samp) | 132 | 6 | 132 | 6 | nd | nd |
| n (Patient) | 132 | 6 | 132 | 6 | nd | nd |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 90.7 | 101 | 90.7 | 101 | 90.7 | 83.7 |
| Average | 319 | 778 | 319 | 778 | 319 | 114 |
| Stdev | 1140 | 1880 | 1140 | 1880 | 1140 | 72.9 |
| p(t-test) |  | 0.33 |  | 0.33 |  | 0.67 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Min | 0.368 | 51.1 | 0.368 | 51.1 | 0.368 | 38.4 |
| Max | 6920 | 5420 | 6920 | 5420 | 6920 | 233 |
| n (Samp) | 64 | 8 | 64 | 8 | 64 | 6 |
| n (Patient) | 64 | 8 | 64 | 8 | 64 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.59 | 0.56 | 0.63 | 0.59 | 0.56 | 0.63 | 0.57 | nd | 0.57 |
| SE | 0.093 | 0.12 | 0.11 | 0.093 | 0.12 | 0.11 | 0.12 | nd | 0.13 |
| p | 0.31 | 0.63 | 0.24 | 0.31 | 0.63 | 0.24 | 0.55 | nd | 0.59 |
| nCohort 1 | 65 | 132 | 64 | 65 | 132 | 64 | 65 | nd | 64 |
| nCohort 2 | 12 | 6 | 8 | 12 | 6 | 8 | 7 | nd | 6 |
| Cutoff 1 | 67.2 | 51.1 | 76.5 | 67.2 | 51.1 | 76.5 | 76.5 | nd | 71.6 |
| Sens 1 | 75% | 83% | 75% | 75% | 83% | 75% | 71% | nd | 83% |
| Spec 1 | 43% | 39% | 47% | 43% | 39% | 47% | 43% | nd | 47% |
| Cutoff 2 | 51.1 | 51.1 | 71.6 | 51.1 | 51.1 | 71.6 | 67.2 | nd | 71.6 |
| Sens 2 | 83% | 83% | 88% | 83% | 83% | 88% | 86% | nd | 83% |
| Spec 2 | 38% | 39% | 47% | 38% | 39% | 47% | 43% | nd | 47% |
| Cutoff 3 | 47.4 | 29.7 | 47.4 | 47.4 | 29.7 | 47.4 | 33.9 | nd | 33.9 |
| Sens 3 | 92% | 100% | 100% | 92% | 100% | 100% | 100% | nd | 100% |
| Spec 3 | 38% | 23% | 41% | 38% | 23% | 41% | 32% | nd | 33% |
| Cutoff 4 | 159 | 158 | 155 | 159 | 158 | 155 | 159 | nd | 155 |
| Sens 4 | 42% | 33% | 38% | 42% | 33% | 38% | 43% | nd | 33% |
| Spec 4 | 71% | 73% | 70% | 71% | 73% | 70% | 71% | nd | 70% |
| Cutoff 5 | 209 | 196 | 190 | 209 | 196 | 190 | 209 | nd | 190 |
| Sens 5 | 33% | 33% | 25% | 33% | 33% | 25% | 29% | nd | 17% |
| Spec 5 | 80% | 81% | 81% | 80% | 81% | 81% | 80% | nd | 81% |
| Cutoff 6 | 370 | 261 | 306 | 370 | 261 | 306 | 370 | nd | 306 |
| Sens 6 | 17% | 17% | 12% | 17% | 17% | 12% | 0% | nd | 0% |
| Spec 6 | 91% | 90% | 91% | 91% | 90% | 91% | 91% | nd | 91% |
| OR Quart 2 | >8.8 | >4.4 | >5.1 | >8.8 | >4.4 | >5.1 | >5.1 | nd | >3.4 |
| p Value | <0.057 | <0.20 | <0.16 | <0.057 | <0.20 | <0.16 | <0.16 | nd | <0.31 |
| 95% CI of | >0.94 | >0.46 | >0.52 | >0.94 | >0.46 | >0.52 | >0.52 | nd | >0.32 |
| OR Quart2 | na | na | na | na | na | na | na | nd | na |
| OR Quart 3 | >2.2 | >0 | >2.2 | >2.2 | >0 | >2.2 | >1.1 | nd | >1.1 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value | <0.53 | <na | <0.52 | <0.53 | <na | <0.52 | <0.97 | nd | <0.97 |
| 95% CI of | >0.19 | >na | >0.19 | >0.19 | >na | >0.19 | >0.061 | nd | >0.061 |
| OR Quart3 | na | na | na | na | na | na | na | nd | na |
| OR Quart 4 | >4.8 | >2.1 | >2.2 | >4.8 | >2.1 | >2.2 | >2.2 | nd | >2.1 |
| p Value | <0.18 | <0.56 | <0.52 | <0.18 | <0.56 | <0.52 | <0.52 | nd | <0.55 |
| 95% CI of | >0.48 | >0.18 | >0.19 | >0.48 | >0.18 | >0.19 | >0.19 | nd | >0.18 |
| OR Quart4 | na | na | na | na | na | na | na | nd | na |

**Interleukin-2 receptor alpha chain**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 22.9 | 93.0 | 22.9 | 85.8 | 22.9 | 89.7 |
| Average | 45.2 | 158 | 45.2 | 152 | 45.2 | 121 |
| Stdev | 56.9 | 163 | 56.9 | 166 | 56.9 | 114 |
| p(t-test) | | 3.7E-5 | | 9.1E-5 | | 0.0041 |
| Min | 0.0359 | 5.03 | 0.0359 | 5.03 | 0.0359 | 12.5 |
| Max | 232 | 533 | 232 | 533 | 232 | 352 |
| n (Samp) | 65 | 12 | 65 | 12 | 65 | 7 |
| n (Patient) | 65 | 12 | 65 | 12 | 65 | 7 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 28.1 | 72.0 | 28.1 | 47.9 | nd | nd |
| Average | 80.9 | 66.1 | 80.9 | 55.7 | nd | nd |
| Stdev | 146 | 51.1 | 146 | 50.1 | nd | nd |
| p(t-test) | | 0.81 | | 0.67 | nd | nd |
| Min | 0.0359 | 5.03 | 0.0359 | 5.03 | nd | nd |
| Max | 1040 | 139 | 1040 | 139 | nd | nd |
| n (Samp) | 132 | 6 | 132 | 6 | nd | nd |
| n (Patient) | 132 | 6 | 132 | 6 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 38.4 | 122 | 38.4 | 122 | 38.4 | 75.9 |
| Average | 55.1 | 196 | 55.1 | 196 | 55.1 | 117 |
| Stdev | 59.7 | 188 | 59.7 | 188 | 59.7 | 125 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 1.9E-5 | | 1.9E-5 | | 0.032 |
| Min | 0.0359 | 12.5 | 0.0359 | 12.5 | 0.0359 | 12.5 |
| Max | 232 | 533 | 232 | 533 | 232 | 352 |
| n (Samp) | 64 | 8 | 64 | 8 | 64 | 6 |
| n (Patient) | 64 | 8 | 64 | 8 | 64 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.78 | 0.60 | 0.77 | 0.76 | 0.57 | 0.77 | 0.77 | nd | 0.69 |
| SE | 0.083 | 0.13 | 0.10 | 0.085 | 0.12 | 0.10 | 0.11 | nd | 0.12 |
| p | 8.5E-4 | 0.40 | 0.0085 | 0.0026 | 0.60 | 0.0085 | 0.013 | nd | 0.13 |
| nCohort 1 | 65 | 132 | 64 | 65 | 132 | 64 | 65 | nd | 64 |
| nCohort 2 | 12 | 6 | 8 | 12 | 6 | 8 | 7 | nd | 6 |
| Cutoff 1 | 61.5 | 10.6 | 61.5 | 33.6 | 10.6 | 61.5 | 61.5 | nd | 29.5 |
| Sens 1 | 75% | 83% | 75% | 75% | 83% | 75% | 71% | nd | 83% |
| Spec 1 | 74% | 36% | 66% | 57% | 36% | 66% | 74% | nd | 47% |
| Cutoff 2 | 29.5 | 10.6 | 29.5 | 29.5 | 10.6 | 29.5 | 29.5 | nd | 29.5 |
| Sens 2 | 83% | 83% | 88% | 83% | 83% | 88% | 86% | nd | 83% |
| Spec 2 | 57% | 36% | 47% | 57% | 36% | 47% | 57% | nd | 47% |
| Cutoff 3 | 10.6 | 4.75 | 10.6 | 10.6 | 4.75 | 10.6 | 10.6 | nd | 10.6 |
| Sens 3 | 92% | 100% | 100% | 92% | 100% | 100% | 100% | nd | 100% |
| Spec 3 | 42% | 28% | 33% | 42% | 28% | 33% | 42% | nd | 33% |
| Cutoff 4 | 51.4 | 68.3 | 74.5 | 51.4 | 68.3 | 74.5 | 51.4 | nd | 74.5 |
| Sens 4 | 75% | 50% | 62% | 67% | 33% | 62% | 71% | nd | 50% |
| Spec 4 | 71% | 70% | 70% | 71% | 70% | 70% | 71% | nd | 70% |
| Cutoff 5 | 89.4 | 132 | 105 | 89.4 | 132 | 105 | 89.4 | nd | 105 |
| Sens 5 | 58% | 17% | 50% | 50% | 17% | 50% | 57% | nd | 33% |
| Spec 5 | 80% | 80% | 81% | 80% | 80% | 81% | 80% | nd | 81% |
| Cutoff 6 | 133 | 203 | 148 | 133 | 203 | 148 | 133 | nd | 148 |
| Sens 6 | 42% | 0% | 50% | 42% | 0% | 50% | 43% | nd | 33% |
| Spec 6 | 91% | 90% | 91% | 91% | 90% | 91% | 91% | nd | 91% |
| OR Quart 2 | >2.2 | >2.1 | >2.2 | >2.2 | >2.1 | >2.2 | >1.1 | nd | >2.1 |
| p Value | <0.53 | <0.56 | <0.52 | <0.53 | <0.56 | <0.52 | <0.97 | nd | <0.55 |
| 95% CI of | >0.19 | >0.18 | >0.19 | >0.19 | >0.18 | >0.19 | >0.061 | nd | >0.18 |
| OR Quart2 | na | na | na | na | na | na | na | nd | na |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart 3 | >3.6 | >2.1 | >2.2 | >5.1 | >3.3 | >2.2 | >2.2 | nd | >1.1 |
| p Value | <0.29 | <0.55 | <0.52 | <0.17 | <0.31 | <0.52 | <0.52 | nd | <0.97 |
| 95% CI of | >0.34 | >0.18 | >0.19 | >0.51 | >0.32 | >0.19 | >0.19 | nd | >0.061 |
| OR Quart3 | na | na | na | na | na | na | na | nd | na |
| OR Quart 4 | >10 | >2.1 | >5.1 | >8.1 | >1.0 | >5.1 | >5.1 | nd | >3.4 |
| p Value | <0.039 | <0.56 | <0.16 | <0.065 | <1.0 | <0.16 | <0.16 | nd | <0.31 |
| 95% CI of | >1.1 | >0.18 | >0.52 | >0.88 | >0.060 | >0.52 | >0.52 | nd | >0.32 |
| OR Quart4 | na | na | na | na | na | na | na | nd | na |

| **Neutrophil collagenase** | | | | | | |
|---|---|---|---|---|---|---|
| sCr or UO | 0hr prior to AKI stage | | 24hr prior to AKI stage | | 48hr prior to AKI stage | |
|  | Cohort 1 | Cohort 2 | Cohort 1 | Cohort 2 | Cohort 1 | Cohort 2 |
| Median | 1220 | 4220 | 1220 | 4220 | nd | nd |
| Average | 2280 | 11600 | 2280 | 11600 | nd | nd |
| Stdev | 2900 | 15500 | 2900 | 15500 | nd | nd |
| p(t-test) |  | 3.1E-6 |  | 3.2E-6 | nd | nd |
| Min | 131 | 21.8 | 131 | 1.14 | nd | nd |
| Max | 16000 | 38700 | 16000 | 38700 | nd | nd |
| n (Samp) | 87 | 8 | 87 | 8 | nd | nd |
| n (Patient) | 87 | 8 | 87 | 8 | nd | nd |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1190 | 5900 | 1190 | 5900 | nd | nd |
| Average | 3030 | 14800 | 3030 | 14800 | nd | nd |
| Stdev | 6790 | 16900 | 6790 | 16900 | nd | nd |
| p(t-test) |  | 5.5E-4 |  | 5.5E-4 | nd | nd |
| Min | 131 | 1500 | 131 | 1500 | nd | nd |
| Max | 55000 | 38700 | 55000 | 38700 | nd | nd |
| n (Samp) | 80 | 6 | 80 | 6 | nd | nd |
| n (Patient) | 80 | 6 | 80 | 6 | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.76 | nd | 0.86 | 0.76 | nd | 0.86 | nd | nd | nd |
| SE | 0.10 | nd | 0.098 | 0.10 | nd | 0.098 | nd | nd | nd |
| p | 0.012 | nd | 2.3E-4 | 0.012 | nd | 2.3E-4 | nd | nd | nd |
| nCohort 1 | 87 | nd | 80 | 87 | nd | 80 | nd | nd | nd |
| nCohort 2 | 8 | nd | 6 | 8 | nd | 6 | nd | nd | nd |
| Cutoff 1 | 2830 | nd | 2830 | 2830 | nd | 2830 | nd | nd | nd |
| Sens 1 | 75% | nd | 83% | 75% | nd | 83% | nd | nd | nd |
| Spec 1 | 77% | nd | 76% | 77% | nd | 76% | nd | nd | nd |
| Cutoff 2 | 1490 | nd | 2830 | 1490 | nd | 2830 | nd | nd | nd |
| Sens 2 | 88% | nd | 83% | 88% | nd | 83% | nd | nd | nd |
| Spec 2 | 62% | nd | 76% | 62% | nd | 76% | nd | nd | nd |
| Cutoff 3 | 0 | nd | 1490 | 0 | nd | 1490 | nd | nd | nd |
| Sens 3 | 100% | nd | 100% | 100% | nd | 100% | nd | nd | nd |
| Spec 3 | 0% | nd | 62% | 0% | nd | 62% | nd | nd | nd |
| Cutoff 4 | 2000 | nd | 2060 | 2000 | nd | 2060 | nd | nd | nd |
| Sens 4 | 75% | nd | 83% | 75% | nd | 83% | nd | nd | nd |
| Spec 4 | 70% | nd | 70% | 70% | nd | 70% | nd | nd | nd |
| Cutoff 5 | 3180 | nd | 3180 | 3180 | nd | 3180 | nd | nd | nd |
| Sens 5 | 62% | nd | 67% | 62% | nd | 67% | nd | nd | nd |
| Spec 5 | 80% | nd | 80% | 80% | nd | 80% | nd | nd | nd |
| Cutoff 6 | 5900 | nd | 5900 | 5900 | nd | 5900 | nd | nd | nd |
| Sens 6 | 38% | nd | 50% | 38% | nd | 50% | nd | nd | nd |
| Spec 6 | 91% | nd | 90% | 91% | nd | 90% | nd | nd | nd |
| OR Quart 2 | 0 | nd | >0 | 0 | nd | >0 | nd | nd | nd |
| p Value | na | nd | <na | na | nd | <na | nd | nd | nd |
| 95% CI of | na | nd | >na | na | nd | >na | nd | nd | nd |
| OR Quart2 | na | nd | na | na | nd | na | nd | nd | nd |
| OR Quart 3 | 2.0 | nd | >2.2 | 2.0 | nd | >2.2 | nd | nd | nd |
| p Value | 0.58 | nd | <0.53 | 0.58 | nd | <0.53 | nd | nd | nd |
| 95% CI of | 0.17 | nd | >0.19 | 0.17 | nd | >0.19 | nd | nd | nd |
| OR Quart3 | 24 | nd | na | 24 | nd | na | nd | nd | nd |
| OR Quart 4 | 5.8 | nd | >4.7 | 5.8 | nd | >4.7 | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value | 0.12 | nd | <0.19 | 0.12 | nd | <0.19 | nd | nd | nd |
| 95% CI of | 0.62 | nd | >0.48 | 0.62 | nd | >0.48 | nd | nd | nd |
| OR Quart4 | 54 | nd | na | 54 | nd | na | nd | nd | nd |

**Protransforming growth factor alpha**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.51 | 10.2 | 2.51 | 10.2 | 2.51 | 8.34 |
| Average | 8.67 | 30.9 | 8.67 | 30.9 | 8.67 | 16.5 |
| Stdev | 35.8 | 50.3 | 35.8 | 50.4 | 35.8 | 23.6 |
| p(t-test) | | 0.068 | | 0.069 | | 0.57 |
| Min | 0.00305 | 0.566 | 0.00305 | 0.227 | 0.00305 | 0.783 |
| Max | 287 | 173 | 287 | 173 | 287 | 68.3 |
| n (Samp) | 65 | 12 | 65 | 12 | 65 | 7 |
| n (Patient) | 65 | 12 | 65 | 12 | 65 | 7 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 2.57 | 4.38 | 2.57 | 4.38 | nd | nd |
| Average | 9.04 | 14.3 | 9.04 | 14.2 | nd | nd |
| Stdev | 30.5 | 24.0 | 30.5 | 24.1 | nd | nd |
| p(t-test) | | 0.68 | | 0.68 | nd | nd |
| Min | 0.00305 | 0.566 | 0.00305 | 0.227 | nd | nd |
| Max | 287 | 62.6 | 287 | 62.6 | nd | nd |
| n (Samp) | 132 | 6 | 132 | 6 | nd | nd |
| n (Patient) | 132 | 6 | 132 | 6 | nd | nd |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 2.34 | 12.3 | 2.34 | 12.3 | 2.34 | 10.2 |
| Average | 8.46 | 38.0 | 8.46 | 38.0 | 8.46 | 19.0 |
| Stdev | 36.1 | 58.4 | 36.1 | 58.4 | 36.1 | 24.9 |
| p(t-test) | | 0.047 | | 0.047 | | 0.49 |
| Min | 0.00305 | 0.783 | 0.00305 | 0.783 | 0.00305 | 0.783 |
| Max | 287 | 173 | 287 | 173 | 287 | 68.3 |
| n (Samp) | 64 | 8 | 64 | 8 | 64 | 6 |
| n (Patient) | 64 | 8 | 64 | 8 | 64 | 6 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| | | | | | | |
| | 0hr prior to AKI stage | | | 24hr prior to AKI stage | | | 48hr prior to AKI stage | | |
| | sCr or UO | sCr only | UO only | sCr or UO | sCr only | UO only | sCr or UO | sCr only | UO only |
| AUC | 0.74 | 0.63 | 0.85 | 0.73 | 0.62 | 0.85 | 0.75 | nd | 0.81 |
| SE | 0.087 | 0.12 | 0.088 | 0.088 | 0.12 | 0.088 | 0.11 | nd | 0.11 |
| p | 0.0060 | 0.28 | 6.8E-5 | 0.0088 | 0.34 | 6.8E-5 | 0.026 | nd | 0.0042 |
| nCohort 1 | 65 | 132 | 64 | 65 | 132 | 64 | 65 | nd | 64 |
| nCohort 2 | 12 | 6 | 8 | 12 | 6 | 8 | 7 | nd | 6 |
| Cutoff 1 | 2.57 | 1.80 | 7.01 | 2.57 | 1.80 | 7.01 | 5.39 | nd | 5.39 |
| Sens 1 | 75% | 83% | 75% | 75% | 83% | 75% | 71% | nd | 83% |
| Spec 1 | 52% | 42% | 89% | 52% | 42% | 89% | 86% | nd | 88% |
| Cutoff 2 | 1.80 | 1.80 | 5.39 | 1.80 | 1.80 | 5.39 | 1.80 | nd | 5.39 |
| Sens 2 | 83% | 83% | 88% | 83% | 83% | 88% | 86% | nd | 83% |
| Spec 2 | 40% | 42% | 88% | 40% | 42% | 88% | 40% | nd | 88% |
| Cutoff 3 | 0.775 | 0.540 | 0.775 | 0.775 | 0.137 | 0.775 | 0.775 | nd | 0.775 |
| Sens 3 | 92% | 100% | 100% | 92% | 100% | 100% | 100% | nd | 100% |
| Spec 3 | 25% | 20% | 30% | 25% | 11% | 30% | 25% | nd | 30% |
| Cutoff 4 | 3.59 | 4.18 | 3.36 | 3.59 | 4.18 | 3.36 | 3.59 | nd | 3.36 |
| Sens 4 | 67% | 50% | 88% | 67% | 50% | 88% | 71% | nd | 83% |
| Spec 4 | 71% | 70% | 70% | 71% | 70% | 70% | 71% | nd | 70% |
| Cutoff 5 | 4.80 | 5.28 | 4.36 | 4.80 | 5.28 | 4.36 | 4.80 | nd | 4.36 |
| Sens 5 | 67% | 50% | 88% | 67% | 50% | 88% | 71% | nd | 83% |
| Spec 5 | 80% | 80% | 81% | 80% | 80% | 81% | 80% | nd | 81% |
| Cutoff 6 | 7.33 | 13.7 | 9.25 | 7.33 | 13.7 | 9.25 | 7.33 | nd | 9.25 |
| Sens 6 | 58% | 17% | 62% | 58% | 17% | 62% | 57% | nd | 50% |
| Spec 6 | 91% | 90% | 91% | 91% | 90% | 91% | 91% | nd | 91% |
| OR Quart 2 | 1.0 | 2.0 | >1.1 | 1.0 | 2.0 | >1.1 | 1.0 | nd | >1.0 |
| p Value | 1.0 | 0.58 | <0.97 | 1.0 | 0.58 | <0.97 | 1.0 | nd | <1.0 |
| 95% CI of | 0.13 | 0.17 | >0.061 | 0.13 | 0.17 | >0.061 | 0.058 | nd | >0.058 |
| OR Quart2 | 7.9 | 23 | na | 7.9 | 23 | na | 17 | nd | na |
| OR Quart 3 | 0 | 0 | >0 | 0 | 0 | >0 | 0 | nd | >0 |
| p Value | na | na | <na | na | na | <na | na | nd | <na |
| 95% CI of | na | na | >na | na | na | >na | na | nd | >na |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart3 | na | na | na | na | na | na | na | nd | na |
| OR Quart 4 | 5.7 | 3.1 | >11 | 5.7 | 3.1 | >11 | 6.5 | nd | >6.5 |
| p Value | 0.048 | 0.34 | <0.032 | 0.048 | 0.34 | <0.032 | 0.10 | nd | <0.10 |
| 95% CI of OR Quart4 | 1.0 | 0.31 | >1.2 | 1.0 | 0.31 | >1.2 | 0.68 | nd | >0.68 |
| | 32 | 31 | na | 32 | 31 | na | 63 | nd | na |

**CA 15-3**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.245 | 0.833 | 0.245 | 0.644 | nd | nd |
| Average | 0.500 | 1.04 | 0.500 | 0.953 | nd | nd |
| Stdev | 0.916 | 0.811 | 0.916 | 0.817 | nd | nd |
| p(t-test) | | 0.11 | | 0.18 | nd | nd |
| Min | 0.0328 | 0.249 | 0.0328 | 0.249 | nd | nd |
| Max | 6.54 | 2.69 | 6.54 | 2.69 | nd | nd |
| n (Samp) | 87 | 8 | 87 | 8 | nd | nd |
| n (Patient) | 87 | 8 | 87 | 8 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.289 | 0.588 | 0.289 | 0.588 | nd | nd |
| Average | 0.616 | 1.01 | 0.616 | 1.01 | nd | nd |
| Stdev | 1.07 | 0.952 | 1.07 | 0.952 | nd | nd |
| p(t-test) | | 0.39 | | 0.39 | nd | nd |
| Min | 0.0328 | 0.249 | 0.0328 | 0.249 | nd | nd |
| Max | 6.54 | 2.69 | 6.54 | 2.69 | nd | nd |
| n (Samp) | 80 | 6 | 80 | 6 | nd | nd |
| n (Patient) | 80 | 6 | 80 | 6 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.82 | nd | 0.75 | 0.81 | nd | 0.75 | nd | nd | nd |
| SE | 0.093 | nd | 0.12 | 0.094 | nd | 0.12 | nd | nd | nd |
| p | 5.7E-4 | nd | 0.039 | 8.9E-4 | nd | 0.039 | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| nCohort 1 | 87 | nd | 80 | 87 | nd | 80 | nd | nd | nd |
| nCohort 2 | 8 | nd | 6 | 8 | nd | 6 | nd | nd | nd |
| Cutoff 1 | 0.476 | nd | 0.346 | 0.476 | nd | 0.346 | nd | nd | nd |
| Sens 1 | 75% | nd | 83% | 75% | nd | 83% | nd | nd | nd |
| Spec 1 | 83% | nd | 59% | 83% | nd | 59% | nd | nd | nd |
| Cutoff 2 | 0.326 | nd | 0.346 | 0.326 | nd | 0.346 | nd | nd | nd |
| Sens 2 | 88% | nd | 83% | 88% | nd | 83% | nd | nd | nd |
| Spec 2 | 68% | nd | 59% | 68% | nd | 59% | nd | nd | nd |
| Cutoff 3 | 0.248 | nd | 0.248 | 0.248 | nd | 0.248 | nd | nd | nd |
| Sens 3 | 100% | nd | 100% | 100% | nd | 100% | nd | nd | nd |
| Spec 3 | 54% | nd | 46% | 54% | nd | 46% | nd | nd | nd |
| Cutoff 4 | 0.353 | nd | 0.402 | 0.353 | nd | 0.402 | nd | nd | nd |
| Sens 4 | 75% | nd | 67% | 75% | nd | 67% | nd | nd | nd |
| Spec 4 | 70% | nd | 70% | 70% | nd | 70% | nd | nd | nd |
| Cutoff 5 | 0.466 | nd | 0.708 | 0.466 | nd | 0.708 | nd | nd | nd |
| Sens 5 | 75% | nd | 33% | 75% | nd | 33% | nd | nd | nd |
| Spec 5 | 80% | nd | 80% | 80% | nd | 80% | nd | nd | nd |
| Cutoff 6 | 1.28 | nd | 1.57 | 1.28 | nd | 1.57 | nd | nd | nd |
| Sens 6 | 25% | nd | 33% | 25% | nd | 33% | nd | nd | nd |
| Spec 6 | 91% | nd | 90% | 91% | nd | 90% | nd | nd | nd |
| OR Quart 2 | >0 | nd | >1.0 | >0 | nd | >1.0 | nd | nd | nd |
| p Value | <na | nd | <1.0 | <na | nd | <1.0 | nd | nd | nd |
| 95% CI of | >na | nd | >0.059 | >na | nd | >0.059 | nd | nd | nd |
| OR Quart2 | na | nd | na | na | nd | na | nd | nd | nd |
| OR Quart 3 | >2.1 | nd | >1.0 | >2.1 | nd | >1.0 | nd | nd | nd |
| p Value | <0.56 | nd | <0.97 | <0.56 | nd | <0.97 | nd | nd | nd |
| 95% CI of | >0.18 | nd | >0.061 | >0.18 | nd | >0.061 | nd | nd | nd |
| OR Quart3 | na | nd | na | na | nd | na | nd | nd | nd |
| OR Quart 4 | >7.7 | nd | >4.7 | >7.7 | nd | >4.7 | nd | nd | nd |
| p Value | <0.070 | nd | <0.19 | <0.070 | nd | <0.19 | nd | nd | nd |
| 95% CI of | >0.85 | nd | >0.48 | >0.85 | nd | >0.48 | nd | nd | nd |
| OR Quart4 | na | nd | na | na | nd | na | nd | nd | nd |

Table 9: Comparison of marker levels in urine samples collected from Cohort 1 (patients that did not progress beyond RIFLE stage 0, R, or I) and in urine samples collected from Cohort 2 (subjects who progress to RIFLE stage F) at 0, 24 hours, and 48 hours prior to the subject reaching RIFLE stage I.

| C-C motif chemokine 18 | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 0.474 | 2.53 | 0.474 | 3.21 | 0.474 | 0.628 |
| Average | 2.27 | 6.02 | 2.27 | 10.9 | 2.27 | 12.6 |
| Stdev | 6.63 | 9.81 | 6.63 | 14.5 | 6.63 | 18.9 |
| p(t-test) | | 0.0092 | | 2.1E-8 | | 1.7E-6 |
| Min | 3.13E-5 | 0.0680 | 3.13E-5 | 0.138 | 3.13E-5 | 0.00268 |
| Max | 40.0 | 40.0 | 40.0 | 40.0 | 40.0 | 40.0 |
| n (Samp) | 1273 | 22 | 1273 | 20 | 1273 | 10 |
| n (Patient) | 451 | 22 | 451 | 20 | 451 | 10 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 0.490 | 4.14 | nd | nd | nd | nd |
| Average | 2.63 | 9.65 | nd | nd | nd | nd |
| Stdev | 7.46 | 10.8 | nd | nd | nd | nd |
| p(t-test) | | 0.0082 | nd | nd | nd | nd |
| Min | 3.13E-5 | 0.204 | nd | nd | nd | nd |
| Max | 40.0 | 31.3 | nd | nd | nd | nd |
| n (Samp) | 1337 | 8 | nd | nd | nd | nd |
| n (Patient) | 466 | 8 | nd | nd | nd | nd |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 0.490 | 2.16 | 0.490 | 3.58 | 0.490 | 0.724 |
| Average | 2.46 | 8.94 | 2.46 | 13.1 | 2.46 | 17.3 |
| Stdev | 6.94 | 14.1 | 6.94 | 16.2 | 6.94 | 21.2 |
| p(t-test) | | 6.6E-4 | | 2.3E-10 | | 4.2E-8 |
| Min | 3.13E-5 | 0.0680 | 3.13E-5 | 0.138 | 3.13E-5 | 0.119 |
| Max | 40.0 | 40.0 | 40.0 | 40.0 | 40.0 | 40.0 |
| n (Samp) | 1119 | 14 | 1119 | 19 | 1119 | 7 |
| n (Patient) | 361 | 14 | 361 | 19 | 361 | 7 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.72 | 0.83 | 0.71 | 0.78 | nd | 0.79 | 0.56 | nd | 0.58 |
| SE | 0.062 | 0.090 | 0.079 | 0.062 | nd | 0.062 | 0.094 | nd | 0.11 |
| p | 4.1E-4 | 2.4E-4 | 0.0090 | 7.2E-6 | nd | 2.8E-6 | 0.53 | nd | 0.48 |
| nCohort 1 | 1273 | 1337 | 1119 | 1273 | nd | 1119 | 1273 | nd | 1119 |
| nCohort 2 | 22 | 8 | 14 | 20 | nd | 19 | 10 | nd | 7 |
| Cutoff 1 | 0.862 | 2.72 | 0.862 | 1.98 | nd | 1.98 | 0.131 | nd | 0.131 |
| Sens 1 | 73% | 75% | 71% | 70% | nd | 74% | 70% | nd | 71% |
| Spec 1 | 65% | 85% | 63% | 83% | nd | 81% | 19% | nd | 17% |
| Cutoff 2 | 0.351 | 2.33 | 0.232 | 0.788 | nd | 0.788 | 0.125 | nd | 0.125 |
| Sens 2 | 82% | 88% | 86% | 80% | nd | 84% | 80% | nd | 86% |
| Spec 2 | 43% | 83% | 29% | 63% | nd | 62% | 18% | nd | 17% |
| Cutoff 3 | 0.204 | 0.204 | 0.164 | 0.270 | nd | 0.142 | 0.118 | nd | 0.118 |
| Sens 3 | 91% | 100% | 93% | 90% | nd | 95% | 90% | nd | 100% |
| Spec 3 | 27% | 27% | 22% | 36% | nd | 19% | 18% | nd | 16% |
| Cutoff 4 | 1.04 | 1.10 | 1.12 | 1.04 | nd | 1.12 | 1.04 | nd | 1.12 |
| Sens 4 | 68% | 88% | 64% | 75% | nd | 79% | 40% | nd | 43% |
| Spec 4 | 70% | 70% | 70% | 70% | nd | 70% | 70% | nd | 70% |
| Cutoff 5 | 1.76 | 1.90 | 1.90 | 1.76 | nd | 1.90 | 1.76 | nd | 1.90 |
| Sens 5 | 55% | 88% | 50% | 70% | nd | 74% | 40% | nd | 43% |
| Spec 5 | 80% | 80% | 80% | 80% | nd | 80% | 80% | nd | 80% |
| Cutoff 6 | 3.72 | 4.03 | 3.96 | 3.72 | nd | 3.96 | 3.72 | nd | 3.96 |
| Sens 6 | 36% | 50% | 43% | 45% | nd | 47% | 40% | nd | 43% |
| Spec 6 | 90% | 90% | 90% | 90% | nd | 90% | 90% | nd | 90% |
| OR Quart 2 | 1.5 | >1.0 | 0.50 | 1.0 | nd | 0.50 | 0 | nd | 0 |
| p Value | 0.66 | <1.00 | 0.57 | 1.0 | nd | 0.57 | na | nd | na |
| 95% CI of | 0.25 | >0.062 | 0.045 | 0.14 | nd | 0.045 | na | nd | na |
| OR Quart2 | 9.0 | na | 5.5 | 7.1 | nd | 5.5 | na | nd | na |
| OR Quart 3 | 2.0 | >0 | 1.5 | 0.50 | nd | 0.50 | 0.50 | nd | 0.33 |
| p Value | 0.42 | <na | 0.66 | 0.57 | nd | 0.57 | 0.42 | nd | 0.34 |
| 95% CI of | 0.36 | >na | 0.25 | 0.045 | nd | 0.045 | 0.090 | nd | 0.034 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart3 | 11 | na | 9.1 | 5.5 | nd | 5.5 | 2.7 | nd | 3.2 |
| OR Quart 4 | 6.7 | >7.1 | 4.1 | 7.8 | nd | 7.8 | 1.00 | nd | 1.00 |
| p Value | 0.013 | <0.067 | 0.077 | 0.0067 | nd | 0.0066 | 1.00 | nd | 1.00 |
| 95% CI of | 1.5 | >0.87 | 0.86 | 1.8 | nd | 1.8 | 0.25 | nd | 0.20 |
| OR Quart4 | 30 | na | 19 | 34 | nd | 35 | 4.0 | nd | 5.0 |

**C-C motif chemokine 24**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 12.9 | 39.0 | 12.9 | 34.2 | 12.9 | 14.0 |
| Average | 28.6 | 216 | 28.6 | 269 | 28.6 | 127 |
| Stdev | 58.5 | 474 | 58.5 | 594 | 58.5 | 368 |
| p(t-test) |  | 1.8E-24 |  | 2.4E-29 |  | 2.6E-6 |
| Min | 0.0120 | 5.58 | 0.0120 | 0.0347 | 0.0120 | 0.0635 |
| Max | 1090 | 1930 | 1090 | 2380 | 1090 | 1170 |
| n (Samp) | 1275 | 22 | 1275 | 20 | 1275 | 10 |
| n (Patient) | 452 | 22 | 452 | 20 | 452 | 10 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 13.7 | 57.3 | nd | nd | nd | nd |
| Average | 42.1 | 88.9 | nd | nd | nd | nd |
| Stdev | 152 | 118 | nd | nd | nd | nd |
| p(t-test) |  | 0.38 | nd | nd | nd | nd |
| Min | 0.0120 | 11.1 | nd | nd | nd | nd |
| Max | 2790 | 373 | nd | nd | nd | nd |
| n (Samp) | 1339 | 8 | nd | nd | nd | nd |
| n (Patient) | 467 | 8 | nd | nd | nd | nd |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 13.2 | 56.4 | 13.2 | 37.2 | 13.2 | 14.8 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 29.6 | 391 | 29.6 | 384 | 29.6 | 178 |
| Stdev | 60.6 | 769 | 60.6 | 710 | 60.6 | 439 |
| p(t-test) | | 6.3E-37 | | 1.8E-42 | | 1.3E-8 |
| Min | 0.0120 | 5.58 | 0.0120 | 0.0347 | 0.0120 | 4.32 |
| Max | 1090 | 2790 | 1090 | 2380 | 1090 | 1170 |
| n (Samp) | 1122 | 14 | 1122 | 19 | 1122 | 7 |
| n (Patient) | 362 | 14 | 362 | 19 | 362 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.73 | 0.79 | 0.76 | 0.71 | nd | 0.76 | 0.50 | nd | 0.53 |
| SE | 0.062 | 0.096 | 0.075 | 0.066 | nd | 0.065 | 0.092 | nd | 0.11 |
| p | 1.7E-4 | 0.0031 | 6.2E-4 | 0.0012 | nd | 6.7E-5 | 1.00 | nd | 0.77 |
| nCohort 1 | 1275 | 1339 | 1122 | 1275 | nd | 1122 | 1275 | nd | 1122 |
| nCohort 2 | 22 | 8 | 14 | 20 | nd | 19 | 10 | nd | 7 |
| Cutoff 1 | 21.9 | 25.9 | 22.5 | 27.0 | nd | 27.2 | 6.94 | nd | 6.94 |
| Sens 1 | 73% | 75% | 71% | 70% | nd | 74% | 70% | nd | 71% |
| Spec 1 | 65% | 68% | 66% | 71% | nd | 71% | 37% | nd | 36% |
| Cutoff 2 | 9.98 | 21.9 | 8.63 | 14.2 | nd | 16.6 | 4.59 | nd | 4.59 |
| Sens 2 | 82% | 88% | 86% | 80% | nd | 84% | 80% | nd | 86% |
| Spec 2 | 44% | 63% | 40% | 53% | nd | 57% | 30% | nd | 29% |
| Cutoff 3 | 8.65 | 11.1 | 5.84 | 3.78 | nd | 3.78 | 4.24 | nd | 4.24 |
| Sens 3 | 91% | 100% | 93% | 90% | nd | 95% | 90% | nd | 100% |
| Spec 3 | 42% | 45% | 32% | 29% | nd | 28% | 30% | nd | 28% |
| Cutoff 4 | 26.4 | 28.1 | 26.7 | 26.4 | nd | 26.7 | 26.4 | nd | 26.7 |
| Sens 4 | 59% | 62% | 64% | 70% | nd | 79% | 10% | nd | 14% |
| Spec 4 | 70% | 70% | 70% | 70% | nd | 70% | 70% | nd | 70% |
| Cutoff 5 | 40.3 | 42.2 | 40.9 | 40.3 | nd | 40.9 | 40.3 | nd | 40.9 |
| Sens 5 | 50% | 62% | 64% | 40% | nd | 47% | 10% | nd | 14% |
| Spec 5 | 80% | 80% | 80% | 80% | nd | 80% | 80% | nd | 80% |
| Cutoff 6 | 69.6 | 78.2 | 72.3 | 69.6 | nd | 72.3 | 69.6 | nd | 72.3 |
| Sens 6 | 32% | 38% | 43% | 35% | nd | 37% | 10% | nd | 14% |
| Spec 6 | 90% | 90% | 90% | 90% | nd | 90% | 90% | nd | 90% |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart 2 | >6.1 | >1.0 | >4.1 | 1.00 | nd | 2.0 | 3.0 | nd | >3.0 |
| p Value | <0.095 | <1.0 | <0.21 | 1.00 | nd | 0.57 | 0.34 | nd | <0.34 |
| 95% CI of | >0.73 | >0.062 | >0.45 | 0.14 | nd | 0.18 | 0.31 | nd | >0.31 |
| OR Quart2 | na | na | na | 7.1 | nd | 22 | 29 | nd | na |
| OR Quart 3 | >5.1 | >2.0 | >1.0 | 3.0 | nd | 5.1 | 5.1 | nd | >3.0 |
| p Value | <0.14 | <0.57 | <1.00 | 0.18 | nd | 0.14 | 0.14 | nd | <0.34 |
| 95% CI of | >0.59 | >0.18 | >0.062 | 0.61 | nd | 0.59 | 0.59 | nd | >0.31 |
| OR Quart3 | na | na | na | 15 | nd | 44 | 44 | nd | na |
| OR Quart 4 | >11 | >5.1 | >9.3 | 5.1 | nd | 11 | 1.00 | nd | >1.0 |
| p Value | <0.020 | <0.14 | <0.035 | 0.036 | nd | 0.020 | 1.00 | nd | <1.0 |
| 95% CI of | >1.5 | >0.59 | >1.2 | 1.1 | nd | 1.5 | 0.062 | nd | >0.062 |
| OR Quart4 | na | na | na | 24 | nd | 89 | 16 | nd | na |

**C-C motif chemokine 8**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.36 | 23.8 | 1.36 | 10.0 | 1.36 | 1.61 |
| Average | 9.75 | 211 | 9.75 | 42.0 | 9.75 | 16.5 |
| Stdev | 26.6 | 775 | 26.6 | 70.5 | 26.6 | 34.1 |
| p(t-test) |  | 1.7E-19 |  | 2.9E-7 |  | 0.43 |
| Min | 0.0250 | 0.206 | 0.0250 | 0.0945 | 0.0250 | 0.0472 |
| Max | 473 | 3670 | 473 | 294 | 473 | 111 |
| n (Samp) | 1275 | 22 | 1275 | 20 | 1275 | 10 |
| n (Patient) | 452 | 22 | 452 | 20 | 452 | 10 |
|  |  |  |  |  |  |  |
| sCr only | 0hr prior to AKI stage | | 24hr prior to AKI stage | | 48hr prior to AKI stage | |
|  | Cohort 1 | Cohort 2 | Cohort 1 | Cohort 2 | Cohort 1 | Cohort 2 |
| Median | 1.42 | 16.3 | nd | nd | nd | nd |
| Average | 14.1 | 29.6 | nd | nd | nd | nd |

(continued)

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | 105 | 40.1 | nd | nd | nd | nd |
| p(t-test) | | 0.68 | nd | nd | nd | nd |
| Min | 0.0250 | 0.206 | nd | nd | nd | nd |
| Max | 3670 | 116 | nd | nd | nd | nd |
| n (Samp) | 1339 | 8 | nd | nd | nd | nd |
| n (Patient) | 467 | 8 | nd | nd | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.36 | 31.3 | 1.36 | 30.8 | 1.36 | 2.77 |
| Average | 10.1 | 59.3 | 10.1 | 51.5 | 10.1 | 20.2 |
| Stdev | 27.7 | 66.2 | 27.7 | 71.6 | 27.7 | 40.5 |
| p(t-test) | | 2.0E-10 | | 9.3E-10 | | 0.34 |
| Min | 0.0250 | 0.829 | 0.0250 | 0.136 | 0.0250 | 0.0472 |
| Max | 473 | 176 | 473 | 294 | 473 | 111 |
| n (Samp) | 1122 | 14 | 1122 | 19 | 1122 | 7 |
| n (Patient) | 362 | 14 | 362 | 19 | 362 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.77 | 0.71 | 0.81 | 0.73 | nd | 0.79 | 0.52 | nd | 0.55 |
| SE | 0.059 | 0.10 | 0.071 | 0.065 | nd | 0.062 | 0.093 | nd | 0.11 |
| p | 3.7E-6 | 0.046 | 1.4E-5 | 4.2E-4 | nd | 3.7E-6 | 0.79 | nd | 0.68 |
| nCohort 1 | 1275 | 1339 | 1122 | 1275 | nd | 1122 | 1275 | nd | 1122 |
| nCohort 2 | 22 | 8 | 14 | 20 | nd | 19 | 10 | nd | 7 |
| Cutoff 1 | 2.74 | 2.74 | 12.6 | 3.20 | nd | 8.37 | 0.240 | nd | 0.438 |
| Sens 1 | 77% | 75% | 71% | 70% | nd | 74% | 70% | nd | 71% |
| Spec 1 | 56% | 55% | 82% | 60% | nd | 74% | 34% | nd | 39% |
| Cutoff 2 | 0.802 | 0.491 | 0.802 | 1.21 | nd | 1.21 | 0.127 | nd | 0.127 |
| Sens 2 | 91% | 88% | 100% | 80% | nd | 84% | 80% | nd | 86% |
| Spec 2 | 44% | 38% | 44% | 49% | nd | 49% | 21% | nd | 20% |
| Cutoff 3 | 0.802 | 0.154 | 0.802 | 0.491 | nd | 0.491 | 0.0705 | nd | 0.0250 |
| Sens 3 | 91% | 100% | 100% | 90% | nd | 95% | 90% | nd | 100% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 3 | 44% | 26% | 44% | 40% | nd | 39% | 11% | nd | 2% |
| Cutoff 4 | 6.78 | 7.38 | 6.78 | 6.78 | nd | 6.78 | 6.78 | nd | 6.78 |
| Sens 4 | 68% | 62% | 71% | 65% | nd | 74% | 40% | nd | 43% |
| Spec 4 | 70% | 70% | 70% | 70% | nd | 70% | 70% | nd | 70% |
| Cutoff 5 | 11.5 | 12.5 | 11.8 | 11.5 | nd | 11.8 | 11.5 | nd | 11.8 |
| Sens 5 | 64% | 50% | 71% | 45% | nd | 58% | 40% | nd | 29% |
| Spec 5 | 80% | 80% | 80% | 80% | nd | 80% | 80% | nd | 80% |
| Cutoff 6 | 22.8 | 27.7 | 23.0 | 22.8 | nd | 23.0 | 22.8 | nd | 23.0 |
| Sens 6 | 50% | 25% | 57% | 45% | nd | 58% | 10% | nd | 14% |
| Spec 6 | 90% | 90% | 90% | 90% | nd | 90% | 90% | nd | 90% |
| OR Quart 2 | >5.1 | >2.0 | >3.0 | 1.00 | nd | 2.0 | 0.66 | nd | 0.50 |
| p Value | <0.14 | <0.57 | <0.34 | 1.00 | nd | 0.57 | 0.66 | nd | 0.57 |
| 95% CI of | >0.59 | >0.18 | >0.31 | 0.14 | nd | 0.18 | 0.11 | nd | 0.045 |
| OR Quart2 | na | na | na | 7.1 | nd | 22 | 4.0 | nd | 5.5 |
| OR Quart 3 | >3.0 | >2.0 | >1.0 | 2.5 | nd | 3.0 | 0.33 | nd | 0.50 |
| p Value | <0.34 | <0.57 | <1.00 | 0.27 | nd | 0.34 | 0.34 | nd | 0.57 |
| 95% CI of | >0.31 | >0.18 | >0.062 | 0.48 | nd | 0.31 | 0.034 | nd | 0.045 |
| OR Quart3 | na | na | na | 13 | nd | 29 | 3.2 | nd | 5.5 |
| OR Quart 4 | >15 | >4.0 | >10 | 5.6 | nd | 14 | 1.3 | nd | 1.5 |
| p Value | <0.0098 | <0.21 | <0.026 | 0.025 | nd | 0.012 | 0.71 | nd | 0.66 |
| 95% CI of | >1.9 | >0.45 | >1.3 | 1.2 | nd | 1.8 | 0.30 | nd | 0.25 |
| OR Quart4 | na | na | na | 26 | nd | 100 | 6.0 | nd | 9.0 |

**Cathepsin D**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 75900 | 115000 | 75900 | 114000 | 75900 | 101000 |
| Average | 81800 | 121000 | 81800 | 117000 | 81800 | 95500 |
| Stdev | 41500 | 43500 | 41500 | 43600 | 41500 | 52800 |
| p(t-test) | | 1.2E-5 | | 1.4E-4 | | 0.30 |

(continued)

| Cathepsin D | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Min | 656 | 63100 | 656 | 45000 | 656 | 4320 |
| Max | 200000 | 200000 | 200000 | 200000 | 200000 | 200000 |
| n (Samp) | 1273 | 22 | 1273 | 20 | 1273 | 10 |
| n (Patient) | 451 | 22 | 451 | 20 | 451 | 10 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 78100 | 99300 | nd | nd | nd | nd |
| Average | 84000 | 109000 | nd | nd | nd | nd |
| Stdev | 43000 | 32700 | nd | nd | nd | nd |
| p(t-test) | | 0.10 | nd | nd | nd | nd |
| Min | 656 | 71400 | nd | nd | nd | nd |
| Max | 200000 | 151000 | nd | nd | nd | nd |
| n (Samp) | 1337 | 8 | nd | nd | nd | nd |
| n (Patient) | 466 | 8 | nd | nd | nd | nd |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 78500 | 132000 | 78500 | 117000 | 78500 | 99800 |
| Average | 84100 | 128000 | 84100 | 123000 | 84100 | 104000 |
| Stdev | 41200 | 47600 | 41200 | 46300 | 41200 | 51000 |
| p(t-test) | | 7.8E-5 | | 4.2E-5 | | 0.21 |
| Min | 2520 | 63100 | 2520 | 45000 | 2520 | 54900 |
| Max | 200000 | 200000 | 200000 | 200000 | 200000 | 200000 |
| n(Samp) | 1119 | 14 | 1119 | 19 | 1119 | 7 |
| n (Patient) | 361 | 14 | 361 | 19 | 361 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.75 | 0.69 | 0.76 | 0.73 | nd | 0.74 | 0.59 | nd | 0.61 |
| SE | 0.061 | 0.10 | 0.075 | 0.065 | nd | 0.066 | 0.095 | nd | 0.11 |
| p | 5.9E-5 | 0.063 | 6.5E-4 | 3.8E-4 | nd | 2.7E-4 | 0.34 | nd | 0.36 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| nCohort 1 | 1273 | 1337 | 1119 | 1273 | nd | 1119 | 1273 | nd | 1119 |
| nCohort 2 | 22 | 8 | 14 | 20 | nd | 19 | 10 | nd | 7 |
| Cutoff 1 | 84700 | 89600 | 89600 | 90800 | nd | 90800 | 66900 | nd | 66900 |
| Sens 1 | 73% | 75% | 71% | 70% | nd | 74% | 70% | nd | 71% |
| Spec 1 | 58% | 60% | 61% | 64% | nd | 62% | 40% | nd | 37% |
| Cutoff 2 | 76300 | 73800 | 76300 | 84400 | nd | 84400 | 63400 | nd | 63400 |
| Sens 2 | 82% | 88% | 86% | 80% | nd | 84% | 80% | nd | 86% |
| Spec 2 | 50% | 46% | 48% | 58% | nd | 56% | 36% | nd | 33% |
| Cutoff 3 | 71400 | 71400 | 70900 | 73700 | nd | 65500 | 54900 | nd | 54900 |
| Sens 3 | 91% | 100% | 93% | 90% | nd | 95% | 90% | nd | 100% |
| Spec 3 | 45% | 43% | 42% | 47% | nd | 35% | 27% | nd | 24% |
| Cutoff 4 | 101000 | 104000 | 103000 | 101000 | nd | 103000 | 101000 | nd | 103000 |
| Sens 4 | 59% | 50% | 64% | 60% | nd | 63% | 50% | nd | 43% |
| Spec 4 | 70% | 70% | 70% | 70% | nd | 70% | 70% | nd | 70% |
| Cutoff 5 | 116000 | 119000 | 118000 | 116000 | nd | 118000 | 116000 | nd | 118000 |
| Sens 5 | 50% | 38% | 57% | 45% | nd | 47% | 30% | nd | 29% |
| Spec 5 | 80% | 80% | 80% | 80% | nd | 80% | 80% | nd | 80% |
| Cutoff 6 | 137000 | 144000 | 140000 | 137000 | nd | 140000 | 137000 | nd | 140000 |
| Sens 6 | 41% | 25% | 50% | 25% | nd | 26% | 10% | nd | 14% |
| Spec 6 | 90% | 90% | 90% | 90% | nd | 90% | 90% | nd | 90% |
| OR Quart 2 | >5.1 | >2.0 | >3.0 | 2.0 | nd | 2.0 | 3.0 | nd | 2.0 |
| p Value | <0.14 | <0.57 | <0.34 | 0.57 | nd | 0.57 | 0.34 | nd | 0.57 |
| 95% CI of | >0.59 | >0.18 | >0.31 | 0.18 | nd | 0.18 | 0.31 | nd | 0.18 |
| OR Quart2 | na | na | na | 22 | nd | 22 | 29 | nd | 22 |
| OR Quart 3 | >4.0 | >3.0 | >3.0 | 5.1 | nd | 5.1 | 3.0 | nd | 2.0 |
| p Value | <0.21 | <0.34 | <0.34 | 0.14 | nd | 0.14 | 0.34 | nd | 0.57 |
| 95% CI of | >0.45 | >0.31 | >0.31 | 0.59 | nd | 0.59 | 0.31 | nd | 0.18 |
| OR Quart3 | na | na | na | 44 | nd | 44 | 29 | nd | 22 |
| OR Quart 4 | >14 | >3.0 | >8.2 | 12 | nd | 11 | 3.0 | nd | 2.0 |
| p Value | <0.012 | <0.34 | <0.048 | 0.016 | nd | 0.020 | 0.34 | nd | 0.57 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of | >1.8 | >0.31 | >1.0 | 1.6 | nd | 1.5 | 0.31 | nd | 0.18 |
| OR Quart4 | na | na | na | 96 | nd | 89 | 29 | nd | 22 |

**C-X-C motif chemokine 13**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0398 | 6.51 | 0.0398 | 13.3 | 0.0398 | 2.67 |
| Average | 6.13 | 110 | 6.13 | 38.0 | 6.13 | 52.4 |
| Stdev | 35.0 | 392 | 35.0 | 47.6 | 35.0 | 138 |
| p(t-test) | | 5.5E-15 | | 6.6E-5 | | 7.7E-5 |
| Min | 0.00269 | 0.0104 | 0.00269 | 0.00471 | 0.00269 | 0.00471 |
| Max | 832 | 1850 | 832 | 131 | 832 | 440 |
| n (Samp) | 1275 | 22 | 1275 | 20 | 1275 | 10 |
| n (Patient) | 452 | 22 | 452 | 20 | 452 | 10 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.145 | 3.98 | nd | nd | nd | nd |
| Average | 11.2 | 13.7 | nd | nd | nd | nd |
| Stdev | 83.4 | 27.2 | nd | nd | nd | nd |
| p(t-test) | | 0.93 | nd | nd | nd | nd |
| Min | 0.00269 | 0.0174 | nd | nd | nd | nd |
| Max | 1930 | 80.5 | nd | nd | nd | nd |
| n (Samp) | 1339 | 8 | nd | nd | nd | nd |
| n (Patient) | 467 | 8 | nd | nd | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.116 | 20.2 | 0.116 | 30.0 | 0.116 | 2.90 |
| Average | 6.45 | 169 | 6.45 | 142 | 6.45 | 74.4 |
| Stdev | 37.0 | 510 | 37.0 | 418 | 37.0 | 163 |
| p(t-test) | | 2.2E-19 | | 3.4E-19 | | 4.1E-6 |
| Min | 0.00269 | 0.0104 | 0.00269 | 0.00471 | 0.00269 | 0.00821 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 832 | 1930 | 832 | 1850 | 832 | 440 |
| n (Samp) | 1122 | 14 | 1122 | 19 | 1122 | 7 |
| n (Patient) | 362 | 14 | 362 | 19 | 362 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.79 | 0.77 | 0.81 | 0.81 | nd | 0.84 | 0.59 | nd | 0.68 |
| SE | 0.058 | 0.098 | 0.071 | 0.059 | nd | 0.058 | 0.095 | nd | 0.11 |
| p | 8.4E-7 | 0.0051 | 1.5E-5 | 2.0E-7 | nd | 6.1E-9 | 0.33 | nd | 0.12 |
| nCohort 1 | 1275 | 1339 | 1122 | 1275 | nd | 1122 | 1275 | nd | 1122 |
| nCohort 2 | 22 | 8 | 14 | 20 | nd | 19 | 10 | nd | 7 |
| Cutoff 1 | 2.00 | 2.57 | 2.86 | 2.60 | nd | 3.17 | 0.0109 | nd | 2.43 |
| Sens 1 | 73% | 75% | 71% | 70% | nd | 74% | 70% | nd | 71% |
| Spec 1 | 74% | 75% | 78% | 77% | nd | 79% | 26% | nd | 76% |
| Cutoff 2 | 0.362 | 2.00 | 0.362 | 1.19 | nd | 1.19 | 0.00930 | nd | 0.00930 |
| Sens 2 | 82% | 88% | 86% | 80% | nd | 84% | 80% | nd | 86% |
| Spec 2 | 55% | 71% | 54% | 67% | nd | 66% | 25% | nd | 23% |
| Cutoff 3 | 0.0163 | 0.0163 | 0.0155 | 0.0193 | nd | 0.0174 | 0.00789 | nd | 0.00789 |
| Sens 3 | 91% | 100% | 93% | 90% | nd | 95% | 90% | nd | 100% |
| Spec 3 | 45% | 44% | 42% | 47% | nd | 44% | 18% | nd | 16% |
| Cutoff 4 | 1.51 | 1.76 | 1.54 | 1.51 | nd | 1.54 | 1.51 | nd | 1.54 |
| Sens 4 | 73% | 88% | 71% | 75% | nd | 79% | 60% | nd | 71% |
| Spec 4 | 70% | 70% | 70% | 70% | nd | 70% | 70% | nd | 70% |
| Cutoff 5 | 3.37 | 3.89 | 3.44 | 3.37 | nd | 3.44 | 3.37 | nd | 3.44 |
| Sens 5 | 55% | 50% | 64% | 60% | nd | 68% | 30% | nd | 43% |
| Spec 5 | 80% | 80% | 80% | 80% | nd | 80% | 80% | nd | 80% |
| Cutoff 6 | 9.59 | 12.3 | 9.51 | 9.59 | nd | 9.51 | 9.59 | nd | 9.51 |
| Sens 6 | 41% | 12% | 57% | 55% | nd | 63% | 20% | nd | 29% |
| Spec 6 | 90% | 90% | 90% | 90% | nd | 90% | 90% | nd | 90% |
| OR Quart 2 | >4.0 | >1.0 | 1.0 | 2.0 | nd | 1.0 | 1.0 | nd | 0 |
| p Value | <0.21 | <1.0 | 1.0 | 0.57 | nd | 1.0 | 1.0 | nd | na |
| 95% CI of | >0.45 | >0.062 | 0.062 | 0.18 | nd | 0.062 | 0.14 | nd | na |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart2 | na | na | 16 | 22 | nd | 16 | 7.1 | nd | na |
| OR Quart 3 | >3.0 | >2.0 | 2.0 | 3.0 | nd | 3.0 | 0 | nd | 0 |
| p Value | <0.34 | <0.57 | 0.57 | 0.34 | nd | 0.34 | na | nd | na |
| 95% CI of | >0.31 | >0.18 | 0.18 | 0.31 | nd | 0.31 | na | nd | na |
| OR Quart3 | na | na | 22 | 29 | nd | 29 | na | nd | na |
| OR Quart 4 | >16 | >5.1 | 10 | 15 | nd | 15 | 3.0 | nd | 2.5 |
| p Value | <0.0079 | <0.14 | 0.026 | 0.0099 | nd | 0.0098 | 0.18 | nd | 0.27 |
| 95% CI of | >2.1 | >0.59 | 1.3 | 1.9 | nd | 1.9 | 0.61 | nd | 0.48 |
| OR Quart4 | na | na | 81 | 110 | nd | 110 | 15 | nd | 13 |

**Insulin-like growth factor-binding protein 3**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 392 | 551 | 392 | 670 | 392 | 609 |
| Average | 672 | 1350 | 672 | 1760 | 672 | 684 |
| Stdev | 1010 | 1580 | 1010 | 2850 | 1010 | 546 |
| p(t-test) | | 0.0012 | | 7.6E-7 | | 0.96 |
| Min | 0.0478 | 88.0 | 0.0478 | 98.3 | 0.0478 | 0.311 |
| Max | 12500 | 5660 | 12500 | 12500 | 12500 | 1550 |
| n (Samp) | 1385 | 24 | 1385 | 24 | 1385 | 13 |
| n (Patient) | 484 | 24 | 484 | 24 | 484 | 13 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 410 | 786 | 410 | 1560 | 410 | 862 |
| Average | 730 | 1260 | 730 | 2430 | 730 | 931 |
| Stdev | 1190 | 1440 | 1190 | 2880 | 1190 | 559 |
| p(t-test) | | 0.21 | | 5.4E-4 | | 0.65 |
| Min | 0.0478 | 88.0 | 0.0478 | 455 | 0.0478 | 267 |
| Max | 12500 | 4040 | 12500 | 8160 | 12500 | 1880 |
| n (Samp) | 1452 | 8 | 1452 | 6 | 1452 | 7 |

(continued)

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Patient) | 500 | 8 | 500 | 6 | 500 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 419 | 993 | 419 | 947 | 419 | 609 |
| Average | 701 | 2620 | 701 | 2080 | 701 | 753 |
| Stdev | 1080 | 3340 | 1080 | 3020 | 1080 | 673 |
| p(t-test) | | 1.0E-10 | | 4.5E-8 | | 0.90 |
| Min | 0.0478 | 119 | 0.0478 | 98.3 | 0.0478 | 65.9 |
| Max | 12500 | 12500 | 12500 | 12500 | 12500 | 1550 |
| n (Samp) | 1243 | 15 | 1243 | 21 | 1243 | 7 |
| n (Patient) | 397 | 15 | 397 | 21 | 397 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.63 | 0.61 | 0.71 | 0.66 | 0.82 | 0.69 | 0.56 | 0.70 | 0.55 |
| SE | 0.062 | 0.11 | 0.076 | 0.061 | 0.10 | 0.065 | 0.083 | 0.11 | 0.11 |
| p | 0.043 | 0.28 | 0.0067 | 0.0074 | 0.0021 | 0.0044 | 0.47 | 0.072 | 0.68 |
| nCohort 1 | 1385 | 1452 | 1243 | 1385 | 1452 | 1243 | 1385 | 1452 | 1243 |
| nCohort 2 | 24 | 8 | 15 | 24 | 6 | 21 | 13 | 7 | 7 |
| Cutoff 1 | 267 | 271 | 388 | 353 | 654 | 353 | 201 | 666 | 201 |
| Sens 1 | 71% | 75% | 73% | 71% | 83% | 71% | 77% | 71% | 71% |
| Spec 1 | 39% | 39% | 48% | 47% | 66% | 45% | 31% | 67% | 29% |
| Cutoff 2 | 185 | 108 | 259 | 245 | 654 | 313 | 77.5 | 395 | 77.5 |
| Sens 2 | 83% | 88% | 80% | 83% | 83% | 81% | 85% | 86% | 86% |
| Spec 2 | 30% | 20% | 36% | 37% | 66% | 41% | 15% | 49% | 13% |
| Cutoff 3 | 108 | 87.7 | 185 | 149 | 455 | 174 | 65.8 | 267 | 65.8 |
| Sens 3 | 92% | 100% | 93% | 92% | 100% | 90% | 92% | 100% | 100% |
| Spec 3 | 20% | 16% | 27% | 25% | 54% | 26% | 13% | 38% | 11% |
| Cutoff 4 | 726 | 751 | 750 | 726 | 751 | 750 | 726 | 751 | 750 |
| Sens 4 | 46% | 50% | 60% | 46% | 67% | 52% | 38% | 57% | 43% |
| Spec 4 | 70% | 70% | 70% | 70% | 70% | 70% | 70% | 70% | 70% |
| Cutoff 5 | 1020 | 1070 | 1040 | 1020 | 1070 | 1040 | 1020 | 1070 | 1040 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 5 | 33% | 38% | 47% | 42% | 67% | 48% | 31% | 43% | 43% |
| Spec 5 | 80% | 80% | 80% | 80% | 80% | 80% | 80% | 80% | 80% |
| Cutoff 6 | 1470 | 1560 | 1470 | 1470 | 1560 | 1470 | 1470 | 1560 | 1470 |
| Sens 6 | 33% | 25% | 47% | 33% | 50% | 43% | 15% | 14% | 29% |
| Spec 6 | 90% | 90% | 90% | 90% | 90% | 90% | 90% | 90% | 90% |
| OR Quart 2 | 1.5 | 0.50 | 4.0 | 1.7 | >0 | 2.5 | 0.33 | >2.0 | 0.50 |
| p Value | 0.53 | 0.57 | 0.21 | 0.48 | <na | 0.27 | 0.34 | <0.57 | 0.57 |
| 95% CI of | 0.42 | 0.045 | 0.45 | 0.40 | >na | 0.49 | 0.034 | >0.18 | 0.045 |
| OR Quart2 | 5.4 | 5.5 | 36 | 7.1 | na | 13 | 3.2 | na | 5.5 |
| OR Quart 3 | 0.75 | 0.50 | 1.0 | 1.7 | >2.0 | 1.5 | 1.3 | >2.0 | 0.50 |
| p Value | 0.71 | 0.57 | 1.0 | 0.48 | <0.57 | 0.66 | 0.70 | <0.57 | 0.57 |
| 95% CI of | 0.17 | 0.045 | 0.062 | 0.40 | >0.18 | 0.25 | 0.30 | >0.18 | 0.045 |
| OR Quart3 | 3.4 | 5.5 | 16 | 7.1 | na | 9.1 | 6.0 | na | 5.5 |
| OR Quart 4 | 2.8 | 2.0 | 9.2 | 3.7 | >4.0 | 5.7 | 1.7 | >3.0 | 1.5 |
| p Value | 0.081 | 0.42 | 0.036 | 0.044 | <0.21 | 0.025 | 0.48 | <0.34 | 0.66 |
| 95% CI of | 0.88 | 0.37 | 1.2 | 1.0 | >0.45 | 1.2 | 0.40 | >0.31 | 0.25 |
| OR Quart4 | 8.9 | 11 | 73 | 14 | na | 26 | 7.0 | na | 9.0 |

**Immunoglogulin G1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3300 | 10100 | 3300 | 13900 | 3300 | 5060 |
| Average | 5860 | 12600 | 5860 | 16000 | 5860 | 7690 |
| Stdev | 6930 | 9230 | 6930 | 16500 | 6930 | 7590 |
| p(t-test) | | 7.5E-6 | | 3.8E-10 | | 0.40 |
| Min | 3.36 | 980 | 3.36 | 1440 | 3.36 | 55.9 |
| Max | 80000 | 35700 | 80000 | 80000 | 80000 | 25300 |
| n (Samp) | 1268 | 22 | 1268 | 20 | 1268 | 10 |
| n (Patient) | 449 | 22 | 449 | 20 | 449 | 10 |

(continued)

| Immunoglogulin G1 | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 3480 | 10000 | nd | nd | nd | nd |
| Average | 6270 | 12600 | nd | nd | nd | nd |
| Stdev | 7490 | 9250 | nd | nd | nd | nd |
| p(t-test) | | 0.018 | nd | nd | nd | nd |
| Min | 3.36 | 980 | nd | nd | nd | nd |
| Max | 80000 | 28300 | nd | nd | nd | nd |
| n (Samp) | 1332 | 8 | nd | nd | nd | nd |
| n (Patient) | 464 | 8 | nd | nd | nd | nd |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 3420 | 11700 | 3420 | 14900 | 3420 | 5570 |
| Average | 5990 | 13400 | 5990 | 18400 | 5990 | 9330 |
| Stdev | 7020 | 9250 | 7020 | 17000 | 7020 | 8180 |
| p(t-test) | | 9.1E-5 | | 2.9E-13 | | 0.21 |
| Min | 3.36 | 1880 | 3.36 | 1440 | 3.36 | 2380 |
| Max | 80000 | 35700 | 80000 | 80000 | 80000 | 25300 |
| n(Samp) | 1114 | 14 | 1114 | 19 | 1114 | 7 |
| n (Patient) | 359 | 14 | 359 | 19 | 359 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.76 | 0.74 | 0.78 | 0.81 | nd | 0.84 | 0.60 | nd | 0.69 |
| SE | 0.060 | 0.10 | 0.074 | 0.059 | nd | 0.057 | 0.095 | nd | 0.11 |
| p | 1.2E-5 | 0.017 | 1.4E-4 | 1.5E-7 | nd | 2.0E-9 | 0.30 | nd | 0.086 |
| nCohort 1 | 1268 | 1332 | 1114 | 1268 | nd | 1114 | 1268 | nd | 1114 |
| nCohort 2 | 22 | 8 | 14 | 20 | nd | 19 | 10 | nd | 7 |
| Cutoff 1 | 6900 | 6900 | 8550 | 9500 | nd | 9500 | 3900 | nd | 4550 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 1 | 73% | 75% | 71% | 70% | nd | 74% | 70% | nd | 71% |
| Spec 1 | 72% | 70% | 78% | 81% | nd | 81% | 56% | nd | 61% |
| Cutoff 2 | 5190 | 5190 | 4150 | 7030 | nd | 7030 | 2380 | nd | 3900 |
| Sens 2 | 82% | 88% | 86% | 80% | nd | 84% | 80% | nd | 86% |
| Spec 2 | 65% | 63% | 57% | 73% | nd | 72% | 37% | nd | 55% |
| Cutoff 3 | 1990 | 977 | 1990 | 4740 | nd | 4740 | 1740 | nd | 2380 |
| Sens 3 | 91% | 100% | 93% | 90% | nd | 95% | 90% | nd | 100% |
| Spec 3 | 31% | 12% | 30% | 63% | nd | 62% | 26% | nd | 36% |
| Cutoff 4 | 6240 | 6760 | 6490 | 6240 | nd | 6490 | 6240 | nd | 6490 |
| Sens 4 | 77% | 75% | 79% | 80% | nd | 84% | 40% | nd | 43% |
| Spec 4 | 70% | 70% | 70% | 70% | nd | 70% | 70% | nd | 70% |
| Cutoff 5 | 8950 | 9780 | 9100 | 8950 | nd | 9100 | 8950 | nd | 9100 |
| Sens 5 | 55% | 50% | 64% | 70% | nd | 74% | 30% | nd | 29% |
| Spec 5 | 80% | 80% | 80% | 80% | nd | 80% | 80% | nd | 80% |
| Cutoff 6 | 14200 | 15400 | 14300 | 14200 | nd | 14300 | 14200 | nd | 14300 |
| Sens 6 | 36% | 38% | 43% | 50% | nd | 58% | 20% | nd | 29% |
| Spec 6 | 90% | 90% | 90% | 90% | nd | 90% | 90% | nd | 90% |
| OR Quart 2 | 2.0 | 0 | >2.0 | 1.0 | nd | 0 | 2.0 | nd | >1.0 |
| p Value | 0.57 | na | <0.57 | 1.0 | nd | na | 0.57 | nd | <1.00 |
| 95% CI of | 0.18 | na | >0.18 | 0.062 | nd | na | 0.18 | nd | >0.062 |
| OR Quart2 | 22 | na | na | 16 | nd | na | 22 | nd | na |
| OR Quart 3 | 4.0 | 2.0 | >1.0 | 3.0 | nd | 3.0 | 3.0 | nd | >3.0 |
| p Value | 0.21 | 0.57 | <1.00 | 0.34 | nd | 0.34 | 0.34 | nd | <0.34 |
| 95% CI of | 0.45 | 0.18 | >0.062 | 0.31 | nd | 0.31 | 0.31 | nd | >0.31 |
| OR Quart3 | 36 | 22 | na | 29 | nd | 29 | 29 | nd | na |
| OR Quart 4 | 16 | 5.1 | >11 | 16 | nd | 16 | 4.0 | nd | >3.0 |
| p Value | 0.0080 | 0.14 | <0.020 | 0.0079 | nd | 0.0078 | 0.21 | nd | <0.34 |
| 95% CI of | 2.1 | 0.59 | >1.5 | 2.1 | nd | 2.1 | 0.45 | nd | >0.31 |
| OR Quart4 | 120 | 44 | na | 120 | nd | 120 | 36 | nd | na |

(continued)

| Immunoglogulin G2 | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 9080 | 24500 | 9080 | 31300 | 9080 | 11300 |
| Average | 18400 | 51400 | 18400 | 57600 | 18400 | 42800 |
| Stdev | 31600 | 63700 | 31600 | 80400 | 31600 | 73900 |
| p(t-test) | | 2.5E-6 | | 1.5E-7 | | 0.017 |
| Min | 119 | 2380 | 119 | 3940 | 119 | 25.4 |
| Max | 240000 | 240000 | 240000 | 240000 | 240000 | 240000 |
| n (Samp) | 1268 | 22 | 1268 | 20 | 1268 | 10 |
| n (Patient) | 449 | 22 | 449 | 20 | 449 | 10 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 9340 | 24600 | nd | nd | nd | nd |
| Average | 20400 | 55100 | nd | nd | nd | nd |
| Stdev | 36100 | 62200 | nd | nd | nd | nd |
| p(t-test) | | 0.0071 | nd | nd | nd | nd |
| Min | 119 | 2380 | nd | nd | nd | nd |
| Max | 240000 | 182000 | nd | nd | nd | nd |
| n (Samp) | 1332 | 8 | nd | nd | nd | nd |
| n (Patient) | 464 | 8 | nd | nd | nd | nd |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 9630 | 24500 | 9630 | 33000 | 9630 | 13800 |
| Average | 18500 | 63500 | 18500 | 76200 | 18500 | 52800 |
| Stdev | 30500 | 81000 | 30500 | 89800 | 30500 | 86800 |
| p(t-test) | | 1.5E-7 | | 2.9E-14 | | 0.0038 |
| Min | 25.4 | 4460 | 25.4 | 3940 | 25.4 | 2320 |
| Max | 240000 | 240000 | 240000 | 240000 | 240000 | 240000 |
| n (Samp) | 1114 | 14 | 1114 | 19 | 1114 | 7 |
| n (Patient) | 359 | 14 | 359 | 19 | 359 | 7 |
| | | | | | | |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.71 | 0.73 | 0.71 | 0.74 | nd | 0.77 | 0.57 | nd | 0.61 |
| SE | 0.063 | 0.10 | 0.078 | 0.064 | nd | 0.064 | 0.095 | nd | 0.11 |
| p | 0.0011 | 0.023 | 0.0064 | 1.5E-4 | nd | 1.9E-5 | 0.48 | nd | 0.32 |
| nCohort 1 | 1268 | 1332 | 1114 | 1268 | nd | 1114 | 1268 | nd | 1114 |
| nCohort 2 | 22 | 8 | 14 | 20 | nd | 19 | 10 | nd | 7 |
| Cutoff 1 | 11800 | 13700 | 13700 | 14100 | nd | 14100 | 8540 | nd | 8840 |
| Sens 1 | 73% | 75% | 71% | 70% | nd | 74% | 70% | nd | 71% |
| Spec 1 | 60% | 65% | 65% | 67% | nd | 66% | 47% | nd | 47% |
| Cutoff 2 | 5910 | 11800 | 5910 | 12400 | nd | 12400 | 6890 | nd | 8540 |
| Sens 2 | 82% | 88% | 86% | 80% | nd | 84% | 80% | nd | 86% |
| Spec 2 | 31% | 59% | 29% | 63% | nd | 61% | 37% | nd | 45% |
| Cutoff 3 | 4910 | 2370 | 5180 | 5300 | nd | 5300 | 2270 | nd | 2270 |
| Sens 3 | 91% | 100% | 93% | 90% | nd | 95% | 90% | nd | 100% |
| Spec 3 | 25% | 8% | 24% | 27% | nd | 25% | 9% | nd | 8% |
| Cutoff 4 | 15200 | 16200 | 16000 | 15200 | nd | 16000 | 15200 | nd | 16000 |
| Sens 4 | 59% | 62% | 57% | 60% | nd | 63% | 30% | nd | 29% |
| Spec 4 | 70% | 70% | 70% | 70% | nd | 70% | 70% | nd | 70% |
| Cutoff 5 | 21200 | 22500 | 21800 | 21200 | nd | 21800 | 21200 | nd | 21800 |
| Sens 5 | 59% | 62% | 57% | 55% | nd | 58% | 30% | nd | 29% |
| Spec 5 | 80% | 80% | 80% | 80% | nd | 80% | 80% | nd | 80% |
| Cutoff 6 | 37600 | 41400 | 37600 | 37600 | nd | 37600 | 37600 | nd | 37600 |
| Sens 6 | 36% | 38% | 43% | 30% | nd | 42% | 30% | nd | 29% |
| Spec 6 | 90% | 90% | 90% | 90% | nd | 90% | 90% | nd | 90% |
| OR Quart 2 | 0.66 | 0 | 0.50 | 1.0 | nd | 2.0 | 1.5 | nd | 2.0 |
| p Value | 0.65 | na | 0.57 | 1.0 | nd | 0.57 | 0.66 | nd | 0.57 |
| 95% CI of | 0.11 | na | 0.045 | 0.14 | nd | 0.18 | 0.25 | nd | 0.18 |
| OR Quart2 | 4.0 | na | 5.5 | 7.1 | nd | 22 | 9.0 | nd | 22 |
| OR Quart 3 | 1.3 | 2.0 | 1.5 | 2.0 | nd | 4.0 | 1.0 | nd | 2.0 |
| p Value | 0.70 | 0.57 | 0.66 | 0.42 | nd | 0.21 | 1.0 | nd | 0.57 |
| 95% CI of | 0.30 | 0.18 | 0.25 | 0.37 | nd | 0.45 | 0.14 | nd | 0.18 |

(continued)

|  | 0hr prior to AKI stage | | | 24hr prior to AKI stage | | | 48hr prior to AKI stage | | |
| --- | --- | --- | --- | --- | --- | --- | --- | --- | --- |
|  | sCr or UO | sCr only | UO only | sCr or UO | sCr only | UO only | sCr or UO | sCr only | UO only |
| OR Quart3 | 6.0 | 22 | 9.1 | 11 | nd | 36 | 7.1 | nd | 22 |
| OR Quart 4 | 4.5 | 5.1 | 4.1 | 6.2 | nd | 12 | 1.5 | nd | 2.0 |
| p Value | 0.021 | 0.14 | 0.077 | 0.018 | nd | 0.016 | 0.66 | nd | 0.57 |
| 95% CI of | 1.3 | 0.59 | 0.86 | 1.4 | nd | 1.6 | 0.25 | nd | 0.18 |
| OR Quart4 | 16 | 44 | 19 | 28 | nd | 96 | 9.0 | nd | 22 |

**Interleukin-11**

| sCr or UO | 0hr prior to AKI stage | | 24hr prior to AKI stage | | 48hr prior to AKI stage | |
| --- | --- | --- | --- | --- | --- | --- |
|  | Cohort 1 | Cohort 2 | Cohort 1 | Cohort 2 | Cohort 1 | Cohort 2 |
| Median | 133 | 222 | 133 | 253 | 133 | 93.2 |
| Average | 212 | 502 | 212 | 422 | 212 | 327 |
| Stdev | 234 | 693 | 234 | 570 | 234 | 645 |
| p(t-test) |  | 6.8E-8 |  | 1.8E-4 |  | 0.13 |
| Min | 0.0822 | 48.0 | 0.0822 | 49.5 | 0.0822 | 14.5 |
| Max | 2260 | 2900 | 2260 | 2590 | 2260 | 2140 |
| n (Samp) | 1275 | 22 | 1275 | 19 | 1275 | 10 |
| n (Patient) | 452 | 22 | 452 | 19 | 452 | 10 |

| sCr only | 0hr prior to AKI stage | | 24hr prior to AKI stage | | 48hr prior to AKI stage | |
| --- | --- | --- | --- | --- | --- | --- |
|  | Cohort 1 | Cohort 2 | Cohort 1 | Cohort 2 | Cohort 1 | Cohort 2 |
| Median | 137 | 124 | nd | nd | nd | nd |
| Average | 231 | 276 | nd | nd | nd | nd |
| Stdev | 302 | 348 | nd | nd | nd | nd |
| p(t-test) |  | 0.68 | nd | nd | nd | nd |
| Min | 0.0822 | 48.0 | nd | nd | nd | nd |
| Max | 3780 | 1040 | nd | nd | nd | nd |
| n (Samp) | 1338 | 8 | nd | nd | nd | nd |
| n (Patient) | 467 | 8 | nd | nd | nd | nd |

| UO only | 0hr prior to AKI stage | | 24hr prior to AKI stage | | 48hr prior to AKI stage | |
| --- | --- | --- | --- | --- | --- | --- |
|  | Cohort 1 | Cohort 2 | Cohort 1 | Cohort 2 | Cohort 1 | Cohort 2 |
| Median | 142 | 384 | 142 | 255 | 142 | 95.5 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 220 | 588 | 220 | 490 | 220 | 430 |
| Stdev | 238 | 740 | 238 | 623 | 238 | 763 |
| p(t-test) | | 5.4E-8 | | 5.2E-6 | | 0.023 |
| Min | 0.0822 | 116 | 0.0822 | 49.5 | 0.0822 | 14.5 |
| Max | 2260 | 2900 | 2260 | 2590 | 2260 | 2140 |
| n(Samp) | 1121 | 14 | 1121 | 18 | 1121 | 7 |
| n (Patient) | 362 | 14 | 362 | 18 | 362 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.68 | 0.51 | 0.75 | 0.69 | nd | 0.69 | 0.46 | nd | 0.50 |
| SE | 0.064 | 0.10 | 0.076 | 0.068 | nd | 0.070 | 0.094 | nd | 0.11 |
| p | 0.0046 | 0.93 | 0.0012 | 0.0043 | nd | 0.0068 | 0.63 | nd | 0.98 |
| nCohort 1 | 1275 | 1338 | 1121 | 1275 | nd | 1121 | 1275 | nd | 1121 |
| nCohort 2 | 22 | 8 | 14 | 19 | nd | 18 | 10 | nd | 7 |
| Cutoff 1 | 131 | 87.1 | 158 | 181 | nd | 179 | 88.1 | nd | 90.5 |
| Sens 1 | 73% | 75% | 71% | 74% | nd | 72% | 70% | nd | 71% |
| Spec 1 | 49% | 32% | 55% | 63% | nd | 60% | 33% | nd | 31% |
| Cutoff 2 | 115 | 76.3 | 131 | 135 | nd | 135 | 85.7 | nd | 85.7 |
| Sens 2 | 82% | 88% | 86% | 84% | nd | 83% | 80% | nd | 86% |
| Spec 2 | 44% | 26% | 46% | 51% | nd | 48% | 32% | nd | 29% |
| Cutoff 3 | 87.1 | 47.7 | 128 | 79.7 | nd | 79.7 | 66.2 | nd | 13.5 |
| Sens 3 | 91% | 100% | 93% | 95% | nd | 94% | 90% | nd | 100% |
| Spec 3 | 32% | 13% | 46% | 29% | nd | 27% | 23% | nd | 1% |
| Cutoff 4 | 226 | 235 | 235 | 226 | nd | 235 | 226 | nd | 235 |
| Sens 4 | 50% | 25% | 57% | 58% | nd | 61% | 30% | nd | 43% |
| Spec 4 | 70% | 70% | 70% | 70% | nd | 70% | 70% | nd | 70% |
| Cutoff 5 | 312 | 329 | 319 | 312 | nd | 319 | 312 | nd | 319 |
| Sens 5 | 45% | 25% | 57% | 32% | nd | 33% | 20% | nd | 29% |
| Spec 5 | 80% | 80% | 80% | 80% | nd | 80% | 80% | nd | 80% |
| Cutoff 6 | 469 | 503 | 474 | 469 | nd | 474 | 469 | nd | 474 |
| Sens 6 | 32% | 25% | 43% | 21% | nd | 28% | 10% | nd | 14% |
| Spec 6 | 90% | 90% | 90% | 90% | nd | 90% | 90% | nd | 90% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart 2 | 6.1 | 4.0 | >3.0 | 2.0 | nd | 4.0 | 0.50 | nd | 0.50 |
| p Value | 0.095 | 0.21 | <0.34 | 0.57 | nd | 0.21 | 0.57 | nd | 0.57 |
| 95% CI of | 0.73 | 0.45 | >0.31 | 0.18 | nd | 0.45 | 0.045 | nd | 0.045 |
| OR Quart2 | 51 | 36 | na | 22 | nd | 36 | 5.5 | nd | 5.5 |
| OR Quart 3 | 5.1 | 1.0 | >3.0 | 8.2 | nd | 5.1 | 2.5 | nd | 1.5 |
| p Value | 0.14 | 1.0 | <0.34 | 0.048 | nd | 0.14 | 0.27 | nd | 0.66 |
| 95% CI of | 0.59 | 0.062 | >0.31 | 1.0 | nd | 0.59 | 0.49 | nd | 0.25 |
| OR Quart3 | 44 | 16 | na | 66 | nd | 44 | 13 | nd | 9.1 |
| OR Quart 4 | 10 | 2.0 | >8.2 | 8.2 | nd | 8.2 | 1.0 | nd | 0.50 |
| p Value | 0.027 | 0.57 | <0.048 | 0.049 | nd | 0.048 | 1.00 | nd | 0.57 |
| 95% CI of | 1.3 | 0.18 | >1.0 | 1.0 | nd | 1.0 | 0.14 | nd | 0.045 |
| OR Quart4 | 81 | 22 | na | 66 | nd | 66 | 7.2 | nd | 5.5 |

**Interleukin-2 receptor alpha chain**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 573 | 935 | 573 | 1390 | 573 | 770 |
| Average | 1010 | 1690 | 1010 | 2110 | 1010 | 1620 |
| Stdev | 1270 | 1810 | 1270 | 2430 | 1270 | 2400 |
| p(t-test) | | 0.014 | | 1.6E-4 | | 0.13 |
| Min | 0.0317 | 0.118 | 0.0317 | 50.9 | 0.0317 | 0.115 |
| Max | 10400 | 6080 | 10400 | 9090 | 10400 | 7740 |
| n (Samp) | 1277 | 22 | 1277 | 20 | 1277 | 10 |
| n (Patient) | 452 | 22 | 452 | 20 | 452 | 10 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 609 | 485 | nd | nd | nd | nd |
| Average | 1090 | 1290 | nd | nd | nd | nd |

(continued)

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | 1420 | 2030 | nd | nd | nd | nd |
| p(t-test) | | 0.70 | nd | nd | nd | nd |
| Min | 0.0317 | 0.118 | nd | nd | nd | nd |
| Max | 10400 | 6080 | nd | nd | nd | nd |
| n (Samp) | 1341 | 8 | nd | nd | nd | nd |
| n (Patient) | 467 | 8 | nd | nd | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 624 | 1540 | 624 | 1700 | 624 | 819 |
| Average | 1050 | 2130 | 1050 | 2300 | 1050 | 2120 |
| Stdev | 1280 | 1940 | 1280 | 2450 | 1280 | 2740 |
| p(t-test) | | 0.0020 | | 3.7E-5 | | 0.029 |
| Min | 0.0317 | 54.4 | 0.0317 | 50.9 | 0.0317 | 19.9 |
| Max | 10400 | 6080 | 10400 | 9090 | 10400 | 7740 |
| n(Samp) | 1124 | 14 | 1124 | 19 | 1124 | 7 |
| n (Patient) | 362 | 14 | 362 | 19 | 362 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.59 | 0.47 | 0.66 | 0.65 | nd | 0.68 | 0.52 | nd | 0.58 |
| SE | 0.064 | 0.10 | 0.080 | 0.067 | nd | 0.068 | 0.093 | nd | 0.11 |
| p | 0.16 | 0.78 | 0.052 | 0.021 | nd | 0.0078 | 0.86 | nd | 0.46 |
| nCohort 1 | 1277 | 1341 | 1124 | 1277 | nd | 1124 | 1277 | nd | 1124 |
| nCohort 2 | 22 | 8 | 14 | 20 | nd | 19 | 10 | nd | 7 |
| Cutoff 1 | 350 | 189 | 477 | 761 | nd | 761 | 227 | nd | 718 |
| Sens 1 | 73% | 75% | 71% | 70% | nd | 74% | 70% | nd | 71% |
| Spec 1 | 37% | 24% | 42% | 57% | nd | 56% | 28% | nd | 54% |
| Cutoff 2 | 189 | 60.5 | 277 | 385 | nd | 385 | 35.7 | nd | 35.7 |
| Sens 2 | 82% | 88% | 86% | 80% | nd | 84% | 80% | nd | 86% |
| Spec 2 | 25% | 12% | 30% | 39% | nd | 37% | 10% | nd | 9% |
| Cutoff 3 | 60.5 | 0.116 | 89.0 | 69.2 | nd | 68.0 | 18.6 | nd | 18.6 |
| Sens 3 | 91% | 100% | 93% | 90% | nd | 95% | 90% | nd | 100% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 3 | 13% | 2% | 13% | 14% | nd | 12% | 8% | nd | 7% |
| Cutoff 4 | 1110 | 1160 | 1140 | 1110 | nd | 1140 | 1110 | nd | 1140 |
| Sens 4 | 45% | 38% | 57% | 55% | nd | 58% | 40% | nd | 43% |
| Spec 4 | 70% | 70% | 70% | 70% | nd | 70% | 70% | nd | 70% |
| Cutoff 5 | 1620 | 1760 | 1670 | 1620 | nd | 1670 | 1620 | nd | 1670 |
| Sens 5 | 36% | 12% | 50% | 45% | nd | 53% | 30% | nd | 43% |
| Spec 5 | 80% | 80% | 80% | 80% | nd | 80% | 80% | nd | 80% |
| Cutoff 6 | 2420 | 2620 | 2480 | 2420 | nd | 2480 | 2420 | nd | 2480 |
| Sens 6 | 32% | 12% | 43% | 30% | nd | 37% | 20% | nd | 29% |
| Spec 6 | 90% | 90% | 90% | 90% | nd | 90% | 90% | nd | 90% |
| OR Quart 2 | 1.00 | 0.50 | 1.5 | 0.50 | nd | 0.66 | 0.33 | nd | 0 |
| p Value | 1.00 | 0.57 | 0.66 | 0.42 | nd | 0.65 | 0.34 | nd | na |
| 95% CI of | 0.29 | 0.045 | 0.25 | 0.090 | nd | 0.11 | 0.034 | nd | na |
| OR Quart2 | 3.5 | 5.5 | 9.0 | 2.7 | nd | 4.0 | 3.2 | nd | na |
| OR Quart 3 | 0.80 | 1.0 | 1.0 | 1.0 | nd | 1.3 | 1.00 | nd | 1.00 |
| p Value | 0.73 | 1.00 | 1.0 | 1.0 | nd | 0.71 | 1.00 | nd | 1.00 |
| 95% CI of | 0.21 | 0.14 | 0.14 | 0.25 | nd | 0.30 | 0.20 | nd | 0.14 |
| OR Quart3 | 3.0 | 7.2 | 7.1 | 4.0 | nd | 6.0 | 5.0 | nd | 7.1 |
| OR Quart 4 | 1.6 | 1.5 | 3.6 | 2.5 | nd | 3.4 | 1.00 | nd | 1.5 |
| p Value | 0.41 | 0.65 | 0.12 | 0.12 | nd | 0.065 | 1.00 | nd | 0.66 |
| 95% CI of | 0.52 | 0.25 | 0.73 | 0.79 | nd | 0.93 | 0.20 | nd | 0.25 |
| OR Quart4 | 5.0 | 9.1 | 17 | 8.2 | nd | 13 | 5.0 | nd | 9.0 |

| **Neutrophil collagenase** | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 3540 | 13400 | 3540 | 13400 | 3540 | 3260 |
| Average | 15000 | 81600 | 15000 | 38600 | 15000 | 11700 |
| Stdev | 41900 | 159000 | 41900 | 49400 | 41900 | 18300 |
| p(t-test) | | 4.3E-12 | | 0.0066 | | 0.78 |

(continued)

| Neutrophil collagenase | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Min | 0.114 | 0.260 | 0.114 | 25.8 | 0.114 | 212 |
| Max | 670000 | 649000 | 670000 | 166000 | 670000 | 66900 |
| n (Samp) | 1382 | 24 | 1382 | 24 | 1382 | 13 |
| n (Patient) | 485 | 24 | 485 | 24 | 485 | 13 |
| | | | | | | |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 3770 | 5520 | 3770 | 14100 | 3770 | 10800 |
| Average | 17100 | 99700 | 17100 | 97000 | 17100 | 10600 |
| Stdev | 47900 | 225000 | 47900 | 160000 | 47900 | 8750 |
| p(t-test) | | 3.9E-6 | | 6.4E-5 | | 0.72 |
| Min | 0.114 | 0.260 | 0.114 | 25.8 | 0.114 | 1120 |
| Max | 670000 | 649000 | 670000 | 401000 | 670000 | 21900 |
| n (Samp) | 1449 | 8 | 1449 | 6 | 1449 | 7 |
| n (Patient) | 501 | 8 | 501 | 6 | 501 | 7 |
| | | | | | | |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 3590 | 22500 | 3590 | 20500 | 3590 | 3260 |
| Average | 14600 | 80500 | 14600 | 61600 | 14600 | 13500 |
| Stdev | 38300 | 147000 | 38300 | 82900 | 38300 | 24100 |
| p(t-test) | | 9.7E-10 | | 7.0E-8 | | 0.94 |
| Min | 0.114 | 1660 | 0.114 | 551 | 0.114 | 576 |
| Max | 670000 | 561000 | 670000 | 327000 | 670000 | 66900 |
| n (Samp) | 1240 | 15 | 1240 | 21 | 1240 | 7 |
| n (Patient) | 398 | 15 | 398 | 21 | 398 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.68 | 0.55 | 0.76 | 0.72 | 0.61 | 0.77 | 0.51 | 0.61 | 0.49 |
| SE | 0.061 | 0.11 | 0.072 | 0.060 | 0.12 | 0.061 | 0.081 | 0.11 | 0.11 |
| p | 0.0024 | 0.64 | 2.5E-4 | 3.1E-4 | 0.38 | 6.5E-6 | 0.90 | 0.34 | 0.95 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| nCohort 1 | 1382 | 1449 | 1240 | 1382 | 1449 | 1240 | 1382 | 1449 | 1240 |
| nCohort 2 | 24 | 8 | 15 | 24 | 6 | 21 | 13 | 7 | 7 |
| Cutoff 1 | 5080 | 3020 | 8450 | 7290 | 978 | 10400 | 1120 | 2970 | 1430 |
| Sens 1 | 71% | 75% | 73% | 71% | 83% | 71% | 77% | 71% | 71% |
| Spec 1 | 58% | 45% | 69% | 65% | 22% | 74% | 25% | 44% | 29% |
| Cutoff 2 | 2970 | 127 | 5080 | 3090 | 978 | 7290 | 576 | 2660 | 576 |
| Sens 2 | 83% | 88% | 80% | 83% | 83% | 81% | 85% | 86% | 86% |
| Spec 2 | 46% | 3% | 58% | 46% | 22% | 65% | 15% | 42% | 15% |
| Cutoff 3 | 1650 | 0.159 | 2030 | 2450 | 23.8 | 2660 | 571 | 1120 | 571 |
| Sens 3 | 92% | 100% | 93% | 92% | 100% | 90% | 92% | 100% | 100% |
| Spec 3 | 34% | 0% | 36% | 42% | 1% | 43% | 15% | 24% | 15% |
| Cutoff 4 | 8980 | 9730 | 8740 | 8980 | 9730 | 8740 | 8980 | 9730 | 8740 |
| Sens 4 | 54% | 38% | 67% | 62% | 50% | 71% | 38% | 57% | 29% |
| Spec 4 | 70% | 70% | 70% | 70% | 70% | 70% | 70% | 70% | 70% |
| Cutoff 5 | 15600 | 17000 | 15100 | 15600 | 17000 | 15100 | 15600 | 17000 | 15100 |
| Sens 5 | 46% | 25% | 53% | 46% | 50% | 52% | 23% | 29% | 14% |
| Spec 5 | 80% | 80% | 80% | 80% | 80% | 80% | 80% | 80% | 80% |
| Cutoff 6 | 32700 | 36000 | 32700 | 32700 | 36000 | 32700 | 32700 | 36000 | 32700 |
| Sens 6 | 33% | 25% | 40% | 33% | 33% | 43% | 8% | 0% | 14% |
| Spec 6 | 90% | 90% | 90% | 90% | 90% | 90% | 90% | 90% | 90% |
| OR Quart 2 | 2.5 | 0.50 | >3.0 | 2.0 | 0 | 3.0 | 0.75 | 2.0 | 0.50 |
| p Value | 0.27 | 0.57 | <0.34 | 0.42 | na | 0.34 | 0.70 | 0.57 | 0.57 |
| 95% CI of | 0.48 | 0.045 | >0.31 | 0.36 | na | 0.31 | 0.17 | 0.18 | 0.045 |
| OR Quart2 | 13 | 5.5 | na | 11 | na | 29 | 3.4 | 22 | 5.5 |
| OR Quart 3 | 2.0 | 1.0 | >2.0 | 2.0 | 0.50 | 3.0 | 0.50 | 1.0 | 1.0 |
| p Value | 0.42 | 1.0 | <0.57 | 0.42 | 0.57 | 0.34 | 0.42 | 1.0 | 1.0 |
| 95% CI of | 0.37 | 0.14 | >0.18 | 0.37 | 0.045 | 0.31 | 0.090 | 0.062 | 0.14 |
| OR Quart3 | 11 | 7.1 | na | 11 | 5.5 | 29 | 2.7 | 16 | 7.1 |
| OR Quart 4 | 6.7 | 1.5 | >10 | 7.2 | 1.5 | 15 | 1.00 | 3.0 | 1.0 |
| Value | 0.013 | 0.66 | <0.027 | 0.0092 | 0.66 | 0.0099 | 1.00 | 0.34 | 1.00 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of | 1.5 | 0.25 | >1.3 | 1.6 | 0.25 | 1.9 | 0.25 | 0.31 | 0.14 |
| OR Quart4 | 30 | 9.0 | na | 32 | 9.0 | 110 | 4.0 | 29 | 7.2 |
| **Protransforming growth factor alpha** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 6.25 | | 13.8 | 6.25 | | 12.5 | 6.25 | | 13.5 |
| Average | 10.9 | | 20.9 | 10.9 | | 20.1 | 10.9 | | 14.9 |
| Stdev | 21.2 | | 26.1 | 21.2 | | 16.0 | 21.2 | | 12.1 |
| p(t-test) | | | 0.028 | | | 0.051 | | | 0.54 |
| Min | 0.00184 | | 0.781 | 0.00184 | | 3.26 | 0.00184 | | 0.00454 |
| Max | 361 | | 122 | 361 | | 50.4 | 361 | | 36.0 |
| n (Samp) | 1271 | | 22 | 1271 | | 20 | 1271 | | 10 |
| n (Patient) | 452 | | 22 | 452 | | 20 | 452 | | 10 |
| | | | | | | | | | |
| sCr only | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 6.57 | | 13.7 | nd | | nd | nd | | nd |
| Average | 11.3 | | 12.4 | nd | | nd | nd | | nd |
| Stdev | 21.2 | | 5.27 | nd | | nd | nd | | nd |
| p(t-test) | | | 0.89 | nd | | nd | nd | | nd |
| Min | 0.00184 | | 1.41 | nd | | nd | nd | | nd |
| Max | 361 | | 18.0 | nd | | nd | nd | | nd |
| n (Samp) | 1335 | | 8 | nd | | nd | nd | | nd |
| n (Patient) | 467 | | 8 | nd | | nd | nd | | nd |
| | | | | | | | | | |
| UO only | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 6.50 | | 16.0 | 6.50 | | 17.8 | 6.50 | | 14.4 |
| Average | 11.3 | | 21.8 | 11.3 | | 21.7 | 11.3 | | 17.6 |
| Stdev | 22.3 | | 19.7 | 22.3 | | 15.6 | 22.3 | | 13.1 |
| p(t-test) | | | 0.080 | | | 0.044 | | | 0.45 |
| Min | 0.00184 | | 0.781 | 0.00184 | | 3.26 | 0.00184 | | 0.564 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 361 | 63.1 | 361 | 50.4 | 361 | 36.0 |
| n (Samp) | 1118 | 14 | 1118 | 19 | 1118 | 7 |
| n (Patient) | 362 | 14 | 362 | 19 | 362 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.72 | 0.70 | 0.73 | 0.74 | nd | 0.78 | 0.65 | nd | 0.70 |
| SE | 0.062 | 0.10 | 0.078 | 0.064 | nd | 0.064 | 0.095 | nd | 0.11 |
| p | 3.3E-4 | 0.061 | 0.0034 | 1.8E-4 | nd | 1.4E-5 | 0.12 | nd | 0.069 |
| nCohort 1 | 1271 | 1335 | 1118 | 1271 | nd | 1118 | 1271 | nd | 1118 |
| nCohort 2 | 22 | 8 | 14 | 20 | nd | 19 | 10 | nd | 7 |
| Cutoff 1 | 9.29 | 11.3 | 8.44 | 10.5 | nd | 11.3 | 9.53 | nd | 12.6 |
| Sens 1 | 73% | 75% | 71% | 70% | nd | 74% | 70% | nd | 71% |
| Spec 1 | 67% | 73% | 63% | 72% | nd | 74% | 68% | nd | 78% |
| Cutoff 2 | 6.62 | 9.29 | 6.53 | 6.37 | nd | 8.14 | 6.20 | nd | 6.20 |
| Sens 2 | 82% | 88% | 86% | 80% | nd | 84% | 80% | nd | 86% |
| Spec 2 | 52% | 65% | 50% | 51% | nd | 62% | 50% | nd | 49% |
| Cutoff 3 | 5.52 | 1.41 | 5.48 | 4.33 | nd | 4.33 | 0.530 | nd | 0.530 |
| Sens 3 | 91% | 100% | 93% | 90% | nd | 95% | 90% | nd | 100% |
| Spec 3 | 45% | 10% | 44% | 35% | nd | 34% | 5% | nd | 5% |
| Cutoff 4 | 9.95 | 10.5 | 10.1 | 9.95 | nd | 10.1 | 9.95 | nd | 10.1 |
| Sens 4 | 68% | 75% | 64% | 70% | nd | 79% | 60% | nd | 71% |
| Spec 4 | 70% | 70% | 70% | 70% | nd | 70% | 70% | nd | 70% |
| Cutoff 5 | 13.4 | 14.1 | 13.7 | 13.4 | nd | 13.7 | 13.4 | nd | 13.7 |
| Sens 5 | 55% | 50% | 57% | 45% | nd | 53% | 50% | nd | 57% |
| Spec 5 | 80% | 80% | 80% | 80% | nd | 80% | 80% | nd | 80% |
| Cutoff 6 | 21.9 | 23.3 | 23.2 | 21.9 | nd | 23.2 | 21.9 | nd | 23.2 |
| Sens 6 | 18% | 0% | 29% | 35% | nd | 37% | 20% | nd | 29% |
| Spec 6 | 90% | 90% | 90% | 90% | nd | 90% | 90% | nd | 90% |
| OR Quart 2 | 0.50 | 0 | 1.0 | >4.0 | nd | >3.0 | 0.50 | nd | 1.0 |
| p Value | 0.57 | na | 1.0 | <0.21 | nd | <0.34 | 0.57 | nd | 1.0 |
| 95% CI of | 0.045 | na | 0.062 | >0.45 | nd | >0.31 | 0.045 | nd | 0.062 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart2 | 5.5 | na | 16 | na | nd | na | 5.5 | nd | 16 |
| OR Quart 3 | 3.0 | 2.0 | 4.0 | >5.1 | nd | >4.1 | 0.50 | nd | 0 |
| p Value | 0.18 | 0.57 | 0.21 | <0.14 | nd | <0.21 | 0.57 | nd | na |
| 95% CI of | 0.61 | 0.18 | 0.45 | >0.59 | nd | >0.45 | 0.045 | nd | na |
| OR Quart3 | 15 | 22 | 36 | na | nd | na | 5.5 | nd | na |
| OR Quart 4 | 6.7 | 5.0 | 8.2 | >11 | nd | >12 | 3.0 | nd | 5.1 |
| p Value | 0.013 | 0.14 | 0.048 | <0.020 | nd | <0.016 | 0.18 | nd | 0.14 |
| 95% CI of | 1.5 | 0.59 | 1.0 | >1.5 | nd | >1.6 | 0.61 | nd | 0.59 |
| OR Quart4 | 30 | 43 | 66 | na | nd | na | 15 | nd | 44 |

**CA 15-3**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 434 | 434 | 434 | 434 | 434 | 434 |
| Average | 326 | 423 | 326 | 325 | 326 | 308 |
| Stdev | 223 | 180 | 223 | 224 | 223 | 191 |
| p(t-test) |  | 0.17 |  | 0.99 |  | 0.82 |
| Min | 1.17 | 22.0 | 1.17 | 39.8 | 1.17 | 4.72 |
| Max | 784 | 784 | 784 | 784 | 784 | 434 |
| n (Samp) | 558 | 10 | 558 | 15 | 558 | 8 |
| n (Patient) | 262 | 10 | 262 | 15 | 262 | 8 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | nd | nd | nd | nd | 434 | 434 |
| Average | nd | nd | nd | nd | 326 | 406 |
| Stdev | nd | nd | nd | nd | 223 | 68.4 |
| p(t-test) | nd | nd | nd | nd |  | 0.38 |
| Min | nd | nd | nd | nd | 1.17 | 266 |
| Max | nd | nd | nd | nd | 784 | 434 |
| n (Samp) | nd | nd | nd | nd | 575 | 6 |

(continued)

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Patient) | nd | nd | nd | nd | 269 | 6 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 434 | 434 | nd | nd |
| Average | nd | nd | 331 | 349 | nd | nd |
| Stdev | nd | nd | 218 | 235 | nd | nd |
| p(t-test) | nd | nd | | 0.80 | nd | nd |
| Min | nd | nd | 1.17 | 52.2 | nd | nd |
| Max | nd | nd | 784 | 784 | nd | nd |
| n (Samp) | nd | nd | 533 | 10 | nd | nd |
| n (Patient) | nd | nd | 235 | 10 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.60 | nd | nd | 0.53 | nd | 0.55 | 0.47 | 0.60 | nd |
| SE | 0.096 | nd | nd | 0.077 | nd | 0.095 | 0.10 | 0.12 | nd |
| p | 0.28 | nd | nd | 0.71 | nd | 0.58 | 0.75 | 0.40 | nd |
| nCohort 1 | 558 | nd | nd | 558 | nd | 533 | 558 | 575 | nd |
| nCohort 2 | 10 | nd | nd | 15 | nd | 10 | 8 | 6 | nd |
| Cutoff 1 | 427 | nd | nd | 88.4 | nd | 239 | 265 | 427 | nd |
| Sens 1 | 80% | nd | nd | 73% | nd | 70% | 75% | 83% | nd |
| Spec 1 | 39% | nd | nd | 28% | nd | 33% | 36% | 39% | nd |
| Cutoff 2 | 427 | nd | nd | 62.5 | nd | 87.5 | 19.7 | 427 | nd |
| Sens 2 | 80% | nd | nd | 80% | nd | 80% | 88% | 83% | nd |
| Spec 2 | 39% | nd | nd | 24% | nd | 26% | 10% | 39% | nd |
| Cutoff 3 | 387 | nd | nd | 45.0 | nd | 62.5 | 4.68 | 265 | nd |
| Sens 3 | 90% | nd | nd | 93% | nd | 90% | 100% | 100% | nd |
| Spec 3 | 39% | nd | nd | 20% | nd | 23% | 1% | 36% | nd |
| Cutoff 4 | 434 | nd | nd | 434 | nd | 434 | 434 | 434 | nd |
| Sens 4 | 10% | nd | nd | 13% | nd | 20% | 0% | 0% | nd |
| Spec 4 | 92% | nd | nd | 92% | nd | 92% | 92% | 91% | nd |
| Cutoff 5 | 434 | nd | nd | 434 | nd | 434 | 434 | 434 | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 5 | 10% | nd | nd | 13% | nd | 20% | 0% | 0% | nd |
| Spec 5 | 92% | nd | nd | 92% | nd | 92% | 92% | 91% | nd |
| Cutoff 6 | 434 | nd | nd | 434 | nd | 434 | 434 | 434 | nd |
| Sens 6 | 10% | nd | nd | 13% | nd | 20% | 0% | 0% | nd |
| Spec 6 | 92% | nd | nd | 92% | nd | 92% | 92% | 91% | nd |
| OR Quart 2 | 8.4 | nd | nd | 0.49 | nd | 0.99 | >0 | >6.3 | nd |
| p Value | 0.046 | nd | nd | 0.42 | nd | 0.99 | <na | <0.091 | nd |
| 95% CI of | 1.0 | nd | nd | 0.089 | nd | 0.14 | >na | >0.74 | nd |
| OR Quart2 | 68 | nd | nd | 2.7 | nd | 7.2 | na | na | nd |
| OR Quart 3 | 0 | nd | nd | 1.8 | nd | 2.0 | >6.3 | >0 | nd |
| p Value | na | nd | nd | 0.36 | nd | 0.42 | <0.091 | <na | nd |
| 95% CI of | na | nd | nd | 0.51 | nd | 0.36 | >0.74 | >na | nd |
| OR Quart3 | na | nd | nd | 6.2 | nd | 11 | na | na | nd |
| OR Quart 4 | 1.0 | nd | nd | 0.49 | nd | 0.99 | >2.0 | >0 | nd |
| p Value | 1.0 | nd | nd | 0.41 | nd | 0.99 | <0.56 | <na | nd |
| 95% CI of | 0.062 | nd | nd | 0.088 | nd | 0.14 | >0.18 | >na | nd |
| OR Quart4 | 16 | nd | nd | 2.7 | nd | 7.2 | na | na | nd |

Table 10: Comparison of marker levels in EDTA samples collected from Cohort 1 (patients that did not progress beyond RIFLE stage 0, R, or I) and in EDTA samples collected from Cohort 2 (subjects who progress to RIFLE stage F) at 0, 24 hours, and 48 hours prior to the subject reaching RIFLE stage I.

| C-C motif chemokine 18 | | | | | | |
|---|---|---|---|---|---|---|
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | nd | nd | 182 | 272 | nd | nd |
| Average | nd | nd | 245 | 513 | nd | nd |
| Stdev | nd | nd | 205 | 604 | nd | nd |
| p(t-test) | nd | nd | | 0.0027 | nd | nd |
| Min | nd | nd | 31.7 | 92.4 | nd | nd |
| Max | nd | nd | 2110 | 1650 | nd | nd |

| C-C motif chemokine 18 | | | | | | | | |
|---|---|---|---|---|---|---|---|---|
| sCr or UO | 0hr prior to AKI stage | | 24hr prior to AKI stage | | 48hr prior to AKI stage | | | |
| | Cohort 1 | Cohort 2 | Cohort 1 | Cohort 2 | Cohort 1 | Cohort 2 | | |
| n (Samp) | nd | nd | 367 | 6 | nd | nd | | |
| n (Patient) | nd | nd | 197 | 6 | nd | nd | | |
| | | | | | | | | |
| | 0hr prior to AKI stage | | | 24hr prior to AKI stage | | | 48hr prior to AKI stage | | |
| | sCr or UO | sCr only | UO only | sCr or UO | sCr only | UO only | sCr or UO | sCr only | UO only |
| AUC | nd | nd | nd | 0.58 | nd | nd | nd | nd | nd |
| SE | nd | nd | nd | 0.12 | nd | nd | nd | nd | nd |
| p | nd | nd | nd | 0.51 | nd | nd | nd | nd | nd |
| nCohort 1 | nd | nd | nd | 367 | nd | nd | nd | nd | nd |
| nCohort 2 | nd | nd | nd | 6 | nd | nd | nd | nd | nd |
| Cutoff 1 | nd | nd | nd | 107 | nd | nd | nd | nd | nd |
| Sens 1 | nd | nd | nd | 83% | nd | nd | nd | nd | nd |
| Spec 1 | nd | nd | nd | 23% | nd | nd | nd | nd | nd |
| Cutoff 2 | nd | nd | nd | 107 | nd | nd | nd | nd | nd |
| Sens 2 | nd | nd | nd | 83% | nd | nd | nd | nd | nd |
| Spec 2 | nd | nd | nd | 23% | nd | nd | nd | nd | nd |
| Cutoff 3 | nd | nd | nd | 90.9 | nd | nd | nd | nd | nd |
| Sens 3 | nd | nd | nd | 100% | nd | nd | nd | nd | nd |
| Spec 3 | nd | nd | nd | 16% | nd | nd | nd | nd | nd |
| Cutoff 4 | nd | nd | nd | 278 | nd | nd | nd | nd | nd |
| Sens 4 | nd | nd | nd | 50% | nd | nd | nd | nd | nd |
| Spec 4 | nd | nd | nd | 70% | nd | nd | nd | nd | nd |
| Cutoff 5 | nd | nd | nd | 346 | nd | nd | nd | nd | nd |
| Sens 5 | nd | nd | nd | 50% | nd | nd | nd | nd | nd |
| Spec 5 | nd | nd | nd | 80% | nd | nd | nd | nd | nd |
| Cutoff 6 | nd | nd | nd | 491 | nd | nd | nd | nd | nd |
| Sens 6 | nd | nd | nd | 33% | nd | nd | nd | nd | nd |
| Spec 6 | nd | nd | nd | 90% | nd | nd | nd | nd | nd |
| OR Quart 2 | nd | nd | nd | 0.49 | nd | nd | nd | nd | nd |
| p Value | nd | nd | nd | 0.57 | nd | nd | nd | nd | nd |
| 95% CI of | nd | nd | nd | 0.044 | nd | nd | nd | nd | nd |
| OR Quart2 | nd | nd | nd | 5.6 | nd | nd | nd | nd | nd |
| OR Quart 3 | nd | nd | nd | 0 | nd | nd | nd | nd | nd |
| p Value | nd | nd | nd | na | nd | nd | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of OR Quart3 | nd | nd | nd | na | nd | nd | nd | nd | nd |
| | nd | nd | nd | na | nd | nd | nd | nd | nd |
| OR Quart 4 | nd | nd | nd | 1.5 | nd | nd | nd | nd | nd |
| p Value | nd | nd | nd | 0.66 | nd | nd | nd | nd | nd |
| 95% CI of OR Quart4 | nd | nd | nd | 0.24 | nd | nd | nd | nd | nd |
| | nd | nd | nd | 9.2 | nd | nd | nd | nd | nd |

**C-C motif chemokine 24**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 254 | 278 | nd | nd |
| Average | nd | nd | 388 | 405 | nd | nd |
| Stdev | nd | nd | 385 | 395 | nd | nd |
| p(t-test) | nd | nd | | 0.92 | nd | nd |
| Min | nd | nd | 0.0260 | 81.9 | nd | nd |
| Max | nd | nd | 2920 | 1100 | nd | nd |
| n (Samp) | nd | nd | 297 | 6 | nd | nd |
| n (Patient) | nd | nd | 166 | 6 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.51 | nd | nd | nd | nd | nd |
| SE | nd | nd | nd | 0.12 | nd | nd | nd | nd | nd |
| p | nd | nd | nd | 0.93 | nd | nd | nd | nd | nd |
| nCohort 1 | nd | nd | nd | 297 | nd | nd | nd | nd | nd |
| nCohort 2 | nd | nd | nd | 6 | nd | nd | nd | nd | nd |
| Cutoff 1 | nd | nd | nd | 95.2 | nd | nd | nd | nd | nd |
| Sens 1 | nd | nd | nd | 83% | nd | nd | nd | nd | nd |
| Spec 1 | nd | nd | nd | 20% | nd | nd | nd | nd | nd |
| Cutoff 2 | nd | nd | nd | 95.2 | nd | nd | nd | nd | nd |
| Sens 2 | nd | nd | nd | 83% | nd | nd | nd | nd | nd |
| Spec 2 | nd | nd | nd | 20% | nd | nd | nd | nd | nd |
| Cutoff 3 | nd | nd | nd | 80.9 | nd | nd | nd | nd | nd |
| Sens 3 | nd | nd | nd | 100% | nd | nd | nd | nd | nd |
| Spec 3 | nd | nd | nd | 16% | nd | nd | nd | nd | nd |
| Cutoff 4 | nd | nd | nd | 475 | nd | nd | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 4 | nd | nd | nd | 33% | nd | nd | nd | nd | nd |
| Spec 4 | nd | nd | nd | 70% | nd | nd | nd | nd | nd |
| Cutoff 5 | nd | nd | nd | 589 | nd | nd | nd | nd | nd |
| Sens 5 | nd | nd | nd | 33% | nd | nd | nd | nd | nd |
| Spec 5 | nd | nd | nd | 80% | nd | nd | nd | nd | nd |
| Cutoff 6 | nd | nd | nd | 900 | nd | nd | nd | nd | nd |
| Sens 6 | nd | nd | nd | 17% | nd | nd | nd | nd | nd |
| Spec 6 | nd | nd | nd | 90% | nd | nd | nd | nd | nd |
| OR Quart 2 | nd | nd | nd | 0.49 | nd | nd | nd | nd | nd |
| p Value | nd | nd | nd | 0.56 | nd | nd | nd | nd | nd |
| 95% CI of | nd | nd | nd | 0.043 | nd | nd | nd | nd | nd |
| OR Quart2 | nd | nd | nd | 5.5 | nd | nd | nd | nd | nd |
| OR Quart 3 | nd | nd | nd | 0.49 | nd | nd | nd | nd | nd |
| p Value | nd | nd | nd | 0.56 | nd | nd | nd | nd | nd |
| 95% CI of | nd | nd | nd | 0.043 | nd | nd | nd | nd | nd |
| OR Quart3 | nd | nd | nd | 5.5 | nd | nd | nd | nd | nd |
| OR Quart 4 | nd | nd | nd | 0.99 | nd | nd | nd | nd | nd |
| p Value | nd | nd | nd | 0.99 | nd | nd | nd | nd | nd |
| 95% CI of | nd | nd | nd | 0.14 | nd | nd | nd | nd | nd |
| OR Quart4 | nd | nd | nd | 7.2 | nd | nd | nd | nd | nd |

**C-C motif chemokine 8**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 13.4 | 16.7 | nd | nd |
| Average | nd | nd | 17.8 | 29.4 | nd | nd |
| Stdev | nd | nd | 23.5 | 35.7 | nd | nd |
| p(t-test) | nd | nd | | 0.24 | nd | nd |
| Min | nd | nd | 0.162 | 6.24 | nd | nd |
| Max | nd | nd | 180 | 101 | nd | nd |
| n (Samp) | nd | nd | 297 | 6 | nd | nd |
| n (Patient) | nd | nd | 166 | 6 | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.62 | nd | nd | nd | nd | nd |
| SE | nd | nd | nd | 0.12 | nd | nd | nd | nd | nd |
| p | nd | nd | nd | 0.34 | nd | nd | nd | nd | nd |
| nCohort 1 | nd | nd | nd | 297 | nd | nd | nd | nd | nd |
| nCohort 2 | nd | nd | nd | 6 | nd | nd | nd | nd | nd |
| Cutoff 1 | nd | nd | nd | 11.5 | nd | nd | nd | nd | nd |
| Sens 1 | nd | nd | nd | 83% | nd | nd | nd | nd | nd |
| Spec 1 | nd | nd | nd | 39% | nd | nd | nd | nd | nd |
| Cutoff 2 | nd | nd | nd | 11.5 | nd | nd | nd | nd | nd |
| Sens 2 | nd | nd | nd | 83% | nd | nd | nd | nd | nd |
| Spec 2 | nd | nd | nd | 39% | nd | nd | nd | nd | nd |
| Cutoff 3 | nd | nd | nd | 5.84 | nd | nd | nd | nd | nd |
| Sens 3 | nd | nd | nd | 100% | nd | nd | nd | nd | nd |
| Spec 3 | nd | nd | nd | 17% | nd | nd | nd | nd | nd |
| Cutoff 4 | nd | nd | nd | 17.1 | nd | nd | nd | nd | nd |
| Sens 4 | nd | nd | nd | 50% | nd | nd | nd | nd | nd |
| Spec 4 | nd | nd | nd | 70% | nd | nd | nd | nd | nd |
| Cutoff 5 | nd | nd | nd | 20.1 | nd | nd | nd | nd | nd |
| Sens 5 | nd | nd | nd | 33% | nd | nd | nd | nd | nd |
| Spec 5 | nd | nd | nd | 80% | nd | nd | nd | nd | nd |
| Cutoff 6 | nd | nd | nd | 30.5 | nd | nd | nd | nd | nd |
| Sens 6 | nd | nd | nd | 17% | nd | nd | nd | nd | nd |
| Spec 6 | nd | nd | nd | 90% | nd | nd | nd | nd | nd |
| | 0hr prior to AKI stage | | | 24hr prior to AKI stage | | | 48hr prior to AKI stage | | |
| | sCr or UO | sCr only | UO only | sCr or UO | sCr only | UO only | sCr or UO | sCr only | UO only |
| OR Quart 2 | nd | nd | nd | 0.99 | nd | nd | nd | nd | nd |
| p Value | nd | nd | nd | 0.99 | nd | nd | nd | nd | nd |
| 95% CI of | nd | nd | nd | 0.061 | nd | nd | nd | nd | nd |
| OR Quart2 | nd | nd | nd | 16 | nd | nd | nd | nd | nd |
| OR Quart 3 | nd | nd | nd | 0.99 | nd | nd | nd | nd | nd |
| p Value | nd | nd | nd | 0.99 | nd | nd | nd | nd | nd |
| 95% CI of | nd | nd | nd | 0.061 | nd | nd | nd | nd | nd |
| OR Quart3 | nd | nd | nd | 16 | nd | nd | nd | nd | nd |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart 4 | nd | nd | nd | 3.0 | nd | nd | nd | nd | nd |
| p Value | nd | nd | nd | 0.34 | nd | nd | nd | nd | nd |
| 95% CI of | nd | nd | nd | 0.31 | nd | nd | nd | nd | nd |
| OR Quart4 | nd | nd | nd | 30 | nd | nd | nd | nd | nd |

**Cathepsin D**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | nd | nd | 225000 | 379000 | nd | nd |
| Average | nd | nd | 260000 | 408000 | nd | nd |
| Stdev | nd | nd | 165000 | 159000 | nd | nd |
| p(t-test) | nd | nd |  | 0.030 | nd | nd |
| Min | nd | nd | 34100 | 230000 | nd | nd |
| Max | nd | nd | 1870000 | 655000 | nd | nd |
| n (Samp) | nd | nd | 366 | 6 | nd | nd |
| n (Patient) | nd | nd | 196 | 6 | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | nd | nd | nd | 0.79 | nd | nd | nd | nd | nd |
| SE | nd | nd | nd | 0.11 | nd | nd | nd | nd | nd |
| p | nd | nd | nd | 0.0096 | nd | nd | nd | nd | nd |
| nCohort 1 | nd | nd | nd | 366 | nd | nd | nd | nd | nd |
| nCohort 2 | nd | nd | nd | 6 | nd | nd | nd | nd | nd |
| Cutoff 1 | nd | nd | nd | 295000 | nd | nd | nd | nd | nd |
| Sens 1 | nd | nd | nd | 83% | nd | nd | nd | nd | nd |
| Spec 1 | nd | nd | nd | 67% | nd | nd | nd | nd | nd |
| Cutoff 2 | nd | nd | nd | 295000 | nd | nd | nd | nd | nd |
| Sens 2 | nd | nd | nd | 83% | nd | nd | nd | nd | nd |
| Spec 2 | nd | nd | nd | 67% | nd | nd | nd | nd | nd |
| Cutoff 3 | nd | nd | nd | 229000 | nd | nd | nd | nd | nd |
| Sens 3 | nd | nd | nd | 100% | nd | nd | nd | nd | nd |
| Spec 3 | nd | nd | nd | 51% | nd | nd | nd | nd | nd |
| Cutoff 4 | nd | nd | nd | 306000 | nd | nd | nd | nd | nd |
| Sens 4 | nd | nd | nd | 67% | nd | nd | nd | nd | nd |
| Spec 4 | nd | nd | nd | 70% | nd | nd | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 5 | nd | nd | nd | 357000 | nd | nd | nd | nd | nd |
| Sens 5 | nd | nd | nd | 50% | nd | nd | nd | nd | nd |
| Spec 5 | nd | nd | nd | 80% | nd | nd | nd | nd | nd |
| Cutoff 6 | nd | nd | nd | 449000 | nd | nd | nd | nd | nd |
| Sens 6 | nd | nd | nd | 50% | nd | nd | nd | nd | nd |
| Spec 6 | nd | nd | nd | 90% | nd | nd | nd | nd | nd |
| OR Quart 2 | nd | nd | nd | >0 | nd | nd | nd | nd | nd |
| p Value | nd | nd | nd | <na | nd | nd | nd | nd | nd |
| 95% CI of | nd | nd | nd | >na | nd | nd | nd | nd | nd |
| OR Quart2 | nd | nd | nd | na | nd | nd | nd | nd | nd |
| OR Quart 3 | nd | nd | nd | >3.1 | nd | nd | nd | nd | nd |
| p Value | nd | nd | nd | <0.33 | nd | nd | nd | nd | nd |
| 95% CI of | nd | nd | nd | >0.32 | nd | nd | nd | nd | nd |
| OR Quart3 | nd | nd | nd | na | nd | nd | nd | nd | nd |
| OR Quart 4 | nd | nd | nd | >3.1 | nd | nd | nd | nd | nd |
| p Value | nd | nd | nd | <0.33 | nd | nd | nd | nd | nd |
| 95% CI of | nd | nd | nd | >0.32 | nd | nd | nd | nd | nd |
| OR Quart4 | nd | nd | nd | na | nd | nd | nd | nd | nd |

**C-X-C motif chemokine 13**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 51.9 | 73.9 | nd | nd |
| Average | nd | nd | 129 | 89.7 | nd | nd |
| Stdev | nd | nd | 258 | 39.2 | nd | nd |
| p(t-test) | nd | nd | | 0.71 | nd | nd |
| Min | nd | nd | 9.90 | 45.0 | nd | nd |
| Max | nd | nd | 2000 | 141 | nd | nd |
| n (Samp) | nd | nd | 297 | 6 | nd | nd |
| n (Patient) | nd | nd | 166 | 6 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.64 | nd | nd | nd | nd | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | nd | nd | nd | 0.12 | nd | nd | nd | nd | nd |
| p | nd | nd | nd | 0.24 | nd | nd | nd | nd | nd |
| nCohort 1 | nd | nd | nd | 297 | nd | nd | nd | nd | nd |
| nCohort 2 | nd | nd | nd | 6 | nd | nd | nd | nd | nd |
| Cutoff 1 | nd | nd | nd | 67.5 | nd | nd | nd | nd | nd |
| Sens 1 | nd | nd | nd | 83% | nd | nd | nd | nd | nd |
| Spec 1 | nd | nd | nd | 58% | nd | nd | nd | nd | nd |
| Cutoff 2 | nd | nd | nd | 67.5 | nd | nd | nd | nd | nd |
| Sens 2 | nd | nd | nd | 83% | nd | nd | nd | nd | nd |
| Spec 2 | nd | nd | nd | 58% | nd | nd | nd | nd | nd |
| Cutoff 3 | nd | nd | nd | 44.8 | nd | nd | nd | nd | nd |
| Sens 3 | nd | nd | nd | 100% | nd | nd | nd | nd | nd |
| Spec 3 | nd | nd | nd | 44% | nd | nd | nd | nd | nd |
| Cutoff 4 | nd | nd | nd | 95.3 | nd | nd | nd | nd | nd |
| Sens 4 | nd | nd | nd | 33% | nd | nd | nd | nd | nd |
| Spec 4 | nd | nd | nd | 70% | nd | nd | nd | nd | nd |
| Cutoff 5 | nd | nd | nd | 134 | nd | nd | nd | nd | nd |
| Sens 5 | nd | nd | nd | 33% | nd | nd | nd | nd | nd |
| Spec 5 | nd | nd | nd | 80% | nd | nd | nd | nd | nd |
| Cutoff 6 | nd | nd | nd | 250 | nd | nd | nd | nd | nd |
| Sens 6 | nd | nd | nd | 0% | nd | nd | nd | nd | nd |
| Spec 6 | nd | nd | nd | 90% | nd | nd | nd | nd | nd |
| OR Quart 2 | nd | nd | nd | >1.0 | nd | nd | nd | nd | nd |
| p Value | nd | nd | nd | <1.0 | nd | nd | nd | nd | nd |
| 95% CI of | nd | nd | nd | >0.061 | nd | nd | nd | nd | nd |
| OR Quart2 | nd | nd | nd | na | nd | nd | nd | nd | nd |
| OR Quart 3 | nd | nd | nd | >3.1 | nd | nd | nd | nd | nd |
| p Value | nd | nd | nd | <0.33 | nd | nd | nd | nd | nd |
| 95% CI of | nd | nd | nd | >0.31 | nd | nd | nd | nd | nd |
| OR Quart3 | nd | nd | nd | na | nd | nd | nd | nd | nd |
| OR Quart 4 | nd | nd | nd | >2.0 | nd | nd | nd | nd | nd |
| p Value | nd | nd | nd | <0.57 | nd | nd | nd | nd | nd |
| 95% CI of | nd | nd | nd | >0.18 | nd | nd | nd | nd | nd |

| OR Quart4 | nd | nd | nd | na | nd | nd | nd | nd | nd |
|-----------|----|----|----|----|----|----|----|----|----|

(continued)

| Insulin-like growth factor-binding protein 3 | | | | | | | |
|---|---|---|---|---|---|---|---|
| sCr or UO | 0hr prior to AKI stage | | 24hr prior to AKI stage | | 48hr prior to AKI stage | | |
| | Cohort 1 | Cohort 2 | Cohort 1 | Cohort 2 | Cohort 1 | Cohort 2 | |
| Median | nd | nd | 3150 | 3200 | nd | nd | |
| Average | nd | nd | 3500 | 3580 | nd | nd | |
| Stdev | nd | nd | 1590 | 2040 | nd | nd | |
| p(t-test) | nd | nd | | 0.90 | nd | nd | |
| Min | nd | nd | 651 | 1380 | nd | nd | |
| Max | nd | nd | 8600 | 7360 | nd | nd | |
| n (Samp) | nd | nd | 367 | 6 | nd | nd | |
| n (Patient) | nd | nd | 197 | 6 | nd | nd | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.50 | nd | nd | nd | nd | nd |
| SE | nd | nd | nd | 0.12 | nd | nd | nd | nd | nd |
| p | nd | nd | nd | 0.98 | nd | nd | nd | nd | nd |
| nCohort 1 | nd | nd | nd | 367 | nd | nd | nd | nd | nd |
| nCohort 2 | nd | nd | nd | 6 | nd | nd | nd | nd | nd |
| Cutoff 1 | nd | nd | nd | 2410 | nd | nd | nd | nd | nd |
| Sens 1 | nd | nd | nd | 83% | nd | nd | nd | nd | nd |
| Spec 1 | nd | nd | nd | 30% | nd | nd | nd | nd | nd |
| Cutoff 2 | nd | nd | nd | 2410 | nd | nd | nd | nd | nd |
| Sens 2 | nd | nd | nd | 83% | nd | nd | nd | nd | nd |
| Spec 2 | nd | nd | nd | 30% | nd | nd | nd | nd | nd |
| Cutoff 3 | nd | nd | nd | 1380 | nd | nd | nd | nd | nd |
| Sens 3 | nd | nd | nd | 100% | nd | nd | nd | nd | nd |
| Spec 3 | nd | nd | nd | 6% | nd | nd | nd | nd | nd |
| Cutoff 4 | nd | nd | nd | 4250 | nd | nd | nd | nd | nd |
| Sens 4 | nd | nd | nd | 17% | nd | nd | nd | nd | nd |
| Spec 4 | nd | nd | nd | 70% | nd | nd | nd | nd | nd |
| Cutoff 5 | nd | nd | nd | 4870 | nd | nd | nd | nd | nd |
| Sens 5 | nd | nd | nd | 17% | nd | nd | nd | nd | nd |
| Spec 5 | nd | nd | nd | 80% | nd | nd | nd | nd | nd |
| Cutoff 6 | nd | nd | nd | 5780 | nd | nd | nd | nd | nd |
| Sens 6 | nd | nd | nd | 17% | nd | nd | nd | nd | nd |
| Spec 6 | nd | nd | nd | 90% | nd | nd | nd | nd | nd |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart 2 | nd | nd | nd | 2.0 | nd | nd | nd | nd | nd |
| p Value | nd | nd | nd | 0.56 | nd | nd | nd | nd | nd |
| 95% CI of | nd | nd | nd | 0.18 | nd | nd | nd | nd | nd |
| OR Quart2 | nd | nd | nd | 23 | nd | nd | nd | nd | nd |
| OR Quart 3 | nd | nd | nd | 2.0 | nd | nd | nd | nd | nd |
| p Value | nd | nd | nd | 0.56 | nd | nd | nd | nd | nd |
| 95% CI of | nd | nd | nd | 0.18 | nd | nd | nd | nd | nd |
| OR Quart3 | nd | nd | nd | 23 | nd | nd | nd | nd | nd |
| OR Quart 4 | nd | nd | nd | 1.0 | nd | nd | nd | nd | nd |
| p Value | nd | nd | nd | 0.99 | nd | nd | nd | nd | nd |
| 95% CI of | nd | nd | nd | 0.062 | nd | nd | nd | nd | nd |
| OR Quart4 | nd | nd | nd | 16 | nd | nd | nd | nd | nd |

**Interleukin-11**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | nd | nd | 62.8 | 83.7 | nd | nd |
| Average | nd | nd | 147 | 102 | nd | nd |
| Stdev | nd | nd | 555 | 53.4 | nd | nd |
| p(t-test) | nd | nd |  | 0.84 | nd | nd |
| Min | nd | nd | 0.359 | 51.1 | nd | nd |
| Max | nd | nd | 6920 | 171 | nd | nd |
| n (Samp) | nd | nd | 298 | 6 | nd | nd |
| n (Patient) | nd | nd | 167 | 6 | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | nd | nd | nd | 0.63 | nd | nd | nd | nd | nd |
| SE | nd | nd | nd | 0.12 | nd | nd | nd | nd | nd |
| p | nd | nd | nd | 0.29 | nd | nd | nd | nd | nd |
| nCohort 1 | nd | nd | nd | 298 | nd | nd | nd | nd | nd |
| nCohort 2 | nd | nd | nd | 6 | nd | nd | nd | nd | nd |
| Cutoff 1 | nd | nd | nd | 51.1 | nd | nd | nd | nd | nd |
| Sens 1 | nd | nd | nd | 83% | nd | nd | nd | nd | nd |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Spec 1 | nd | nd | nd | 46% | nd | nd | nd | nd | nd |
| Cutoff 2 | nd | nd | nd | 51.1 | nd | nd | nd | nd | nd |
| Sens 2 | nd | nd | nd | 83% | nd | nd | nd | nd | nd |
| Spec 2 | nd | nd | nd | 46% | nd | nd | nd | nd | nd |
| Cutoff 3 | nd | nd | nd | 47.4 | nd | nd | nd | nd | nd |
| Sens 3 | nd | nd | nd | 100% | nd | nd | nd | nd | nd |
| Spec 3 | nd | nd | nd | 46% | nd | nd | nd | nd | nd |
| Cutoff 4 | nd | nd | nd | 111 | nd | nd | nd | nd | nd |
| Sens 4 | nd | nd | nd | 33% | nd | nd | nd | nd | nd |
| Spec 4 | nd | nd | nd | 70% | nd | nd | nd | nd | nd |
| Cutoff 5 | nd | nd | nd | 158 | nd | nd | nd | nd | nd |
| Sens 5 | nd | nd | nd | 33% | nd | nd | nd | nd | nd |
| Spec 5 | nd | nd | nd | 81% | nd | nd | nd | nd | nd |
| Cutoff 6 | nd | nd | nd | 221 | nd | nd | nd | nd | nd |
| Sens 6 | nd | nd | nd | 0% | nd | nd | nd | nd | nd |
| Spec 6 | nd | nd | nd | 90% | nd | nd | nd | nd | nd |
| OR Quart 2 | nd | nd | nd | >2.1 | nd | nd | nd | nd | nd |
| p Value | nd | nd | nd | <0.56 | nd | nd | nd | nd | nd |
| 95% CI of | nd | nd | nd | >0.18 | nd | nd | nd | nd | nd |
| OR Quart2 | nd | nd | nd | na | nd | nd | nd | nd | nd |
| OR Quart 3 | nd | nd | nd | >2.1 | nd | nd | nd | nd | nd |
| p Value | nd | nd | nd | <0.56 | nd | nd | nd | nd | nd |
| 95% CI of | nd | nd | nd | >0.18 | nd | nd | nd | nd | nd |
| OR Quart3 | nd | nd | nd | na | nd | nd | nd | nd | nd |
| OR Quart 4 | nd | nd | nd | >2.1 | nd | nd | nd | nd | nd |
| p Value | nd | nd | nd | <0.56 | nd | nd | nd | nd | nd |
| 95% CI of | nd | nd | nd | >0.18 | nd | nd | nd | nd | nd |
| OR Quart4 | nd | nd | nd | na | nd | nd | nd | nd | nd |

**Interleukin-2 receptor alpha chain**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | nd | nd | 19.4 | 28.9 | nd | nd |
| Average | nd | nd | 70.5 | 48.1 | nd | nd |
| Stdev | nd | nd | 148 | 56.2 | nd | nd |

(continued)

| Interleukin-2 receptor alpha chain | | | | | | | |
|---|---|---|---|---|---|---|---|
| sCr or UO | 0hr prior to AKI stage | | 24hr prior to AKI stage | | 48hr prior to AKI stage | | |
| | Cohort 1 | Cohort 2 | Cohort 1 | Cohort 2 | Cohort 1 | Cohort 2 | |
| p(t-test) | nd | nd | | 0.71 | nd | nd | |
| Min | nd | nd | 0.0290 | 0.0290 | nd | nd | |
| Max | nd | nd | 1040 | 154 | nd | nd | |
| n (Samp) | nd | nd | 298 | 6 | nd | nd | |
| n (Patient) | nd | nd | 167 | 6 | nd | nd | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.54 | nd | nd | nd | nd | nd |
| SE | nd | nd | nd | 0.12 | nd | nd | nd | nd | nd |
| p | nd | nd | nd | 0.77 | nd | nd | nd | nd | nd |
| nCohort 1 | nd | nd | nd | 298 | nd | nd | nd | nd | nd |
| nCohort 2 | nd | nd | nd | 6 | nd | nd | nd | nd | nd |
| Cutoff 1 | nd | nd | nd | 11.9 | nd | nd | nd | nd | nd |
| Sens 1 | nd | nd | nd | 83% | nd | nd | nd | nd | nd |
| Spec 1 | nd | nd | nd | 43% | nd | nd | nd | nd | nd |
| Cutoff 2 | nd | nd | nd | 11.9 | nd | nd | nd | nd | nd |
| Sens 2 | nd | nd | nd | 83% | nd | nd | nd | nd | nd |
| Spec 2 | nd | nd | nd | 43% | nd | nd | nd | nd | nd |
| Cutoff 3 | nd | nd | nd | 0 | nd | nd | nd | nd | nd |
| Sens 3 | nd | nd | nd | 100% | nd | nd | nd | nd | nd |
| Spec 3 | nd | nd | nd | 0% | nd | nd | nd | nd | nd |
| Cutoff 4 | nd | nd | nd | 50.7 | nd | nd | nd | nd | nd |
| Sens 4 | nd | nd | nd | 33% | nd | nd | nd | nd | nd |
| Spec 4 | nd | nd | nd | 70% | nd | nd | nd | nd | nd |
| Cutoff 5 | nd | nd | nd | 92.7 | nd | nd | nd | nd | nd |
| Sens 5 | nd | nd | nd | 17% | nd | nd | nd | nd | nd |
| Spec 5 | nd | nd | nd | 80% | nd | nd | nd | nd | nd |
| Cutoff 6 | nd | nd | nd | 167 | nd | nd | nd | nd | nd |
| Sens 6 | nd | nd | nd | 0% | nd | nd | nd | nd | nd |
| Spec 6 | nd | nd | nd | 90% | nd | nd | nd | nd | nd |
| OR Quart 2 | nd | nd | nd | 1.0 | nd | nd | nd | nd | nd |
| p Value | nd | nd | nd | 1.0 | nd | nd | nd | nd | nd |
| 95% CI of | nd | nd | nd | 0.061 | nd | nd | nd | nd | nd |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart2 | nd | nd | nd | 16 | nd | nd | nd | nd | nd |
| OR Quart 3 | nd | nd | nd | 3.1 | nd | nd | nd | nd | nd |
| p Value | nd | nd | nd | 0.33 | nd | nd | nd | nd | nd |
| 95% CI of | nd | nd | nd | 0.31 | nd | nd | nd | nd | nd |
| OR Quart3 | nd | nd | nd | 30 | nd | nd | nd | nd | nd |
| OR Quart 4 | nd | nd | nd | 1.0 | nd | nd | nd | nd | nd |
| p Value | nd | nd | nd | 1.0 | nd | nd | nd | nd | nd |
| 95% CI of | nd | nd | nd | 0.061 | nd | nd | nd | nd | nd |
| OR Quart4 | nd | nd | nd | 16 | nd | nd | nd | nd | nd |

**Neutrophil collagenase**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | nd | nd | 1100 | 5350 | nd | nd |
| Average | nd | nd | 3160 | 14200 | nd | nd |
| Stdev | nd | nd | 6940 | 17400 | nd | nd |
| p(t-test) | nd | nd |  | 2.3E-4 | nd | nd |
| Min | nd | nd | 40.0 | 1.14 | nd | nd |
| Max | nd | nd | 55000 | 38700 | nd | nd |
| n (Samp) | nd | nd | 366 | 6 | nd | nd |
| n (Patient) | nd | nd | 197 | 6 | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | nd | nd | nd | 0.72 | nd | nd | nd | nd | nd |
| SE | nd | nd | nd | 0.12 | nd | nd | nd | nd | nd |
| p | nd | nd | nd | 0.059 | nd | nd | nd | nd | nd |
| nCohort 1 | nd | nd | nd | 366 | nd | nd | nd | nd | nd |
| nCohort 2 | nd | nd | nd | 6 | nd | nd | nd | nd | nd |
| Cutoff 1 | nd | nd | nd | 1500 | nd | nd | nd | nd | nd |
| Sens 1 | nd | nd | nd | 83% | nd | nd | nd | nd | nd |
| Spec 1 | nd | nd | nd | 64% | nd | nd | nd | nd | nd |
| Cutoff 2 | nd | nd | nd | 1500 | nd | nd | nd | nd | nd |
| Sens 2 | nd | nd | nd | 83% | nd | nd | nd | nd | nd |
| Spec 2 | nd | nd | nd | 64% | nd | nd | nd | nd | nd |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Cutoff 3 | nd | nd | nd | 0 | nd | nd | nd | nd | nd |
| Sens 3 | nd | nd | nd | 100% | nd | nd | nd | nd | nd |
| Spec 3 | nd | nd | nd | 0% | nd | nd | nd | nd | nd |
| Cutoff 4 | nd | nd | nd | 1830 | nd | nd | nd | nd | nd |
| Sens 4 | nd | nd | nd | 67% | nd | nd | nd | nd | nd |
| Spec 4 | nd | nd | nd | 70% | nd | nd | nd | nd | nd |
| Cutoff 5 | nd | nd | nd | 3110 | nd | nd | nd | nd | nd |
| Sens 5 | nd | nd | nd | 67% | nd | nd | nd | nd | nd |
| Spec 5 | nd | nd | nd | 80% | nd | nd | nd | nd | nd |
| Cutoff 6 | nd | nd | nd | 6510 | nd | nd | nd | nd | nd |
| Sens 6 | nd | nd | nd | 50% | nd | nd | nd | nd | nd |
| Spec 6 | nd | nd | nd | 90% | nd | nd | nd | nd | nd |
| OR Quart 2 | nd | nd | nd | 0 | nd | nd | nd | nd | nd |
| p Value | nd | nd | nd | na | nd | nd | nd | nd | nd |
| 95% CI of | nd | nd | nd | na | nd | nd | nd | nd | nd |
| OR Quart2 | nd | nd | nd | na | nd | nd | nd | nd | nd |
| OR Quart 3 | nd | nd | nd | 1.0 | nd | nd | nd | nd | nd |
| p Value | nd | nd | nd | 1.0 | nd | nd | nd | nd | nd |
| 95% CI of | nd | nd | nd | 0.062 | nd | nd | nd | nd | nd |
| OR Quart3 | nd | nd | nd | 16 | nd | nd | nd | nd | nd |
| OR Quart 4 | nd | nd | nd | 4.1 | nd | nd | nd | nd | nd |
| p Value | nd | nd | nd | 0.21 | nd | nd | nd | nd | nd |
| 95% CI of | nd | nd | nd | 0.45 | nd | nd | nd | nd | nd |
| OR Quart4 | nd | nd | nd | 38 | nd | nd | nd | nd | nd |

**Protransforming growth factor alpha**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | nd | nd | 1.45 | 6.40 | nd | nd |
| Average | nd | nd | 7.21 | 17.3 | nd | nd |
| Stdev | nd | nd | 27.5 | 26.5 | nd | nd |
| p(t-test) | nd | nd |  | 0.37 | nd | nd |
| Min | nd | nd | 0.00228 | 0.0105 | nd | nd |
| Max | nd | nd | 287 | 68.3 | nd | nd |
| n (Samp) | nd | nd | 298 | 6 | nd | nd |

(continued)

| Protransforming growth factor alpha | | | | | | | | |
|---|---|---|---|---|---|---|---|---|
| sCr or UO | 0hr prior to AKI stage | | 24hr prior to AKI stage | | 48hr prior to AKI stage | | | |
| | Cohort 1 | Cohort 2 | Cohort 1 | Cohort 2 | Cohort 1 | Cohort 2 | | |
| n (Patient) | nd | nd | 167 | 6 | nd | nd | | |
| | | | | | | | | |
| | 0hr prior to AKI stage | | | 24hr prior to AKI stage | | | 48hr prior to AKI stage | | |
| | sCr or UO | sCr only | UO only | sCr or UO | sCr only | UO only | sCr or UO | sCr only | UO only |
| AUC | nd | nd | nd | 0.60 | nd | nd | nd | nd | nd |
| SE | nd | nd | nd | 0.12 | nd | nd | nd | nd | nd |
| p | nd | nd | nd | 0.42 | nd | nd | nd | nd | nd |
| nCohort 1 | nd | nd | nd | 298 | nd | nd | nd | nd | nd |
| nCohort 2 | nd | nd | nd | 6 | nd | nd | nd | nd | nd |
| Cutoff 1 | nd | nd | nd | 0.190 | nd | nd | nd | nd | nd |
| Sens 1 | nd | nd | nd | 83% | nd | nd | nd | nd | nd |
| Spec 1 | nd | nd | nd | 23% | nd | nd | nd | nd | nd |
| Cutoff 2 | nd | nd | nd | 0.190 | nd | nd | nd | nd | nd |
| Sens 2 | nd | nd | nd | 83% | nd | nd | nd | nd | nd |
| Spec 2 | nd | nd | nd | 23% | nd | nd | nd | nd | nd |
| Cutoff 3 | nd | nd | nd | 0.00584 | nd | nd | nd | nd | nd |
| Sens 3 | nd | nd | nd | 100% | nd | nd | nd | nd | nd |
| Spec 3 | nd | nd | nd | 16% | nd | nd | nd | nd | nd |
| Cutoff 4 | nd | nd | nd | 3.12 | nd | nd | nd | nd | nd |
| Sens 4 | nd | nd | nd | 50% | nd | nd | nd | nd | nd |
| Spec 4 | nd | nd | nd | 70% | nd | nd | nd | nd | nd |
| Cutoff 5 | nd | nd | nd | 4.80 | nd | nd | nd | nd | nd |
| Sens 5 | nd | nd | nd | 50% | nd | nd | nd | nd | nd |
| Spec 5 | nd | nd | nd | 81% | nd | nd | nd | nd | nd |
| Cutoff 6 | nd | nd | nd | 9.07 | nd | nd | nd | nd | nd |
| Sens 6 | nd | nd | nd | 50% | nd | nd | nd | nd | nd |
| Spec 6 | nd | nd | nd | 90% | nd | nd | nd | nd | nd |
| OR Quart 2 | nd | nd | nd | 0.49 | nd | nd | nd | nd | nd |
| p Value | nd | nd | nd | 0.57 | nd | nd | nd | nd | nd |
| 95% CI of | nd | nd | nd | 0.044 | nd | nd | nd | nd | nd |
| OR Quart2 | nd | nd | nd | 5.6 | nd | nd | nd | nd | nd |
| OR Quart 3 | nd | nd | nd | 0 | nd | nd | nd | nd | nd |
| p Value | nd | nd | nd | na | nd | nd | nd | nd | nd |
| 95% CI of | nd | nd | nd | na | nd | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart3 | nd | nd | nd | na | nd | nd | nd | nd | nd |
| OR Quart 4 | nd | nd | nd | 1.5 | nd | nd | nd | nd | nd |
| p Value | nd | nd | nd | 0.65 | nd | nd | nd | nd | nd |
| 95% CI of | nd | nd | nd | 0.25 | nd | nd | nd | nd | nd |
| OR Quart4 | nd | nd | nd | 9.4 | nd | nd | nd | nd | nd |

**CA 15-3**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 0.286 | 0.833 | nd | nd |
| Average | nd | nd | 1.00 | 1.13 | nd | nd |
| Stdev | nd | nd | 4.15 | 0.884 | nd | nd |
| p(t-test) | nd | nd | | 0.94 | nd | nd |
| Min | nd | nd | 0.00154 | 0.249 | nd | nd |
| Max | nd | nd | 60.0 | 2.69 | nd | nd |
| n (Samp) | nd | nd | 367 | 6 | nd | nd |
| n (Patient) | nd | nd | 197 | 6 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.80 | nd | nd | nd | nd | nd |
| SE | nd | nd | nd | 0.11 | nd | nd | nd | nd | nd |
| p | nd | nd | nd | 0.0065 | nd | nd | nd | nd | nd |
| nCohort 1 | nd | nd | nd | 367 | nd | nd | nd | nd | nd |
| nCohort 2 | nd | nd | nd | 6 | nd | nd | nd | nd | nd |
| Cutoff 1 | nd | nd | nd | 0.597 | nd | nd | nd | nd | nd |
| Sens 1 | nd | nd | nd | 83% | nd | nd | nd | nd | nd |
| Spec 1 | nd | nd | nd | 78% | nd | nd | nd | nd | nd |
| Cutoff 2 | nd | nd | nd | 0.597 | nd | nd | nd | nd | nd |
| Sens 2 | nd | nd | nd | 83% | nd | nd | nd | nd | nd |
| Spec 2 | nd | nd | nd | 78% | nd | nd | nd | nd | nd |
| Cutoff 3 | nd | nd | nd | 0.249 | nd | nd | nd | nd | nd |
| Sens 3 | nd | nd | nd | 100% | nd | nd | nd | nd | nd |
| Spec 3 | nd | nd | nd | 46% | nd | nd | nd | nd | nd |
| Cutoff 4 | nd | nd | nd | 0.466 | nd | nd | nd | nd | nd |

| Sens 4 | nd | nd | nd | 83% | nd | nd | nd | nd | nd |
|--------|----|----|----|-----|----|----|----|----|----|

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 4 | nd | nd | nd | 70% | nd | nd | nd | nd | nd |
| Cutoff 5 | nd | nd | nd | 0.680 | nd | nd | nd | nd | nd |
| Sens 5 | nd | nd | nd | 67% | nd | nd | nd | nd | nd |
| Spec 5 | nd | nd | nd | 80% | nd | nd | nd | nd | nd |
| Cutoff 6 | nd | nd | nd | 1.26 | nd | nd | nd | nd | nd |
| Sens 6 | nd | nd | nd | 33% | nd | nd | nd | nd | nd |
| Spec 6 | nd | nd | nd | 90% | nd | nd | nd | nd | nd |
| OR Quart 2 | nd | nd | nd | >1.0 | nd | nd | nd | nd | nd |
| p Value | nd | nd | nd | <0.99 | nd | nd | nd | nd | nd |
| 95% CI of | nd | nd | nd | >0.062 | nd | nd | nd | nd | nd |
| OR Quart2 | nd | nd | nd | na | nd | nd | nd | nd | nd |
| OR Quart 3 | nd | nd | nd | >0 | nd | nd | nd | nd | nd |
| p Value | nd | nd | nd | <na | nd | nd | nd | nd | nd |
| 95% CI of | nd | nd | nd | >na | nd | nd | nd | nd | nd |
| OR Quart3 | nd | nd | nd | na | nd | nd | nd | nd | nd |
| OR Quart 4 | nd | nd | nd | >5.2 | nd | nd | nd | nd | nd |
| p Value | nd | nd | nd | <0.13 | nd | nd | nd | nd | nd |
| 95% CI of | nd | nd | nd | >0.60 | nd | nd | nd | nd | nd |
| OR Quart4 | nd | nd | nd | na | nd | nd | nd | nd | nd |

Table 11: Comparison of marker levels in enroll urine samples collected from Cohort 1 (patients that did not progress beyond RIFLE stage 0 or R within 48hrs) and in enroll urine samples collected from Cohort 2 (subjects reaching RIFLE stage I or F within 48hrs). Enroll samples from patients already at RIFLE stage I or F were included in Cohort 2.

| C-C motif chemokine 18 | | | | | | |
|---|---|---|---|---|---|---|
| | sCr or UO | | sCr only | | UO only | |
| | Cohort 1 | Cohort 2 | Cohort 1 | Cohort 2 | Cohort 1 | Cohort 2 |
| Median | 0.460 | 1.04 | 0.486 | 2.03 | 0.488 | 1.04 |
| Average | 1.95 | 6.61 | 2.58 | 9.28 | 2.03 | 7.35 |
| | sCr or UO | | sCr only | | UO only | |
| | Cohort 1 | Cohort 2 | Cohort 1 | Cohort 2 | Cohort 1 | Cohort 2 |
| Stdev | 5.90 | 12.7 | 7.41 | 14.4 | 5.85 | 13.5 |
| p(t-test) | | 2.7E-7 | | 2.0E-4 | | 3.3E-7 |
| Min | 3.13E-5 | 0.00268 | 3.13E-5 | 0.204 | 3.13E-5 | 0.00268 |
| Max | 40.0 | 40.0 | 40.0 | 40.0 | 40.0 | 40.0 |

(continued)

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| n (Samp) | 383 | 92 | 451 | 20 | 296 | 79 |
| n (Patient) | 383 | 92 | 451 | 20 | 296 | 79 |
| | | | | | | |
| | | At Enrollment | | | | |
| | | sCr or UO | sCr only | | UO only | |
| AUC | | 0.66 | 0.75 | | 0.64 | |
| SE | | 0.034 | 0.064 | | 0.037 | |
| p | | 2.3E-6 | 9.2E-5 | | 1.4E-4 | |
| nCohort 1 | | 383 | 451 | | 296 | |
| nCohort 2 | | 92 | 20 | | 79 | |
| Cutoff 1 | | 0.402 | 0.906 | | 0.362 | |
| Sens 1 | | 71% | 70% | | 71% | |
| Spec 1 | | 48% | 67% | | 43% | |
| Cutoff 2 | | 0.274 | 0.826 | | 0.264 | |
| Sens 2 | | 80% | 80% | | 81% | |
| Spec 2 | | 39% | 63% | | 33% | |
| Cutoff 3 | | 0.147 | 0.340 | | 0.141 | |
| Sens 3 | | 90% | 90% | | 91% | |
| Spec 3 | | 24% | 42% | | 19% | |
| Cutoff 4 | | 0.928 | 1.10 | | 1.01 | |
| Sens 4 | | 53% | 60% | | 52% | |
| Spec 4 | | 70% | 70% | | 70% | |
| Cutoff 5 | | 1.61 | 1.84 | | 1.81 | |
| Sens 5 | | 41% | 50% | | 39% | |
| Spec 5 | | 80% | 80% | | 80% | |
| Cutoff 6 | | 3.46 | 3.81 | | 3.59 | |
| Sens 6 | | 26% | 35% | | 25% | |
| Spec 6 | | 90% | 90% | | 90% | |
| OR Quart 2 | | 2.3 | >3.1 | | 1.7 | |
| p Value | | 0.031 | <0.34 | | 0.18 | |
| 95% CI of | | 1.1 | >0.31 | | 0.78 | |
| OR Quart2 | | 5.0 | na | | 3.8 | |
| OR Quart 3 | | 1.8 | >6.3 | | 1.3 | |
| p Value | | 0.13 | <0.092 | | 0.55 | |
| 95% CI of | | 0.84 | >0.74 | | 0.56 | |
| OR Quart3 | | 4.1 | na | | 2.9 | |
| OR Quart 4 | | 4.7 | >12 | | 3.7 | |

(continued)

|  | At Enrollment | | |
|---|---|---|---|
|  | sCr or UO | sCr only | UO only |
| p Value | 2.9E-5 | <0.018 | 6.0E-4 |
| 95% CI of | 2.3 | >1.5 | 1.7 |
| OR Quart4 | 9.8 | na | 7.7 |

**C-C motif chemokine 24**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 10.6 | 36.2 | 13.9 | 79.4 | 11.4 | 36.1 |
| Average | 24.0 | 134 | 39.6 | 172 | 24.8 | 144 |
| Stdev | 47.0 | 357 | 147 | 418 | 49.4 | 384 |
| p(t-test) |  | 1.1E-8 |  | 5.4E-4 |  | 3.3E-7 |
| Min | 0.0120 | 0.0347 | 0.0120 | 3.66 | 0.0120 | 0.0347 |
| Max | 554 | 2380 | 2380 | 1930 | 554 | 2380 |
| n (Samp) | 383 | 92 | 451 | 20 | 297 | 79 |
| n (Patient) | 383 | 92 | 451 | 20 | 297 | 79 |

|  | At Enrollment | | |
|---|---|---|---|
|  | sCr or UO | sCr only | UO only |
| AUC | 0.76 | 0.81 | 0.75 |
| SE | 0.031 | 0.059 | 0.034 |
| p | 0 | 1.4E-7 | 2.5E-13 |
| nCohort 1 | 383 | 451 | 297 |
| nCohort 2 | 92 | 20 | 79 |
| Cutoff 1 | 24.0 | 27.3 | 21.2 |
| Sens 1 | 71% | 70% | 71% |
| Spec 1 | 72% | 71% | 69% |
| Cutoff 2 | 14.5 | 27.0 | 11.7 |
| Sens 2 | 80% | 80% | 81% |
| Spec 2 | 58% | 71% | 51% |
| Cutoff 3 | 7.72 | 20.4 | 5.54 |
| Sens 3 | 90% | 90% | 91% |
| Spec 3 | 42% | 61% | 35% |
| Cutoff 4 | 22.4 | 26.6 | 22.4 |
| Sens 4 | 71% | 80% | 70% |
| Spec 4 | 70% | 70% | 70% |
| Cutoff 5 | 31.0 | 38.5 | 29.7 |
| Sens 5 | 54% | 60% | 57% |
| Spec 5 | 80% | 80% | 80% |

(continued)

|  | At Enrollment | | |
|---|---|---|---|
|  | sCr or UO | sCr only | UO only |
| Cutoff 6 | 57.3 | 67.2 | 57.9 |
| Sens 6 | 37% | 55% | 35% |
| Spec 6 | 90% | 90% | 90% |
| OR Quart 2 | 2.8 | >2.0 | 2.6 |
| p Value | 0.061 | <0.57 | 0.084 |
| 95% CI of | 0.96 | >0.18 | 0.88 |
| OR Quart2 | 8.0 | na | 7.7 |
| OR Quart 3 | 6.6 | >6.3 | 5.4 |
| p Value | 1.9E-4 | <0.092 | 0.0011 |
| 95% CI of | 2.5 | >0.74 | 2.0 |
| OR Quart3 | 18 | na | 15 |
| OR Quart 4 | 15 | >13 | 13 |
| p Value | 5.1E-8 | <0.014 | 3.2E-7 |
| 95% CI of | 5.6 | >1.7 | 4.9 |
| OR Quart4 | 39 | na | 35 |

**C-C motif chemokine 8**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 1.36 | 6.27 | 1.51 | 19.7 | 1.55 | 6.84 |
| Average | 11.6 | 106 | 14.8 | 376 | 13.1 | 74.4 |
| Stdev | 35.6 | 494 | 45.8 | 1020 | 39.9 | 345 |
| p(t-test) |  | 2.2E-4 |  | 3.3E-13 |  | 0.0029 |
| Min | 0.0250 | 0.0250 | 0.0250 | 0.0701 | 0.0250 | 0.0250 |
| Max | 473 | 3670 | 492 | 3670 | 473 | 3020 |
| n (Samp) | 383 | 92 | 451 | 20 | 297 | 79 |
| n (Patient) | 383 | 92 | 451 | 20 | 297 | 79 |

|  | At Enrollment | | |
|---|---|---|---|
|  | sCr or UO | sCr only | UO only |
| AUC | 0.64 | 0.70 | 0.63 |
| SE | 0.034 | 0.067 | 0.037 |
| p | 3.2E-5 | 0.0024 | 3.7E-4 |
| nCohort 1 | 383 | 451 | 297 |
| nCohort 2 | 92 | 20 | 79 |
| Cutoff 1 | 0.491 | 3.16 | 0.491 |
| Sens 1 | 71% | 70% | 71% |
| Spec 1 | 38% | 58% | 35% |

(continued)

|  | At Enrollment | | |
|---|---|---|---|
|  | sCr or UO | sCr only | UO only |
| Cutoff 2 | 0.240 | 0.491 | 0.240 |
| Sens 2 | 80% | 80% | 82% |
| Spec 2 | 35% | 37% | 31% |
| Cutoff 3 | 0.127 | 0.154 | 0.127 |
| Sens 3 | 91% | 90% | 91% |
| Spec 3 | 27% | 28% | 23% |
| Cutoff 4 | 7.03 | 7.99 | 7.18 |
| Sens 4 | 48% | 60% | 49% |
| Spec 4 | 70% | 70% | 70% |
| Cutoff 5 | 12.0 | 12.7 | 12.2 |
| Sens 5 | 36% | 55% | 34% |
| Spec 5 | 80% | 80% | 80% |
| Cutoff 6 | 24.5 | 30.8 | 24.5 |
| Sens 6 | 27% | 35% | 28% |
| Spec 6 | 90% | 90% | 91% |
| OR Quart 2 | 3.4 | 5.1 | 1.8 |
| p Value | 0.0031 | 0.14 | 0.16 |
| 95% CI of | 1.5 | 0.59 | 0.79 |
| OR Quart2 | 7.6 | 45 | 4.0 |
| OR Quart 3 | 2.4 | 2.0 | 2.2 |
| p Value | 0.035 | 0.57 | 0.056 |
| 95% CI of | 1.1 | 0.18 | 0.98 |
| OR Quart3 | 5.6 | 22 | 4.8 |
| OR Quart 4 | 5.5 | 13 | 3.4 |
| p Value | 2.1E-5 | 0.014 | 0.0019 |
| 95% CI of | 2.5 | 1.7 | 1.6 |
| OR Quart4 | 12 | 100 | 7.2 |

**Cathepsin D**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 72700 | 105000 | 75800 | 130000 | 75500 | 104000 |
| Average | 76500 | 111000 | 81000 | 130000 | 80300 | 106000 |
| Stdev | 40100 | 42500 | 41700 | 40400 | 39500 | 41400 |
| p(t-test) |  | 2.0E-12 |  | 4.8E-7 |  | 3.7E-7 |
| Min | 656 | 4320 | 656 | 55700 | 2520 | 4320 |
| Max | 200000 | 200000 | 200000 | 200000 | 200000 | 200000 |
| n (Samp) | 383 | 92 | 451 | 20 | 296 | 79 |

(continued)

| Cathepsin D | | | | | | |
|---|---|---|---|---|---|---|
| | sCr or UO | | sCr only | | UO only | |
| | Cohort 1 | Cohort 2 | Cohort 1 | Cohort 2 | Cohort 1 | Cohort 2 |
| n (Patient) | 383 | 92 | 451 | 20 | 296 | 79 |
| | | | | | | |
| | | At Enrollment | | | | |
| | | sCr or UO | sCr only | | UO only | |
| AUC | | 0.72 | 0.80 | | 0.68 | |
| SE | | 0.032 | 0.060 | | 0.036 | |
| p | | 2.7E-12 | 6.5E-7 | | 4.3E-7 | |
| nCohort 1 | | 383 | 451 | | 296 | |
| nCohort 2 | | 92 | 20 | | 79 | |
| Cutoff 1 | | 84100 | 108000 | | 78200 | |
| Sens 1 | | 71% | 70% | | 71% | |
| Spec 1 | | 63% | 76% | | 53% | |
| Cutoff 2 | | 71300 | 99200 | | 70400 | |
| Sens 2 | | 80% | 80% | | 81% | |
| Spec 2 | | 49% | 69% | | 46% | |
| Cutoff 3 | | 63600 | 84400 | | 62300 | |
| Sens 3 | | 90% | 90% | | 91% | |
| Spec 3 | | 42% | 59% | | 37% | |
| Cutoff 4 | | 95800 | 102000 | | 99200 | |
| Sens 4 | | 60% | 70% | | 54% | |
| Spec 4 | | 70% | 70% | | 70% | |
| Cutoff 5 | | 109000 | 116000 | | 115000 | |
| Sens 5 | | 47% | 65% | | 39% | |
| Spec 5 | | 80% | 80% | | 80% | |
| Cutoff 6 | | 133000 | 138000 | | 134000 | |
| Sens 6 | | 27% | 35% | | 24% | |
| Spec 6 | | 90% | 90% | | 90% | |
| OR Quart 2 | | 5.4 | >2.0 | | 4.8 | |
| p Value | | 0.0029 | <0.57 | | 0.0029 | |
| 95% CI of | | 1.8 | >0.18 | | 1.7 | |
| OR Quart2 | | 16 | na | | 13 | |
| OR Quart 3 | | 8.0 | >5.2 | | 5.1 | |
| p Value | | 1.8E-4 | <0.14 | | 0.0019 | |
| 95% CI of | | 2.7 | >0.60 | | 1.8 | |
| OR Quart3 | | 24 | na | | 14 | |
| OR Quart 4 | | 16 | >14 | | 9.5 | |

(continued)

|  | At Enrollment | | |
|---|---|---|---|
|  | sCr or UO | sCr only | UO only |
| p Value | 3.1E-7 | <0.011 | 9.2E-6 |
| 95% CI of | 5.6 | >1.9 | 3.5 |
| OR Quart4 | 47 | na | 26 |

**C-X-C motif chemokine 13**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 0.0733 | 2.45 | 0.236 | 7.12 | 0.156 | 2.43 |
| Average | 6.47 | 46.6 | 9.11 | 131 | 7.64 | 51.3 |
| Stdev | 37.8 | 201 | 42.6 | 413 | 42.7 | 216 |
| p(t-test) |  | 2.8E-4 |  | 1.8E-8 |  | 0.0012 |
| Min | 0.00269 | 0.00269 | 0.00269 | 0.00442 | 0.00269 | 0.00269 |
| Max | 652 | 1850 | 652 | 1850 | 652 | 1850 |
| n (Samp) | 383 | 92 | 451 | 20 | 297 | 79 |
| n (Patient) | 383 | 92 | 451 | 20 | 297 | 79 |

|  | At Enrollment | | |
|---|---|---|---|
|  | sCr or UO | sCr only | UO only |
| AUC | 0.69 | 0.74 | 0.69 |
| SE | 0.033 | 0.065 | 0.036 |
| p | 3.5E-9 | 2.0E-4 | 1.8E-7 |
| nCohort 1 | 383 | 451 | 297 |
| nCohort 2 | 92 | 20 | 79 |
| Cutoff 1 | 0.236 | 2.65 | 0.236 |
| Sens 1 | 71% | 70% | 71% |
| Spec 1 | 53% | 76% | 52% |
| Cutoff 2 | 0.0151 | 0.244 | 0.0151 |
| Sens 2 | 82% | 80% | 81% |
| Spec 2 | 40% | 50% | 37% |
| Cutoff 3 | 0.0109 | 0.0160 | 0.0109 |
| Sens 3 | 90% | 90% | 91% |
| Spec 3 | 33% | 39% | 28% |
| Cutoff 4 | 1.19 | 1.65 | 1.14 |
| Sens 4 | 58% | 75% | 57% |
| Spec 4 | 70% | 70% | 70% |
| Cutoff 5 | 3.20 | 4.17 | 3.20 |
| Sens 5 | 43% | 60% | 43% |
| Spec 5 | 80% | 80% | 80% |

(continued)

|  | At Enrollment | | |
|---|---|---|---|
|  | sCr or UO | sCr only | UO only |
| Cutoff 6 | 9.95 | 15.0 | 12.8 |
| Sens 6 | 30% | 40% | 28% |
| Spec 6 | 90% | 90% | 90% |
| OR Quart 2 | 2.4 | 1.5 | 2.2 |
| p Value | 0.035 | 0.66 | 0.086 |
| 95% CI of | 1.1 | 0.25 | 0.89 |
| OR Quart2 | 5.6 | 9.1 | 5.4 |
| OR Quart 3 | 2.7 | 0.99 | 3.1 |
| p Value | 0.016 | 0.99 | 0.011 |
| 95% CI of | 1.2 | 0.14 | 1.3 |
| OR Quart3 | 6.3 | 7.2 | 7.4 |
| OR Quart 4 | 6.4 | 7.1 | 6.1 |
| p Value | 3.1E-6 | 0.011 | 2.4E-5 |
| 95% CI of | 2.9 | 1.6 | 2.6 |
| OR Quart4 | 14 | 32 | 14 |

| **Insulin-like growth factor-binding protein 3** | | | | | | |
|---|---|---|---|---|---|---|
|  | sCr or UO | | sCr only | | UO only | |
|  | Cohort 1 | Cohort 2 | Cohort 1 | Cohort 2 | Cohort 1 | Cohort 2 |
| Median | 339 | 706 | 370 | 1120 | 364 | 793 |
| Average | 575 | 1170 | 652 | 1490 | 607 | 1230 |
| Stdev | 735 | 1640 | 939 | 1810 | 762 | 1740 |
| p(t-test) |  | 7.8E-8 |  | 1.7E-4 |  | 1.4E-6 |
| Min | 3.84 | 0.311 | 0.311 | 44.1 | 3.84 | 0.311 |
| Max | 7920 | 12500 | 12500 | 8160 | 7920 | 12500 |
| n (Samp) | 412 | 96 | 482 | 21 | 325 | 83 |
| n (Patient) | 412 | 96 | 482 | 21 | 325 | 83 |

|  | At Enrollment | | |
|---|---|---|---|
|  | sCr or UO | sCr only | UO only |
| AUC | 0.68 | 0.68 | 0.66 |
| SE | 0.032 | 0.066 | 0.035 |
| p | 5.3E-8 | 0.0050 | 3.1E-6 |
| nCohort 1 | 412 | 482 | 325 |
| nCohort 2 | 96 | 21 | 83 |
| Cutoff 1 | 371 | 425 | 369 |
| Sens 1 | 71% | 71% | 71% |
| Spec 1 | 54% | 54% | 50% |

(continued)

| | At Enrollment | | |
|---|---|---|---|
| | sCr or UO | sCr only | UO only |
| Cutoff 2 | 293 | 269 | 293 |
| Sens 2 | 80% | 81% | 81% |
| Spec 2 | 45% | 40% | 42% |
| Cutoff 3 | 114 | 108 | 125 |
| Sens 3 | 91% | 90% | 90% |
| Spec 3 | 26% | 23% | 23% |
| Cutoff 4 | 667 | 736 | 703 |
| Sens 4 | 52% | 52% | 52% |
| Spec 4 | 70% | 70% | 70% |
| Cutoff 5 | 1010 | 1070 | 1030 |
| Sens 5 | 41% | 52% | 40% |
| Spec 5 | 80% | 80% | 80% |
| Cutoff 6 | 1350 | 1470 | 1370 |
| Sens 6 | 28% | 38% | 29% |
| Spec 6 | 90% | 90% | 90% |
| OR Quart 2 | 1.7 | 0.99 | 2.1 |
| p Value | 0.17 | 0.99 | 0.075 |
| 95% CI of | 0.79 | 0.20 | 0.93 |
| OR Quart2 | 3.9 | 5.0 | 4.8 |
| OR Quart 3 | 2.8 | 1.3 | 2.2 |
| p Value | 0.0063 | 0.71 | 0.052 |
| 95% CI of | 1.3 | 0.29 | 0.99 |
| OR Quart3 | 6.0 | 6.1 | 5.1 |
| OR Quart 4 | 4.8 | 3.9 | 4.6 |
| p Value | 1.9E-5 | 0.041 | 1.1E-4 |
| 95% CI of | 2.4 | 1.1 | 2.1 |
| OR Quart4 | 10.0 | 14 | 10.0 |

| **Immunoglogulin G1** | | | | | | |
|---|---|---|---|---|---|---|
| | sCr or UO | | sCr only | | UO only | |
| | Cohort 1 | Cohort 2 | Cohort 1 | Cohort 2 | Cohort 1 | Cohort 2 |
| Median | 3190 | 7960 | 3630 | 14900 | 3200 | 7950 |
| Average | 5790 | 11100 | 6510 | 14000 | 5780 | 11500 |
| Stdev | 7390 | 11900 | 8530 | 10300 | 7370 | 12400 |
| p(t-test) | | 9.1E-8 | | 1.7E-4 | | 2.8E-7 |
| Min | 59.7 | 55.9 | 55.9 | 177 | 59.7 | 55.9 |
| Max | 80000 | 80000 | 80000 | 28800 | 80000 | 80000 |
| n (Samp) | 379 | 92 | 447 | 20 | 292 | 79 |

(continued)

| Immunoglogulin G1 | | | | | | |
|---|---|---|---|---|---|---|
| | sCr or UO | | sCr only | | UO only | |
| | Cohort 1 | Cohort 2 | Cohort 1 | Cohort 2 | Cohort 1 | Cohort 2 |
| n (Patient) | 379 | 92 | 447 | 20 | 292 | 79 |
| | | | | | | |
| | | At Enrollment | | | | |
| | | sCr or UO | sCr only | | UO only | |
| AUC | | 0.66 | 0.70 | | 0.67 | |
| SE | | 0.033 | 0.066 | | 0.036 | |
| p | | 9.5E-7 | 0.0021 | | 1.4E-6 | |
| nCohort 1 | | 379 | 447 | | 292 | |
| nCohort 2 | | 92 | 20 | | 79 | |
| Cutoff 1 | | 3420 | 7950 | | 3630 | |
| Sens 1 | | 71% | 70% | | 71% | |
| Spec 1 | | 53% | 75% | | 53% | |
| Cutoff 2 | | 1900 | 2280 | | 2320 | |
| Sens 2 | | 80% | 80% | | 81% | |
| Spec 2 | | 28% | 34% | | 36% | |
| Cutoff 3 | | 1420 | 1250 | | 1440 | |
| Sens 3 | | 90% | 90% | | 91% | |
| Spec 3 | | 21% | 16% | | 20% | |
| Cutoff 4 | | 6150 | 6670 | | 6150 | |
| Sens 4 | | 57% | 70% | | 57% | |
| Spec 4 | | 70% | 70% | | 70% | |
| Cutoff 5 | | 8420 | 9330 | | 8540 | |
| Sens 5 | | 47% | 60% | | 46% | |
| Spec 5 | | 80% | 80% | | 80% | |
| Cutoff 6 | | 13900 | 16200 | | 13400 | |
| Sens 6 | | 32% | 40% | | 35% | |
| Spec 6 | | 90% | 90% | | 90% | |
| OR Quart 2 | | 0.85 | 0.99 | | 0.75 | |
| p Value | | 0.68 | 0.99 | | 0.50 | |
| 95% CI of | | 0.39 | 0.20 | | 0.32 | |
| OR Quart2 | | 1.8 | 5.0 | | 1.7 | |
| OR Quart 3 | | 1.2 | 0.32 | | 1.3 | |
| p Value | | 0.60 | 0.33 | | 0.46 | |
| 95% CI of | | 0.59 | 0.033 | | 0.62 | |
| OR Quart3 | | 2.5 | 3.2 | | 2.9 | |
| OR Quart 4 | | 3.6 | 4.7 | | 3.5 | |

(continued)

|  | At Enrollment | | |
|---|---|---|---|
|  | sCr or UO | sCr only | UO only |
| p Value | 9.8E-5 | 0.018 | 4.7E-4 |
| 95% CI of | 1.9 | 1.3 | 1.7 |
| OR Quart4 | 6.9 | 17 | 7.1 |

**Immunoglogulin G2**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 8420 | 18400 | 9310 | 36400 | 9250 | 18300 |
| Average | 17300 | 46300 | 20800 | 69400 | 17000 | 48900 |
| Stdev | 30200 | 64200 | 37700 | 72400 | 28000 | 67700 |
| p(t-test) |  | 4.4E-10 |  | 1.3E-7 |  | 7.9E-10 |
| Min | 119 | 25.4 | 25.4 | 2380 | 334 | 25.4 |
| Max | 240000 | 240000 | 240000 | 203000 | 240000 | 240000 |
| n (Samp) | 379 | 92 | 447 | 20 | 292 | 79 |
| n (Patient) | 379 | 92 | 447 | 20 | 292 | 79 |

|  | At Enrollment | | |
|---|---|---|---|
|  | sCr or UO | sCr only | UO only |
| AUC | 0.69 | 0.72 | 0.69 |
| SE | 0.033 | 0.066 | 0.036 |
| p | 2.1E-8 | 8.8E-4 | 1.4E-7 |
| nCohort 1 | 379 | 447 | 292 |
| nCohort 2 | 92 | 20 | 79 |
| Cutoff 1 | 8630 | 13500 | 8840 |
| Sens 1 | 71% | 70% | 71% |
| Spec 1 | 52% | 64% | 50% |
| Cutoff 2 | 7050 | 7680 | 7950 |
| Sens 2 | 80% | 80% | 81% |
| Spec 2 | 44% | 43% | 46% |
| Cutoff 3 | 3980 | 4240 | 3980 |
| Sens 3 | 90% | 90% | 91% |
| Spec 3 | 21% | 21% | 20% |
| Cutoff 4 | 14400 | 15900 | 14500 |
| Sens 4 | 57% | 65% | 57% |
| Spec 4 | 70% | 70% | 70% |
| Cutoff 5 | 19800 | 22200 | 19800 |
| Sens 5 | 45% | 55% | 46% |
| Spec 5 | 80% | 80% | 80% |

(continued)

|  | At Enrollment | | |
|---|---|---|---|
|  | sCr or UO | sCr only | UO only |
| Cutoff 6 | 35300 | 41300 | 31100 |
| Sens 6 | 30% | 45% | 33% |
| Spec 6 | 90% | 90% | 90% |
| OR Quart 2 | 1.5 | 0.66 | 1.5 |
| p Value | 0.34 | 0.65 | 0.40 |
| 95% CI of | 0.67 | 0.11 | 0.61 |
| OR Quart2 | 3.2 | 4.0 | 3.5 |
| OR Quart 3 | 2.0 | 1.3 | 2.1 |
| p Value | 0.071 | 0.71 | 0.078 |
| 95% CI of | 0.94 | 0.29 | 0.92 |
| OR Quart3 | 4.3 | 6.1 | 4.8 |
| OR Quart 4 | 4.7 | 3.9 | 5.2 |
| p Value | 2.0E-5 | 0.040 | 3.4E-5 |
| 95% CI of | 2.3 | 1.1 | 2.4 |
| OR Quart4 | 9.5 | 14 | 11 |

**Interleukin-11**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 122 | 203 | 129 | 258 | 139 | 196 |
| Average | 194 | 364 | 217 | 438 | 209 | 354 |
| Stdev | 247 | 441 | 288 | 499 | 260 | 424 |
| p(t-test) |  | 9.3E-7 |  | 0.0013 |  | 1.9E-4 |
| Min | 0.154 | 28.3 | 0.154 | 41.6 | 2.83 | 28.3 |
| Max | 2260 | 2140 | 2260 | 2020 | 2260 | 2140 |
| n (Samp) | 385 | 91 | 452 | 20 | 298 | 78 |
| n (Patient) | 385 | 91 | 452 | 20 | 298 | 78 |

|  | At Enrollment | | |
|---|---|---|---|
|  | sCr or UO | sCr only | UO only |
| AUC | 0.66 | 0.71 | 0.63 |
| SE | 0.034 | 0.066 | 0.037 |
| p | 1.2E-6 | 0.0019 | 5.6E-4 |
| nCohort 1 | 385 | 452 | 298 |
| nCohort 2 | 91 | 20 | 78 |
| Cutoff 1 | 117 | 201 | 113 |
| Sens 1 | 70% | 70% | 71% |
| Spec 1 | 49% | 70% | 42% |

(continued)

| | At Enrollment | | |
|---|---|---|---|
| | sCr or UO | sCr only | UO only |
| Cutoff 2 | 94.5 | 179 | 94.5 |
| Sens 2 | 80% | 80% | 81% |
| Spec 2 | 42% | 65% | 35% |
| Cutoff 3 | 63.9 | 56.7 | 64.5 |
| Sens 3 | 90% | 90% | 91% |
| Spec 3 | 29% | 23% | 24% |
| Cutoff 4 | 185 | 203 | 203 |
| Sens 4 | 54% | 65% | 47% |
| Spec 4 | 70% | 70% | 70% |
| Cutoff 5 | 253 | 286 | 274 |
| Sens 5 | 41% | 40% | 38% |
| Spec 5 | 80% | 80% | 80% |
| Cutoff 6 | 416 | 479 | 436 |
| Sens 6 | 22% | 25% | 22% |
| Spec 6 | 90% | 90% | 90% |
| OR Quart 2 | 2.9 | 0.33 | 2.7 |
| p Value | 0.010 | 0.34 | 0.015 |
| 95% CI of | 1.3 | 0.034 | 1.2 |
| OR Quart2 | 6.6 | 3.2 | 6.1 |
| OR Quart 3 | 2.6 | 1.7 | 1.5 |
| p Value | 0.022 | 0.48 | 0.38 |
| 95% CI of | 1.1 | 0.40 | 0.62 |
| OR Quart3 | 6.0 | 7.3 | 3.5 |
| OR Quart 4 | 5.7 | 3.9 | 4.1 |
| p Value | 1.2E-5 | 0.039 | 3.9E-4 |
| 95% CI of | 2.6 | 1.1 | 1.9 |
| OR Quart4 | 13 | 15 | 9.1 |

**Interleukin-2 receptor alpha chain**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 518 | 937 | 566 | 1560 | 524 | 913 |
| Average | 922 | 1840 | 1040 | 2330 | 943 | 1910 |
| Stdev | 1120 | 2190 | 1310 | 2770 | 1100 | 2260 |
| p(t-test) | | 2.1E-8 | | 6.2E-5 | | 1.6E-7 |
| Min | 0.0339 | 0.0482 | 0.0339 | 0.0482 | 0.0339 | 0.115 |
| Max | 8370 | 10100 | 9660 | 10100 | 6540 | 10100 |
| n (Samp) | 385 | 92 | 453 | 20 | 298 | 79 |

| Interleukin-2 receptor alpha chain | | | | | | |
|---|---|---|---|---|---|---|
| | sCr or UO | | sCr only | | UO only | |
| | Cohort 1 | Cohort 2 | Cohort 1 | Cohort 2 | Cohort 1 | Cohort 2 |
| n (Patient) | 385 | 92 | 453 | 20 | 298 | 79 |

| | At Enrollment | | |
|---|---|---|---|
| | sCr or UO | sCr only | UO only |
| AUC | 0.64 | 0.61 | 0.65 |
| SE | 0.034 | 0.068 | 0.037 |
| p | 3.4E-5 | 0.096 | 7.0E-5 |
| nCohort 1 | 385 | 453 | 298 |
| nCohort 2 | 92 | 20 | 79 |
| Cutoff 1 | 467 | 385 | 484 |
| Sens 1 | 71% | 70% | 71% |
| Spec 1 | 48% | 41% | 48% |
| Cutoff 2 | 325 | 60.5 | 333 |
| Sens 2 | 80% | 80% | 81% |
| Spec 2 | 41% | 13% | 39% |
| Cutoff 3 | 91.3 | 0.120 | 152 |
| Sens 3 | 90% | 90% | 91% |
| Spec 3 | 17% | 2% | 23% |
| Cutoff 4 | 1090 | 1170 | 1110 |
| Sens 4 | 42% | 60% | 41% |
| Spec 4 | 70% | 70% | 70% |
| Cutoff 5 | 1600 | 1690 | 1660 |
| Sens 5 | 36% | 50% | 33% |
| Spec 5 | 80% | 80% | 80% |
| Cutoff 6 | 2280 | 2590 | 2390 |
| Sens 6 | 26% | 30% | 27% |
| Spec 6 | 90% | 90% | 90% |
| OR Quart 2 | 1.6 | 0.39 | 2.4 |
| p Value | 0.24 | 0.27 | 0.058 |
| 95% CI of | 0.73 | 0.074 | 0.97 |
| OR Quart2 | 3.5 | 2.0 | 5.8 |
| OR Quart 3 | 2.9 | 0.59 | 4.1 |
| p Value | 0.0045 | 0.48 | 0.0012 |
| 95% CI of | 1.4 | 0.14 | 1.7 |
| OR Quart3 | 6.0 | 2.5 | 9.7 |
| OR Quart 4 | 3.4 | 2.1 | 4.5 |

(continued)

|  | At Enrollment | | |
|---|---|---|---|
|  | sCr or UO | sCr only | UO only |
| p Value | 8.9E-4 | 0.20 | 5.2E-4 |
| 95% CI of | 1.6 | 0.69 | 1.9 |
| OR Quart4 | 6.9 | 6.3 | 10 |

**Neutrophil collagenase**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 3190 | 9770 | 3750 | 21900 | 3260 | 10400 |
| Average | 13100 | 49700 | 16000 | 113000 | 11100 | 46700 |
| Stdev | 44400 | 112000 | 52900 | 169000 | 27800 | 99400 |
| p(t-test) |  | 4.0E-7 |  | 5.7E-12 |  | 2.6E-8 |
| Min | 0.114 | 25.8 | 0.114 | 25.8 | 0.114 | 198 |
| Max | 670000 | 670000 | 670000 | 649000 | 300000 | 670000 |
| n (Samp) | 412 | 96 | 482 | 21 | 326 | 83 |
| n (Patient) | 412 | 96 | 482 | 21 | 326 | 83 |

|  | At Enrollment | | |
|---|---|---|---|
|  | sCr or UO | sCr only | UO only |
| AUC | 0.71 | 0.72 | 0.72 |
| SE | 0.032 | 0.064 | 0.034 |
| p | 1.1E-10 | 7.6E-4 | 5.9E-11 |
| nCohort 1 | 412 | 482 | 326 |
| nCohort 2 | 96 | 21 | 83 |
| Cutoff 1 | 4980 | 8140 | 5380 |
| Sens 1 | 71% | 71% | 71% |
| Spec 1 | 60% | 68% | 63% |
| Cutoff 2 | 2810 | 2810 | 3170 |
| Sens 2 | 80% | 81% | 81% |
| Spec 2 | 48% | 44% | 49% |
| Cutoff 3 | 1020 | 191 | 1630 |
| Sens 3 | 91% | 90% | 90% |
| Spec 3 | 27% | 5% | 35% |
| Cutoff 4 | 7060 | 8960 | 6920 |
| Sens 4 | 66% | 67% | 66% |
| Spec 4 | 70% | 70% | 70% |
| Cutoff 5 | 11900 | 15600 | 10700 |
| Sens 5 | 46% | 62% | 49% |
| Spec 5 | 80% | 80% | 80% |

| | At Enrollment | | |
|---|---|---|---|
| | sCr or UO | sCr only | UO only |
| Cutoff 6 | 22200 | 30800 | 21900 |
| Sens 6 | 34% | 48% | 36% |
| Spec 6 | 90% | 90% | 90% |
| OR Quart 2 | 1.7 | 0.24 | 3.0 |
| p Value | 0.22 | 0.21 | 0.030 |
| 95% CI of | 0.73 | 0.027 | 1.1 |
| OR Quart2 | 3.9 | 2.2 | 8.0 |
| OR Quart 3 | 3.0 | 0.74 | 4.4 |
| p Value | 0.0054 | 0.69 | 0.0022 |
| 95% CI of | 1.4 | 0.16 | 1.7 |
| OR Quart3 | 6.5 | 3.4 | 11 |
| OR Quart 4 | 6.2 | 3.5 | 9.8 |
| p Value | 1.4E-6 | 0.033 | 1.1E-6 |
| 95% CI of | 3.0 | 1.1 | 3.9 |
| OR Quart4 | 13 | 11 | 24 |

**Protransforming growth factor alpha**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 6.28 | 12.5 | 6.98 | 15.7 | 6.62 | 12.3 |
| Average | 11.2 | 17.0 | 11.7 | 28.1 | 12.3 | 14.7 |
| Stdev | 24.0 | 18.0 | 22.5 | 32.1 | 26.8 | 10.2 |
| p(t-test) | | 0.030 | | 0.0019 | | 0.45 |
| Min | 0.00262 | 0.00454 | 0.00262 | 1.41 | 0.00262 | 0.00454 |
| Max | 361 | 122 | 361 | 122 | 361 | 50.4 |
| n (Samp) | 385 | 92 | 453 | 20 | 298 | 79 |
| n (Patient) | 385 | 92 | 453 | 20 | 298 | 79 |

| | At Enrollment | | |
|---|---|---|---|
| | sCr or UO | sCr only | UO only |
| AUC | 0.71 | 0.74 | 0.69 |
| SE | 0.033 | 0.065 | 0.036 |
| p | 3.0E-10 | 2.9E-4 | 1.5E-7 |
| nCohort 1 | 385 | 453 | 298 |
| nCohort 2 | 92 | 20 | 79 |
| Cutoff 1 | 8.32 | 11.3 | 8.57 |
| Sens 1 | 71% | 70% | 71% |
| Spec 1 | 62% | 69% | 61% |

(continued)

|  | At Enrollment | | |
|---|---|---|---|
|  | sCr or UO | sCr only | UO only |
| Cutoff 2 | 6.15 | 6.78 | 6.19 |
| Sens 2 | 80% | 80% | 81% |
| Spec 2 | 49% | 49% | 47% |
| Cutoff 3 | 4.83 | 4.92 | 4.83 |
| Sens 3 | 90% | 90% | 91% |
| Spec 3 | 42% | 37% | 39% |
| Cutoff 4 | 10.4 | 11.6 | 10.8 |
| Sens 4 | 61% | 65% | 59% |
| Spec 4 | 70% | 70% | 70% |
| Cutoff 5 | 13.8 | 15.2 | 14.7 |
| Sens 5 | 41% | 60% | 37% |
| Spec 5 | 80% | 80% | 80% |
| Cutoff 6 | 21.9 | 23.8 | 24.9 |
| Sens 6 | 20% | 35% | 10% |
| Spec 6 | 90% | 90% | 90% |
| OR Quart 2 | 2.1 | 4.1 | 2.0 |
| p Value | 0.12 | 0.21 | 0.16 |
| 95% CI of | 0.83 | 0.45 | 0.76 |
| OR Quart2 | 5.5 | 37 | 5.2 |
| OR Quart 3 | 5.9 | 2.0 | 5.3 |
| p Value | 5.9E-5 | 0.57 | 2.4E-4 |
| 95% CI of | 2.5 | 0.18 | 2.2 |
| OR Quart3 | 14 | 23 | 13 |
| OR Quart 4 | 7.7 | 14 | 6.0 |
| p Value | 2.8E-6 | 0.011 | 6.6E-5 |
| 95% CI of | 3.3 | 1.8 | 2.5 |
| OR Quart4 | 18 | 110 | 15 |

| CA 15-3 | | | | | | |
|---|---|---|---|---|---|---|
|  | sCr or UO | | sCr only | | UO only | |
|  | Cohort 1 | Cohort 2 | Cohort 1 | Cohort 2 | Cohort 1 | Cohort 2 |
| Median | 434 | 434 | nd | nd | 434 | 434 |
| Average | 300 | 382 | nd | nd | 303 | 391 |
| Stdev | 250 | 216 | nd | nd | 251 | 213 |
| p(t-test) |  | 0.070 | nd | nd |  | 0.059 |
| Min | 2.21 | 4.72 | nd | nd | 2.21 | 4.72 |
| Max | 784 | 784 | nd | nd | 784 | 784 |
| n (Samp) | 169 | 36 | nd | nd | 150 | 34 |

(continued)

| CA 15-3 | | | | | | |
|---|---|---|---|---|---|---|
| | sCr or UO | | sCr only | | UO only | |
| | Cohort 1 | Cohort 2 | Cohort 1 | Cohort 2 | Cohort 1 | Cohort 2 |
| n (Patient) | 169 | 36 | nd | nd | 150 | 34 |
| | | | | | | |
| | | At Enrollment | | | | |
| | | sCr or UO | sCr only | | UO only | |
| AUC | | 0.62 | nd | | 0.62 | |
| SE | | 0.054 | nd | | 0.056 | |
| p | | 0.033 | nd | | 0.028 | |
| nCohort 1 | | 169 | nd | | 150 | |
| nCohort 2 | | 36 | nd | | 34 | |
| Cutoff 1 | | 427 | nd | | 427 | |
| Sens 1 | | 75% | nd | | 76% | |
| Spec 1 | | 49% | nd | | 48% | |
| Cutoff 2 | | 80.1 | nd | | 80.1 | |
| Sens 2 | | 81% | nd | | 82% | |
| Spec 2 | | 36% | nd | | 37% | |
| Cutoff 3 | | 36.1 | nd | | 51.3 | |
| Sens 3 | | 92% | nd | | 91% | |
| Spec 3 | | 24% | nd | | 31% | |
| Cutoff 4 | | 434 | nd | | 434 | |
| Sens 4 | | 14% | nd | | 15% | |
| Spec 4 | | 88% | nd | | 88% | |
| Cutoff 5 | | 434 | nd | | 434 | |
| Sens 5 | | 14% | nd | | 15% | |
| Spec 5 | | 88% | nd | | 88% | |
| Cutoff 6 | | 784 | nd | | 784 | |
| Sens 6 | | 0% | nd | | 0% | |
| Spec 6 | | 100% | nd | | 100% | |
| OR Quart 2 | | 1.3 | nd | | 2.1 | |
| p Value | | 0.73 | nd | | 0.30 | |
| 95% CI of | | 0.32 | nd | | 0.50 | |
| OR Quart2 | | 5.1 | nd | | 9.2 | |
| OR Quart 3 | | 8.9 | nd | | 11 | |
| p Value | | 2.2E-4 | nd | | 3.2E-4 | |
| 95% CI of | | 2.8 | nd | | 3.0 | |
| OR Quart3 | | 28 | nd | | 41 | |
| OR Quart 4 | | 1.2 | nd | | 1.7 | |

(continued)

|  | At Enrollment | | |
|---|---|---|---|
|  | sCr or UO | sCr only | UO only |
| p Value | 0.75 | nd | 0.46 |
| 95% CI of | 0.32 | nd | 0.39 |
| OR Quart4 | 4.9 | nd | 7.8 |

Table 12: Comparison of marker levels in enroll EDTA samples collected from Cohort 1 (patients that did not progress beyond RIFLE stage 0 or R within 48hrs) and in enroll EDTA samples collected from Cohort 2 (subjects reaching RIFLE stage I or F within 48hrs). Enroll samples from patients already at stage I or F were included in Cohort 2.

| C-C motif chemokine 18 | | | | | | |
|---|---|---|---|---|---|---|
|  | sCr or UO | | sCr only | | UO only | |
|  | Cohort 1 | Cohort 2 | Cohort 1 | Cohort 2 | Cohort 1 | Cohort 2 |
| Median | 171 | 222 | nd | nd | 160 | 241 |
| Average | 238 | 264 | nd | nd | 230 | 269 |
| Stdev | 192 | 149 | nd | nd | 180 | 151 |
| p(t-test) |  | 0.51 | nd | nd |  | 0.32 |
| Min | 48.2 | 103 | nd | nd | 48.2 | 103 |
| Max | 1020 | 682 | nd | nd | 817 | 682 |
| n (Samp) | 109 | 28 | nd | nd | 98 | 25 |
| n (Patient) | 109 | 28 | nd | nd | 98 | 25 |

|  | At Enrollment | | |
|---|---|---|---|
|  | sCr or UO | sCr only | UO only |
| AUC | 0.61 | nd | 0.63 |
| SE | 0.062 | nd | 0.065 |
| p | 0.078 | nd | 0.047 |
| nCohort 1 | 109 | nd | 98 |
| nCohort 2 | 28 | nd | 25 |
| Cutoff 1 | 157 | nd | 158 |
| Sens 1 | 71% | nd | 72% |
| Spec 1 | 48% | nd | 50% |
| Cutoff 2 | 150 | nd | 151 |
| Sens 2 | 82% | nd | 80% |
| Spec 2 | 45% | nd | 48% |
| Cutoff 3 | 117 | nd | 117 |
| Sens 3 | 93% | nd | 92% |
| Spec 3 | 31% | nd | 31% |
| Cutoff 4 | 264 | nd | 260 |
| Sens 4 | 39% | nd | 44% |

(continued)

| | At Enrollment | | | |
|---|---|---|---|---|
| | sCr or UO | | sCr only | UO only |
| Spec 4 | 71% | | nd | 70% |
| Cutoff 5 | 340 | | nd | 321 |
| Sens 5 | 25% | | nd | 24% |
| Spec 5 | 81% | | nd | 81% |
| Cutoff 6 | 548 | | nd | 548 |
| Sens 6 | 4% | | nd | 4% |
| Spec 6 | 91% | | nd | 91% |
| OR Quart 2 | 4.9 | | nd | 3.4 |
| p Value | 0.056 | | nd | 0.16 |
| 95% CI of | 0.96 | | nd | 0.62 |
| OR Quart2 | 25 | | nd | 18 |
| OR Quart 3 | 5.8 | | nd | 5.7 |
| p Value | | 0.034 | nd | 0.036 |
| 95% CI of | | 1.1 | nd | 1.1 |
| OR Quart3 | | 29 | nd | 29 |
| OR Quart 4 | | 5.5 | nd | 4.9 |
| p Value | | 0.038 | nd | 0.059 |
| 95% CI of | | 1.1 | nd | 0.94 |
| OR Quart4 | | 28 | nd | 25 |

| **C-C motif chemokine 24** | | | | | | |
|---|---|---|---|---|---|---|
| | sCr or UO | | sCr only | | UO only | |
| | Cohort 1 | Cohort 2 | Cohort 1 | Cohort 2 | Cohort 1 | Cohort 2 |
| Median | 268 | 224 | 257 | 509 | 275 | 179 |
| Average | 445 | 370 | 419 | 569 | 468 | 291 |
| Stdev | 477 | 370 | 455 | 465 | 495 | 298 |
| p(t-test) | | 0.46 | | 0.40 | | 0.11 |
| Min | 0.0260 | 12.4 | 0.0260 | 68.0 | 13.1 | 12.4 |
| Max | 2920 | 1290 | 2920 | 1290 | 2920 | 1100 |
| n (Samp) | 89 | 26 | 108 | 7 | 81 | 22 |
| n (Patient) | 89 | 26 | 108 | 7 | 81 | 22 |

| | At Enrollment | | |
|---|---|---|---|
| | sCr or UO | sCr only | UO only |
| AUC | 0.44 | 0.62 | 0.37 |
| SE | 0.065 | 0.12 | 0.070 |
| p | 0.40 | 0.29 | 0.066 |
| nCohort 1 | 89 | 108 | 81 |

(continued)

| | At Enrollment | | |
|---|---|---|---|
| | sCr or UO | sCr only | UO only |
| nCohort 2 | 26 | 7 | 22 |
| Cutoff 1 | 94.1 | 279 | 86.2 |
| Sens 1 | 73% | 71% | 73% |
| Spec 1 | 17% | 56% | 14% |
| Cutoff 2 | 67.2 | 169 | 39.1 |
| Sens 2 | 81% | 86% | 82% |
| Spec 2 | 9% | 36% | 4% |
| Cutoff 3 | 25.7 | 67.2 | 25.7 |
| Sens 3 | 92% | 100% | 91% |
| Spec 3 | 3% | 12% | 2% |
| Cutoff 4 | 511 | 490 | 542 |
| Sens 4 | 27% | 57% | 14% |
| Spec 4 | 71% | 70% | 70% |
| Cutoff 5 | 737 | 668 | 761 |
| Sens 5 | 15% | 29% | 9% |
| Spec 5 | 81% | 81% | 80% |
| Cutoff 6 | 1110 | 1080 | 1110 |
| Sens 6 | 4% | 29% | 0% |
| Spec 6 | 91% | 91% | 90% |
| OR Quart 2 | 1.5 | 0.96 | 1.8 |
| p Value | 0.52 | 0.98 | 0.45 |

| 95% CI of | 0.42 | | 0.057 | 0.39 |
|---|---|---|---|---|
| OR Quart2 | 5.5 | | 16 | 8.6 |
| OR Quart 3 | 1.0 | | 3.1 | 1.8 |
| p Value | 1.0 | | 0.34 | 0.45 |
| 95% CI of | 0.26 | | 0.30 | 0.39 |
| OR Quart3 | 3.9 | | 32 | 8.6 |
| OR Quart 4 | 2.3 | | 2.0 | 4.3 |
| p Value | 0.20 | | 0.58 | 0.049 |
| 95% CI of | 0.65 | | 0.17 | 1.0 |
| OR Quart4 | 7.9 | | 23 | 18 |

**C-C motif chemokine 8**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 12.8 | 15.4 | 14.0 | 14.4 | 12.8 | 15.7 |
| Average | 21.9 | 51.7 | 21.2 | 143 | 22.4 | 57.6 |
| Stdev | 31.4 | 144 | 29.0 | 270 | 32.9 | 156 |

(continued)

| C-C motif chemokine 8 | | | | | | |
|---|---|---|---|---|---|---|
| | sCr or UO | | sCr only | | UO only | |
| | Cohort 1 | Cohort 2 | Cohort 1 | Cohort 2 | Cohort 1 | Cohort 2 |
| p(t-test) | | 0.070 | | 1.3E-5 | | 0.060 |
| Min | 0.162 | 6.24 | 0.162 | 12.4 | 0.162 | 6.24 |
| Max | 177 | 740 | 177 | 740 | 177 | 740 |
| n (Samp) | 89 | 26 | 108 | 7 | 81 | 22 |
| n (Patient) | 89 | 26 | 108 | 7 | 81 | 22 |
| | | | | | | |
| | At Enrollment | | | | | |
| | sCr or UO | | sCr only | | UO only | |
| AUC | 0.60 | | 0.68 | | 0.61 | |
| SE | 0.065 | | 0.11 | | 0.070 | |
| p | 0.13 | | 0.11 | | 0.11 | |
| nCohort 1 | 89 | | 108 | | 81 | |
| nCohort 2 | 26 | | 7 | | 22 | |
| Cutoff 1 | 13.0 | | 13.7 | | 13.7 | |
| Sens 1 | 73% | | 71% | | 73% | |
| Spec 1 | 51% | | 47% | | 54% | |
| Cutoff 2 | 12.4 | | 13.0 | | 12.3 | |
| Sens 2 | 81% | | 86% | | 82% | |
| Spec 2 | 45% | | 47% | | 47% | |
| Cutoff 3 | 9.32 | | 12.3 | | 9.32 | |
| Sens 3 | 92% | | 100% | | 91% | |
| Spec 3 | 27% | | 41% | | 28% | |
| Cutoff 4 | 19.9 | | 18.9 | | 18.9 | |
| Sens 4 | 27% | | 43% | | 27% | |
| Spec 4 | 71% | | 70% | | 70% | |
| Cutoff 5 | 22.4 | | 22.4 | | 22.4 | |
| Sens 5 | 27% | | 43% | | 27% | |
| Spec 5 | 81% | | 81% | | 80% | |
| Cutoff 6 | 34.9 | | 33.4 | | 34.9 | |
| Sens 6 | 15% | | 43% | | 14% | |
| Spec 6 | 91% | | 91% | | 90% | |
| OR Quart 2 | 2.7 | | >3.2 | | 2.7 | |
| p Value | 0.19 | | <0.32 | | 0.26 | |
| 95% CI of | 0.61 | | >0.32 | | 0.48 | |
| OR Quart2 | 12 | | na | | 16 | |
| OR Quart 3 | 3.8 | | >1.0 | | 6.1 | |

(continued)

| | At Enrollment | | |
|---|---|---|---|
| | sCr or UO | sCr only | UO only |
| p Value | 0.071 | <1.0 | 0.032 |
| 95% CI of | 0.89 | >0.060 | 1.2 |
| OR Quart3 | 16 | na | 32 |
| OR Quart 4 | 2.7 | >3.2 | 3.4 |
| p Value | 0.19 | <0.32 | 0.16 |
| 95% CI of | 0.61 | >0.32 | 0.62 |
| OR Quart4 | 12 | na | 19 |

**Cathepsin D**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 234000 | 230000 | nd | nd | 240000 | 244000 |
| Average | 266000 | 284000 | nd | nd | 278000 | 279000 |
| Stdev | 146000 | 165000 | nd | nd | 153000 | 154000 |
| p(t-test) | | 0.55 | nd | nd | | 0.99 |
| Min | 34100 | 126000 | nd | nd | 54000 | 126000 |
| Max | 802000 | 655000 | nd | nd | 802000 | 618000 |
| n (Samp) | 109 | 28 | nd | nd | 98 | 25 |
| n (Patient) | 109 | 28 | nd | nd | 98 | 25 |

| | At Enrollment | | |
|---|---|---|---|
| | sCr or UO | sCr only | UO only |
| AUC | 0.53 | nd | 0.50 |
| SE | 0.062 | nd | 0.065 |
| p | 0.66 | nd | 1.0 |
| nCohort 1 | 109 | nd | 98 |
| nCohort 2 | 28 | nd | 25 |
| Cutoff 1 | 170000 | nd | 170000 |
| Sens 1 | 71% | nd | 72% |
| Spec 1 | 36% | nd | 34% |
| Cutoff 2 | 144000 | nd | 144000 |
| Sens 2 | 82% | nd | 80% |
| Spec 2 | 21% | nd | 18% |
| Cutoff 3 | 135000 | nd | 130000 |
| Sens 3 | 93% | nd | 92% |
| Spec 3 | 17% | nd | 14% |
| Cutoff 4 | 324000 | nd | 333000 |
| Sens 4 | 29% | nd | 28% |

(continued)

| | At Enrollment | | |
|---|---|---|---|
| | sCr or UO | sCr only | UO only |
| Spec 4 | 71% | nd | 70% |
| Cutoff 5 | 375000 | nd | 382000 |
| Sens 5 | 25% | nd | 24% |
| Spec 5 | 81% | nd | 81% |
| Cutoff 6 | 471000 | nd | 489000 |
| Sens 6 | 18% | nd | 16% |
| Spec 6 | 91% | nd | 91% |
| OR Quart 2 | 1.4 | nd | 0.82 |
| p Value | 0.55 | nd | 0.76 |
| 95% CI of | 0.44 | nd | 0.24 |
| OR Quart2 | 4.7 | nd | 2.8 |
| OR Quart 3 | 1.0 | nd | 0.82 |
| p Value | 1.0 | nd | 0.76 |
| 95% CI of | 0.29 | nd | 0.24 |
| OR Quart3 | 3.5 | nd | 2.8 |
| OR Quart 4 | 1.4 | nd | 0.86 |
| p Value | 0.59 | nd | 0.81 |
| 95% CI of | 0.42 | nd | 0.25 |
| OR Quart4 | 4.5 | nd | 2.9 |

**C-X-C motif chemokine 13**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 55.0 | 53.1 | 53.1 | 54.5 | 55.6 | 53.1 |
| Average | 155 | 212 | 139 | 612 | 164 | 239 |
| Stdev | 292 | 530 | 268 | 949 | 304 | 573 |
| p(t-test) | | 0.48 | | 5.5E-4 | | 0.41 |
| Min | 9.90 | 15.1 | 9.90 | 15.1 | 9.90 | 15.6 |
| Max | 1790 | 2000 | 1790 | 2000 | 1790 | 2000 |
| n (Samp) | 89 | 26 | 108 | 7 | 81 | 22 |
| n (Patient) | 89 | 26 | 108 | 7 | 81 | 22 |

| | At Enrollment | | |
|---|---|---|---|
| | sCr or UO | sCr only | UO only |
| AUC | 0.47 | 0.57 | 0.48 |
| SE | 0.065 | 0.12 | 0.070 |
| p | 0.67 | 0.55 | 0.76 |
| nCohort 1 | 89 | 108 | 81 |

(continued)

| | At Enrollment | | |
|---|---|---|---|
| | sCr or UO | sCr only | UO only |
| nCohort 2 | 26 | 7 | 22 |
| Cutoff 1 | 34.2 | 35.0 | 34.2 |
| Sens 1 | 73% | 71% | 73% |
| Spec 1 | 33% | 32% | 32% |
| Cutoff 2 | 28.5 | 34.2 | 28.5 |
| Sens 2 | 81% | 86% | 82% |
| Spec 2 | 25% | 32% | 23% |
| Cutoff 3 | 15.6 | 14.8 | 18.1 |
| Sens 3 | 92% | 100% | 91% |
| Spec 3 | 6% | 4% | 12% |
| Cutoff 4 | 114 | 101 | 116 |
| Sens 4 | 19% | 43% | 18% |
| Spec 4 | 71% | 70% | 70% |
| Cutoff 5 | 194 | 157 | 194 |
| Sens 5 | 12% | 29% | 14% |
| Spec 5 | 81% | 81% | 80% |
| Cutoff 6 | 271 | 267 | 271 |
| Sens 6 | 12% | 29% | 14% |
| Spec 6 | 91% | 91% | 90% |
| OR Quart 2 | 1.8 | 2.0 | 1.6 |
| p Value | 0.35 | 0.58 | 0.48 |
| 95% CI of | 0.52 | 0.17 | 0.41 |
| OR Quart2 | 6.5 | 23 | 6.7 |
| OR Quart 3 | 1.5 | 0.96 | 2.0 |
| p Value | 0.52 | 0.98 | 0.31 |
| 95% CI of | 0.42 | 0.057 | 0.51 |
| OR Quart3 | 5.5 | 16 | 8.0 |
| OR Quart 4 | 1.3 | 3.1 | 1.4 |
| p Value | 0.69 | 0.34 | 0.67 |
| 95% CI of | 0.35 | 0.30 | 0.32 |
| OR Quart4 | 4.9 | 32 | 5.8 |

**Insulin-like growth factor-binding protein 3**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 3010 | 3200 | nd | nd | 2940 | 2720 |
| Average | 3450 | 3420 | nd | nd | 3380 | 3240 |
| Stdev | 1530 | 1520 | nd | nd | 1500 | 1470 |

(continued)

| Insulin-like growth factor-binding protein 3 | | | | | | |
|---|---|---|---|---|---|---|
| | sCr or UO | | sCr only | | UO only | |
| | Cohort 1 | Cohort 2 | Cohort 1 | Cohort 2 | Cohort 1 | Cohort 2 |
| p(t-test) | | 0.93 | nd | nd | | 0.67 |
| Min | 730 | 1120 | nd | nd | 730 | 1120 |
| Max | 7370 | 7100 | nd | nd | 7260 | 7100 |
| n (Samp) | 109 | 28 | nd | nd | 98 | 25 |
| n (Patient) | 109 | 28 | nd | nd | 98 | 25 |

| | At Enrollment | | |
|---|---|---|---|
| | sCr or UO | sCr only | UO only |
| AUC | 0.50 | nd | 0.48 |
| SE | 0.061 | nd | 0.065 |
| p | 0.97 | nd | 0.70 |
| nCohort 1 | 109 | nd | 98 |
| nCohort 2 | 28 | nd | 25 |
| Cutoff 1 | 2410 | nd | 2370 |
| Sens 1 | 71% | nd | 72% |
| Spec 1 | 32% | nd | 32% |
| Cutoff 2 | 2050 | nd | 2050 |
| Sens 2 | 82% | nd | 80% |
| Spec 2 | 19% | nd | 20% |
| Cutoff 3 | 1400 | nd | 1400 |
| Sens 3 | 93% | nd | 92% |
| Spec 3 | 6% | nd | 6% |
| Cutoff 4 | 4410 | nd | 4350 |
| Sens 4 | 29% | nd | 24% |
| Spec 4 | 71% | nd | 70% |
| Cutoff 5 | 4970 | nd | 4900 |
| Sens 5 | 14% | nd | 12% |
| Spec 5 | 81% | nd | 81% |
| Cutoff 6 | 5550 | nd | 5520 |
| Sens 6 | 7% | nd | 4% |
| Spec 6 | 91% | nd | 91% |
| OR Quart 2 | 1.2 | nd | 1.5 |
| p Value | 0.72 | nd | 0.52 |
| 95% CI of | 0.39 | nd | 0.42 |
| OR Quart2 | 3.9 | nd | 5.4 |
| OR Quart 3 | 0.86 | nd | 1.2 |

(continued)

| | At Enrollment | | | | | |
|---|---|---|---|---|---|---|
| | sCr or UO | | sCr only | | UO only | |
| p Value | 0.80 | | nd | | 0.74 | |
| 95% CI of | 0.26 | | nd | | 0.34 | |
| OR Quart3 | 2.9 | | nd | | 4.6 | |
| OR Quart 4 | 1.0 | | nd | | 1.6 | |
| p Value | 0.95 | | nd | | 0.48 | |
| 95% CI of | 0.32 | | nd | | 0.44 | |
| OR Quart4 | 3.4 | | nd | | 5.7 | |
| **Immunoglogulin G1** | | | | | | |
| | sCr or UO | | sCr only | | UO only | |
| | Cohort 1 | Cohort 2 | Cohort 1 | Cohort 2 | Cohort 1 | Cohort 2 |
| Median | 8690000 | 1.04E7 | nd | nd | 8030000 | 1.04E7 |
| Average | 1.02E7 | 1.14E7 | nd | nd | 9810000 | 1.14E7 |
| Stdev | 5690000 | 5580000 | nd | nd | 5570000 | 5580000 |
| p(t-test) | | 0.44 | nd | nd | | 0.30 |
| Min | 3270000 | 3270000 | nd | nd | 3270000 | 3270000 |
| Max | 3.57E7 | 2.63E7 | nd | nd | 3.57E7 | 2.63E7 |
| n(Samp) | 81 | 17 | nd | nd | 74 | 17 |
| n (Patient) | 81 | 17 | nd | nd | 74 | 17 |
| | | | | | | |
| | At Enrollment | | | | | |
| | sCr or UO | | sCr only | | UO only | |
| AUC | 0.59 | | nd | | 0.61 | |
| SE | 0.079 | | nd | | 0.079 | |
| p | 0.24 | | nd | | 0.15 | |
| nCohort 1 | 81 | | nd | | 74 | |
| nCohort 2 | 17 | | nd | | 17 | |
| Cutoff 1 | 9280000 | | nd | | 9280000 | |
| Sens 1 | 71% | | nd | | 71% | |
| Spec 1 | 56% | | nd | | 59% | |
| Cutoff 2 | 7600000 | | nd | | 7600000 | |
| Sens 2 | 82% | | nd | | 82% | |
| Spec 2 | 43% | | nd | | 46% | |
| Cutoff 3 | 4390000 | | nd | | 4390000 | |
| Sens 3 | 94% | | nd | | 94% | |
| Spec 3 | 6% | | nd | | 7% | |
| Cutoff 4 | 1.11E7 | | nd | | 1.09E7 | |
| Sens 4 | 41% | | nd | | 47% | |

(continued)

|  | At Enrollment | | | | | |
|---|---|---|---|---|---|---|
|  | sCr or UO | | sCr only | | UO only | |
| Spec 4 | 70% | | nd | | 70% | |
| Cutoff 5 | 1.34E7 | | nd | | 1.32E7 | |
| Sens 5 | 18% | | nd | | 18% | |
| Spec 5 | 80% | | nd | | 81% | |
| Cutoff 6 | 1.65E7 | | nd | | 1.52E7 | |
| Sens 6 | 18% | | nd | | 18% | |
| Spec 6 | 90% | | nd | | 91% | |
| OR Quart 2 | 1.5 | | nd | | 1.5 | |
| p Value | 0.67 | | nd | | 0.67 | |
| 95% CI of | 0.23 | | nd | | 0.23 | |
| OR Quart2 | 9.9 | | nd | | 10.0 | |
| OR Quart 3 | 6.6 | | nd | | 5.3 | |
| p Value | 0.026 | | nd | | 0.052 | |
| 95% CI of | 1.2 | | nd | | 0.99 | |
| OR Quart3 | 35 | | nd | | 29 | |
| OR Quart 4 | 1.5 | | nd | | 2.1 | |
| p Value | 0.67 | | nd | | 0.42 | |
| 95% CI of | 0.23 | | nd | | 0.34 | |
| OR Quart4 | 9.9 | | nd | | 13 | |
| **Immunoglogulin G2** | | | | | | |
|  | sCr or UO | | sCr only | | UO only | |
|  | Cohort 1 | Cohort 2 | Cohort 1 | Cohort 2 | Cohort 1 | Cohort 2 |
| Median | 1.08E7 | 1.08E7 | nd | nd | 1.03E7 | 1.08E7 |
| Average | 1.38E7 | 1.11E7 | nd | nd | 1.32E7 | 1.11E7 |
| Stdev | 9680000 | 6680000 | nd | nd | 8830000 | 6680000 |
| p(t-test) |  | 0.28 | nd | nd |  | 0.37 |
| Min | 563000 | 2450000 | nd | nd | 1750000 | 2450000 |
| Max | 4.88E7 | 2.70E7 | nd | nd | 4.10E7 | 2.70E7 |
| n(Samp) | 81 | 17 | nd | nd | 74 | 17 |
| n (Patient) | 81 | 17 | nd | nd | 74 | 17 |
|  | | | | | | |
|  | At Enrollment | | | | | |
|  | sCr or UO | | sCr only | | UO only | |
| AUC | 0.44 | | nd | | 0.46 | |
| SE | 0.079 | | nd | | 0.079 | |
| p | 0.45 | | nd | | 0.58 | |
| nCohort 1 | 81 | | nd | | 74 | |

(continued)

| | At Enrollment | | | | |
|---|---|---|---|---|---|
| | sCr or UO | | sCr only | | UO only |
| nCohort 2 | 17 | | nd | | 17 |
| Cutoff 1 | 7850000 | | nd | | 7850000 |
| Sens 1 | 71% | | nd | | 71% |
| Spec 1 | 28% | | nd | | 30% |
| Cutoff 2 | 1750000 | | nd | | 1750000 |
| Sens 2 | 100% | | nd | | 100% |
| Spec 2 | 7% | | nd | | 7% |
| Cutoff 3 | 1750000 | | nd | | 1750000 |
| Sens 3 | 100% | | nd | | 100% |
| Spec 3 | 7% | | nd | | 7% |
| Cutoff 4 | 1.64E7 | | nd | | 1.64E7 |
| Sens 4 | 12% | | nd | | 12% |
| Spec 4 | 70% | | nd | | 73% |
| Cutoff 5 | 2.01E7 | | nd | | 1.84E7 |
| Sens 5 | 6% | | nd | | 6% |
| Spec 5 | 80% | | nd | | 81% |
| Cutoff 6 | 2.66E7 | | nd | | 2.55E7 |
| Sens 6 | 6% | | nd | | 6% |
| Spec 6 | 90% | | nd | | 91% |
| OR Quart 2 | 4.7 | | nd | | 4.6 |
| p Value | 0.072 | | nd | | 0.079 |
| 95% CI of | 0.87 | | nd | | 0.84 |
| OR Quart2 | 26 | | nd | | 25 |
| OR Quart 3 | 1.6 | | nd | | 2.2 |
| p Value | 0.64 | | nd | | 0.39 |
| 95% CI of | 0.24 | | nd | | 0.36 |
| OR Quart3 | 10 | | nd | | 13 |
| OR Quart 4 | 3.0 | | nd | | 2.3 |
| p Value | 0.21 | | nd | | 0.36 |
| 95% CI of | 0.53 | | nd | | 0.38 |
| OR Quart4 | 17 | | nd | | 14 |
| **Interleukin-11** | | | | | |
| | sCr or UO | | sCr only | | UO only | |
| | Cohort 1 | Cohort 2 | Cohort 1 | Cohort 2 | Cohort 1 | Cohort 2 |
| Median | 61.5 | 89.1 | 62.2 | 111 | 61.5 | 97.6 |
| Average | 97.6 | 146 | 95.1 | 316 | 98.2 | 142 |
| Stdev | 111 | 226 | 105 | 390 | 113 | 225 |

(continued)

| Interleukin-11 | | | | | | |
|---|---|---|---|---|---|---|
| | sCr or UO | | sCr only | | UO only | |
| | Cohort 1 | Cohort 2 | Cohort 1 | Cohort 2 | Cohort 1 | Cohort 2 |
| p(t-test) | | 0.14 | | 5.8E-5 | | 0.20 |
| Min | 0.359 | 0.480 | 0.359 | 0.920 | 0.359 | 0.480 |
| Max | 741 | 1060 | 741 | 1060 | 741 | 1060 |
| n (Samp) | 90 | 26 | 109 | 7 | 82 | 22 |
| n (Patient) | 90 | 26 | 109 | 7 | 82 | 22 |
| | | | | | | |
| | At Enrollment | | | | | |
| | sCr or UO | | sCr only | | UO only | |
| AUC | 0.53 | | 0.64 | | 0.54 | |
| SE | 0.065 | | 0.12 | | 0.070 | |
| p | 0.63 | | 0.22 | | 0.54 | |
| nCohort 1 | 90 | | 109 | | 82 | |
| nCohort 2 | 26 | | 7 | | 22 | |
| Cutoff 1 | 20.4 | | 47.4 | | 33.9 | |
| Sens 1 | 73% | | 71% | | 73% | |
| Spec 1 | 27% | | 46% | | 32% | |
| Cutoff 2 | 15.1 | | 20.4 | | 15.1 | |
| Sens 2 | 81% | | 86% | | 82% | |
| Spec 2 | 21% | | 28% | | 21% | |
| Cutoff 3 | 0.736 | | 0.736 | | 6.00 | |
| Sens 3 | 92% | | 100% | | 91% | |
| Spec 3 | 11% | | 11% | | 15% | |
| Cutoff 4 | 132 | | 132 | | 132 | |
| Sens 4 | 31% | | 43% | | 32% | |
| Spec 4 | 70% | | 71% | | 71% | |
| Cutoff 5 | 173 | | 171 | | 177 | |
| Sens 5 | 19% | | 43% | | 18% | |
| Spec 5 | 80% | | 81% | | 80% | |
| Cutoff 6 | 209 | | 209 | | 209 | |
| Sens 6 | 15% | | 43% | | 14% | |
| Spec 6 | 90% | | 91% | | 90% | |
| OR Quart 2 | 1.0 | | 2.1 | | 0.76 | |
| p Value | 1.0 | | 0.56 | | 0.71 | |
| 95% CI of | 0.28 | | 0.18 | | 0.18 | |
| OR Quart2 | 3.6 | | 24 | | 3.2 | |
| OR Quart 3 | 1.2 | | 1.0 | | 1.5 | |

(continued)

| | At Enrollment | | | | | |
|---|---|---|---|---|---|---|
| | sCr or UO | | sCr only | | UO only | |
| p Value | 0.75 | | 1.0 | | 0.51 | |
| 95% CI of | 0.35 | | 0.060 | | 0.42 | |
| OR Quart3 | 4.2 | | 17 | | 5.7 | |
| OR Quart 4 | 1.2 | | 3.2 | | 1.3 | |
| p Value | 0.75 | | 0.32 | | 0.73 | |
| 95% CI of | 0.35 | | 0.32 | | 0.33 | |
| OR Quart4 | 4.2 | | 33 | | 4.8 | |

**Interleukin-2 receptor alpha chain**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 25.5 | 27.4 | 25.4 | 33.6 | 28.1 | 27.4 |
| Average | 69.7 | 82.6 | 70.8 | 102 | 74.9 | 93.3 |
| Stdev | 141 | 146 | 139 | 193 | 147 | 157 |
| p(t-test) | | 0.68 | | 0.58 | | 0.61 |
| Min | 0.0290 | 0.0461 | 0.0290 | 0.0569 | 0.0290 | 0.0461 |
| Max | 918 | 562 | 918 | 533 | 918 | 562 |
| n (Samp) | 90 | 26 | 109 | 7 | 82 | 22 |
| n (Patient) | 90 | 26 | 109 | 7 | 82 | 22 |

| | At Enrollment | | | | | |
|---|---|---|---|---|---|---|
| | sCr or UO | | sCr only | | UO only | |
| AUC | 0.53 | | 0.51 | | 0.52 | |
| SE | 0.065 | | 0.11 | | 0.070 | |
| p | 0.69 | | 0.90 | | 0.73 | |
| nCohort 1 | 90 | | 109 | | 82 | |
| nCohort 2 | 26 | | 7 | | 22 | |
| Cutoff 1 | 5.04 | | 4.75 | | 10.6 | |
| Sens 1 | 73% | | 71% | | 73% | |
| Spec 1 | 30% | | 28% | | 32% | |
| Cutoff 2 | 0.0515 | | 0.0515 | | 0.0515 | |
| Sens 2 | 88% | | 100% | | 86% | |
| Spec 2 | 16% | | 16% | | 15% | |
| Cutoff 3 | 0.0359 | | 0.0515 | | 0.0359 | |
| Sens 3 | 100% | | 100% | | 100% | |
| Spec 3 | 9% | | 16% | | 7% | |
| Cutoff 4 | 50.7 | | 60.8 | | 52.5 | |
| Sens 4 | 42% | | 29% | | 45% | |

(continued)

| | At Enrollment | | | | | |
|---|---|---|---|---|---|---|
| | sCr or UO | | sCr only | | UO only | |
| Spec 4 | 70% | | 71% | | 71% | |
| Cutoff 5 | 93.9 | | 94.9 | | 104 | |
| Sens 5 | 19% | | 14% | | 18% | |
| Spec 5 | 80% | | 81% | | 80% | |
| Cutoff 6 | 153 | | 155 | | 155 | |
| Sens 6 | 15% | | 14% | | 14% | |
| Spec 6 | 90% | | 91% | | 90% | |
| OR Quart 2 | 1.2 | | 0.48 | | 1.3 | |
| p Value | 0.75 | | 0.56 | | 0.73 | |
| 95% CI of | 0.35 | | 0.041 | | 0.33 | |
| OR Quart2 | 4.2 | | 5.6 | | 4.8 | |
| OR Quart 3 | 1.0 | | 1.0 | | 1.0 | |
| p Value | 1.0 | | 1.0 | | 1.0 | |
| 95% CI of | 0.28 | | 0.13 | | 0.25 | |
| OR Quart3 | 3.6 | | 7.6 | | 4.0 | |
| OR Quart 4 | 1.2 | | 1.0 | | 1.3 | |
| p Value | 0.75 | | 1.0 | | 0.73 | |
| 95% CI of | 0.35 | | 0.13 | | 0.33 | |
| OR Quart4 | 4.2 | | 7.6 | | 4.8 | |

**Neutrophil collagenase**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 1260 | 1780 | nd | nd | 1190 | 2410 |
| Average | 3150 | 8240 | nd | nd | 3130 | 9130 |
| Stdev | 5690 | 17400 | nd | nd | 5890 | 18200 |
| p(t-test) | | 0.011 | nd | nd | | 0.0067 |
| Min | 83.9 | 1.14 | nd | nd | 1.14 | 241 |
| Max | 44900 | 83900 | nd | nd | 44900 | 83900 |
| n (Samp) | 108 | 28 | nd | nd | 97 | 25 |
| n (Patient) | 108 | 28 | nd | nd | 97 | 25 |

| | At Enrollment | | | | | |
|---|---|---|---|---|---|---|
| | sCr or UO | | sCr only | | UO only | |
| AUC | 0.58 | | nd | | 0.63 | |
| SE | 0.062 | | nd | | 0.065 | |
| p | 0.19 | | nd | | 0.041 | |
| nCohort 1 | 108 | | nd | | 97 | |
| nCohort 2 | 28 | | nd | | 25 | |

(continued)

|  | At Enrollment | | | | | |
| --- | --- | --- | --- | --- | --- | --- |
|  | sCr or UO | | sCr only | | UO only | |
| Cutoff 1 | 1040 | | nd | | 1130 | |
| Sens 1 | 71% | | nd | | 72% | |
| Spec 1 | 41% | | nd | | 49% | |
| Cutoff 2 | 810 | | nd | | 994 | |
| Sens 2 | 82% | | nd | | 80% | |
| Spec 2 | 29% | | nd | | 41% | |
| Cutoff 3 | 261 | | nd | | 712 | |
| Sens 3 | 93% | | nd | | 92% | |
| Spec 3 | 6% | | nd | | 30% | |
| Cutoff 4 | 2370 | | nd | | 2340 | |
| Sens 4 | 46% | | nd | | 52% | |
| Spec 4 | 70% | | nd | | 70% | |
| Cutoff 5 | 3750 | | nd | | 3580 | |
| Sens 5 | 29% | | nd | | 32% | |
| Spec 5 | 81% | | nd | | 80% | |
| Cutoff 6 | 7620 | | nd | | 7370 | |
| Sens 6 | 21% | | nd | | 24% | |
| Spec 6 | 91% | | nd | | 91% | |
| OR Quart 2 | 2.7 | | nd | | 5.7 | |
| p Value | 0.13 | | nd | | 0.036 | |
| 95% CI of | 0.74 | | nd | | 1.1 | |
| OR Quart2 | 9.8 | | nd | | 29 | |
| OR Quart 3 | 1.6 | | nd | | 2.8 | |
| p Value | 0.50 | | nd | | 0.24 | |
| 95% CI of | 0.41 | | nd | | 0.50 | |
| OR Quart3 | 6.3 | | nd | | 16 | |
| OR Quart 4 | 2.7 | | nd | | 5.7 | |
| p Value | 0.13 | | nd | | 0.036 | |
| 95% CI of | 0.74 | | nd | | 1.1 | |
| OR Quart4 | 9.8 | | nd | | 29 | |
| **Protransforming growth factor alpha** | | | | | | |
|  | sCr or UO | | sCr only | | UO only | |
|  | Cohort 1 | Cohort 2 | Cohort 1 | Cohort 2 | Cohort 1 | Cohort 2 |
| Median | 1.47 | 4.03 | 1.70 | 7.33 | 1.40 | 4.03 |
| Average | 8.43 | 12.3 | 8.77 | 17.3 | 8.98 | 12.7 |
| Stdev | 32.1 | 18.1 | 30.0 | 22.4 | 33.6 | 19.0 |
| p(t-test) |  | 0.56 |  | 0.46 |  | 0.62 |

(continued)

| Protransforming growth factor alpha | | | | | | |
|---|---|---|---|---|---|---|
| | sCr or UO | | sCr only | | UO only | |
| | Cohort 1 | Cohort 2 | Cohort 1 | Cohort 2 | Cohort 1 | Cohort 2 |
| Min | 0.00228 | 0.00228 | 0.00228 | 0.227 | 0.00228 | 0.00228 |
| Max | 245 | 68.3 | 245 | 62.6 | 245 | 68.3 |
| n (Samp) | 90 | 26 | 109 | 7 | 82 | 22 |
| n (Patient) | 90 | 26 | 109 | 7 | 82 | 22 |
| | At Enrollment | | | | | |
| | sCr or UO | | sCr only | | UO only | |
| AUC | 0.68 | | 0.76 | | 0.69 | |
| SE | 0.063 | | 0.11 | | 0.068 | |
| p | 0.0044 | | 0.018 | | 0.0058 | |
| nCohort 1 | 90 | | 109 | | 82 | |
| nCohort 2 | 26 | | 7 | | 22 | |
| Cutoff 1 | 1.78 | | 5.22 | | 1.78 | |
| Sens 1 | 73% | | 71% | | 73% | |
| Spec 1 | 56% | | 83% | | 59% | |
| Cutoff 2 | 0.885 | | 2.57 | | 0.885 | |
| Sens 2 | 81% | | 86% | | 82% | |
| Spec 2 | 39% | | 59% | | 41% | |
| Cutoff 3 | 0.141 | | 0.141 | | 0.783 | |
| Sens 3 | 92% | | 100% | | 91% | |
| Spec 3 | 21% | | 19% | | 39% | |
| Cutoff 4 | 2.99 | | 3.27 | | 2.93 | |
| Sens 4 | 50% | | 71% | | 50% | |
| Spec 4 | 70% | | 71% | | 71% | |
| Cutoff 5 | 4.00 | | 4.91 | | 4.23 | |
| Sens 5 | 50% | | 71% | | 50% | |
| Spec 5 | 80% | | 81% | | 80% | |
| Cutoff 6 | 5.64 | | 13.6 | | 6.65 | |
| Sens 6 | 42% | | 29% | | 41% | |
| Spec 6 | 90% | | 91% | | 90% | |
| OR Quart 2 | 1.8 | | 0 | | 2.2 | |
| p Value | 0.45 | | na | | 0.39 | |
| 95% CI of | 0.39 | | na | | 0.36 | |
| OR Quart2 | 8.4 | | na | | 13 | |
| OR Quart 3 | 1.8 | | 1.0 | | 2.9 | |
| p Value | 0.45 | | 1.0 | | 0.24 | |
| 95% CI of | 0.39 | | 0.060 | | 0.50 | |

(continued)

| | At Enrollment | | | | | |
|---|---|---|---|---|---|---|
| | sCr or UO | | sCr only | | UO only | |
| OR Quart3 | 8.4 | | 17 | | 16 | |
| OR Quart 4 | 7.0 | | 5.8 | | 8.8 | |
| p Value | 0.0063 | | 0.12 | | 0.0093 | |
| 95% CI of | 1.7 | | 0.64 | | 1.7 | |
| OR Quart4 | 29 | | 53 | | 45 | |

| **CA 15-3** | | | | | | |
|---|---|---|---|---|---|---|
| | sCr or UO | | sCr only | | UO only | |
| | Cohort 1 | Cohort 2 | Cohort 1 | Cohort 2 | Cohort 1 | Cohort 2 |
| Median | 0.309 | 0.529 | nd | nd | 0.324 | 0.506 |
| Average | 1.36 | 0.742 | nd | nd | 1.48 | 0.757 |
| Stdev | 5.87 | 0.707 | nd | nd | 6.18 | 0.748 |
| p(t-test) | | 0.58 | nd | nd | | 0.56 |
| Min | 0.0328 | 0.109 | nd | nd | 0.0328 | 0.109 |
| Max | 60.0 | 3.12 | nd | nd | 60.0 | 3.12 |
| n (Samp) | 109 | 28 | nd | nd | 98 | 25 |
| n (Patient) | 109 | 28 | nd | nd | 98 | 25 |

| | At Enrollment | | |
|---|---|---|---|
| | sCr or UO | sCr only | UO only |
| AUC | 0.65 | nd | 0.62 |
| SE | 0.061 | nd | 0.066 |
| p | 0.017 | nd | 0.071 |
| nCohort 1 | 109 | nd | 98 |
| nCohort 2 | 28 | nd | 25 |
| Cutoff 1 | 0.392 | nd | 0.385 |
| Sens 1 | 71% | nd | 72% |
| Spec 1 | 59% | nd | 55% |
| Cutoff 2 | 0.208 | nd | 0.208 |
| Sens 2 | 86% | nd | 84% |
| Spec 2 | 35% | nd | 34% |
| Cutoff 3 | 0.181 | nd | 0.181 |
| Sens 3 | 93% | nd | 92% |
| Spec 3 | 32% | nd | 31% |
| Cutoff 4 | 0.510 | nd | 0.535 |
| Sens 4 | 54% | nd | 44% |
| Spec 4 | 71% | nd | 70% |
| Cutoff 5 | 0.762 | nd | 0.864 |
| Sens 5 | 29% | nd | 24% |

(continued)

| | At Enrollment | | |
| --- | --- | --- | --- |
| | sCr or UO | sCr only | UO only |
| Spec 5 | 81% | nd | 81% |
| Cutoff 6 | 1.91 | nd | 2.03 |
| Sens 6 | 7% | nd | 8% |
| Spec 6 | 91% | nd | 91% |
| OR Quart 2 | 2.8 | nd | 2.7 |
| p Value | 0.25 | nd | 0.26 |
| 95% CI of | 0.50 | nd | 0.48 |
| OR Quart2 | 15 | nd | 15 |
| OR Quart 3 | 8.7 | nd | 6.7 |
| p Value | 0.0077 | nd | 0.022 |
| 95% CI of | 1.8 | nd | 1.3 |
| OR Quart3 | 43 | nd | 34 |
| OR Quart 4 | 5.5 | nd | 4.9 |
| p Value | 0.038 | nd | 0.059 |
| 95% CI of | 1.1 | nd | 0.94 |
| OR Quart4 | 28 | nd | 25 |

While the invention has been described and exemplified in sufficient detail for those skilled in this art to make and use it, various alternatives, modifications, and improvements should be apparent without departing from the spirit and scope of the invention. The examples provided herein are representative of preferred embodiments, are exemplary, and are not intended as limitations on the scope of the invention. Modifications therein and other uses will occur to those skilled in the art. These modifications are encompassed within the spirit of the invention and are defined by the scope of the claims.

It will be readily apparent to a person skilled in the art that varying substitutions and modifications may be made to the invention disclosed herein without departing from the scope and spirit of the invention.

All patents and publications mentioned in the specification are indicative of the levels of those of ordinary skill in the art to which the invention pertains. All patents and publications are herein incorporated by reference to the same extent as if each individual publication was specifically and individually indicated to be incorporated by reference.

The invention illustratively described herein suitably may be practiced in the absence of any element or elements, limitation or limitations which is not specifically disclosed herein. Thus, for example, in each instance herein any of the terms "comprising", "consisting essentially of" and "consisting of" may be replaced with either of the other two terms. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

Other embodiments are set forth within the following claims.

The invention is further characterized by the following aspects:

1. A method for evaluating renal status in a subject, comprising:

performing one or more assays configured to detect one or more biomarkers selected from the group consisting of Cancer antigen CA 15-3, C-C Motif chemokine 18, C-C Motif chemokine 24, Cathepsin D, C-X-C Motif chemokine 13, C-C motif chemokine 8, Interleukin-2 receptor alpha chain, Insulin-like growth factor-binding protein 3, Interleukin-11, Matrix Metalloproteinase-8, Transforming growth factor alpha, IgG1, and IgG2 on a body fluid sample obtained from the subject to provide an assay result; and correlating the assay result(s) to the renal status of the subject.

2. A method according to aspect 1, wherein said correlation step comprises correlating the assay result(s) to one or more of risk stratification, diagnosis, staging, prognosis, classifying and monitoring of the renal status of the subject.

3. A method according to aspect 1, wherein said correlating step comprises assigning a likelihood of one or more future changes in renal status to the subject based on the assay result(s).

4. A method according to aspect 3, wherein said one or more future changes in renal status comprise one or more of a future injury to renal function, future reduced renal function, future improvement in renal function, and future acute renal failure (ARF).

5. A method according to one of aspects 1-4, wherein said assay results comprise at least 2, 3, 4, or 5 of:

a measured concentration of Cancer antigen CA 15-3,

a measured concentration of C-C Motif chemokine 18,

a measured concentration of C-C Motif chemokine 24,

a measured concentration of Cathepsin D,

a measured concentration of C-X-C Motif chemokine 13,

a measured concentration of C-C motif chemokine 8,

a measured concentration of Interleukin-2 receptor alpha chain,

a measured concentration of Insulin-like growth factor-binding protein 3,

a measured concentration of Interleukin-11,

a measured concentration of Matrix Metalloproteinase-8,

a measured concentration of Transforming growth factor alpha,

a measured concentration of IgG1, and

a measured concentration of IgG2.

6. A method according to one of aspects 1-5, wherein a plurality of assay results are combined using a function that converts the plurality of assay results into a single composite result.

7. A method according to aspect 3, wherein said one or more future changes in renal status comprise a clinical outcome related to a renal injury suffered by the subject.

8. A method according to aspect 3, wherein the likelihood of one or more future changes in renal status is that an event of interest is more or less likely to occur within 30 days of the time at which the body fluid sample is obtained from the subject.

9. A method according to aspect 8, wherein the likelihood of one or more future changes in renal status is that an event of interest is more or less likely to occur within a period selected from the group consisting of 21 days, 14 days, 7 days, 5 days, 96 hours, 72 hours, 48 hours, 36 hours, 24 hours, and 12 hours.

10. A method according to one of aspects 1-5, wherein the subject is selected for evaluation of renal status based on the pre-existence in the subject of one or more known risk factors for prerenal, intrinsic renal, or postrenal ARF.

11. A method according to one of aspects 1-5, wherein the subject is selected for evaluation of renal status based on an existing diagnosis of one or more of congestive heart failure, preeclampsia, eclampsia, diabetes mellitus, hypertension, coronary artery disease, proteinuria, renal insufficiency, glomerular filtration below the normal range, cirrhosis, serum creatinine above the normal range, sepsis, injury to renal function, reduced renal function, or ARF, or based on undergoing or having undergone major vascular surgery, coronary artery bypass, or other cardiac surgery, or based on exposure to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin.

12. A method according to one of aspects 1-5, wherein said correlating step comprises assigning a diagnosis of the occurrence or nonoccurrence of one or more of an injury to renal function, reduced renal function, or ARF to the subject based on the assay result(s).

13. A method according to one of aspects 1-5, wherein said correlating step comprises assessing whether or not renal function is improving or worsening in a subject who has suffered from an injury to renal function, reduced renal function, or ARF based on the assay result(s).

14. A method according to one of aspects 1-5, wherein said method is a method of diagnosing the occurrence or nonoccurrence of an injury to renal function in said subject.

15. A method according to one of aspects 1-5, wherein said method is a method of diagnosing the occurrence or nonoccurrence of reduced renal function in said subject.

16. A method according to one of aspects 1-5, wherein said method is a method of diagnosing the occurrence or nonoccurrence of acute renal failure in said subject.

17. A method according to one of aspects 1-5, wherein said method is a method of diagnosing the occurrence or nonoccurrence of a need for renal replacement therapy in said subject.

18. A method according to one of aspects 1-5, wherein said method is a method of diagnosing the occurrence or nonoccurrence of a need for renal transplantation in said subject.

19. A method according to one of aspects 1-5, wherein said method is a method of assigning a risk of the future occurrence or nonoccurrence of an injury to renal function in said subject.

20. A method according to one of aspects 1-5, wherein said method is a method of assigning a risk of the future occurrence or nonoccurrence of reduced renal function in said subject.

21. A method according to one of aspects 1-5, wherein said method is a method of assigning a risk of the future occurrence or nonoccurrence of acute renal failure in said subject.

22. A method according to one of aspects 1-5, wherein said method is a method of assigning a risk of the future occurrence or nonoccurrence of a need for renal replacement therapy in said subject.

23. A method according to one of aspects 1-5, wherein said method is a method of assigning a risk of the future occurrence or nonoccurrence of a need for renal transplantation in said subject.

24. A method according to one of aspects 1-5, wherein said one or more future changes in renal status comprise one or more of a future injury to renal function, future reduced renal function, future improvement in renal function, and future acute renal failure (ARF) within 72 hours of the time at which the body fluid sample is obtained.

25. A method according to one of aspects 1-5, wherein said one or more future changes in renal status comprise one or more of a future injury to renal function, future reduced renal function, future improvement in renal function, and future acute renal failure (ARF) within 48 hours of the time at which the body fluid sample is obtained.

26. A method according to one of aspects 1-5, wherein said one or more future changes in renal status comprise one or more of a future injury to renal function, future reduced renal function, future improvement in renal function, and future acute renal failure (ARF) within 24 hours of the time at which the body fluid sample is obtained.

27. A method according to one of aspects 1-5, wherein the subject is in RIFLE stage 0 or R.

28. A method according to aspect 27, wherein the subject is in RIFLE stage 0, and said correlating step comprises assigning a likelihood that the subject will reach RIFLE stage R, I or F within 72 hours.

29. A method according to aspect 28, wherein the subject is in RIFLE stage 0, and said correlating step comprises assigning a likelihood that the subject will reach RIFLE stage I or F within 72 hours.

30. A method according to aspect 28, wherein the subject is in RIFLE stage 0, and said correlating step comprises assigning a likelihood that the subject will reach RIFLE stage F within 72 hours.

31. A method according to aspect 27, wherein the subject is in RIFLE stage 0 or R, and said correlating step comprises assigning a likelihood that the subject will reach RIFLE stage I or F within 72 hours.

32. A method according to aspect 31, wherein the subject is in RIFLE stage 0 or R, and said correlating step comprises assigning a likelihood that the subject will reach RIFLE stage F within 72 hours.

33. A method according to aspect 27, wherein the subject is in RIFLE stage R, and said correlating step comprises assigning a likelihood that the subject will reach RIFLE stage I or F within 72 hours.

34. A method according to aspect 33, wherein the subject is in RIFLE stage R, and said correlating step comprises assigning a likelihood that the subject will reach RIFLE stage F within 72 hours.

35. A method according to one of aspects 1-5, wherein the subject is in RIFLE stage 0, R, or I, and said correlating step comprises assigning a likelihood that the subject will reach RIFLE stage F within 72 hours.

36. A method according to aspect 35, wherein the subject is in RIFLE stage I, and said correlating step comprises assigning a likelihood that the subject will reach RIFLE stage F within 72 hours.

37. A method according to aspect 28, wherein said correlating step comprises assigning a likelihood that the subject will reach RIFLE stage R, I or F within 48 hours.

38. A method according to aspect 29, wherein said correlating step comprises assigning a likelihood that the subject will reach RIFLE stage I or F within 48 hours.

39. A method according to aspect 30, wherein said correlating step comprises assigning a likelihood that the subject will reach RIFLE stage F within 48 hours.

40. A method according to aspect 31, wherein said correlating step comprises assigning a likelihood that the subject will reach RIFLE stage I or F within 48 hours.

41. A method according to aspect 32, wherein said correlating step comprises assigning a likelihood that the subject will reach RIFLE stage F within 48 hours.

42. A method according to aspect 33, wherein said correlating step comprises assigning a likelihood that the subject will reach RIFLE stage I or F within 48 hours.

43. A method according to aspect 34, wherein said correlating step comprises assigning a likelihood that the subject will reach RIFLE stage F within 48 hours.

44. A method according to aspect 35, wherein said correlating step comprises assigning a likelihood that the subject will reach RIFLE stage F within 48 hours.

45. A method according to aspect 36, wherein said correlating step comprises assigning a likelihood that the subject will reach RIFLE stage F within 48 hours.

46. A method according to aspect 28, wherein said correlating step comprises assigning a likelihood that the subject will reach RIFLE stage R, I or F within 24 hours.

47. A method according to aspect 29, wherein said correlating step comprises assigning a likelihood that the subject will reach RIFLE stage I or F within 24 hours.

48. A method according to aspect 30, wherein said correlating step comprises assigning a likelihood that the subject will reach RIFLE stage F within 24 hours.

49. A method according to aspect 31, wherein said correlating step comprises assigning a likelihood that the subject will reach RIFLE stage I or F within 24 hours.

50. A method according to aspect 32, wherein said correlating step comprises assigning a likelihood that the subject will reach RIFLE stage F within 24 hours.

51. A method according to aspect 33, wherein said correlating step comprises assigning a likelihood that the subject will reach RIFLE stage I or F within 24 hours.

52. A method according to aspect 34, wherein said correlating step comprises assigning a likelihood that the subject will reach RIFLE stage F within 24 hours.

53. A method according to aspect 35, wherein said correlating step comprises assigning a likelihood that the subject will reach RIFLE stage F within 24 hours.

54. A method according to aspect 36, wherein said correlating step comprises assigning a likelihood that the subject will reach RIFLE stage F within 24 hours.

55. A method according to one of aspects 1-5, wherein the subject is not in acute renal failure.

56. A method according to one of aspects 1-5, wherein the subject has not experienced a 1.5-fold or greater increase in serum creatinine over a baseline value determined prior to the time at which the body fluid sample is obtained.

57. A method according to one of aspects 1-5, wherein the subject has a urine output of at least 0.5 ml/kg/hr over the 6 hours preceding the time at which the body fluid sample is obtained.

58. A method according to one of aspects 1-5, wherein the subject has not experienced an increase of 0.3 mg/dL or greater in serum creatinine over a baseline value determined prior to the time at which the body fluid sample is obtained.

59. A method according to one of aspects 1-5, wherein the subject (i) has not experienced a 1.5-fold or greater increase in serum creatinine over a baseline value determined prior to the time at which the body fluid sample is obtained, (ii) has a urine output of at least 0.5 ml/kg/hr over the 6 hours preceding the time at which the body fluid sample is obtained, and (iii) has not experienced an increase of 0.3 mg/dL or greater in serum creatinine over a baseline value determined prior to the time at which the body fluid sample is obtained.

60. A method according to one of aspects 1-5, wherein the subject has not experienced a 1.5-fold or greater increase in serum creatinine over a baseline value determined prior to the time at which the body fluid sample is obtained.

61. A method according to one of aspects 1-5, wherein the subject has a urine output of at least 0.5 ml/kg/hr over the 6 hours preceding the time at which the body fluid sample is obtained.

62. A method according to one of aspects 1-5, wherein the subject (i) has not experienced a 1.5-fold or greater increase in serum creatinine over a baseline value determined prior to the time at which the body fluid sample is obtained, (ii) has a urine output of at least 0.5 ml/kg/hr over the 12 hours preceding the time at which the body fluid sample is obtained, and (iii) has not experienced an increase of 0.3 mg/dL or greater in serum creatinine over a baseline value determined prior to the time at which the body fluid sample is obtained.

63. A method according to one of aspects 1-5, wherein said correlating step comprises assigning one or more of: a likelihood that within 72 hours the subject will (i) experience a 1.5-fold or greater increase in serum creatinine (ii) have a urine output of less than 0.5 ml/kg/hr over a 6 hour period, or (iii) experience an increase of 0.3 mg/dL or greater in serum creatinine.

64. A method according to aspect 63, wherein said correlating step comprises assigning one or more of: a likelihood that within 48 hours the subject will (i) experience a 1.5-fold or greater increase in serum creatinine (ii) have a urine output of less than 0.5 ml/kg/hr over a 6 hour period, or (iii) experience an increase of 0.3 mg/dL or greater in serum creatinine.

65. A method according to aspect 63, wherein said correlating step comprises assigning one or more of: a likelihood that within 24 hours the subject will (i) experience a 1.5-fold or greater increase in serum creatinine (ii) have a urine output of less than 0.5 ml/kg/hr over a 6 hour period, or (iii) experience an increase of 0.3 mg/dL or greater in serum creatinine.

66. A method according to aspect 63, wherein said correlating step comprises assigning a likelihood that within 72 hours the subject will experience a 1.5-fold or greater increase in serum creatinine.

67. A method according to aspect 63, wherein said correlating step comprises assigning a likelihood that within 72 hours the subject will have a urine output of less than 0.5 ml/kg/hr over a 6 hour period.

68. A method according to aspect 63, wherein said correlating step comprises assigning a likelihood that within 72 hours the subject will experience an increase of 0.3 mg/dL or greater in serum creatinine.

69. A method according to aspect 63, wherein said correlating step comprises assigning a likelihood that within 48 hours the subject will experience a 1.5-fold or greater increase in serum creatinine.

70. A method according to aspect 63, wherein said correlating step comprises assigning a likelihood that within 48 hours the subject will have a urine output of less than 0.5 ml/kg/hr over a 6 hour period.

71. A method according to aspect 63, wherein said correlating step comprises assigning a likelihood that within 48 hours the subject will experience an increase of 0.3 mg/dL or greater in serum creatinine.

72. A method according to aspect 63, wherein said correlating step comprises assigning a likelihood that within 24 hours the subject will experience a 1.5-fold or greater increase in serum creatinine.

73. A method according to aspect 63, wherein said correlating step comprises assigning a likelihood that within 24 hours the subject will have a urine output of less than 0.5 ml/kg/hr over a 6 hour period.

74. A method according to aspect 63, wherein said correlating step comprises assigning a likelihood that within 24 hours the subject will experience an increase of 0.3 mg/dL or greater in serum creatinine.

75. A method according to one of aspects 1-5, wherein the subject has not experienced a 2-fold or greater increase in serum creatinine over a baseline value determined prior to the time at which the body fluid sample is obtained.

76. A method according to one of aspects 1-5, wherein the subject has a urine output of at least 0.5 ml/kg/hr over the 12 hours preceding the time at which the body fluid sample is obtained.

77. A method according to one of aspects 1-5, wherein the subject (i) has not experienced a 2-fold or greater increase in serum creatinine over a baseline value determined prior to the time at which the body fluid sample is obtained, (ii) has a urine output of at least 0.5 ml/kg/hr over the 2 hours preceding the time at which the body fluid sample is obtained, and (iii) has not experienced an increase of 0.3 mg/dL or greater in serum creatinine over a baseline value determined prior to the time at which the body fluid sample is obtained.

78. A method according to one of aspects 1-5, wherein the subject has not experienced a 3-fold or greater increase in serum creatinine over a baseline value determined prior to the time at which the body fluid sample is obtained.

79. A method according to one of aspects 1-5, wherein the subject has a urine output of at least 0.3 ml/kg/hr over the 24 hours preceding the time at which the body fluid sample is obtained, or anuria over the 12 hours preceding the time at which the body fluid sample is obtained.

80. A method according to one of aspects 1-5, wherein the subject (i) has not experienced a 3-fold or greater increase in serum creatinine over a baseline value determined prior to the time at which the body fluid sample is obtained, (ii) has a urine output of at least 0.3 ml/kg/hr over the 24 hours preceding the time at which the body fluid sample is obtained, or anuria over the 12 hours preceding the time at which the body fluid sample is obtained, and (iii) has not experienced an increase of 0.3 mg/dL or greater in serum creatinine over a baseline value determined prior to the time at which the body fluid sample is obtained.

81. A method according to one of aspects 1-5, wherein said correlating step comprises assigning one or more of: a likelihood that within 72 hours the subject will (i) experience a 2-fold or greater increase in serum creatinine (ii) have a urine output of less than 0.5 ml/kg/hr over a 12 hour period, or (iii) experience an increase of 0.3 mg/dL or greater in serum creatinine.

82. A method according to aspect 81, wherein said correlating step comprises assigning one or more of: a likelihood that within 48 hours the subject will (i) experience a 2-fold or greater increase in serum creatinine (ii) have a urine output of less than 0.5 ml/kg/hr over a 6 hour period, or (iii) experience an increase of 0.3 mg/dL or greater in serum creatinine.

83. A method according to aspect 81, wherein said correlating step comprises assigning one or more of: a likelihood that within 24 hours the subject will (i) experience a 2-fold or greater increase in serum creatinine, or (ii) have a urine output of less than 0.5 ml/kg/hr over a 6 hour period.

84. A method according to aspect 81, wherein said correlating step comprises assigning a likelihood that within 72 hours the subject will experience a 2-fold or greater increase in serum creatinine.

85. A method according to aspect 81, wherein said correlating step comprises assigning a likelihood that within 72 hours the subject will have a urine output of less than 0.5 ml/kg/hr over a 6 hour period.

86. A method according to aspect 81, wherein said correlating step comprises assigning a likelihood that within 48 hours the subject will experience a 2-fold or greater increase in serum creatinine.

87. A method according to aspect 81, wherein said correlating step comprises assigning a likelihood that within 48 hours the subject will have a urine output of less than 0.5 ml/kg/hr over a 6 hour period.

88. A method according to aspect 81, wherein said correlating step comprises assigning a likelihood that within 24 hours the subject will experience a 2-fold or greater increase in serum creatinine.

89. A method according to aspect 81, wherein said correlating step comprises assigning a likelihood that within 24 hours the subject will have a urine output of less than 0.5 ml/kg/hr over a 6 hour period.

90. A method according to one of aspects 1-5, wherein said correlating step comprises assigning one or more of: a likelihood that within 72 hours the subject will (i) experience a 3-fold or greater increase in serum creatinine, or (ii) have a urine output of less than 0.3 ml/kg/hr over a 24 hour period or anuria over a 12 hour period.

91. A method according to aspect 90, wherein said correlating step comprises assigning one or more of: a likelihood that within 48 hours the subject will (i) experience a 3-fold or greater increase in serum creatinine, or (ii) have a urine output of less than 0.3 ml/kg/hr over a 24 hour period or anuria over a 12 hour period.

92. A method according to aspect 90, wherein said correlating step comprises assigning one or more of: a likelihood that within 24 hours the subject will (i) experience a 3-fold or greater increase in serum creatinine, or (ii) have a urine output of less than 0.3 ml/kg/hr over a 24 hour period or anuria over a 12 hour period.

93. A method according to aspect 90, wherein said correlating step comprises assigning a likelihood that within 72 hours the subject will experience a 3-fold or greater increase in serum creatinine.

94. A method according to aspect 90, wherein said correlating step comprises assigning a likelihood that within 72 hours the subject will have a urine output of less than 0.3 ml/kg/hr over a 24 hour period or anuria over a 12 hour period.

95. A method according to aspect 90, wherein said correlating step comprises assigning a likelihood that within 48 hours the subject will experience a 3-fold or greater increase in serum creatinine.

96. A method according to aspect 90, wherein said correlating step comprises assigning a likelihood that within 48 hours the subject will have a urine output of less than 0.3 ml/kg/hr over a 24 hour period or anuria over a 12 hour period.

97. A method according to aspect 90, wherein said correlating step comprises assigning a likelihood that within 24 hours the subject will experience a 3-fold or greater increase in serum creatinine.

98. A method according to aspect 90, wherein said correlating step comprises assigning a likelihood that within 24 hours the subject will have a urine output of less than 0.3 ml/kg/hr over a 24 hour period or anuria over a 12 hour period.

99. A method according to one of aspects 1-98, wherein the body fluid sample is a urine sample.

100. A method according to one of aspects 1-99, wherein said method comprises performing assays that detect one, two or three, or more of Cancer antigen CA 15-3, C-C Motif chemokine 18, C-C Motif chemokine 24, Cathepsin D, C-X-C Motif chemokine 13, C-C motif chemokine 8, Interleukin-2 receptor alpha chain, Insulin-like growth factor-binding protein 3, Interleukin-11, Matrix Metalloproteinase-8, Transforming growth factor alpha, IgG1, and IgG2.

101. Measurement of one or more biomarkers selected from the group consisting of Cancer antigen CA 15-3, C-C Motif chemokine 18, C-C Motif chemokine 24, Cathepsin D, C-X-C Motif chemokine 13, C-C motif chemokine 8, Interleukin-2 receptor alpha chain, Insulin-like growth factor-binding protein 3, Interleukin-11, Matrix Metalloproteinase-8, Transforming growth factor alpha, IgG1, and IgG2 for the evaluation of renal injury.

102. Measurement of one or more biomarkers selected from the group consisting of Cancer antigen CA 15-3, C-C Motif chemokine 18, C-C Motif chemokine 24, Cathepsin D, C-X-C Motif chemokine 13, C-C motif chemokine 8, Interleukin-2 receptor alpha chain, Insulin-like growth factor-binding protein 3, Interleukin-11, Matrix Metalloproteinase-8, Transforming growth factor alpha, IgG1, and IgG2 for the evaluation of acute renal injury.

103. A kit, comprising:
reagents for performing one or more assays configured to detect one or more kidney injury markers selected from the group consisting of Cancer antigen CA 15-3, C-C Motif chemokine 18, C-C Motif chemokine 24, Cathepsin D, C-X-C Motif chemokine 13, C-C motif chemokine 8, Interleukin-2 receptor alpha chain, Insulin-like growth factor-binding protein 3, Interleukin-11, Matrix Metalloproteinase-8, Transforming growth factor alpha, IgG1, and IgG2.

104. A kit according to aspect 103, wherein said reagents comprise one or more binding reagents, each of which specifically binds one of said of kidney injury markers.

105. A kit according to aspect 104, wherein a plurality of binding reagents are contained in a single assay device.

106. A kit according to aspect 103, wherein at least one of said assays is configured as a sandwich binding assay.

107. A kit according to aspect 103, wherein at least one of said assays is configured as a competitive binding assay.

108. A kit according to one of aspects 103-107, wherein said one or more assays comprise assays that detect one, two or three, or more of Cancer antigen CA 15-3, C-C Motif chemokine 18, C-C Motif chemokine 24, Cathepsin D, C-X-C Motif chemokine 13, C-C motif chemokine 8, Interleukin-2 receptor alpha chain, Insulin-like growth factor-binding protein 3, Interleukin-11, Matrix Metalloproteinase-8, Transforming growth factor alpha, IgG1, and IgG2.

**Claims**

1. A method for evaluating renal status in a subject, comprising:

    performing one or more assays configured to detect Insulin-like growth factor-binding protein 3, and optionally one or more biomarkers selected from the group consisting of Cancer antigen CA 15-3, C-C Motif chemokine 24, C-C Motif chemokine 18, Cathepsin D, C-X-C Motif chemokine 13, C-C motif chemokine 8, Interleukin-2 receptor alpha chain, , Interleukin-11, Matrix Metalloproteinase-8, Transforming growth factor alpha, IgG1, and IgG2 on a body fluid sample obtained from the subject to provide an assay result; and correlating the assay result(s) to the renal status of the subject.

2. A method according to claim 1, wherein said correlation step comprises correlating the assay result(s) to one or more of risk stratification, diagnosis, staging, prognosis, classifying and monitoring of the renal status of the subject.

3. A method according to claim 1, wherein said correlating step comprises assigning a likelihood of one or more future changes in renal status to the subject based on the assay result(s).

4. A method according to claim 3, wherein said one or more future changes in renal status comprise one or more of a future injury to renal function, future reduced renal function, future improvement in renal function, and future acute renal failure (ARF).

5. A method according to one of claims 1-4, wherein said assay results comprise at least 2, 3, 4, or 5 of:

> a measured concentration of Insulin-like growth factor-binding protein 3,
> a measured concentration of C-C Motif chemokine 24,
> a measured concentration of Cancer antigen CA 15-3,
> a measured concentration of C-C Motif chemokine 18,
> a measured concentration of Cathepsin D,
> a measured concentration of C-X-C Motif chemokine 13,
> a measured concentration of C-C motif chemokine 8,
> a measured concentration of Interleukin-2 receptor alpha chain,
> a measured concentration of Interleukin-11,
> a measured concentration of Matrix Metalloproteinase-8,
> a measured concentration of Transforming growth factor alpha,
> a measured concentration of IgG1, and
> a measured concentration of IgG2.

6. A method according to claim 3, wherein said one or more future changes in renal status comprise a clinical outcome related to a renal injury suffered by the subject, or
wherein the likelihood of one or more future changes in renal status is that an event of interest is more or less likely to occur within 30 days of the time at which the body fluid sample is obtained from the subject.
wherein the likelihood of one or more future changes in renal status preferably is that an event of interest is more or less likely to occur within a period selected from the group consisting of 21 days, 14 days, 7 days, 5 days, 96 hours, 72 hours, 48 hours, 36 hours, 24 hours, and 12 hours.

7. A method according to one of claims 1-5, wherein a plurality of assay results are combined using a function that converts the plurality of assay results into a single composite result, or
wherein the subject is selected for evaluation of renal status based on the pre-existence in the subject of one or more known risk factors for prerenal, intrinsic renal, or postrenal ARF, or
wherein the subject is selected for evaluation of renal status based on an existing diagnosis of one or more of congestive heart failure, preeclampsia, eclampsia, diabetes mellitus, hypertension, coronary artery disease, proteinuria, renal insufficiency, glomerular filtration below the normal range, cirrhosis, serum creatinine above the normal range, sepsis, injury to renal function, reduced renal function, or ARF, or based on undergoing or having undergone major vascular surgery, coronary artery bypass, or other cardiac surgery, or based on exposure to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin, or
wherein said correlating step comprises assigning a diagnosis of the occurrence or nonoccurrence of one or more of an injury to renal function, reduced renal function, or ARF to the subject based on the assay result(s), or
wherein said correlating step comprises assessing whether or not renal function is improving or worsening in a subject who has suffered from an injury to renal function, reduced renal function, or ARF based on the assay result(s), or
wherein said method is a method of diagnosing the occurrence or nonoccurrence of an injury to renal function in said subject, or
wherein said method is a method of diagnosing the occurrence or nonoccurrence of reduced renal function in said subject, or
wherein said method is a method of diagnosing the occurrence or nonoccurrence of acute renal failure in said subject, or
wherein said method is a method of diagnosing the occurrence or nonoccurrence of a need for renal replacement therapy in said subject, or
wherein said method is a method of diagnosing the occurrence or nonoccurrence of a need for renal transplantation in said subject, or
wherein said method is a method of assigning a risk of the future occurrence or nonoccurrence of an injury to renal function in said subject, or
wherein said method is a method of assigning a risk of the future occurrence or nonoccurrence of reduced renal function in said subject, or
wherein said method is a method of assigning a risk of the future occurrence or nonoccurrence of acute renal failure in said subject, or
wherein said method is a method of assigning a risk of the future occurrence or nonoccurrence of a need for renal replacement therapy in said subject, or

wherein said method is a method of assigning a risk of the future occurrence or nonoccurrence of a need for renal transplantation in said subject, or

wherein said one or more future changes in renal status comprise one or more of a future injury to renal function, future reduced renal function, future improvement in renal function, and future acute renal failure (ARF) within 72 hours, within 48 hours or within 24 hours of the time at which the body fluid sample is obtained.

**8.** A method according to one of claims 1-5, wherein the subject is in RIFLE stage 0 or R.

**9.** A method according to claim 8, wherein the subject is in RIFLE stage 0, and said correlating step comprises assigning a likelihood that the subject will reach RIFLE stage R, I or F within 72 hours, within 48 hours or within 24 hours.

**10.** A method according to claim 9, wherein the subject is in RIFLE stage 0, and said correlating step comprises assigning a likelihood that the subject will reach RIFLE stage I or F within 72 hours, within 48 hours or within 24 hours,

wherein the subject is in RIFLE stage 0, and said correlating step comprises assigning a likelihood that the subject will reach RIFLE stage F within 72 hours, within 48 hours or within 24 hours,

wherein the subject is in RIFLE stage 0 or R, and said correlating step comprises assigning a likelihood that the subject will reach RIFLE stage I or F within 72 hours, within 48 hours or within 24 hours,

wherein the subject is in RIFLE stage 0 or R, and said correlating step comprises assigning a likelihood that the subject will reach RIFLE stage F within 72 hours, within 48 hours or within 24 hours,

wherein the subject is in RIFLE stage R, and said correlating step comprises assigning a likelihood that the subject will reach RIFLE stage I or F within 72 hours, within 48 hours or within 24 hours,

wherein the subject is in RIFLE stage R, and said correlating step comprises assigning a likelihood that the subject will reach RIFLE stage F within 72 hours, within 48 hours or within 24 hours,

wherein the subject is in RIFLE stage 0, R, or I, and said correlating step comprises assigning a likelihood that the subject will reach RIFLE stage F within 72 hours, within 48 hours or within 24 hours,

wherein the subject preferably is in RIFLE stage I, and said correlating step comprises assigning a likelihood that the subject will reach RIFLE stage F within 72 hours, within 48 hours or within 24 hours.

**11.** A method according to one of claims 1-5, wherein the subject is not in acute renal failure, or

wherein the subject has not experienced a 1.5-fold or greater increase in serum creatinine over a baseline value determined prior to the time at which the body fluid sample is obtained, or wherein the subject has a urine output of at least 0.5 ml/kg/hr over the 6 hours preceding the time at which the body fluid sample is obtained, or

wherein the subject has not experienced an increase of 0.3 mg/dL or greater in serum creatinine over a baseline value determined prior to the time at which the body fluid sample is obtained, or

wherein the subject (i) has not experienced a 1.5-fold or greater increase in serum creatinine over a baseline value determined prior to the time at which the body fluid sample is obtained, (ii) has a urine output of at least 0.5 ml/kg/hr over the 6 hours preceding the time at which the body fluid sample is obtained, and (iii) has not experienced an increase of 0.3 mg/dL or greater in serum creatinine over a baseline value determined prior to the time at which the body fluid sample is obtained, or

wherein the subject has not experienced a 1.5-fold or greater increase in serum creatinine over a baseline value determined prior to the time at which the body fluid sample is obtained, or

wherein the subject has a urine output of at least 0.5 ml/kg/hr over the 6 hours preceding the time at which the body fluid sample is obtained, or

wherein the subject (i) has not experienced a 1.5-fold or greater increase in serum creatinine over a baseline value determined prior to the time at which the body fluid sample is obtained, (ii) has a urine output of at least 0.5 ml kg/hr over the 12 hours preceding the time at which the body fluid sample is obtained, and (iii) has not experienced an increase of 0.3 mg/dL or greater in serum creatinine over a baseline value determined prior to the time at which the body fluid sample is obtained, or

wherein said correlating step comprises assigning one or more of: a likelihood that within 72 hours the subject will (i) experience a 1.5-fold or greater increase in serum creatinine (ii) have a urine output of less than 0.5 ml kg/hr over a 6 hour period, or (iii) experience an increase of 0.3 mg/dL or greater in serum creatinine,

wherein said correlating step preferably comprises assigning one or more of: a likelihood that within 48 hours the subject will (i) experience a 1.5-fold or greater increase in serum creatinine (ii) have a urine output of less than 0.5 ml/kg/hr over a 6 hour period, or (iii) experience an increase of 0.3 mg/dL or greater in serum creatinine, or

wherein said correlating step preferably comprises assigning one or more of: a likelihood that within 24 hours the subject will (i) experience a 1.5-fold or greater increase in serum creatinine (ii) have a urine output of less than 0.5 ml/kg/hr over a 6 hour period, or (iii) experience an increase of 0.3 mg/dL or greater in serum creatinine, or

wherein said correlating step preferably comprises assigning a likelihood that within 72 hours the subject will experience a 1.5-fold or greater increase in serum creatinine, or

wherein said correlating step preferably comprises assigning a likelihood that within 72 hours the subject will have a urine output of less than 0.5 ml/kg/hr over a 6 hour period, or

wherein said correlating step preferably comprises assigning a likelihood that within 72 hours the subject will experience an increase of 0.3 mg/dL or greater in serum creatinine, or

wherein said correlating step preferably comprises assigning a likelihood that within 48 hours the subject will experience a 1.5-fold or greater increase in serum creatinine, or

wherein said correlating step preferably comprises assigning a likelihood that within 48 hours the subject will have a urine output of less than 0.5 ml/kg/hr over a 6 hour period, or

wherein said correlating step preferably comprises assigning a likelihood that within 48 hours the subject will experience an increase of 0.3 mg/dL or greater in serum creatinine, or

wherein said correlating step preferably comprises assigning a likelihood that within 24 hours the subject will experience a 1.5-fold or greater increase in serum creatinine, or

wherein said correlating step preferably comprises assigning a likelihood that within 24 hours the subject will have a urine output of less than 0.5 ml/kg/hr over a 6 hour period, or

wherein said correlating step preferably comprises assigning a likelihood that within 24 hours the subject will experience an increase of 0.3 mg/dL or greater in serum creatinine, or

wherein the subject has not experienced a 2-fold or greater increase in serum creatinine over a baseline value determined prior to the time at which the body fluid sample is obtained, or

wherein the subject has a urine output of at least 0.5 ml/kg/hr over the 12 hours preceding the time at which the body fluid sample is obtained, or

wherein the subject (i) has not experienced a 2-fold or greater increase in serum creatinine over a baseline value determined prior to the time at which the body fluid sample is obtained, (ii) has a urine output of at least 0.5 ml/kg/hr over the 2 hours preceding the time at which the body fluid sample is obtained, and (iii) has not experienced an increase of 0.3 mg/dL or greater in serum creatinine over a baseline value determined prior to the time at which the body fluid sample is obtained, or

wherein the subject has not experienced a 3-fold or greater increase in serum creatinine over a baseline value determined prior to the time at which the body fluid sample is obtained, or

wherein the subject has a urine output of at least 0.3 ml/kg/hr over the 24 hours preceding the time at which the body fluid sample is obtained, or anuria over the 12 hours preceding the time at which the body fluid sample is obtained, or

wherein the subject (i) has not experienced a 3-fold or greater increase in serum creatinine over a baseline value determined prior to the time at which the body fluid sample is obtained, (ii) has a urine output of at least 0.3 ml/kg/hr over the 24 hours preceding the time at which the body fluid sample is obtained, or anuria over the 12 hours preceding the time at which the body fluid sample is obtained, and (iii) has not experienced an increase of 0.3 mg/dL or greater in serum creatinine over a baseline value determined prior to the time at which the body fluid sample is obtained, or

wherein said correlating step comprises assigning one or more of: a likelihood that within 72 hours the subject will (i) experience a 2-fold or greater increase in serum creatinine (ii) have a urine output of less than 0.5 ml/kg/hr over a 12 hour period, or (iii) experience an increase of 0.3 mg/dL or greater in serum creatinine, wherein said correlating step preferably comprises assigning one or more of: a likelihood that within 48 hours the subject will (i) experience a 2-fold or greater increase in serum creatinine (ii) have a urine output of less than 0.5 ml/kg/hr over a 6 hour period, or (iii) experience an increase of 0.3 mg/dL or greater in serum creatinine, or

wherein said correlating step preferably comprises assigning one or more of: a likehhood that within 24 hours the subject will (i) experience a 2-fold or greater increase in serum creatinine, or (ii) have a urine output of less than 0.5 ml/kg/hr over a 6 hour period, or

wherein said correlating step preferably comprises assigning a likelihood that within 72 hours the subject will experience a 2-fold or greater increase in serum creatinine, or

wherein said correlating step preferably comprises assigning a likelihood that within 72 hours the subject will have a urine output of less than 0.5 ml kg/hr over a 6 hour period, or

wherein said correlating step preferably comprises assigning a likelihood that within 48 hours the subject will experience a 2-fold or greater increase in serum creatinine, or

wherein said correlating step preferably comprises assigning a likelihood that within 48 hours the subject will have a urine output of less than 0.5 ml kg/hr over a 6 hour period, or

wherein said correlating step preferably comprises assigning a likelihood that within 24 hours the subject will experience a 2-fold or greater increase in serum creatinine, or

wherein said correlating step preferably comprises assigning a likelihood that within 24 hours the subject will have a urine output of less than 0.5 ml/kg/hr over a 6 hour period, or

wherein said correlating step comprises assigning one or more of: a likelihood that within 72 hours the subject will (i) experience a 3-fold or greater increase in serum creatinine, or (ii) have a urine output of less than 0.3 ml/kg/hr over a 24 hour period or anuria over a 12 hour period,

wherein said correlating step preferably comprises assigning one or more of: a likelihood that within 48 hours the subject will (i) experience a 3-fold or greater increase in serum creatinine, or (ii) have a urine output of less than 0.3 ml/kg/hr over a 24 hour period or anuria over a 12 hour period, or

wherein said correlating step preferably comprises assigning one or more of: a likelihood that within 24 hours the subject will (i) experience a 3-fold or greater increase in serum creatinine, or (ii) have a urine output of less than 0.3 ml/kg/hr over a 24 hour period or anuria over a 12 hour period, or

wherein said correlating step preferably comprises assigning a likelihood that within 72 hours the subject will experience a 3-fold or greater increase in serum creatinine, or

wherein said correlating step preferably comprises assigning a likelihood that within 72 hours the subject will have a urine output of less than 0.3 ml/kg/hr over a 24 hour period or anuria over a 12 hour period, or

wherein said correlating step preferably comprises assigning a likelihood that within 48 hours the subject will experience a 3-fold or greater increase in serum creatinine, or

wherein said correlating step preferably comprises assigning a likelihood that within 48 hours the subject will have a urine output of less than 0.3 ml kg/hr over a 24 hour period or anuria over a 12 hour period, or

wherein said correlating step preferably comprises assigning a likelihood that within 24 hours the subject will experience a 3-fold or greater increase in serum creatinine, or

wherein said correlating step preferably comprises assigning a likelihood that within 24 hours the subject will have a urine output of less than 0.3 ml/kg/hr over a 24 hour period or anuria over a 12 hour period.

12. A method according to one of claims 1-11, wherein the body fluid sample is a urine sample, and/or

wherein said method comprises performing assays that detect a measured concentration of Insulin-like growth factor-binding protein 3 and optionally one, two or three, or more of C-C Motif chemokine 24, Cancer antigen CA 15-3, C-C Motif chemokine 18, Cathepsin D, C-X-C Motif chemokine 13, C-C motif chemokine 8, Interleukin-2 receptor alpha chain, Interleukin-11, Matrix Metalloproteinase-8, Transforming growth factor alpha, IgG1 and IgG2.

13. Use of Insulin-like growth factor-binding protein 3 and optionally one or more biomarkers selected from the group consisting of Cancer antigen CA 15-3, C-C Motif chemokine 18, C-C Motif chemokine 24, Cathepsin D, C-X-C Motif chemokine 13, C-C motif chemokine 8, Interleukin-2 receptor alpha chain, Interleukin-11, Matrix Metalloproteinase-8, Transforming growth factor alpha, IgG 1, and IgG2 for the evaluation of renal injury, preferably for the evaluation of acute renal injury.

14. A kit, comprising:

reagents for performing one or more assays configured to detect Insulin-like growth factor-binding protein 3, and optionally one or more kidney injury markers selected from the group consisting of Cancer antigen CA 15-3, C-C Motif chemokine 18, C-C Motif chemokine 24, Cathepsin D, C-X-C Motif chemokine 13, C-C motif chemokine 8, Interleukin-2 receptor alpha chain, Interleukin-11, Matrix Metalloproteinase-8, Transforming growth factor alpha, IgG1, and IgG2,

wherein at least one of said assays is preferably configured as a sandwich binding assay, or

wherein at least one of said assays is preferably configured as a competitive binding assay, or

wherein said reagents preferably comprise one or more binding reagents, each of which specifically binds one of said of kidney injury markers,

wherein a plurality of binding reagents are preferably contained in a single assay device.

15. A kit according to claim 14, wherein said one or more assays comprise assays that detect Insulin-like growth factor-binding protein 3 and optionally one, two or three, or more of C-C Motif chemokine 24, Cancer antigen CA 15-3, C-C Motif chemokine 18, Cathepsin D, C-X-C Motif chemokine 13, C-C motif chemokine 8, Interleukin-2 receptor alpha chain, Interleukin-11, Matrix Metalloproteinase-8, Transforming growth factor alpha, IgG1 and IgG2.

Europäisches
Patentamt

European
Patent Office

Office européen
des brevets

# EUROPEAN SEARCH REPORT

Application Number

EP 18 18 9709

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CHUANG SHANG-TIAN ET AL: "Over Expression of Insulin-Like Growth Factor Binding Protein 3 in Clear Cell Renal Cell Carcinoma", THE JOURNAL OF UROLOGY, ELSEVIER INC, US, vol. 179, no. 2, 31 January 2008 (2008-01-31), pages 445-449, XP009511424, ISSN: 1527-3792 * abstract * * page 448, right-hand column, paragraph 3 - page 449, left-hand column, paragraph 1 * | 1-15 | INV. G01N33/68 |
| E | WO 2011/075744 A1 (ASTUTE MEDICAL INC [US]; ANDERBERG JOSEPH [US]; GRAY JEFF [US]; MCPHER) 23 June 2011 (2011-06-23) * claims 1-132 * | 1-15 | |
| X | D R Powell ET AL: "Characterization of Insulin-Like Growth Factor Binding Protein-3 in Chronic Renal Failure Serum", Pediatric research, 1 February 1993 (1993-02-01), pages 136-143, XP055559310, United States DOI: 10.1203/00006450-199302000-00011 Retrieved from the Internet: URL:https://www.nature.com/articles/pr1993 30.pdf * abstract * | 1-15 | TECHNICAL FIELDS SEARCHED (IPC) G01N |

-/--

The present search report has been drawn up for all claims

| Place of search | Date of completion of the search | Examiner |
|---|---|---|
| Munich | 21 February 2019 | Fleitmann, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt
European
Patent Office
Office européen
des brevets

**EUROPEAN SEARCH REPORT**

Application Number

EP 18 18 9709

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ARTHUR JOHN M ET AL: "Diagnostic and prognostic biomarkers in acute renal failure", CONTRIBUTIONS TO NEPHROLOGY, KARGER, BASEL, CH, vol. 160, 1 January 2008 (2008-01-01), pages 53-64, XP009108671, ISSN: 0302-5144, DOI: 10.1159/000125929 * abstract * | 1-15 | |
| X | FRIEDLAENDER M M ET AL: "The insulin-like growth factor-I axis in acute renal failure", RENAL FAIL,, vol. 20, no. 2, 1 March 1998 (1998-03-01), pages 343-348, XP008172681, ISSN: 0886-022X, DOI: 10.3109/08860229809045120 * page 346, paragraph 2 * | 1-15 | |

TECHNICAL FIELDS
SEARCHED      (IPC)

The present search report has been drawn up for all claims

| Place of search | Date of completion of the search | Examiner |
|---|---|---|
| Munich | 21 February 2019 | Fleitmann, J |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 18 18 9709

This annex lists the patent family members relating to the patent documents cited in the above-mentioned European search report.
The members are as contained in the European Patent Office EDP file on
The European Patent Office is in no way liable for these particulars which are merely given for the purpose of information.

21-02-2019

| Patent document cited in search report | Publication date | Patent family member(s) | Publication date |
|---|---|---|---|
| WO 2011075744 A1 | 23-06-2011 | AU 2010330735 A1 | 19-07-2012 |
| | | AU 2014227447 A1 | 02-10-2014 |
| | | CA 2784889 A1 | 23-06-2011 |
| | | CN 102725636 A | 10-10-2012 |
| | | CN 104698161 A | 10-06-2015 |
| | | EA 201290387 A1 | 28-02-2013 |
| | | EP 2513649 A1 | 24-10-2012 |
| | | EP 3112871 A1 | 04-01-2017 |
| | | ES 2592386 T3 | 29-11-2016 |
| | | HK 1171260 A1 | 22-01-2016 |
| | | HK 1211082 A1 | 13-05-2016 |
| | | JP 5763098 B2 | 12-08-2015 |
| | | JP 6309434 B2 | 11-04-2018 |
| | | JP 2013515243 A | 02-05-2013 |
| | | JP 2015064371 A | 09-04-2015 |
| | | KR 20120123056 A | 07-11-2012 |
| | | KR 20180063352 A | 11-06-2018 |
| | | MX 342967 B | 19-10-2016 |
| | | NZ 600828 A | 26-09-2014 |
| | | NZ 625423 A | 27-02-2015 |
| | | NZ 702527 A | 29-07-2016 |
| | | US 2012283128 A1 | 08-11-2012 |
| | | US 2017343562 A1 | 30-11-2017 |
| | | WO 2011075744 A1 | 23-06-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61357965 A
- US 61357956 A
- US 61364304 A
- US 61364300 A
- US 6143576 A
- US 6113855 A
- US 6019944 A
- US 5985579 A
- US 5947124 A
- US 5939272 A
- US 5922615 A
- US 5885527 A
- US 5851776 A
- US 5824799 A
- US 5679526 A
- US 5525524 A
- US 5480792 A
- US 5631171 A
- US 5955377 A
- US 5571698 A, Ladner
- US 6057098 A

**Non-patent literature cited in the description**

- Harrison's Principles of Internal Medicine. McGraw Hill, 1741-1830
- Current Medical Diagnosis & Treatment. McGraw Hill, 2008, 785-815
- *Merck Manual*
- **PRAUGHT ; SHLIPAK.** *Curr Opin Nephrol Hypertens,* 2005, vol. 14, 265-270
- **CHERTOW et al.** *J Am Soc Nephrol,* 2005, vol. 16, 3365-3370
- **LASSNIGG.** *J Am Soc Nephrol,* 2004, vol. 15, 1597-1605
- **BELLOMO et al.** *Crit Care.,* 2004, vol. 8 (4), R204-12
- **KELLUM.** *Crit. Care Med.,* 2008, vol. 36, 141-45
- **RICCI et al.** *Kidney Int.,* 2008, vol. 73, 538-546
- **MCCOLLOUGH et al.** *Rev Cardiovasc Med.,* 2006, vol. 7 (4), 177-197
- **THAKAR et al.** *J. Am. Soc. Nephrol.,* 2005, vol. 16, 162-68
- **MEHRAN et al.** *J. Am. Coll. Cardiol.,* 2004, vol. 44, 1393-99
- **WIJEYSUNDERA et al.** *JAMA,* 2007, vol. 297, 1801-9
- **GOLDSTEIN ; CHAWLA.** *Clin. J. Am. Soc. Nephrol.,* 2010, vol. 5, 943-49
- **CHAWLA et al.** *Kidney Intl.,* 2005, vol. 68, 2274-80
- The Immunoassay Handbook. Stockton Press, 1994
- Fundamental Immunology. Raven Press, 1993
- **WILSON.** *J. Immunol. Methods,* 1994, vol. 175, 267-273
- **YARMUSH.** *J. Biochem. Biophys. Methods,* 1992, vol. 25, 85-97
- **WARD et al.** *Nature,* 1989, vol. 341, 544-546
- **VAN ERP et al.** *J. Immunoassay,* 1991, vol. 12, 425-43
- **NELSON ; GRISWOLD.** *Comput. Methods Programs Biomed.,* 1988, vol. 27, 65-8
- **CWIRLA et al.** *Proc. Natl. Acad. Sci. USA,* 1990, vol. 87, 6378-82
- **DEVLIN et al.** *Science,* 1990, vol. 249, 404-6
- **SCOTT ; SMITH.** *Science,* 1990, vol. 249, 386-88
- **FISCHER et al.** *Intensive Care Med.,* 2003, vol. 29, 1043-51
- **BAGSHAW et al.** *Nephrol. Dial. Transplant.,* 2008, vol. 23, 1203-1210
- Merck Manual of Diagnosis and Therapy. Merck Research Laboratories, 1999
- **HANLEY, J. A. ; MCNEIL, B.J.** The meaning and use of the area under a receiver operating characteristic (ROC) curve. *Radiology,* 1982, vol. 143, 29-36

SEQUENCE LISTING

<110> ASTUTE MEDICAL, INC.

<120> METHODS AND COMPOSITIONS FOR DIAGNOSIS AND PROGNOSIS OF RENAL
      INJURY AND RENAL FAILURE

<130> BLG14661PCTEPD2

<140> Divisional Application of EP 15 151 607.7
<141> 2011-06-23

<150> 61/364,300
<151> 2010-07-14

<150> 61/364,304
<151> 2010-07-14

<150> 61/357,956
<151> 2010-06-23

<150> 61/357,965
<151> 2010-06-23

<160> 13

<170> PatentIn version 3.5

<210> 1
<211> 99
<212> PRT
<213> Homo sapiens

<400> 1
Met Lys Val Ser Ala Ala Leu Leu Cys Leu Leu Leu Met Ala Ala Thr
1               5                   10                  15


Phe Ser Pro Gln Gly Leu Ala Gln Pro Asp Ser Val Ser Ile Pro Ile
            20                  25                  30


Thr Cys Cys Phe Asn Val Ile Asn Arg Lys Ile Pro Ile Gln Arg Leu
        35                  40                  45


Glu Ser Tyr Thr Arg Ile Thr Asn Ile Gln Cys Pro Lys Glu Ala Val
    50                  55                  60


Ile Phe Lys Thr Lys Arg Gly Lys Glu Val Cys Ala Asp Pro Lys Glu
65                  70                  75                  80


Arg Trp Val Arg Asp Ser Met Lys His Leu Asp Gln Ile Phe Gln Asn
                85                  90                  95


Leu Lys Pro


<210> 2

```
<211> 272
<212> PRT
<213> Homo sapiens

<400> 2
Met Asp Ser Tyr Leu Leu Met Trp Gly Leu Leu Thr Phe Ile Met Val
1               5                   10                  15

Pro Gly Cys Gln Ala Glu Leu Cys Asp Asp Asp Pro Pro Glu Ile Pro
            20                  25                  30

His Ala Thr Phe Lys Ala Met Ala Tyr Lys Glu Gly Thr Met Leu Asn
        35                  40                  45

Cys Glu Cys Lys Arg Gly Phe Arg Arg Ile Lys Ser Gly Ser Leu Tyr
    50                  55                  60

Met Leu Cys Thr Gly Asn Ser Ser His Ser Ser Trp Asp Asn Gln Cys
65                  70                  75                  80

Gln Cys Thr Ser Ser Ala Thr Arg Asn Thr Thr Lys Gln Val Thr Pro
            85                  90                  95

Gln Pro Glu Glu Gln Lys Glu Arg Lys Thr Thr Glu Met Gln Ser Pro
            100                 105                 110

Met Gln Pro Val Asp Gln Ala Ser Leu Pro Gly His Cys Arg Glu Pro
        115                 120                 125

Pro Pro Trp Glu Asn Glu Ala Thr Glu Arg Ile Tyr His Phe Val Val
        130                 135                 140

Gly Gln Met Val Tyr Tyr Gln Cys Val Gln Gly Tyr Arg Ala Leu His
145                 150                 155                 160

Arg Gly Pro Ala Glu Ser Val Cys Lys Met Thr His Gly Lys Thr Arg
            165                 170                 175

Trp Thr Gln Pro Gln Leu Ile Cys Thr Gly Glu Met Glu Thr Ser Gln
            180                 185                 190

Phe Pro Gly Glu Glu Lys Pro Gln Ala Ser Pro Glu Gly Arg Pro Glu
        195                 200                 205

Ser Glu Thr Ser Cys Leu Val Thr Thr Thr Asp Phe Gln Ile Gln Thr
        210                 215                 220

Glu Met Ala Ala Thr Met Glu Thr Ser Ile Phe Thr Thr Glu Tyr Gln
225                 230                 235                 240
```

```
Val Ala Val Ala Gly Cys Val Phe Leu Leu Ile Ser Val Leu Leu Leu
            245             250             255

Ser Gly Leu Thr Trp Gln Arg Arg Gln Arg Lys Ser Arg Arg Thr Ile
            260             265             270


<210> 3
<211> 291
<212> PRT
<213> Homo sapiens

<400> 3
Met Gln Arg Ala Arg Pro Thr Leu Trp Ala Ala Ala Leu Thr Leu Leu
1               5               10              15

Val Leu Leu Arg Gly Pro Pro Val Ala Arg Ala Gly Ala Ser Ser Ala
            20              25              30

Gly Leu Gly Pro Val Val Arg Cys Glu Pro Cys Asp Ala Arg Ala Leu
            35              40              45

Ala Gln Cys Ala Pro Pro Pro Ala Val Cys Ala Glu Leu Val Arg Glu
    50              55              60

Pro Gly Cys Gly Cys Cys Leu Thr Cys Ala Leu Ser Glu Gly Gln Pro
65              70              75              80

Cys Gly Ile Tyr Thr Glu Arg Cys Gly Ser Gly Leu Arg Cys Gln Pro
            85              90              95

Ser Pro Asp Glu Ala Arg Pro Leu Gln Ala Leu Leu Asp Gly Arg Gly
            100             105             110

Leu Cys Val Asn Ala Ser Ala Val Ser Arg Leu Arg Ala Tyr Leu Leu
            115             120             125

Pro Ala Pro Pro Ala Pro Gly Asn Ala Ser Glu Ser Glu Glu Asp Arg
    130             135             140

Ser Ala Gly Ser Val Glu Ser Pro Ser Val Ser Ser Thr His Arg Val
145             150             155             160

Ser Asp Pro Lys Phe His Pro Leu His Ser Lys Ile Ile Ile Ile Lys
            165             170             175

Lys Gly His Ala Lys Asp Ser Gln Arg Tyr Lys Val Asp Tyr Glu Ser
            180             185             190
```

```
Gln Ser Thr Asp Thr Gln Asn Phe Ser Ser Glu Ser Lys Arg Glu Thr
        195             200             205

Glu Tyr Gly Pro Cys Arg Arg Glu Met Glu Asp Thr Leu Asn His Leu
        210             215             220

Lys Phe Leu Asn Val Leu Ser Pro Arg Gly Val His Ile Pro Asn Cys
225             230             235             240

Asp Lys Lys Gly Phe Tyr Lys Lys Lys Gln Cys Arg Pro Ser Lys Gly
            245             250             255

Arg Lys Arg Gly Phe Cys Trp Cys Val Asp Lys Tyr Gly Gln Pro Leu
            260             265             270

Pro Gly Tyr Thr Thr Lys Gly Lys Glu Asp Val His Cys Tyr Ser Met
        275             280             285

Gln Ser Lys
        290
```

<210> 4
<211> 199
<212> PRT
<213> Homo sapiens

<400> 4

```
Met Asn Cys Val Cys Arg Leu Val Leu Val Val Leu Ser Leu Trp Pro
1               5               10              15

Asp Thr Ala Val Ala Pro Gly Pro Pro Gly Pro Pro Arg Val Ser
            20              25              30

Pro Asp Pro Arg Ala Glu Leu Asp Ser Thr Val Leu Leu Thr Arg Ser
        35              40              45

Leu Leu Ala Asp Thr Arg Gln Leu Ala Ala Gln Leu Arg Asp Lys Phe
    50              55              60

Pro Ala Asp Gly Asp His Asn Leu Asp Ser Leu Pro Thr Leu Ala Met
65              70              75              80

Ser Ala Gly Ala Leu Gly Ala Leu Gln Leu Pro Gly Val Leu Thr Arg
            85              90              95

Leu Arg Ala Asp Leu Leu Ser Tyr Leu Arg His Val Gln Trp Leu Arg
        100             105             110
```

```
Arg Ala Gly Gly Ser Ser Leu Lys Thr Leu Glu Pro Glu Leu Gly Thr
        115                 120                 125


Leu Gln Ala Arg Leu Asp Arg Leu Leu Arg Arg Leu Gln Leu Leu Met
        130                 135                 140


Ser Arg Leu Ala Leu Pro Gln Pro Pro Asp Pro Pro Ala Pro Pro
145                 150                 155                 160


Leu Ala Pro Pro Ser Ser Ala Trp Gly Gly Ile Arg Ala Ala His Ala
                165                 170                 175


Ile Leu Gly Gly Leu His Leu Thr Leu Asp Trp Ala Val Arg Gly Leu
                180                 185                 190


Leu Leu Leu Lys Thr Arg Leu
                195
```

```
<210> 5
<211> 467
<212> PRT
<213> Homo sapiens

<400> 5
Met Phe Ser Leu Lys Thr Leu Pro Phe Leu Leu Leu Leu His Val Gln
1               5                   10                  15


Ile Ser Lys Ala Phe Pro Val Ser Ser Lys Glu Lys Asn Thr Lys Thr
            20                  25                  30


Val Gln Asp Tyr Leu Glu Lys Phe Tyr Gln Leu Pro Ser Asn Gln Tyr
        35                  40                  45


Gln Ser Thr Arg Lys Asn Gly Thr Asn Val Ile Val Glu Lys Leu Lys
        50                  55                  60


Glu Met Gln Arg Phe Phe Gly Leu Asn Val Thr Gly Lys Pro Asn Glu
65                  70                  75                  80


Glu Thr Leu Asp Met Met Lys Lys Pro Arg Cys Gly Val Pro Asp Ser
                85                  90                  95


Gly Gly Phe Met Leu Thr Pro Gly Asn Pro Lys Trp Glu Arg Thr Asn
                100                 105                 110


Leu Thr Tyr Arg Ile Arg Asn Tyr Thr Pro Gln Leu Ser Glu Ala Glu
        115                 120                 125


Val Glu Arg Ala Ile Lys Asp Ala Phe Glu Leu Trp Ser Val Ala Ser
```

353

```
              130                        135                          140


    Pro Leu Ile Phe Thr Arg Ile Ser Gln Gly Glu Ala Asp Ile Asn Ile
    145                 150                 155                 160


    Ala Phe Tyr Gln Arg Asp His Gly Asp Asn Ser Pro Phe Asp Gly Pro
                    165                 170                 175


    Asn Gly Ile Leu Ala His Ala Phe Gln Pro Gly Gln Gly Ile Gly Gly
                    180                 185                 190


    Asp Ala His Phe Asp Ala Glu Glu Thr Trp Thr Asn Thr Ser Ala Asn
                195                 200                 205


    Tyr Asn Leu Phe Leu Val Ala Ala His Glu Phe Gly His Ser Leu Gly
        210                 215                 220


    Leu Ala His Ser Ser Asp Pro Gly Ala Leu Met Tyr Pro Asn Tyr Ala
    225                 230                 235                 240


    Phe Arg Glu Thr Ser Asn Tyr Ser Leu Pro Gln Asp Asp Ile Asp Gly
                    245                 250                 255


    Ile Gln Ala Ile Tyr Gly Leu Ser Ser Asn Pro Ile Gln Pro Thr Gly
                260                 265                 270


    Pro Ser Thr Pro Lys Pro Cys Asp Pro Ser Leu Thr Phe Asp Ala Ile
                275                 280                 285


    Thr Thr Leu Arg Gly Glu Ile Leu Phe Phe Lys Asp Arg Tyr Phe Trp
        290                 295                 300


    Arg Arg His Pro Gln Leu Gln Arg Val Glu Met Asn Phe Ile Ser Leu
    305                 310                 315                 320


    Phe Trp Pro Ser Leu Pro Thr Gly Ile Gln Ala Ala Tyr Glu Asp Phe
                    325                 330                 335


    Asp Arg Asp Leu Ile Phe Leu Phe Lys Gly Asn Gln Tyr Trp Ala Leu
                    340                 345                 350


    Ser Gly Tyr Asp Ile Leu Gln Gly Tyr Pro Lys Asp Ile Ser Asn Tyr
            355                 360                 365


    Gly Phe Pro Ser Ser Val Gln Ala Ile Asp Ala Ala Val Phe Tyr Arg
        370                 375                 380
```

354

Ser Lys Thr Tyr Phe Phe Val Asn Asp Gln Phe Trp Arg Tyr Asp Asn
385 390 395 400

Gln Arg Gln Phe Met Glu Pro Gly Tyr Pro Lys Ser Ile Ser Gly Ala
405 410 415

Phe Pro Gly Ile Glu Ser Lys Val Asp Ala Val Phe Gln Gln Glu His
420 425 430

Phe Phe His Val Phe Ser Gly Pro Arg Tyr Tyr Ala Phe Asp Leu Ile
435 440 445

Ala Gln Arg Val Thr Arg Val Ala Arg Gly Asn Lys Trp Leu Asn Cys
450 455 460

Arg Tyr Gly
465


<210> 6
<211> 160
<212> PRT
<213> Homo sapiens

<400> 6
Met Val Pro Ser Ala Gly Gln Leu Ala Leu Phe Ala Leu Gly Ile Val
1 5 10 15

Leu Ala Ala Cys Gln Ala Leu Glu Asn Ser Thr Ser Pro Leu Ser Ala
20 25 30

Asp Pro Pro Val Ala Ala Ala Val Val Ser His Phe Asn Asp Cys Pro
35 40 45

Asp Ser His Thr Gln Phe Cys Phe His Gly Thr Cys Arg Phe Leu Val
50 55 60

Gln Glu Asp Lys Pro Ala Cys Val Cys His Ser Gly Tyr Val Gly Ala
65 70 75 80

Arg Cys Glu His Ala Asp Leu Leu Ala Val Val Ala Ala Ser Gln Lys
85 90 95

Lys Gln Ala Ile Thr Ala Leu Val Val Val Ser Ile Val Ala Leu Ala
100 105 110

Val Leu Ile Ile Thr Cys Val Leu Ile His Cys Cys Gln Val Arg Lys
115 120 125

His Cys Glu Trp Cys Arg Ala Leu Ile Cys Arg His Glu Lys Pro Ser

```
        130                    135                      140

        Ala Leu Leu Lys Gly Arg Thr Ala Cys Cys His Ser Glu Thr Val Val
        145                 150             155                     160


<210> 7
<211> 22152
<212> PRT
<213> Homo sapiens


<220>
<221> MOD_RES
<222> (13877)..(13878)
<223> Any amino acid

<220>
<221> MOD_RES
<222> (13880)..(13880)
<223> Any amino acid

<220>
<221> MOD_RES
<222> (13887)..(13887)
<223> Any amino acid

<220>
<221> MOD_RES
<222> (13890)..(13891)
<223> Any amino acid

<220>
<221> MOD_RES
<222> (13893)..(13893)
<223> Any amino acid

<220>
<221> MOD_RES
<222> (13903)..(13903)
<223> Any amino acid

<220>
<221> MOD_RES
<222> (13913)..(13914)
<223> Any amino acid

<220>
<221> MOD_RES
<222> (13916)..(13916)
<223> Any amino acid

<220>
<221> MOD_RES
<222> (13928)..(13929)
<223> Any amino acid

<220>
<221> MOD_RES
<222> (13938)..(13938)
<223> Any amino acid
```

```
<220>
<221> MOD_RES
<222> (13940)..(13941)
<223> Any amino acid

<220>
<221> MOD_RES
<222> (14569)..(14571)
<223> Any amino acid

<220>
<221> MOD_RES
<222> (14575)..(14575)
<223> Any amino acid

<220>
<221> MOD_RES
<222> (14579)..(14579)
<223> Any amino acid

<220>
<221> MOD_RES
<222> (14581)..(14581)
<223> Any amino acid

<220>
<221> MOD_RES
<222> (14587)..(14591)
<223> Any amino acid

<220>
<221> MOD_RES
<222> (14593)..(14594)
<223> Any amino acid

<220>
<221> MOD_RES
<222> (14725)..(14727)
<223> Any amino acid

<220>
<221> MOD_RES
<222> (14731)..(14731)
<223> Any amino acid

<220>
<221> MOD_RES
<222> (14735)..(14735)
<223> Any amino acid

<220>
<221> MOD_RES
<222> (14737)..(14737)
<223> Any amino acid

<220>
<221> MOD_RES
<222> (14743)..(14747)
<223> Any amino acid

<220>
<221> MOD_RES
<222> (14749)..(14750)
```

```
<223> Any amino acid

<220>
<221> MOD_RES
<222> (15661)..(15663)
<223> Any amino acid

<220>
<221> MOD_RES
<222> (15667)..(15667)
<223> Any amino acid

<220>
<221> MOD_RES
<222> (15671)..(15671)
<223> Any amino acid

<220>
<221> MOD_RES
<222> (15673)..(15673)
<223> Any amino acid

<220>
<221> MOD_RES
<222> (15679)..(15683)
<223> Any amino acid

<220>
<221> MOD_RES
<222> (15685)..(15686)
<223> Any amino acid

<220>
<221> MOD_RES
<222> (15972)..(15974)
<223> Any amino acid

<220>
<221> MOD_RES
<222> (15978)..(15978)
<223> Any amino acid

<220>
<221> MOD_RES
<222> (15982)..(15982)
<223> Any amino acid

<220>
<221> MOD_RES
<222> (15984)..(15984)
<223> Any amino acid

<220>
<221> MOD_RES
<222> (15990)..(15994)
<223> Any amino acid

<220>
<221> MOD_RES
<222> (15996)..(15997)
<223> Any amino acid

<220>
```

```
<221> MOD_RES
<222> (16008)..(16008)
<223> Any amino acid

<220>
<221> MOD_RES
<222> (16015)..(16015)
<223> Any amino acid

<220>
<221> MOD_RES
<222> (16017)..(16017)
<223> Any amino acid

<220>
<221> MOD_RES
<222> (16021)..(16021)
<223> Any amino acid

<220>
<221> MOD_RES
<222> (16025)..(16025)
<223> Any amino acid

<220>
<221> MOD_RES
<222> (16034)..(16034)
<223> Any amino acid

<220>
<221> MOD_RES
<222> (16037)..(16037)
<223> Any amino acid

<220>
<221> MOD_RES
<222> (16040)..(16040)
<223> Any amino acid

<220>
<221> MOD_RES
<222> (16046)..(16046)
<223> Any amino acid

<220>
<221> MOD_RES
<222> (16051)..(16051)
<223> Any amino acid

<220>
<221> MOD_RES
<222> (16053)..(16055)
<223> Any amino acid

<220>
<221> MOD_RES
<222> (16058)..(16058)
<223> Any amino acid

<220>
<221> MOD_RES
<222> (16062)..(16063)
<223> Any amino acid
```

```
<220>
<221> MOD_RES
<222> (16065)..(16065)
<223> Any amino acid

<220>
<221> MOD_RES
<222> (16072)..(16072)
<223> Any amino acid

<220>
<221> MOD_RES
<222> (16075)..(16076)
<223> Any amino acid

<220>
<221> MOD_RES
<222> (16078)..(16078)
<223> Any amino acid

<220>
<221> MOD_RES
<222> (16088)..(16088)
<223> Any amino acid

<220>
<221> MOD_RES
<222> (16268)..(16269)
<223> Any amino acid

<220>
<221> MOD_RES
<222> (16278)..(16278)
<223> Any amino acid

<220>
<221> MOD_RES
<222> (16280)..(16281)
<223> Any amino acid

<220>
<221> MOD_RES
<222> (16373)..(16374)
<223> Any amino acid

<220>
<221> MOD_RES
<222> (16376)..(16376)
<223> Any amino acid

<220>
<221> MOD_RES
<222> (16383)..(16383)
<223> Any amino acid

<220>
<221> MOD_RES
<222> (16386)..(16387)
<223> Any amino acid

<220>
<221> MOD_RES
```

```
<222> (16389)..(16389)
<223> Any amino acid

<220>
<221> MOD_RES
<222> (16399)..(16399)
<223> Any amino acid

<220>
<221> MOD_RES
<222> (16409)..(16410)
<223> Any amino acid

<220>
<221> MOD_RES
<222> (16412)..(16412)
<223> Any amino acid

<220>
<221> MOD_RES
<222> (16424)..(16425)
<223> Any amino acid

<220>
<221> MOD_RES
<222> (16434)..(16434)
<223> Any amino acid

<220>
<221> MOD_RES
<222> (16436)..(16437)
<223> Any amino acid

<220>
<221> MOD_RES
<222> (16439)..(16441)
<223> Any amino acid

<220>
<221> MOD_RES
<222> (16445)..(16445)
<223> Any amino acid

<220>
<221> MOD_RES
<222> (16449)..(16449)
<223> Any amino acid

<220>
<221> MOD_RES
<222> (16451)..(16451)
<223> Any amino acid

<220>
<221> MOD_RES
<222> (16457)..(16461)
<223> Any amino acid

<220>
<221> MOD_RES
<222> (16463)..(16464)
<223> Any amino acid
```

```
<220>
<221> MOD_RES
<222> (16841)..(16842)
<223> Any amino acid

<220>
<221> MOD_RES
<222> (16844)..(16844)
<223> Any amino acid

<220>
<221> MOD_RES
<222> (16851)..(16851)
<223> Any amino acid

<220>
<221> MOD_RES
<222> (16854)..(16855)
<223> Any amino acid

<220>
<221> MOD_RES
<222> (16857)..(16857)
<223> Any amino acid

<220>
<221> MOD_RES
<222> (16867)..(16867)
<223> Any amino acid

<220>
<221> MOD_RES
<222> (16877)..(16878)
<223> Any amino acid

<220>
<221> MOD_RES
<222> (16880)..(16880)
<223> Any amino acid

<220>
<221> MOD_RES
<222> (16892)..(16893)
<223> Any amino acid

<220>
<221> MOD_RES
<222> (16902)..(16902)
<223> Any amino acid

<220>
<221> MOD_RES
<222> (16904)..(16905)
<223> Any amino acid

<220>
<221> MOD_RES
<222> (16907)..(16909)
<223> Any amino acid

<220>
<221> MOD_RES
<222> (16913)..(16913)
```

```
<223> Any amino acid

<220>
<221> MOD_RES
<222> (16917)..(16917)
<223> Any amino acid

<220>
<221> MOD_RES
<222> (16919)..(16919)
<223> Any amino acid

<220>
<221> MOD_RES
<222> (16925)..(16929)
<223> Any amino acid

<220>
<221> MOD_RES
<222> (16931)..(16932)
<223> Any amino acid

<220>
<221> MOD_RES
<222> (17465)..(17466)
<223> Any amino acid

<220>
<221> MOD_RES
<222> (17468)..(17468)
<223> Any amino acid

<220>
<221> MOD_RES
<222> (17475)..(17475)
<223> Any amino acid

<220>
<221> MOD_RES
<222> (17478)..(17479)
<223> Any amino acid

<220>
<221> MOD_RES
<222> (17481)..(17481)
<223> Any amino acid

<220>
<221> MOD_RES
<222> (17491)..(17491)
<223> Any amino acid

<220>
<221> MOD_RES
<222> (17501)..(17502)
<223> Any amino acid

<220>
<221> MOD_RES
<222> (17504)..(17504)
<223> Any amino acid

<220>
```

```
<221> MOD_RES
<222> (17516)..(17517)
<223> Any amino acid

<220>
<221> MOD_RES
<222> (17526)..(17526)
<223> Any amino acid

<220>
<221> MOD_RES
<222> (17528)..(17529)
<223> Any amino acid

<220>
<221> MOD_RES
<222> (17531)..(17533)
<223> Any amino acid

<220>
<221> MOD_RES
<222> (17537)..(17537)
<223> Any amino acid

<220>
<221> MOD_RES
<222> (17541)..(17541)
<223> Any amino acid

<220>
<221> MOD_RES
<222> (17543)..(17543)
<223> Any amino acid

<220>
<221> MOD_RES
<222> (17549)..(17553)
<223> Any amino acid

<220>
<221> MOD_RES
<222> (17555)..(17556)
<223> Any amino acid

<220>
<221> MOD_RES
<222> (17567)..(17567)
<223> Any amino acid

<220>
<221> MOD_RES
<222> (17574)..(17574)
<223> Any amino acid

<220>
<221> MOD_RES
<222> (17576)..(17576)
<223> Any amino acid

<220>
<221> MOD_RES
<222> (17580)..(17580)
<223> Any amino acid
```

```
<220>
<221> MOD_RES
<222> (17584)..(17584)
<223> Any amino acid

<220>
<221> MOD_RES
<222> (17593)..(17593)
<223> Any amino acid

<220>
<221> MOD_RES
<222> (17596)..(17596)
<223> Any amino acid

<220>
<221> MOD_RES
<222> (17599)..(17599)
<223> Any amino acid

<220>
<221> MOD_RES
<222> (17605)..(17605)
<223> Any amino acid

<220>
<221> MOD_RES
<222> (17610)..(17610)
<223> Any amino acid

<220>
<221> MOD_RES
<222> (17612)..(17614)
<223> Any amino acid

<220>
<221> MOD_RES
<222> (17617)..(17617)
<223> Any amino acid

<220>
<221> MOD_RES
<222> (17621)..(17622)
<223> Any amino acid

<220>
<221> MOD_RES
<222> (17624)..(17624)
<223> Any amino acid

<220>
<221> MOD_RES
<222> (17631)..(17631)
<223> Any amino acid

<220>
<221> MOD_RES
<222> (17777)..(17778)
<223> Any amino acid

<220>
<221> MOD_RES
```

```
<222> (17780)..(17780)
<223> Any amino acid

<220>
<221> MOD_RES
<222> (17787)..(17787)
<223> Any amino acid

<220>
<221> MOD_RES
<222> (17790)..(17791)
<223> Any amino acid

<220>
<221> MOD_RES
<222> (17793)..(17793)
<223> Any amino acid

<220>
<221> MOD_RES
<222> (17803)..(17803)
<223> Any amino acid

<220>
<221> MOD_RES
<222> (17813)..(17814)
<223> Any amino acid

<220>
<221> MOD_RES
<222> (17816)..(17816)
<223> Any amino acid

<220>
<221> MOD_RES
<222> (17828)..(17829)
<223> Any amino acid

<220>
<221> MOD_RES
<222> (17838)..(17838)
<223> Any amino acid

<220>
<221> MOD_RES
<222> (17840)..(17841)
<223> Any amino acid

<220>
<221> MOD_RES
<222> (17843)..(17845)
<223> Any amino acid

<220>
<221> MOD_RES
<222> (17849)..(17849)
<223> Any amino acid

<220>
<221> MOD_RES
<222> (17853)..(17853)
<223> Any amino acid
```

```
<220>
<221> MOD_RES
<222> (17855)..(17855)
<223> Any amino acid

<220>
<221> MOD_RES
<222> (17861)..(17865)
<223> Any amino acid

<220>
<221> MOD_RES
<222> (17867)..(17868)
<223> Any amino acid

<220>
<221> MOD_RES
<222> (17879)..(17879)
<223> Any amino acid

<220>
<221> MOD_RES
<222> (17886)..(17886)
<223> Any amino acid

<220>
<221> MOD_RES
<222> (17888)..(17888)
<223> Any amino acid

<220>
<221> MOD_RES
<222> (17892)..(17892)
<223> Any amino acid

<220>
<221> MOD_RES
<222> (17896)..(17896)
<223> Any amino acid

<220>
<221> MOD_RES
<222> (17905)..(17905)
<223> Any amino acid

<220>
<221> MOD_RES
<222> (17908)..(17908)
<223> Any amino acid

<220>
<221> MOD_RES
<222> (17911)..(17911)
<223> Any amino acid

<220>
<221> MOD_RES
<222> (17917)..(17917)
<223> Any amino acid

<220>
<221> MOD_RES
<222> (17922)..(17922)
```

```
<223> Any amino acid

<220>
<221> MOD_RES
<222> (17924)..(17926)
<223> Any amino acid

<220>
<221> MOD_RES
<222> (17929)..(17929)
<223> Any amino acid

<220>
<221> MOD_RES
<222> (17933)..(17934)
<223> Any amino acid

<220>
<221> MOD_RES
<222> (17936)..(17936)
<223> Any amino acid

<220>
<221> MOD_RES
<222> (17943)..(17943)
<223> Any amino acid

<220>
<221> MOD_RES
<222> (17946)..(17947)
<223> Any amino acid

<220>
<221> MOD_RES
<222> (17949)..(17949)
<223> Any amino acid

<220>
<221> MOD_RES
<222> (17959)..(17959)
<223> Any amino acid

<220>
<221> MOD_RES
<222> (18089)..(18090)
<223> Any amino acid

<220>
<221> MOD_RES
<222> (18092)..(18092)
<223> Any amino acid

<220>
<221> MOD_RES
<222> (18099)..(18099)
<223> Any amino acid

<220>
<221> MOD_RES
<222> (18102)..(18103)
<223> Any amino acid

<220>
```

```
<221> MOD_RES
<222> (18105)..(18105)
<223> Any amino acid

<220>
<221> MOD_RES
<222> (18115)..(18115)
<223> Any amino acid

<220>
<221> MOD_RES
<222> (18125)..(18126)
<223> Any amino acid

<220>
<221> MOD_RES
<222> (18128)..(18128)
<223> Any amino acid

<220>
<221> MOD_RES
<222> (18140)..(18141)
<223> Any amino acid

<220>
<221> MOD_RES
<222> (18150)..(18150)
<223> Any amino acid

<220>
<221> MOD_RES
<222> (18152)..(18153)
<223> Any amino acid

<220>
<221> MOD_RES
<222> (18155)..(18157)
<223> Any amino acid

<220>
<221> MOD_RES
<222> (18161)..(18161)
<223> Any amino acid

<220>
<221> MOD_RES
<222> (18165)..(18165)
<223> Any amino acid

<220>
<221> MOD_RES
<222> (18167)..(18167)
<223> Any amino acid

<220>
<221> MOD_RES
<222> (18173)..(18177)
<223> Any amino acid

<220>
<221> MOD_RES
<222> (18179)..(18180)
<223> Any amino acid
```

```
<220>
<221> MOD_RES
<222> (18191)..(18191)
<223> Any amino acid

<220>
<221> MOD_RES
<222> (18198)..(18198)
<223> Any amino acid

<220>
<221> MOD_RES
<222> (18200)..(18200)
<223> Any amino acid

<220>
<221> MOD_RES
<222> (18204)..(18204)
<223> Any amino acid

<220>
<221> MOD_RES
<222> (18208)..(18208)
<223> Any amino acid

<220>
<221> MOD_RES
<222> (18217)..(18217)
<223> Any amino acid

<220>
<221> MOD_RES
<222> (18220)..(18220)
<223> Any amino acid

<220>
<221> MOD_RES
<222> (18223)..(18223)
<223> Any amino acid

<220>
<221> MOD_RES
<222> (18229)..(18229)
<223> Any amino acid

<220>
<221> MOD_RES
<222> (18234)..(18234)
<223> Any amino acid

<220>
<221> MOD_RES
<222> (18236)..(18238)
<223> Any amino acid

<220>
<221> MOD_RES
<222> (18241)..(18241)
<223> Any amino acid

<220>
<221> MOD_RES
```

```
<222> (18245)..(18246)
<223> Any amino acid

<220>
<221> MOD_RES
<222> (18248)..(18248)
<223> Any amino acid

<220>
<221> MOD_RES
<222> (18255)..(18255)
<223> Any amino acid

<220>
<221> MOD_RES
<222> (18258)..(18259)
<223> Any amino acid

<220>
<221> MOD_RES
<222> (18261)..(18261)
<223> Any amino acid

<220>
<221> MOD_RES
<222> (18271)..(18271)
<223> Any amino acid

<220>
<221> MOD_RES
<222> (18401)..(18402)
<223> Any amino acid

<220>
<221> MOD_RES
<222> (18404)..(18404)
<223> Any amino acid

<220>
<221> MOD_RES
<222> (18411)..(18411)
<223> Any amino acid

<220>
<221> MOD_RES
<222> (18414)..(18415)
<223> Any amino acid

<220>
<221> MOD_RES
<222> (18417)..(18417)
<223> Any amino acid

<220>
<221> MOD_RES
<222> (18427)..(18427)
<223> Any amino acid

<220>
<221> MOD_RES
<222> (18437)..(18438)
<223> Any amino acid
```

```
<220>
<221> MOD_RES
<222> (18440)..(18440)
<223> Any amino acid

<220>
<221> MOD_RES
<222> (18452)..(18453)
<223> Any amino acid

<220>
<221> MOD_RES
<222> (18462)..(18462)
<223> Any amino acid

<220>
<221> MOD_RES
<222> (18464)..(18465)
<223> Any amino acid

<220>
<221> MOD_RES
<222> (18467)..(18469)
<223> Any amino acid

<220>
<221> MOD_RES
<222> (18473)..(18473)
<223> Any amino acid

<220>
<221> MOD_RES
<222> (18477)..(18477)
<223> Any amino acid

<220>
<221> MOD_RES
<222> (18479)..(18479)
<223> Any amino acid

<220>
<221> MOD_RES
<222> (18485)..(18489)
<223> Any amino acid

<220>
<221> MOD_RES
<222> (18491)..(18492)
<223> Any amino acid

<220>
<221> MOD_RES
<222> (18503)..(18503)
<223> Any amino acid

<220>
<221> MOD_RES
<222> (18510)..(18510)
<223> Any amino acid

<220>
<221> MOD_RES
<222> (18512)..(18512)
```

```
<223> Any amino acid

<220>
<221> MOD_RES
<222> (18516)..(18516)
<223> Any amino acid

<220>
<221> MOD_RES
<222> (18520)..(18520)
<223> Any amino acid

<220>
<221> MOD_RES
<222> (18529)..(18529)
<223> Any amino acid

<220>
<221> MOD_RES
<222> (18532)..(18532)
<223> Any amino acid

<220>
<221> MOD_RES
<222> (18535)..(18535)
<223> Any amino acid

<220>
<221> MOD_RES
<222> (18541)..(18541)
<223> Any amino acid

<220>
<221> MOD_RES
<222> (18546)..(18546)
<223> Any amino acid

<220>
<221> MOD_RES
<222> (18548)..(18550)
<223> Any amino acid

<220>
<221> MOD_RES
<222> (18553)..(18553)
<223> Any amino acid

<220>
<221> MOD_RES
<222> (18557)..(18558)
<223> Any amino acid

<220>
<221> MOD_RES
<222> (18560)..(18560)
<223> Any amino acid

<220>
<221> MOD_RES
<222> (18567)..(18567)
<223> Any amino acid

<220>
```

```
<221> MOD_RES
<222> (18570)..(18571)
<223> Any amino acid

<220>
<221> MOD_RES
<222> (18573)..(18573)
<223> Any amino acid

<220>
<221> MOD_RES
<222> (18583)..(18583)
<223> Any amino acid

<220>
<221> MOD_RES
<222> (18713)..(18714)
<223> Any amino acid

<220>
<221> MOD_RES
<222> (18716)..(18716)
<223> Any amino acid

<220>
<221> MOD_RES
<222> (18723)..(18723)
<223> Any amino acid

<220>
<221> MOD_RES
<222> (18726)..(18727)
<223> Any amino acid

<220>
<221> MOD_RES
<222> (18729)..(18729)
<223> Any amino acid

<220>
<221> MOD_RES
<222> (18739)..(18739)
<223> Any amino acid

<220>
<221> MOD_RES
<222> (18749)..(18750)
<223> Any amino acid

<220>
<221> MOD_RES
<222> (18752)..(18752)
<223> Any amino acid

<220>
<221> MOD_RES
<222> (18764)..(18765)
<223> Any amino acid

<220>
<221> MOD_RES
<222> (18774)..(18774)
<223> Any amino acid
```

```
<220>
<221> MOD_RES
<222> (18776)..(18777)
<223> Any amino acid

<220>
<221> MOD_RES
<222> (18779)..(18781)
<223> Any amino acid

<220>
<221> MOD_RES
<222> (18785)..(18785)
<223> Any amino acid

<220>
<221> MOD_RES
<222> (18789)..(18789)
<223> Any amino acid

<220>
<221> MOD_RES
<222> (18791)..(18791)
<223> Any amino acid

<220>
<221> MOD_RES
<222> (18797)..(18801)
<223> Any amino acid

<220>
<221> MOD_RES
<222> (18803)..(18804)
<223> Any amino acid

<220>
<221> MOD_RES
<222> (18815)..(18815)
<223> Any amino acid

<220>
<221> MOD_RES
<222> (18822)..(18822)
<223> Any amino acid

<220>
<221> MOD_RES
<222> (18824)..(18824)
<223> Any amino acid

<220>
<221> MOD_RES
<222> (18828)..(18828)
<223> Any amino acid

<220>
<221> MOD_RES
<222> (18832)..(18832)
<223> Any amino acid

<220>
<221> MOD_RES
```

```
<222> (18841)..(18841)
<223> Any amino acid

<220>
<221> MOD_RES
<222> (18844)..(18844)
<223> Any amino acid

<220>
<221> MOD_RES
<222> (18847)..(18847)
<223> Any amino acid

<220>
<221> MOD_RES
<222> (18853)..(18853)
<223> Any amino acid

<220>
<221> MOD_RES
<222> (18858)..(18858)
<223> Any amino acid

<220>
<221> MOD_RES
<222> (18860)..(18862)
<223> Any amino acid

<220>
<221> MOD_RES
<222> (18865)..(18865)
<223> Any amino acid

<220>
<221> MOD_RES
<222> (18869)..(18870)
<223> Any amino acid

<220>
<221> MOD_RES
<222> (18872)..(18872)
<223> Any amino acid

<220>
<221> MOD_RES
<222> (18879)..(18879)
<223> Any amino acid

<220>
<221> MOD_RES
<222> (18882)..(18883)
<223> Any amino acid

<220>
<221> MOD_RES
<222> (18885)..(18885)
<223> Any amino acid

<220>
<221> MOD_RES
<222> (18895)..(18895)
<223> Any amino acid
```

```
<220>
<221> MOD_RES
<222> (19091)..(19093)
<223> Any amino acid

<220>
<221> MOD_RES
<222> (19097)..(19097)
<223> Any amino acid

<220>
<221> MOD_RES
<222> (19101)..(19101)
<223> Any amino acid

<220>
<221> MOD_RES
<222> (19103)..(19103)
<223> Any amino acid

<220>
<221> MOD_RES
<222> (19109)..(19113)
<223> Any amino acid

<220>
<221> MOD_RES
<222> (19115)..(19116)
<223> Any amino acid

<220>
<221> MOD_RES
<222> (19127)..(19127)
<223> Any amino acid

<220>
<221> MOD_RES
<222> (19134)..(19134)
<223> Any amino acid

<220>
<221> MOD_RES
<222> (19136)..(19136)
<223> Any amino acid

<220>
<221> MOD_RES
<222> (19140)..(19140)
<223> Any amino acid

<220>
<221> MOD_RES
<222> (19144)..(19144)
<223> Any amino acid

<220>
<221> MOD_RES
<222> (19153)..(19153)
<223> Any amino acid

<220>
<221> MOD_RES
<222> (19156)..(19156)
```

```
<223> Any amino acid

<220>
<221> MOD_RES
<222> (19159)..(19159)
<223> Any amino acid

<220>
<221> MOD_RES
<222> (19165)..(19165)
<223> Any amino acid

<220>
<221> MOD_RES
<222> (19170)..(19170)
<223> Any amino acid

<220>
<221> MOD_RES
<222> (19172)..(19174)
<223> Any amino acid

<220>
<221> MOD_RES
<222> (19177)..(19177)
<223> Any amino acid

<220>
<221> MOD_RES
<222> (19181)..(19182)
<223> Any amino acid

<220>
<221> MOD_RES
<222> (19184)..(19184)
<223> Any amino acid

<220>
<221> MOD_RES
<222> (19191)..(19191)
<223> Any amino acid

<220>
<221> MOD_RES
<222> (19194)..(19195)
<223> Any amino acid

<220>
<221> MOD_RES
<222> (19197)..(19197)
<223> Any amino acid

<220>
<221> MOD_RES
<222> (19207)..(19207)
<223> Any amino acid

<220>
<221> MOD_RES
<222> (19337)..(19338)
<223> Any amino acid

<220>
```

```
<221> MOD_RES
<222> (19340)..(19340)
<223> Any amino acid

<220>
<221> MOD_RES
<222> (19347)..(19347)
<223> Any amino acid

<220>
<221> MOD_RES
<222> (19350)..(19351)
<223> Any amino acid

<220>
<221> MOD_RES
<222> (19353)..(19353)
<223> Any amino acid

<220>
<221> MOD_RES
<222> (19363)..(19363)
<223> Any amino acid

<220>
<221> MOD_RES
<222> (19373)..(19374)
<223> Any amino acid

<220>
<221> MOD_RES
<222> (19376)..(19376)
<223> Any amino acid

<220>
<221> MOD_RES
<222> (19388)..(19389)
<223> Any amino acid

<220>
<221> MOD_RES
<222> (19398)..(19398)
<223> Any amino acid

<220>
<221> MOD_RES
<222> (19400)..(19401)
<223> Any amino acid

<220>
<221> MOD_RES
<222> (19403)..(19405)
<223> Any amino acid

<220>
<221> MOD_RES
<222> (19409)..(19409)
<223> Any amino acid

<220>
<221> MOD_RES
<222> (19413)..(19413)
<223> Any amino acid
```

```
<220>
<221> MOD_RES
<222> (19415)..(19415)
<223> Any amino acid

<220>
<221> MOD_RES
<222> (19421)..(19425)
<223> Any amino acid

<220>
<221> MOD_RES
<222> (19427)..(19428)
<223> Any amino acid

<220>
<221> MOD_RES
<222> (19439)..(19439)
<223> Any amino acid

<220>
<221> MOD_RES
<222> (19446)..(19446)
<223> Any amino acid

<220>
<221> MOD_RES
<222> (19448)..(19448)
<223> Any amino acid

<220>
<221> MOD_RES
<222> (19452)..(19452)
<223> Any amino acid

<220>
<221> MOD_RES
<222> (19456)..(19456)
<223> Any amino acid

<220>
<221> MOD_RES
<222> (19465)..(19465)
<223> Any amino acid

<220>
<221> MOD_RES
<222> (19468)..(19468)
<223> Any amino acid

<220>
<221> MOD_RES
<222> (19471)..(19471)
<223> Any amino acid

<220>
<221> MOD_RES
<222> (19477)..(19477)
<223> Any amino acid

<220>
<221> MOD_RES
```

```
<222> (19482)..(19482)
<223> Any amino acid

<220>
<221> MOD_RES
<222> (19484)..(19486)
<223> Any amino acid

<220>
<221> MOD_RES
<222> (19489)..(19489)
<223> Any amino acid

<220>
<221> MOD_RES
<222> (19493)..(19494)
<223> Any amino acid

<220>
<221> MOD_RES
<222> (19496)..(19496)
<223> Any amino acid

<220>
<221> MOD_RES
<222> (19503)..(19503)
<223> Any amino acid

<220>
<221> MOD_RES
<222> (19506)..(19507)
<223> Any amino acid

<220>
<221> MOD_RES
<222> (19509)..(19509)
<223> Any amino acid

<220>
<221> MOD_RES
<222> (19519)..(19519)
<223> Any amino acid

<220>
<221> MOD_RES
<222> (19649)..(19650)
<223> Any amino acid

<220>
<221> MOD_RES
<222> (19652)..(19652)
<223> Any amino acid

<220>
<221> MOD_RES
<222> (19659)..(19659)
<223> Any amino acid

<220>
<221> MOD_RES
<222> (19662)..(19663)
<223> Any amino acid
```

```
<220>
<221> MOD_RES
<222> (19665)..(19665)
<223> Any amino acid

<220>
<221> MOD_RES
<222> (19675)..(19675)
<223> Any amino acid

<220>
<221> MOD_RES
<222> (19685)..(19686)
<223> Any amino acid

<220>
<221> MOD_RES
<222> (19688)..(19688)
<223> Any amino acid

<220>
<221> MOD_RES
<222> (19700)..(19701)
<223> Any amino acid

<220>
<221> MOD_RES
<222> (19710)..(19710)
<223> Any amino acid

<220>
<221> MOD_RES
<222> (19712)..(19713)
<223> Any amino acid

<220>
<221> MOD_RES
<222> (19715)..(19717)
<223> Any amino acid

<220>
<221> MOD_RES
<222> (19721)..(19721)
<223> Any amino acid

<220>
<221> MOD_RES
<222> (19725)..(19725)
<223> Any amino acid

<220>
<221> MOD_RES
<222> (19727)..(19727)
<223> Any amino acid

<220>
<221> MOD_RES
<222> (19733)..(19737)
<223> Any amino acid

<220>
<221> MOD_RES
<222> (19739)..(19740)
```

```
<223> Any amino acid

<220>
<221> MOD_RES
<222> (19751)..(19751)
<223> Any amino acid

<220>
<221> MOD_RES
<222> (19758)..(19758)
<223> Any amino acid

<220>
<221> MOD_RES
<222> (19760)..(19760)
<223> Any amino acid

<220>
<221> MOD_RES
<222> (19764)..(19764)
<223> Any amino acid

<220>
<221> MOD_RES
<222> (19768)..(19768)
<223> Any amino acid

<220>
<221> MOD_RES
<222> (19777)..(19777)
<223> Any amino acid

<220>
<221> MOD_RES
<222> (19780)..(19780)
<223> Any amino acid

<220>
<221> MOD_RES
<222> (19783)..(19783)
<223> Any amino acid

<220>
<221> MOD_RES
<222> (19789)..(19789)
<223> Any amino acid

<220>
<221> MOD_RES
<222> (19794)..(19794)
<223> Any amino acid

<220>
<221> MOD_RES
<222> (19796)..(19798)
<223> Any amino acid

<220>
<221> MOD_RES
<222> (19801)..(19801)
<223> Any amino acid

<220>
```

```
<221> MOD_RES
<222> (19805)..(19806)
<223> Any amino acid

<220>
<221> MOD_RES
<222> (19808)..(19808)
<223> Any amino acid

<220>
<221> MOD_RES
<222> (19815)..(19815)
<223> Any amino acid

<220>
<221> MOD_RES
<222> (19818)..(19819)
<223> Any amino acid

<220>
<221> MOD_RES
<222> (19821)..(19821)
<223> Any amino acid

<220>
<221> MOD_RES
<222> (19831)..(19831)
<223> Any amino acid

<220>
<221> MOD_RES
<222> (19960)..(19961)
<223> Any amino acid

<220>
<221> MOD_RES
<222> (19963)..(19963)
<223> Any amino acid

<220>
<221> MOD_RES
<222> (19970)..(19970)
<223> Any amino acid

<220>
<221> MOD_RES
<222> (19973)..(19974)
<223> Any amino acid

<220>
<221> MOD_RES
<222> (19976)..(19976)
<223> Any amino acid

<220>
<221> MOD_RES
<222> (19986)..(19986)
<223> Any amino acid

<220>
<221> MOD_RES
<222> (19996)..(19997)
<223> Any amino acid
```

```
<220>
<221> MOD_RES
<222> (19999)..(19999)
<223> Any amino acid

<220>
<221> MOD_RES
<222> (20011)..(20012)
<223> Any amino acid

<220>
<221> MOD_RES
<222> (20021)..(20021)
<223> Any amino acid

<220>
<221> MOD_RES
<222> (20023)..(20024)
<223> Any amino acid

<220>
<221> MOD_RES
<222> (20026)..(20028)
<223> Any amino acid

<220>
<221> MOD_RES
<222> (20032)..(20032)
<223> Any amino acid

<220>
<221> MOD_RES
<222> (20036)..(20036)
<223> Any amino acid

<220>
<221> MOD_RES
<222> (20038)..(20038)
<223> Any amino acid

<220>
<221> MOD_RES
<222> (20044)..(20048)
<223> Any amino acid

<220>
<221> MOD_RES
<222> (20050)..(20051)
<223> Any amino acid

<220>
<221> MOD_RES
<222> (20062)..(20062)
<223> Any amino acid

<220>
<221> MOD_RES
<222> (20069)..(20069)
<223> Any amino acid

<220>
<221> MOD_RES
```

```
<222> (20071)..(20071)
<223> Any amino acid

<220>
<221> MOD_RES
<222> (20075)..(20075)
<223> Any amino acid

<220>
<221> MOD_RES
<222> (20079)..(20079)
<223> Any amino acid

<220>
<221> MOD_RES
<222> (20088)..(20088)
<223> Any amino acid

<220>
<221> MOD_RES
<222> (20091)..(20091)
<223> Any amino acid

<220>
<221> MOD_RES
<222> (20094)..(20094)
<223> Any amino acid

<220>
<221> MOD_RES
<222> (20100)..(20100)
<223> Any amino acid

<220>
<221> MOD_RES
<222> (20105)..(20105)
<223> Any amino acid

<220>
<221> MOD_RES
<222> (20107)..(20109)
<223> Any amino acid

<220>
<221> MOD_RES
<222> (20112)..(20112)
<223> Any amino acid

<220>
<221> MOD_RES
<222> (20116)..(20117)
<223> Any amino acid

<220>
<221> MOD_RES
<222> (20119)..(20119)
<223> Any amino acid

<220>
<221> MOD_RES
<222> (20126)..(20126)
<223> Any amino acid
```

```
<220>
<221> MOD_RES
<222> (20129)..(20130)
<223> Any amino acid

<220>
<221> MOD_RES
<222> (20132)..(20132)
<223> Any amino acid

<220>
<221> MOD_RES
<222> (20142)..(20142)
<223> Any amino acid

<220>
<221> MOD_RES
<222> (20272)..(20273)
<223> Any amino acid

<220>
<221> MOD_RES
<222> (20275)..(20275)
<223> Any amino acid

<220>
<221> MOD_RES
<222> (20282)..(20282)
<223> Any amino acid

<220>
<221> MOD_RES
<222> (20285)..(20286)
<223> Any amino acid

<220>
<221> MOD_RES
<222> (20288)..(20288)
<223> Any amino acid

<220>
<221> MOD_RES
<222> (20298)..(20298)
<223> Any amino acid

<220>
<221> MOD_RES
<222> (20308)..(20309)
<223> Any amino acid

<220>
<221> MOD_RES
<222> (20311)..(20311)
<223> Any amino acid

<220>
<221> MOD_RES
<222> (20323)..(20324)
<223> Any amino acid

<220>
<221> MOD_RES
<222> (20333)..(20333)
```

<223> Any amino acid

<220>
<221> MOD_RES
<222> (20335)..(20336)
<223> Any amino acid

<220>
<221> MOD_RES
<222> (20806)..(20808)
<223> Any amino acid

<220>
<221> MOD_RES
<222> (20812)..(20812)
<223> Any amino acid

<220>
<221> MOD_RES
<222> (20816)..(20816)
<223> Any amino acid

<220>
<221> MOD_RES
<222> (20818)..(20818)
<223> Any amino acid

<220>
<221> MOD_RES
<222> (20824)..(20828)
<223> Any amino acid

<220>
<221> MOD_RES
<222> (20830)..(20831)
<223> Any amino acid

<400> 7
Met Leu Lys Pro Ser Gly Leu Pro Gly Ser Ser Ser Pro Thr Arg Ser
1               5                   10                  15

Leu Met Thr Gly Ser Arg Ser Thr Lys Ala Thr Pro Glu Met Asp Ser
            20                  25                  30

Gly Leu Thr Gly Ala Thr Leu Ser Pro Lys Thr Ser Thr Gly Ala Ile
            35                  40                  45

Val Val Thr Glu His Thr Leu Pro Phe Thr Ser Pro Asp Lys Thr Leu
        50                  55                  60

Ala Ser Pro Thr Ser Ser Val Val Gly Arg Thr Thr Gln Ser Leu Gly
65                  70                  75                  80

Val Met Ser Ser Ala Leu Pro Glu Ser Thr Ser Arg Gly Met Thr His
                85                  90                  95

Ser Glu Gln Arg Thr Ser Pro Ser Leu Ser Pro Gln Val Asn Gly Thr

                    100                    105                    110

Pro Ser Arg Asn Tyr Pro Ala Thr Ser Met Val Ser Gly Leu Ser Ser
        115                 120                 125

Pro Arg Thr Arg Thr Ser Ser Thr Glu Gly Asn Phe Thr Lys Glu Ala
        130                 135                 140

Ser Thr Tyr Thr Leu Thr Val Glu Thr Thr Ser Gly Pro Val Thr Glu
145                 150                 155                 160

Lys Tyr Thr Val Pro Thr Glu Thr Ser Thr Thr Glu Gly Asp Ser Thr
                165                 170                 175

Glu Thr Pro Trp Asp Thr Arg Tyr Ile Pro Val Lys Ile Thr Ser Pro
                180                 185                 190

Met Lys Thr Phe Ala Asp Ser Thr Ala Ser Lys Glu Asn Ala Pro Val
        195                 200                 205

Ser Met Thr Pro Ala Glu Thr Thr Val Thr Asp Ser His Thr Pro Gly
        210                 215                 220

Arg Thr Asn Pro Ser Phe Gly Thr Leu Tyr Ser Ser Phe Leu Asp Leu
225                 230                 235                 240

Ser Pro Lys Gly Thr Pro Asn Ser Arg Gly Glu Thr Ser Leu Glu Leu
                245                 250                 255

Ile Leu Ser Thr Thr Gly Tyr Pro Phe Ser Ser Pro Glu Pro Gly Ser
                260                 265                 270

Ala Gly His Ser Arg Ile Ser Thr Ser Ala Pro Leu Ser Ser Ser Ala
        275                 280                 285

Ser Val Leu Asp Asn Lys Ile Ser Glu Thr Ser Ile Phe Ser Gly Gln
        290                 295                 300

Ser Leu Thr Ser Pro Leu Ser Pro Gly Val Pro Glu Ala Arg Ala Ser
305                 310                 315                 320

Thr Met Pro Asn Ser Ala Ile Pro Phe Ser Met Thr Leu Ser Asn Ala
                325                 330                 335

Glu Thr Ser Ala Glu Arg Val Arg Ser Thr Ile Ser Ser Leu Gly Thr
        340                 345                 350

Pro Ser Ile Ser Thr Lys Gln Thr Ala Glu Thr Ile Leu Thr Phe His
        355                 360                 365

Ala Phe Ala Glu Thr Met Asp Ile Pro Ser Thr His Ile Ala Lys Thr
        370                 375                 380

Leu Ala Ser Glu Trp Leu Gly Ser Pro Gly Thr Leu Gly Gly Thr Ser
385                 390                 395                 400

Thr Ser Ala Leu Thr Thr Thr Ser Pro Ser Thr Thr Leu Val Ser Glu
        405                 410                 415

Glu Thr Asn Thr His His Ser Thr Ser Gly Lys Glu Thr Glu Gly Thr
        420                 425                 430

Leu Asn Thr Ser Met Thr Pro Leu Glu Thr Ser Ala Pro Gly Glu Glu
        435                 440                 445

Ser Glu Met Thr Ala Thr Leu Val Pro Thr Leu Gly Phe Thr Thr Leu
        450                 455                 460

Asp Ser Lys Ile Arg Ser Pro Ser Gln Val Ser Ser Ser His Pro Thr
465                 470                 475                 480

Arg Glu Leu Arg Thr Thr Gly Ser Thr Ser Gly Arg Gln Ser Ser Ser
                485                 490                 495

Thr Ala Ala His Gly Ser Ser Asp Ile Leu Arg Ala Thr Thr Ser Ser
                500                 505                 510

Thr Ser Lys Ala Ser Ser Trp Thr Ser Glu Ser Thr Ala Gln Gln Phe
        515                 520                 525

Ser Glu Pro Gln His Thr Gln Trp Val Glu Thr Ser Pro Ser Met Lys
        530                 535                 540

Thr Glu Arg Pro Pro Ala Ser Thr Ser Val Ala Ala Pro Ile Thr Thr
545                 550                 555                 560

Ser Val Pro Ser Val Val Ser Gly Phe Thr Thr Leu Lys Thr Ser Ser
                565                 570                 575

Thr Lys Gly Ile Trp Leu Glu Glu Thr Ser Ala Asp Thr Leu Ile Gly
                580                 585                 590

Glu Ser Thr Ala Gly Pro Thr Thr His Gln Phe Ala Val Pro Thr Gly
        595                 600                 605

Ile Ser Met Thr Gly Gly Ser Ser Thr Arg Gly Ser Gln Gly Thr Thr
610                     615                 620

His Leu Leu Thr Arg Ala Thr Ala Ser Ser Glu Thr Ser Ala Asp Leu
625             630             635             640

Thr Leu Ala Thr Asn Gly Val Pro Val Ser Val Ser Pro Ala Val Ser
            645             650             655

Lys Thr Ala Ala Gly Ser Ser Pro Pro Gly Gly Thr Lys Pro Ser Tyr
        660             665             670

Thr Met Val Ser Ser Val Ile Pro Glu Thr Ser Ser Leu Gln Ser Ser
        675             680             685

Ala Phe Arg Glu Gly Thr Ser Leu Gly Leu Thr Pro Leu Asn Thr Arg
    690             695             700

His Pro Phe Ser Ser Pro Glu Pro Asp Ser Ala Gly His Thr Lys Ile
705             710             715             720

Ser Thr Ser Ile Pro Leu Leu Ser Ser Ala Ser Val Leu Glu Asp Lys
            725             730             735

Val Ser Ala Thr Ser Thr Phe Ser His His Lys Ala Thr Ser Ser Ile
            740             745             750

Thr Thr Gly Thr Pro Glu Ile Ser Thr Lys Thr Lys Pro Ser Ser Ala
        755             760             765

Val Leu Ser Ser Met Thr Leu Ser Asn Ala Ala Thr Ser Pro Glu Arg
    770             775             780

Val Arg Asn Ala Thr Ser Pro Leu Thr His Pro Ser Pro Ser Gly Glu
785             790             795             800

Glu Thr Ala Gly Ser Val Leu Thr Leu Ser Thr Ser Ala Glu Thr Thr
            805             810             815

Asp Ser Pro Asn Ile His Pro Thr Gly Thr Leu Thr Ser Glu Ser Ser
            820             825             830

Glu Ser Pro Ser Thr Leu Ser Leu Pro Ser Val Ser Gly Val Lys Thr
        835             840             845

Thr Phe Ser Ser Ser Thr Pro Ser Thr His Leu Phe Thr Ser Gly Glu
    850             855             860

Glu Thr Glu Glu Thr Ser Asn Pro Ser Val Ser Gln Pro Glu Thr Ser
865                     870                 875                 880

Val Ser Arg Val Arg Thr Thr Leu Ala Ser Thr Ser Val Pro Thr Pro
                    885                 890                 895

Val Phe Pro Thr Met Asp Thr Trp Pro Thr Arg Ser Ala Gln Phe Ser
                900                 905                 910

Ser Ser His Leu Val Ser Glu Leu Arg Ala Thr Ser Ser Thr Ser Val
            915                 920                 925

Thr Asn Ser Thr Gly Ser Ala Leu Pro Lys Ile Ser His Leu Thr Gly
        930                 935                 940

Thr Ala Thr Met Ser Gln Thr Asn Arg Asp Thr Phe Asn Asp Ser Ala
945                 950                 955                 960

Ala Pro Gln Ser Thr Thr Trp Pro Glu Thr Ser Pro Arg Phe Lys Thr
                965                 970                 975

Gly Leu Pro Ser Ala Thr Thr Thr Val Ser Thr Ser Ala Thr Ser Leu
            980                 985                 990

Ser Ala Thr Val Met Val Ser Lys  Phe Thr Ser Pro Ala  Thr Ser Ser
        995                 1000                 1005

Met Glu  Ala Thr Ser Ile Arg  Glu Pro Ser Thr Thr  Ile Leu Thr
    1010                 1015                 1020

Thr Glu  Thr Thr Asn Gly Pro  Gly Ser Met Ala Val  Ala Ser Thr
    1025                 1030                 1035

Asn Ile  Pro Ile Gly Lys Gly  Tyr Ile Thr Glu Gly  Arg Leu Asp
    1040                 1045                 1050

Thr Ser  His Leu Pro Ile Gly  Thr Thr Ala Ser Ser  Glu Thr Ser
    1055                 1060                 1065

Met Asp  Phe Thr Met Ala Lys  Glu Ser Val Ser Met  Ser Val Ser
    1070                 1075                 1080

Pro Ser  Gln Ser Met Asp Ala  Ala Gly Ser Ser Thr  Pro Gly Arg
    1085                 1090                 1095

Thr Ser  Gln Phe Val Asp Thr  Phe Ser Asp Asp Val  Tyr His Leu

```
            1100                        1105                          1110


        Thr Ser  Arg Glu Ile Thr Ile  Pro Arg Asp Gly Thr  Ser Ser Ala
            1115                        1120                      1125


        Leu Thr  Pro Gln Met Thr Ala  Thr His Pro Pro Ser  Pro Asp Pro
            1130                        1135                      1140


        Gly Ser  Ala Arg Ser Thr Trp  Leu Gly Ile Leu Ser  Ser Ser Pro
            1145                        1150                      1155


        Ser Ser  Pro Thr Pro Lys Val  Thr Met Ser Ser Thr  Phe Ser Thr
            1160                        1165                      1170


        Gln Arg  Val Thr Thr Ser Met  Ile Met Asp Thr Val  Glu Thr Ser
            1175                        1180                      1185


        Arg Trp  Asn Met Pro Asn Leu  Pro Ser Thr Thr Ser  Leu Thr Pro
            1190                        1195                      1200


        Ser Asn  Ile Pro Thr Ser Gly  Ala Ile Gly Lys Ser  Thr Leu Val
            1205                        1210                      1215


        Pro Leu  Asp Thr Pro Ser Pro  Ala Thr Ser Leu Glu  Ala Ser Glu
            1220                        1225                      1230


        Gly Gly  Leu Pro Thr Leu Ser  Thr Tyr Pro Glu Ser  Thr Asn Thr
            1235                        1240                      1245


        Pro Ser  Ile His Leu Gly Ala  His Ala Ser Ser Glu  Ser Pro Ser
            1250                        1255                      1260


        Thr Ile  Lys Leu Thr Met Ala  Ser Val Val Lys Pro  Gly Ser Tyr
            1265                        1270                      1275


        Thr Pro  Leu Thr Phe Pro Ser  Ile Glu Thr His Ile  His Val Ser
            1280                        1285                      1290


        Thr Ala  Arg Met Ala Tyr Ser  Ser Gly Ser Ser Pro  Glu Met Thr
            1295                        1300                      1305


        Ala Pro  Gly Glu Thr Asn Thr  Gly Ser Thr Trp Asp  Pro Thr Thr
            1310                        1315                      1320


        Tyr Ile  Thr Thr Thr Asp Pro  Lys Asp Thr Ser Ser  Ala Gln Val
            1325                        1330                      1335
```

```
Ser Thr Pro His Ser Val Arg  Thr Leu Arg Thr Thr  Glu Asn His
    1340             1345             1350


Pro Lys Thr Glu Ser Ala Thr  Pro Ala Ala Tyr Ser  Gly Ser Pro
    1355             1360             1365


Lys Ile Ser Ser Ser Pro Asn  Leu Thr Ser Pro Ala  Thr Lys Ala
    1370             1375             1380


Trp Thr Ile Thr Asp Thr Thr  Glu His Ser Thr Gln  Leu His Tyr
    1385             1390             1395


Thr Lys Leu Ala Glu Lys Ser  Ser Gly Phe Glu Thr  Gln Ser Ala
    1400             1405             1410


Pro Gly Pro Val Ser Val Val  Ile Pro Thr Ser Pro  Thr Ile Gly
    1415             1420             1425


Ser Ser Thr Leu Glu Leu Thr  Ser Asp Val Pro Gly  Glu Pro Leu
    1430             1435             1440


Val Leu Ala Pro Ser Glu Gln  Thr Thr Ile Thr Leu  Pro Met Ala
    1445             1450             1455


Thr Trp Leu Ser Thr Ser Leu  Thr Glu Glu Met Ala  Ser Thr Asp
    1460             1465             1470


Leu Asp Ile Ser Ser Pro Ser  Ser Pro Met Ser Thr  Phe Ala Ile
    1475             1480             1485


Phe Pro Pro Met Ser Thr Pro  Ser His Glu Leu Ser  Lys Ser Glu
    1490             1495             1500


Ala Asp Thr Ser Ala Ile Arg  Asn Thr Asp Ser Thr  Thr Leu Asp
    1505             1510             1515


Gln His Leu Gly Ile Arg Ser  Leu Gly Arg Thr Gly  Asp Leu Thr
    1520             1525             1530


Thr Val Pro Ile Thr Pro Leu  Thr Thr Thr Trp Thr  Ser Val Ile
    1535             1540             1545


Glu His Ser Thr Gln Ala Gln  Asp Thr Leu Ser Ala  Thr Met Ser
    1550             1555             1560


Pro Thr His Val Thr Gln Ser  Leu Lys Asp Gln Thr  Ser Ile Pro
    1565             1570             1575
```

Ala Ser Ala Ser Pro Ser His Leu Thr Glu Val Tyr Pro Glu Leu
1580 1585 1590

Gly Thr Gln Gly Arg Ser Ser Ser Glu Ala Thr Thr Phe Trp Lys
1595 1600 1605

Pro Ser Thr Asp Thr Leu Ser Arg Glu Ile Glu Thr Gly Pro Thr
1610 1615 1620

Asn Ile Gln Ser Thr Pro Pro Met Asp Asn Thr Thr Thr Gly Ser
1625 1630 1635

Ser Ser Ser Gly Val Thr Leu Gly Ile Ala His Leu Pro Ile Gly
1640 1645 1650

Thr Ser Ser Pro Ala Glu Thr Ser Thr Asn Met Ala Leu Glu Arg
1655 1660 1665

Arg Ser Ser Thr Ala Thr Val Ser Met Ala Gly Thr Met Gly Leu
1670 1675 1680

Leu Val Thr Ser Ala Pro Gly Arg Ser Ile Ser Gln Ser Leu Gly
1685 1690 1695

Arg Val Ser Ser Val Leu Ser Glu Ser Thr Thr Glu Gly Val Thr
1700 1705 1710

Asp Ser Ser Lys Gly Ser Ser Pro Arg Leu Asn Thr Gln Gly Asn
1715 1720 1725

Thr Ala Leu Ser Ser Ser Leu Glu Pro Ser Tyr Ala Glu Gly Ser
1730 1735 1740

Gln Met Ser Thr Ser Ile Pro Leu Thr Ser Ser Pro Thr Thr Pro
1745 1750 1755

Asp Val Glu Phe Ile Gly Gly Ser Thr Phe Trp Thr Lys Glu Val
1760 1765 1770

Thr Thr Val Met Thr Ser Asp Ile Ser Lys Ser Ser Ala Arg Thr
1775 1780 1785

Glu Ser Ser Ser Ala Thr Leu Met Ser Thr Ala Leu Gly Ser Thr
1790 1795 1800

Glu Asn Thr Gly Lys Glu Lys Leu Arg Thr Ala Ser Met Asp Leu
1805 1810 1815

```
Pro Ser Pro Thr Pro Ser Met  Glu Val Thr Pro Trp  Ile Ser Leu
    1820            1825          1830

Thr Leu Ser Asn Ala Pro Asn  Thr Thr Asp Ser Leu  Asp Leu Ser
    1835            1840          1845

His Gly Val His Thr Ser Ser  Ala Gly Thr Leu Ala  Thr Asp Arg
    1850            1855          1860

Ser Leu Asn Thr Gly Val Thr  Arg Ala Ser Arg Leu  Glu Asn Gly
    1865            1870          1875

Ser Asp Thr Ser Ser Lys Ser  Leu Ser Met Gly Asn  Ser Thr His
    1880            1885          1890

Thr Ser Met Thr Asp Thr Glu  Lys Ser Glu Val Ser  Ser Ser Ile
    1895            1900          1905

His Pro Arg Pro Glu Thr Ser  Ala Pro Gly Ala Glu  Thr Thr Leu
    1910            1915          1920

Thr Ser Thr Pro Gly Asn Arg  Ala Ile Ser Leu Thr  Leu Pro Phe
    1925            1930          1935

Ser Ser Ile Pro Val Glu Glu  Val Ile Ser Thr Gly  Ile Thr Ser
    1940            1945          1950

Gly Pro Asp Ile Asn Ser Ala  Pro Met Thr His Ser  Pro Ile Thr
    1955            1960          1965

Pro Pro Thr Ile Val Trp Thr  Ser Thr Gly Thr Ile  Glu Gln Ser
    1970            1975          1980

Thr Gln Pro Leu His Ala Val  Ser Ser Glu Lys Val  Ser Val Gln
    1985            1990          1995

Thr Gln Ser Thr Pro Tyr Val  Asn Ser Val Ala Val  Ser Ala Ser
    2000            2005          2010

Pro Thr His Glu Asn Ser Val  Ser Ser Gly Ser Ser  Thr Ser Ser
    2015            2020          2025

Pro Tyr Ser Ser Ala Ser Leu  Glu Ser Leu Asp Ser  Thr Ile Ser
    2030            2035          2040

Arg Arg Asn Ala Ile Thr Ser  Trp Leu Trp Asp Leu  Thr Thr Ser
```

```
        2045                    2050                    2055


Leu Pro Thr Thr Thr Trp Pro  Ser Thr Ser Leu Ser  Glu Ala Leu
    2060                2065                2070


Ser Ser Gly His Ser Gly Val  Ser Asn Pro Ser Ser  Thr Thr Thr
    2075                2080                2085


Glu Phe Pro Leu Phe Ser Ala  Ala Ser Thr Ser Ala  Ala Lys Gln
    2090                2095                2100


Arg Asn Pro Glu Thr Glu Thr  His Gly Pro Gln Asn  Thr Ala Ala
    2105                2110                2115


Ser Thr Leu Asn Thr Asp Ala  Ser Ser Val Thr Gly  Leu Ser Glu
    2120                2125                2130


Thr Pro Val Gly Ala Ser Ile  Ser Ser Glu Val Pro  Leu Pro Met
    2135                2140                2145


Ala Ile Thr Ser Arg Ser Asp  Val Ser Gly Leu Thr  Ser Glu Ser
    2150                2155                2160


Thr Ala Asn Pro Ser Leu Gly  Thr Ala Ser Ser Ala  Gly Thr Lys
    2165                2170                2175


Leu Thr Arg Thr Ile Ser Leu  Pro Thr Ser Glu Ser  Leu Val Ser
    2180                2185                2190


Phe Arg Met Asn Lys Asp Pro  Trp Thr Val Ser Ile  Pro Leu Gly
    2195                2200                2205


Ser His Pro Thr Thr Asn Thr  Glu Thr Ser Ile Pro  Val Asn Ser
    2210                2215                2220


Ala Gly Pro Pro Gly Leu Ser  Thr Val Ala Ser Asp  Val Ile Asp
    2225                2230                2235


Thr Pro Ser Asp Gly Ala Glu  Ser Ile Pro Thr Val  Ser Phe Ser
    2240                2245                2250


Pro Ser Pro Asp Thr Glu Val  Thr Thr Ile Ser His  Phe Pro Glu
    2255                2260                2265


Lys Thr Thr His Ser Phe Arg  Thr Ile Ser Ser Leu  Thr His Glu
    2270                2275                2280
```

397

```
Leu Thr   Ser Arg Val Thr Pro   Ile Pro Gly Asp Trp   Met Ser Ser
    2285              2290               2295

Ala Met   Ser Thr Lys Pro Thr   Gly Ala Ser Pro Ser   Ile Thr Leu
    2300              2305               2310

Gly Glu   Arg Arg Thr Ile Thr   Ser Ala Ala Pro Thr   Thr Ser Pro
    2315              2320               2325

Ile Val   Leu Thr Ala Ser Phe   Thr Glu Thr Ser Thr   Val Ser Leu
    2330              2335               2340

Asp Asn   Glu Thr Thr Val Lys   Thr Ser Asp Ile Leu   Asp Ala Arg
    2345              2350               2355

Lys Thr   Asn Glu Leu Pro Ser   Asp Ser Ser Ser Ser   Ser Asp Leu
    2360              2365               2370

Ile Asn   Thr Ser Ile Ala Ser   Ser Thr Met Asp Val   Thr Lys Thr
    2375              2380               2385

Ala Ser   Ile Ser Pro Thr Ser   Ile Ser Gly Met Thr   Ala Ser Ser
    2390              2395               2400

Ser Pro   Ser Leu Phe Ser Ser   Asp Arg Pro Gln Val   Pro Thr Ser
    2405              2410               2415

Thr Thr   Glu Thr Asn Thr Ala   Thr Ser Pro Ser Val   Ser Ser Asn
    2420              2425               2430

Thr Tyr   Ser Leu Asp Gly Gly   Ser Asn Val Gly Gly   Thr Pro Ser
    2435              2440               2445

Thr Leu   Pro Pro Phe Thr Ile   Thr His Pro Val Glu   Thr Ser Ser
    2450              2455               2460

Ala Leu   Leu Ala Trp Ser Arg   Pro Val Arg Thr Phe   Ser Thr Met
    2465              2470               2475

Val Ser   Thr Asp Thr Ala Ser   Gly Glu Asn Pro Thr   Ser Ser Asn
    2480              2485               2490

Ser Val   Val Thr Ser Val Pro   Ala Pro Gly Thr Trp   Ala Ser Val
    2495              2500               2505

Gly Ser   Thr Thr Asp Leu Pro   Ala Met Gly Phe Leu   Lys Thr Ser
    2510              2515               2520
```

```
Pro Ala Gly Glu Ala His Ser  Leu Leu Ala Ser Thr  Ile Glu Pro
    2525                2530                2535

Ala Thr Ala Phe Thr Pro His  Leu Ser Ala Ala Val  Val Thr Gly
    2540                2545                2550

Ser Ser Ala Thr Ser Glu Ala  Ser Leu Leu Thr Thr  Ser Glu Ser
    2555                2560                2565

Lys Ala Ile His Ser Ser Pro  Gln Thr Pro Thr Thr  Pro Thr Ser
    2570                2575                2580

Gly Ala Asn Trp Glu Thr Ser  Ala Thr Pro Glu Ser  Leu Leu Val
    2585                2590                2595

Val Thr Glu Thr Ser Asp Thr  Thr Leu Thr Ser Lys  Ile Leu Val
    2600                2605                2610

Thr Asp Thr Ile Leu Phe Ser  Thr Val Ser Thr Pro  Pro Ser Lys
    2615                2620                2625

Phe Pro Ser Thr Gly Thr Leu  Ser Gly Ala Ser Phe  Pro Thr Leu
    2630                2635                2640

Leu Pro Asp Thr Pro Ala Ile  Pro Leu Thr Ala Thr  Glu Pro Thr
    2645                2650                2655

Ser Ser Leu Ala Thr Ser Phe  Asp Ser Thr Pro Leu  Val Thr Ile
    2660                2665                2670

Ala Ser Asp Ser Leu Gly Thr  Val Pro Glu Thr Thr  Leu Thr Met
    2675                2680                2685

Ser Glu Thr Ser Asn Gly Asp  Ala Leu Val Leu Lys  Thr Val Ser
    2690                2695                2700

Asn Pro Asp Arg Ser Ile Pro  Gly Ile Thr Ile Gln  Gly Val Thr
    2705                2710                2715

Glu Ser Pro Leu His Pro Ser  Ser Thr Ser Pro Ser  Lys Ile Val
    2720                2725                2730

Ala Pro Arg Asn Thr Thr Tyr  Glu Gly Ser Ile Thr  Val Ala Leu
    2735                2740                2745

Ser Thr Leu Pro Ala Gly Thr  Thr Gly Ser Leu Val  Phe Ser Gln
    2750                2755                2760
```

399

```
Ser Ser  Glu Asn Ser Glu Thr  Thr Ala Leu Val Asp  Ser Ser Ala
    2765             2770             2775

Gly Leu  Glu Arg Ala Ser Val  Met Pro Leu Thr Thr  Gly Ser Gln
    2780             2785             2790

Gly Met  Ala Ser Ser Gly Gly  Ile Arg Ser Gly Ser  Thr His Ser
    2795             2800             2805

Thr Gly  Thr Lys Thr Phe Ser  Ser Leu Pro Leu Thr  Met Asn Pro
    2810             2815             2820

Gly Glu  Val Thr Ala Met Ser  Glu Ile Thr Thr Asn  Arg Leu Thr
    2825             2830             2835

Ala Thr  Gln Ser Thr Ala Pro  Lys Gly Ile Pro Val  Lys Pro Thr
    2840             2845             2850

Ser Ala  Glu Ser Gly Leu Leu  Thr Pro Val Ser Ala  Ser Ser Ser
    2855             2860             2865

Pro Ser  Lys Ala Phe Ala Ser  Leu Thr Thr Ala Pro  Pro Ser Thr
    2870             2875             2880

Trp Gly  Ile Pro Gln Ser Thr  Leu Thr Phe Glu Phe  Ser Glu Val
    2885             2890             2895

Pro Ser  Leu Asp Thr Lys Ser  Ala Ser Leu Pro Thr  Pro Gly Gln
    2900             2905             2910

Ser Leu  Asn Thr Ile Pro Asp  Ser Asp Ala Ser Thr  Ala Ser Ser
    2915             2920             2925

Ser Leu  Ser Lys Ser Pro Glu  Lys Asn Pro Arg Ala  Arg Met Met
    2930             2935             2940

Thr Ser  Thr Lys Ala Ile Ser  Ala Ser Ser Phe Gln  Ser Thr Gly
    2945             2950             2955

Phe Thr  Glu Thr Pro Glu Gly  Ser Ala Ser Pro Ser  Met Ala Gly
    2960             2965             2970

His Glu  Pro Arg Val Pro Thr  Ser Gly Thr Gly Asp  Pro Arg Tyr
    2975             2980             2985

Ala Ser  Glu Ser Met Ser Tyr  Pro Asp Pro Ser Lys  Ala Ser Ser
```

```
            2990                      2995                      3000


     Ala Met   Thr Ser Thr Ser Leu   Ala Ser Lys Leu Thr   Thr Leu Phe
         3005                  3010                  3015


     Ser Thr   Gly Gln Ala Ala Arg   Ser Gly Ser Ser Ser   Ser Pro Ile
         3020                  3025                  3030


     Ser Leu   Ser Thr Glu Lys Glu   Thr Ser Phe Leu Ser   Pro Thr Ala
         3035                  3040                  3045


     Ser Thr   Ser Arg Lys Thr Ser   Leu Phe Leu Gly Pro   Ser Met Ala
         3050                  3055                  3060


     Arg Gln   Pro Asn Ile Leu Val   His Leu Gln Thr Ser   Ala Leu Thr
         3065                  3070                  3075


     Leu Ser   Pro Thr Ser Thr Leu   Asn Met Ser Gln Glu   Glu Pro Pro
         3080                  3085                  3090


     Glu Leu   Thr Ser Ser Gln Thr   Ile Ala Glu Glu Glu   Gly Thr Thr
         3095                  3100                  3105


     Ala Glu   Thr Gln Thr Leu Thr   Phe Thr Pro Ser Glu   Thr Pro Thr
         3110                  3115                  3120


     Ser Leu   Leu Pro Val Ser Ser   Pro Thr Glu Pro Thr   Ala Arg Arg
         3125                  3130                  3135


     Lys Ser   Ser Pro Glu Thr Trp   Ala Ser Ser Ile Ser   Val Pro Ala
         3140                  3145                  3150


     Lys Thr   Ser Leu Val Glu Thr   Thr Asp Gly Thr Leu   Val Thr Thr
         3155                  3160                  3165


     Ile Lys   Met Ser Ser Gln Ala   Ala Gln Gly Asn Ser   Thr Trp Pro
         3170                  3175                  3180


     Ala Pro   Ala Glu Glu Thr Gly   Thr Ser Pro Ala Gly   Thr Ser Pro
         3185                  3190                  3195


     Gly Ser   Pro Glu Val Ser Thr   Thr Leu Lys Ile Met   Ser Ser Lys
         3200                  3205                  3210


     Glu Pro   Ser Ile Ser Pro Glu   Ile Arg Ser Thr Val   Arg Asn Ser
         3215                  3220                  3225
```

401

```
Pro Trp Lys Thr Pro Glu Thr   Thr Val Pro Met Glu   Thr Thr Val
    3230                3235                3240

Glu Pro Val Thr Leu Gln Ser   Thr Ala Leu Gly Ser   Gly Ser Thr
    3245                3250                3255

Ser Ile Ser His Leu Pro Thr   Gly Thr Thr Ser Pro   Thr Lys Ser
    3260                3265                3270

Pro Thr Glu Asn Met Leu Ala   Thr Glu Arg Val Ser   Leu Ser Pro
    3275                3280                3285

Ser Pro Pro Glu Ala Trp Thr   Asn Leu Tyr Ser Gly   Thr Pro Gly
    3290                3295                3300

Gly Thr Arg Gln Ser Leu Ala   Thr Met Ser Ser Val   Ser Leu Glu
    3305                3310                3315

Ser Pro Thr Ala Arg Ser Ile   Thr Gly Thr Gly Gln   Gln Ser Ser
    3320                3325                3330

Pro Glu Leu Val Ser Lys Thr   Thr Gly Met Glu Phe   Ser Met Trp
    3335                3340                3345

His Gly Ser Thr Gly Gly Thr   Thr Gly Asp Thr His   Val Ser Leu
    3350                3355                3360

Ser Thr Ser Ser Asn Ile Leu   Glu Asp Pro Val Thr   Ser Pro Asn
    3365                3370                3375

Ser Val Ser Ser Leu Thr Asp   Lys Ser Lys His Lys   Thr Glu Thr
    3380                3385                3390

Trp Val Ser Thr Thr Ala Ile   Pro Ser Thr Val Leu   Asn Asn Lys
    3395                3400                3405

Ile Met Ala Ala Glu Gln Gln   Thr Ser Arg Ser Val   Asp Glu Ala
    3410                3415                3420

Tyr Ser Ser Thr Ser Ser Trp   Ser Asp Gln Thr Ser   Gly Ser Asp
    3425                3430                3435

Ile Thr Leu Gly Ala Ser Pro   Asp Val Thr Asn Thr   Leu Tyr Ile
    3440                3445                3450

Thr Ser Thr Ala Gln Thr Thr   Ser Leu Val Ser Leu   Pro Ser Gly
    3455                3460                3465
```

Asp Gln Gly Ile Thr Ser Leu Thr Asn Pro Ser Gly Gly Lys Thr
    3470            3475            3480

Ser Ser Ala Ser Ser Val Thr Ser Pro Ser Ile Gly Leu Glu Thr
    3485            3490            3495

Leu Arg Ala Asn Val Ser Ala Val Lys Ser Asp Ile Ala Pro Thr
    3500            3505            3510

Ala Gly His Leu Ser Gln Thr Ser Ser Pro Ala Glu Val Ser Ile
    3515            3520            3525

Leu Asp Val Thr Thr Ala Pro Thr Pro Gly Ile Ser Thr Thr Ile
    3530            3535            3540

Thr Thr Met Gly Thr Asn Ser Ile Ser Thr Thr Thr Pro Asn Pro
    3545            3550            3555

Glu Val Gly Met Ser Thr Met Asp Ser Thr Pro Ala Thr Glu Arg
    3560            3565            3570

Arg Thr Thr Ser Thr Glu His Pro Ser Thr Trp Ser Ser Thr Ala
    3575            3580            3585

Ala Ser Asp Ser Trp Thr Val Thr Asp Met Thr Ser Asn Leu Lys
    3590            3595            3600

Val Ala Arg Ser Pro Gly Thr Ile Ser Thr Met His Thr Thr Ser
    3605            3610            3615

Phe Leu Ala Ser Ser Thr Glu Leu Asp Ser Met Ser Thr Pro His
    3620            3625            3630

Gly Arg Ile Thr Val Ile Gly Thr Ser Leu Val Thr Pro Ser Ser
    3635            3640            3645

Asp Ala Ser Ala Val Lys Thr Glu Thr Ser Thr Ser Glu Arg Thr
    3650            3655            3660

Leu Ser Pro Ser Asp Thr Thr Ala Ser Thr Pro Ile Ser Thr Phe
    3665            3670            3675

Ser Arg Val Gln Arg Met Ser Ile Ser Val Pro Asp Ile Leu Ser
    3680            3685            3690

Thr Ser Trp Thr Pro Ser Ser Thr Glu Ala Glu Asp Val Pro Val
    3695            3700            3705

403

Ser Met Val Ser Thr Asp His Ala Ser Thr Lys Thr Asp Pro Asn
    3710              3715              3720

Thr Pro Leu Ser Thr Phe Leu Phe Asp Ser Leu Ser Thr Leu Asp
    3725              3730              3735

Trp Asp Thr Gly Arg Ser Leu Ser Ser Ala Thr Ala Thr Thr Ser
    3740              3745              3750

Ala Pro Gln Gly Ala Thr Thr Pro Gln Glu Leu Thr Leu Glu Thr
    3755              3760              3765

Met Ile Ser Pro Ala Thr Ser Gln Leu Pro Phe Ser Ile Gly His
    3770              3775              3780

Ile Thr Ser Ala Val Thr Pro Ala Ala Met Ala Arg Ser Ser Gly
    3785              3790              3795

Val Thr Phe Ser Arg Pro Asp Pro Thr Ser Lys Lys Ala Glu Gln
    3800              3805              3810

Thr Ser Thr Gln Leu Pro Thr Thr Thr Ser Ala His Pro Gly Gln
    3815              3820              3825

Val Pro Arg Ser Ala Ala Thr Thr Leu Asp Val Ile Pro His Thr
    3830              3835              3840

Ala Lys Thr Pro Asp Ala Thr Phe Gln Arg Gln Gly Gln Thr Ala
    3845              3850              3855

Leu Thr Thr Glu Ala Arg Ala Thr Ser Asp Ser Trp Asn Glu Lys
    3860              3865              3870

Glu Lys Ser Thr Pro Ser Ala Pro Trp Ile Thr Glu Met Met Asn
    3875              3880              3885

Ser Val Ser Glu Asp Thr Ile Lys Glu Val Thr Ser Ser Ser Ser
    3890              3895              3900

Val Leu Lys Asp Pro Glu Tyr Ala Gly His Lys Leu Gly Ile Trp
    3905              3910              3915

Asp Asp Phe Ile Pro Lys Phe Gly Lys Ala Ala His Met Arg Glu
    3920              3925              3930

Leu Pro Leu Leu Ser Pro Pro Gln Asp Lys Glu Ala Ile His Pro

404

```
                3935                    3940                    3945


        Ser Thr Asn Thr Val Glu Thr   Thr Gly Trp Val Thr   Ser Ser Glu
            3950                    3955                    3960


        His Ala Ser His Ser Thr Ile   Pro Ala His Ser Ala   Ser Ser Lys
            3965                    3970                    3975


        Leu Thr Ser Pro Val Val Thr   Thr Ser Thr Arg Glu   Gln Ala Ile
            3980                    3985                    3990


        Val Ser Met Ser Thr Thr Thr   Trp Pro Glu Ser Thr   Arg Ala Arg
            3995                    4000                    4005


        Thr Glu Pro Asn Ser Phe Leu   Thr Ile Glu Leu Arg   Asp Val Ser
            4010                    4015                    4020


        Pro Tyr Met Asp Thr Ser Ser   Thr Thr Gln Thr Ser   Ile Ile Ser
            4025                    4030                    4035


        Ser Pro Gly Ser Thr Ala Ile   Thr Lys Gly Pro Arg   Thr Glu Ile
            4040                    4045                    4050


        Thr Ser Ser Lys Arg Ile Ser   Ser Ser Phe Leu Ala   Gln Ser Met
            4055                    4060                    4065


        Arg Ser Ser Asp Ser Pro Ser   Glu Ala Ile Thr Arg   Leu Ser Asn
            4070                    4075                    4080


        Phe Pro Ala Met Thr Glu Ser   Gly Gly Met Ile Leu   Ala Met Gln
            4085                    4090                    4095


        Thr Ser Pro Pro Gly Ala Thr   Ser Leu Ser Ala Pro   Thr Leu Asp
            4100                    4105                    4110


        Thr Ser Ala Thr Ala Ser Trp   Thr Gly Thr Pro Leu   Ala Thr Thr
            4115                    4120                    4125


        Gln Arg Phe Thr Tyr Ser Glu   Lys Thr Thr Leu Phe   Ser Lys Gly
            4130                    4135                    4140


        Pro Glu Asp Thr Ser Gln Pro   Ser Pro Pro Ser Val   Glu Glu Thr
            4145                    4150                    4155


        Ser Ser Ser Ser Ser Leu Val   Pro Ile His Ala Thr   Thr Ser Pro
            4160                    4165                    4170
```

```
Ser Asn  Ile Leu Leu Thr Ser  Gln Gly His Ser Pro  Ser Ser Thr
    4175             4180             4185

Pro Pro  Val Thr Ser Val Phe  Leu Ser Glu Thr Ser  Gly Leu Gly
    4190             4195             4200

Lys Thr  Thr Asp Met Ser Arg  Ile Ser Leu Glu Pro  Gly Thr Ser
    4205             4210             4215

Leu Pro  Pro Asn Leu Ser Ser  Thr Ala Gly Glu Ala  Leu Ser Thr
    4220             4225             4230

Tyr Glu  Ala Ser Arg Asp Thr  Lys Ala Ile His His  Ser Ala Asp
    4235             4240             4245

Thr Ala  Val Thr Asn Met Glu  Ala Thr Ser Ser Glu  Tyr Ser Pro
    4250             4255             4260

Ile Pro  Gly His Thr Lys Pro  Ser Lys Ala Thr Ser  Pro Leu Val
    4265             4270             4275

Thr Ser  His Ile Met Gly Asp  Ile Thr Ser Ser Thr  Ser Val Phe
    4280             4285             4290

Gly Ser  Ser Glu Thr Thr Glu  Ile Glu Thr Val Ser  Ser Val Asn
    4295             4300             4305

Gln Gly  Leu Gln Glu Arg Ser  Thr Ser Gln Val Ala  Ser Ser Ala
    4310             4315             4320

Thr Glu  Thr Ser Thr Val Ile  Thr His Val Ser Ser  Gly Asp Ala
    4325             4330             4335

Thr Thr  His Val Thr Lys Thr  Gln Ala Thr Phe Ser  Ser Gly Thr
    4340             4345             4350

Ser Ile  Ser Ser Pro His Gln  Phe Ile Thr Ser Thr  Asn Thr Phe
    4355             4360             4365

Thr Asp  Val Ser Thr Asn Pro  Ser Thr Ser Leu Ile  Met Thr Glu
    4370             4375             4380

Ser Ser  Gly Val Thr Ile Thr  Thr Gln Thr Gly Pro  Thr Gly Ala
    4385             4390             4395

Ala Thr  Gln Gly Pro Tyr Leu  Leu Asp Thr Ser Thr  Met Pro Tyr
    4400             4405             4410
```

```
Leu Thr Glu Thr Pro Leu Ala  Val Thr Pro Asp Phe  Met Gln Ser
    4415              4420             4425

Glu Lys Thr Thr Leu Ile Ser  Lys Gly Pro Lys Asp  Val Thr Trp
    4430              4435             4440

Thr Ser Pro Pro Ser Val Ala  Glu Thr Ser Tyr Pro  Ser Ser Leu
    4445              4450             4455

Thr Pro Phe Leu Val Thr Thr  Ile Pro Pro Ala Thr  Ser Thr Leu
    4460              4465             4470

Gln Gly Gln His Thr Ser Ser  Pro Val Ser Ala Thr  Ser Val Leu
    4475              4480             4485

Thr Ser Gly Leu Val Lys Thr  Thr Asp Met Leu Asn  Thr Ser Met
    4490              4495             4500

Glu Pro Val Thr Asn Ser Pro  Gln Asn Leu Asn Asn  Pro Ser Asn
    4505              4510             4515

Glu Ile Leu Ala Thr Leu Ala  Ala Thr Thr Asp Ile  Glu Thr Ile
    4520              4525             4530

His Pro Ser Ile Asn Lys Ala  Val Thr Asn Met Gly  Thr Ala Ser
    4535              4540             4545

Ser Ala His Val Leu His Ser  Thr Leu Pro Val Ser  Ser Glu Pro
    4550              4555             4560

Ser Thr Ala Thr Ser Pro Met  Val Pro Ala Ser Ser  Met Gly Asp
    4565              4570             4575

Ala Leu Ala Ser Ile Ser Ile  Pro Gly Ser Glu Thr  Thr Asp Ile
    4580              4585             4590

Glu Gly Glu Pro Thr Ser Ser  Leu Thr Ala Gly Arg  Lys Glu Asn
    4595              4600             4605

Ser Thr Leu Gln Glu Met Asn  Ser Thr Thr Glu Ser  Asn Ile Ile
    4610              4615             4620

Leu Ser Asn Val Ser Val Gly  Ala Ile Thr Glu Ala  Thr Lys Met
    4625              4630             4635

Glu Val Pro Ser Phe Asp Ala  Thr Phe Ile Pro Thr  Pro Ala Gln
    4640              4645             4650
```

407

Ser Thr Lys Phe Pro Asp Ile   Phe Ser Val Ala Ser   Ser Arg Leu
    4655            4660               4665

Ser Asn Ser Pro Pro Met Thr   Ile Ser Thr His Met   Thr Thr Thr
    4670            4675               4680

Gln Thr Gly Ser Ser Gly Ala   Thr Ser Lys Ile Pro   Leu Ala Leu
    4685            4690               4695

Asp Thr Ser Thr Leu Glu Thr   Ser Ala Gly Thr Pro   Ser Val Val
    4700            4705               4710

Thr Glu Gly Phe Ala His Ser   Lys Ile Thr Thr Ala   Met Asn Asn
    4715            4720               4725

Asp Val Lys Asp Val Ser Gln   Thr Asn Pro Pro Phe   Gln Asp Glu
    4730            4735               4740

Ala Ser Ser Pro Ser Ser Gln   Ala Pro Val Leu Val   Thr Thr Leu
    4745            4750               4755

Pro Ser Ser Val Ala Phe Thr   Pro Gln Trp His Ser   Thr Ser Ser
    4760            4765               4770

Pro Val Ser Met Ser Ser Val   Leu Thr Ser Ser Leu   Val Lys Thr
    4775            4780               4785

Ala Gly Lys Val Asp Thr Ser   Leu Glu Thr Val Thr   Ser Ser Pro
    4790            4795               4800

Gln Ser Met Ser Asn Thr Leu   Asp Asp Ile Ser Val   Thr Ser Ala
    4805            4810               4815

Ala Thr Thr Asp Ile Glu Thr   Thr His Pro Ser Ile   Asn Thr Val
    4820            4825               4830

Val Thr Asn Val Gly Thr Thr   Gly Ser Ala Phe Glu   Ser His Ser
    4835            4840               4845

Thr Val Ser Ala Tyr Pro Glu   Pro Ser Lys Val Thr   Ser Pro Asn
    4850            4855               4860

Val Thr Thr Ser Thr Met Glu   Asp Thr Thr Ile Ser   Arg Ser Ile
    4865            4870               4875

Pro Lys Ser Ser Lys Thr Thr   Arg Thr Glu Thr Glu   Thr Thr Ser

```
                    4880                          4885                              4890


          Ser Leu   Thr Pro Lys Leu Arg   Glu Thr Ser Ile Ser   Gln Glu Ile
              4895                          4900                      4905


          Thr Ser   Ser Thr Glu Thr Ser   Thr Val Pro Tyr Lys   Glu Leu Thr
              4910                          4915                      4920


          Gly Ala   Thr Thr Glu Val Ser   Arg Thr Asp Val Thr   Ser Ser Ser
              4925                          4930                      4935


          Ser Thr   Ser Phe Pro Gly Pro   Asp Gln Ser Thr Val   Ser Leu Asp
              4940                          4945                      4950


          Ile Ser   Thr Glu Thr Asn Thr   Arg Leu Ser Thr Ser   Pro Ile Met
              4955                          4960                      4965


          Thr Glu   Ser Ala Glu Ile Thr   Ile Thr Thr Gln Thr   Gly Pro His
              4970                          4975                      4980


          Gly Ala   Thr Ser Gln Asp Thr   Phe Thr Met Asp Pro   Ser Asn Thr
              4985                          4990                      4995


          Thr Pro   Gln Ala Gly Ile His   Ser Ala Met Thr His   Gly Phe Ser
              5000                          5005                      5010


          Gln Leu   Asp Val Thr Thr Leu   Met Ser Arg Ile Pro   Gln Asp Val
              5015                          5020                      5025


          Ser Trp   Thr Ser Pro Pro Ser   Val Asp Lys Thr Ser   Ser Pro Ser
              5030                          5035                      5040


          Ser Phe   Leu Ser Ser Pro Ala   Met Thr Thr Pro Ser   Leu Ile Ser
              5045                          5050                      5055


          Ser Thr   Leu Pro Glu Asp Lys   Leu Ser Ser Pro Met   Thr Ser Leu
              5060                          5065                      5070


          Leu Thr   Ser Gly Leu Val Lys   Ile Thr Asp Ile Leu   Arg Thr Arg
              5075                          5080                      5085


          Leu Glu   Pro Val Thr Ser Ser   Leu Pro Asn Phe Ser   Ser Thr Ser
              5090                          5095                      5100


          Asp Lys   Ile Leu Ala Thr Ser   Lys Asp Ser Lys Asp   Thr Lys Glu
              5105                          5110                      5115
```

```
Ile Phe Pro Ser Ile Asn Thr   Glu Glu Thr Asn Val   Lys Ala Asn
    5120                5125                5130

Asn Ser Gly His Glu Ser His   Ser Pro Ala Leu Ala   Asp Ser Glu
    5135                5140                5145

Thr Pro Lys Ala Thr Thr Gln   Met Val Ile Thr Thr   Thr Val Gly
    5150                5155                5160

Asp Pro Ala Pro Ser Thr Ser   Met Pro Val His Gly   Ser Ser Glu
    5165                5170                5175

Thr Thr Asn Ile Lys Arg Glu   Pro Thr Tyr Phe Leu   Thr Pro Arg
    5180                5185                5190

Leu Arg Glu Thr Ser Thr Ser   Gln Glu Ser Ser Phe   Pro Thr Asp
    5195                5200                5205

Thr Ser Phe Leu Leu Ser Lys   Val Pro Thr Gly Thr   Ile Thr Glu
    5210                5215                5220

Val Ser Ser Thr Gly Val Asn   Ser Ser Ser Lys Ile   Ser Thr Pro
    5225                5230                5235

Asp His Asp Lys Ser Thr Val   Pro Pro Asp Thr Phe   Thr Gly Glu
    5240                5245                5250

Ile Pro Arg Val Phe Thr Ser   Ser Ile Lys Thr Lys   Ser Ala Glu
    5255                5260                5265

Met Thr Ile Thr Thr Gln Ala   Ser Pro Pro Glu Ser   Ala Ser His
    5270                5275                5280

Ser Thr Leu Pro Leu Asp Thr   Ser Thr Thr Leu Ser   Gln Gly Gly
    5285                5290                5295

Thr His Ser Thr Val Thr Gln   Gly Phe Pro Tyr Ser   Glu Val Thr
    5300                5305                5310

Thr Leu Met Gly Met Gly Pro   Gly Asn Val Ser Trp   Met Thr Thr
    5315                5320                5325

Pro Pro Val Glu Glu Thr Ser   Ser Val Ser Ser Leu   Met Ser Ser
    5330                5335                5340

Pro Ala Met Thr Ser Pro Ser   Pro Val Ser Ser Thr   Ser Pro Gln
    5345                5350                5355
```

410

Ser Ile Pro Ser Ser Pro Leu Pro Val Thr Ala Leu Pro Thr Ser
5360 5365 5370

Val Leu Val Thr Thr Thr Asp Val Leu Gly Thr Thr Ser Pro Glu
5375 5380 5385

Ser Val Thr Ser Ser Pro Pro Asn Leu Ser Ser Ile Thr His Glu
5390 5395 5400

Arg Pro Ala Thr Tyr Lys Asp Thr Ala His Thr Glu Ala Ala Met
5405 5410 5415

His His Ser Thr Asn Thr Ala Val Thr Asn Val Gly Thr Ser Gly
5420 5425 5430

Ser Gly His Lys Ser Gln Ser Ser Val Leu Ala Asp Ser Glu Thr
5435 5440 5445

Ser Lys Ala Thr Pro Leu Met Ser Thr Thr Ser Thr Leu Gly Asp
5450 5455 5460

Thr Ser Val Ser Thr Ser Thr Pro Asn Ile Ser Gln Thr Asn Gln
5465 5470 5475

Ile Gln Thr Glu Pro Thr Ala Ser Leu Ser Pro Arg Leu Arg Glu
5480 5485 5490

Ser Ser Thr Ser Glu Lys Thr Ser Ser Thr Thr Glu Thr Asn Thr
5495 5500 5505

Ala Phe Ser Tyr Val Pro Thr Gly Ala Ile Thr Gln Ala Ser Arg
5510 5515 5520

Thr Glu Ile Ser Ser Ser Arg Thr Ser Ile Ser Asp Leu Asp Arg
5525 5530 5535

Pro Thr Ile Ala Pro Asp Ile Ser Thr Gly Met Ile Thr Arg Leu
5540 5545 5550

Phe Thr Ser Pro Ile Met Thr Lys Ser Ala Glu Met Thr Val Thr
5555 5560 5565

Thr Gln Thr Thr Thr Pro Gly Ala Thr Ser Gln Gly Ile Leu Pro
5570 5575 5580

Trp Asp Thr Ser Thr Thr Leu Phe Gln Gly Gly Thr His Ser Thr
5585 5590 5595

411

Val Ser Gln Gly Phe Pro His Ser Glu Ile Thr Thr Leu Arg Ser
5600 5605 5610

Arg Thr Pro Gly Asp Val Ser Trp Met Thr Thr Pro Pro Val Glu
5615 5620 5625

Glu Thr Ser Ser Gly Phe Ser Leu Met Ser Pro Ser Met Thr Ser
5630 5635 5640

Pro Ser Pro Val Ser Ser Thr Ser Pro Glu Ser Ile Pro Ser Ser
5645 5650 5655

Pro Leu Pro Val Thr Ala Leu Leu Thr Ser Val Leu Val Thr Thr
5660 5665 5670

Thr Asn Val Leu Gly Thr Thr Ser Pro Glu Thr Val Thr Ser Ser
5675 5680 5685

Pro Pro Asn Leu Ser Ser Pro Thr Gln Glu Arg Leu Thr Thr Tyr
5690 5695 5700

Lys Asp Thr Ala His Thr Glu Ala Met His Ala Ser Met His Thr
5705 5710 5715

Asn Thr Ala Val Ala Asn Val Gly Thr Ser Ile Ser Gly His Glu
5720 5725 5730

Ser Gln Ser Ser Val Pro Ala Asp Ser His Thr Ser Lys Ala Thr
5735 5740 5745

Ser Pro Met Gly Ile Thr Phe Ala Met Gly Asp Thr Ser Val Ser
5750 5755 5760

Thr Ser Thr Pro Ala Phe Phe Glu Thr Arg Ile Gln Thr Glu Ser
5765 5770 5775

Thr Ser Ser Leu Ile Pro Gly Leu Arg Asp Thr Arg Thr Ser Glu
5780 5785 5790

Glu Ile Asn Thr Val Thr Glu Thr Ser Thr Val Leu Ser Glu Val
5795 5800 5805

Pro Thr Thr Thr Thr Thr Glu Val Ser Arg Thr Glu Val Ile Thr
5810 5815 5820

Ser Ser Arg Thr Thr Ile Ser Gly Pro Asp His Ser Lys Met Ser

412

<pre>
                    5825                          5830                          5835


        Pro Tyr  Ile Ser Thr Glu Thr  Ile Thr Arg Leu Ser  Thr Phe Pro
            5840                          5845                          5850


        Phe Val  Thr Gly Ser Thr Glu  Met Ala Ile Thr Asn  Gln Thr Gly
            5855                          5860                          5865


        Pro Ile  Gly Thr Ile Ser Gln  Ala Thr Leu Thr Leu  Asp Thr Ser
            5870                          5875                          5880


        Ser Thr  Ala Ser Trp Glu Gly  Thr His Ser Pro Val  Thr Gln Arg
            5885                          5890                          5895


        Phe Pro  His Ser Glu Glu Thr  Thr Thr Met Ser Arg  Ser Thr Lys
            5900                          5905                          5910


        Gly Val  Ser Trp Gln Ser Pro  Pro Ser Val Glu Glu  Thr Ser Ser
            5915                          5920                          5925


        Pro Ser  Ser Pro Val Pro Leu  Pro Ala Ile Thr Ser  His Ser Ser
            5930                          5935                          5940


        Leu Tyr  Ser Ala Val Ser Gly  Ser Ser Pro Thr Ser  Ala Leu Pro
            5945                          5950                          5955


        Val Thr  Ser Leu Leu Thr Ser  Gly Arg Arg Lys Thr  Ile Asp Met
            5960                          5965                          5970


        Leu Asp  Thr His Ser Glu Leu  Val Thr Ser Ser Leu  Pro Ser Ala
            5975                          5980                          5985


        Ser Ser  Phe Ser Gly Glu Ile  Leu Thr Ser Glu Ala  Ser Thr Asn
            5990                          5995                          6000


        Thr Glu  Thr Ile His Phe Ser  Glu Asn Thr Ala Glu  Thr Asn Met
            6005                          6010                          6015


        Gly Thr  Thr Asn Ser Met His  Lys Leu His Ser Ser  Val Ser Ile
            6020                          6025                          6030


        His Ser  Gln Pro Ser Gly His  Thr Pro Pro Lys Val  Thr Gly Ser
            6035                          6040                          6045


        Met Met  Glu Asp Ala Ile Val  Ser Thr Ser Thr Pro  Gly Ser Pro
            6050                          6055                          6060
</pre>

413

```
Glu Thr Lys Asn Val Asp Arg Asp Ser Thr Ser Pro Leu Thr Pro
    6065             6070             6075

Glu Leu Lys Glu Asp Ser Thr Ala Leu Val Met Asn Ser Thr Thr
    6080             6085             6090

Glu Ser Asn Thr Val Phe Ser Ser Val Ser Leu Asp Ala Ala Thr
    6095             6100             6105

Glu Val Ser Arg Ala Glu Val Thr Tyr Tyr Asp Pro Thr Phe Met
    6110             6115             6120

Pro Ala Ser Ala Gln Ser Thr Lys Ser Pro Asp Ile Ser Pro Glu
    6125             6130             6135

Ala Ser Ser Ser His Ser Asn Ser Pro Pro Leu Thr Ile Ser Thr
    6140             6145             6150

His Lys Thr Ile Ala Thr Gln Thr Gly Pro Ser Gly Val Thr Ser
    6155             6160             6165

Leu Gly Gln Leu Thr Leu Asp Thr Ser Thr Ile Ala Thr Ser Ala
    6170             6175             6180

Gly Thr Pro Ser Ala Arg Thr Gln Asp Phe Val Asp Ser Glu Thr
    6185             6190             6195

Thr Ser Val Met Asn Asn Asp Leu Asn Asp Val Leu Lys Thr Ser
    6200             6205             6210

Pro Phe Ser Ala Glu Glu Ala Asn Ser Leu Ser Ser Gln Ala Pro
    6215             6220             6225

Leu Leu Val Thr Thr Ser Pro Ser Pro Val Thr Ser Thr Leu Gln
    6230             6235             6240

Glu His Ser Thr Ser Ser Leu Val Ser Val Thr Ser Val Pro Thr
    6245             6250             6255

Pro Thr Leu Ala Lys Ile Thr Asp Met Asp Thr Asn Leu Glu Pro
    6260             6265             6270

Val Thr Arg Ser Pro Gln Asn Leu Arg Asn Thr Leu Ala Thr Ser
    6275             6280             6285

Glu Ala Thr Thr Asp Thr His Thr Met His Pro Ser Ile Asn Thr
    6290             6295             6300
```

414

```
Ala Met Ala Asn Val Gly Thr   Thr Ser Ser Pro Asn  Glu Phe Tyr
    6305                6310                6315

Phe Thr Val Ser Pro Asp Ser   Asp Pro Tyr Lys Ala  Thr Ser Ala
    6320                6325                6330

Val Val Ile Thr Ser Thr Ser   Gly Asp Ser Ile Val  Ser Thr Ser
    6335                6340                6345

Met Pro Arg Ser Ser Ala Met   Lys Lys Ile Glu Ser  Glu Thr Thr
    6350                6355                6360

Phe Ser Leu Ile Phe Arg Leu   Arg Glu Thr Ser Thr  Ser Gln Lys
    6365                6370                6375

Ile Gly Ser Ser Ser Asp Thr   Ser Thr Val Phe Asp  Lys Ala Phe
    6380                6385                6390

Thr Ala Ala Thr Thr Glu Val   Ser Arg Thr Glu Leu  Thr Ser Ser
    6395                6400                6405

Ser Arg Thr Ser Ile Gln Gly   Thr Glu Lys Pro Thr  Met Ser Pro
    6410                6415                6420

Asp Thr Ser Thr Arg Ser Val   Thr Met Leu Ser Thr  Phe Ala Gly
    6425                6430                6435

Leu Thr Lys Ser Glu Glu Arg   Thr Ile Ala Thr Gln  Thr Gly Pro
    6440                6445                6450

His Arg Ala Thr Ser Gln Gly   Thr Leu Thr Trp Asp  Thr Ser Ile
    6455                6460                6465

Thr Thr Ser Gln Ala Gly Thr   His Ser Ala Met Thr  His Gly Phe
    6470                6475                6480

Ser Gln Leu Asp Leu Ser Thr   Leu Thr Ser Arg Val  Pro Glu Tyr
    6485                6490                6495

Ile Ser Gly Thr Ser Pro Pro   Ser Val Glu Lys Thr  Ser Ser Ser
    6500                6505                6510

Ser Ser Leu Leu Ser Leu Pro   Ala Ile Thr Ser Pro  Ser Pro Val
    6515                6520                6525

Pro Thr Thr Leu Pro Glu Ser   Arg Pro Ser Ser Pro  Val His Leu
    6530                6535                6540
```

415

```
Thr Ser  Leu Pro Thr Ser Gly  Leu Val Lys Thr Thr  Asp Met Leu
    6545             6550             6555

Ala Ser  Val Ala Ser Leu Pro  Pro Asn Leu Gly Ser  Thr Ser His
    6560             6565             6570

Lys Ile  Pro Thr Thr Ser Glu  Asp Ile Lys Asp Thr  Glu Lys Met
    6575             6580             6585

Tyr Pro  Ser Thr Asn Ile Ala  Val Thr Asn Val Gly  Thr Thr Thr
    6590             6595             6600

Ser Glu  Lys Glu Ser Tyr Ser  Ser Val Pro Ala Tyr  Ser Glu Pro
    6605             6610             6615

Pro Lys  Val Thr Ser Pro Met  Val Thr Ser Phe Asn  Ile Arg Asp
    6620             6625             6630

Thr Ile  Val Ser Thr Ser Met  Pro Gly Ser Ser Glu  Ile Thr Arg
    6635             6640             6645

Ile Glu  Met Glu Ser Thr Phe  Ser Val Ala His Gly  Leu Lys Gly
    6650             6655             6660

Thr Ser  Thr Ser Gln Asp Pro  Ile Val Ser Thr Glu  Lys Ser Ala
    6665             6670             6675

Val Leu  His Lys Leu Thr Thr  Gly Ala Thr Glu Thr  Ser Arg Thr
    6680             6685             6690

Glu Val  Ala Ser Ser Arg Arg  Thr Ser Ile Pro Gly  Pro Asp His
    6695             6700             6705

Ser Thr  Glu Ser Pro Asp Ile  Ser Thr Glu Val Ile  Pro Ser Leu
    6710             6715             6720

Pro Ile  Ser Leu Gly Ile Thr  Glu Ser Ser Asn Met  Thr Ile Ile
    6725             6730             6735

Thr Arg  Thr Gly Pro Pro Leu  Gly Ser Thr Ser Gln  Gly Thr Phe
    6740             6745             6750

Thr Leu  Asp Thr Pro Thr Thr  Ser Ser Arg Ala Gly  Thr His Ser
    6755             6760             6765

Met Ala  Thr Gln Glu Phe Pro  His Ser Glu Met Thr  Thr Val Met
```

```
                6770                    6775                     6780


        Asn Lys  Asp Pro Glu Ile Leu  Ser Trp Thr Ile Pro  Pro Ser Ile
            6785                    6790                     6795


        Glu Lys  Thr Ser Phe Ser Ser  Ser Leu Met Pro Ser  Pro Ala Met
            6800                    6805                     6810


        Thr Ser  Pro Pro Val Ser Ser  Thr Leu Pro Lys Thr  Ile His Thr
            6815                    6820                     6825


        Thr Pro  Ser Pro Met Thr Ser  Leu Leu Thr Pro Ser  Leu Val Met
            6830                    6835                     6840


        Thr Thr  Asp Thr Leu Gly Thr  Ser Pro Glu Pro Thr  Thr Ser Ser
            6845                    6850                     6855


        Pro Pro  Asn Leu Ser Ser Thr  Ser His Val Ile Leu  Thr Thr Asp
            6860                    6865                     6870


        Glu Asp  Thr Thr Ala Ile Glu  Ala Met His Pro Ser  Thr Ser Thr
            6875                    6880                     6885


        Ala Ala  Thr Asn Val Glu Thr  Thr Cys Ser Gly His  Gly Ser Gln
            6890                    6895                     6900


        Ser Ser  Val Leu Thr Asp Ser  Glu Lys Thr Lys Ala  Thr Ala Pro
            6905                    6910                     6915


        Met Asp  Thr Thr Ser Thr Met  Gly His Thr Thr Val  Ser Thr Ser
            6920                    6925                     6930


        Met Ser  Val Ser Ser Glu Thr  Thr Lys Ile Lys Arg  Glu Ser Thr
            6935                    6940                     6945


        Tyr Ser  Leu Thr Pro Gly Leu  Arg Glu Thr Ser Ile  Ser Gln Asn
            6950                    6955                     6960


        Ala Ser  Phe Ser Thr Asp Thr  Ser Ile Val Leu Ser  Glu Val Pro
            6965                    6970                     6975


        Thr Gly  Thr Thr Ala Glu Val  Ser Arg Thr Glu Val  Thr Ser Ser
            6980                    6985                     6990


        Gly Arg  Thr Ser Ile Pro Gly  Pro Ser Gln Ser Thr  Val Leu Pro
            6995                    7000                     7005
```

417

```
Glu Ile Ser Thr Arg Thr Met  Thr Arg Leu Phe Ala  Ser Pro Thr
    7010            7015            7020

Met Thr Glu Ser Ala Glu Met  Thr Ile Pro Thr Gln  Thr Gly Pro
    7025            7030            7035

Ser Gly Ser Thr Ser Gln Asp  Thr Leu Thr Leu Asp  Thr Ser Thr
    7040            7045            7050

Thr Lys Ser Gln Ala Lys Thr  His Ser Thr Leu Thr  Gln Arg Phe
    7055            7060            7065

Pro His Ser Glu Met Thr Thr  Leu Met Ser Arg Gly  Pro Gly Asp
    7070            7075            7080

Met Ser Trp Gln Ser Ser Pro  Ser Leu Glu Asn Pro  Ser Ser Leu
    7085            7090            7095

Pro Ser Leu Leu Ser Leu Pro  Ala Thr Thr Ser Pro  Pro Pro Ile
    7100            7105            7110

Ser Ser Thr Leu Pro Val Thr  Ile Ser Ser Ser Pro  Leu Pro Val
    7115            7120            7125

Thr Ser Leu Leu Thr Ser Ser  Pro Val Thr Thr Thr  Asp Met Leu
    7130            7135            7140

His Thr Ser Pro Glu Leu Val  Thr Ser Ser Pro Pro  Lys Leu Ser
    7145            7150            7155

His Thr Ser Asp Glu Arg Leu  Thr Thr Gly Lys Asp  Thr Thr Asn
    7160            7165            7170

Thr Glu Ala Val His Pro Ser  Thr Asn Thr Ala Ala  Ser Asn Val
    7175            7180            7185

Glu Ile Pro Ser Phe Gly His  Glu Ser Pro Ser Ser  Ala Leu Ala
    7190            7195            7200

Asp Ser Glu Thr Ser Lys Ala  Thr Ser Pro Met Phe  Ile Thr Ser
    7205            7210            7215

Thr Gln Glu Asp Thr Thr Val  Ala Ile Ser Thr Pro  His Phe Leu
    7220            7225            7230

Glu Thr Ser Arg Ile Gln Lys  Glu Ser Ile Ser Ser  Leu Ser Pro
    7235            7240            7245
```

418

```
Lys Leu Arg Glu Thr Gly Ser  Ser Val Glu Thr Ser  Ser Ala Ile
    7250            7255             7260

Glu Thr Ser Ala Val Leu Ser  Glu Val Ser Ile Gly  Ala Thr Thr
    7265            7270             7275

Glu Ile Ser Arg Thr Glu Val  Thr Ser Ser Ser Arg  Thr Ser Ile
    7280            7285             7290

Ser Gly Ser Ala Glu Ser Thr  Met Leu Pro Glu Ile  Ser Thr Thr
    7295            7300             7305

Arg Lys Ile Ile Lys Phe Pro  Thr Ser Pro Ile Leu  Ala Glu Ser
    7310            7315             7320

Ser Glu Met Thr Ile Lys Thr  Gln Thr Ser Pro Pro  Gly Ser Thr
    7325            7330             7335

Ser Glu Ser Thr Phe Thr Leu  Asp Thr Ser Thr Thr  Pro Ser Leu
    7340            7345             7350

Val Ile Thr His Ser Thr Met  Thr Gln Arg Leu Pro  His Ser Glu
    7355            7360             7365

Ile Thr Thr Leu Val Ser Arg  Gly Ala Gly Asp Val  Pro Arg Pro
    7370            7375             7380

Ser Ser Leu Pro Val Glu Glu  Thr Ser Pro Pro Ser  Ser Gln Leu
    7385            7390             7395

Ser Leu Ser Ala Met Ile Ser  Pro Ser Pro Val Ser  Ser Thr Leu
    7400            7405             7410

Pro Ala Ser Ser His Ser Ser  Ser Ala Ser Val Thr  Ser Pro Leu
    7415            7420             7425

Thr Pro Gly Gln Val Lys Thr  Thr Glu Val Leu Asp  Ala Ser Ala
    7430            7435             7440

Glu Pro Glu Thr Ser Ser Pro  Pro Ser Leu Ser Ser  Thr Ser Val
    7445            7450             7455

Glu Ile Leu Ala Thr Ser Glu  Val Thr Thr Asp Thr  Glu Lys Ile
    7460            7465             7470

His Pro Phe Pro Asn Thr Ala  Val Thr Lys Val Gly  Thr Ser Ser
    7475            7480             7485
```

419

```
Ser Gly His Glu Ser Pro Ser  Ser Val Leu Pro Asp  Ser Glu Thr
    7490             7495             7500

Thr Lys Ala Thr Ser Ala Met  Gly Thr Ile Ser Ile  Met Gly Asp
    7505             7510             7515

Thr Ser Val Ser Thr Leu Thr  Pro Ala Leu Ser Asn  Thr Arg Lys
    7520             7525             7530

Ile Gln Ser Glu Pro Ala Ser  Ser Leu Thr Thr Arg  Leu Arg Glu
    7535             7540             7545

Thr Ser Thr Ser Glu Glu Thr  Ser Leu Ala Thr Glu  Ala Asn Thr
    7550             7555             7560

Val Leu Ser Lys Val Ser Thr  Gly Ala Thr Thr Glu  Val Ser Arg
    7565             7570             7575

Thr Glu Ala Ile Ser Phe Ser  Arg Thr Ser Met Ser  Gly Pro Glu
    7580             7585             7590

Gln Ser Thr Met Ser Gln Asp  Ile Ser Ile Gly Thr  Ile Pro Arg
    7595             7600             7605

Ile Ser Ala Ser Ser Val Leu  Thr Glu Ser Ala Lys  Met Thr Ile
    7610             7615             7620

Thr Thr Gln Thr Gly Pro Ser  Glu Ser Thr Leu Glu  Ser Thr Leu
    7625             7630             7635

Asn Leu Asn Thr Ala Thr Thr  Pro Ser Trp Val Glu  Thr His Ser
    7640             7645             7650

Ile Val Ile Gln Gly Phe Pro  His Pro Glu Met Thr  Thr Ser Met
    7655             7660             7665

Gly Arg Gly Pro Gly Gly Val  Ser Trp Pro Ser Pro  Pro Phe Val
    7670             7675             7680

Lys Glu Thr Ser Pro Pro Ser  Ser Pro Leu Ser Leu  Pro Ala Val
    7685             7690             7695

Thr Ser Pro His Pro Val Ser  Thr Thr Phe Leu Ala  His Ile Pro
    7700             7705             7710

Pro Ser Pro Leu Pro Val Thr  Ser Leu Leu Thr Ser  Gly Pro Ala
```

420

```
                7715                        7720                        7725


        Thr Thr  Thr Asp Ile Leu Gly  Thr Ser Thr Glu Pro  Gly Thr Ser
                 7730                   7735                 7740


        Ser Ser  Ser Ser Leu Ser Thr  Thr Ser His Glu Arg  Leu Thr Thr
                 7745                   7750                 7755


        Tyr Lys  Asp Thr Ala His Thr  Glu Ala Val His Pro  Ser Thr Asn
                 7760                   7765                 7770


        Thr Gly  Gly Thr Asn Val Ala  Thr Thr Ser Ser Gly  Tyr Lys Ser
                 7775                   7780                 7785


        Gln Ser  Ser Val Leu Ala Asp  Ser Ser Pro Met Cys  Thr Thr Ser
                 7790                   7795                 7800


        Thr Met  Gly Asp Thr Ser Val  Leu Thr Ser Thr Pro  Ala Phe Leu
                 7805                   7810                 7815


        Glu Thr  Arg Arg Ile Gln Thr  Glu Leu Ala Ser Ser  Leu Thr Pro
                 7820                   7825                 7830


        Gly Leu  Arg Glu Ser Ser Gly  Ser Glu Gly Thr Ser  Ser Gly Thr
                 7835                   7840                 7845


        Lys Met  Ser Thr Val Leu Ser  Lys Val Pro Thr Gly  Ala Thr Thr
                 7850                   7855                 7860


        Glu Ile  Ser Lys Glu Asp Val  Thr Ser Ile Pro Gly  Pro Ala Gln
                 7865                   7870                 7875


        Ser Thr  Ile Ser Pro Asp Ile  Ser Thr Arg Thr Val  Ser Trp Phe
                 7880                   7885                 7890


        Ser Thr  Ser Pro Val Met Thr  Glu Ser Ala Glu Ile  Thr Met Asn
                 7895                   7900                 7905


        Thr His  Thr Ser Pro Leu Gly  Ala Thr Thr Gln Gly  Thr Ser Thr
                 7910                   7915                 7920


        Leu Ala  Thr Ser Ser Thr Thr  Ser Leu Thr Met Thr  His Ser Thr
                 7925                   7930                 7935


        Ile Ser  Gln Gly Phe Ser His  Ser Gln Met Ser Thr  Leu Met Arg
                 7940                   7945                 7950
```

421

Arg Gly Pro Glu Asp Val Ser Trp Met Ser Pro Pro Leu Leu Glu
    7955                7960            7965

Lys Thr Arg Pro Ser Phe Ser Leu Met Ser Ser Pro Ala Thr Thr
    7970                7975            7980

Ser Pro Ser Pro Val Ser Ser Thr Leu Pro Glu Ser Ile Ser Ser
    7985                7990            7995

Ser Pro Leu Pro Val Thr Ser Leu Leu Thr Ser Gly Leu Ala Lys
    8000                8005            8010

Thr Thr Asp Met Leu His Lys Ser Ser Glu Pro Val Thr Asn Ser
    8015                8020            8025

Pro Ala Asn Leu Ser Ser Thr Ser Val Glu Ile Leu Ala Thr Ser
    8030                8035            8040

Glu Val Thr Thr Asp Thr Glu Lys Thr His Pro Ser Ser Asn Arg
    8045                8050            8055

Thr Val Thr Asp Val Gly Thr Ser Ser Ser Gly His Glu Ser Thr
    8060                8065            8070

Ser Phe Val Leu Ala Asp Ser Gln Thr Ser Lys Val Thr Ser Pro
    8075                8080            8085

Met Val Ile Thr Ser Thr Met Glu Asp Thr Ser Val Ser Thr Ser
    8090                8095            8100

Thr Pro Gly Phe Phe Glu Thr Ser Arg Ile Gln Thr Glu Pro Thr
    8105                8110            8115

Ser Ser Leu Thr Leu Gly Leu Arg Lys Thr Ser Ser Ser Glu Gly
    8120                8125            8130

Thr Ser Leu Ala Thr Glu Met Ser Thr Val Leu Ser Gly Val Pro
    8135                8140            8145

Thr Gly Ala Thr Ala Glu Val Ser Arg Thr Glu Val Thr Ser Ser
    8150                8155            8160

Ser Arg Thr Ser Ile Ser Gly Phe Ala Gln Leu Thr Val Ser Pro
    8165                8170            8175

Glu Thr Ser Thr Glu Thr Ile Thr Arg Leu Pro Thr Ser Ser Ile
    8180                8185            8190

```
Met Thr  Glu Ser Ala Glu Met  Met Ile Lys Thr Gln  Thr Asp Pro
    8195             8200             8205
```

```
Pro Gly  Ser Thr Pro Glu Ser  Thr His Thr Val Asp  Ile Ser Thr
    8210             8215             8220
```

```
Thr Pro  Asn Trp Val Glu Thr  His Ser Thr Val Thr  Gln Arg Phe
    8225             8230             8235
```

```
Ser His  Ser Glu Met Thr Thr  Leu Val Ser Arg Ser  Pro Gly Asp
    8240             8245             8250
```

```
Met Leu  Trp Pro Ser Gln Ser  Ser Val Glu Glu Thr  Ser Ser Ala
    8255             8260             8265
```

```
Ser Ser  Leu Leu Ser Leu Pro  Ala Thr Thr Ser Pro  Ser Pro Val
    8270             8275             8280
```

```
Ser Ser  Thr Leu Val Glu Asp  Phe Pro Ser Ala Ser  Leu Pro Val
    8285             8290             8295
```

```
Thr Ser  Leu Leu Thr Pro Gly  Leu Val Ile Thr Thr  Asp Arg Met
    8300             8305             8310
```

```
Gly Ile  Ser Arg Glu Pro Gly  Thr Ser Ser Thr Ser  Asn Leu Ser
    8315             8320             8325
```

```
Ser Thr  Ser His Glu Arg Leu  Thr Thr Leu Glu Asp  Thr Val Asp
    8330             8335             8340
```

```
Thr Glu  Asp Met Gln Pro Ser  Thr His Thr Ala Val  Thr Asn Val
    8345             8350             8355
```

```
Arg Thr  Ser Ile Ser Gly His  Glu Ser Gln Ser Ser  Val Leu Ser
    8360             8365             8370
```

```
Asp Ser  Glu Thr Pro Lys Ala  Thr Ser Pro Met Gly  Thr Thr Tyr
    8375             8380             8385
```

```
Thr Met  Gly Glu Thr Ser Val  Ser Ile Ser Thr Ser  Asp Phe Phe
    8390             8395             8400
```

```
Glu Thr  Ser Arg Ile Gln Ile  Glu Pro Thr Ser Ser  Leu Thr Ser
    8405             8410             8415
```

```
Gly Leu  Arg Glu Thr Ser Ser  Ser Glu Arg Ile Ser  Ser Ala Thr
    8420             8425             8430
```

423

```
Glu Gly  Ser Thr Val Leu Ser  Glu Val Pro Ser Gly  Ala Thr Thr
    8435             8440              8445

Glu Val  Ser Arg Thr Glu Val  Ile Ser Ser Arg Gly  Thr Ser Met
    8450             8455              8460

Ser Gly  Pro Asp Gln Phe Thr  Ile Ser Pro Asp Ile  Ser Thr Glu
    8465             8470              8475

Ala Ile  Thr Arg Leu Ser Thr  Ser Pro Ile Met Thr  Glu Ser Ala
    8480             8485              8490

Glu Ser  Ala Ile Thr Ile Glu  Thr Gly Ser Pro Gly  Ala Thr Ser
    8495             8500              8505

Glu Gly  Thr Leu Thr Leu Asp  Thr Ser Thr Thr Thr  Phe Trp Ser
    8510             8515              8520

Gly Thr  His Ser Thr Ala Ser  Pro Gly Phe Ser His  Ser Glu Met
    8525             8530              8535

Thr Thr  Leu Met Ser Arg Thr  Pro Gly Asp Val Pro  Trp Pro Ser
    8540             8545              8550

Leu Pro  Ser Val Glu Glu Ala  Ser Ser Val Ser Ser  Ser Leu Ser
    8555             8560              8565

Ser Pro  Ala Met Thr Ser Thr  Ser Phe Phe Ser Ala  Leu Pro Glu
    8570             8575              8580

Ser Ile  Ser Ser Ser Pro His  Pro Val Thr Ala Leu  Leu Thr Leu
    8585             8590              8595

Gly Pro  Val Lys Thr Thr Asp  Met Leu Arg Thr Ser  Ser Glu Pro
    8600             8605              8610

Glu Thr  Ser Ser Pro Pro Asn  Leu Ser Ser Thr Ser  Ala Glu Ile
    8615             8620              8625

Leu Ala  Thr Ser Glu Val Thr  Lys Asp Arg Glu Lys  Ile His Pro
    8630             8635              8640

Ser Ser  Asn Thr Pro Val Val  Asn Val Gly Thr Val  Ile Tyr Lys
    8645             8650              8655

His Leu  Ser Pro Ser Ser Val  Leu Ala Asp Leu Val  Thr Thr Lys
```

```
        8660                    8665                        8670


        Pro Thr  Ser Pro Met Ala Thr  Thr Ser Thr Leu Gly  Asn Thr Ser
            8675                    8680                    8685


        Val Ser  Thr Ser Thr Pro Ala  Phe Pro Glu Thr Met  Met Thr Gln
            8690                    8695                    8700


        Pro Thr  Ser Ser Leu Thr Ser  Gly Leu Arg Glu Ile  Ser Thr Ser
            8705                    8710                    8715


        Gln Glu  Thr Ser Ser Ala Thr  Glu Arg Ser Ala Ser  Leu Ser Gly
            8720                    8725                    8730


        Met Pro  Thr Gly Ala Thr Thr  Lys Val Ser Arg Thr  Glu Ala Leu
            8735                    8740                    8745


        Ser Leu  Gly Arg Thr Ser Thr  Pro Gly Pro Ala Gln  Ser Thr Ile
            8750                    8755                    8760


        Ser Pro  Glu Ile Ser Thr Glu  Thr Ile Thr Arg Ile  Ser Thr Pro
            8765                    8770                    8775


        Leu Thr  Thr Thr Gly Ser Ala  Glu Met Thr Ile Thr  Pro Lys Thr
            8780                    8785                    8790


        Gly His  Ser Gly Ala Ser Ser  Gln Gly Thr Phe Thr  Leu Asp Thr
            8795                    8800                    8805


        Ser Ser  Arg Ala Ser Trp Pro  Gly Thr His Ser Ala  Ala Thr His
            8810                    8815                    8820


        Arg Ser  Pro His Ser Gly Met  Thr Thr Pro Met Ser  Arg Gly Pro
            8825                    8830                    8835


        Glu Asp  Val Ser Trp Pro Ser  Arg Pro Ser Val Glu  Lys Thr Ser
            8840                    8845                    8850


        Pro Pro  Ser Ser Leu Val Ser  Leu Ser Ala Val Thr  Ser Pro Ser
            8855                    8860                    8865


        Pro Leu  Tyr Ser Thr Pro Ser  Glu Ser Ser His Ser  Ser Pro Leu
            8870                    8875                    8880


        Arg Val  Thr Ser Leu Phe Thr  Pro Val Met Met Lys  Thr Thr Asp
            8885                    8890                    8895
```

```
Met Leu Asp Thr Ser Leu Glu  Pro Val Thr Thr Ser  Pro Pro Ser
    8900              8905              8910

Met Asn Ile Thr Ser Asp Glu  Ser Leu Ala Thr Ser  Lys Ala Thr
    8915              8920              8925

Met Glu Thr Glu Ala Ile Gln  Leu Ser Glu Asn Thr  Ala Val Thr
    8930              8935              8940

Gln Met Gly Thr Ile Ser Ala  Arg Gln Glu Phe Tyr  Ser Ser Tyr
    8945              8950              8955

Pro Gly Leu Pro Glu Pro Ser  Lys Val Thr Ser Pro  Val Val Thr
    8960              8965              8970

Ser Ser Thr Ile Lys Asp Ile  Val Ser Thr Thr Ile  Pro Ala Ser
    8975              8980              8985

Ser Glu Ile Thr Arg Ile Glu  Met Glu Ser Thr Ser  Thr Leu Thr
    8990              8995              9000

Pro Thr Pro Arg Glu Thr Ser  Thr Ser Gln Glu Ile  His Ser Ala
    9005              9010              9015

Thr Lys Pro Ser Thr Val Pro  Tyr Lys Ala Leu Thr  Ser Ala Thr
    9020              9025              9030

Ile Glu Asp Ser Met Thr Gln  Val Met Ser Ser Ser  Arg Gly Pro
    9035              9040              9045

Ser Pro Asp Gln Ser Thr Met  Ser Gln Asp Ile Ser  Ser Glu Val
    9050              9055              9060

Ile Thr Arg Leu Ser Thr Ser  Pro Ile Lys Ala Glu  Ser Thr Glu
    9065              9070              9075

Met Thr Ile Thr Thr Gln Thr  Gly Ser Pro Gly Ala  Thr Ser Arg
    9080              9085              9090

Gly Thr Leu Thr Leu Asp Thr  Ser Thr Thr Phe Met  Ser Gly Thr
    9095              9100              9105

His Ser Thr Ala Ser Gln Gly  Phe Ser His Ser Gln  Met Thr Ala
    9110              9115              9120

Leu Met Ser Arg Thr Pro Gly  Asp Val Pro Trp Leu  Ser His Pro
    9125              9130              9135
```

```
Ser Val Glu Glu Ala Ser Ser  Ala Ser Phe Ser Leu  Ser Ser Pro
    9140             9145              9150

Val Met Thr Ser Ser Ser Pro  Val Ser Ser Thr Leu  Pro Asp Ser
    9155             9160              9165

Ile His Ser Ser Ser Leu Pro  Val Thr Ser Leu Leu  Thr Ser Gly
    9170             9175              9180

Leu Val Lys Thr Thr Glu Leu  Leu Gly Thr Ser Ser  Glu Pro Glu
    9185             9190              9195

Thr Ser Ser Pro Pro Asn Leu  Ser Ser Thr Ser Ala  Glu Ile Leu
    9200             9205              9210

Ala Thr Thr Glu Val Thr Thr  Asp Thr Glu Lys Leu  Glu Met Thr
    9215             9220              9225

Asn Val Val Thr Ser Gly Tyr  Thr His Glu Ser Pro  Ser Ser Val
    9230             9235              9240

Leu Ala Asp Ser Val Thr Thr  Lys Ala Thr Ser Ser  Met Gly Ile
    9245             9250              9255

Thr Tyr Pro Thr Gly Asp Thr  Asn Val Leu Thr Ser  Thr Pro Ala
    9260             9265              9270

Phe Ser Asp Thr Ser Arg Ile  Gln Thr Lys Ser Lys  Leu Ser Leu
    9275             9280              9285

Thr Pro Gly Leu Met Glu Thr  Ser Ile Ser Glu Glu  Thr Ser Ser
    9290             9295              9300

Ala Thr Glu Lys Ser Thr Val  Leu Ser Ser Val Pro  Thr Gly Ala
    9305             9310              9315

Thr Thr Glu Val Ser Arg Thr  Glu Ala Ile Ser Ser  Ser Arg Thr
    9320             9325              9330

Ser Ile Pro Gly Pro Ala Gln  Ser Thr Met Ser Ser  Asp Thr Ser
    9335             9340              9345

Met Glu Thr Ile Thr Arg Ile  Ser Thr Pro Leu Thr  Arg Lys Glu
    9350             9355              9360

Ser Thr Asp Met Ala Ile Thr  Pro Lys Thr Gly Pro  Ser Gly Ala
    9365             9370              9375
```

427

Thr Ser Gln Gly Thr Phe Thr Leu Asp Ser Ser Ser Thr Ala Ser
9380 9385 9390

Trp Pro Gly Thr His Ser Ala Thr Thr Gln Arg Phe Pro Gln Ser
9395 9400 9405

Val Val Thr Thr Pro Met Ser Arg Gly Pro Glu Asp Val Ser Trp
9410 9415 9420

Pro Ser Pro Leu Ser Val Glu Lys Asn Ser Pro Pro Ser Ser Leu
9425 9430 9435

Val Ser Ser Ser Ser Val Thr Ser Pro Ser Pro Leu Tyr Ser Thr
9440 9445 9450

Pro Ser Gly Ser Ser His Ser Ser Pro Val Pro Val Thr Ser Leu
9455 9460 9465

Phe Thr Ser Ile Met Met Lys Ala Thr Asp Met Leu Asp Ala Ser
9470 9475 9480

Leu Glu Pro Glu Thr Thr Ser Ala Pro Asn Met Asn Ile Thr Ser
9485 9490 9495

Asp Glu Ser Leu Ala Thr Ser Lys Ala Thr Thr Glu Thr Glu Ala
9500 9505 9510

Ile His Val Phe Glu Asn Thr Ala Ala Ser His Val Glu Thr Thr
9515 9520 9525

Ser Ala Thr Glu Glu Leu Tyr Ser Ser Ser Pro Gly Phe Ser Glu
9530 9535 9540

Pro Thr Lys Val Ile Ser Pro Val Val Thr Ser Ser Ser Ile Arg
9545 9550 9555

Asp Asn Met Val Ser Thr Thr Met Pro Gly Ser Ser Gly Ile Thr
9560 9565 9570

Arg Ile Glu Ile Glu Ser Met Ser Ser Leu Thr Pro Gly Leu Arg
9575 9580 9585

Glu Thr Arg Thr Ser Gln Asp Ile Thr Ser Ser Thr Glu Thr Ser
9590 9595 9600

Thr Val Leu Tyr Lys Met Ser Ser Gly Ala Thr Pro Glu Val Ser

428

9605 9610 9615

Arg Thr Glu Val Met Pro Ser Ser Arg Thr Ser Ile Pro Gly Pro
9620 9625 9630

Ala Gln Ser Thr Met Ser Leu Asp Ile Ser Asp Glu Val Val Thr
9635 9640 9645

Arg Leu Ser Thr Ser Pro Ile Met Thr Glu Ser Ala Glu Ile Thr
9650 9655 9660

Ile Thr Thr Gln Thr Gly Tyr Ser Leu Ala Thr Ser Gln Val Thr
9665 9670 9675

Leu Pro Leu Gly Thr Ser Met Thr Phe Leu Ser Gly Thr His Ser
9680 9685 9690

Thr Met Ser Gln Gly Leu Ser His Ser Glu Met Thr Asn Leu Met
9695 9700 9705

Ser Arg Gly Pro Glu Ser Leu Ser Trp Thr Ser Pro Arg Phe Val
9710 9715 9720

Glu Thr Thr Arg Ser Ser Ser Ser Leu Thr Ser Leu Pro Leu Thr
9725 9730 9735

Thr Ser Leu Ser Pro Val Ser Ser Thr Leu Leu Asp Ser Ser Pro
9740 9745 9750

Ser Ser Pro Leu Pro Val Thr Ser Leu Ile Leu Pro Gly Leu Val
9755 9760 9765

Lys Thr Thr Glu Val Leu Asp Thr Ser Ser Glu Pro Lys Thr Ser
9770 9775 9780

Ser Ser Pro Asn Leu Ser Ser Thr Ser Val Glu Ile Pro Ala Thr
9785 9790 9795

Ser Glu Ile Met Thr Asp Thr Glu Lys Ile His Pro Ser Ser Asn
9800 9805 9810

Thr Ala Val Ala Lys Val Arg Thr Ser Ser Ser Val His Glu Ser
9815 9820 9825

His Ser Ser Val Leu Ala Asp Ser Glu Thr Thr Ile Thr Ile Pro
9830 9835 9840

429

```
Ser Met Gly Ile Thr Ser Ala  Val Asp Asp Thr Thr  Val Phe Thr
    9845              9850              9855

Ser Asn Pro Ala Phe Ser Glu  Thr Arg Arg Ile Pro  Thr Glu Pro
    9860              9865              9870

Thr Phe Ser Leu Thr Pro Gly  Phe Arg Glu Thr Ser  Thr Ser Glu
    9875              9880              9885

Glu Thr Thr Ser Ile Thr Glu  Thr Ser Ala Val Leu  Tyr Gly Val
    9890              9895              9900

Pro Thr Ser Ala Thr Thr Glu  Val Ser Met Thr Glu  Ile Met Ser
    9905              9910              9915

Ser Asn Arg Thr His Ile Pro  Asp Ser Asp Gln Ser  Thr Met Ser
    9920              9925              9930

Pro Asp Ile Ile Thr Glu Val  Ile Thr Arg Leu Ser  Ser Ser Ser
    9935              9940              9945

Met Met Ser Glu Ser Thr Gln  Met Thr Ile Thr Thr  Gln Lys Ser
    9950              9955              9960

Ser Pro Gly Ala Thr Ala Gln  Ser Thr Leu Thr Leu  Ala Thr Thr
    9965              9970              9975

Thr Ala Pro Leu Ala Arg Thr  His Ser Thr Val Pro  Pro Arg Phe
    9980              9985              9990

Leu His Ser Glu Met Thr Thr  Leu Met Ser Arg Ser  Pro Glu Asn
    9995              10000             10005

Pro Ser Trp Lys Ser Ser Pro  Phe Val Glu Lys Thr  Ser Ser Ser
    10010             10015             10020

Ser Ser Leu Leu Ser Leu Pro  Val Thr Thr Ser Pro  Ser Val Ser
    10025             10030             10035

Ser Thr Leu Pro Gln Ser Ile  Pro Ser Ser Ser Phe  Ser Val Thr
    10040             10045             10050

Ser Leu Leu Thr Pro Gly Met  Val Lys Thr Thr Asp  Thr Ser Thr
    10055             10060             10065

Glu Pro Gly Thr Ser Leu Ser  Pro Asn Leu Ser Gly  Thr Ser Val
    10070             10075             10080
```

430

```
Glu Ile   Leu Ala Ala Ser Glu   Val Thr Thr Asp Thr   Glu Lys Ile
    10085               10090               10095

His Pro   Ser Ser Ser Met Ala   Val Thr Asn Val Gly   Thr Thr Ser
    10100               10105               10110

Ser Gly   His Glu Leu Tyr Ser   Ser Val Ser Ile His   Ser Glu Pro
    10115               10120               10125

Ser Lys   Ala Thr Tyr Pro Val   Gly Thr Pro Ser Ser   Met Ala Glu
    10130               10135               10140

Thr Ser   Ile Ser Thr Ser Met   Pro Ala Asn Phe Glu   Thr Thr Gly
    10145               10150               10155

Phe Glu   Ala Glu Pro Phe Ser   His Leu Thr Ser Gly   Phe Arg Lys
    10160               10165               10170

Thr Asn   Met Ser Leu Asp Thr   Ser Ser Val Thr Pro   Thr Asn Thr
    10175               10180               10185

Pro Ser   Ser Pro Gly Ser Thr   His Leu Leu Gln Ser   Ser Lys Thr
    10190               10195               10200

Asp Phe   Thr Ser Ser Ala Lys   Thr Ser Ser Pro Asp   Trp Pro Pro
    10205               10210               10215

Ala Ser   Gln Tyr Thr Glu Ile   Pro Val Asp Ile Ile   Thr Pro Phe
    10220               10225               10230

Asn Ala   Ser Pro Ser Ile Thr   Glu Ser Thr Gly Ile   Thr Ser Phe
    10235               10240               10245

Pro Glu   Ser Arg Phe Thr Met   Ser Val Thr Glu Ser   Thr His His
    10250               10255               10260

Leu Ser   Thr Asp Leu Leu Pro   Ser Ala Glu Thr Ile   Ser Thr Gly
    10265               10270               10275

Thr Val   Met Pro Ser Leu Ser   Glu Ala Met Thr Ser   Phe Ala Thr
    10280               10285               10290

Thr Gly   Val Pro Arg Ala Ile   Ser Gly Ser Gly Ser   Pro Phe Ser
    10295               10300               10305

Arg Thr   Glu Ser Gly Pro Gly   Asp Ala Thr Leu Ser   Thr Ile Ala
    10310               10315               10320
```

Glu Ser    Leu Pro Ser Ser Thr    Pro Val Pro Phe Ser    Ser Ser Thr
    10325                  10330                10335

Phe Thr    Thr Thr Asp Ser Ser    Thr Ile Pro Ala Leu    His Glu Ile
    10340                  10345                10350

Thr Ser    Ser Ser Ala Thr Pro    Tyr Arg Val Asp Thr    Ser Leu Gly
    10355                  10360                10365

Thr Glu    Ser Ser Thr Thr Glu    Gly Arg Leu Val Met    Val Ser Thr
    10370                  10375                10380

Leu Asp    Thr Ser Ser Gln Pro    Gly Arg Thr Ser Ser    Thr Pro Ile
    10385                  10390                10395

Leu Asp    Thr Arg Met Thr Glu    Ser Val Glu Leu Gly    Thr Val Thr
    10400                  10405                10410

Ser Ala    Tyr Gln Val Pro Ser    Leu Ser Thr Arg Leu    Thr Arg Thr
    10415                  10420                10425

Asp Gly    Ile Met Glu His Ile    Thr Lys Ile Pro Asn    Glu Ala Ala
    10430                  10435                10440

His Arg    Gly Thr Ile Arg Pro    Val Lys Gly Pro Gln    Thr Ser Thr
    10445                  10450                10455

Ser Pro    Ala Ser Pro Lys Gly    Leu His Thr Gly Gly    Thr Lys Arg
    10460                  10465                10470

Met Glu    Thr Thr Thr Thr Ala    Leu Lys Thr Thr Thr    Thr Ala Leu
    10475                  10480                10485

Lys Thr    Thr Ser Arg Ala Thr    Leu Thr Thr Ser Val    Tyr Thr Pro
    10490                  10495                10500

Thr Leu    Gly Thr Leu Thr Pro    Leu Asn Ala Ser Arg    Gln Met Ala
    10505                  10510                10515

Ser Thr    Ile Leu Thr Glu Met    Met Ile Thr Thr Pro    Tyr Val Phe
    10520                  10525                10530

Pro Asp    Val Pro Glu Thr Thr    Ser Ser Leu Ala Thr    Ser Leu Gly
    10535                  10540                10545

Ala Glu    Thr Ser Thr Ala Leu    Pro Arg Thr Thr Pro    Ser Val Leu

432

Asn Arg Glu Ser Glu Thr Thr   Ala Ser Leu Val Ser   Arg Ser Gly

Ala Glu Arg Ser Pro Val Ile   Gln Thr Leu Asp Val   Ser Ser Ser

Glu Pro Asp Thr Thr Ala Ser   Trp Val Ile His Pro   Ala Glu Thr

Ile Pro Thr Val Ser Lys Thr   Thr Pro Asn Phe Phe   His Ser Glu

Leu Asp Thr Val Ser Ser Thr   Ala Thr Ser His Gly   Ala Asp Val

Ser Ser Ala Ile Pro Thr Asn   Ile Ser Pro Ser Glu   Leu Asp Ala

Leu Thr Pro Leu Val Thr Ile   Ser Gly Thr Asp Thr   Ser Thr Thr

Phe Pro Thr Leu Thr Lys Ser   Pro His Glu Thr Glu   Thr Arg Thr

Thr Trp Leu Thr His Pro Ala   Glu Thr Ser Ser Thr   Ile Pro Arg

Thr Ile Pro Asn Phe Ser His   His Glu Ser Asp Ala   Thr Pro Ser

Ile Ala Thr Ser Pro Gly Ala   Glu Thr Ser Ser Ala   Ile Pro Ile

Met Thr Val Ser Pro Gly Ala   Glu Asp Leu Val Thr   Ser Gln Val

Thr Ser Ser Gly Thr Asp Arg   Asn Met Thr Ile Pro   Thr Leu Thr

Leu Ser Pro Gly Glu Pro Lys   Thr Ile Ala Ser Leu   Val Thr His

Pro Glu Ala Gln Thr Ser Ser   Ala Ile Pro Thr Ser   Thr Ile Ser

```
Pro Ala    Val Ser Arg Leu Val    Thr Ser Met Val Thr    Ser Leu Ala
    10790                  10795                  10800

Ala Lys    Thr Ser Thr Thr Asn    Arg Ala Leu Thr Asn    Ser Pro Gly
    10805                  10810                  10815

Glu Pro    Ala Thr Thr Val Ser    Leu Val Thr His Pro    Ala Gln Thr
    10820                  10825                  10830

Ser Pro    Thr Val Pro Trp Thr    Thr Ser Ile Phe Phe    His Ser Lys
    10835                  10840                  10845

Ser Asp    Thr Thr Pro Ser Met    Thr Thr Ser His Gly    Ala Glu Ser
    10850                  10855                  10860

Ser Ser    Ala Val Pro Thr Pro    Thr Val Ser Thr Glu    Val Pro Gly
    10865                  10870                  10875

Val Val    Thr Pro Leu Val Thr    Ser Ser Arg Ala Val    Ile Ser Thr
    10880                  10885                  10890

Thr Ile    Pro Ile Leu Thr Leu    Ser Pro Gly Glu Pro    Glu Thr Thr
    10895                  10900                  10905

Pro Ser    Met Ala Thr Ser His    Gly Glu Glu Ala Ser    Ser Ala Ile
    10910                  10915                  10920

Pro Thr    Pro Thr Val Ser Pro    Gly Val Pro Gly Val    Val Thr Ser
    10925                  10930                  10935

Leu Val    Thr Ser Ser Arg Ala    Val Thr Ser Thr Thr    Ile Pro Ile
    10940                  10945                  10950

Leu Thr    Phe Ser Leu Gly Glu    Pro Glu Thr Thr Pro    Ser Met Ala
    10955                  10960                  10965

Thr Ser    His Gly Thr Glu Ala    Gly Ser Ala Val Pro    Thr Val Leu
    10970                  10975                  10980

Pro Glu    Val Pro Gly Met Val    Thr Ser Leu Val Ala    Ser Ser Arg
    10985                  10990                  10995

Ala Val    Thr Ser Thr Thr Leu    Pro Thr Leu Thr Leu    Ser Pro Gly
    11000                  11005                  11010

Glu Pro    Glu Thr Thr Pro Ser    Met Ala Thr Ser His    Gly Ala Glu
    11015                  11020                  11025
```

```
Ala Ser   Ser Thr Val Pro Thr   Val Ser Pro Glu Val   Pro Gly Val
    11030                11035                11040

Val Thr   Ser Leu Val Thr Ser   Ser Ser Gly Val Asn   Ser Thr Ser
    11045                11050                11055

Ile Pro   Thr Leu Ile Leu Ser   Pro Gly Glu Leu Glu   Thr Thr Pro
    11060                11065                11070

Ser Met   Ala Thr Ser His Gly   Ala Glu Ala Ser Ser   Ala Val Pro
    11075                11080                11085

Thr Pro   Thr Val Ser Pro Gly   Val Ser Gly Val Val   Thr Pro Leu
    11090                11095                11100

Val Thr   Ser Ser Arg Ala Val   Thr Ser Thr Thr Ile   Pro Ile Leu
    11105                11110                11115

Thr Leu   Ser Ser Ser Glu Pro   Glu Thr Thr Pro Ser   Met Ala Thr
    11120                11125                11130

Ser His   Gly Val Glu Ala Ser   Ser Ala Val Leu Thr   Val Ser Pro
    11135                11140                11145

Glu Val   Pro Gly Met Val Thr   Ser Leu Val Thr Ser   Ser Arg Ala
    11150                11155                11160

Val Thr   Ser Thr Thr Ile Pro   Thr Leu Thr Ile Ser   Ser Asp Glu
    11165                11170                11175

Pro Glu   Thr Thr Thr Ser Leu   Val Thr His Ser Glu   Ala Lys Met
    11180                11185                11190

Ile Ser   Ala Ile Pro Thr Leu   Ala Val Ser Pro Thr   Val Gln Gly
    11195                11200                11205

Leu Val   Thr Ser Leu Val Thr   Ser Ser Gly Ser Glu   Thr Ser Ala
    11210                11215                11220

Phe Ser   Asn Leu Thr Val Ala   Ser Ser Gln Pro Glu   Thr Ile Asp
    11225                11230                11235

Ser Trp   Val Ala His Pro Gly   Thr Glu Ala Ser Ser   Val Val Pro
    11240                11245                11250

Thr Leu   Thr Val Ser Thr Gly   Glu Pro Phe Thr Asn   Ile Ser Leu
    11255                11260                11265
```

435

```
Val Thr   His Pro Ala Glu Ser   Ser Ser Thr Leu Pro   Arg Thr Thr
    11270                 11275             11280

Ser Arg   Phe Ser His Ser Glu   Leu Asp Thr Met Pro   Ser Thr Val
    11285                 11290             11295

Thr Ser   Pro Glu Ala Glu Ser   Ser Ser Ala Ile Ser   Thr Thr Ile
    11300                 11305             11310

Ser Pro   Gly Ile Pro Gly Val   Leu Thr Ser Leu Val   Thr Ser Ser
    11315                 11320             11325

Gly Arg   Asp Ile Ser Ala Thr   Phe Pro Thr Val Pro   Glu Ser Pro
    11330                 11335             11340

His Glu   Ser Glu Ala Thr Ala   Ser Trp Val Thr His   Pro Ala Val
    11345                 11350             11355

Thr Ser   Thr Thr Val Pro Arg   Thr Thr Pro Asn Tyr   Ser His Ser
    11360                 11365             11370

Glu Pro   Asp Thr Thr Pro Ser   Ile Ala Thr Ser Pro   Gly Ala Glu
    11375                 11380             11385

Ala Thr   Ser Asp Phe Pro Thr   Ile Thr Val Ser Pro   Asp Val Pro
    11390                 11395             11400

Asp Met   Val Thr Ser Gln Val   Thr Ser Ser Gly Thr   Asp Thr Ser
    11405                 11410             11415

Ile Thr   Ile Pro Thr Leu Thr   Leu Ser Ser Gly Glu   Pro Glu Thr
    11420                 11425             11430

Thr Thr   Ser Phe Ile Thr Tyr   Ser Glu Thr His Thr   Ser Ser Ala
    11435                 11440             11445

Ile Pro   Thr Leu Pro Val Ser   Pro Gly Ala Ser Lys   Met Leu Thr
    11450                 11455             11460

Ser Leu   Val Ile Ser Ser Gly   Thr Asp Ser Thr Thr   Thr Phe Pro
    11465                 11470             11475

Thr Leu   Thr Glu Thr Pro Tyr   Glu Pro Glu Thr Thr   Ala Ile Gln
    11480                 11485             11490

Leu Ile   His Pro Ala Glu Thr   Asn Thr Met Val Pro   Lys Thr Thr
```

11495          11500          11505

Pro Lys Phe Ser His Ser Lys   Ser Asp Thr Thr Leu   Pro Val Ala
    11510          11515          11520

Ile Thr Ser Pro Gly Pro Glu   Ala Ser Ser Ala Val   Ser Thr Thr
    11525          11530          11535

Thr Ile Ser Pro Asp Met Ser   Asp Leu Val Thr Ser   Leu Val Pro
    11540          11545          11550

Ser Ser Gly Thr Asp Thr Ser   Thr Thr Phe Pro Thr   Leu Ser Glu
    11555          11560          11565

Thr Pro Tyr Glu Pro Glu Thr   Thr Val Thr Trp Leu   Thr His Pro
    11570          11575          11580

Ala Glu Thr Ser Thr Thr Val   Ser Gly Thr Ile Pro   Asn Phe Ser
    11585          11590          11595

His Arg Gly Ser Asp Thr Ala   Pro Ser Met Val Thr   Ser Pro Gly
    11600          11605          11610

Val Asp Thr Arg Ser Gly Val   Pro Thr Thr Thr Ile   Pro Pro Ser
    11615          11620          11625

Ile Pro Gly Val Val Thr Ser   Gln Val Thr Ser Ser   Ala Thr Asp
    11630          11635          11640

Thr Ser Thr Ala Ile Pro Thr   Leu Thr Pro Ser Pro   Gly Glu Pro
    11645          11650          11655

Glu Thr Thr Ala Ser Ser Ala   Thr His Pro Gly Thr   Gln Thr Gly
    11660          11665          11670

Phe Thr Val Pro Ile Arg Thr   Val Pro Ser Ser Glu   Pro Asp Thr
    11675          11680          11685

Met Ala Ser Trp Val Thr His   Pro Pro Gln Thr Ser   Thr Pro Val
    11690          11695          11700

Ser Arg Thr Thr Ser Ser Phe   Ser His Ser Ser Pro   Asp Ala Thr
    11705          11710          11715

Pro Val Met Ala Thr Ser Pro   Arg Thr Glu Ala Ser   Ser Ala Val
    11720          11725          11730

```
Leu Thr    Thr Ile Ser Pro Gly    Ala Pro Glu Met Val    Thr Ser Gln
    11735                  11740              11745

Ile Thr    Ser Ser Gly Ala Ala    Thr Ser Thr Thr Val    Pro Thr Leu
    11750                  11755              11760

Thr His    Ser Pro Gly Met Pro    Glu Thr Thr Ala Leu    Leu Ser Thr
    11765                  11770              11775

His Pro    Arg Thr Gly Thr Ser    Lys Thr Phe Pro Ala    Ser Thr Val
    11780                  11785              11790

Phe Pro    Gln Val Ser Glu Thr    Thr Ala Ser Leu Thr    Ile Arg Pro
    11795                  11800              11805

Gly Ala    Glu Thr Ser Thr Ala    Leu Pro Thr Gln Thr    Thr Ser Ser
    11810                  11815              11820

Leu Phe    Thr Leu Leu Val Thr    Gly Thr Ser Arg Val    Asp Leu Ser
    11825                  11830              11835

Pro Thr    Ala Ser Pro Gly Val    Ser Ala Lys Thr Ala    Pro Leu Ser
    11840                  11845              11850

Thr His    Pro Gly Thr Glu Thr    Ser Thr Met Ile Pro    Thr Ser Thr
    11855                  11860              11865

Leu Ser    Leu Gly Leu Leu Glu    Thr Thr Gly Leu Leu    Ala Thr Ser
    11870                  11875              11880

Ser Ser    Ala Glu Thr Ser Thr    Ser Thr Leu Thr Leu    Thr Val Ser
    11885                  11890              11895

Pro Ala    Val Ser Gly Leu Ser    Ser Ala Ser Ile Thr    Thr Asp Lys
    11900                  11905              11910

Pro Gln    Thr Val Thr Ser Trp    Asn Thr Glu Thr Ser    Pro Ser Val
    11915                  11920              11925

Thr Ser    Val Gly Pro Pro Glu    Phe Ser Arg Thr Val    Thr Gly Thr
    11930                  11935              11940

Thr Met    Thr Leu Ile Pro Ser    Glu Met Pro Thr Pro    Pro Lys Thr
    11945                  11950              11955

Ser His    Gly Glu Gly Val Ser    Pro Thr Thr Ile Leu    Arg Thr Thr
    11960                  11965              11970
```

438

Met Val Glu Ala Thr Asn Leu Ala Thr Thr Gly Ser Ser Pro Thr
11975 11980 11985

Val Ala Lys Thr Thr Thr Thr Phe Asn Thr Leu Ala Gly Ser Leu
11990 11995 12000

Phe Thr Pro Leu Thr Thr Pro Gly Met Ser Thr Leu Ala Ser Glu
12005 12010 12015

Ser Val Thr Ser Arg Thr Ser Tyr Asn His Arg Ser Trp Ile Ser
12020 12025 12030

Thr Thr Ser Ser Tyr Asn Arg Arg Tyr Trp Thr Pro Ala Thr Ser
12035 12040 12045

Thr Pro Val Thr Ser Thr Phe Ser Pro Gly Ile Ser Thr Ser Ser
12050 12055 12060

Ile Pro Ser Ser Thr Ala Ala Thr Val Pro Phe Met Val Pro Phe
12065 12070 12075

Thr Leu Asn Phe Thr Ile Thr Asn Leu Gln Tyr Glu Glu Asp Met
12080 12085 12090

Arg His Pro Gly Ser Arg Lys Phe Asn Ala Thr Glu Arg Glu Leu
12095 12100 12105

Gln Gly Leu Leu Lys Pro Leu Phe Arg Asn Ser Ser Leu Glu Tyr
12110 12115 12120

Leu Tyr Ser Gly Cys Arg Leu Ala Ser Leu Arg Pro Glu Lys Asp
12125 12130 12135

Ser Ser Ala Met Ala Val Asp Ala Ile Cys Thr His Arg Pro Asp
12140 12145 12150

Pro Glu Asp Leu Gly Leu Asp Arg Glu Arg Leu Tyr Trp Glu Leu
12155 12160 12165

Ser Asn Leu Thr Asn Gly Ile Gln Glu Leu Gly Pro Tyr Thr Leu
12170 12175 12180

Asp Arg Asn Ser Leu Tyr Val Asn Gly Phe Thr His Arg Ser Ser
12185 12190 12195

Met Pro Thr Thr Ser Thr Pro Gly Thr Ser Thr Val Asp Val Gly
12200 12205 12210

439

```
Thr Ser   Gly Thr Pro Ser Ser   Ser Pro Ser Pro Thr   Ala Ala Gly
    12215                 12220             12225

Pro Leu   Leu Met Pro Phe Thr   Leu Asn Phe Thr Ile   Thr Asn Leu
    12230                 12235             12240

Gln Tyr   Glu Glu Asp Met Arg   Arg Thr Gly Ser Arg   Lys Phe Asn
    12245                 12250             12255

Thr Met   Glu Ser Val Leu Gln   Gly Leu Leu Lys Pro   Leu Phe Lys
    12260                 12265             12270

Asn Thr   Ser Val Gly Pro Leu   Tyr Ser Gly Cys Arg   Leu Thr Leu
    12275                 12280             12285

Leu Arg   Pro Glu Lys Asp Gly   Ala Ala Thr Gly Val   Asp Ala Ile
    12290                 12295             12300

Cys Thr   His Arg Leu Asp Pro   Lys Ser Pro Gly Leu   Asn Arg Glu
    12305                 12310             12315

Gln Leu   Tyr Trp Glu Leu Ser   Lys Leu Thr Asn Asp   Ile Glu Glu
    12320                 12325             12330

Leu Gly   Pro Tyr Thr Leu Asp   Arg Asn Ser Leu Tyr   Val Asn Gly
    12335                 12340             12345

Phe Thr   His Gln Ser Ser Val   Ser Thr Thr Ser Thr   Pro Gly Thr
    12350                 12355             12360

Ser Thr   Val Asp Leu Arg Thr   Ser Gly Thr Pro Ser   Ser Leu Ser
    12365                 12370             12375

Ser Pro   Thr Ile Met Ala Ala   Gly Pro Leu Leu Val   Pro Phe Thr
    12380                 12385             12390

Leu Asn   Phe Thr Ile Thr Asn   Leu Gln Tyr Gly Glu   Asp Met Gly
    12395                 12400             12405

His Pro   Gly Ser Arg Lys Phe   Asn Thr Thr Glu Arg   Val Leu Gln
    12410                 12415             12420

Gly Leu   Leu Gly Pro Ile Phe   Lys Asn Thr Ser Val   Gly Pro Leu
    12425                 12430             12435

Tyr Ser   Gly Cys Arg Leu Thr   Ser Leu Arg Ser Glu   Lys Asp Gly
```

```
              12440                    12445                       12450


        Ala  Ala   Thr Gly Val Asp Ala   Ile Cys Ile His His   Leu Asp Pro
             12455                   12460               12465


        Lys  Ser   Pro Gly Leu Asn Arg   Glu Arg Leu Tyr Trp   Glu Leu Ser
             12470                   12475               12480


        Gln  Leu   Thr Asn Gly Ile Lys   Glu Leu Gly Pro Tyr   Thr Leu Asp
             12485                   12490               12495


        Arg  Asn   Ser Leu Tyr Val Asn   Gly Phe Thr His Arg   Thr Ser Val
             12500                   12505               12510


        Pro  Thr   Thr Ser Thr Pro Gly   Thr Ser Thr Val Asp   Leu Gly Thr
             12515                   12520               12525


        Ser  Gly   Thr Pro Phe Ser Leu   Pro Ser Pro Ala Thr   Ala Gly Pro
             12530                   12535               12540


        Leu  Leu   Val Leu Phe Thr Leu   Asn Phe Thr Ile Thr   Asn Leu Lys
             12545                   12550               12555


        Tyr  Glu   Glu Asp Met His Arg   Pro Gly Ser Arg Lys   Phe Asn Thr
             12560                   12565               12570


        Thr  Glu   Arg Val Leu Gln Thr   Leu Leu Gly Pro Met   Phe Lys Asn
             12575                   12580               12585


        Thr  Ser   Val Gly Leu Leu Tyr   Ser Gly Cys Arg Leu   Thr Leu Leu
             12590                   12595               12600


        Arg  Ser   Glu Lys Asp Gly Ala   Ala Thr Gly Val Asp   Ala Ile Cys
             12605                   12610               12615


        Thr  His   Arg Leu Asp Pro Lys   Ser Pro Gly Leu Asp   Arg Glu Gln
             12620                   12625               12630


        Leu  Tyr   Trp Glu Leu Ser Gln   Leu Thr Asn Gly Ile   Lys Glu Leu
             12635                   12640               12645


        Gly  Pro   Tyr Thr Leu Asp Arg   Asn Ser Leu Tyr Val   Asn Gly Phe
             12650                   12655               12660


        Thr  His   Trp Ile Pro Val Pro   Thr Ser Ser Thr Pro   Gly Thr Ser
             12665                   12670               12675
```

Thr Val Asp Leu Gly Ser Gly    Thr Pro Ser Ser Leu    Pro Ser Pro
    12680                12685                12690

Thr Ala Ala Gly Pro Leu Leu    Val Pro Phe Thr Leu    Asn Phe Thr
    12695                12700                12705

Ile Thr Asn Leu Gln Tyr Glu    Glu Asp Met His His    Pro Gly Ser
    12710                12715                12720

Arg Lys Phe Asn Thr Thr Glu    Arg Val Leu Gln Gly    Leu Leu Gly
    12725                12730                12735

Pro Met Phe Lys Asn Thr Ser    Val Gly Leu Leu Tyr    Ser Gly Cys
    12740                12745                12750

Arg Leu Thr Leu Leu Arg Ser    Glu Lys Asp Gly Ala    Ala Thr Gly
    12755                12760                12765

Val Asp Ala Ile Cys Thr His    Arg Leu Asp Pro Lys    Ser Pro Gly
    12770                12775                12780

Val Asp Arg Glu Gln Leu Tyr    Trp Glu Leu Ser Gln    Leu Thr Asn
    12785                12790                12795

Gly Ile Lys Glu Leu Gly Pro    Tyr Thr Leu Asp Arg    Asn Ser Leu
    12800                12805                12810

Tyr Val Asn Gly Phe Thr His    Gln Thr Ser Ala Pro    Asn Thr Ser
    12815                12820                12825

Thr Pro Gly Thr Ser Thr Val    Asp Leu Gly Thr Ser    Gly Thr Pro
    12830                12835                12840

Ser Ser Leu Pro Ser Pro Thr    Ser Ala Gly Pro Leu    Leu Val Pro
    12845                12850                12855

Phe Thr Leu Asn Phe Thr Ile    Thr Asn Leu Gln Tyr    Glu Glu Asp
    12860                12865                12870

Met Arg His Pro Gly Ser Arg    Lys Phe Asn Thr Thr    Glu Arg Val
    12875                12880                12885

Leu Gln Gly Leu Leu Lys Pro    Leu Phe Lys Ser Thr    Ser Val Gly
    12890                12895                12900

Pro Leu Tyr Ser Gly Cys Arg    Leu Thr Leu Leu Arg    Ser Glu Lys
    12905                12910                12915

```
Asp Gly Ala Ala Thr Gly Val   Asp Ala Ile Cys Thr   His Arg Leu
    12920             12925             12930

Asp Pro Lys Ser Pro Gly Val   Asp Arg Glu Gln Leu   Tyr Trp Glu
    12935             12940             12945

Leu Ser Gln Leu Thr Asn Gly   Ile Lys Glu Leu Gly   Pro Tyr Thr
    12950             12955             12960

Leu Asp Arg Asn Ser Leu Tyr   Val Asn Gly Phe Thr   His Gln Thr
    12965             12970             12975

Ser Ala Pro Asn Thr Ser Thr   Pro Gly Thr Ser Thr   Val Asp Leu
    12980             12985             12990

Gly Thr Ser Gly Thr Pro Ser   Ser Leu Pro Ser Pro   Thr Ser Ala
    12995             13000             13005

Gly Pro Leu Leu Val Pro Phe   Thr Leu Asn Phe Thr   Ile Thr Asn
    13010             13015             13020

Leu Gln Tyr Glu Glu Asp Met   His His Pro Gly Ser   Arg Lys Phe
    13025             13030             13035

Asn Thr Thr Glu Arg Val Leu   Gln Gly Leu Leu Gly   Pro Met Phe
    13040             13045             13050

Lys Asn Thr Ser Val Gly Leu   Leu Tyr Ser Gly Cys   Arg Leu Thr
    13055             13060             13065

Leu Leu Arg Pro Glu Lys Asn   Gly Ala Ala Thr Gly   Met Asp Ala
    13070             13075             13080

Ile Cys Ser His Arg Leu Asp   Pro Lys Ser Pro Gly   Leu Asn Arg
    13085             13090             13095

Glu Gln Leu Tyr Trp Glu Leu   Ser Gln Leu Thr His   Gly Ile Lys
    13100             13105             13110

Glu Leu Gly Pro Tyr Thr Leu   Asp Arg Asn Ser Leu   Tyr Val Asn
    13115             13120             13125

Gly Phe Thr His Arg Ser Ser   Val Ala Pro Thr Ser   Thr Pro Gly
    13130             13135             13140

Thr Ser Thr Val Asp Leu Gly   Thr Ser Gly Thr Pro   Ser Ser Leu
    13145             13150             13155
```

443

```
Pro Ser  Pro Thr Thr Ala Val  Pro Leu Leu Val Pro  Phe Thr Leu
    13160                13165            13170

Asn Phe  Thr Ile Thr Asn Leu  Gln Tyr Gly Glu Asp  Met Arg His
    13175                13180            13185

Pro Gly  Ser Arg Lys Phe Asn  Thr Thr Glu Arg Val  Leu Gln Gly
    13190                13195            13200

Leu Leu  Gly Pro Leu Phe Lys  Asn Ser Ser Val Gly  Pro Leu Tyr
    13205                13210            13215

Ser Gly  Cys Arg Leu Ile Ser  Leu Arg Ser Glu Lys  Asp Gly Ala
    13220                13225            13230

Ala Thr  Gly Val Asp Ala Ile  Cys Thr His His Leu  Asn Pro Gln
    13235                13240            13245

Ser Pro  Gly Leu Asp Arg Glu  Gln Leu Tyr Trp Gln  Leu Ser Gln
    13250                13255            13260

Met Thr  Asn Gly Ile Lys Glu  Leu Gly Pro Tyr Thr  Leu Asp Arg
    13265                13270            13275

Asn Ser  Leu Tyr Val Asn Gly  Phe Thr His Arg Ser  Ser Gly Leu
    13280                13285            13290

Thr Thr  Ser Thr Pro Trp Thr  Ser Thr Val Asp Leu  Gly Thr Ser
    13295                13300            13305

Gly Thr  Pro Ser Pro Val Pro  Ser Pro Thr Thr Ala  Gly Pro Leu
    13310                13315            13320

Leu Val  Pro Phe Thr Leu Asn  Phe Thr Ile Thr Asn  Leu Gln Tyr
    13325                13330            13335

Glu Glu  Asp Met His Arg Pro  Gly Ser Arg Lys Phe  Asn Thr Thr
    13340                13345            13350

Glu Arg  Val Leu Gln Gly Leu  Leu Ser Pro Ile Phe  Lys Asn Ser
    13355                13360            13365

Ser Val  Gly Pro Leu Tyr Ser  Gly Cys Arg Leu Thr  Ser Leu Arg
    13370                13375            13380

Pro Glu  Lys Asp Gly Ala Ala  Thr Gly Met Asp Ala  Val Cys Leu
```

444

13385                          13390                          13395

Tyr His Pro Asn Pro Lys Arg  Pro Gly Leu Asp Arg  Glu Gln Leu
    13400               13405               13410

Tyr Trp Glu Leu Ser Gln Leu  Thr His Asn Ile Thr  Glu Leu Gly
    13415               13420               13425

Pro Tyr Ser Leu Asp Arg Asp  Ser Leu Tyr Val Asn  Gly Phe Thr
    13430               13435               13440

His Gln Asn Ser Val Pro Thr  Thr Ser Thr Pro Gly  Thr Ser Thr
    13445               13450               13455

Val Tyr Trp Ala Thr Thr Gly  Thr Pro Ser Ser Phe  Pro Gly His
    13460               13465               13470

Thr Glu Pro Gly Pro Leu Leu  Ile Pro Phe Thr Phe  Asn Phe Thr
    13475               13480               13485

Ile Thr Asn Leu His Tyr Glu  Glu Asn Met Gln His  Pro Gly Ser
    13490               13495               13500

Arg Lys Phe Asn Thr Thr Glu  Arg Val Leu Gln Gly  Leu Leu Lys
    13505               13510               13515

Pro Leu Phe Lys Asn Thr Ser  Val Gly Pro Leu Tyr  Ser Gly Cys
    13520               13525               13530

Arg Leu Thr Ser Leu Arg Pro  Glu Lys Asp Gly Ala  Ala Thr Gly
    13535               13540               13545

Met Asp Ala Val Cys Leu Tyr  His Pro Asn Pro Lys  Arg Pro Gly
    13550               13555               13560

Leu Asp Arg Glu Gln Leu Tyr  Trp Glu Leu Ser Gln  Leu Thr His
    13565               13570               13575

Asn Ile Thr Glu Leu Gly Pro  Tyr Ser Leu Asp Arg  Asp Ser Leu
    13580               13585               13590

Tyr Val Asn Gly Phe Thr His  Gln Asn Ser Val Pro  Thr Thr Ser
    13595               13600               13605

Thr Pro Gly Thr Ser Thr Val  Tyr Trp Ala Thr Thr  Gly Thr Pro
    13610               13615               13620

445

```
Ser Ser   Phe Pro Gly His Thr   Glu Pro Gly Pro Leu   Leu Ile Pro
    13625                13630            13635

Phe Thr   Phe Asn Phe Thr Ile   Thr Asn Leu His Tyr   Glu Glu Asn
    13640                13645            13650

Met Gln   His Pro Gly Ser Arg   Lys Phe Asn Thr Thr   Glu Arg Val
    13655                13660            13665

Leu Gln   Gly Leu Leu Lys Pro   Leu Phe Lys Asn Thr   Ser Val Gly
    13670                13675            13680

Pro Leu   Tyr Ser Gly Cys Arg   Leu Thr Leu Leu Arg   Pro Glu Lys
    13685                13690            13695

His Glu   Ala Ala Thr Gly Val   Asp Thr Ile Cys Thr   His Arg Val
    13700                13705            13710

Asp Pro   Ile Gly Pro Gly Leu   Asp Arg Glu Arg Leu   Tyr Trp Glu
    13715                13720            13725

Leu Ser   Gln Leu Thr Asn Ser   Ile Thr Glu Leu Gly   Pro Tyr Thr
    13730                13735            13740

Leu Asp   Arg Asp Ser Leu Tyr   Val Asn Gly Phe Asn   Pro Arg Ser
    13745                13750            13755

Ser Val   Pro Thr Thr Ser Thr   Pro Gly Thr Ser Thr   Val His Leu
    13760                13765            13770

Ala Thr   Ser Gly Thr Pro Ser   Ser Leu Pro Gly His   Thr Ala Pro
    13775                13780            13785

Val Pro   Leu Leu Ile Pro Phe   Thr Leu Asn Phe Thr   Ile Thr Asn
    13790                13795            13800

Leu His   Tyr Glu Glu Asn Met   Gln His Pro Gly Ser   Arg Lys Phe
    13805                13810            13815

Asn Thr   Thr Glu Arg Val Leu   Gln Gly Leu Leu Lys   Pro Leu Phe
    13820                13825            13830

Lys Asn   Thr Ser Val Gly Pro   Leu Tyr Ser Gly Cys   Arg Leu Thr
    13835                13840            13845

Leu Leu   Arg Pro Glu Lys His   Glu Ala Ala Thr Gly   Val Asp Thr
    13850                13855            13860
```

446

EP 3 489 688 A1

```
Ile Cys    Thr His Arg Val Asp    Pro Ile Gly Pro Gly    Leu Xaa Xaa
    13865                  13870                  13875

Glu Xaa    Leu Tyr Trp Glu Leu    Ser Xaa Leu Thr Xaa    Xaa Ile Xaa
    13880                  13885                  13890

Glu Leu    Gly Pro Tyr Thr Leu    Asp Arg Xaa Ser Leu    Tyr Val Asn
    13895                  13900                  13905

Gly Phe    Thr His Xaa Xaa Ser    Xaa Pro Thr Thr Ser    Thr Pro Gly
    13910                  13915                  13920

Thr Ser    Thr Val Xaa Xaa Gly    Thr Ser Gly Thr Pro    Ser Ser Xaa
    13925                  13930                  13935

Pro Xaa    Xaa Thr Ser Ala Gly    Pro Leu Leu Val Pro    Phe Thr Leu
    13940                  13945                  13950

Asn Phe    Thr Ile Thr Asn Leu    Gln Tyr Glu Glu Asp    Met His His
    13955                  13960                  13965

Pro Gly    Ser Arg Lys Phe Asn    Thr Thr Glu Arg Val    Leu Gln Gly
    13970                  13975                  13980

Leu Leu    Gly Pro Met Phe Lys    Asn Thr Ser Val Gly    Leu Leu Tyr
    13985                  13990                  13995

Ser Gly    Cys Arg Leu Thr Leu    Leu Arg Pro Glu Lys    Asn Gly Ala
    14000                  14005                  14010

Ala Thr    Gly Met Asp Ala Ile    Cys Ser His Arg Leu    Asp Pro Lys
    14015                  14020                  14025

Ser Pro    Gly Leu Asp Arg Glu    Gln Leu Tyr Trp Glu    Leu Ser Gln
    14030                  14035                  14040

Leu Thr    His Gly Ile Lys Glu    Leu Gly Pro Tyr Thr    Leu Asp Arg
    14045                  14050                  14055

Asn Ser    Leu Tyr Val Asn Gly    Phe Thr His Arg Ser    Ser Val Ala
    14060                  14065                  14070

Pro Thr    Ser Thr Pro Gly Thr    Ser Thr Val Asp Leu    Gly Thr Ser
    14075                  14080                  14085

Gly Thr    Pro Ser Ser Leu Pro    Ser Pro Thr Thr Ala    Val Pro Leu
    14090                  14095                  14100
```

447

Leu Val Pro Phe Thr Leu Asn     Phe Thr Ile Thr Asn     Leu Gln Tyr
    14105            14110                14115

Gly Glu Asp Met Arg His Pro     Gly Ser Arg Lys Phe     Asn Thr Thr
    14120            14125                14130

Glu Arg Val Leu Gln Gly Leu     Leu Gly Pro Leu Phe     Lys Asn Ser
    14135            14140                14145

Ser Val Gly Pro Leu Tyr Ser     Gly Cys Arg Leu Ile     Ser Leu Arg
    14150            14155                14160

Ser Glu Lys Asp Gly Ala Ala     Thr Gly Val Asp Ala     Ile Cys Thr
    14165            14170                14175

His His Leu Asn Pro Gln Ser     Pro Gly Leu Asp Arg     Glu Gln Leu
    14180            14185                14190

Tyr Trp Gln Leu Ser Gln Met     Thr Asn Gly Ile Lys     Glu Leu Gly
    14195            14200                14205

Pro Tyr Thr Leu Asp Arg Asn     Ser Leu Tyr Val Asn     Gly Phe Thr
    14210            14215                14220

His Arg Ser Ser Gly Leu Thr     Thr Ser Thr Pro Trp     Thr Ser Thr
    14225            14230                14235

Val Asp Leu Gly Thr Ser Gly     Thr Pro Ser Pro Val     Pro Ser Pro
    14240            14245                14250

Thr Thr Ala Gly Pro Leu Leu     Val Pro Phe Thr Leu     Asn Phe Thr
    14255            14260                14265

Ile Thr Asn Leu Gln Tyr Glu     Glu Asp Met His Arg     Pro Gly Ser
    14270            14275                14280

Arg Lys Phe Asn Ala Thr Glu     Arg Val Leu Gln Gly     Leu Leu Ser
    14285            14290                14295

Pro Ile Phe Lys Asn Ser Ser     Val Gly Pro Leu Tyr     Ser Gly Cys
    14300            14305                14310

Arg Leu Thr Ser Leu Arg Pro     Glu Lys Asp Gly Ala     Ala Thr Gly
    14315            14320                14325

Met Asp Ala Val Cys Leu Tyr     His Pro Asn Pro Lys     Arg Pro Gly

448

14330                     14335                          14340

Leu Asp  Arg Glu Gln Leu Tyr   Trp Glu Leu Ser Gln   Leu Thr His
     14345                     14350                    14355

Asn Ile  Thr Glu Leu Gly Pro   Tyr Ser Leu Asp Arg   Asp Ser Leu
     14360                     14365                    14370

Tyr Val  Asn Gly Phe Thr His   Gln Ser Ser Met Thr   Thr Thr Arg
     14375                     14380                    14385

Thr Pro  Asp Thr Ser Thr Met   His Leu Ala Thr Ser   Arg Thr Pro
     14390                     14395                    14400

Ala Ser  Leu Ser Gly Pro Thr   Thr Ala Ser Pro Leu   Leu Val Leu
     14405                     14410                    14415

Phe Thr  Ile Asn Cys Thr Ile   Thr Asn Leu Gln Tyr   Glu Glu Asp
     14420                     14425                    14430

Met Arg  Arg Thr Gly Ser Arg   Lys Phe Asn Thr Met   Glu Ser Val
     14435                     14440                    14445

Leu Gln  Gly Leu Leu Lys Pro   Leu Phe Lys Asn Thr   Ser Val Gly
     14450                     14455                    14460

Pro Leu  Tyr Ser Gly Cys Arg   Leu Thr Leu Leu Arg   Pro Lys Lys
     14465                     14470                    14475

Asp Gly  Ala Ala Thr Gly Val   Asp Ala Ile Cys Thr   His Arg Leu
     14480                     14485                    14490

Asp Pro  Lys Ser Pro Gly Leu   Asn Arg Glu Gln Leu   Tyr Trp Glu
     14495                     14500                    14505

Leu Ser  Lys Leu Thr Asn Asp   Ile Glu Glu Leu Gly   Pro Tyr Thr
     14510                     14515                    14520

Leu Asp  Arg Asn Ser Leu Tyr   Val Asn Gly Phe Thr   His Gln Ser
     14525                     14530                    14535

Ser Val  Ser Thr Thr Ser Thr   Pro Gly Thr Ser Thr   Val Asp Leu
     14540                     14545                    14550

Arg Thr  Ser Gly Thr Pro Ser   Ser Leu Ser Ser Pro   Thr Ile Met
     14555                     14560                    14565

```
Xaa Xaa   Xaa Pro Leu Leu Xaa   Pro Phe Thr Xaa Asn   Xaa Thr Ile
    14570                 14575               14580

Thr Asn   Leu Xaa Xaa Xaa Xaa   Xaa Met Xaa Xaa Pro   Gly Ser Arg
    14585                 14590               14595

Lys Phe   Asn Thr Thr Glu Arg   Val Leu Gln Gly Leu   Leu Arg Pro
    14600                 14605               14610

Leu Phe   Lys Asn Thr Ser Val   Ser Ser Leu Tyr Ser   Gly Cys Arg
    14615                 14620               14625

Leu Thr   Leu Leu Arg Pro Glu   Lys Asp Gly Ala Ala   Thr Arg Val
    14630                 14635               14640

Asp Ala   Ala Cys Thr Tyr Arg   Pro Asp Pro Lys Ser   Pro Gly Leu
    14645                 14650               14655

Asp Arg   Glu Gln Leu Tyr Trp   Glu Leu Ser Gln Leu   Thr His Ser
    14660                 14665               14670

Ile Thr   Glu Leu Gly Pro Tyr   Thr Leu Asp Arg Val   Ser Leu Tyr
    14675                 14680               14685

Val Asn   Gly Phe Asn Pro Arg   Ser Ser Val Pro Thr   Thr Ser Thr
    14690                 14695               14700

Pro Gly   Thr Ser Thr Val His   Leu Ala Thr Ser Gly   Thr Pro Ser
    14705                 14710               14715

Ser Leu   Pro Gly His Thr Xaa   Xaa Xaa Pro Leu Leu   Xaa Pro Phe
    14720                 14725               14730

Thr Xaa   Asn Xaa Thr Ile Thr   Asn Leu Xaa Xaa Xaa   Xaa Xaa Met
    14735                 14740               14745

Xaa Xaa   Pro Gly Ser Arg Lys   Phe Asn Thr Thr Glu   Arg Val Leu
    14750                 14755               14760

Gln Gly   Leu Leu Lys Pro Leu   Phe Arg Asn Ser Ser   Leu Glu Tyr
    14765                 14770               14775

Leu Tyr   Ser Gly Cys Arg Leu   Ala Ser Leu Arg Pro   Glu Lys Asp
    14780                 14785               14790

Ser Ser   Ala Met Ala Val Asp   Ala Ile Cys Thr His   Arg Pro Asp
    14795                 14800               14805
```

```
Pro Glu   Asp Leu Gly Leu Asp   Arg Glu Arg Leu Tyr   Trp Glu Leu
    14810                 14815                 14820

Ser Asn   Leu Thr Asn Gly Ile   Gln Glu Leu Gly Pro   Tyr Thr Leu
    14825                 14830                 14835

Asp Arg   Asn Ser Leu Tyr Val   Asn Gly Phe Thr His   Arg Ser Ser
    14840                 14845                 14850

Gly Leu   Thr Thr Ser Thr Pro   Trp Thr Ser Thr Val   Asp Leu Gly
    14855                 14860                 14865

Thr Ser   Gly Thr Pro Ser Pro   Val Pro Ser Pro Thr   Thr Ala Gly
    14870                 14875                 14880

Pro Leu   Leu Val Pro Phe Thr   Leu Asn Phe Thr Ile   Thr Asn Leu
    14885                 14890                 14895

Gln Tyr   Glu Glu Asp Met His   Arg Pro Gly Ser Arg   Arg Phe Asn
    14900                 14905                 14910

Thr Thr   Glu Arg Val Leu Gln   Gly Leu Leu Thr Pro   Leu Phe Lys
    14915                 14920                 14925

Asn Thr   Ser Val Gly Pro Leu   Tyr Ser Gly Cys Arg   Leu Thr Leu
    14930                 14935                 14940

Leu Arg   Pro Glu Lys Gln Glu   Ala Ala Thr Gly Val   Asp Thr Ile
    14945                 14950                 14955

Cys Thr   His Arg Val Asp Pro   Ile Gly Pro Gly Leu   Asp Arg Glu
    14960                 14965                 14970

Arg Leu   Tyr Trp Glu Leu Ser   Gln Leu Thr Asn Ser   Ile Thr Glu
    14975                 14980                 14985

Leu Gly   Pro Tyr Thr Leu Asp   Arg Asp Ser Leu Tyr   Val Asn Gly
    14990                 14995                 15000

Phe Asn   Pro Trp Ser Ser Val   Pro Thr Thr Ser Thr   Pro Gly Thr
    15005                 15010                 15015

Ser Thr   Val His Leu Ala Thr   Ser Gly Thr Pro Ser   Ser Leu Pro
    15020                 15025                 15030

Gly His   Thr Ala Pro Val Pro   Leu Leu Ile Pro Phe   Thr Leu Asn
    15035                 15040                 15045
```

451

```
Phe Thr   Ile Thr Asp Leu His   Tyr Glu Glu Asn Met   Gln His Pro
    15050             15055             15060

Gly Ser   Arg Lys Phe Asn Thr   Thr Glu Arg Val Leu   Gln Gly Leu
    15065             15070             15075

Leu Lys   Pro Leu Phe Lys Ser   Thr Ser Val Gly Pro   Leu Tyr Ser
    15080             15085             15090

Gly Cys   Arg Leu Thr Leu Leu   Arg Pro Glu Lys His   Gly Ala Ala
    15095             15100             15105

Thr Gly   Val Asp Ala Ile Cys   Thr Leu Arg Leu Asp   Pro Thr Gly
    15110             15115             15120

Pro Gly   Leu Asp Arg Glu Arg   Leu Tyr Trp Glu Leu   Ser Gln Leu
    15125             15130             15135

Thr Asn   Ser Val Thr Glu Leu   Gly Pro Tyr Thr Leu   Asp Arg Asp
    15140             15145             15150

Ser Leu   Tyr Val Asn Gly Phe   Thr His Arg Ser Ser   Val Pro Thr
    15155             15160             15165

Thr Ser   Ile Pro Gly Thr Ser   Ala Val His Leu Glu   Thr Ser Gly
    15170             15175             15180

Thr Pro   Ala Ser Leu Pro Gly   His Thr Ala Pro Gly   Pro Leu Leu
    15185             15190             15195

Val Pro   Phe Thr Leu Asn Phe   Thr Ile Thr Asn Leu   Gln Tyr Glu
    15200             15205             15210

Glu Asp   Met Arg His Pro Gly   Ser Arg Lys Phe Ser   Thr Thr Glu
    15215             15220             15225

Arg Val   Leu Gln Gly Leu Leu   Lys Pro Leu Phe Lys   Asn Thr Ser
    15230             15235             15240

Val Ser   Ser Leu Tyr Ser Gly   Cys Arg Leu Thr Leu   Leu Arg Pro
    15245             15250             15255

Glu Lys   Asp Gly Ala Ala Thr   Arg Val Asp Ala Val   Cys Thr His
    15260             15265             15270

Arg Pro   Asp Pro Lys Ser Pro   Gly Leu Asp Arg Glu   Arg Leu Tyr
```

452

```
         15275                    15280                    15285


Trp Lys Leu Ser Gln Leu Thr  His Gly Ile Thr Glu  Leu Gly Pro
    15290                    15295                15300


Tyr Thr Leu Asp Arg His Ser  Leu Tyr Val Asn Gly  Phe Thr His
    15305                    15310                15315


Gln Ser Ser Met Thr Thr Thr  Arg Thr Pro Asp Thr  Ser Thr Met
    15320                    15325                15330


His Leu Ala Thr Ser Arg Thr  Pro Ala Ser Leu Ser  Gly Pro Thr
    15335                    15340                15345


Thr Ala Ser Pro Leu Leu Val  Leu Phe Thr Ile Asn  Phe Thr Ile
    15350                    15355                15360


Thr Asn Leu Arg Tyr Glu Glu  Asn Met His His Pro  Gly Ser Arg
    15365                    15370                15375


Lys Phe Asn Thr Thr Glu Arg  Val Leu Gln Gly Leu  Leu Arg Pro
    15380                    15385                15390


Val Phe Lys Asn Thr Ser Val  Gly Pro Leu Tyr Ser  Gly Cys Arg
    15395                    15400                15405


Leu Thr Thr Leu Arg Pro Lys  Lys Asp Gly Ala Ala  Thr Lys Val
    15410                    15415                15420


Asp Ala Ile Cys Thr Tyr Arg  Pro Asp Pro Lys Ser  Pro Gly Leu
    15425                    15430                15435


Asp Arg Glu Gln Leu Tyr Trp  Glu Leu Ser Gln Leu  Thr His Ser
    15440                    15445                15450


Ile Thr Glu Leu Gly Pro Tyr  Thr Gln Asp Arg Asp  Ser Leu Tyr
    15455                    15460                15465


Val Asn Gly Phe Thr His Arg  Ser Ser Val Pro Thr  Thr Ser Ile
    15470                    15475                15480


Pro Gly Thr Ser Ala Val His  Leu Glu Thr Ser Gly  Thr Pro Ala
    15485                    15490                15495


Ser Leu Pro Gly His Thr Ala  Pro Gly Pro Leu Leu  Val Pro Phe
    15500                    15505                15510
```

```
Thr Leu Asn Phe Thr Ile Thr   Asn Leu Gln Tyr Glu   Glu Asp Met
    15515               15520               15525

Arg His Pro Gly Ser Arg Lys   Phe Asn Thr Thr Glu   Arg Val Leu
    15530               15535               15540

Gln Gly Leu Leu Lys Pro Leu   Phe Lys Ser Thr Ser   Val Gly Pro
    15545               15550               15555

Leu Tyr Ser Gly Cys Arg Leu   Thr Leu Leu Arg Pro   Glu Lys Arg
    15560               15565               15570

Gly Ala Ala Thr Gly Val Asp   Thr Ile Cys Thr His   Arg Leu Asp
    15575               15580               15585

Pro Leu Asn Pro Gly Leu Asp   Arg Glu Gln Leu Tyr   Trp Glu Leu
    15590               15595               15600

Ser Lys Leu Thr Arg Gly Ile   Ile Glu Leu Gly Pro   Tyr Leu Leu
    15605               15610               15615

Asp Arg Gly Ser Leu Tyr Val   Asn Gly Phe Thr His   Arg Thr Ser
    15620               15625               15630

Val Pro Thr Thr Ser Thr Pro   Gly Thr Ser Thr Val   Asp Leu Gly
    15635               15640               15645

Thr Ser Gly Thr Pro Phe Ser   Leu Pro Ser Pro Ala   Xaa Xaa Xaa
    15650               15655               15660

Pro Leu Leu Xaa Pro Phe Thr   Xaa Asn Xaa Thr Ile   Thr Asn Leu
    15665               15670               15675

Xaa Xaa Xaa Xaa Xaa Met Xaa   Xaa Pro Gly Ser Arg   Lys Phe Asn
    15680               15685               15690

Thr Thr Glu Arg Val Leu Gln   Thr Leu Leu Gly Pro   Met Phe Lys
    15695               15700               15705

Asn Thr Ser Val Gly Leu Leu   Tyr Ser Gly Cys Arg   Leu Thr Leu
    15710               15715               15720

Leu Arg Ser Glu Lys Asp Gly   Ala Ala Thr Gly Val   Asp Ala Ile
    15725               15730               15735

Cys Thr His Arg Leu Asp Pro   Lys Ser Pro Gly Val   Asp Arg Glu
    15740               15745               15750
```

454

```
Gln Leu   Tyr Trp Glu Leu Ser   Gln Leu Thr Asn Gly   Ile Lys Glu
    15755             15760               15765

Leu Gly   Pro Tyr Thr Leu Asp   Arg Asn Ser Leu Tyr   Val Asn Gly
    15770             15775               15780

Phe Thr   His Trp Ile Pro Val   Pro Thr Ser Ser Thr   Pro Gly Thr
    15785             15790               15795

Ser Thr   Val Asp Leu Gly Ser   Gly Thr Pro Ser Ser   Leu Pro Ser
    15800             15805               15810

Pro Thr   Thr Ala Gly Pro Leu   Leu Val Pro Phe Thr   Leu Asn Phe
    15815             15820               15825

Thr Ile   Thr Asn Leu Lys Tyr   Glu Glu Asp Met His   Cys Pro Gly
    15830             15835               15840

Ser Arg   Lys Phe Asn Thr Thr   Glu Arg Val Leu Gln   Ser Leu Leu
    15845             15850               15855

Gly Pro   Met Phe Lys Asn Thr   Ser Val Gly Pro Leu   Tyr Ser Gly
    15860             15865               15870

Cys Arg   Leu Thr Leu Leu Arg   Ser Glu Lys Asp Gly   Ala Ala Thr
    15875             15880               15885

Gly Val   Asp Ala Ile Cys Thr   His Arg Leu Asp Pro   Lys Ser Pro
    15890             15895               15900

Gly Val   Asp Arg Glu Gln Leu   Tyr Trp Glu Leu Ser   Gln Leu Thr
    15905             15910               15915

Asn Gly   Ile Lys Glu Leu Gly   Pro Tyr Thr Leu Asp   Arg Asn Ser
    15920             15925               15930

Leu Tyr   Val Asn Gly Phe Thr   His Gln Thr Ser Ala   Pro Asn Thr
    15935             15940               15945

Ser Thr   Pro Gly Thr Ser Thr   Val Asp Leu Gly Thr   Ser Gly Thr
    15950             15955               15960

Pro Ser   Ser Leu Pro Ser Pro   Thr Xaa Xaa Xaa Pro   Leu Leu Xaa
    15965             15970               15975

Pro Phe   Thr Xaa Asn Xaa Thr   Ile Thr Asn Leu Xaa   Xaa Xaa Xaa
    15980             15985               15990
```

```
Xaa Met     Xaa Xaa Pro Gly Ser     Arg Lys Phe Asn Thr     Thr Glu Xaa
    15995                   16000                   16005

Val Leu     Gln Gly Leu Leu Xaa     Pro Xaa Phe Lys Asn     Xaa Ser Val
    16010                   16015                   16020

Gly Xaa     Leu Tyr Ser Gly Cys     Arg Leu Thr Xaa Leu     Arg Xaa Glu
    16025                   16030                   16035

Lys Xaa     Gly Ala Ala Thr Gly     Xaa Asp Ala Ile Cys     Xaa His Xaa
    16040                   16045                   16050

Xaa Xaa     Pro Lys Xaa Pro Gly     Leu Xaa Xaa Glu Xaa     Leu Tyr Trp
    16055                   16060                   16065

Glu Leu     Ser Xaa Leu Thr Xaa     Xaa Ile Xaa Glu Leu     Gly Pro Tyr
    16070                   16075                   16080

Thr Leu     Asp Arg Xaa Ser Leu     Tyr Val Asn Gly Phe     Thr His Trp
    16085                   16090                   16095

Ile Pro     Val Pro Thr Ser Ser     Thr Pro Gly Thr Ser     Thr Val Asp
    16100                   16105                   16110

Leu Gly     Ser Gly Thr Pro Ser     Ser Leu Pro Ser Pro     Thr Thr Ala
    16115                   16120                   16125

Gly Pro     Leu Leu Val Pro Phe     Thr Leu Asn Phe Thr     Ile Thr Asn
    16130                   16135                   16140

Leu Lys     Tyr Glu Glu Asp Met     His Cys Pro Gly Ser     Arg Lys Phe
    16145                   16150                   16155

Asn Thr     Thr Glu Arg Val Leu     Gln Ser Leu Leu Gly     Pro Met Phe
    16160                   16165                   16170

Lys Asn     Thr Ser Val Gly Pro     Leu Tyr Ser Gly Cys     Arg Leu Thr
    16175                   16180                   16185

Ser Leu     Arg Ser Glu Lys Asp     Gly Ala Ala Thr Gly     Val Asp Ala
    16190                   16195                   16200

Ile Cys     Thr His Arg Val Asp     Pro Lys Ser Pro Gly     Val Asp Arg
    16205                   16210                   16215

Glu Gln     Leu Tyr Trp Glu Leu     Ser Gln Leu Thr Asn     Gly Ile Lys
```

```
          16220                  16225                  16230

      Glu Leu  Gly Pro Tyr Thr Leu  Asp Arg Asn Ser Leu  Tyr Val Asn
          16235                  16240                  16245

      Gly Phe  Thr His Gln Thr Ser  Ala Pro Asn Thr Ser  Thr Pro Gly
          16250                  16255                  16260

      Thr Ser  Thr Val Xaa Xaa Gly  Thr Ser Gly Thr Pro  Ser Ser Xaa
          16265                  16270                  16275

      Pro Xaa  Xaa Thr Ser Ala Gly  Pro Leu Leu Val Pro  Phe Thr Leu
          16280                  16285                  16290

      Asn Phe  Thr Ile Thr Asn Leu  Gln Tyr Glu Glu Asp  Met His His
          16295                  16300                  16305

      Pro Gly  Ser Arg Lys Phe Asn  Thr Thr Glu Arg Val  Leu Gln Gly
          16310                  16315                  16320

      Leu Leu  Gly Pro Met Phe Lys  Asn Thr Ser Val Gly  Leu Leu Tyr
          16325                  16330                  16335

      Ser Gly  Cys Arg Leu Thr Leu  Leu Arg Pro Glu Lys  Asn Gly Ala
          16340                  16345                  16350

      Thr Thr  Gly Met Asp Ala Ile  Cys Thr His Arg Leu  Asp Pro Lys
          16355                  16360                  16365

      Ser Pro  Gly Leu Xaa Xaa Glu  Xaa Leu Tyr Trp Glu  Leu Ser Xaa
          16370                  16375                  16380

      Leu Thr  Xaa Xaa Ile Xaa Glu  Leu Gly Pro Tyr Thr  Leu Asp Arg
          16385                  16390                  16395

      Xaa Ser  Leu Tyr Val Asn Gly  Phe Thr His Xaa Xaa  Ser Xaa Pro
          16400                  16405                  16410

      Thr Thr  Ser Thr Pro Gly Thr  Ser Thr Val Xaa Xaa  Gly Thr Ser
          16415                  16420                  16425

      Gly Thr  Pro Ser Ser Xaa Pro  Xaa Xaa Thr Xaa Xaa  Xaa Pro Leu
          16430                  16435                  16440

      Leu Xaa  Pro Phe Thr Xaa Asn  Xaa Thr Ile Thr Asn  Leu Xaa Xaa
          16445                  16450                  16455
```

```
Xaa Xaa   Xaa Met Xaa Xaa Pro   Gly Ser Arg Lys Phe   Asn Thr Thr
    16460             16465             16470

Glu Arg   Val Leu Gln Gly Leu   Leu Lys Pro Leu Phe   Arg Asn Ser
    16475             16480             16485

Ser Leu   Glu Tyr Leu Tyr Ser   Gly Cys Arg Leu Ala   Ser Leu Arg
    16490             16495             16500

Pro Glu   Lys Asp Ser Ser Ala   Met Ala Val Asp Ala   Ile Cys Thr
    16505             16510             16515

His Arg   Pro Asp Pro Glu Asp   Leu Gly Leu Asp Arg   Glu Arg Leu
    16520             16525             16530

Tyr Trp   Glu Leu Ser Asn Leu   Thr Asn Gly Ile Gln   Glu Leu Gly
    16535             16540             16545

Pro Tyr   Thr Leu Asp Arg Asn   Ser Leu Tyr Val Asn   Gly Phe Thr
    16550             16555             16560

His Arg   Ser Ser Met Pro Thr   Thr Ser Thr Pro Gly   Thr Ser Thr
    16565             16570             16575

Val Asp   Val Gly Thr Ser Gly   Thr Pro Ser Ser Ser   Pro Ser Pro
    16580             16585             16590

Thr Thr   Ala Gly Pro Leu Leu   Ile Pro Phe Thr Leu   Asn Phe Thr
    16595             16600             16605

Ile Thr   Asn Leu Gln Tyr Gly   Glu Asp Met Gly His   Pro Gly Ser
    16610             16615             16620

Arg Lys   Phe Asn Thr Thr Glu   Arg Val Leu Gln Gly   Leu Leu Gly
    16625             16630             16635

Pro Ile   Phe Lys Asn Thr Ser   Val Gly Pro Leu Tyr   Ser Gly Cys
    16640             16645             16650

Arg Leu   Thr Ser Leu Arg Ser   Glu Lys Asp Gly Ala   Ala Thr Gly
    16655             16660             16665

Val Asp   Ala Ile Cys Ile His   His Leu Asp Pro Lys   Ser Pro Gly
    16670             16675             16680

Leu Asn   Arg Glu Arg Leu Tyr   Trp Glu Leu Ser Gln   Leu Thr Asn
    16685             16690             16695
```

EP 3 489 688 A1

Gly Ile Lys Glu Leu Gly Pro Tyr Thr Leu Asp Arg Asn Ser Leu
16700 16705 16710

Tyr Val Asn Gly Phe Thr His Arg Thr Ser Val Pro Thr Thr Ser
16715 16720 16725

Thr Pro Gly Thr Ser Thr Val Asp Leu Gly Thr Ser Gly Thr Pro
16730 16735 16740

Phe Ser Leu Pro Ser Pro Ala Thr Ala Gly Pro Leu Leu Val Leu
16745 16750 16755

Phe Thr Leu Asn Phe Thr Ile Thr Asn Leu Lys Tyr Glu Glu Asp
16760 16765 16770

Met His Arg Pro Gly Ser Arg Lys Phe Asn Thr Thr Glu Arg Val
16775 16780 16785

Leu Gln Thr Leu Leu Gly Pro Met Phe Lys Asn Thr Ser Val Gly
16790 16795 16800

Leu Leu Tyr Ser Gly Cys Arg Leu Thr Leu Leu Arg Ser Glu Lys
16805 16810 16815

Asp Gly Ala Ala Thr Gly Val Asp Ala Ile Cys Thr His Arg Leu
16820 16825 16830

Asp Pro Lys Ser Pro Gly Leu Xaa Xaa Glu Xaa Leu Tyr Trp Glu
16835 16840 16845

Leu Ser Xaa Leu Thr Xaa Xaa Ile Xaa Glu Leu Gly Pro Tyr Thr
16850 16855 16860

Leu Asp Arg Xaa Ser Leu Tyr Val Asn Gly Phe Thr His Xaa Xaa
16865 16870 16875

Ser Xaa Pro Thr Thr Ser Thr Pro Gly Thr Ser Thr Val Xaa Xaa
16880 16885 16890

Gly Thr Ser Gly Thr Pro Ser Ser Xaa Pro Xaa Xaa Thr Xaa Xaa
16895 16900 16905

Xaa Pro Leu Leu Xaa Pro Phe Thr Xaa Asn Xaa Thr Ile Thr Asn
16910 16915 16920

Leu Xaa Xaa Xaa Xaa Xaa Met Xaa Xaa Pro Gly Ser Arg Lys Phe
16925 16930 16935

459

```
Asn Thr   Thr Glu Arg Val Leu   Gln Gly Leu Leu Arg   Pro Val Phe
    16940             16945               16950

Lys Asn   Thr Ser Val Gly Pro   Leu Tyr Ser Gly Cys   Arg Leu Thr
    16955             16960               16965

Leu Leu   Arg Pro Lys Lys Asp   Gly Ala Ala Thr Lys   Val Asp Ala
    16970             16975               16980

Ile Cys   Thr Tyr Arg Pro Asp   Pro Lys Ser Pro Gly   Leu Asp Arg
    16985             16990               16995

Glu Gln   Leu Tyr Trp Glu Leu   Ser Gln Leu Thr His   Ser Ile Thr
    17000             17005               17010

Glu Leu   Gly Pro Tyr Thr Gln   Asp Arg Asp Ser Leu   Tyr Val Asn
    17015             17020               17025

Gly Phe   Thr His Arg Ser Ser   Val Pro Thr Thr Ser   Ile Pro Gly
    17030             17035               17040

Thr Ser   Ala Val His Leu Glu   Thr Thr Gly Thr Pro   Ser Ser Phe
    17045             17050               17055

Pro Gly   His Thr Glu Pro Gly   Pro Leu Leu Ile Pro   Phe Thr Phe
    17060             17065               17070

Asn Phe   Thr Ile Thr Asn Leu   Arg Tyr Glu Glu Asn   Met Gln His
    17075             17080               17085

Pro Gly   Ser Arg Lys Phe Asn   Thr Thr Glu Arg Val   Leu Gln Gly
    17090             17095               17100

Leu Leu   Thr Pro Leu Phe Lys   Asn Thr Ser Val Gly   Pro Leu Tyr
    17105             17110               17115

Ser Gly   Cys Arg Leu Thr Leu   Leu Arg Pro Glu Lys   Gln Glu Ala
    17120             17125               17130

Ala Thr   Gly Val Asp Thr Ile   Cys Thr His Arg Val   Asp Pro Ile
    17135             17140               17145

Gly Pro   Gly Leu Asp Arg Glu   Arg Leu Tyr Trp Glu   Leu Ser Gln
    17150             17155               17160

Leu Thr   Asn Ser Ile Thr Glu   Leu Gly Pro Tyr Thr   Leu Asp Arg
```

460

17165　　　　　　　　17170　　　　　　　　17175

Asp Ser Leu Tyr Val Asp Gly Phe Asn Pro Trp Ser Ser Val Pro
17180　　　　　　　　17185　　　　　　　　17190

Thr Thr Ser Thr Pro Gly Thr Ser Thr Val His Leu Ala Thr Ser
17195　　　　　　　　17200　　　　　　　　17205

Gly Thr Pro Ser Pro Leu Pro Gly His Thr Ala Pro Val Pro Leu
17210　　　　　　　　17215　　　　　　　　17220

Leu Ile Pro Phe Thr Leu Asn Phe Thr Ile Thr Asp Leu His Tyr
17225　　　　　　　　17230　　　　　　　　17235

Glu Glu Asn Met Gln His Pro Gly Ser Arg Lys Phe Asn Thr Thr
17240　　　　　　　　17245　　　　　　　　17250

Glu Arg Val Leu Gln Gly Leu Leu Lys Pro Leu Phe Lys Ser Thr
17255　　　　　　　　17260　　　　　　　　17265

Ser Val Gly Pro Leu Tyr Ser Gly Cys Arg Leu Thr Leu Leu Arg
17270　　　　　　　　17275　　　　　　　　17280

Pro Glu Lys His Gly Ala Ala Thr Gly Val Asp Ala Ile Cys Thr
17285　　　　　　　　17290　　　　　　　　17295

Leu Arg Leu Asp Pro Thr Gly Pro Gly Leu Asp Arg Glu Arg Leu
17300　　　　　　　　17305　　　　　　　　17310

Tyr Trp Glu Leu Ser Gln Leu Thr Asn Ser Ile Thr Glu Leu Gly
17315　　　　　　　　17320　　　　　　　　17325

Pro Tyr Thr Leu Asp Arg Asp Ser Leu Tyr Val Asn Gly Phe Asn
17330　　　　　　　　17335　　　　　　　　17340

Pro Trp Ser Ser Val Pro Thr Thr Ser Thr Pro Gly Thr Ser Thr
17345　　　　　　　　17350　　　　　　　　17355

Val His Leu Ala Thr Ser Gly Thr Pro Ser Ser Leu Pro Gly His
17360　　　　　　　　17365　　　　　　　　17370

Thr Thr Ala Gly Pro Leu Leu Val Pro Phe Thr Leu Asn Phe Thr
17375　　　　　　　　17380　　　　　　　　17385

Ile Thr Asn Leu Lys Tyr Glu Glu Asp Met His Cys Pro Gly Ser
17390　　　　　　　　17395　　　　　　　　17400

```
Arg Lys Phe Asn Thr Thr Glu   Arg Val Leu Gln Ser   Leu His Gly
    17405             17410             17415

Pro Met Phe Lys Asn Thr Ser   Val Gly Pro Leu Tyr   Ser Gly Cys
    17420             17425             17430

Arg Leu Thr Leu Leu Arg Ser   Glu Lys Asp Gly Ala   Ala Thr Gly
    17435             17440             17445

Val Asp Ala Ile Cys Thr His   Arg Leu Asp Pro Lys   Ser Pro Gly
    17450             17455             17460

Leu Xaa Xaa Glu Xaa Leu Tyr   Trp Glu Leu Ser Xaa   Leu Thr Xaa
    17465             17470             17475

Xaa Ile Xaa Glu Leu Gly Pro   Tyr Thr Leu Asp Arg   Xaa Ser Leu
    17480             17485             17490

Tyr Val Asn Gly Phe Thr His   Xaa Xaa Ser Xaa Pro   Thr Thr Ser
    17495             17500             17505

Thr Pro Gly Thr Ser Thr Val   Xaa Xaa Gly Thr Ser   Gly Thr Pro
    17510             17515             17520

Ser Ser Xaa Pro Xaa Xaa Thr   Xaa Xaa Xaa Pro Leu   Leu Xaa Pro
    17525             17530             17535

Phe Thr Xaa Asn Xaa Thr Ile   Thr Asn Leu Xaa Xaa   Xaa Xaa Xaa
    17540             17545             17550

Met Xaa Xaa Pro Gly Ser Arg   Lys Phe Asn Thr Thr   Glu Xaa Val
    17555             17560             17565

Leu Gln Gly Leu Leu Xaa Pro   Xaa Phe Lys Asn Xaa   Ser Val Gly
    17570             17575             17580

Xaa Leu Tyr Ser Gly Cys Arg   Leu Thr Xaa Leu Arg   Xaa Glu Lys
    17585             17590             17595

Xaa Gly Ala Ala Thr Gly Xaa   Asp Ala Ile Cys Xaa   His Xaa Xaa
    17600             17605             17610

Xaa Pro Lys Xaa Pro Gly Leu   Xaa Xaa Glu Xaa Leu   Tyr Trp Glu
    17615             17620             17625

Leu Ser Xaa Leu Thr Asn Ser   Ile Thr Glu Leu Gly   Pro Tyr Thr
    17630             17635             17640
```

```
Leu Asp   Arg Asp Ser Leu Tyr   Val Asn Gly Phe Thr   His Arg Ser
    17645                 17650                 17655

Ser Met   Pro Thr Thr Ser Ile   Pro Gly Thr Ser Ala   Val His Leu
    17660                 17665                 17670

Glu Thr   Ser Gly Thr Pro Ala   Ser Leu Pro Gly His   Thr Ala Pro
    17675                 17680                 17685

Gly Pro   Leu Leu Val Pro Phe   Thr Leu Asn Phe Thr   Ile Thr Asn
    17690                 17695                 17700

Leu Gln   Tyr Glu Glu Asp Met   Arg His Pro Gly Ser   Arg Lys Phe
    17705                 17710                 17715

Asn Thr   Thr Glu Arg Val Leu   Gln Gly Leu Leu Lys   Pro Leu Phe
    17720                 17725                 17730

Lys Ser   Thr Ser Val Gly Pro   Leu Tyr Ser Gly Cys   Arg Leu Thr
    17735                 17740                 17745

Leu Leu   Arg Pro Glu Lys Arg   Gly Ala Ala Thr Gly   Val Asp Thr
    17750                 17755                 17760

Ile Cys   Thr His Arg Leu Asp   Pro Leu Asn Pro Gly   Leu Xaa Xaa
    17765                 17770                 17775

Glu Xaa   Leu Tyr Trp Glu Leu   Ser Xaa Leu Thr Xaa   Xaa Ile Xaa
    17780                 17785                 17790

Glu Leu   Gly Pro Tyr Thr Leu   Asp Arg Xaa Ser Leu   Tyr Val Asn
    17795                 17800                 17805

Gly Phe   Thr His Xaa Xaa Ser   Xaa Pro Thr Thr Ser   Thr Pro Gly
    17810                 17815                 17820

Thr Ser   Thr Val Xaa Xaa Gly   Thr Ser Gly Thr Pro   Ser Ser Xaa
    17825                 17830                 17835

Pro Xaa   Xaa Thr Xaa Xaa Xaa   Pro Leu Leu Xaa Pro   Phe Thr Xaa
    17840                 17845                 17850

Asn Xaa   Thr Ile Thr Asn Leu   Xaa Xaa Xaa Xaa Xaa   Met Xaa Xaa
    17855                 17860                 17865

Pro Gly   Ser Arg Lys Phe Asn   Thr Thr Glu Xaa Val   Leu Gln Gly
    17870                 17875                 17880
```

```
Leu Leu   Xaa Pro Xaa Phe Lys   Asn Xaa Ser Val Gly   Xaa Leu Tyr
    17885             17890             17895

Ser Gly   Cys Arg Leu Thr Xaa   Leu Arg Xaa Glu Lys   Xaa Gly Ala
    17900             17905             17910

Ala Thr   Gly Xaa Asp Ala Ile   Cys Xaa His Xaa Xaa   Xaa Pro Lys
    17915             17920             17925

Xaa Pro   Gly Leu Xaa Xaa Glu   Xaa Leu Tyr Trp Glu   Leu Ser Xaa
    17930             17935             17940

Leu Thr   Xaa Xaa Ile Xaa Glu   Leu Gly Pro Tyr Thr   Leu Asp Arg
    17945             17950             17955

Xaa Ser   Leu Tyr Val Asn Gly   Phe His Pro Arg Ser   Ser Val Pro
    17960             17965             17970

Thr Thr   Ser Thr Pro Gly Thr   Ser Thr Val His Leu   Ala Thr Ser
    17975             17980             17985

Gly Thr   Pro Ser Ser Leu Pro   Gly His Thr Ala Pro   Val Pro Leu
    17990             17995             18000

Leu Ile   Pro Phe Thr Leu Asn   Phe Thr Ile Thr Asn   Leu His Tyr
    18005             18010             18015

Glu Glu   Asn Met Gln His Pro   Gly Ser Arg Lys Phe   Asn Thr Thr
    18020             18025             18030

Glu Arg   Val Leu Gln Gly Leu   Leu Gly Pro Met Phe   Lys Asn Thr
    18035             18040             18045

Ser Val   Gly Leu Leu Tyr Ser   Gly Cys Arg Leu Thr   Leu Leu Arg
    18050             18055             18060

Pro Glu   Lys Asn Gly Ala Ala   Thr Gly Met Asp Ala   Ile Cys Ser
    18065             18070             18075

His Arg   Leu Asp Pro Lys Ser   Pro Gly Leu Xaa Xaa   Glu Xaa Leu
    18080             18085             18090

Tyr Trp   Glu Leu Ser Xaa Leu   Thr Xaa Xaa Ile Xaa   Glu Leu Gly
    18095             18100             18105

Pro Tyr   Thr Leu Asp Arg Xaa   Ser Leu Tyr Val Asn   Gly Phe Thr
```

```
            18110                      18115                      18120


      His Xaa   Xaa Ser Xaa Pro Thr   Thr Ser Thr Pro Gly   Thr Ser Thr
          18125                      18130                      18135


      Val Xaa   Xaa Gly Thr Ser Gly   Thr Pro Ser Ser Xaa   Pro Xaa Xaa
          18140                      18145                      18150


      Thr Xaa   Xaa Xaa Pro Leu Leu   Xaa Pro Phe Thr Xaa   Asn Xaa Thr
          18155                      18160                      18165


      Ile Thr   Asn Leu Xaa Xaa Xaa   Xaa Xaa Met Xaa Xaa   Pro Gly Ser
          18170                      18175                      18180


      Arg Lys   Phe Asn Thr Thr Glu   Xaa Val Leu Gln Gly   Leu Leu Xaa
          18185                      18190                      18195


      Pro Xaa   Phe Lys Asn Xaa Ser   Val Gly Xaa Leu Tyr   Ser Gly Cys
          18200                      18205                      18210


      Arg Leu   Thr Xaa Leu Arg Xaa   Glu Lys Xaa Gly Ala   Ala Thr Gly
          18215                      18220                      18225


      Xaa Asp   Ala Ile Cys Xaa His   Xaa Xaa Xaa Pro Lys   Xaa Pro Gly
          18230                      18235                      18240


      Leu Xaa   Xaa Glu Xaa Leu Tyr   Trp Glu Leu Ser Xaa   Leu Thr Xaa
          18245                      18250                      18255


      Xaa Ile   Xaa Glu Leu Gly Pro   Tyr Thr Leu Asp Arg   Xaa Ser Leu
          18260                      18265                      18270


      Tyr Val   Asn Gly Phe Thr His   Gln Asn Ser Val Pro   Thr Thr Ser
          18275                      18280                      18285


      Thr Pro   Gly Thr Ser Thr Val   Tyr Trp Ala Thr Thr   Gly Thr Pro
          18290                      18295                      18300


      Ser Ser   Phe Pro Gly His Thr   Glu Pro Gly Pro Leu   Leu Ile Pro
          18305                      18310                      18315


      Phe Thr   Phe Asn Phe Thr Ile   Thr Asn Leu His Tyr   Glu Glu Asn
          18320                      18325                      18330


      Met Gln   His Pro Gly Ser Arg   Lys Phe Asn Thr Thr   Glu Arg Val
          18335                      18340                      18345
```

Leu Gln Gly Leu Leu Thr Pro Leu Phe Lys Asn Thr Ser Val Gly
    18350           18355           18360

Pro Leu Tyr Ser Gly Cys Arg Leu Thr Leu Leu Arg Pro Glu Lys
    18365           18370           18375

Gln Glu Ala Ala Thr Gly Val Asp Thr Ile Cys Thr His Arg Val
    18380           18385           18390

Asp Pro Ile Gly Pro Gly Leu Xaa Xaa Glu Xaa Leu Tyr Trp Glu
    18395           18400           18405

Leu Ser Xaa Leu Thr Xaa Xaa Ile Xaa Glu Leu Gly Pro Tyr Thr
    18410           18415           18420

Leu Asp Arg Xaa Ser Leu Tyr Val Asn Gly Phe Thr His Xaa Xaa
    18425           18430           18435

Ser Xaa Pro Thr Thr Ser Thr Pro Gly Thr Ser Thr Val Xaa Xaa
    18440           18445           18450

Gly Thr Ser Gly Thr Pro Ser Ser Xaa Pro Xaa Xaa Thr Xaa Xaa
    18455           18460           18465

Xaa Pro Leu Leu Xaa Pro Phe Thr Xaa Asn Xaa Thr Ile Thr Asn
    18470           18475           18480

Leu Xaa Xaa Xaa Xaa Xaa Met Xaa Xaa Pro Gly Ser Arg Lys Phe
    18485           18490           18495

Asn Thr Thr Glu Xaa Val Leu Gln Gly Leu Leu Xaa Pro Xaa Phe
    18500           18505           18510

Lys Asn Xaa Ser Val Gly Xaa Leu Tyr Ser Gly Cys Arg Leu Thr
    18515           18520           18525

Xaa Leu Arg Xaa Glu Lys Xaa Gly Ala Ala Thr Gly Xaa Asp Ala
    18530           18535           18540

Ile Cys Xaa His Xaa Xaa Xaa Pro Lys Xaa Pro Gly Leu Xaa Xaa
    18545           18550           18555

Glu Xaa Leu Tyr Trp Glu Leu Ser Xaa Leu Thr Xaa Xaa Ile Xaa
    18560           18565           18570

Glu Leu Gly Pro Tyr Thr Leu Asp Arg Xaa Ser Leu Tyr Val Asn
    18575           18580           18585

```
Gly Phe    Thr His Arg Ser Ser    Val Pro Thr Thr Ser    Ser Pro Gly
    18590                 18595                 18600

Thr Ser    Thr Val His Leu Ala    Thr Ser Gly Thr Pro    Ser Ser Leu
    18605                 18610                 18615

Pro Gly    His Thr Ala Pro Val    Pro Leu Leu Ile Pro    Phe Thr Leu
    18620                 18625                 18630

Asn Phe    Thr Ile Thr Asn Leu    His Tyr Glu Glu Asn    Met Gln His
    18635                 18640                 18645

Pro Gly    Ser Arg Lys Phe Asn    Thr Thr Glu Arg Val    Leu Gln Gly
    18650                 18655                 18660

Leu Leu    Lys Pro Leu Phe Lys    Ser Thr Ser Val Gly    Pro Leu Tyr
    18665                 18670                 18675

Ser Gly    Cys Arg Leu Thr Leu    Leu Arg Pro Glu Lys    His Gly Ala
    18680                 18685                 18690

Ala Thr    Gly Val Asp Ala Ile    Cys Thr Leu Arg Leu    Asp Pro Thr
    18695                 18700                 18705

Gly Pro    Gly Leu Xaa Xaa Glu    Xaa Leu Tyr Trp Glu    Leu Ser Xaa
    18710                 18715                 18720

Leu Thr    Xaa Xaa Ile Xaa Glu    Leu Gly Pro Tyr Thr    Leu Asp Arg
    18725                 18730                 18735

Xaa Ser    Leu Tyr Val Asn Gly    Phe Thr His Xaa Xaa    Ser Xaa Pro
    18740                 18745                 18750

Thr Thr    Ser Thr Pro Gly Thr    Ser Thr Val Xaa Xaa    Gly Thr Ser
    18755                 18760                 18765

Gly Thr    Pro Ser Ser Xaa Pro    Xaa Xaa Thr Xaa Xaa    Xaa Pro Leu
    18770                 18775                 18780

Leu Xaa    Pro Phe Thr Xaa Asn    Xaa Thr Ile Thr Asn    Leu Xaa Xaa
    18785                 18790                 18795

Xaa Xaa    Xaa Met Xaa Xaa Pro    Gly Ser Arg Lys Phe    Asn Thr Thr
    18800                 18805                 18810

Glu Xaa    Val Leu Gln Gly Leu    Leu Xaa Pro Xaa Phe    Lys Asn Xaa
    18815                 18820                 18825
```

Ser Val   Gly Xaa Leu Tyr Ser   Gly Cys Arg Leu Thr   Xaa Leu Arg

Xaa Glu   Lys Xaa Gly Ala Ala   Thr Gly Xaa Asp Ala   Ile Cys Xaa

His Xaa   Xaa Xaa Pro Lys Xaa   Pro Gly Leu Xaa Xaa   Glu Xaa Leu

Tyr Trp   Glu Leu Ser Xaa Leu   Thr Xaa Xaa Ile Xaa   Glu Leu Gly

Pro Tyr   Thr Leu Asp Arg Xaa   Ser Leu Tyr Val Asn   Gly Phe Thr

His Arg   Thr Ser Val Pro Thr   Thr Ser Thr Pro Gly   Thr Ser Thr

Val His   Leu Ala Thr Ser Gly   Thr Pro Ser Ser Leu   Pro Gly His

Thr Ala   Pro Val Pro Leu Leu   Ile Pro Phe Thr Leu   Asn Phe Thr

Ile Thr   Asn Leu Gln Tyr Glu   Glu Asp Met His Arg   Pro Gly Ser

Arg Lys   Phe Asn Thr Thr Glu   Arg Val Leu Gln Gly   Leu Leu Ser

Pro Ile   Phe Lys Asn Ser Ser   Val Gly Pro Leu Tyr   Ser Gly Cys

Arg Leu   Thr Ser Leu Arg Pro   Glu Lys Asp Gly Ala   Ala Thr Gly

Met Asp   Ala Val Cys Leu Tyr   His Pro Asn Pro Lys   Arg Pro Gly

Leu Asp   Arg Glu Gln Leu Tyr   Cys Glu Leu Ser Gln   Leu Thr His

Asn Ile   Thr Glu Leu Gly Pro   Tyr Ser Leu Asp Arg   Asp Ser Leu

Tyr Val   Asn Gly Phe Thr His   Gln Asn Ser Val Pro   Thr Thr Ser

```
                    19055                      19060                        19065


        Thr Pro   Gly Thr Ser Thr Val     Tyr Trp Ala Thr Thr     Gly Thr Pro
            19070                     19075                 19080


        Ser Ser   Phe Pro Gly His Thr     Xaa Xaa Xaa Pro Leu     Leu Xaa Pro
            19085                     19090                 19095


        Phe Thr   Xaa Asn Xaa Thr Ile     Thr Asn Leu Xaa Xaa     Xaa Xaa Xaa
            19100                     19105                 19110


        Met Xaa   Xaa Pro Gly Ser Arg     Lys Phe Asn Thr Thr     Glu Xaa Val
            19115                     19120                 19125


        Leu Gln   Gly Leu Leu Xaa Pro     Xaa Phe Lys Asn Xaa     Ser Val Gly
            19130                     19135                 19140


        Xaa Leu   Tyr Ser Gly Cys Arg     Leu Thr Xaa Leu Arg     Xaa Glu Lys
            19145                     19150                 19155


        Xaa Gly   Ala Ala Thr Gly Xaa     Asp Ala Ile Cys Xaa     His Xaa Xaa
            19160                     19165                 19170


        Xaa Pro   Lys Xaa Pro Gly Leu     Xaa Xaa Glu Xaa Leu     Tyr Trp Glu
            19175                     19180                 19185


        Leu Ser   Xaa Leu Thr Xaa Xaa     Ile Xaa Glu Leu Gly     Pro Tyr Thr
            19190                     19195                 19200


        Leu Asp   Arg Xaa Ser Leu Tyr     Val Asn Gly Phe Thr     His Trp Ser
            19205                     19210                 19215


        Ser Gly   Leu Thr Thr Ser Thr     Pro Trp Thr Ser Thr     Val Asp Leu
            19220                     19225                 19230


        Gly Thr   Ser Gly Thr Pro Ser     Pro Val Pro Ser Pro     Thr Thr Ala
            19235                     19240                 19245


        Gly Pro   Leu Leu Val Pro Phe     Thr Leu Asn Phe Thr     Ile Thr Asn
            19250                     19255                 19260


        Leu Gln   Tyr Glu Glu Asp Met     His Arg Pro Gly Ser     Arg Lys Phe
            19265                     19270                 19275


        Asn Ala   Thr Glu Arg Val Leu     Gln Gly Leu Leu Ser     Pro Ile Phe
            19280                     19285                 19290
```

```
Lys Asn   Thr Ser Val Gly Pro   Leu Tyr Ser Gly Cys   Arg Leu Thr
    19295               19300               19305

Leu Leu   Arg Pro Glu Lys Gln   Glu Ala Ala Thr Gly   Val Asp Thr
    19310               19315               19320

Ile Cys   Thr His Arg Val Asp   Pro Ile Gly Pro Gly   Leu Xaa Xaa
    19325               19330               19335

Glu Xaa   Leu Tyr Trp Glu Leu   Ser Xaa Leu Thr Xaa   Xaa Ile Xaa
    19340               19345               19350

Glu Leu   Gly Pro Tyr Thr Leu   Asp Arg Xaa Ser Leu   Tyr Val Asn
    19355               19360               19365

Gly Phe   Thr His Xaa Xaa Ser   Xaa Pro Thr Thr Ser   Thr Pro Gly
    19370               19375               19380

Thr Ser   Thr Val Xaa Xaa Gly   Thr Ser Gly Thr Pro   Ser Ser Xaa
    19385               19390               19395

Pro Xaa   Xaa Thr Xaa Xaa Xaa   Pro Leu Leu Xaa Pro   Phe Thr Xaa
    19400               19405               19410

Asn Xaa   Thr Ile Thr Asn Leu   Xaa Xaa Xaa Xaa Xaa   Met Xaa Xaa
    19415               19420               19425

Pro Gly   Ser Arg Lys Phe Asn   Thr Thr Glu Xaa Val   Leu Gln Gly
    19430               19435               19440

Leu Leu   Xaa Pro Xaa Phe Lys   Asn Xaa Ser Val Gly   Xaa Leu Tyr
    19445               19450               19455

Ser Gly   Cys Arg Leu Thr Xaa   Leu Arg Xaa Glu Lys   Xaa Gly Ala
    19460               19465               19470

Ala Thr   Gly Xaa Asp Ala Ile   Cys Xaa His Xaa Xaa   Xaa Pro Lys
    19475               19480               19485

Xaa Pro   Gly Leu Xaa Xaa Glu   Xaa Leu Tyr Trp Glu   Leu Ser Xaa
    19490               19495               19500

Leu Thr   Xaa Xaa Ile Xaa Glu   Leu Gly Pro Tyr Thr   Leu Asp Arg
    19505               19510               19515

Xaa Ser   Leu Tyr Val Asn Gly   Phe Thr His Arg Ser   Phe Gly Leu
    19520               19525               19530
```

470

```
Thr Thr  Ser Thr Pro Trp Thr    Ser Thr Val Asp Leu    Gly Thr Ser
    19535               19540                19545

Gly Thr  Pro Ser Pro Val Pro    Ser Pro Thr Thr Ala    Gly Pro Leu
    19550               19555                19560

Leu Val  Pro Phe Thr Leu Asn    Phe Thr Ile Thr Asn    Leu Gln Tyr
    19565               19570                19575

Glu Glu  Asp Met His Arg Pro    Gly Ser Arg Lys Phe    Asn Thr Thr
    19580               19585                19590

Glu Arg  Val Leu Gln Gly Leu    Leu Thr Pro Leu Phe    Arg Asn Thr
    19595               19600                19605

Ser Val  Ser Ser Leu Tyr Ser    Gly Cys Arg Leu Thr    Leu Leu Arg
    19610               19615                19620

Pro Glu  Lys Asp Gly Ala Ala    Thr Arg Val Asp Ala    Val Cys Thr
    19625               19630                19635

His Arg  Pro Asp Pro Lys Ser    Pro Gly Leu Xaa Xaa    Glu Xaa Leu
    19640               19645                19650

Tyr Trp  Glu Leu Ser Xaa Leu    Thr Xaa Xaa Ile Xaa    Glu Leu Gly
    19655               19660                19665

Pro Tyr  Thr Leu Asp Arg Xaa    Ser Leu Tyr Val Asn    Gly Phe Thr
    19670               19675                19680

His Xaa  Xaa Ser Xaa Pro Thr    Thr Ser Thr Pro Gly    Thr Ser Thr
    19685               19690                19695

Val Xaa  Xaa Gly Thr Ser Gly    Thr Pro Ser Ser Xaa    Pro Xaa Xaa
    19700               19705                19710

Thr Xaa  Xaa Xaa Pro Leu Leu    Xaa Pro Phe Thr Xaa    Asn Xaa Thr
    19715               19720                19725

Ile Thr  Asn Leu Xaa Xaa Xaa    Xaa Xaa Met Xaa Xaa    Pro Gly Ser
    19730               19735                19740

Arg Lys  Phe Asn Thr Thr Glu    Xaa Val Leu Gln Gly    Leu Leu Xaa
    19745               19750                19755

Pro Xaa  Phe Lys Asn Xaa Ser    Val Gly Xaa Leu Tyr    Ser Gly Cys
    19760               19765                19770
```

Arg Leu Thr Xaa Leu Arg Xaa Glu Lys Xaa Gly Ala Ala Thr Gly
19775 19780 19785

Xaa Asp Ala Ile Cys Xaa His Xaa Xaa Xaa Pro Lys Xaa Pro Gly
19790 19795 19800

Leu Xaa Xaa Glu Xaa Leu Tyr Trp Glu Leu Ser Xaa Leu Thr Xaa
19805 19810 19815

Xaa Ile Xaa Glu Leu Gly Pro Tyr Thr Leu Asp Arg Xaa Ser Leu
19820 19825 19830

Tyr Val Asn Gly Phe Thr His Trp Ile Pro Val Pro Thr Ser Ser
19835 19840 19845

Thr Pro Gly Thr Ser Thr Val Asp Leu Gly Ser Gly Thr Pro Ser
19850 19855 19860

Ser Leu Pro Ser Pro Thr Thr Ala Gly Pro Leu Leu Val Pro Phe
19865 19870 19875

Thr Leu Asn Phe Thr Ile Thr Asn Leu Gln Tyr Gly Glu Asp Met
19880 19885 19890

Gly His Pro Gly Ser Arg Lys Phe Asn Thr Thr Glu Arg Val Leu
19895 19900 19905

Gln Gly Leu Leu Gly Pro Ile Phe Lys Asn Thr Ser Val Gly Pro
19910 19915 19920

Leu Tyr Ser Gly Cys Arg Leu Thr Ser Leu Arg Ser Glu Lys Asp
19925 19930 19935

Gly Ala Ala Thr Gly Val Asp Ala Ile Cys Ile His His Leu Asp
19940 19945 19950

Pro Lys Ser Pro Gly Leu Xaa Xaa Glu Xaa Leu Tyr Trp Glu Leu
19955 19960 19965

Ser Xaa Leu Thr Xaa Xaa Ile Xaa Glu Leu Gly Pro Tyr Thr Leu
19970 19975 19980

Asp Arg Xaa Ser Leu Tyr Val Asn Gly Phe Thr His Xaa Xaa Ser
19985 19990 19995

Xaa Pro Thr Thr Ser Thr Pro Gly Thr Ser Thr Val Xaa Xaa Gly

472

20000                    20005                        20010

Thr Ser   Gly Thr Pro Ser Ser     Xaa Pro Xaa Xaa Thr     Xaa Xaa Xaa
          20015                20020                20025

Pro Leu   Leu Xaa Pro Phe Thr     Xaa Asn Xaa Thr Ile     Thr Asn Leu
          20030                20035                20040

Xaa Xaa   Xaa Xaa Xaa Met Xaa     Xaa Pro Gly Ser Arg     Lys Phe Asn
          20045                20050                20055

Thr Thr   Glu Xaa Val Leu Gln     Gly Leu Leu Xaa Pro     Xaa Phe Lys
          20060                20065                20070

Asn Xaa   Ser Val Gly Xaa Leu     Tyr Ser Gly Cys Arg     Leu Thr Xaa
          20075                20080                20085

Leu Arg   Xaa Glu Lys Xaa Gly     Ala Ala Thr Gly Xaa     Asp Ala Ile
          20090                20095                20100

Cys Xaa   His Xaa Xaa Xaa Pro     Lys Xaa Pro Gly Leu     Xaa Xaa Glu
          20105                20110                20115

Xaa Leu   Tyr Trp Glu Leu Ser     Xaa Leu Thr Xaa Xaa     Ile Xaa Glu
          20120                20125                20130

Leu Gly   Pro Tyr Thr Leu Asp     Arg Xaa Ser Leu Tyr     Val Asn Gly
          20135                20140                20145

Phe Thr   His Gln Thr Phe Ala     Pro Asn Thr Ser Thr     Pro Gly Thr
          20150                20155                20160

Ser Thr   Val Asp Leu Gly Thr     Ser Gly Thr Pro Ser     Ser Leu Pro
          20165                20170                20175

Ser Pro   Thr Ser Ala Gly Pro     Leu Leu Val Pro Phe     Thr Leu Asn
          20180                20185                20190

Phe Thr   Ile Thr Asn Leu Gln     Tyr Glu Glu Asp Met     His His Pro
          20195                20200                20205

Gly Ser   Arg Lys Phe Asn Thr     Thr Glu Arg Val Leu     Gln Gly Leu
          20210                20215                20220

Leu Gly   Pro Met Phe Lys Asn     Thr Ser Val Gly Leu     Leu Tyr Ser
          20225                20230                20235

Gly Cys Arg Leu Thr Leu Leu Arg Pro Glu Lys Asn Gly Ala Ala
20240                20245            20250

Thr Arg Val Asp Ala Val Cys Thr His Arg Pro Asp Pro Lys Ser
20255                20260            20265

Pro Gly Leu Xaa Xaa Glu Xaa Leu Tyr Trp Glu Leu Ser Xaa Leu
20270                20275            20280

Thr Xaa Xaa Ile Xaa Glu Leu Gly Pro Tyr Thr Leu Asp Arg Xaa
20285                20290            20295

Ser Leu Tyr Val Asn Gly Phe Thr His Xaa Xaa Ser Xaa Pro Thr
20300                20305            20310

Thr Ser Thr Pro Gly Thr Ser Thr Val Xaa Xaa Gly Thr Ser Gly
20315                20320            20325

Thr Pro Ser Ser Xaa Pro Xaa Xaa Thr Ala Pro Val Pro Leu Leu
20330                20335            20340

Ile Pro Phe Thr Leu Asn Phe Thr Ile Thr Asn Leu His Tyr Glu
20345                20350            20355

Glu Asn Met Gln His Pro Gly Ser Arg Lys Phe Asn Thr Thr Glu
20360                20365            20370

Arg Val Leu Gln Gly Leu Leu Lys Pro Leu Phe Lys Ser Thr Ser
20375                20380            20385

Val Gly Pro Leu Tyr Ser Gly Cys Arg Leu Thr Leu Leu Arg Pro
20390                20395            20400

Glu Lys His Gly Ala Ala Thr Gly Val Asp Ala Ile Cys Thr Leu
20405                20410            20415

Arg Leu Asp Pro Thr Gly Pro Gly Leu Asp Arg Glu Arg Leu Tyr
20420                20425            20430

Trp Glu Leu Ser Gln Leu Thr Asn Ser Val Thr Glu Leu Gly Pro
20435                20440            20445

Tyr Thr Leu Asp Arg Asp Ser Leu Tyr Val Asn Gly Phe Thr Gln
20450                20455            20460

Arg Ser Ser Val Pro Thr Thr Ser Ile Pro Gly Thr Ser Ala Val
20465                20470            20475

474

His Leu Glu Thr Ser Gly Thr     Pro Ala Ser Leu Pro     Gly His Thr
    20480               20485               20490

Ala Pro Gly Pro Leu Leu Val     Pro Phe Thr Leu Asn     Phe Thr Ile
    20495               20500               20505

Thr Asn Leu Gln Tyr Glu Val     Asp Met Arg His Pro     Gly Ser Arg
    20510               20515               20520

Lys Phe Asn Thr Thr Glu Arg     Val Leu Gln Gly Leu     Leu Lys Pro
    20525               20530               20535

Leu Phe Lys Ser Thr Ser Val     Gly Pro Leu Tyr Ser     Gly Cys Arg
    20540               20545               20550

Leu Thr Leu Leu Arg Pro Glu     Lys Arg Gly Ala Ala     Thr Gly Val
    20555               20560               20565

Asp Thr Ile Cys Thr His Arg     Leu Asp Pro Leu Asn     Pro Gly Leu
    20570               20575               20580

Asp Arg Glu Gln Leu Tyr Trp     Glu Leu Ser Lys Leu     Thr Arg Gly
    20585               20590               20595

Ile Ile Glu Leu Gly Pro Tyr     Leu Leu Asp Arg Gly     Ser Leu Tyr
    20600               20605               20610

Val Asn Gly Phe Thr His Arg     Asn Phe Val Pro Ile     Thr Ser Thr
    20615               20620               20625

Pro Gly Thr Ser Thr Val His     Leu Gly Thr Ser Glu     Thr Pro Ser
    20630               20635               20640

Ser Leu Pro Arg Pro Ile Val     Pro Gly Pro Leu Leu     Val Pro Phe
    20645               20650               20655

Thr Leu Asn Phe Thr Ile Thr     Asn Leu Gln Tyr Glu     Glu Ala Met
    20660               20665               20670

Arg His Pro Gly Ser Arg Lys     Phe Asn Thr Thr Glu     Arg Val Leu
    20675               20680               20685

Gln Gly Leu Leu Arg Pro Leu     Phe Lys Asn Thr Ser     Ile Gly Pro
    20690               20695               20700

Leu Tyr Ser Ser Cys Arg Leu     Thr Leu Leu Arg Pro     Glu Lys Asp
    20705               20710               20715

475

```
Lys Ala   Ala Thr Arg Val Asp   Ala Ile Cys Thr His   His Pro Asp
    20720                 20725             20730

Pro Gln   Ser Pro Gly Leu Asn   Arg Glu Gln Leu Tyr   Trp Glu Leu
    20735                 20740             20745

Ser Gln   Leu Thr His Gly Ile   Thr Glu Leu Gly Pro   Tyr Thr Leu
    20750                 20755             20760

Asp Arg   Asp Ser Leu Tyr Val   Asp Gly Phe Thr His   Trp Ser Pro
    20765                 20770             20775

Ile Pro   Thr Thr Ser Thr Pro   Gly Thr Ser Ile Val   Asn Leu Gly
    20780                 20785             20790

Thr Ser   Gly Ile Pro Pro Ser   Leu Pro Glu Thr Thr   Xaa Xaa Xaa
    20795                 20800             20805

Pro Leu   Leu Xaa Pro Phe Thr   Xaa Asn Xaa Thr Ile   Thr Asn Leu
    20810                 20815             20820

Xaa Xaa   Xaa Xaa Xaa Met Xaa   Xaa Pro Gly Ser Arg   Lys Phe Asn
    20825                 20830             20835

Thr Thr   Glu Arg Val Leu Gln   Gly Leu Leu Lys Pro   Leu Phe Lys
    20840                 20845             20850

Ser Thr   Ser Val Gly Pro Leu   Tyr Ser Gly Cys Arg   Leu Thr Leu
    20855                 20860             20865

Leu Arg   Pro Glu Lys Asp Gly   Val Ala Thr Arg Val   Asp Ala Ile
    20870                 20875             20880

Cys Thr   His Arg Pro Asp Pro   Lys Ile Pro Gly Leu   Asp Arg Gln
    20885                 20890             20895

Gln Leu   Tyr Trp Glu Leu Ser   Gln Leu Thr His Ser   Ile Thr Glu
    20900                 20905             20910

Leu Gly   Pro Tyr Thr Leu Asp   Arg Asp Ser Leu Tyr   Val Asn Gly
    20915                 20920             20925

Phe Thr   Gln Arg Ser Ser Val   Pro Thr Thr Ser Thr   Pro Gly Thr
    20930                 20935             20940

Phe Thr   Val Gln Pro Glu Thr   Ser Glu Thr Pro Ser   Ser Leu Pro
```

476

20945                    20950                    20955

Gly Pro Thr Ala Thr Gly Pro   Val Leu Leu Pro Phe   Thr Leu Asn
    20960                20965            20970

Phe Thr Ile Thr Asn Leu Gln   Tyr Glu Glu Asp Met   His Arg Pro
    20975                20980            20985

Gly Ser Arg Lys Phe Asn Thr   Thr Glu Arg Val Leu   Gln Gly Leu
    20990                20995            21000

Leu Met Pro Leu Phe Lys Asn   Thr Ser Val Ser Ser   Leu Tyr Ser
    21005                21010            21015

Gly Cys Arg Leu Thr Leu Leu   Arg Pro Glu Lys Asp   Gly Ala Ala
    21020                21025            21030

Thr Arg Val Asp Ala Val Cys   Thr His Arg Pro Asp   Pro Lys Ser
    21035                21040            21045

Pro Gly Leu Asp Arg Glu Arg   Leu Tyr Trp Lys Leu   Ser Gln Leu
    21050                21055            21060

Thr His Gly Ile Thr Glu Leu   Gly Pro Tyr Thr Leu   Asp Arg His
    21065                21070            21075

Ser Leu Tyr Val Asn Gly Phe   Thr His Gln Ser Ser   Met Thr Thr
    21080                21085            21090

Thr Arg Thr Pro Asp Thr Ser   Thr Met His Leu Ala   Thr Ser Arg
    21095                21100            21105

Thr Pro Ala Ser Leu Ser Gly   Pro Thr Thr Ala Ser   Pro Leu Leu
    21110                21115            21120

Val Leu Phe Thr Ile Asn Phe   Thr Ile Thr Asn Leu   Arg Tyr Glu
    21125                21130            21135

Glu Asn Met His His Pro Gly   Ser Arg Lys Phe Asn   Thr Thr Glu
    21140                21145            21150

Arg Val Leu Gln Gly Leu Leu   Arg Pro Val Phe Lys   Asn Thr Ser
    21155                21160            21165

Val Gly Pro Leu Tyr Ser Gly   Cys Arg Leu Thr Leu   Leu Arg Pro
    21170                21175            21180

```
Lys Lys   Asp Gly Ala Ala Thr   Lys Val Asp Ala Ile   Cys Thr Tyr
    21185                 21190                 21195

Arg Pro   Asp Pro Lys Ser Pro   Gly Leu Asp Arg Glu   Gln Leu Tyr
    21200                 21205                 21210

Trp Glu   Leu Ser Gln Leu Thr   His Ser Ile Thr Glu   Leu Gly Pro
    21215                 21220                 21225

Tyr Thr   Leu Asp Arg Asp Ser   Leu Tyr Val Asn Gly   Phe Thr Gln
    21230                 21235                 21240

Arg Ser   Ser Val Pro Thr Thr   Ser Ile Pro Gly Thr   Pro Thr Val
    21245                 21250                 21255

Asp Leu   Gly Thr Ser Gly Thr   Pro Val Ser Lys Pro   Gly Pro Ser
    21260                 21265                 21270

Ala Ala   Ser Pro Leu Leu Val   Leu Phe Thr Leu Asn   Phe Thr Ile
    21275                 21280                 21285

Thr Asn   Leu Arg Tyr Glu Glu   Asn Met Gln His Pro   Gly Ser Arg
    21290                 21295                 21300

Lys Phe   Asn Thr Thr Glu Arg   Val Leu Gln Gly Leu   Leu Arg Ser
    21305                 21310                 21315

Leu Phe   Lys Ser Thr Ser Val   Gly Pro Leu Tyr Ser   Gly Cys Arg
    21320                 21325                 21330

Leu Thr   Leu Leu Arg Pro Glu   Lys Asp Gly Thr Ala   Thr Gly Val
    21335                 21340                 21345

Asp Ala   Ile Cys Thr His His   Pro Asp Pro Lys Ser   Pro Arg Leu
    21350                 21355                 21360

Asp Arg   Glu Gln Leu Tyr Trp   Glu Leu Ser Gln Leu   Thr His Asn
    21365                 21370                 21375

Ile Thr   Glu Leu Gly His Tyr   Ala Leu Asp Asn Asp   Ser Leu Phe
    21380                 21385                 21390

Val Asn   Gly Phe Thr His Arg   Ser Ser Val Ser Thr   Thr Ser Thr
    21395                 21400                 21405

Pro Gly   Thr Pro Thr Val Tyr   Leu Gly Ala Ser Lys   Thr Pro Ala
    21410                 21415                 21420
```

478

Ser Ile Phe Gly Pro Ser Ala Ala Ser His Leu Leu Ile Leu Phe
    21425               21430               21435

Thr Leu Asn Phe Thr Ile Thr Asn Leu Arg Tyr Glu Glu Asn Met
    21440               21445               21450

Trp Pro Gly Ser Arg Lys Phe Asn Thr Thr Glu Arg Val Leu Gln
    21455               21460               21465

Gly Leu Leu Arg Pro Leu Phe Lys Asn Thr Ser Val Gly Pro Leu
    21470               21475               21480

Tyr Ser Gly Ser Arg Leu Thr Leu Leu Arg Pro Glu Lys Asp Gly
    21485               21490               21495

Glu Ala Thr Gly Val Asp Ala Ile Cys Thr His Arg Pro Asp Pro
    21500               21505               21510

Thr Gly Pro Gly Leu Asp Arg Glu Gln Leu Tyr Leu Glu Leu Ser
    21515               21520               21525

Gln Leu Thr His Ser Ile Thr Glu Leu Gly Pro Tyr Thr Leu Asp
    21530               21535               21540

Arg Asp Ser Leu Tyr Val Asn Gly Phe Thr His Arg Ser Ser Val
    21545               21550               21555

Pro Thr Thr Ser Thr Gly Val Val Ser Glu Glu Pro Phe Thr Leu
    21560               21565               21570

Asn Phe Thr Ile Asn Asn Leu Arg Tyr Met Ala Asp Met Gly Gln
    21575               21580               21585

Pro Gly Ser Leu Lys Phe Asn Ile Thr Asp Asn Val Met Lys His
    21590               21595               21600

Leu Leu Ser Pro Leu Phe Gln Arg Ser Ser Leu Gly Ala Arg Tyr
    21605               21610               21615

Thr Gly Cys Arg Val Ile Ala Leu Arg Ser Val Lys Asn Gly Ala
    21620               21625               21630

Glu Thr Arg Val Asp Leu Leu Cys Thr Tyr Leu Gln Pro Leu Ser
    21635               21640               21645

Gly Pro Gly Leu Pro Ile Lys Gln Val Phe His Glu Leu Ser Gln
    21650               21655               21660

```
Gln Thr   His Gly Ile Thr Arg   Leu Gly Pro Tyr Ser   Leu Asp Lys
    21665                 21670              21675

Asp Ser   Leu Tyr Leu Asn Gly   Tyr Asn Glu Pro Gly   Leu Asp Glu
    21680                 21685              21690

Pro Pro   Thr Thr Pro Lys Pro   Ala Thr Thr Phe Leu   Pro Pro Leu
    21695                 21700              21705

Ser Glu   Ala Thr Thr Ala Met   Gly Tyr His Leu Lys   Thr Leu Thr
    21710                 21715              21720

Leu Asn   Phe Thr Ile Ser Asn   Leu Gln Tyr Ser Pro   Asp Met Gly
    21725                 21730              21735

Lys Gly   Ser Ala Thr Phe Asn   Ser Thr Glu Gly Val   Leu Gln His
    21740                 21745              21750

Leu Leu   Arg Pro Leu Phe Gln   Lys Ser Ser Met Gly   Pro Phe Tyr
    21755                 21760              21765

Leu Gly   Cys Gln Leu Ile Ser   Leu Arg Pro Glu Lys   Asp Gly Ala
    21770                 21775              21780

Ala Thr   Gly Val Asp Thr Thr   Cys Thr Tyr His Pro   Asp Pro Val
    21785                 21790              21795

Gly Pro   Gly Leu Asp Ile Gln   Gln Leu Tyr Trp Glu   Leu Ser Gln
    21800                 21805              21810

Leu Thr   His Gly Val Thr Gln   Leu Gly Phe Tyr Val   Leu Asp Arg
    21815                 21820              21825

Asp Ser   Leu Phe Ile Asn Gly   Tyr Ala Pro Gln Asn   Leu Ser Ile
    21830                 21835              21840

Arg Gly   Glu Tyr Gln Ile Asn   Phe His Ile Val Asn   Trp Asn Leu
    21845                 21850              21855

Ser Asn   Pro Asp Pro Thr Ser   Ser Glu Tyr Ile Thr   Leu Leu Arg
    21860                 21865              21870

Asp Ile   Gln Asp Lys Val Thr   Thr Leu Tyr Lys Gly   Ser Gln Leu
    21875                 21880              21885

His Asp   Thr Phe Arg Phe Cys   Leu Val Thr Asn Leu   Thr Met Asp
```

21890        21895        21900

Ser Val Leu Val Thr Val Lys   Ala Leu Phe Ser Ser   Asn Leu Asp
21905        21910        21915

Pro Ser Leu Val Glu Gln Val   Phe Leu Asp Lys Thr   Leu Asn Ala
21920        21925        21930

Ser Phe His Trp Leu Gly Ser   Thr Tyr Gln Leu Val   Asp Ile His
21935        21940        21945

Val Thr Glu Met Glu Ser Ser   Val Tyr Gln Pro Thr   Ser Ser Ser
21950        21955        21960

Ser Thr Gln His Phe Tyr Leu   Asn Phe Thr Ile Thr   Asn Leu Pro
21965        21970        21975

Tyr Ser Gln Asp Lys Ala Gln   Pro Gly Thr Thr Asn   Tyr Gln Arg
21980        21985        21990

Asn Lys Arg Asn Ile Glu Asp   Ala Leu Asn Gln Leu   Phe Arg Asn
21995        22000        22005

Ser Ser Ile Lys Ser Tyr Phe   Ser Asp Cys Gln Val   Ser Thr Phe
22010        22015        22020

Arg Ser Val Pro Asn Arg His   His Thr Gly Val Asp   Ser Leu Cys
22025        22030        22035

Asn Phe Ser Pro Leu Ala Arg   Arg Val Asp Arg Val   Ala Ile Tyr
22040        22045        22050

Glu Glu Phe Leu Arg Met Thr   Arg Asn Gly Thr Gln   Leu Gln Asn
22055        22060        22065

Phe Thr Leu Asp Arg Ser Ser   Val Leu Val Asp Gly   Tyr Ser Pro
22070        22075        22080

Asn Arg Asn Glu Pro Leu Thr   Gly Asn Ser Asp Leu   Pro Phe Trp
22085        22090        22095

Ala Val Ile Leu Ile Gly Leu   Ala Gly Leu Leu Gly   Leu Ile Thr
22100        22105        22110

Cys Leu Ile Cys Gly Val Leu   Val Thr Thr Arg Arg   Arg Lys Lys
22115        22120        22125

```
Glu Gly   Glu Tyr Asn Val Gln   Gln Gln Cys Pro Gly   Tyr Tyr Gln
    22130                 22135               22140

Ser His   Leu Asp Leu Glu Asp   Leu Gln
    22145                 22150
```

```
<210> 8
<211> 89
<212> PRT
<213> Homo sapiens

<400> 8
Met Lys Gly Leu Ala Ala Ala Leu Leu Val Leu Val Cys Thr Met Ala
1               5               10              15

Leu Cys Ser Cys Ala Gln Val Gly Thr Asn Lys Glu Leu Cys Cys Leu
            20              25              30

Val Tyr Thr Ser Trp Gln Ile Pro Gln Lys Phe Ile Val Asp Tyr Ser
        35              40              45

Glu Thr Ser Pro Gln Cys Pro Lys Pro Gly Val Ile Leu Leu Thr Lys
    50              55              60

Arg Gly Arg Gln Ile Cys Ala Asp Pro Asn Lys Lys Trp Val Gln Lys
65              70              75              80

Tyr Ile Ser Asp Leu Lys Leu Asn Ala
                85
```

```
<210> 9
<211> 119
<212> PRT
<213> Homo sapiens

<400> 9
Met Ala Gly Leu Met Thr Ile Val Thr Ser Leu Leu Phe Leu Gly Val
1               5               10              15

Cys Ala His His Ile Ile Pro Thr Gly Ser Val Val Ile Pro Ser Pro
            20              25              30

Cys Cys Met Phe Phe Val Ser Lys Arg Ile Pro Glu Asn Arg Val Val
        35              40              45

Ser Tyr Gln Leu Ser Ser Arg Ser Thr Cys Leu Lys Ala Gly Val Ile
    50              55              60

Phe Thr Thr Lys Lys Gly Gln Gln Phe Cys Gly Asp Pro Lys Gln Glu
65              70              75              80
```

```
Trp Val Gln Arg Tyr Met Lys Asn Leu Asp Ala Lys Gln Lys Lys Ala
                85                  90                  95

Ser Pro Arg Ala Arg Ala Val Ala Val Lys Gly Pro Val Gln Arg Tyr
            100             105             110

Pro Gly Asn Gln Thr Thr Cys
            115
```

<210> 10
<211> 119
<212> PRT
<213> Homo sapiens

<400> 10
```
Met Ala Gly Leu Met Thr Ile Val Thr Ser Leu Leu Phe Leu Gly Val
1               5               10                  15

Cys Ala His His Ile Ile Pro Thr Gly Ser Val Val Ile Pro Ser Pro
            20              25                  30

Cys Cys Met Phe Phe Val Ser Lys Arg Ile Pro Glu Asn Arg Val Val
        35              40                  45

Ser Tyr Gln Leu Ser Ser Arg Ser Thr Cys Leu Lys Ala Gly Val Ile
    50              55                  60

Phe Thr Thr Lys Lys Gly Gln Gln Phe Cys Gly Asp Pro Lys Gln Glu
65              70                  75                  80

Trp Val Gln Arg Tyr Met Lys Asn Leu Asp Ala Lys Gln Lys Lys Ala
                85                  90                  95

Ser Pro Arg Ala Arg Ala Val Ala Val Lys Gly Pro Val Gln Arg Tyr
            100             105             110

Pro Gly Asn Gln Thr Thr Cys
            115
```

<210> 11
<211> 109
<212> PRT
<213> Homo sapiens

<400> 11
```
Met Lys Phe Ile Ser Thr Ser Leu Leu Leu Met Leu Leu Val Ser Ser
1               5               10                  15

Leu Ser Pro Val Gln Gly Val Leu Glu Val Tyr Tyr Thr Ser Leu Arg
            20              25                  30
```

```
Cys Arg Cys Val Gln Glu Ser Ser Val Phe Ile Pro Arg Arg Phe Ile
        35                  40                  45


Asp Arg Ile Gln Ile Leu Pro Arg Gly Asn Gly Cys Pro Arg Lys Glu
        50                  55                  60


Ile Ile Val Trp Lys Lys Asn Lys Ser Ile Val Cys Val Asp Pro Gln
65                  70                  75                  80


Ala Glu Trp Ile Gln Arg Met Met Glu Val Leu Arg Lys Arg Ser Ser
                85                  90                  95


Ser Thr Leu Pro Val Pro Val Phe Lys Arg Lys Ile Pro
            100                 105
```

```
<210> 12
<211> 4
<212> PRT
<213> Homo sapiens

<400> 12
Ala Thr Leu Gly
1


<210> 13
<211> 5
<212> PRT
<213> Homo sapiens

<400> 13
Gly Cys Arg Leu Tyr
1               5
```